# EUROPEAN PATENT APPLICATION

(11) **EP 1 816 212 A2**
(43) Date of publication of application: **08.08.2007**
(21) Application number: 06024411.8
(22) Date of filing: 27.12.2001
(51) Int. Cl.: C12Q 1/68

(54) **Detection of genetic polymorphisms in genes associated with pharmacogenomics**

(30) Priority: 27.12.2000 JP 2000399443; 02.05.2001 JP 2001135256; 27.08.2001 JP 2001256862
(62) Divisional of application: 01272361.5
(71) Applicant: Riken, Wako-shi, Saitama 351-0198 (JP)
(72) Inventor: Nakamura, Yusuke, Yokohama-shi Kanagawa 225-0011 (JP); Sekine, Akihiro, Kunitachi-shi Tokyo 186-0001 (JP); Iida, Aritoshi, Kawasaki-shi Kanagawa 211-0014 (JP); Saito, Susumu, Ome-shi Tokyo 198-0042 (JP)
(74) Representative: Westphal, Thomas

(57) **Abstract**

The present invention relates to genetic polymorphism data, compositions and methods for detecting genetic polymorphisms, methods for evaluating drugs using genetic polymorphisms and screening methods for drugs.

## Description

The present invention claims priority to Japanese Patent Application Ser. Nos. 2000-399,443 filed December 27, 2000, 2001-135,256 field May 2, 2001, and 2001-256,862 filed August 27, 2001.

### FIELD OF THE INVENTION

The present invention relates to genetic polymorphism data, compositions and methods for detecting genetic polymorphisms, methods for evaluating drugs using genetic polymorphisms and screening methods for drugs.

### BACKGROUND

Human beings come in all shapes and sizes, and over three billion genetic codes are located in somewhat different sites in each human being. Individual DNA sequence variations in the human genome are known to directly cause specific diseases or conditions, to predispose certain individuals to specific diseases or conditions, and to affect responses of individuals to treatments such as drugs. Such variations also modulate the severity or progression of many diseases. Additionally, DNA sequences vary between populations. Therefore, determining DNA sequence variations in the human genome is useful for making accurate diagnoses, for finding suitable therapies, and for understanding the relationship between genome variations and environmental factors in the pathogenesis of diseases, the prevalence of conditions and the efficacy of therapies.

There are several types of DNA sequence variations in the human genome. These variations include insertions, deletions and copy number differences of repeated sequences. These differences in the genetic code are called genetic polymorphisms. The most common DNA sequence variations in the human genome are single base pair substitutions. These are generally referred to as single nucleotide polymorphisms (SNPs) when the variant allele has a population frequency of at least 1%. SNPs may be classified by where they appear in the genome. For example, a single nucleotide polymorphism may be classified as a coding SNP (cSNP) when it is in a region encoding a protein, or genome SNP (gSNP) when it is detected anywhere in a genome, without reference to whether it is in a coding region. Coding SNPs include silent SNPs (sSNP), and SNPs that may be in regions associated with coding sequences, such as regulatory regions or elements (*e.g*., regulatory SNPs, or rSNPs) and introns (*e.g*., intron SNPs, or iSNPs).

SNPs are particularly useful in studying the relationship between DNA sequence variations and human diseases, conditions and drug responses because SNPs are stable in populations, occur frequently, and have lower mutation rates than other genome variations such as repeating sequences. In addition, methods for detecting SNPs are more amenable to being automated and used for large-scale studies than methods for detecting other, less common DNA sequence variations.

Single nucleotide polymorphisms are useful as polymorphism markers for discovering genes that cause or exacerbate certain diseases. This is directly related in clinical medicine to diagnosing the risk for a disease and determining the proper pharmaceutical treatment. There is currently a worldwide effort going on to develop drugs based on the target genes that cause diseases. Individual patients also react differently when a drug is administered. In some patients, a drug may have a significant effect, in others a lesser effect and in still others no effect at all. In other words, there is a major difference in patient reactions to the same drug. Patients may also metabolize drugs at different rates. In addition to differences in therapeutic reactions among patients to drugs, there is also the possibility of strong and even fatal side effects due to genetically linked differences in, e.g., drug metabolism, drug transport or drug receptor function. Analysis of genetic polymorphisms such as SNPs allows for the selection of drugs and the development of treatment protocols tailored to each individual patient (so-called "personalized" medical treatments). Instead of the using trial-and-error methods of matching patients with the right drugs, doctors may, for example, be able to analyze a patient's genetic profile and prescribe the best available drug therapy from the beginning. Not only would this take the guesswork out of finding the right drug, it would reduce the likelihood of adverse reactions, thus increasing safety.

### SUMMARY OF THE INVENTION

The present invention identifies genetic polymorphisms relating to genes associated with drug metabolism. In some embodiments, the present invention provides methods for determining variations in sequences and genes associated with drug-metabolizing enzymes. In preferred embodiments, the present invention provides methods for collecting genetic polymorphism data for use in evaluating the effectiveness and safety of a drug based on the data, and screening drugs using the data. In some preferred embodiments, the polymorphisms of the present invention are used to evaluate a causal relationship between the genetic make-up of a patient and a response to an administered drug.

The present invention relates to genes encoding enzymes associated with drug metabolism (drug metabolizing enzymes, or DMEs). In particular, the present invention relates to sequence variations associated with variations in DMEs. In some embodiments, variations occur in coding regions of DMEs, such as may alter a function of the DMEs, (*e.g.,* by increasing or decreasing its level of activity, or shifting its activity to an alternative target or function). In other embodiments, the variations occur in non-coding regions of the genome, such as may alter expression of a DME (*e.g*., increasing or decreasing the amount of an enzyme produced in a cell) or processing of an RNA transcript encoding a DME (*e.g*., by altering splicing).

In some embodiments, the present invention provides methods for detecting DME-related sequence variations. In some preferred embodiments, the methods of the present invention are used to create a profile of DME-related polymorphisms in a test subject.

In other embodiments, the present invention provides isolated nucleic acid sequences encoding variant DMEs. For example, the present invention provides a recombinant DNA vector comprising DNA having a nucleotide sequence encoding a variant DME, the nucleotide sequence comprising a sequence including, but not limited to, SEQ ID NOS:1-3360 and 3361-7669, and substantially similar sequences. In a preferred embodiment, the invention provides a host cell transformed with a recombinant DNA vector comprising DNA having a nucleotide sequence encoding a variant DME. The invention is not limited by the nature of the host cell employed. The art is well aware of expression vectors suitable for the expression of nucleotide sequences encoding variant DMEs that can be expressed in a variety of prokaryotic and eukaryotic host cells. In some preferred embodiments, the host cell is a eukaryotic cell grown in culture, such as for use in *in vitro* drug screening (*e.g*., by monitoring the expression of genes associated with the pathways targeted by a particular test drug). In other preferred embodiments, the host cell is *in vivo.*

The present invention provides systems and methods for detection of polymorphisms associated with genes encoding enzymes associated with drug metabolism. The present invention is not limited in the nature of the detection assay used for detection or identification of such polymorphisms. Such detection assays include, but are not limited to, hybridization methods and array technologies (e.g., technologies available from Aclara BioSciences, Haywood, CA; Affymetrix, Santa Clara, CA; Agilent Technologies, Inc., Palo Alto, CA; Aviva Biosciences Corp., San Diego, CA; Caliper Technologies Corp., Palo Alto, CA; Celera, Rockville, MD; CuraGen Corp., New Haven, CT; Hyseq Inc., Sunnyvale, CA; Illumina, Inc., San Diego, CA; Incyte Genomics, Palo Alto, CA; Motorola BioChip Systems; Nanogen, San Diego, CA; Orchid BioSciences, Inc., Princeton, NJ; Applera Corp., Foster City, CA; Rosetta Inpharmatics, Kirkland, WA; and Sequenom, San Diego, CA); polymerase chain reaction-based methods (*e.g.*,TAQMAN, Applera Corp., GENECODE system, EraGen, Middleton, WI); branched hybridization methods; enzyme mismatch cleavage methods; NASBA; sandwich hybridization methods; methods employing molecular beacons; ligase chain reactions, and the like.

Methods of the present invention find application in improving the drug discovery and approval processes. For example, the costs and risks of drug development may be reduced if only those persons capable of responding to a drug are selected for clinical trials. In addition, previously failed drug candidates may be revived as they are matched with more appropriate patient populations. Decreases in the number of adverse drug reactions, the number of failed drug trials, the time it takes to get a drug approved, the length of time patients are on medication, the number of medications patients must take to find an effective therapy, and an increase in the range of possible drug targets will promote a net decrease in the cost of health care.

Thus, in some embodiments, the present invention provides a method of identifying individuals having a polymorphism, comprising providing nucleic acid from a subject; and detecting the presence of at least one polymorphism in said nucleic acid, said at least one polymorphism including, but not limited to, polymorphisms found in SEQ ID Nos:1-3360 and 3361-7669. In some embodiments, the method further provides the step of providing a prognosis (e.g., a genotype relative risk or a population attributable risk) to the subject based on the presence or absence of the at least one polymorphism. In some embodiments, the detecting step is carried out using a detection assay including, but not limited to, a hybridization assay, a TAQMAN assay, an invasive cleavage assay, use of mass spectroscopy, a microarray, a polymerase chain reaction, a rolling circle extension assay, a sequencing assay, a hybridization assay employing a probe complementary to a polymorphism, a bead array assay, a primer extension assay, an enzyme mismatch cleavage assay, a branched hybridization assay, a NASBA assay, a molecular beacon assay, a cycling probe assay, a ligase chain reaction assay, and a sandwich hybridization assay.

The present invention also provides a nucleic acid (e.g., a gene, a probe, a primer, etc.) comprising a sequence selected from the group consisting of SEQ ID NO: 1-3360 and 3361-7669 or complements thereof. In some embodiments, the nucleic acid molecule comprises a label. In some embodiments, the nucleic acid is attached to a solid support (e.g., as part of a microarray). The present invention also provides vectors comprising the nucleic acid and host cell comprising the vector, as well as polypeptide encoded by the nucleic acid. Methods of producing and purifying polypeptides are well known in the art.

The present invention further provides kits for detecting a polymorphism, comprising at least one reagent that specifically detects a polymorphism in a sequence including, but not limited to, SEQ ID Nos: 1-3360 and 3361-7669. In some embodiments, the kit further comprising instructions for determining whether the subject is at increased risk of having a drug metabolism disorder. In some embodiments, the at least one reagent comprises a nucleic acid probe. The kits can be configured for a variety of uses including, but not limited to, use as an in vitro diagnostic detection assay, an analyte specific reagent detection assay, and a research-use-only detection assay.

The present invention also provides a method for screening subjects for genetic markers associated with drug metabolizing enzyme(s), comprising: a) providing a biological sample comprising a nucleic acid from a subject; b) testing the nucleic acid for a polymorphism in a genetic marker associated with a drug metabolizing enzyme, said genetic marker comprising one or more nucleotide polymorphisms designated by n, said n selected from a base substitution, an insertion, or a deletion found in a sequence selected from the group consisting of SEQ ID Nos:1-3360 and 3361-7669. The present invention is not limited by the source of the nucleic acid. In some embodiments, the biological sample comprises blood, saliva, amniotic fluid, and tissue. In some embodiments, the subject is a human. In some preferred embodiments, the nucleic acid comprises DNA and/or RNA.

The present invention further provides a composition comprising an array of detection assays, said array comprising a plurality of drug metabolizing enzyme nucleotide polymorphism detection assays, one or more of said detection assays being capable of detecting one or more nucleotide polymorphisms designated by n in SEQ ID Nos:1-3360 and 3361-7669, wherein n represents a base substitution, insertion, or deletion compared to a wild-type sequence.

The present invention also provides a composition comprising a detection probe for determining the presense or absence a single nucleotide polymorphism in a gene encoding a drug metabolizing enzyme, said gene comprising a sequence selected from the group consisting of SEQ ID Nos:1-3360 and 3361-7669.

The present invention further provides a method of determining the effectiveness of or side-effect of a drug or treatment protocol, comprising; a) administering a drug or treatment protocol to one or more subjects; b) obtaining nucleic acid from said one or more subjects; c) using a detection assay to detect the presence of at least one polymorphism in said nucleic acid from said one or more of subjects, said at least one polymorphism selected from the group consisting of polymorphisms found in SEQ ID Nos:1-3360 and 3361-7669; and d) assigning an effectiveness rating, side-effect rating, or score for said drug or treatment protocol based upon a result of one or more said detection assays *(See e.g.,* Toxicology Testing Handbook: Principles, Applications, and Data Interpretation, ed. Jacobson-Kram and Keller, 2001, herein incorporated by reference in its entirety).

The present invention also provides a method of prescribing a drug to or treatment protocol for a subject, comprising; providing nucleic acid from said subject; using a detection assay to detect the presence of at least one polymorphism in the nucleic acid, said at least one polymorphism selected from the group consisting of polymorphisms found in SEQ ID Nos:1-3360 and 3361-7669; and, prescribing said drug or treatment protocol based upon the result of said detection assay.

The present invention further provides a method for generating assay data comprising: obtaining a sample from a subject containing nucleic acid; transferring said sample to a laboratory; and receiving data from said laboratory, wherein said data corresponds to the presence of at least one polymorphism in said nucleic acid, said at least one polymorphism selected from the group consisting of polymorphisms found in SEQ ID Nos:1-3360 and 3361-7669. The present further provides data sets generated by this method.

### DEFINITIONS

To facilitate an understanding of the present invention, a number of terms and phrases are defined below:

As used herein, the terms "complementary" or "complementarity" are used in reference to polynucleotides (*i.e*., a sequence of nucleotides such as an oligonucleotide or a target nucleic acid) related by the base-pairing rules. For example, for the sequence " 5'-A-G-T-3'," is complementary to the sequence " 3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids. Either term may also be used in reference to individual nucleotides, especially within the context of polynucleotides. For example, a particular nucleotide within an oligonucleotide may be noted for its complementarity, or lack thereof, to a nucleotide within another nucleic acid strand, in contrast or comparison to the complementarity between the rest of the oligonucleotide and the nucleic acid strand. Nucleotide analogs used to form non-standard base pairs, whether with another nucleotide analog (e.g., an IsoC/IsoG base pair), or with a naturally occurring nucleotide (e.g., as described in U.S. Patent 5,912,340, herein incorporated by reference in its entirety) are also considered to be complementary to a base pairing partner within the meaning this definition.

The term "homology" and "homologous" refers to a degree of identity. There may be partial homology or complete homology. A partially homologous sequence is one that is less than 100% identical to another sequence.

As used herein, the term "hybridization" is used in reference to the pairing of complementary nucleic acids. Hybridization and the strength of hybridization (i.e., the strength of the association between the nucleic acids) is influenced by such factors as the degree of complementary between the nucleic acids, stringency of the conditions involved, and the Tₘ of the formed hybrid. "Hybridization" methods involve the annealing of one nucleic acid to another, complementary nucleic acid, i.e., a nucleic acid having a complementary nucleotide sequence. The ability of two polymers of nucleic acid containing complementary sequences to find each other and anneal through base pairing interaction is a well-recognized phenomenon. The initial observations of the "hybridization" process by Marmur and Lane, Proc. Natl. Acad. Sci. USA 46:453 (1960) and Doty et al., Proc. Natl. Acad. Sci. USA 46:461 (1960) have been followed by the refinement of this process into an essential tool of modern biology.

With regard to complementarity, it is important for some diagnostic applications to determine whether the hybridization represents complete or partial complementarity. For example, where it is desired to detect simply the presence or absence of a foreign DNA sequence, it is only important that the hybridization method ensures hybridization when the relevant sequence is present; conditions can be selected where both partially complementary probes and completely complementary probes will hybridize. Other diagnostic applications, however, may require that the hybridization method distinguish between partial and complete complementarity. It may be of interest to detect genetic polymorphisms. For example, human hemoglobin is composed, in part, of four polypeptide chains. Two of these chains are identical chains of 141 amino acids (alpha chains) and two of these chains are identical chains of 146 amino acids (beta chains). The gene encoding the beta chain is known to exhibit polymorphism. The normal allele encodes a beta chain having glutamic acid at the sixth position. The mutant allele encodes a beta chain having valine at the sixth position. This difference in amino acids has a profound (most profound when the individual is homozygous for the mutant allele) physiological impact known clinically as sickle cell anemia. It is well known that the genetic basis of the amino acid change involves a single base difference between the normal allele DNA sequence and the mutant allele DNA sequence.

The complement of a nucleic acid sequence as used herein refers to an oligonucleotide which, when aligned with the nucleic acid sequence such that the 5' end of one sequence is paired with the 3' end of the other, is in "antiparallel association." Certain bases not commonly found in natural nucleic acids may be included in the nucleic acids of the present invention and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs.

As used herein, the term "Tₘ" is used in reference to the "melting temperature." The melting temperature is the temperature at which a population of double-stranded nucleic acid molecules becomes half dissociated into single strands. Several equations for calculating the Tₘ of nucleic acids are well known in the art. As indicated by standard references, a simple estimate of the Tₘ value may be calculated by the equation: Tₘ = 81.5 + 0.41(% G + C), when a nucleic acid is in aqueous solution at 1 M NaCl (*see e.g.,* Anderson and Young, Quantitative Filter Hybridization, in Nucleic Acid Hybridization (1985). Other references (*e.g*., Allawi, H.T. & SantaLucia, J., Jr. Thermodynamics and NMR of internal G.T mismatches in DNA. Biochemistry 36, 10581-94 (1997) include more sophisticated computations which take structural and environmental, as well as sequence characteristics into account for the calculation of Tₘ.

As used herein the term "stringency" is used in reference to the conditions of temperature, ionic strength, and the presence of other compounds, under which nucleic acid hybridizations are conducted. With "high stringency" conditions, nucleic acid base pairing will occur only between nucleic acid fragments that have a high frequency of complementary base sequences. Thus, conditions of "weak" or "low" stringency are often required when it is desired that nucleic acids that are not completely complementary to one another be hybridized or annealed together.

"High stringency conditions" when used in reference to nucleic acid hybridization comprise conditions equivalent to binding or hybridization at 42 C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42 C when a probe of about 500 nucleotides in length is employed.

"Medium stringency conditions" when used in reference to nucleic acid hybridization comprise conditions equivalent to binding or hybridization at 42 C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE,. 1.0% SDS at 42 C when a probe of about 500 nucleotides in length is employed.

"Low stringency conditions" comprise conditions equivalent to binding or hybridization at 42 C in a solution consisting of 5X SSPE (43.8 g/l NaCl, 6.9 g/l NaH₂PO₄ H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 g/ml denatured salmon sperm DNA followed by washing in a solution comprising 5X SSPE, 0.1 % SDS at 42 C when a probe of about 500 nucleotides in length is employed.

The term "gene" refers to a DNA sequence that comprises control and coding sequences necessary for the production of an RNA having a non-coding function (e.g., a ribosomal or transfer RNA), a polypeptide or a precursor. The RNA or polypeptide can be encoded by a full-length coding sequence or by any portion of the coding sequence so long as the desired activity or function is retained.

The term "wild-type" refers to a gene or a gene product that has the characteristics of that gene or gene product when isolated from a naturally occurring source. A wild-type gene is that which is most frequently observed in a population and is thus arbitrarily designated the "normal" or "wild-type" form of the gene. In contrast, the term "modified," "mutant," or "polymorphic" refers to a gene or gene product that displays modifications in sequence and or functional properties (*i.e*., altered characteristics) when compared to the wild-type gene or gene product. It is noted that naturally-occurring mutants can be isolated; these are identified by the fact that they have altered characteristics when compared to the wild-type gene or gene product.

The term "oligonucleotide" as used herein is defined as a molecule comprising two or more deoxyribonucleotides or ribonucleotides, preferably at least 5 nucleotides, more preferably at least about 10-15 nucleotides and more preferably at least about 15 to 30 nucleotides. The exact size will depend on many factors, which in turn depend on the ultimate function or use of the oligonucleotide. The oligonucleotide may be generated in any manner, including chemical synthesis, DNA replication, reverse transcription, PCR, or a combination thereof.

Because mononucleotides are reacted to make oligonucleotides in a manner such that the 5' phosphate of one mononucleotide pentose ring is attached to the 3' oxygen of its neighbor in one direction via a phosphodiester linkage, an end of an oligonucleotide is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have 5' and 3' ends. A first region along a nucleic acid strand is said to be upstream of another region if the 3' end of the first region is before the 5' end of the second region when moving along a strand of nucleic acid in a 5' to 3' direction.

When two different, non-overlapping oligonucleotides anneal to different regions of the same linear complementary nucleic acid sequence, and the 3' end of one oligonucleotide points towards the 5' end of the other, the former may be called the "upstream" oligonucleotide and the latter the "downstream" oligonucleotide. Similarly, when two overlapping oligonucleotides are hybridized to the same linear complementary nucleic acid sequence, with the first oligonucleotide positioned such that its 5' end is upstream of the 5' end of the second oligonucleotide, and the 3' end of the first oligonucleotide is upstream of the 3' end of the second oligonucleotide, the first oligonucleotide may be called the "upstream" oligonucleotide and the second oligonucleotide may be called the "downstream" oligonucleotide.

The term "primer" refers to an oligonucleotide that is capable of acting as a point of initiation of synthesis when placed under conditions in which primer extension is initiated. An oligonucleotide "primer" may occur naturally, as in a purified restriction digest or may be produced synthetically.

A primer is selected to be "substantially" complementary to a strand of specific sequence of the template. A primer must be sufficiently complementary to hybridize with a template strand for primer elongation to occur. A primer sequence need not reflect the exact sequence of the template. For example, a non-complementary nucleotide fragment may be attached to the 5' end of the primer, with the remainder of the primer sequence being substantially complementary to the strand. Non-complementary bases or longer sequences can be interspersed into the primer, provided that the primer sequence has sufficient complementarity with the sequence of the template to hybridize and thereby form a template primer complex for synthesis of the extension product of the primer.

The term "label" as used herein refers to any atom or molecule that can be used to provide a detectable (preferably quantifiable) effect, and that can be attached to a nucleic acid or protein. Labels include but are not limited to dyes; radiolabels such as ³²P; binding moieties such as biotin; haptens such as digoxgenin; luminogenic, phosphorescent or fluorogenic moieties; and fluorescent dyes alone or in combination with moieties that can suppress or shift emission spectra by fluorescence resonance energy transfer (FRET). Labels may provide signals detectable by fluorescence, radioactivity, colorimetry, gravimetry, X-ray diffraction or absorption, magnetism, enzymatic activity, and the like. A label may be a charged moiety (positive or negative charge) or alternatively, may be charge neutral. Labels can include or consist of nucleic acid or protein sequence, so long as the sequence comprising the label is detectable.

The term "signal" as used herein refers to any detectable effect, such as would be caused or provided by a label or an assay reaction.

As used herein, the term "detector" refers to a system or component of a system, e.g., an instrument (e.g. a camera, fluorimeter, charge-coupled device, scintillation counter, etc.) or a reactive medium (X-ray or camera film, pH indicator, etc.), that can convey to a user or to another component of a system (e.g., a computer or controller) the presence of a signal or effect. A detector can be a photometric or spectrophotometric system, which can detect ultraviolet, visible or infrared light, including fluorescence or chemiluminescence; a radiation detection system; a spectroscopic system such as nuclear magnetic resonance spectroscopy, mass spectrometry or surface enhanced Raman spectrometry; a system such as gel or capillary electrophoresis or gel exclusion chromatography; or other detection systems known in the art, or combinations thereof.

The term "sequence variation" as used herein refers to differences in nucleic acid sequence between two nucleic acids. For example, a wild-type structural gene and a mutant form of this wild-type structural gene may vary in sequence by the presence of single base substitutions and/or deletions or insertions of one or more nucleotides. These two forms of the structural gene are said to vary in sequence from one another. A second mutant form of the structural gene may exist. This second mutant form is said to vary in sequence from both the wild-type gene and the first mutant form of the gene.

The term "nucleotide analog" as used herein refers to modified or non-naturally occurring nucleotides such as 7-deaza purines (*i.e*., 7-deaza-dATP and 7-deaza-dGTP). Nucleotide analogs include base analogs and comprise modified forms of deoxyribonucleotides as well as ribonucleotides.

The term "polymorphism" refers to the coexistence of more than one form of a gene or portion thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

The term "polymorphic locus" is a locus present in a population that shows variation between members of the population (*e.g*., the most common allele has a frequency of less than 0.95). In contrast, a "monomorphic locus" is a genetic locus at little or no variations seen between members of the population (generally taken to be a locus at which the most common allele exceeds a frequency of 0.95 in the gene pool of the population).

A "non-human animal" of the invention can include mammals such as rodents, non-human primates, sheep, goats, horses, dogs, cows, chickens, amphibians, reptiles, etc. Preferred non-human animals are selected from the rodent family including rat and mouse, most preferably mouse, though transgenic amphibians, such as members of the Xenopus genus, and transgenic chickens can also provide important tools for understanding and identifying drugs that can affect processes, *e.g*., embryogenesis and tissue formation.

The term "operably linked" is intended to mean that the promoter is associated with the nucleic acid in such a manner as to facilitate transcription of the nucleic acid from the promoter.

The terms "protein", "polypeptide" and "peptide" are used interchangeably herein when referring to a gene product.

The term "recombinant protein" refers to a polypeptide which is produced by recombinant DNA techniques, wherein generally, DNA encoding the polypeptide is inserted into a suitable expression vector which is in turn used to transform a host cell to produce the heterologous protein.

A "regulatory element", also termed herein "regulatory sequence" is intended to include elements which are capable of modulating transcription from a basic promoter and include elements such as enhancers and silencers. The term "enhancer", also referred to herein as "enhancer element", is intended to include regulatory elements capable of increasing, stimulating, or enhancing transcription from a basic promoter. The term "silencer", also referred to herein as "silencer element" is intended to include regulatory elements capable of decreasing, inhibiting, or repressing transcription from a basic promoter. Regulatory elements are typically present in 5' flanking regions of genes. However, regulatory elements have also been shown to be present in other regions of a gene, in particular in introns. Regulatory elements may also be present downstream of coding regions. Thus, it is possible that DME genes have regulatory elements located in introns, exons, coding regions, and 3' flanking sequences. Such regulatory elements are also intended to be encompassed by the present invention and polymorphisms in such elements can be identified by any of the assays that can be used to identify polymorphisms in regulatory elements in 5' flanking regions of genes.

The term "regulatory element" further encompasses "tissue specific" regulatory elements, i.e., regulatory elements that affect expression of a DME gene preferentially in specific cells (*e.g*., cells of a specific tissue). Gene expression occurs preferentially in a specific cell if expression in this cell type is significantly higher than expression in other cell types. The term "regulatory element" also encompasses non-tissue specific regulatory elements, i.e., regulatory elements that are active in most cell types. Furthermore, a regulatory element can be a constitutive regulatory element, *i.e.,* a regulatory element that constitutively regulates transcription, as opposed to a regulatory element that is inducible, *i.e*., a regulatory element which is active primarily in response to a stimulus. A stimulus can be, *e.g*., a molecule, such as a hormone, cytokine, heavy metal, phorbol ester, cyclic AMP (cAMP), or retinoic acid.

As used herein, the term "transfection" means the introduction of a nucleic acid, e.g., an expression vector, into a recipient cell by nucleic acid-mediated gene transfer. The term "transduction" is generally used herein when the transfection with a nucleic acid is by viral delivery of the nucleic acid. "Transformation", as used herein, refers to a process in which a cell's genotype is changed as a result of the cellular uptake of exogenous DNA or RNA, and, for example, the transformed cell expresses a recombinant form of a polypeptide or, in the case of anti-sense expression from the transferred gene, the expression of a naturally-occurring form of the recombinant protein is disrupted.

As used herein, the term "transgene" refers to a nucleic acid sequence that has been introduced into a cell. Daughter cells deriving from a cell in which a transgene has been introduced are also said to contain the transgene (unless it has been deleted). A transgene can encode, e.g., a polypeptide, or an antisense transcript, partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). Alternatively, a transgene can also be present in an episome. A transgene can include one or more transcriptional regulatory sequence and any other nucleic acid, (e.g. intron), that may be necessary for optimal expression of a selected nucleic acid.

A "transgenic animal" refers to any animal, preferably a non-human animal, e.g. a mammal, bird or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or in vitro fertilization, but rather is directed to the introduction of a recombinant DNA molecule. This molecule may be integrated within a chromosome, or it may be extrachromosomally replicating DNA. In the typical transgenic animals described herein, the transgene causes cells to express a recombinant form of one of a protein, e.g. either agonistic or antagonistic forms. However, transgenic animals in which the recombinant gene is silent are also contemplated. Moreover, "transgenic animal" also includes those recombinant animals in which gene disruption of one or more genes is caused by human intervention, including both recombination and antisense techniques.

The term "treating" as used herein is intended to encompass curing as well as ameliorating at least one symptom of the condition or disease.

The term "sample" in the present specification and claims is used in its broadest sense. On the one hand it is meant to include a biological (*e.g*., human) specimen. On the other hand, a sample may include a specimen of synthetic origin.

Biological samples may be animal, including human, fluid, solid (*e.g*., stool) or tissue, as well as liquid and solid food and feed products and ingredients such as dairy items, vegetables, meat and meat by-products, and waste. Biological samples may be obtained from all of the various families of domestic animals, as well as feral or wild animals, including, but not limited to, such animals as ungulates, bear, fish, lagamorphs, rodents, etc.

The term "source of target nucleic acid" refers to any sample that contains or is suspected to contain nucleic acids (RNA or DNA). Particularly preferred sources of target nucleic acids are biological samples including, but not limited to blood, saliva, cerebral spinal fluid, pleural fluid, milk, lymph, sputum and semen.

The term "polymerization means" or "polymerization agent" refers to any agent capable of facilitating the addition of nucleoside triphosphates to an oligonucleotide. Preferred polymerization means comprise DNA and RNA polymerases.

The term "ligation means" or "ligation agent" refers to any agent capable of facilitating the ligation (i.e., the formation of a phosphodiester bond between a 3'-OH and a 5' P located at the termini of two strands of nucleic acid). Preferred ligation means comprise DNA ligases and RNA ligases.

The term "reactant" is used herein in its broadest sense. The reactant can comprise, for example, an enzymatic reactant, a chemical reactant or light (e.g., ultraviolet light, particularly short wavelength ultraviolet light is known to break oligonucleotide chains). Any agent capable of reacting with an oligonucleotide to either shorten (i.e., cleave) or elongate the oligonucleotide is encompassed within the term "reactant."

The term "nucleic acid sequence" as used herein refers to an oligonucleotide, nucleotide or polynucleotide, and fragments or portions thereof, and to DNA or RNA of genomic or synthetic origin that may be single or double stranded, and represent the sense or antisense strand. Similarly, "amino acid sequence" as used herein refers to peptide or protein sequence.

The term "peptide nucleic acid" ("PNA") as used herein refers to a molecule comprising bases or base analogs such as would be found in natural nucleic acid, but attached to a peptide backbone rather than the sugar-phosphate backbone typical of nucleic acids. The attachment of the bases to the peptide is such as to allow the bases to base pair with complementary bases of nucleic acid in a manner similar to that of an oligonucleotide. These small molecules, also designated anti gene agents, stop transcript elongation by binding to their complementary strand of nucleic acid (Nielsen, *et al.* Anticancer Drug Des. 8:53 63 [1993]).

As used herein, the terms "purified" or "substantially purified" refer to molecules, either nucleic or amino acid sequences, that are removed from their natural environment, isolated or separated, and are at least 60% free, preferably 75% free, and most preferably 90% free from other components with which they are naturally associated. An "isolated polynucleotide" or "isolated oligonucleotide" is therefore a substantially purified polynucleotide.

As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to a delivery systems comprising two or more separate containers that each contain a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

As used herein, the term "information" refers to any collection of facts or data. In reference to information stored or processed using a computer system(s), including but not limited to internets, the term refers to any data stored in any format (e.g., analog, digital, optical, etc.). As used herein, the term "information related to a subject" refers to facts or data pertaining to a subject (e.g., a human, plant, or animal). The term "genomic information" refers to information pertaining to a genome including, but not limited to, nucleic acid sequences, genes, allele frequencies, RNA expression levels, protein expression, phenotypes correlating to genotypes, etc. "Allele frequency information" refers to facts or data pertaining allele frequencies, including, but not limited to, allele identities, statistical correlations between the presence of an allele and a characteristic of a subject (e.g., a human subject), the presence or absence of an allele in a individual or population, the percentage likelihood of an allele being present in an individual having one or more particular characteristics, etc.

The term "cleavage structure" as used herein, refers to a structure that is formed by the interaction of at least one probe oligonucleotide and a target nucleic acid, forming a structure comprising a duplex, the resulting structure being cleavable by a cleavage agent, including but not limited to an enzyme. The cleavage structure is a substrate for specific cleavage by the cleavage means in contrast to a nucleic acid molecule that is a substrate for non-specific cleavage by agents such as phosphodiesterases that cleave nucleic acid molecules without regard to secondary structure (i.e., no formation of a duplexed structure is required).

### DESCRIPTION OF THE DRAWINGS

Figure 1 shows sample embodiments of TAQMAN probes.
Figure 2 represents one embodiment of the TAQMAN PCR method.
Figure 3 shows examples of probes labeled with fluorescent dyes.
Figure 4 shows a sample embodiment of an invasive cleavage structure, e.g., for an INVADER assay.
Figure 5 shows one embodiment of a FRET probe, e.g., for an INVADER assay.
Figure 6 shows one embodiment of an INVADER assay.
Figure 7 shows a diagram of an INVADER assay probe in which the allele does not match the probe.

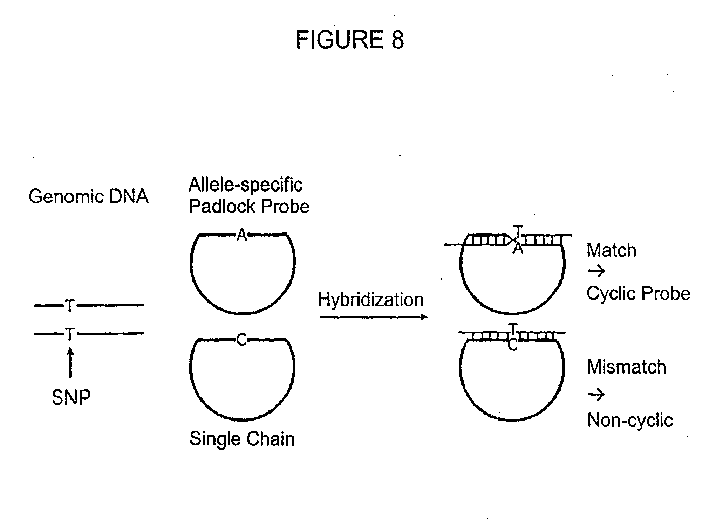

Figure 8 shows one embodiment of allele identification using a ligation reaction.
Figure 9 shows a drawing of the structure of and SNP position in the ATP-binding cassette subfamily B member 2 (ABCB2) gene.
Figure 10 shows a drawing of the structure of and SNP position in the ATP-binding cassette subfamily B member 4 (ABCB4) gene.
Figure 11 shows a drawing of the structure of and SNP position in the microsomal epoxide hydrogenase 1 (EPHX1) gene.
Figure 12 shows a drawing of the structure of and SNP position in the cytoplasmic epoxide hydrogenase 2 (EPHX2) gene.
Figure 13 shows a drawing of the structure of and SNP position in the guanidinoacetate-N-methyltransferase (GAMT) gene.
Figure 14 shows a drawing of the structure of and SNP position in the nicotinamide-N-methyltransferase (NNMT) gene.
Figure 15 shows a drawing of the structure of and SNP position in the phenylethanolamine-N-methyltransferase (PNMT) gene.
Figure 16 shows a drawing of the structure of and SNP position in the phosphatidylethanolamine-N-methyltransferase (PEMT) gene.
Figure 17 shows a drawing of the structure of and SNP position in the glutathione-S-methyltransferase 3 (GSTM3) gene.
Figure 18 shows a drawing of the structure of and SNP position in the aldehyde dehydrogenase 5 (ALDH5) gene.
Figure 19 shows a drawing of the structure of and SNP position in the transglutaminase (TGM1) gene.
Figure 20 shows a drawing of the structure of and SNP position in the gamma glutamyltransferase (GGT1) gene.
Figure 21 shows a drawing of the structure of and SNP position in the NAD(P)H: quinone oxidetransferase (NQ01) gene.
Figure 22 shows a drawing of the structure of and SNP position in the p53-induced gene 3 (PIG3) of a quinone oxide transferase homologue.
Figure 23 shows a drawing of the structure of and SNP position in the NRH: quinone oxide transferase 2 (NQ02) gene.
Figure 24 shows a drawing of the structure of and SNP position in the sulfotransferase 1A1 (SULTIA1/STP1) gene.
Figure 25 shows a drawing of the structure of and SNP position in the sulfotransferase 1A2 (SULT1A2/STP2) gene.
Figure 26 shows a drawing of the structure of and SNP position in the sulfotransferase-related protein 3 (SULTX3) gene.
Figure 27 shows a drawing of the structure of and SNP position in the tyrosyl protein sulfotransferase (TPST1) gene.
Figure 28 shows a drawing of the structure of and SNP position in the tyrosyl protein sulfotransferase (TPST2) gene.
Figure 29 shows a drawing of the structure of and SNP position in the sulfotransferase 1A3 (SULT1A3/ STM/HAST) gene.
Figure 30 shows a drawing of the structure of and SNP position in the cerebroside transferase (CST) gene.
Figure 31 shows a drawing of the structure of and SNP position in the sulfotransferase 1C1 (SULT1C1) gene.
Figure 32 shows a drawing of the structure of and SNP position in the sulfotransferase 1C2 (SULT1C2) gene.
Figure 33 shows a drawing of the structure of and SNP position in the thyroid hormone sulfotransferase (ST1B2) gene.
Figure 34 shows a drawing of the structure of and SNP position in the hydrocarbon sulfotransferase 2 (CHST2) gene.
Figure 35 shows a drawing of the structure of and SNP position in the sulfotransferase 2A1 (SULT2A1) gene.
Figure 36 shows a drawing of the structure of and SNP position in the sulfotransferase 2B1 (SULT2B1) gene.
Figure 37 shows a drawing of the structure of and SNP position in the hydrocarbon sulfotransferase 4 (CHST4) gene.
Figure 38 shows a drawing of the structure of and SNP position in the hydrocarbon sulfotransferase 5 (CHST5) gene.
Figure 39 shows a drawing of the structure of and SNP position in the HNK-sulfotransferase (NHK-1ST) gene.
Figure 40 shows a drawing of the structure of and SNP position in the estrogen sulfotransferase (STE) gene.
Figure 41 shows a drawing of the structure of and SNP position in the alcohol dehydrogenase 1 (ADH1) gene.
Figure 42 shows a drawing of the structure of and SNP position in the alcohol dehydrogenase 2 (ADH2) gene.
Figure 43 shows a drawing of the structure of and SNP position in the alcohol dehydrogenase 3 (ADH3) gene.
Figure 44 shows a drawing of the structure of and SNP position in the alcohol dehydrogenase 6 (ADH6) gene.
Figure 45 shows a drawing of the structure of and SNP position in the alcohol dehydrogenase 7 (ADH7) gene.
Figure 46 shows a drawing of the structure of and SNP position in the short-chained alcohol dehydrogenase family (HEP27) gene.
Figure 47 shows a drawing of the structure of and SNP position in the L1 intracellular adhesion molecule (L1CAM) gene.
Figure 48 shows a drawing of the structure of and SNP position in the arylalkylamine-N-acetyltransferase (AANAT) gene.
Figure 49 shows a drawing of the structure of and SNP position in the N-actyltransferase homologue (ARD1) gene of Saccharomyces cerevisiae.
Figure 50 shows a drawing of the structure of and SNP position in the N-actyltransferase 1 (NAT1) gene.

Figure 51 shows a drawing of the structure of and SNP position in the N-actyltransferase 2 (NAT2) gene.
Figure 52 shows a drawing of the structure of and SNP position in the granzyme A (GZMA) gene.
Figure 53 shows a drawing of the structure of and SNP position in the granzyme B (GZMB) gene.
Figure 54 shows a drawing of the structure of and SNP position in the esterase D-formylglutathione hydrolase (ESD) gene.
Figure 55 shows a drawing of the structure of and SNP position in the dolichyl-diphosphooligosaccharide-protein glycosyltransferase (DDOST) gene.
Figure 56 shows a drawing of the structure of and SNP position in the microsomal glutathione-S-transferase (MGST1) gene.
Figure 57 shows a drawing of the structure of and SNP position in the alcohol dehydrogenase 5 (ADH5) gene.
Figure 58 shows a drawing of the structure of and SNP position in the glutathione-S-transferase M1 (GSTM1) gene.
Figure 59 shows a drawing of the structure of and SNP position in the glutathione-S-transferase M2 (GSTM2) gene.
Figure 60 shows a drawing of the structure of and SNP position in the glutathione-S-transferase M4 (GSTM4) gene.
Figure 61 shows a drawing of the structure of and SNP position in the glutathione-S-transferase Z1 (GSTZ1) gene.
Figure 62 shows a drawing of the structure of and SNP position in the glutathione-S-transferase P (GSTZPi) gene.
Figure 63 shows a drawing of the structure of and SNP position in the glutathione-S-transferase q1 (GSTT1) gene.
Figure 64 shows a drawing of the structure of and SNP position in the microsomal glutathione-S-transferase 1L1 (MGST1L1) gene.
Figure 65 shows a drawing of the structure of and SNP position in the microsomal glutathione-S-transferase 2 (MGST2) gene.
Figure 66 shows a drawing of the structure of and SNP position in the microsomal glutathione-S-transferase 3 (MGST3) gene.
Figure 67 shows a drawing of the structure of and SNP position in the glutathione-S-transferase A1 (GSTA1) gene.
Figure 68 shows a drawing of the structure of and SNP position in the glutathione-S-transferase A4 (GSTA4) gene.
Figure 69 shows a drawing of the structure of and SNP position in the NADH-ubiquinone oxide reductase 1a subcomplex 1 (NDUFA1) gene.
Figure 70 shows a drawing of the structure of and SNP position in the NADH-ubiquinone oxide reductase 1a subcomplex 2 (NDUFA2) gene.
Figure 71 shows a drawing of the structure of and SNP position in the NADH-ubiquinone oxide reductase 1a subcomplex 3 (NDUFA3) gene.
Figure 72 shows a drawing of the structure of and SNP position in the NADH-ubiquinone oxide reductase 1a subcomplex 5 (NDUFA5) gene.
Figure 73 shows a drawing of the structure of and SNP position in the NADH-ubiquinone oxide reductase 1a subcomplex 6 (NDUFA6) gene.
Figure 74 shows a drawing of the structure of and SNP position in the NADH-ubiquinone oxide reductase 1a subcomplex 7 (NDUFA7) gene.
Figure 75 shows a drawing of the structure of and SNP position in the NADH-ubiquinone oxide reductase 1a subcomplex 8 (NDUFA8) gene.
Figure 76 shows a drawing of the structure of and SNP position in the NADH-ubiquinone oxide reductase 1a/b subcomplex 1 (NDUFAB1) gene.
Figure 77 shows a drawing of the structure of and SNP position in the NADH-ubiquinone oxide reductase 1a subcomplex 9 (NDUFA9) gene.
Figure 78 shows a drawing of the structure of and SNP position in the NADH-ubiquinone oxide reductase Fe-S protein 1 (NDUFS1) gene.
Figure 79 shows a drawing of the structure of and SNP position in the NADH-ubiquinone oxide reductase Fe-S protein 3 (NDUFS3) gene.
Figure 80 shows a drawing of the structure of and SNP position in the NADH-ubiquinone oxide reductase Fe-S protein 4 (NDUFS4) gene.
Figure 81 shows a drawing of the structure of and SNP position in the NADH-ubiquinone oxide reductase Fe-S protein 5 (NDUFS5) gene.
Figure 82 shows a drawing of the structure of and SNP position in the NADH-ubiquinone oxide reductase Fe-S protein 6 (NDUFS6) gene.
Figure 83 shows a drawing of the structure of and SNP position in the NADH-ubiquinone oxide reductase Fe-S protein 8 (NDUFS8) gene.
Figure 84 shows a drawing of the structure of and SNP position in the NADH-ubiquinone oxide reductase 1b subcomplex 3 (NDUFB3) gene.
Figure 85 shows a drawing of the structure of and SNP position in the NADH-ubiquinone oxide reductase 1b subcomplex 5 (NDUFB5) gene.
Figure 86 shows a drawing of the structure of and SNP position in the NADH-ubiquinone oxide reductase 1b subcomplex 7 (NDUFB7) gene.
Figure 87 shows a drawing of the structure of and SNP position in the ATP-binding cassette subfamily A member 1 (ABCA1) gene.
Figure 88 shows a drawing of the structure of and SNP position in the catechol-0-methyltransferase (COMT) gene.
Figure 89 shows a drawing of the structure of and SNP position in the vitamin-N-transferase (HNMT) gene.
Figure 90 shows a drawing of the structure of and SNP position in the cytochrome P450 subfamily 1 (aromatic compound-induced) polypeptide 1 (CYP1A1) gene.
Figure 91 shows a drawing of the structure of and SNP position in the cytochrome P450 subfamily 1 (aromatic compound-induced) polypeptide 2 (CYP1A2) gene.
Figure 92 shows a drawing of the structure of and SNP position in the cytochrome P450 subfamily 1 (dioxin-induced) polypeptide 1 (CYP1B1) gene.
Figure 93 shows a drawing of the structure of and SNP position in the arylacetamide deactylase (AADAC) gene.
Figure 94 shows a drawing of the structure of and SNP position in the neuropathy target esterase (NTE) gene.
Figure 95 shows a drawing of the structure of and SNP position in the ATP-binding cassette subfamily C (CFTR/MRP) member 2 (MRP2) gene.
Figure 96 shows a drawing of the structure of and SNP position in the ATP-binding cassette subfamily B member 1 (ABCB1) gene.
Figure 97 shows a drawing of the structure of and SNP position in the ATP-binding cassette subfamily B member 3 (ABCB3) gene.
Figure 98 shows a drawing of the structure of and SNP position in the ATP-binding cassette subfamily B member 7 (ABCB7) gene.
Figure 99 shows a drawing of the structure of and SNP position in the ATP-binding cassette subfamily B member 8 (ABCB8) gene.
Figure 100 shows a drawing of the structure of and SNP position in the ATP-binding cassette subfamily B member 9 (ABCB9) gene.

Figure 101 shows a drawing of the structure of and SNP position in the ATP-binding cassette subfamily B member 10 (ABCB10) gene.
Figure 102 shows a drawing of the structure of and SNP position in the ATP-binding cassette subfamily B member 11 (ABCB11) gene.
Figure 103 shows a drawing of the structure of and SNP position in the cytochrome P450 subfamily IVB polypeptide 1 (CYP4B1) gene.
Figure 104 shows a drawing of the structure of and SNP position in the cytochrome P450 subfamily XXVIIA polypeptide 1 (CYP27A1) gene.
Figure 105 shows a drawing of the structure of and SNP position in the cytochrome P450 subfamily IVF polypeptide 1 (CYP4F2) gene.
Figure 106 shows a drawing of the structure of and SNP position in the cytochrome P450 subfamily 4F polypeptide 3 (CYP4F3) gene.
Figure 107 shows a drawing of the structure of and SNP position in the cytochrome P450 subfamily 4F polypeptide 8 (CYP4F8) gene.
Figure 108 shows a drawing of the structure of and SNP position in the aldehyde dehydrogenase 1 (ALDH1) gene.
Figure 109 shows a drawing of the structure of and SNP position in the aldehyde dehydrogenase 2 (ALDH2) gene.
Figure 110 shows a drawing of the structure of and SNP position in the aldehyde dehydrogenase 7 (ALDH7) gene.
Figure 111 shows a drawing of the structure of and SNP position in the aldehyde dehydrogenase 8 (ALDH8) gene.
Figure 112 shows a drawing of the structure of and SNP position in the aldehyde dehydrogenase 9 (ALDH9) gene.
Figure 113 shows a drawing of the structure of and SNP position in the aldehyde dehydrogenase 10 (ALDH10) gene.
Figure 114 shows a drawing of the structure of and SNP position in the ATP-binding cassette subfamily C member 7 (ABCC7) gene.
Figure 115 shows a drawing of the structure of and SNP position in the ATP-binding cassette subfamily C member 8 (ABCC8) gene.
Figure 116 shows a drawing of the structure of and SNP position in the ATP-binding cassette subfamily C member 9 (ABCC9) gene.
Figure 117 shows a drawing of the structure of and SNP position in the carboxylesterase 1 (CES1) gene.
Figure 118 shows a drawing of the structure of and SNP position in the ATP-binding cassette subfamily A member 4 (ABCC4) gene.
Figure 119 shows a drawing of the structure of and SNP position in the ATP-binding cassette subfamily A member 7 (ABCC7) gene.
Figure 120 shows a drawing of the structure of and SNP position in the ATP-binding cassette subfamily G member 1 (ABCG1) gene.
Figure 121 shows a drawing of the structure of and SNP position in the ATP-binding cassette subfamily G member 2 (ABCG2) gene.
Figure 122 shows a drawing of the structure of and SNP position in the ATP-binding cassette subfamily G member 4 (ABCG4) gene.
Figure 123 shows a drawing of the structure of and SNP position in the ATP-binding cassette subfamily E member 1 (ABCE1) gene.
Figure 124 shows a drawing of the structure of and SNP position in the carbohydrate sulfotransferase 1 (CHST1) gene.
Figure 125 shows a drawing of the structure of and SNP position in the carbohydrate sulfotransferase 3 (CHST3) gene.
Figure 126 shows a drawing of the structure of and SNP position in the NADH: ubiquinone dehydrogenase flavoprotein 1 (NDUFV1) gene.
Figure 127 shows a drawing of the structure of and SNP position in the NADH: ubiquinone dehydrogenase flavoprotein 2 (NDUFV2) gene.
Figure 128 shows a drawing of the structure of and SNP position in the NADH: ubiquinone dehydrogenase flavoprotein 3 (NDUFV3) gene.
Figure 129 shows a drawing of the structure of and SNP position in the NADH: ubiquinone oxide reductase A10 (NDUFA10) gene.
Figure 130 shows a drawing of the structure of and SNP position in the high-mobility group protein 17-like 1 (HMG17L1) gene.
Figure 131 shows a drawing of the structure of and SNP position in the UDP glycoxyl transferase 2 family polypeptide A1 (UGT2A1) gene.
Figure 132 shows a drawing of the structure of and SNP position in the human organic anion transporter polypeptide 1 (hOATP1) gene.
Figure 133 shows a drawing of the structure of and SNP position in the human organic anion transporter polypeptide 2 (hOATP2) gene.
Figure 134 shows a drawing of the structure of and SNP position in the human organic anion transporter polypeptide 8 (hOATP8) gene.
Figure 135 shows a drawing of the structure of and SNP position in the human organic anion transporter 1 (hOAT1) gene.
Figure 136 shows a drawing of the structure of and SNP position in the human organic anion transporter 2 (hOAT2) gene.
Figure 137 shows a drawing of the structure of and SNP position in the human organic anion transporter 3 (hOAT3) gene.
Figure 138 shows a drawing of the structure of and SNP position in the aldehyde dehydrogenase 1 family member A2 (ALDH1A2) gene.
Figure 139 shows a drawing of the structure of and SNP position in the aldehyde dehydrogenase 1 family member A3 (ALDH1A3) gene.
Figure 140 shows a drawing of the structure of and SNP position in the formyltetrahydroforate dehydrogenase (FTHFD/ALDH1L1) gene.
Figure 141 shows a drawing of the structure of and SNP position in the cytochrome P450 subfamily IIIA (aromatic compound-induced) polypeptide 4 (CYP3A4) gene.
Figure 142 shows graph of the results of typing performed on two different groups of subjects using the INVADER assay method.
Figure 143 shows a summary of genetic information.
Figure 144A shows a structure of ATP-binding cassette subfamily A member 1 (ABCA1) gene and the SNP location therein.
   Accession No.: AF275948.1 and AL359846.11
Figure 144B shows a structure of ATP-binding cassette subfamily A member 1 (ABCA1) gene and the SNP location therein. (continuation of Figure 9A)
   Accession No.: AF275948.1 and AL359846.11
Figure 145 shows a structure of ATP-binding cassette subfamily A member 4 (ABCA4) gene and the SNP location therein.
   Accession No.: NT_019258.1
Figure 146 shows a structure of ATP-binding cassette subfamily A member 7 (ABCA7) gene and the SNP location therein.
   Accession No.: NT_025194.1
Figure 147 shows a structure of ATP-binding cassette subfamily A member 8 (ABCA8) gene and the SNP location therein.
   Accession No.: AC005922.1 and AC015844.5
Figure 148 shows a structure of ATP-binding cassette subfamily B member 1 (ABCB1) gene and the SNP location therein.
   Accession No.: AC002457.1 and AC005068.1
Figure 149 shows a structure of ATP-binding cassette subfamily B member 4 (ABCB4) gene and the SNP location therein.
   Accession No.: AC079S91.1, AC079303.3 and AC005045.2
Figure 150 shows a structure of ATP-binding cassette subfamily B member 7 (ABCB7) gene and the SNP location therein.
   Accession No.: AL360179.3 and AC002417.1

Figure 151 shows a structure of ATP-binding cassette subfamily B member 8 (ABCBB) gene and the SNP location therein.
   Accession No.: AC010973.4
Figure 152 shows a structure of ATP-binding cassette subfamily B member 9 (ABCB9) gene and the SNP location therein.
   Accession No.: AC026362.9 and AC073857.10
Figure 153 shows a structure of ATP-binding cassette subfamily B member 10 (ABCB 10) gene and the SNP location therein.
   Accession No.: AL121990.9
Figure 154 shows a structure of ATP-binding cassette subfamily B member 11 (ABCB11) gene and the SNP location therein.
   Accession No.: AC008177.3 and AC069137.3
Figure 155 shows a structure of ATP-binding cassette subfamily C member 1 (ABCC1) gene and the SNP location therein.
   Accession No.: AC026452.5 and AC025778.4
Figure 156 shows a structure of ATP-binding cassette subfamily C member 2 (ABCC2) gene and the SNP location therein.
   Accession No.: AL392107.4
Figure 157 shows a structure of ATP-binding cassette subfamily C member 3 (ABCC3) gene and the SNP location therein.
   Accession No.: AC004590.1 and AC005921.3
Figure 158A shows a structure of ATP-binding cassette subfamily C member 4 (ABCC4) gene and the SNP location therein.
   Accession No.: AL356257.11, AL157818.12 and AL139381.12
Figure 158B shows a structure of ATP-binding cassette subfamily C member 4 (ABCC4) gene and the SNP location therein. (continuation of Figure 158A)
   Accession No.: AL356257.11, AL157818.12, and AL139381.12
Figure 159 shows a structure of ATP-binding cassette subfamily C member 5 (ABCC5) gene and the SNP location therein.
   Accession No.: AC068644.5
Figure 160 shows a structure of ATP-binding cassette subfamily C member 7 (ABCC7) gene and the SNP location therein.
   Accession No.: AC000111.1 and AC000061.1
Figure 161 shows a structure of ATP-binding cassette subfamily C member 8 (ABCC8) gene and the SNP location therein.
   Accession No.: AC000406.1
Figure 162 shows a structure of ATP-binding cassette subfamily C member 9 (ABCC9) gene and the SNP location therein.
   Accession No.: AC084806.9 andAC008250.23
Figure 163 shows a structure of ATP-binding cassette subfamily D member 1 (ABCD1) gene and the SNP location therein.
   Accession No.: U52111.2
Figure 164 shows a structure of ATP-binding cassette subfamily D member 3 (ABCD3) gene and the SNP location therein.
   Accession No.: NT_019284.3
Figure 165 shows a structure of ATP-binding cassette subfamily D member 4 (ABCD4) gene and the SNP location therein.
   Accession No.: AC005519.3
Figure 166 shows a structure of ATP-binding cassette subfamily G member 1 (ABCG1) gene and the SNP location therein.
   Accession No.: AP001746.1
Figure 167 shows a structure of ATP-binding cassette subfamily G member 2 (ABCG2) gene and the SNP location therein.
   Accession No.: NT_022959.2
Figure 168 shows a structure of ATP-binding cassette subfamily G member 4 (ABCG4) gene and the SNP location therein.
   Accession No.: AP001315.3
Figure 169 shows a structure of ATP-binding cassette subfamily G member 5 (ABCG5) gene and the SNP location therein.
   Accession No.: AC084265.2 and AC011242.8
Figure 170 shows a structure of ATP-binding cassette subfamily G member 8 (ABCG8) gene and the SNP location therein.
   Accession No.: AC084265.2
Figure 171 shows a structure of ATP-binding cassette subfamily E member 1 (ABCE1) gene and the SNP location therein.
   Accession No.: NT_006296.2
Figure 172 shows a structure of ATP-binding cassette subfamily F member 1 (ABCF1) gene and the SNP location therein.
   Accession No.: NT_007592.3
Figure 173 shows a structure of organic anion transporter 1 (OAT1) gene and the SNP location therein.
   Accession No.: AP001858.3, AJ249369.1, and AP000438.4
Figure 174 shows a structure of organic anion transporter 2 (OAT2) gene and the SNP location therein.
   Accession No.: AC26532.3
Figure 175 shows a structure of organic anion transporter 3 (OAT3) gene and the SNP location therein.
   Accession No.: AP001858.3
Figure 176 shows a structure of organic anion transporter polypeptide 1 (OATP1) gene and the SNP location therein.
   Accession No.: AC022224.22
Figure 177 shows a structure of organic anion transporter polypeptide 2 (OATP2) gene and the SNP location therein.
   Accession No.: NT_024399.2
Figure 178 shows a structure of organic anion transporter polypeptide 8 (OATP8) gene and the SNP location therein.
   Accession No.: NT_024399.2
Figure 179 shows a structure of transporter 1 ATP-binding cassette subfamily B (TAP1) gene and the SNP location therein.
   Accession No.: X66401.1
Figure 180 shows a structure of transporter 2 ATP-binding cassette subfamily B (TAP2) gene and the SNP location therein.
   Accession No.: X66401.1
Figure 181 shows a structure of SLC22A4 solute carrier family 22 (organic cation transporter) member 4 (OCTN1) gene and the SNP location therein.
   Accession No.: AC008599.6
Figure 182 shows a structure of SLC22A5 solute carrier family 22 (organic cation transporter) member 5 (OCTN2) gene and the SNP location therein.
   Accession No.: AC023861.3
Figure 183 shows a structure of SLC22A1 solute carrier family 22 (organic cation transporter) member 1 (OCT1) gene and the SNP location therein.
   Accession No.: AL35625.5
Figure 184 shows a structure of SLC22A2 solute carrier family 22 (organic cation transporter) member 2 (OCT2) gene and the SNP location therein.
   Accession No.: AL162582.18
Figure 185 shows a structure of SLC10A2 solute carrier family 10 (sodium/bile acid cotransporter family) member 2 (NTCP) gene and the SNP location therein.
   Accession No.: AL157789.6
Figure 186 shows a structure of SLC15A1 solute carrier family 15 (oligopeptide transporter) member 1 (PEPT1) gene and the SNP location therein.
   Accession No.: AL353574.8 and AL391670.6
Figure 187 shows a structure of microsomal epoxide hydrolase 1 (EPHX1) gene and the SNP location therein.
   Accession No.: AC058782.8
Figure 188 shows a structure of cytoplasmic epoxide hydrolase (EPHX2) gene and the SNP location therein.
   Accession No.: AC010856.3
Figure 189 shows a structure of catechol-O-methyl transferase (COMT) gene and the SNP location therein.
   Accession No.: AC000080.2
Figure 190 shows a structure of guanidinoacetate N-methyl transferase (GAMT) gene and the SNP location therein.
   Accession No.: NT_000879.1
Figure 191 shows a structure of phenyl ethanolamine N-methyl transferase (PNMT) gene and the SNP location therein.
   Accession No.: AC040933.3
Figure 192 shows a structure of histamine N-methyl transferase (HNMT) gene and the SNP location therein.
   Accession No.: AC019304.3
Figure 193 shows a structure of nicotinamide N-methyl transferase (NNMT) gene and the SNP location therein.
   Accession No.: AC019290.3
Figure 194 shows a structure of phosphatidylethanolamine N-methyl transferase (PEMT) gene and the SNP location therein.
   Accession No.: AC020558.3
Figure 195 shows a structure of aldehyde dehydrogenase 1 family member A1 (ALDH1A1) gene and the SNP location therein.
   Accession No.: AC009284.2 and AL162416.3
Figure 196 shows a structure of aldehyde dehydrogenase 1 family member A2 (ALDH1A2) gene and the SNP location therein.
   Accession No.: AC025431.7 and AC012653.8
Figure 197 shows a structure of aldehyde dehydrogenase 1 family member A3 (ALDH1A3) gene and the SNP location therein.
   Accession No.: AC015712.7
Figure 198 shows a structure of aldehyde dehydrogenase 1 family member B 1 (ALDH1B1) gene and the SNP location therein.
   Accession No.: AL135785.9
Figure 199A shows a structure of formyl tetrahydrofolate dehydrogenase (ALDH1L1) gene and the SNP location therein.
   Accession No.: AC079848.6
Figure 199B shows a structure of formyl tetrahydrofolate dehydrogenase (ALDH1L1) gene and the SNP location therein. (continuation of Figure 199A)
   Accession No.: AC079848.6
Figure 200 shows a structure of aldehyde dehydrogenase 2 (ALDH2) gene and the SNP location therein.
   Accession No.: AC002996.1 and AC003029.2

Figure 201 shows a structure of aldehyde dehydrogenase 3 family member A1 (ALDH3A1) gene and the SNP location therein.
   Accession No.: AC005722.1
Figure 202 shows a structure of aldehyde dehydrogenase 3 family member A2 (ALDH3A2) gene and the SNP location therein.
   Accession No.: AC005722.1
Figure 203 shows a structure of aldehyde dehydrogenase 3 family member B1 (ALDH3B1) gene and the SNP location therein.
   Accession No.: AC004923.2
Figure 204 shows a structure of aldehyde dehydrogenase 3 family member B2 (ALDH3B2) gene and the SNP location therein.
   Accession No.: AC021987.3
Figure 205 shows a structure of aldehyde dehydrogenase 5 family member A1 (ALDH5A1) gene and the SNP location therein.
   Accession No.: AL031230.1
Figure 206 shows a structure of aldehyde dehydrogenase 6 family member A1 (ALDH6A1) gene and the SNP location therein.
   Accession No.: AC005484.2
Figure 207 shows a structure of aldehyde dehydrogenase 8 family member A1 (ALDH8A1) gene and the SNP location therein.
   Accession No.: AL445190.9 and AL021939.1
Figure 208 shows a structure of aldehyde dehydrogenase 9 family member A1 (ALDH9A1) gene and the SNP location therein.
   Accession No.: AL451074.4
Figure 209 shows a structure of alcohol dehydrogenase 1 (ADH1) gene and the SNP location therein.
   Accession No.: AP002027.1
Figure 210 shows a structure of alcohol dehydrogenase 2 (ADH2) gene and the SNP location therein.
   Accession No.: AP002027.1
Figure 211 shows a structure of alcohol dehydrogenase 3 (ADH3) gene and the SNP location therein.
   Accession No.: AP002027.1
Figure 212 shows a structure of alcohol dehydrogenase 4 (ADH4) gene and the SNP location therein.
   Accession No.: AP002026.1
Figure 213 shows a structure of alcohol dehydrogenase 5 (ADH5) gene and the SNP location therein.
   Accession No.: AC019131.4
Figure 214 shows a structure of alcohol dehydrogenase 6 (ADH6) gene and the SNP location therein.
   Accession No.: AP002026.1
Figure 215 shows a structure of alcohol dehydrogenase 7 (ADH7) gene and the SNP location therein.
   Accession No.: AC027065.3
Figure 216 shows a structure of short-chain alcohol dehydrogenase family gene (HEP27) and the SNP location therein.
   Accession No.: AL135999.3
Figure 217 shows a structure of UDP glycosyltransferase 1 family polypeptide A 1 (UGT1A1) and the SNP location therein.
   Accession No.: AC006985.2
Figure 218 shows a structure of UDP glycosyltransferase 2 family polypeptide A1 (UGT2A1) and the SNP location therein.
   Accession No.: AC011254.3
Figure 219 shows a structure of UDP glycosyltransferase 2 family polypeptide B 15 (UGT2B 15) and the SNP location therein.
   Accession No.: AC019173.4
Figure 220 shows a structure of UDP glycosyltransferase 8 (UGT8) and the SNP location therein.
   Accession No.: U31353.1
Figure 221 shows a structure of glutathione S transferase A1 (GSTA1) gene and the SNP location therein.
   Accession No.: AC021133.4
Figure 222 shows a structure of glutathione S transferase A4 (GSTA4) gene and the SNP location therein.
   Accession No.: AC025085.4
Figure 223 shows a structure of glutathione S transferase M1 (GSTM1) gene and the SNP location therein.
   Accession No.: AC000032.7
Figure 224 shows a structure of glutathione S transferase M2 (GSTM2) gene and the SNP location therein.
   Accession No.: AC000031.5
Figure 225 shows a structure of glutathione S transferase Z1 (GSTZ1) gene and the SNP location therein.
   Accession No.: AC007954.7
Figure 226 shows a structure of glutathione S transferase Pi (GSTPi) gene and the SNP location therein.
   Accession No.: X08058.1 and M24485.1
Figure 227 shows a structure of glutathione S transferase T1 (GSTT1) gene and the SNP location therein.
   Accession No.: AF240786.1 and AP000351.3
Figure 228 shows a structure of microsomal glutathione S transferase 1 (MGST1) gene and the SNP location therein.
   Accession No.: AC007528.5
Figure 229 shows a structure of microsomal glutathione S transferase 1-like 1 (MGST1L1) gene and the SNP location therein.
   Accession No.: AC007936.2
Figure 230 shows a structure of microsomal glutathione S transferase T2 (MGST2) gene and the SNP location therein.
   Accession No.: AC019049.4
Figure 231 shows a structure of microsomal glutathione S transferase T3 (MGST3) gene and the SNP location therein.
   Accession No.: AC064827.2
Figure 232 shows a structure of sulfotransferase 1A1 (SULT1A1/STP1) gene and the SNP location therein.
   Accession No.: U52852.2
Figure 233 shows a structure of sulfotransferase 1A2 (SULT1A2/STP2) gene and the SNP location therein.
   Accession No.: U33886.1, U34804.1 and AC020765.5
Figure 234 shows a structure of sulfotransferase 1A3 (SULT1A3/STM/HAST) gene and the SNP location therein
   Accession No.: L34160.1 and AC012645.4
Figure 235 shows a structure of sulfotransferase 1C1 (SULT1C1) gene and the SNP location therein.
   Accession No.: AC019100.4
Figure 236 shows a structure of sulfotransferase 1C2 (SULT1C2) gene and the SNP location therein.
   Accession No.: AF186263.1
Figure 237 shows a structure of sulfotransferase 2A1 (SULT2A1) gene and the SNP location therein.
   Accession No.: AC024582.4, AC008745.5, NT_011190.1, and AC024582.4
Figure 238 shows a structure of sulfotransferase 2B1 (SULT2B1) gene and the SNP location therein.
   Accession No.: AC040922.2 and AC008403.6
Figure 239 shows a structure of sulfotransferase-associated protein 3 (SULTX3) gene and the SNP location therein.
   Accession No.: Z97055.1
Figure 240 shows a structure of tyrosyl protein sulfotransferase 1 (TPST1) gene and the SNP location therein.
   Accession No.: AC026281.5
Figure 241 shows a structure of tyrosyl protein sulfotransferase 2 (TPST2) gene and the SNP location therein.
   Accession No.: Z95115.1
Figure 242 shows a structure of cerebroside sulfotransferase (CST) gene and the SNP location therein.
   Accession No.: AC005006.2
Figure 243 shows a structure of thyroid hormone sulfotransferase (ST1B2) gene and the SNP location therein.
   Accession No.: AC027059.2
Figure 244 shows a structure of carbohydorate sulfotransferase 1 (CHST1) gene and the SNP location therein.
   Accession No.: NT_008982.1
Figure 245 shows a structure of carbohydorate sulfotransferase 2 (CHST2) gene and the SNP location therein.
   Accession No.: AC055737.10
Figure 246 shows a structure of carbohydorate sulfotransferase 3 (CHST3) gene and the SNP location therein.
   Accession No.: AC073370.3
Figure 247 shows a structure of carbohydorate sulfotransferase 4 (CHST4) gene and the SNP location therein.
   Accession No.: AC010547.5
Figure 248 shows a structure of carbohydorate sulfotransferase 5 (CHST5) gene and the SNP location therein.
   Accession No.: AC025287.3
Figure 249 shows a structure of HNK-sulfotransferase (HINK-1ST) gene and the SNP location therein.
   Accession No.: AC012493.4
Figure 250 shows a structure of estrogen sulfotransferase (STE) gene and the SNP location therein.
   Accession No.: AC074273.1

Figure 251 shows a structure of NAD (P)H: quinone oxidoreductase 1 (NQO1) gene and the SNP location therein.
   Accession No.: M81596.1
Figure 252 shows a structure of NRH: quinone oxidoreductase 2 (NQO2) gene and the SNP location therein.
   Accession No.: AB050248.1
Figure 253 shows a structure of p53-inducible gene 3 (PIG3) in a quinone oxidoreductase homolog and the SNP location therein.
   Accession No.: AC008073.3
Figure 254 shows a structure of NADH-dehydrogenase(ubiquinone) 1α-subcomplex 1 (NDUFA1) gene and the SNP location therein.
   Accession No.: AC002477.1
Figure 255 shows a structure of NADH-dehydrogenase(ubiquinone)1α-subcomplex 2 (NDUFA2) gene and the SNP location therein.
   Accession No.: AB054976.1
Figure 256 shows a structure of NADH-dehydrogenase(ubiquinone)1α-subcomplex 3 (NDUFA3) gene and the SNP location therein.
   Accession No.: AC009968.6
Figure 257 shows a structure of NADH-dehydrogenase(ubiquinone) 1α-subcomplex 5 (NDUFA5) gene and the SNP location therein.
   Accession No.: AC073323.5
Figure 258 shows a structure of NADH-dehydrogenase(ubiquinone)1α-subcomplex 6 (NDUFA6) gene and the SNP location therein.
   Accession No.: AL021878.1
Figure 259 shows a structure of NADH-dehydrogenase(ubiquinone)1α-subcomplex 7 (NDUFA7) gene and the SNP location therein.
   Accession No.: AC010323.6
Figure 260 shows a structure of NADH-dehydrogenase(ubiquinone) 1α-subcomplex 8 (NDUFA8) gene and the SNP location therein.
   Accession No.: AL162423.10
Figure 261 shows a structure of NADH-dehydrogenase(ubiquinone)1α-subcomplex 9 (NDUFA9) gene and the SNP location therein.
   Accession No.: AC005832.1
Figure 262 shows a structure of NADH-dehydrogenase(ubiquinone)1α-subcomplex 10 (NDUFA10) gene and the SNP location therein.
   Accession No.: AC013469.8
Figure 263 shows a structure of NADH-dehydrogenase(ubiquinone)1α/β-subcomplex 1 (NDUFAB1) gene and the SNP location therein.
   Accession No.: AC008870.6
Figure 264 shows a structure of NADH-dehydrogenase(ubiquinone)1β-subcomplex 3 (NDUFB3) gene and the SNP location therein.
   Accession No.: AC007272.3
Figure 265 shows a structure of NADH-dehydrogenase(ubiquinone) 1β-subcomplex 5 (NDUFB5) gene and the SNP location therein.
   Accession No.: AC068361.2
Figure 266 shows a structure of NADH-dehydrogenase(ubiquinone)1β-subcomplex 7 (NDUFB7) gene and the SNP location therein.
   Accession No.: AC010527.4
Figure 267 shows a structure of NADH-dehydrogenase(ubiquinone)Fe-S protein 1 (NDUFS1) gene and the SNP location therein.
   Accession No.: AC007383.4
Figure 268 shows a structure of NADH-dehydrogenase(ubiquinone)Fe-S protein 3 (NDUFS3) gene and the SNP location therein.
   Accession No.: AC067943.4
Figure 269 shows a structure of NADH-dehydrogenase(ubiquinone)Fe-S protein 4 (NDUFS4) gene and the SNP location therein.
   Accession No.: AC024569.3
Figure 270 shows a structure of NADH-dehydrogenase(ubiquinone)Fe-S protein 5 (NDUFS5) gene and the SNP location therein.
   Accession No.: AL139015.5
Figure 271 shows a structure of NADH-dehydrogenase(ubiquinone)Fe-S protein 6 (NDUFS6) gene and the SNP location therein.
   Accession No.: AC026443.2
Figure 272 shows a structure of NADH-dehydrogenase(ubiquinone)Fe-S protein 8 (NDUFS8) gene and the SNP location therein.
   Accession No.: AC034259.2
Figure 273 shows a structure of NADH-dehydrogenase(ubiquinone)flavoprotein 1 (NDUFV1) gene and the SNP location therein.
   Accession No.: NT_009304.2
Figure 274 shows a structure of NADH-dehydrogenase(ubiquinone)flavoprotein 2 (NDUFV2) gene and the SNP location therein.
   Accession No.: NT_011024.2
Figure 275 shows a structure of NADH-dehydrogenase(ubiquinone)flavoprotein 3 (NDUFV3) gene and the SNP location therein.
   Accession No.: AP001748.1
Figure 276 shows a structure of gamma-glutamyl transferase 1 (GGT1) gene and the SNP location therein.
   Accession No.: D87002.1
Figure 277 shows a structure of transglutaminase 1 (TGM1) gene and the SNP location therein.
   Accession No.: M98447.1
Figure 278 shows a structure of cytochrome P450 subfamily 1 (aromatic compound-inducible) polypeptide 1 (CYP1A1) gene and the SNP location therein.
   Accession No.: X04300.1 and AC020705.4
Figure 279 shows a structure of cytochrome P450 subfamily 1 (aromatic compound-inducible) polypeptide 2 (CYP1A2) gene and the SNP location therein.
   Accession No.: AC020705.4
Figure 280 shows a structure of cytochrome P450 subfamily 1 (dioxin-inducible) polypeptide 1 (CYP1B1) gene and the SNP location therein.
   Accession No.: AC009229.4
Figure 281 shows a structure of cytochrome P450 subfamily 3A (aromatic compound-inducible) polypeptide 4 (CYP3A4) gene and the SNP location therein.
   Accession No.: AF280107.1
Figure 282 shows a structure of cytochrome P450 subfamily 3A (aromatic compound-inducible) polypeptide 5 (CYP3A5) gene and the SNP location therein.
   Accession No.: AC005020.5
Figure 283 shows a structure of cytochrome P450 subfamily 3A polypeptide 7 (CYP3A7) gene and the SNP location therein.
   Accession No.: AF280107.1
Figure 284 shows a structure of cytochrome P450 polypeptide 43 (CYP3A43) gene and the SNP location therein.
   Accession No.: AC011904.3
Figure 285 shows a structure of cytochrome P450 subfamily IVB polypeptide 1 (CYP4B1) gene and the SNP location therein.
   Accession No.: AL356793.10
Figure 286 shows a structure of cytochrome P450 subfamily IVF polypeptide 2 (CYP4F2) gene and the SNP location therein.
   Accession No.: AC005336.1
Figure 287 shows a structure of cytochrome P450 subfamily IVF polypeptide 3 (CYP4F3) gene and the SNP location therein.
   Accession No.: AD000685.1
Figure 288 shows a structure of cytochrome P450 subfamily IVF polypeptide 8 (CYP4F8) gene and the SNP location therein.
   Accession No.: AC068845.3
Figure 289 shows a structure of cytochrome P450 subfamily XXVIIA polypeptide 1 (CYP27A1) gene and the SNP location therein.
   Accession No.: AC009974.7
Figure 290 shows a structure of cytochrome P450 subfamily XXVIIB polypeptide 1 (CYP27B1) gene and the SNP location therein.
   Accession No.: AC025165.27
Figure 291 shows a structure of allylacetamide deacetylase (AADAC) gene and the SNP location therein.
   Accession No.: AC068647.4
Figure 292 shows a structure of carboxyl esterase 1 (CES1) gene and the SNP location therein
   Accession No.: AC007602.4
Figure 293 shows a structure of carboxyl esterase 2 (CES2) gene and the SNP location therein
   Accession No.: AC027131.4
Figure 294 shows a structure of granzyme A (GZMA) gene and the SNP location therein.
   Accession No.: AC091977.1
Figure 295 shows a structure of granzyme B (GZMB) gene and the SNP location therein.
   Accession No.: AL136018.3
Figure 296 shows a structure of esterase D/formylglutathione hydrolase (ESD) gene and the SNP location therein.
   Accession No.: AL136958.9
Figure 297A shows a structure of carboxyl ester lipase (bile salt-stimulated lipase) (CEL) gene and the SNP location therein.
   Accession No.: AL138750.8, AL162417.20 and AF072711.1
Figure 297B shows a structure of carboxyl ester lipase (bile salt-stimulated lipase) (CEL) gene and the SNP location therein. (continuation of Figure 297A)
   Accession No.: AL138750., AL162417.20 and AF072711.1
Figure 298 shows a structure of interleukin 17 (cytotoxic T lymphocyte-associated serine esterase 8) (IL17) gene and the SNP location therein.
   Accession No.: AL355513.11
Figure 299 shows a structure of ubiquitin carboxyl terminal esterase L3 (ubiquitin thiol esterase) (UCHL3) gene and the SNP location therein.
   Accession No.: AL137244.28
Figure 300 shows a structure of dolichyl-diphosphooligosaccharide-protein glycosyltransferase (DDOST) gene and the SNP location therein.
   Accession No.: D89060

Figure 301 shows a structure of neuropathy target esterase (NTE) gene and the SNP location therein.
   Accession No.: AC021153
Figure 302 shows a structure of L1 cell adhesion molecule (L1CAM) gene and the SNP location therein.
   Accession No.: U52112
Figure 303 shows a structure of arylalkylamine N-acetyltransferase (AANAT) gene and the SNP location therein.
   Accession No.: U40391
Figure 304 shows a structure of N-acetyltransferase homolog (ARD1) gene of *Saccharomyces cerevisiae* and the SNP location therein.
   Accession No.: U52112
Figure 305 shows a structure of N-acetyltransferase (NAT1) gene and the SNP location therein.
   Accession No.: X17059
Figure 306 shows a structure of N-acetyltransferase 2 (NAT2) gene and the SNP location therein.
   Accession No.: D10870
Figure 307 shows a structure of ATP-binding cassette subfamily B member 2 (ABCB2) gene and the SNP location therein.
   Accession No.: X66401
Figure 308 shows a structure of ATP-binding cassette subfamily B member 3 (ABCB3) gene and the SNP location therein.
   Accession No.: X66401
Figure 309 shows a structure of glutathione S transferase M3 (GSTM3) gene and the SNP location therein.
   Accession No.: AF043105.1
Figure 310 shows a structure of glutathione S transferase M4 (GSTM4) gene and the SNP location therein.
   Accession No.: M96233.1
Figure 311 shows a structure of aldehyde dehydrogenase 7 (ALDH7) gene and the SNP location therein.
   Accession No.: AC004923
Figure 312 shows a structure of high-mobility group protein 17-like 1 (HMG17L1) gene and the SNP location therein.
   Accession No.: Z97055.1

### GENERAL DESCRIPTION OF THE INVENTION

The present invention provides a method of analysis of drug metabolizing enzymes by analysis of SNPs associated with their encoding genes. In some embodiments, the method of the present invention can be used in the selection of drugs based on, e.g., particular characteristics of an individual patient or on characteristics of a target disease.

In some embodiments, the present invention provides a method for detecting a genetic polymorphism associated with a DME, wherein an oligonucleotide probe and/or oligonucleotide primer is created so as to include the genetic polymorphism site from genetic polymorphism data in a gene for encoding a drug metabolizing enzyme or so as to include the genetic polymorphism site in an amplified fragment when the gene encoding the drug metabolizing enzyme has been amplified, and wherein at least one genetic polymorphism in a gene for encoding the target drug metabolizing enzyme is detected using the oligonucleotide probe and/or oligonucleotide primer thus obtained.

The present invention further provides methods for evaluating a drug, wherein the effectiveness and safety of a drug metabolized by the drug metabolizing enzyme are evaluated based on the results obtained by the detection method.

In some embodiments, the present invention provides a method for screening a drug, wherein the drug to be used is selected based on the results obtained in the evaluation method. In other embodiments, the present invention provides a method for screening a drug, wherein the genetic polymorphism data associated with the gene encoding a DME in a control subject is compared to the genetic polymorphism data associated with the same gene in a test subject, and wherein a drug to be used is selected from the results of an analysis of the effectiveness and/or safety of the drugs metabolized by the drug metabolizing enzyme.

The invention further features predictive medicines, which are based, at least in part, on determination of the identity of DME polymorphic regions that are associated with particular drug responses. For example, information obtained using the diagnostic assays described herein (alone or in conjunction with information on another genetic defect, which contributes to the same disease) is useful for determining if a test subject has an allele of a polymorphic region that is associated with a particular drug response. Knowledge of the DME profile in an individual (the DME genetic profile), alone or in conjunction with information on other genetic defects contributing to the same disease (the genetic profile of the particular disease) allows customization of therapy for a particular disease to the individual's genetic profile, the goal of "pharmacogenomics." For example, an individual's DME genetic profile can enable a doctor: 1) to more effectively prescribe a drug that will address the molecular basis of the disease or condition; and 2) to better determine the appropriate dosage of a particular drug.

The ability to target populations expected to show the highest clinical benefit, based on the DME genetic profile, allows: 1) the repositioning of marketed drugs with disappointing market results; 2) the rescue of drug candidates whose clinical development has been discontinued as a result of safety or efficacy limitations, which are patient subgroup-specific; and 3) an accelerated and less costly development for drug candidates and more optimal drug labeling (e.g. since the use of DMEs as markers is useful for optimizing effective dose).

### DETAILED DESCRIPTION OF THE INVENTION

Examples of genetic polymorphism data related to a DME, and useful in the detection, evaluation method and screening methods of the present invention are shown in Table 1. In some embodiments, a drug-metabolizing enzyme is at least one of the following: epoxide hydrolase, methyltransferase, N-acetyltransferase, sulfotransferase, quinone oxidereductase, glutathione S-transferase, UDP-glycosyltransferase, aldehyde dehydrogenase, alcohol dehydrogenase, esterase, NDUF, cytochrome P450 (CYP) and ATP-binding cassette.

The present invention relates to a method for detecting a genetic polymorphism in a test subject using the genetic polymorphism data related to a drug metabolizing enzyme. The present invention analyzes the effectiveness, safety and strength of drugs metabolized by a drug metabolizing enzyme. The relationship between a disease and the drug to be evaluated is based on the results of the analysis. The genetic polymorphism data for the drug metabolizing enzyme is different for each patient with a given disease. Therefore, the effectiveness and safety of a specific drug depends on drug metabolism in the presence of certain genetic polymorphism data and the side effects in the presence of certain genetic polymorphism data. As a result, a physician can determine whether a certain drug should be used by a certain patient and can tailor drugs for use by a certain patient based on the genetic polymorphism data (so-called "made-to-order" treatments).

"Drug metabolizing enzymes" refer to a group of enzymes that catalyze *in vivo* structural changes in exogenous materials including drugs. When used for clinical purposes, the group of metabolizing enzymes includes some endogenous materials. Because drug-metabolizing enzymes absorb, metabolize and secrete drugs, the polymorphism of an enzyme depends on the amount of enzyme expressed (transcription and translation) and the amount of activity. As a result, there are blood serum concentrations of both unchanged materials and metabolites.

Drug metabolizing enzymes expressed by the genes that are targeted for genetic polymorphism analysis in the present invention include, but are not limited to the following classes of enzymes:
Epoxide hydrolases
Methyltransferases
N-acetyltransferases
Sulfotransferases
Quinone oxidereductases
Glutathione S-transferases
UDP-glycosyltransferases
Aldehyde dehydrogenases
Alcohol dehydrogenases
Esterases
Ubiquinone dehydrogenases : NDUF
Cytochrome P450s (CYPs)
ATP-binding cassettes
ATP-binding cassettes / Transporters
Examples and descriptions of these enzymes are provided below.
(1) Epoxide hydrolases are enzymes that hydrolyze epoxide using a transcleavage mechanism to produce 1,2-glycol. Examples include microsomal epoxide hydrolase 1 and cytoplasmic epoxide hydrolase 2.
(2) Methyltransferases are enzymes that catalyze transmethylation in amino groups, hydroxyl groups and thiol groups. Examples include the following.
   Catechol-O-methyltransferase
   Vitamin-N-methyltransferase
   Phenylethanolamine-N-methyltransferase
   Phosphatidylethanolamine-N-methyltransferase
   Nicotinamide-N-methyltransferase
   Acetylserotonin-O-methyltransferase
   Thiopurine S-methyltransferase
(3) N-acetyltransferases are enzymes that catalyze transacetylation in amino groups, sulfonamide groups and hydrazine groups. Examples include the following.
   Arylamine-N-acetyltransferase 1, 2
   Arylalkylamine-N-acetyltransferase
   N-acetyltransferase homologues of saccharomyces cerevisiae
   LI intracellular adhesion molecules
(4) Sulfotransferases are enzymes that contribute to sulfate conjugation and catalyzes trans-sulfonylation in phenols, steroids, arylamines and biliary acid. Examples include the following.
   Sulfotransferase 1A1, 1A2, 1A3, 1C1, 1C2, 2A1, 2B1
   Thyroid hormone sulfotransferase
   Tyrosyl protein sulfotransferase 1, 2
   Sulfotransferase-opening protein 3
   Estrogen sulfotransferase
   Cerebroside sulfotransferase
   HNK-sulfotransferase 1
   Carbohydrate sulfotransferase 2, 4, 5
   Carbohydrate sulfotransferase 1, 3
(5) Quinone oxidereductases are enzymes that catalyze the reduction of quinones such as o-quinone and p-quinone. Examples include the following.
   NAD(P)H: Quinone oxidereductase 1
   NRH: Quinone oxidereductase 2
   Quinone oxidereductase homologues
   p53-induced gene 3 (PIG3) of a quinone oxide transferase homologue
(6) Glutathione S-transferases are enzymes that catalyze the conjugation of glutathione. Examples include the following.
   Glutathione S-transferase Mu1, Mu2, Mu3, Mu4, Mu5
   Glutathione S-transferase Z (zeta)
   Glutathione S-transferase P (pi)
   Glutathione S-transferase 1 T1 (zeta)
   Glutathione S-transferase 1 Theta 1, Theta 2
   Microsomal Glutathione S-transferase 1
   Microsomal Glutathione S-transferase 1-1
   Microsomal Glutathione S-transferase 2, 3
   Microsomal Glutathione S-transferase Ha Subunit 1, 2
   Microsomal Glutathione S-transferase A3, A4
   Glutathione S-transferase A1, A4
   Glutathione S-transferase M1, M2, M3, M4
(7) UDP-glycosyltransferases are enzymes that catalyze the contribution of glucuronic acid to functional groups such as hydroxyl groups, carboxyl groups, amino groups and thiol groups after their introduction in the 1st drug metabolism route. Examples include the following.
   UDP-glycosyltransferase 1
   UDP-glycosyltransferase 1 Family Polypeptide A1
   UDP-glycosyltransferase 2 Family Polypeptide A1, B7, B10, B4, B11, B15, B17
   UDP-glycosyltransferase 8
   Dolichyl-diP-oligosaccharide protein glycosyl transferase
(8) Aldehyde dehydrogenases are enzyme that converts aldehydes into carboxylic acids. Examples include Aldehyde dehydrogenase 1 through 10.
   Aldehyde dehydrogenase 1 family member A1, A2, A3
   Aldehyde dehydrogenase 1 family member B1
   Formyltetrahydroforate dehydrogenase
   Aldehyde dehydrogenase 2
   Aldehyde dehydrogenase 3 family member A1, A2
   Aldehyde dehydrogenase 3 family member B1, B2
   Aldehyde dehydrogenase 5 family member A1
   Aldehyde dehydrogenase 6 family member A1
   Aldehyde dehydrogenase 8 family member A1
   Aldehyde dehydrogenase 9 family member A1
(9) Alcohol dehydrogenases are enzymes that convert alcohols into aldehydes or ketones. Examples include the following.
   Alcohol dehydrogenase 1 through 7
   Hydroxy-CoA-dehydrogenase
   Short-chain alcohol dehydrogenase family genes
(10) Esterases are enzymes that hydrolyze some esters. Examples include the following.
   Arylacetoamide deacetylase
   Granzyme A
   Granzyme B
   Interleukin 17
   Ubiquitin carboxyl-terminal esterase L1, 3
   Carboxyl esterase 1
   Lipase A
   Esterase D-formylglutathione hydrolase
   Carboxylester lipase
   Dolichyl-diphosphooligosaccharide-protein glycosyltransferase (DDOST)
   Neuropathy target esterase
(11) Ubiquinone dehydrogenases (NDUF) are enzymes that support energy metabolism, *e.g*., as in the mitochondrial respiratory chain. Examples include NADH ubiquinone dehydrogenase 1a Subunit 1 through 10.
   NADH-dehydrogenase (ubiquinone)1α-subcomplex 1 through 3 and 5 through 10
   NADH-dehydrogenase (ubiquinone)1α/β-subcomplex 1
   NADH-dehydrogenase (ubiquinone)1β-subcomplex 3, 5, 7
   NADH-dehydrogenase (ubiquinone)Fe-S protein 1, 3, 4, 5, 6, 8
   NADH-dehydrogenase (ubiquinone) flavoprotein 1 through 3
(12) Cytochrome P450s (CYPs) are enzymes that regulate 1st drug metabolism and introduce oxygen atoms to the drug. Examples include Cytochrome P450 (CYP) 1A1, CYP 1A2, CYP1B1, CYP 2A6, CYP 2B6, CYP 2C8, CYP 2C18, CYP 2C9, CYP 2C19, CYP 2E1, CYP 2D6, CYP 2E1, CYP 2F1, CYP 3A3, CYP 3A4, CYP 3A5, CYP 3A7, CYP 3A43, CYP 4A 11, CYP 4B 1, CYP 4F2, CYP 4F3, CYP 4F8, CYP 11B1, CYP 11B2, CYP 17, CYP 19, CYP 21A2, CYP 21A1, CYP 27B1 and CYP 27.
(13) ATP-binding cassettes absorb the drug and adjust the interstitial concentration with a transporter. Examples include the following.
   ATP-Binding Cassette Subfamily A Members 1 through 6, 8
   ATP-Binding Cassette Subfamily A Members 1, 4, 7, 8
   ATP-Binding Cassette Subfamily B Members 1 through 11
   ATP-Binding Cassette Subfamily B Members 1, 4, 7, 8, 9, 10, 11
   ATP-Binding Cassette Subfamily C Members 1 through 6, 8 through 10
   ATP-Binding Cassette Subfamily C Members 1, 2, 3, 4, 5, 7, 8, 9
   ATP-Binding Cassette Subfamily D Members 1 through 4
   ATP-Binding Cassette Subfamily D Members 1, 3,4
   ATP-Binding Cassette Subfamily E Members 1
   ATP-Binding Cassette Subfamily F Members 1 through 3
   ATP-Binding Cassette Subfamily F Member 1
   ATP-Binding Cassette Subfamily G Members 1
   ATP-Binding Cassette Subfamily G Members 1, 2, 4, 8
   Organic anion transporters 1, 2, 3
   Organic anion transporter polypeptides 1, 2, 8
   Transporter 1 ATP-binding cassette subfamily B
   Transporter 2 ATP-binding cassette subfamily B
   SLC22A4 solute carrier family 22 (organic cation transporter) member 4
   SLC22A5 solute carrier family 22 (organic cation transporter) member 5
   SLC22A1 solute carrier family 22 (organic cation transporter) member 1
   SLC22A2 solute carrier family 22 (organic cation transporter) member 2
   SLC10A2 solute carrier family 10 (sodium/bile acid cotransporter family) member 2
   SLC15A1 solute carrier family 15 (oligopeptide transporter) member 1
(14) Other enzymes include gamma glutamyl transferase 1, transglutaminase 1 and dihydropyrimidine dihydrogenase.

Genetic polymorphism data relating to DMEs can be obtained using any general genetic polymorphism detection method. Examples include, but are not limited to, PCR or other amplification methods, hybridization methods using an allele-specific oligonucleotide matrix (e.g., TAQMAN PCR method, INVADER assay method), primer extension reaction methods, sequencing methods, MALDI-TOF/MS methods and the DNA chip methods (e.g., microarrays). Examples of detection methods that are applicable to analysis of the DME associated polymorphisms of the present invention include but are not limited to those listed below.

### 1. Direct sequencing Assays

In some embodiments of the present invention, variant sequences are detected using a direct sequencing technique. In these assays, DNA samples are first isolated from a subject using any suitable method. In some embodiments, the region of interest is cloned into a suitable vector and amplified by growth in a host cell (*e.g*., a bacteria). In other embodiments, DNA in the region of interest is amplified using PCR.

Following amplification, DNA in the region of interest (*e.g*., the region containing the SNP or mutation of interest) is sequenced using any suitable method, including but not limited to manual sequencing using radioactive marker nucleotides, or automated sequencing. The results of the sequencing are displayed using any suitable method. The sequence is examined and the presence or absence of a given SNP or mutation is determined.

### 2. PCR Assay

In some embodiments of the present invention, variant sequences are detected using a PCR-based assay. In some embodiments, the PCR assay comprises the use of oligonucleotide primers that hybridize only to the variant or wild type allele (*e.g*., to the region of polymorphism or mutation). Both sets of primers are used to amplify a sample of DNA. If only the mutant primers result in a PCR product, then the patient has the mutant allele. If only the wild-type primers result in a PCR product, then the patient has the wild type allele.

### 3. Fragment Length Polymorphism Assays

In some embodiments of the present invention, variant sequences are detected using a fragment length polymorphism assay. In a fragment length polymorphism assay, a unique DNA banding pattern based on cleaving the DNA at a series of positions is generated using an enzyme (*e.g*., a restriction enzyme or a CLEAVASE I [Third Wave Technologies, Madison, WI] enzyme). DNA fragments from a sample containing a SNP or a mutation will have a different banding pattern than wild type.

### a. RFLP Assay

In some embodiments of the present invention, variant sequences are detected using a restriction fragment length polymorphism assay (RFLP). The region of interest is first isolated using PCR. The PCR products are then cleaved with restriction enzymes known to give a unique length fragment for a given polymorphism. The restriction-enzyme digested PCR products are generally separated by gel electrophoresis and may be visualized by ethidium bromide staining. The length of the fragments is compared to molecular weight markers and fragments generated from wild-type and mutant controls.

### b. CFLP Assay

In other embodiments, variant sequences are detected using a CLEAVASE fragment length polymorphism assay (CFLP; Third Wave Technologies, Madison, WI; *See e.g.,* U.S. Patent Nos. 5,843,654; 5,843,669; 5,719,208; and 5,888,780; each of which is herein incorporated by reference). This assay is based on the observation that when single strands of DNA fold on themselves, they assume higher order structures that are highly individual to the precise sequence of the DNA molecule. These secondary structures involve partially duplexed regions of DNA such that single stranded regions are juxtaposed with double stranded DNA hairpins. The CLEAVASE I enzyme, is a structure-specific, thermostable nuclease that recognizes and cleaves the junctions between these single-stranded and double-stranded regions.

The region of interest is first isolated, for example, using PCR. In preferred embodiments, one or both strands are labeled. Then, DNA strands are separated by heating. Next, the reactions are cooled to allow intrastrand secondary structure to form. The PCR products are then treated with the CLEAVASE I enzyme to generate a series of fragments that are unique to a given SNP or mutation. The CLEAVASE enzyme treated PCR products are separated and detected (*e.g*., by denaturing gel electrophoresis) and visualized (*e.g*., by autoradiography, fluorescence imaging or staining). The length of the fragments is compared to molecular weight markers and fragments generated from wild-type and mutant controls.

### 4. Hybridization Assays

In preferred embodiments of the present invention, variant sequences are detected a hybridization assay. In a hybridization assay, the presence of absence of a given SNP or mutation is determined based on the ability of the DNA from the sample to hybridize to a complementary DNA molecule (*e.g*., a oligonucleotide probe). A variety of hybridization assays using a variety of technologies for hybridization and detection are available. A description of a selection of assays is provided below.

### a. Direct Detection of Hybridization

In some embodiments, hybridization of a probe to the sequence of interest (*e.g*., a SNP or mutation) is detected directly by visualizing a bound probe (*e.g*., a Northern or Southern assay; *See e.g.,* Ausabel et al. (eds.), Current Protocols in Molecular Biology, John Wiley & Sons, NY [1991]). In a these assays, genomic DNA (Southern) or RNA (Northern) is isolated from a subject. The DNA or RNA is then cleaved with a series of restriction enzymes that cleave infrequently in the genome and not near any of the markers being assayed. The DNA or RNA is then separated (*e.g*., on an agarose gel) and transferred to a membrane. A labeled (*e.g.,* by incorporating a radionucleotide) probe or probes specific for the SNP or mutation being detected is allowed to contact the membrane under a condition or low, medium, or high stringency conditions. Unbound probe is removed and the presence of binding is detected by visualizing the labeled probe.

### b. Detection of Hybridization Using "DNA Chip" Assays

In some embodiments of the present invention, variant sequences are detected using a DNA chip hybridization assay. In this assay, a series of oligonucleotide probes are affixed to a solid support. The oligonucleotide probes are designed to be unique to a given SNP or mutation. The DNA sample of interest is contacted with the DNA "chip" and hybridization is detected.

In some embodiments, the DNA chip assay is a GeneChip (Affymetrix, Santa Clara, CA; *See e.g.,* U.S. Patent Nos. 6,045,996; 5,925,525; and 5,858,659; each of which is herein incorporated by reference) assay. The GeneChip technology uses miniaturized, high-density arrays of oligonucleotide probes affixed to a "chip." Probe arrays are manufactured by Affymetrix's light-directed chemical synthesis process, which combines solid-phase chemical synthesis with photolithographic fabrication techniques employed in the semiconductor industry. Using a series of photolithographic masks to define chip exposure sites, followed by specific chemical synthesis steps, the process constructs high-density arrays of oligonucleotides, with each probe in a predefined position in the array. Multiple probe arrays are synthesized simultaneously on a large glass wafer. The wafers are then diced, and individual probe arrays are packaged in injection-molded plastic cartridges, which protect them from the environment and serve as chambers for hybridization.

The nucleic acid to be analyzed is isolated, amplified by PCR, and labeled with a fluorescent reporter group. The labeled DNA is then incubated with the array using a fluidics station. The array is then inserted into the scanner, where patterns of hybridization are detected. The hybridization data are collected as light emitted from the fluorescent reporter groups already incorporated into the target, which is bound to the probe array. Probes that perfectly match the target generally produce stronger signals than those that have mismatches. Since the sequence and position of each probe on the array are known, by complementarity, the identity of the target nucleic acid applied to the probe array can be determined.

In other embodiments, a DNA microchip containing electronically captured probes (Nanogen, San Diego, CA) is utilized *(See e.g.,* U.S. Patent Nos. 6,017,696; 6,068,818; and 6,051,380; each of which are herein incorporated by reference). Through the use of microelectronics, Nanogen's technology enables the active movement and concentration of charged molecules to and from designated test sites on its semiconductor microchip. DNA capture probes unique to a given SNP or mutation are electronically placed at, or "addressed" to, specific sites on the microchip. Since DNA has a strong negative charge, it can be electronically moved to an area of positive charge.

First, a test site or a row of test sites on the microchip is electronically activated with a positive charge. Next, a solution containing the DNA probes is introduced onto the microchip. The negatively charged probes rapidly move to the positively charged sites, where they concentrate and are chemically bound to a site on the microchip. The microchip is then washed and another solution of distinct DNA probes is added until the array of specifically bound DNA probes is complete.

A test sample is then analyzed for the presence of target DNA molecules by determining which of the DNA capture probes hybridize, with complementary DNA in the test sample (e.g., a PCR amplified gene of interest). An electronic charge is also used to move and concentrate target molecules to one or more test sites on the microchip. The electronic concentration of sample DNA at each test site promotes rapid hybridization of sample DNA with complementary capture probes (hybridization may occur in minutes). To remove any unbound or nonspecifically bound DNA from each site, the polarity or charge of the site is reversed to negative, thereby forcing any unbound or nonspecifically bound DNA back into solution away from the capture probes. A laser-based fluorescence scanner is used to detect binding,

In still further embodiments, an array technology based upon the segregation of fluids on a flat surface (chip) by differences in surface tension (ProtoGene, Palo Alto, CA) is utilized *(See e.g.,* U.S. Patent Nos. 6,001,311; 5,985,551; and 5,474,796; each of which is herein incorporated by reference). Protogene's technology is based on the fact that fluids can be segregated on a flat surface by differences in surface tension that have been imparted by chemical coatings. Once so segregated, oligonucleotide probes are synthesized directly on the chip by ink-jet printing of reagents. The array with its reaction sites defined by surface tension is mounted on a X/Y translation stage under a set of four piezoelectric nozzles, one for each of the four standard DNA bases. The translation stage moves along each of the rows of the array and the appropriate reagent is delivered to each of the reaction site. For example, the A amidite is delivered only to the sites where amidite A is to be coupled during that synthesis step and so on. Common reagents and washes are delivered by flooding the entire surface and then removing them by spinning.

DNA probes unique for the SNP or mutation of interest are affixed to the chip using Protogene's technology. The chip is then contacted with the PCR-amplified genes of interest. Following hybridization, unbound DNA is removed and hybridization is detected using any suitable method (e.g., by fluorescence de-quenching of an incorporated fluorescent group).

In yet other embodiments, a "bead array" is used for the detection of polymorphisms (Illumina, San Diego, CA; *See e.g.,* PCT Publications WO 99/67641 and WO 00/39587, each of which is herein incorporated by reference). lllumina uses a BEAD ARRAY technology that combines fiber optic bundles and beads that self-assemble into an array. Each fiber optic bundle contains thousands to millions of individual fibers depending on the diameter of the bundle. The beads are coated with an oligonucleotide specific for the detection of a given SNP or mutation. Batches of beads are combined to form a pool specific to the array. To perform an assay, the BEAD ARRAY is contacted with a prepared subject sample (e.g., DNA). Hybridization is detected using any suitable method.

### c. Enzymatic Detection of Hybridization

In some embodiments of the present invention, hybridization is detected by enzymatic cleavage of specific structures (INVADER assay, Third Wave Technologies; *See e.g.,* U.S. Patent Nos. 5,846,717, 6,090,543; 6,001,567; 5,985,557; and 5,994,069; each of which is herein incorporated by reference). The INVADER assay detects specific DNA and RNA sequences by using structure-specific enzymes to cleave a complex formed by the hybridization of overlapping oligonucleotide probes. Elevated temperature and an excess of one of the probes enable multiple probes to be cleaved for each target sequence present without temperature cycling. These cleaved probes then direct cleavage of a second labeled probe. The secondary probe oligonucleotide can be 5'-end labeled with a fluorescent dye that is quenched by a second dye or other quenching moiety. Upon cleavage, the de-quenched dye-labeled product may be detected using a standard fluorescence plate reader, or an instrument configured to collect fluorescence data during the course of the reaction (i.e., a "real-time" fluorescence detector, such as an ABI 7700 Sequence Detection System, Applied Biosystems, Foster City, CA).

The INVADER assay detects specific mutations and SNPs in unamplified genomic DNA. In an embodiment of the INVADER assay used for detecting SNPs in genomic DNA, two oligonucleotides (a primary probe specific either for a SNP/mutation or wild type sequence, and an INVADER oligonucleotide) hybridize in tandem to the genomic DNA to form an overlapping structure. A structure-specific nuclease enzyme recognizes this overlapping structure and cleaves the primary probe. In a secondary reaction, cleaved primary probe combines with a fluorescence-labeled secondary probe to create another overlapping structure that is cleaved by the enzyme. The initial and secondary reactions can run concurrently in the same vessel. Cleavage of the secondary probe is detected by using a fluorescence detector, as described above. The signal of the test sample may be compared to known positive and negative controls.

In some embodiments, hybridization of a bound probe is detected using a TAQMAN assay (PE Biosystems, Foster City, CA; *See e.g.,* U.S. Patent Nos. 5,962,233 and 5,538,848, each of which is herein incorporated by reference). The assay is performed during a PCR reaction. The TAQMAN assay exploits the 5'-3' exonuclease activity of DNA polymerases such as AMPLITAQ DNA polymerase. A probe, specific for a given allele or mutation, is included in the PCR reaction. The probe consists of an oligonucleotide with a 5'-reporter dye (e.g., a fluorescent dye) and a 3'-quencher dye. During PCR, if the probe is bound to its target, the 5'-3' nucleolytic activity of the AMPLITAQ polymerase cleaves the probe between the reporter and the quencher dye. The separation of the reporter dye from the quencher dye results in an increase of fluorescence. The signal accumulates with each cycle of PCR and can be monitored with a fluorimeter.

In still further embodiments, polymorphisms are detected using the SNP-IT primer extension assay (Orchid Biosciences, Princeton, NJ; *See e.g.,* U.S. Patent Nos. 5,952,174 and 5,919,626, each of which is herein incorporated by reference). In this assay, SNPs are identified by using a specially synthesized DNA primer and a DNA polymerase to selectively extend the DNA chain by one base at the suspected SNP location. DNA in the region of interest is amplified and denatured. Polymerase reactions are then performed using miniaturized systems called microfluidics. Detection is accomplished by adding a label to the nucleotide suspected of being at the SNP or mutation location. Incorporation of the label into the DNA can be detected by any suitable method (e.g., if the nucleotide contains a biotin label, detection is via a fluorescently labelled antibody specific for biotin).

### 5. Other Detection Assays

Additional detection assays that are produced and utilized using the systems and methods of the present invention include, but are not limited to, enzyme mismatch cleavage methods (e.g., Variagenics, U.S. Pat. Nos. 6,110,684, 5,958,692, 5,851,770, herein incorporated by reference in their entireties); polymerase chain reaction; branched hybridization methods (e.g., Chiron, U.S. Pat. Nos. 5,849,481, 5,710,264,5,124,246, and 5,624,802, herein incorporated by reference in their entireties); rolling circle replication (e.g., U.S. Pat. Nos. 6,210,884 and 6,183,960, herein incorporated by reference in their entireties); NASBA (e.g., U.S. Pat. No. 5,409,818, herein incorporated by reference in its entirety); molecular beacon technology (e.g., U.S. Pat. No. 6,150,097, herein incorporated by reference in its entirety); E-sensor technology (Motorola, U.S. Pat. Nos. 6,248,229, 6,221,583, 6,013,170, and 6,063,573, herein incorporated by reference in their entireties); cycling probe technology (e.g., U.S. Pat. Nos. 5,403,711, 5,011,769, and 5,660,988, herein incorporated by reference in their entireties); Dade Behring signal amplification methods (e.g., U.S. Pat. Nos. 6,121,001, 6,110,677, 5,914,230, 5,882,867, and 5,792,614, herein incorporated by reference in their entireties); ligase chain reaction (Barnay Proc. Natl. Acad. Sci USA 88, 189-93 (1991)); and sandwich hybridization methods (e.g., U.S. Pat. No. 5,288,609, herein incorporated by reference in its entirety).

### 6. Mass Spectroscopy Assay

In some embodiments, a MassARRAY system (Sequenom, San Diego, CA.) is used to detect variant sequences (*See e.g.,* U.S. Patent Nos. 6,043,031; 5,777,324; and 5,605,798; each of which is herein incorporated by reference). DNA is isolated from blood samples using standard procedures. Next, specific DNA regions containing the mutation or SNP of interest, about 200 base pairs in length, are amplified by PCR. The amplified fragments are then attached by one strand to a solid surface and the non-immobilized strands are removed by standard denaturation and washing. The remaining immobilized single strand then serves as a template for automated enzymatic reactions that produce genotype specific diagnostic products.

Very small quantities of the enzymatic products, typically five to ten nanoliters, are then transferred to a SpectroCHIP array for subsequent automated analysis with the SpectroREADER mass spectrometer. Each spot is preloaded with light absorbing crystals that form a matrix with the dispensed diagnostic product. The MassARRAY system uses MALDI-TOF (Matrix Assisted Laser Desorption Ionization - Time of Flight) mass spectrometry. In a process known as desorption, the matrix is hit with a pulse from a laser beam. Energy from the laser beam is transferred to the matrix and it is vaporized resulting in a small amount of the diagnostic product being expelled into a flight tube. As the diagnostic product is charged when an electrical field pulse is subsequently applied to the tube they are launched down the flight tube towards a detector. The time between application of the electrical field pulse and collision of the diagnostic product with the detector is referred to as the time of flight. This is a very precise measure of the product's molecular weight, as a molecule's mass correlates directly with time of flight with smaller molecules flying faster than larger molecules. The entire assay is completed in less than one thousandth of a second, enabling samples to be analyzed in a total of 3-5 second including repetitive data collection. The SpectroTYPER software then calculates, records, compares and reports the genotypes at the rate of three seconds per sample.

In some embodiments, the present invention provides an oligonucleotide comprising a DME related sequence, or a complement of a DME-related sequence. In preferred embodiments, an oligonucleotide of the present invention comprises a sequence or a complement of a sequence selected from the group consisting SEQ ID NOs. 1-3360 and 3361-7669, or a substantially similar sequence.

In some embodiments, an oligonucleotide probe or oligonucleotide primer is created so the 5' terminus, 3' terminus or central base contains the genetic polymorphism site. In some preferred embodiments, an oligonucleotide is created comprising at least 13 contiguous bases of a sequence selected from SEQ ID NOs 1 through 3360 or the complement thereto, and further comprising the 21^{st} nucleotide of the sequence selected from SEQ ID NOs 1 through 3360, or the complement thereto.

In some embodiments, an oligonucleotide of the present invention flanks or is adjacent to a polymorphic site, such that the presence of the polymorphism can be detected by modification of the oligonucleotide in a manner dependent on the presence or absence of the polymorphism.

In some embodiments, the present invention provides kits comprising one or more of the components necessary for practicing the present invention. For example, the present invention provides kits for storing or delivering the enzymes of the present invention and/or the reaction components necessary to practice a cleavage assay (e.g., the INVADER assay). The kit may include any and all components necessary or desired for the enzymes or assays including, but not limited to, the reagents themselves, buffers, control reagents (e.g., tissue samples, positive and negative control target oligonucleotides, etc.), solid supports, labels, written and/or pictorial instructions and product information, inhibitors, labeling and/or detection reagents, package environmental controls (e.g., ice, desiccants, etc.), and the like. In some embodiments, the kits provide a sub-set of the required components, wherein it is expected that the user will supply the remaining components. In some embodiments, the kits comprise two or more separate containers wherein each container houses a subset of the components to be delivered. For example, a first container (e.g., box) may contain an enzyme (e.g., structure specific cleavage enzyme in a suitable storage buffer and container), while a second box may contain oligonucleotides (e.g., INVADER oligonucleotides, probe oligonucleotides, control target oligonucleotides, etc.). In some embodiments one or more the reaction components may be provided in a predispensed format (i.e., premeasured for use in a step of the procedure without re-measurement or re-dispensing). In some embodiments, selected reaction components are mixed and predispensed together. In preferred embodiments, predispensed reaction components are predispensed and are provided in a reaction vessel (including but not limited to a reaction tube or a well, as in, e.g., a microtiter plate). In particularly preferred embodiments, predispensed reaction components are dried down (e.g., desiccated or lyophilized) in a reaction vessel.

Examples of genetic polymorphism data (especially the SNP data) that can be used in the method of the present invention are shown in Table 1.

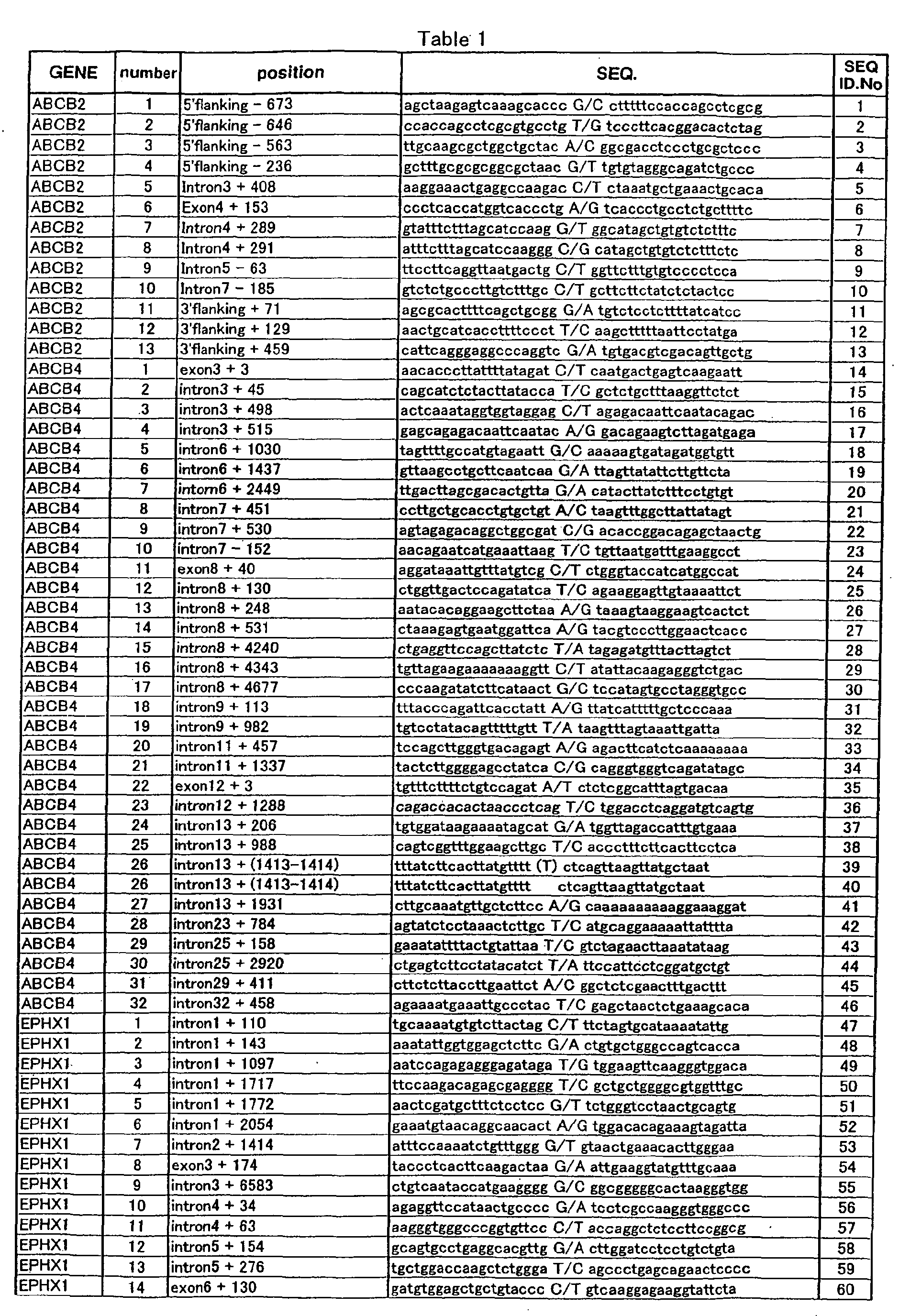

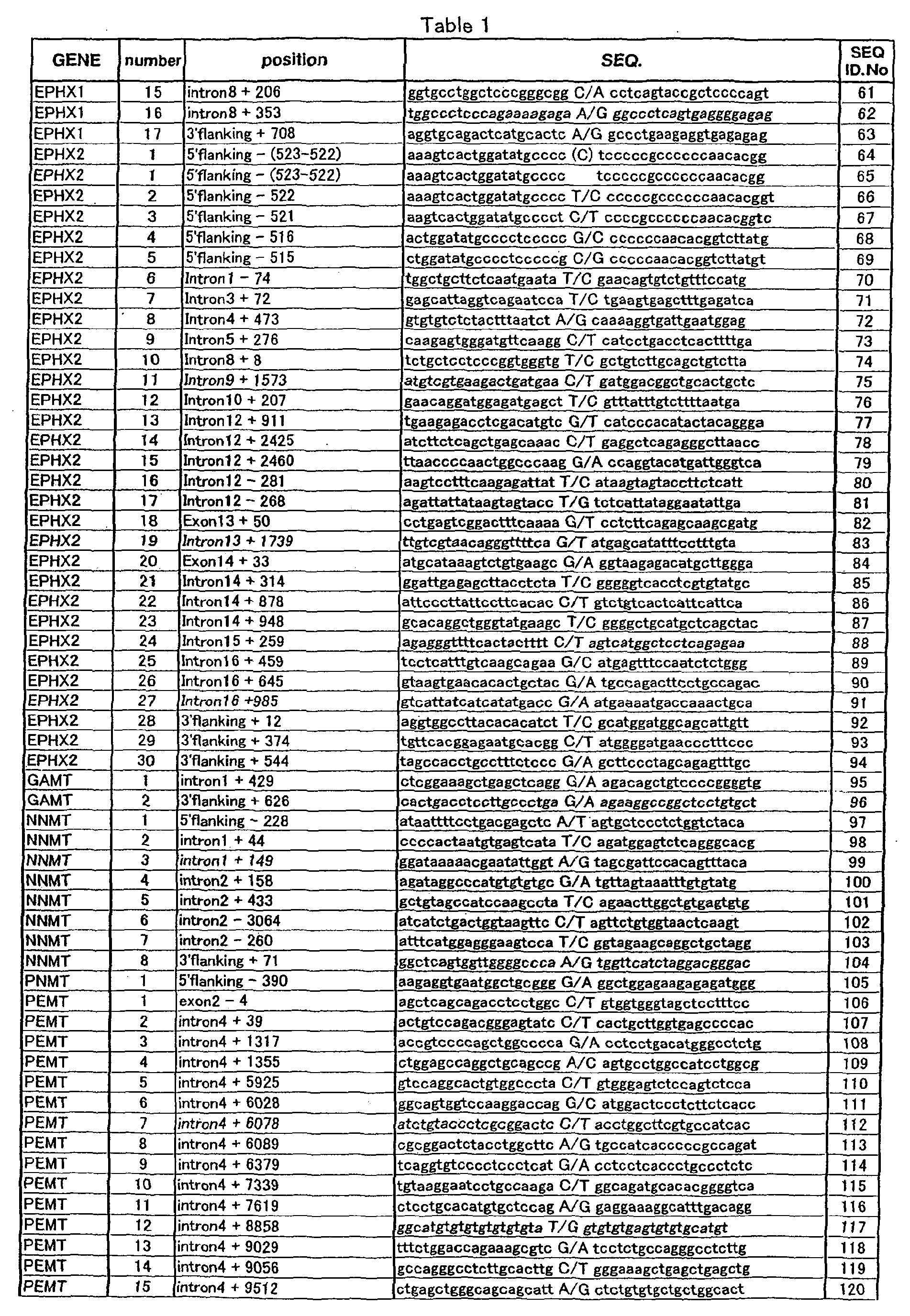

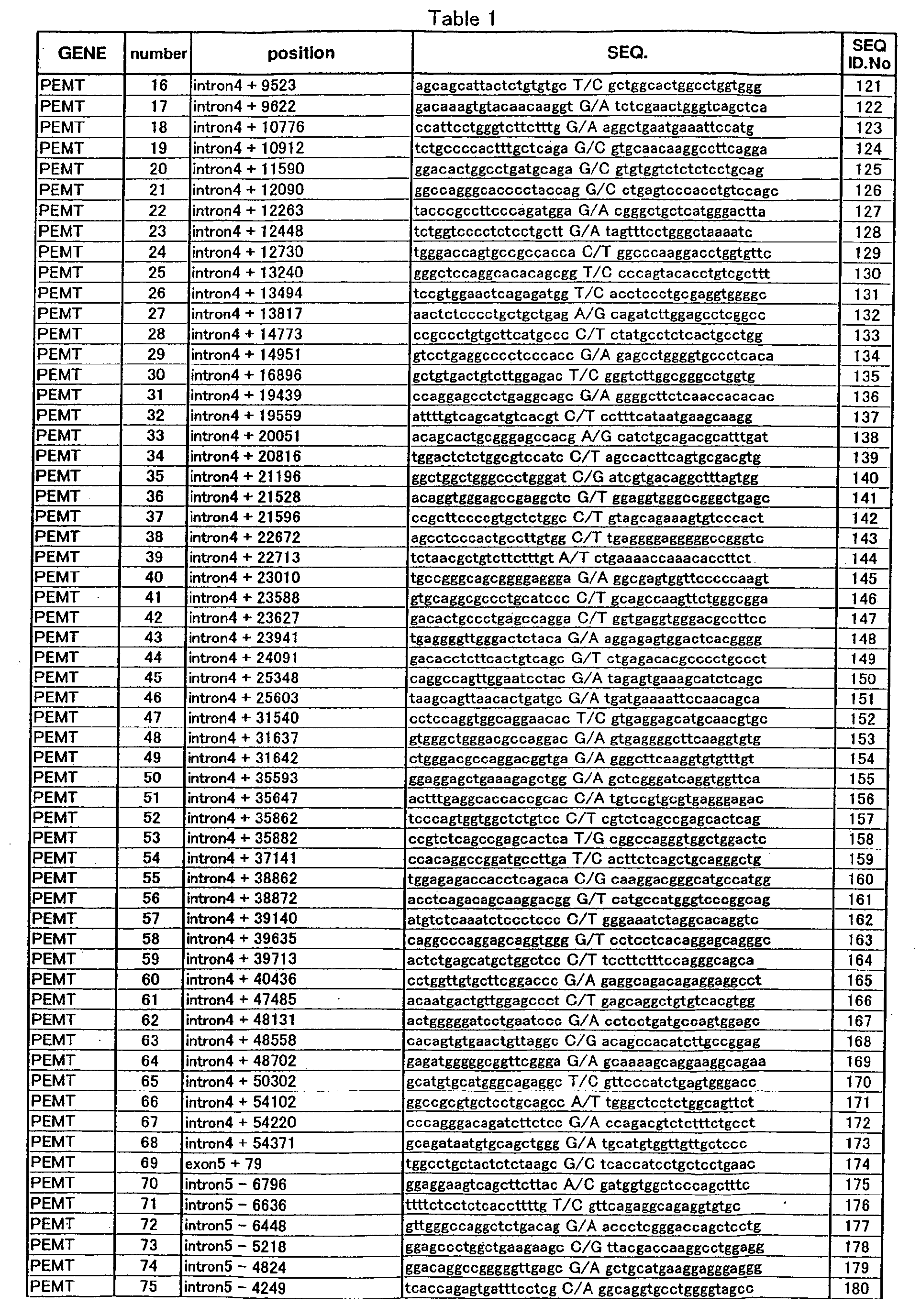

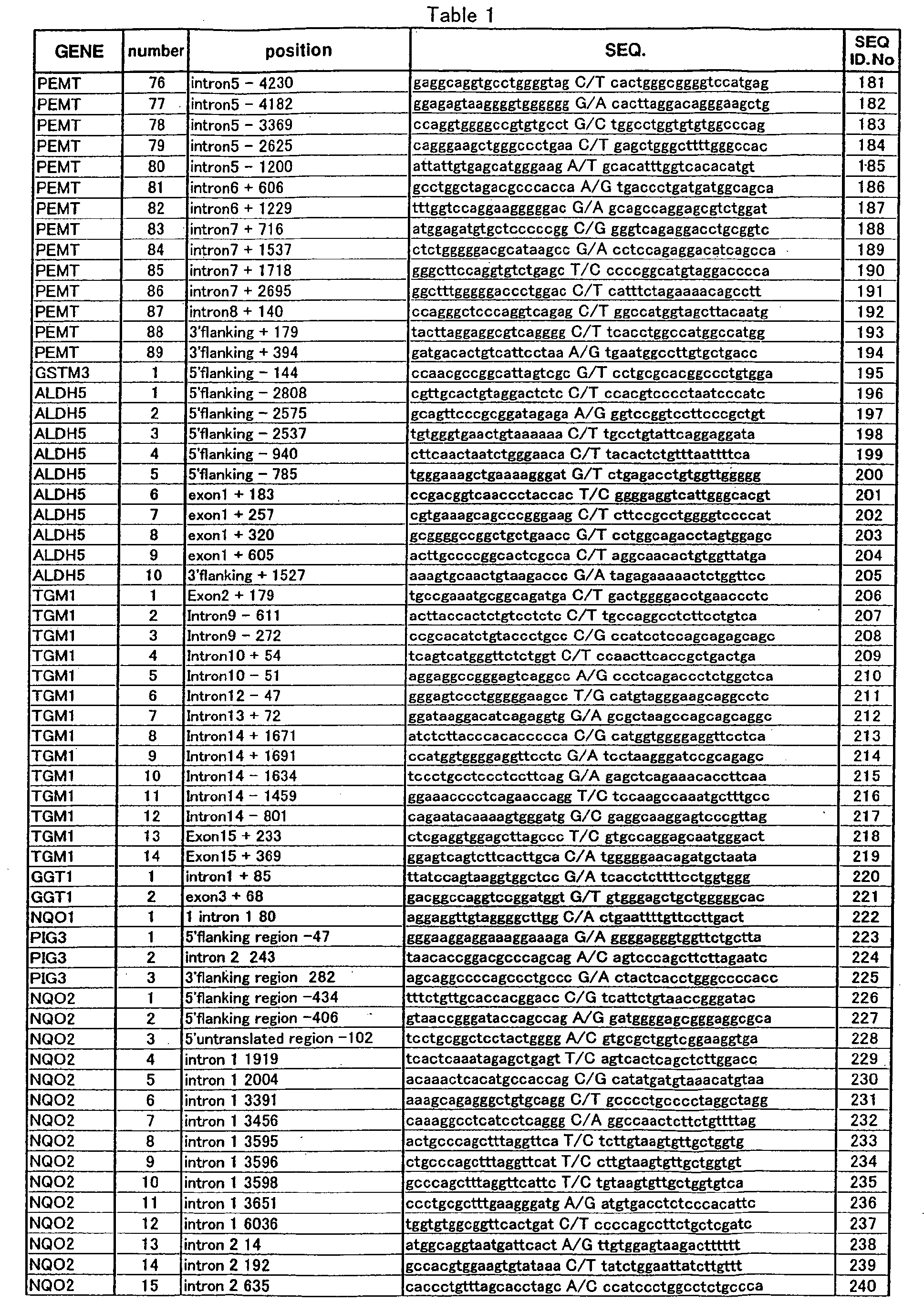

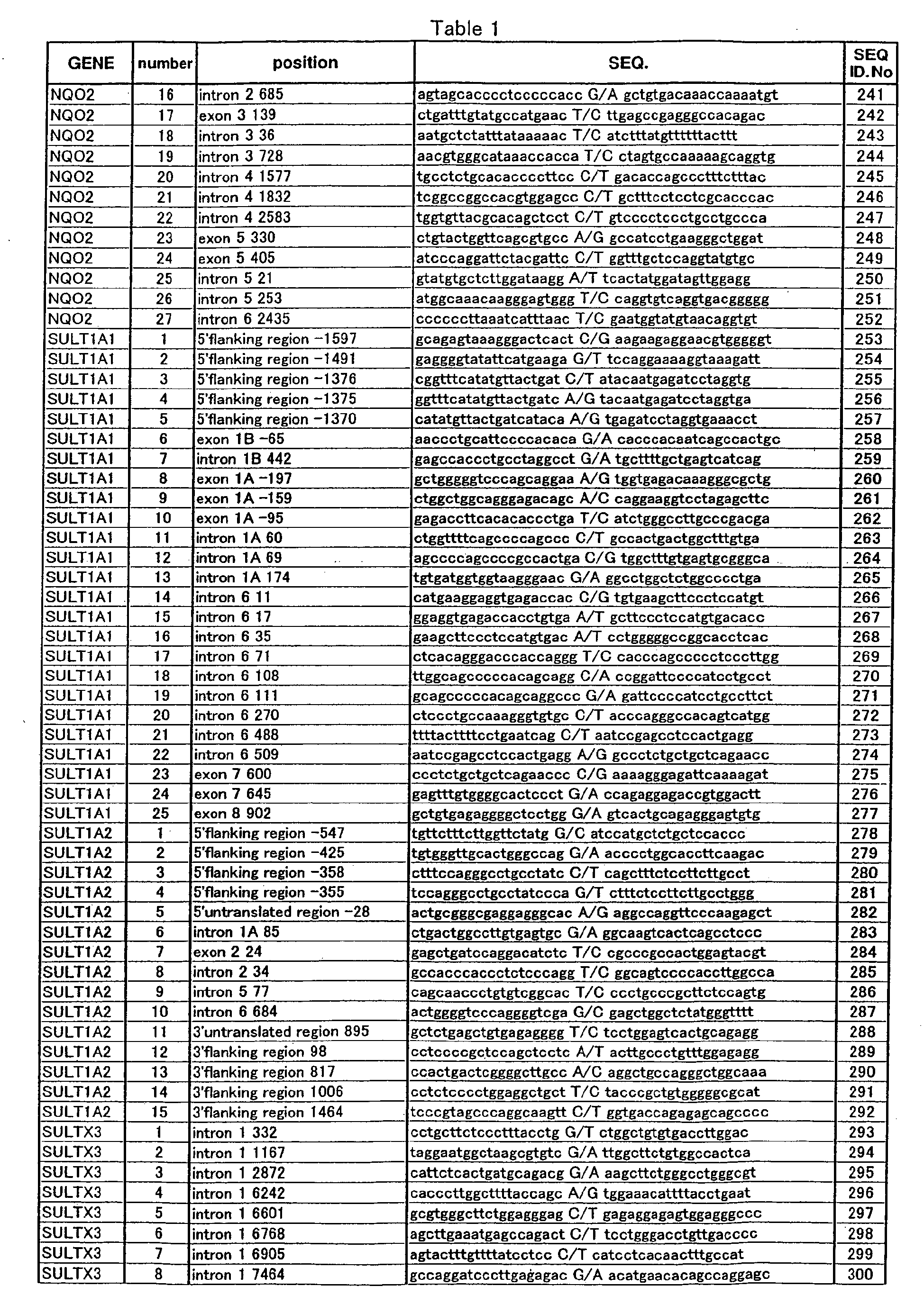

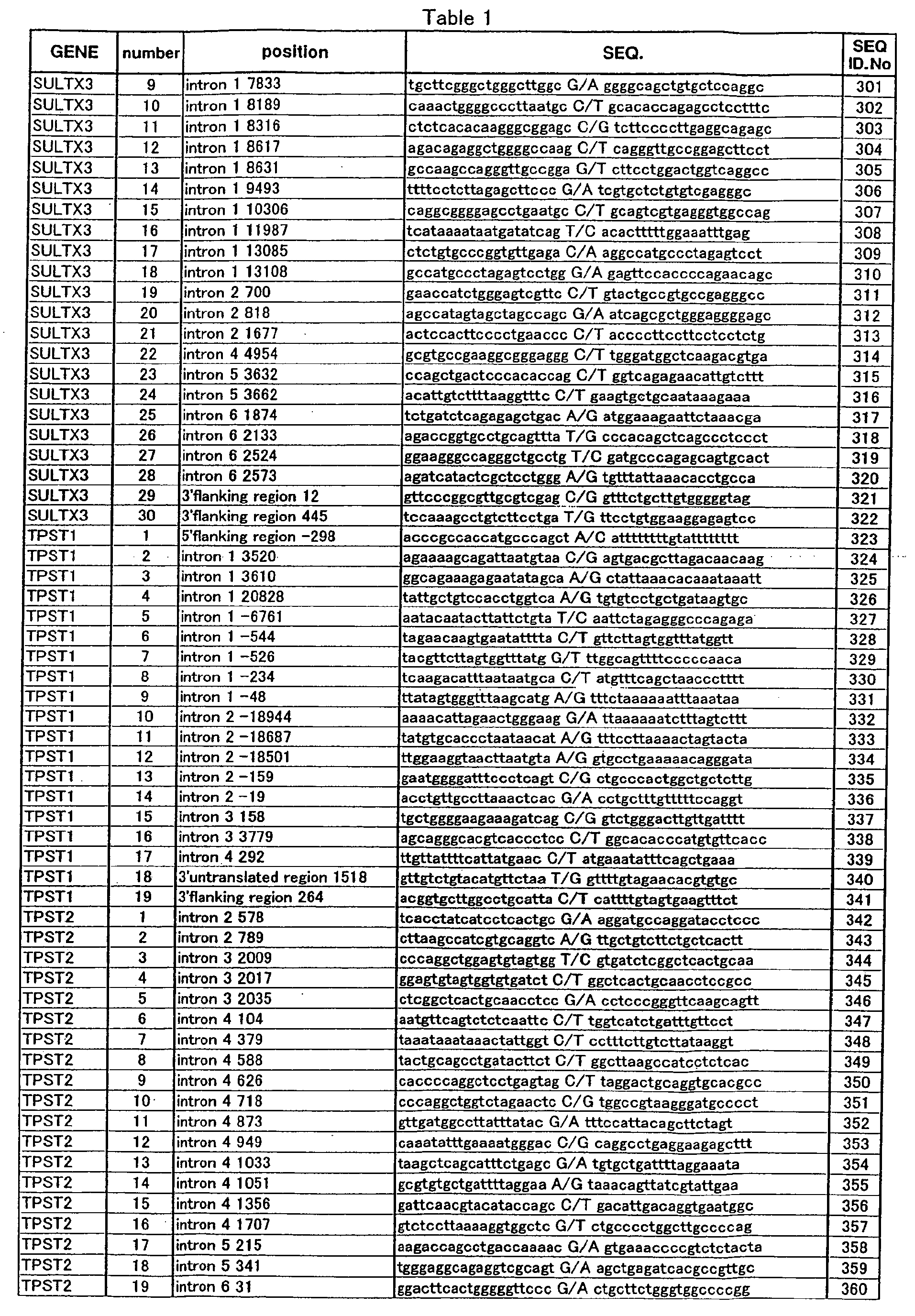

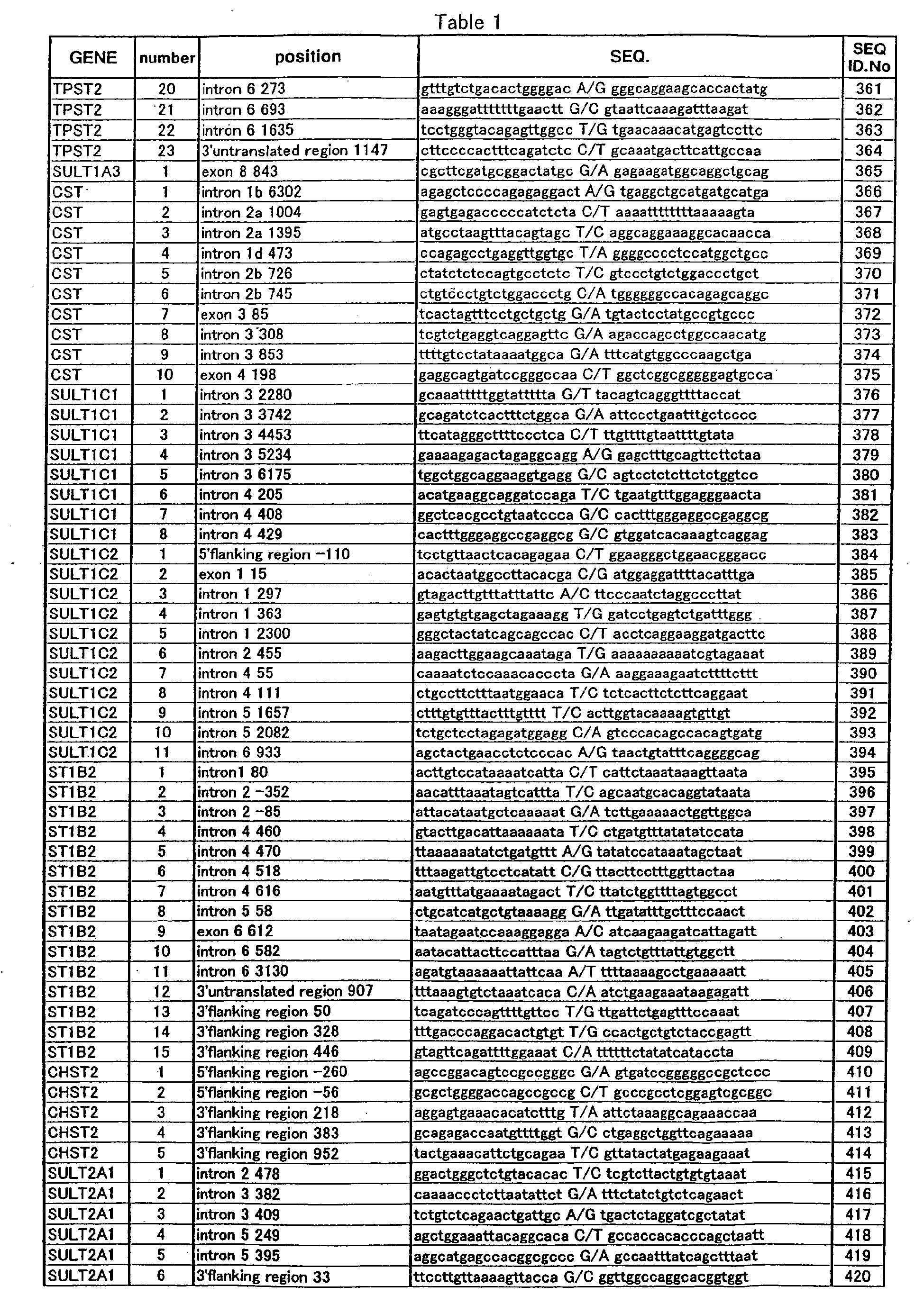

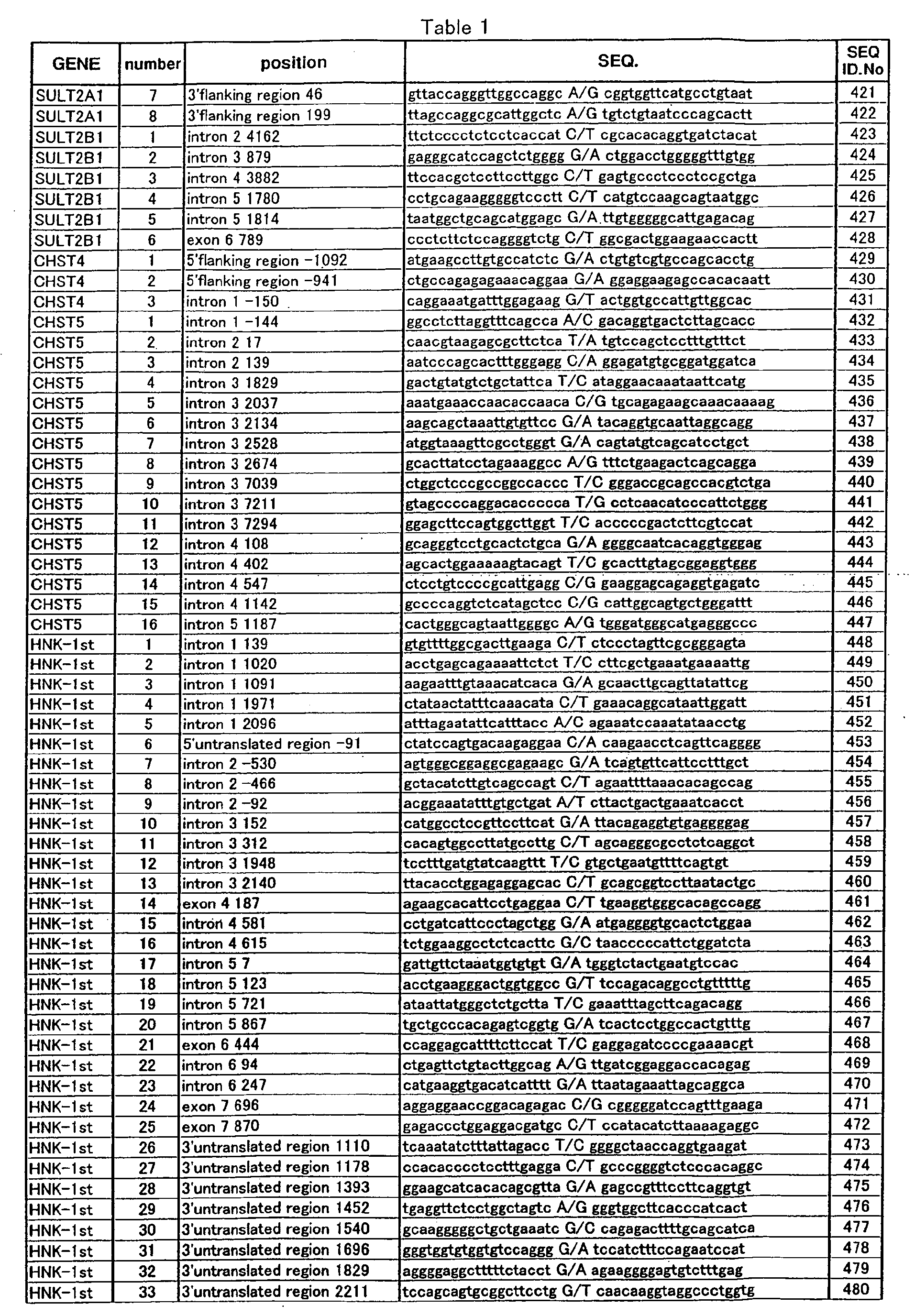

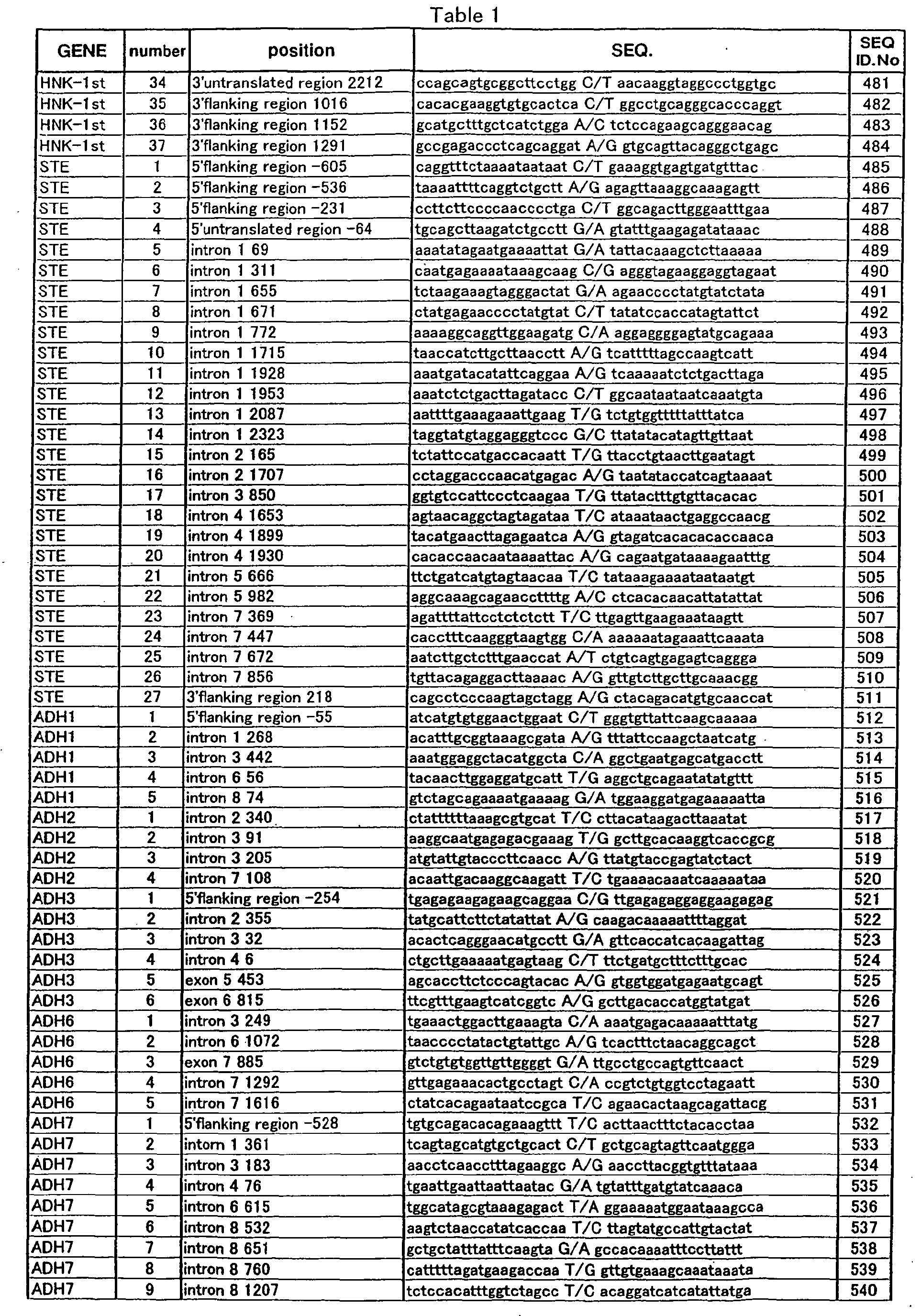

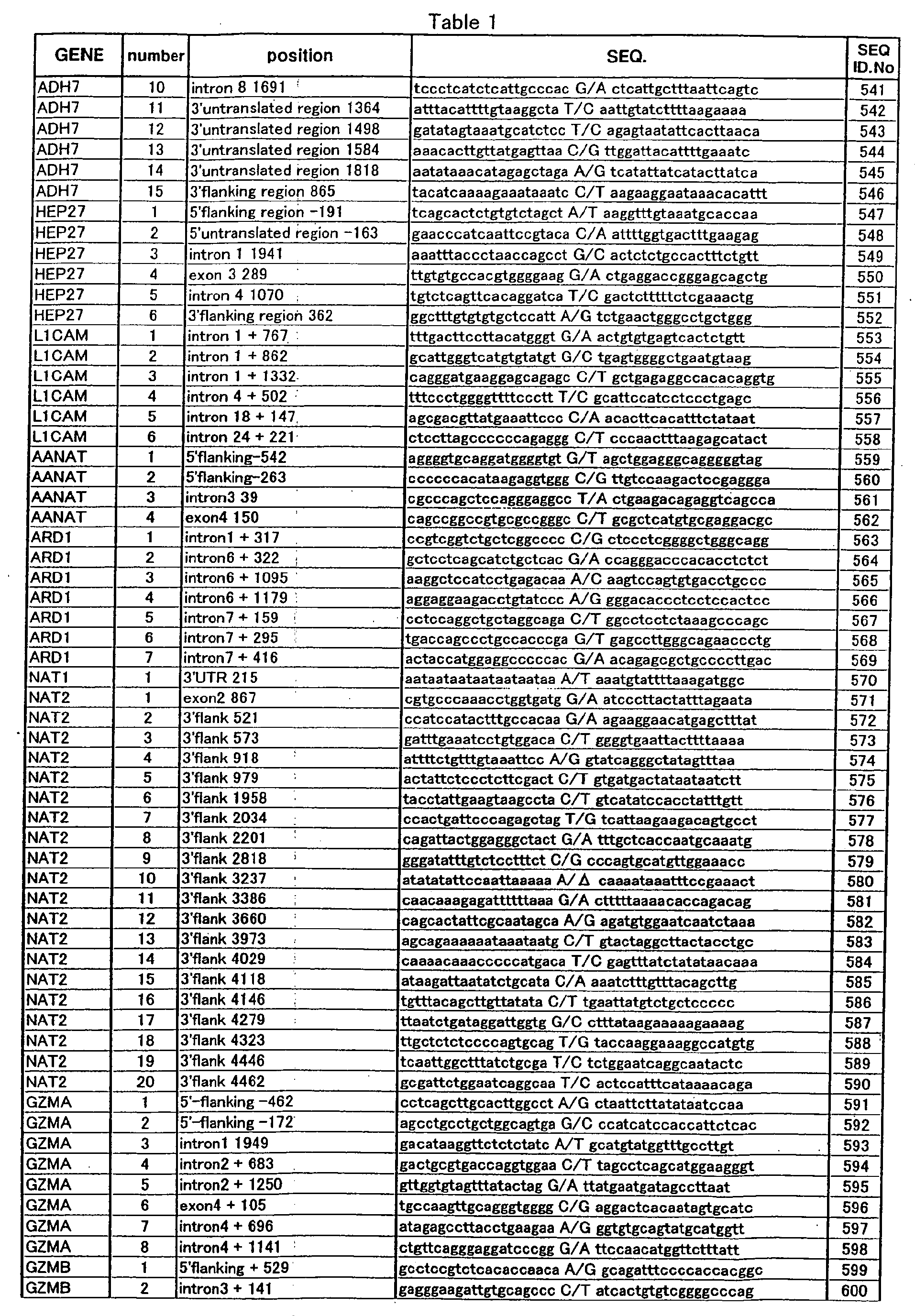

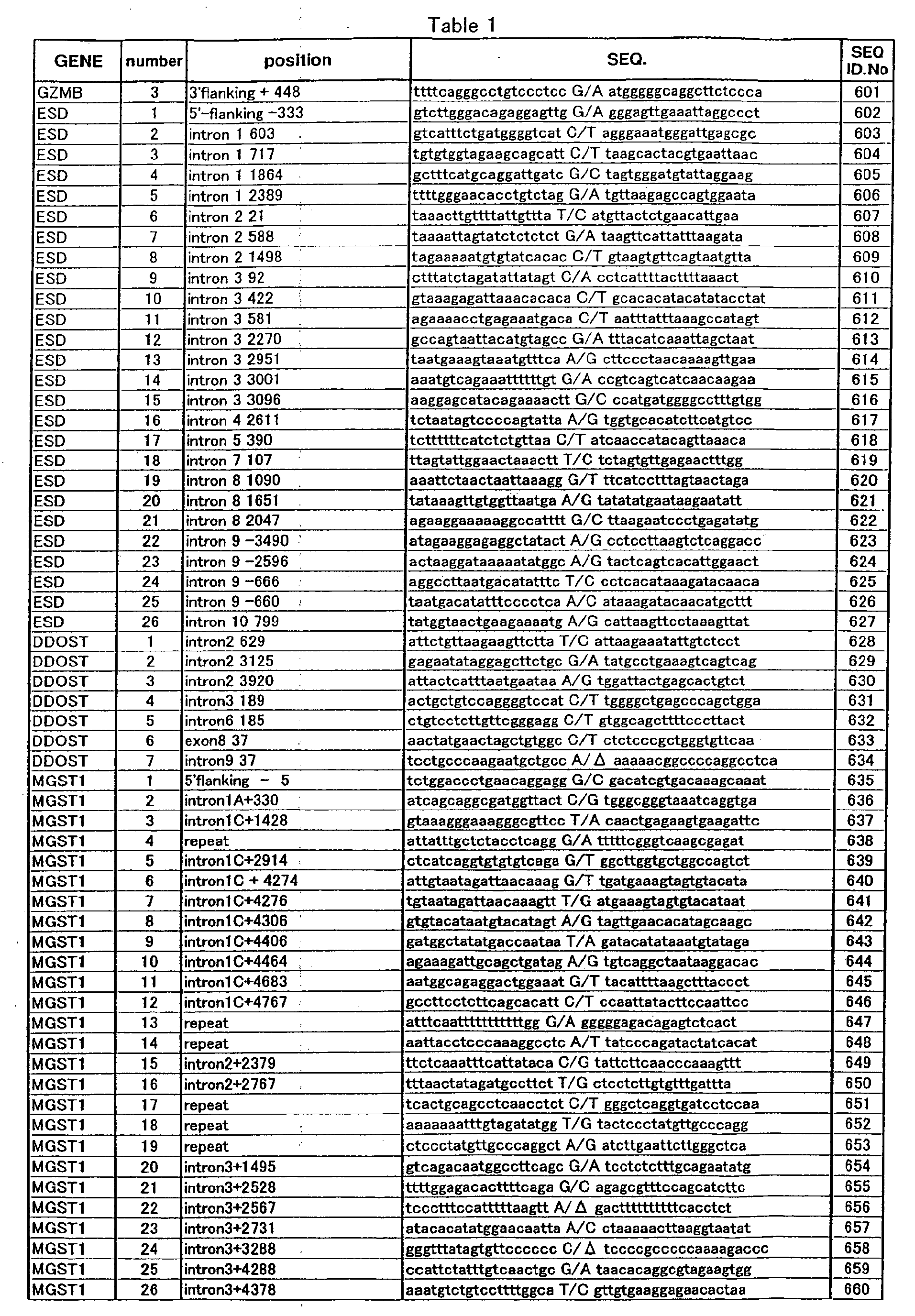

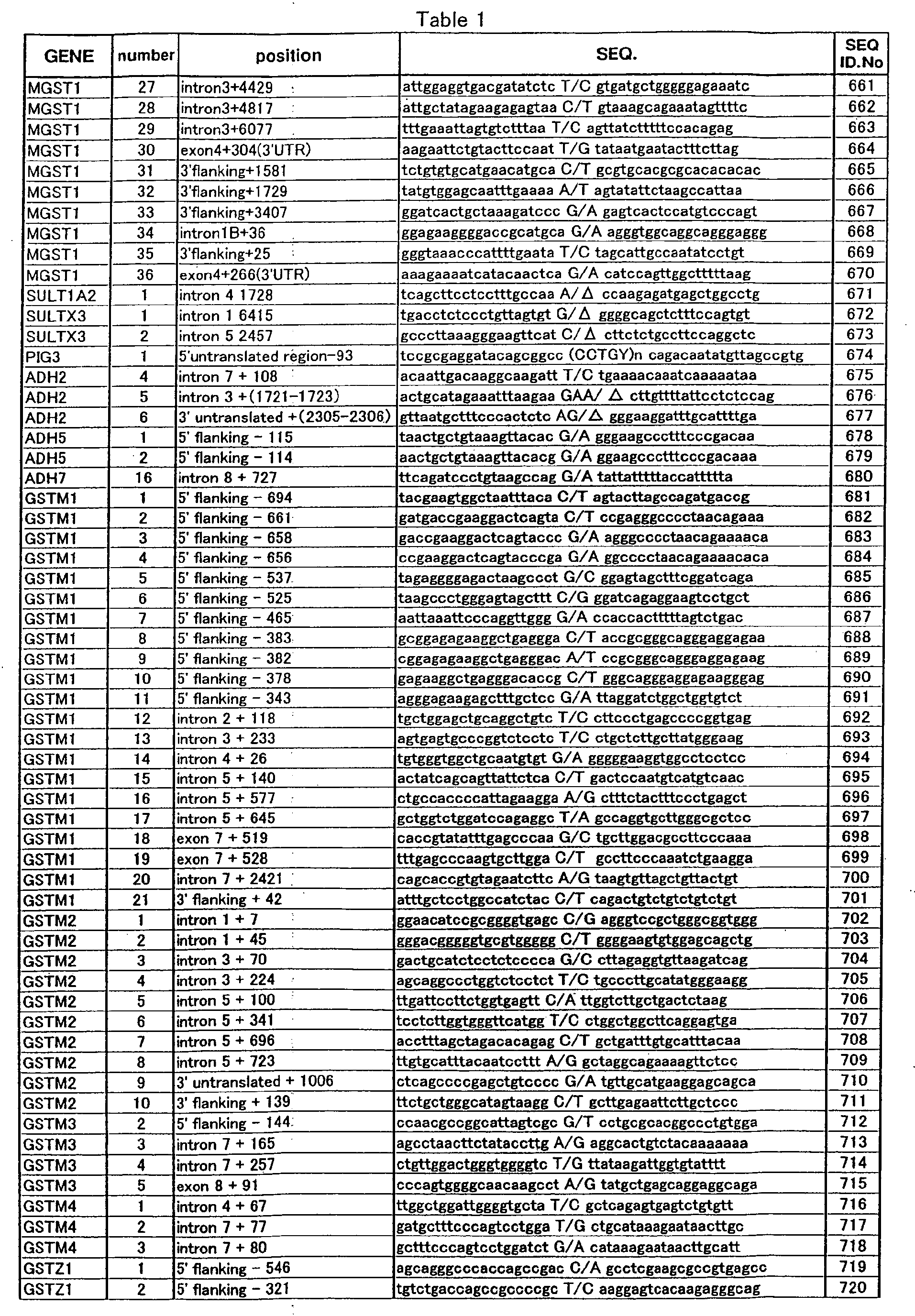

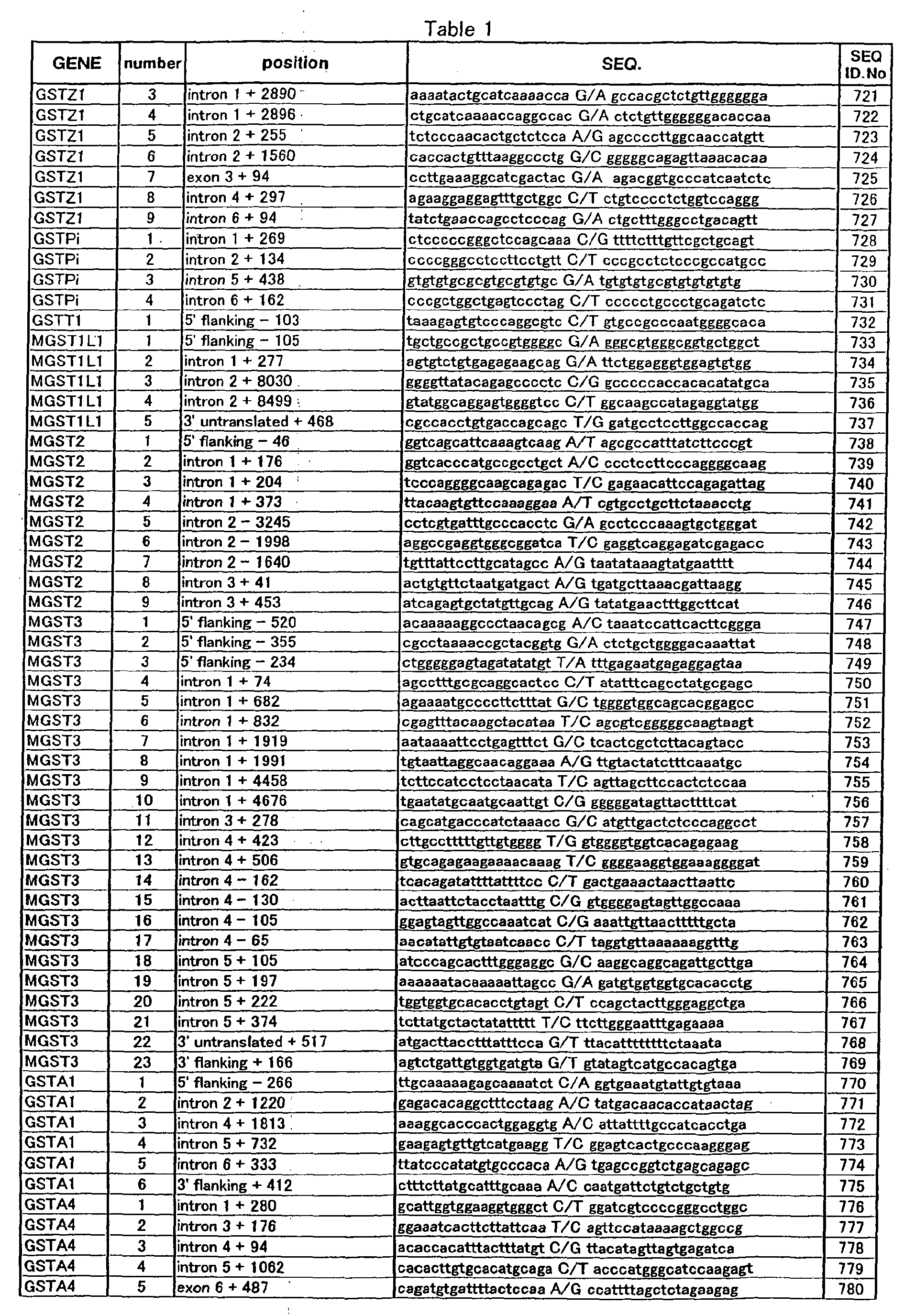

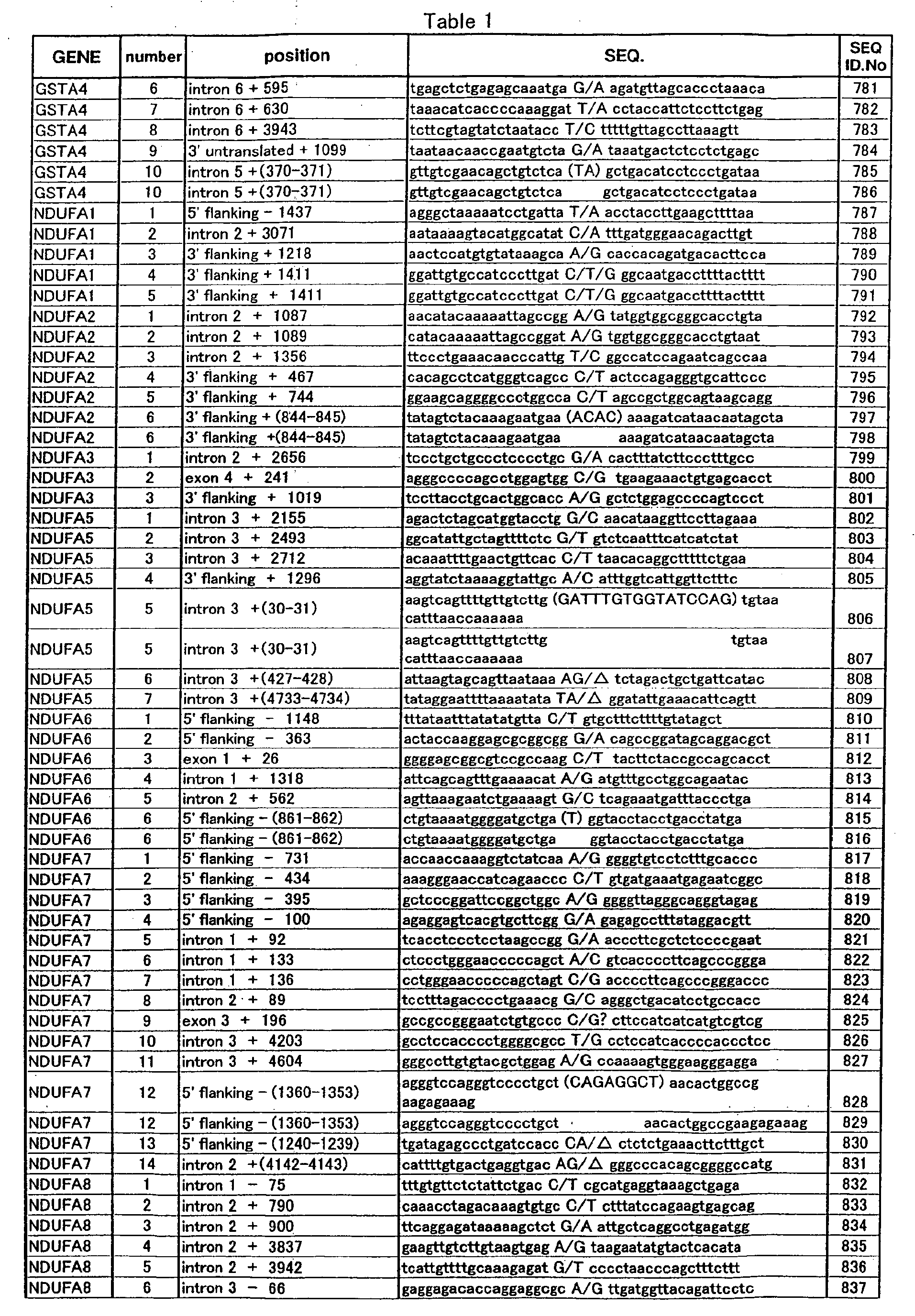

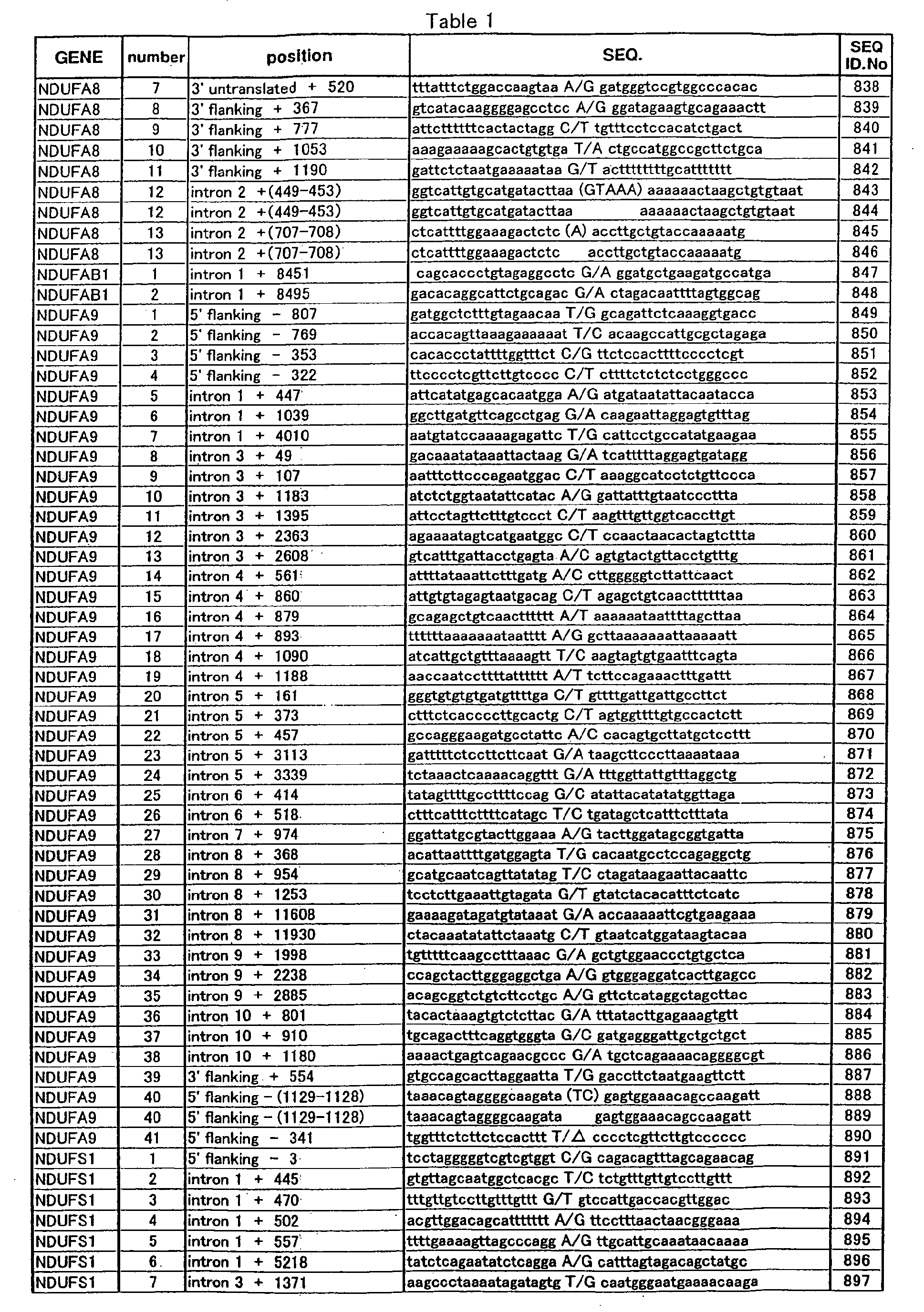

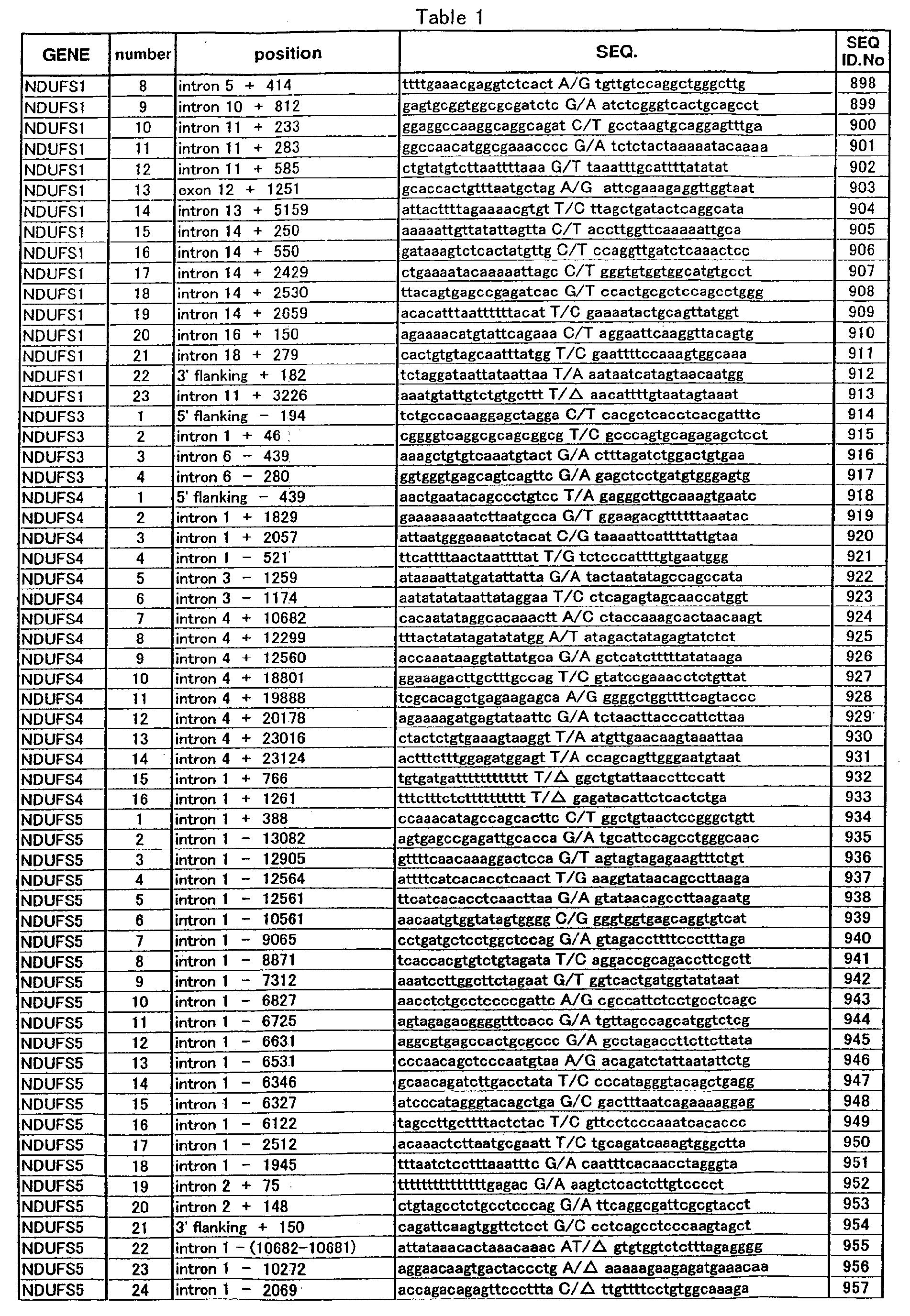

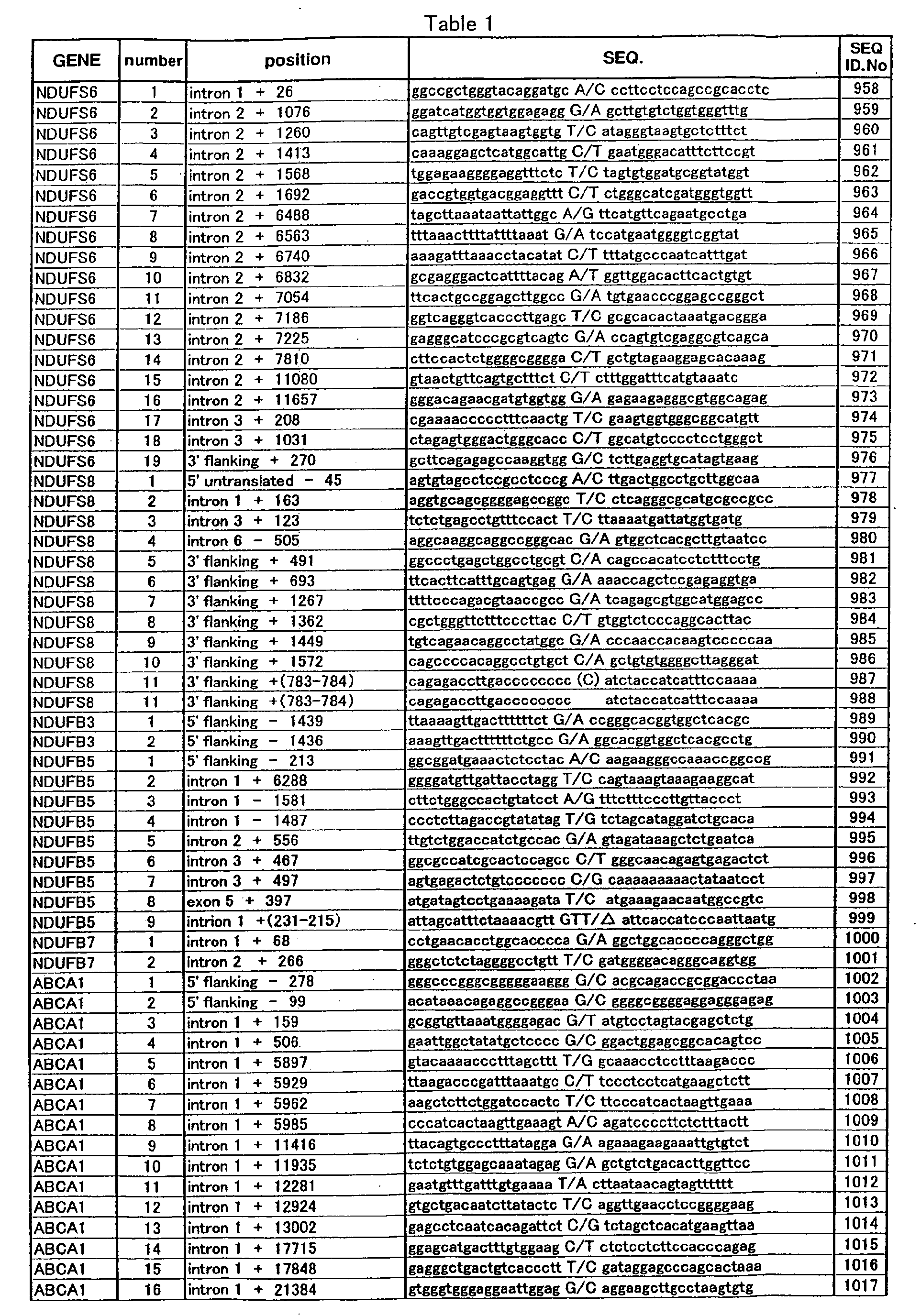

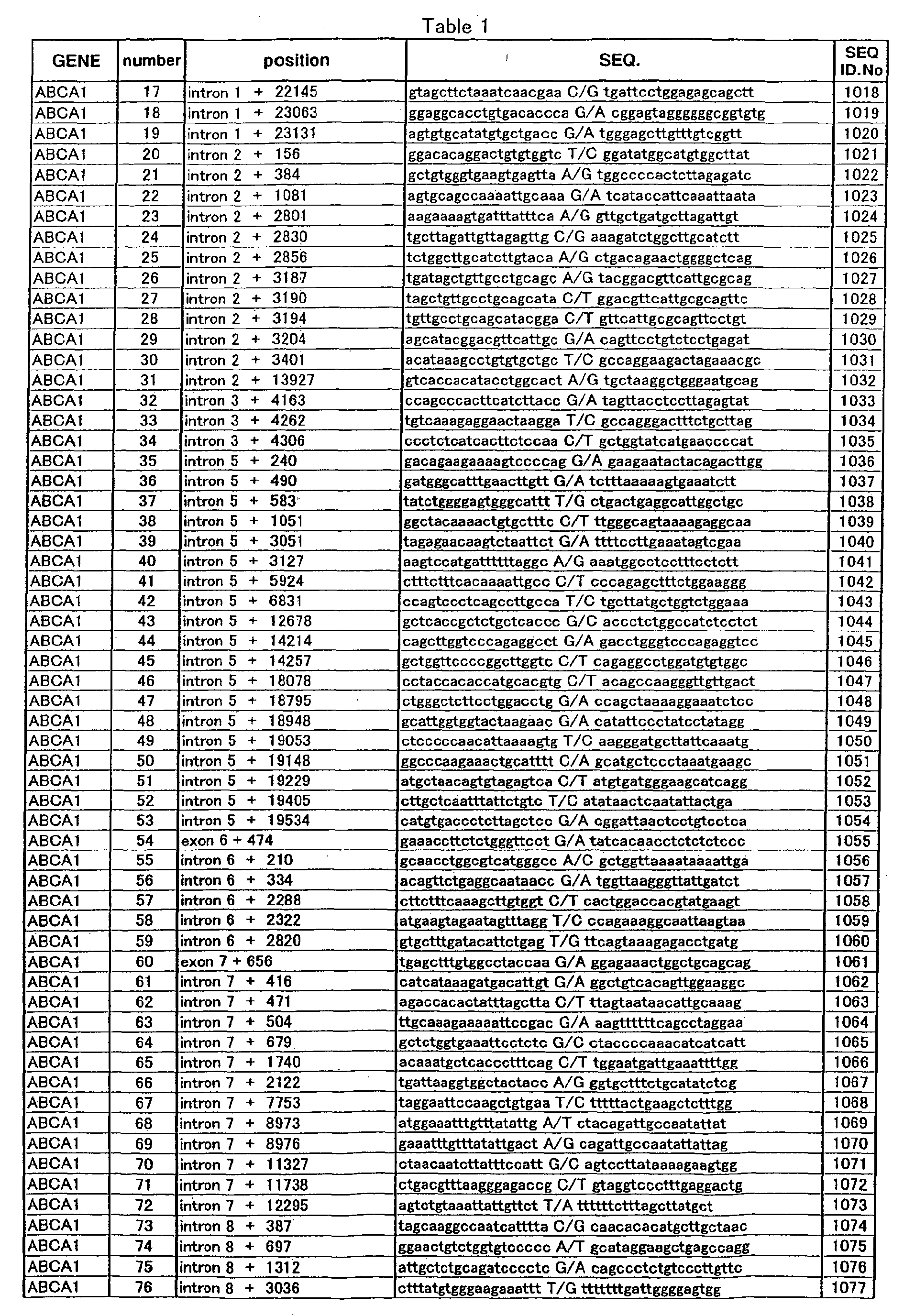

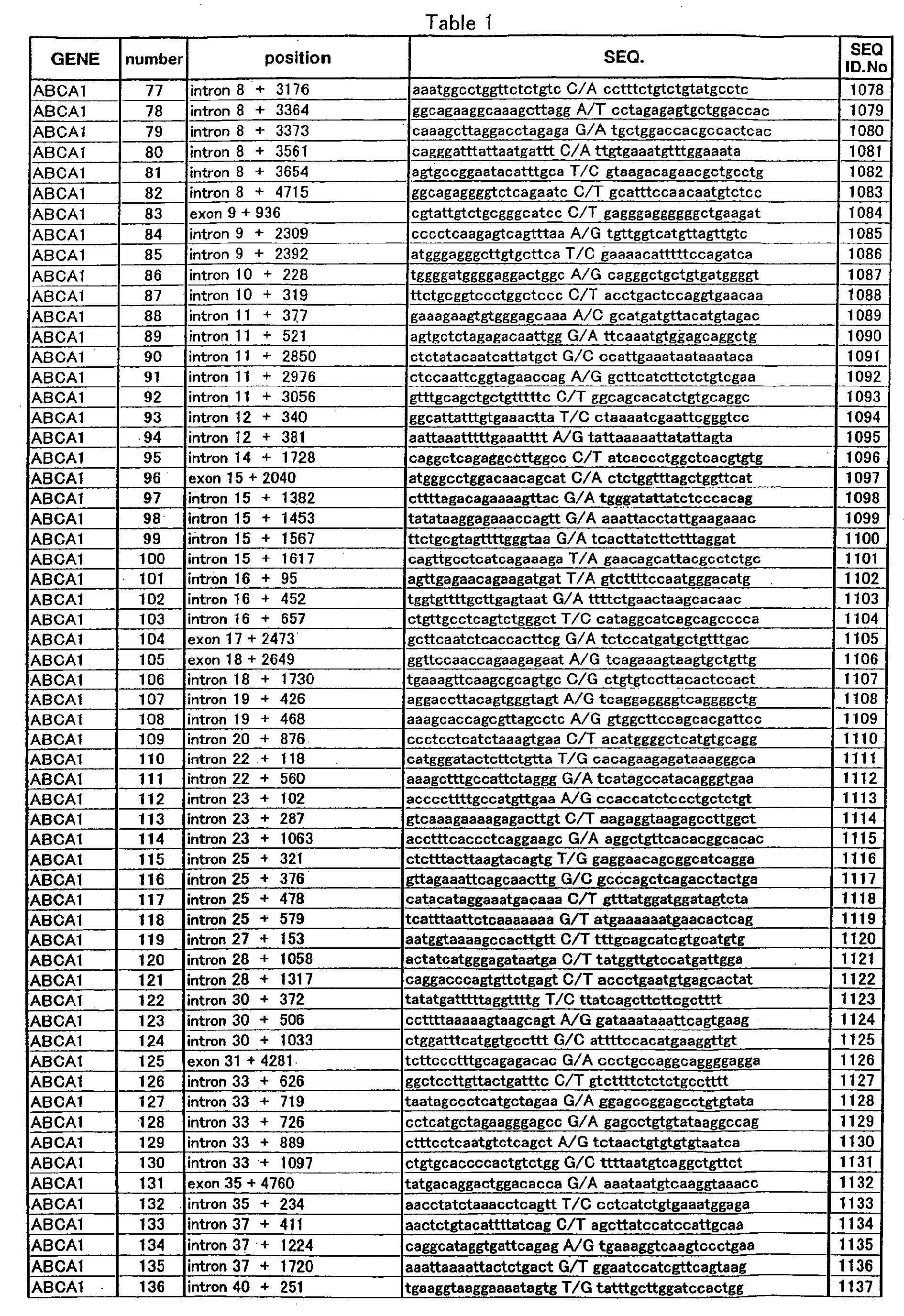

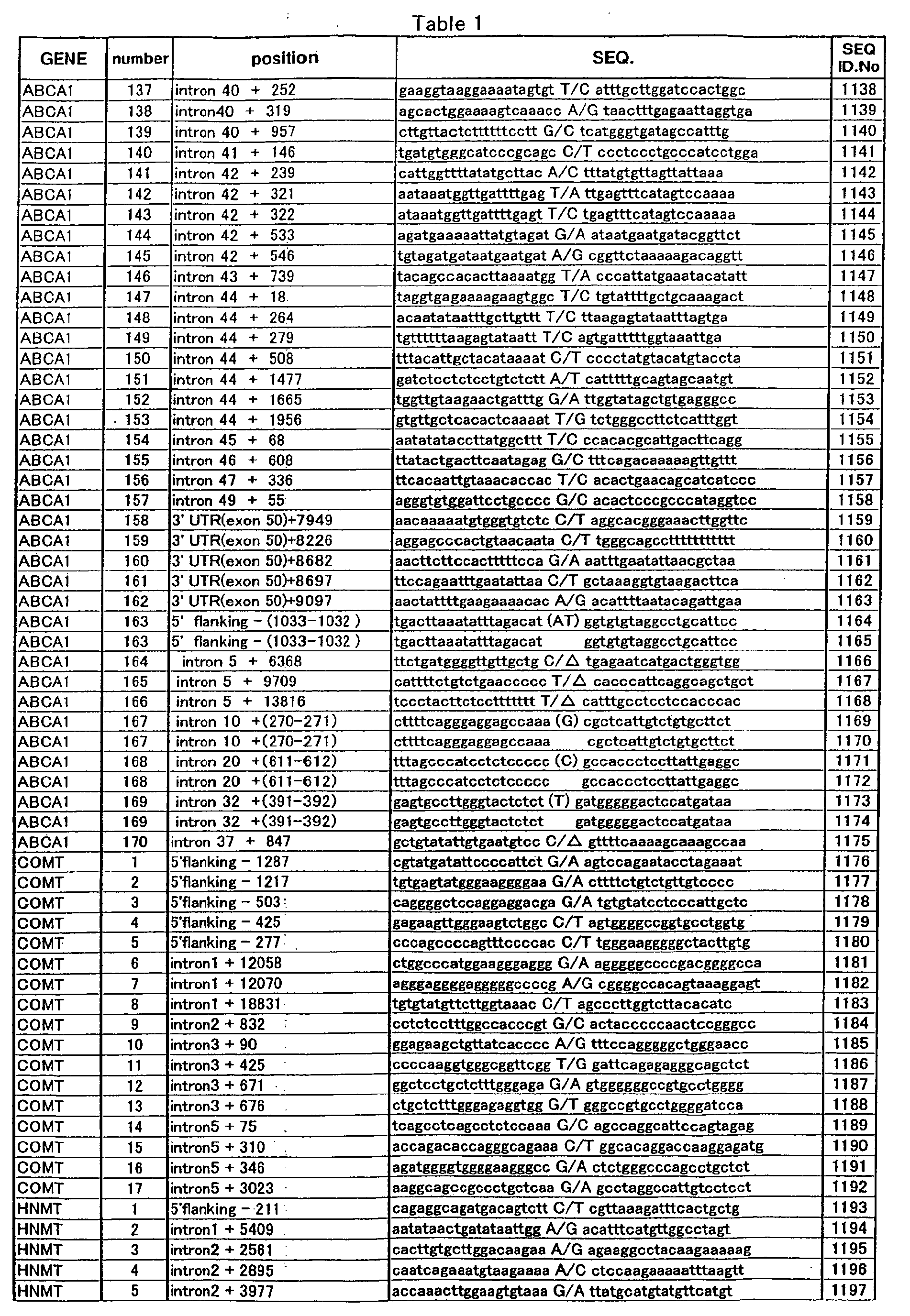

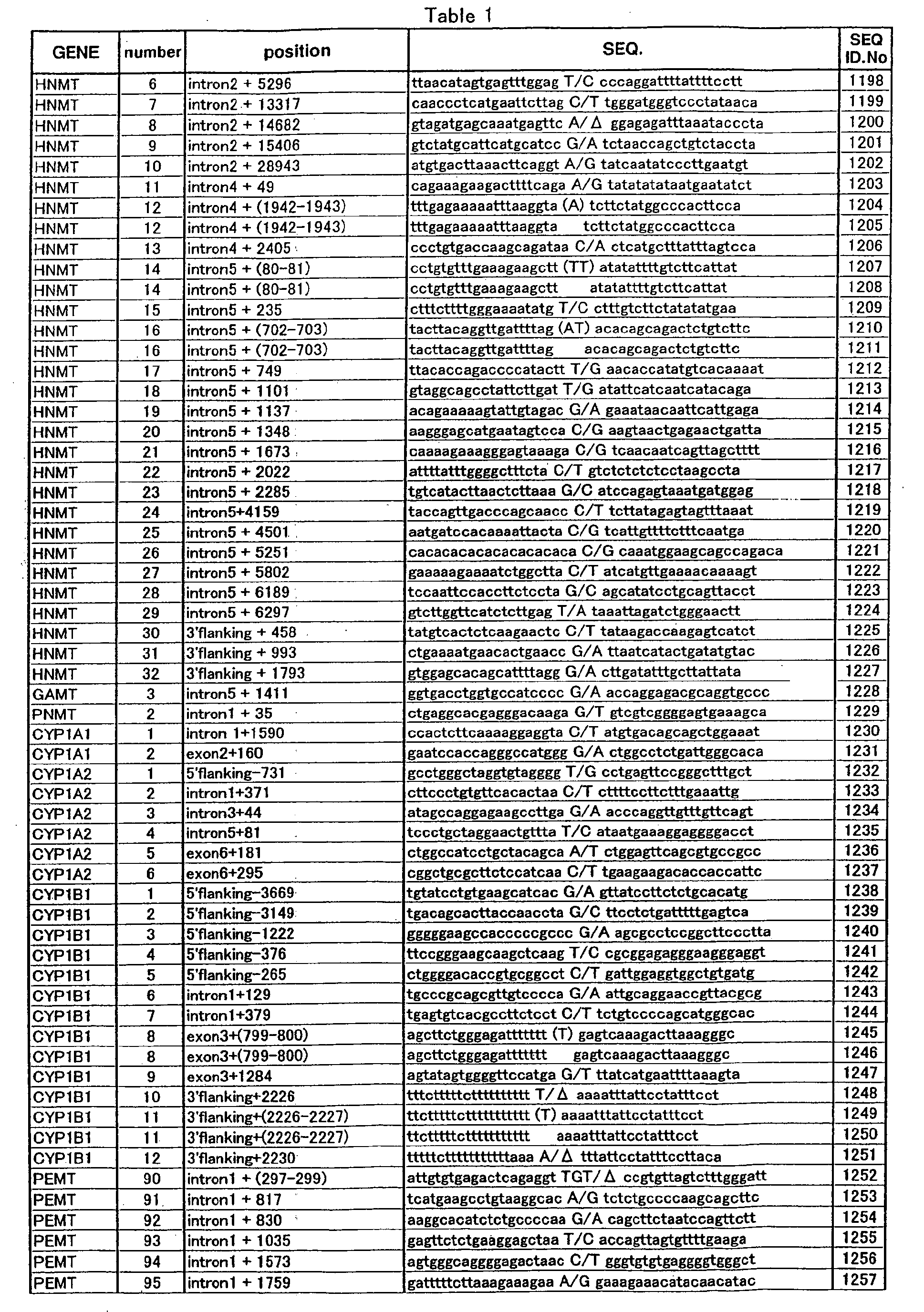

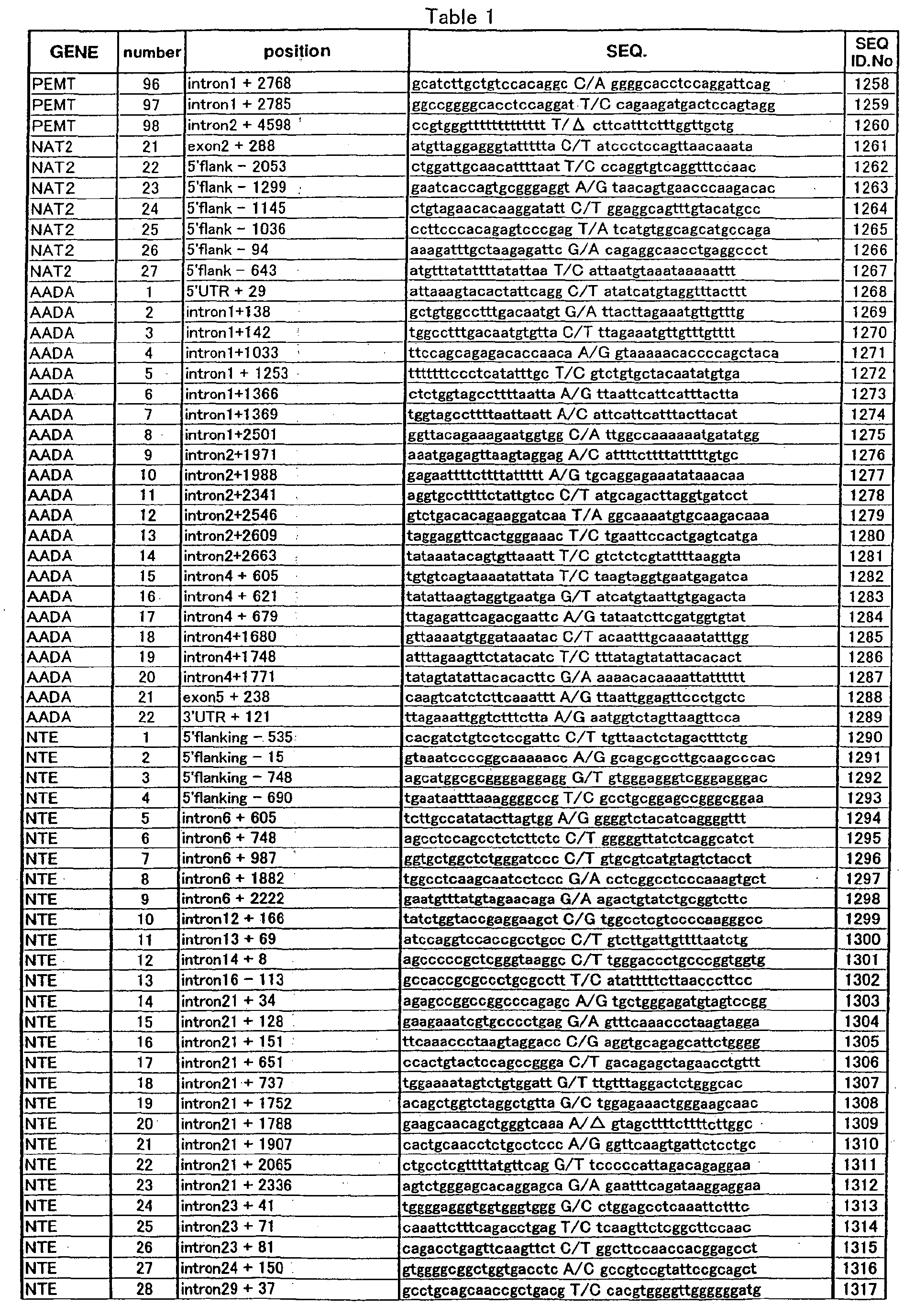

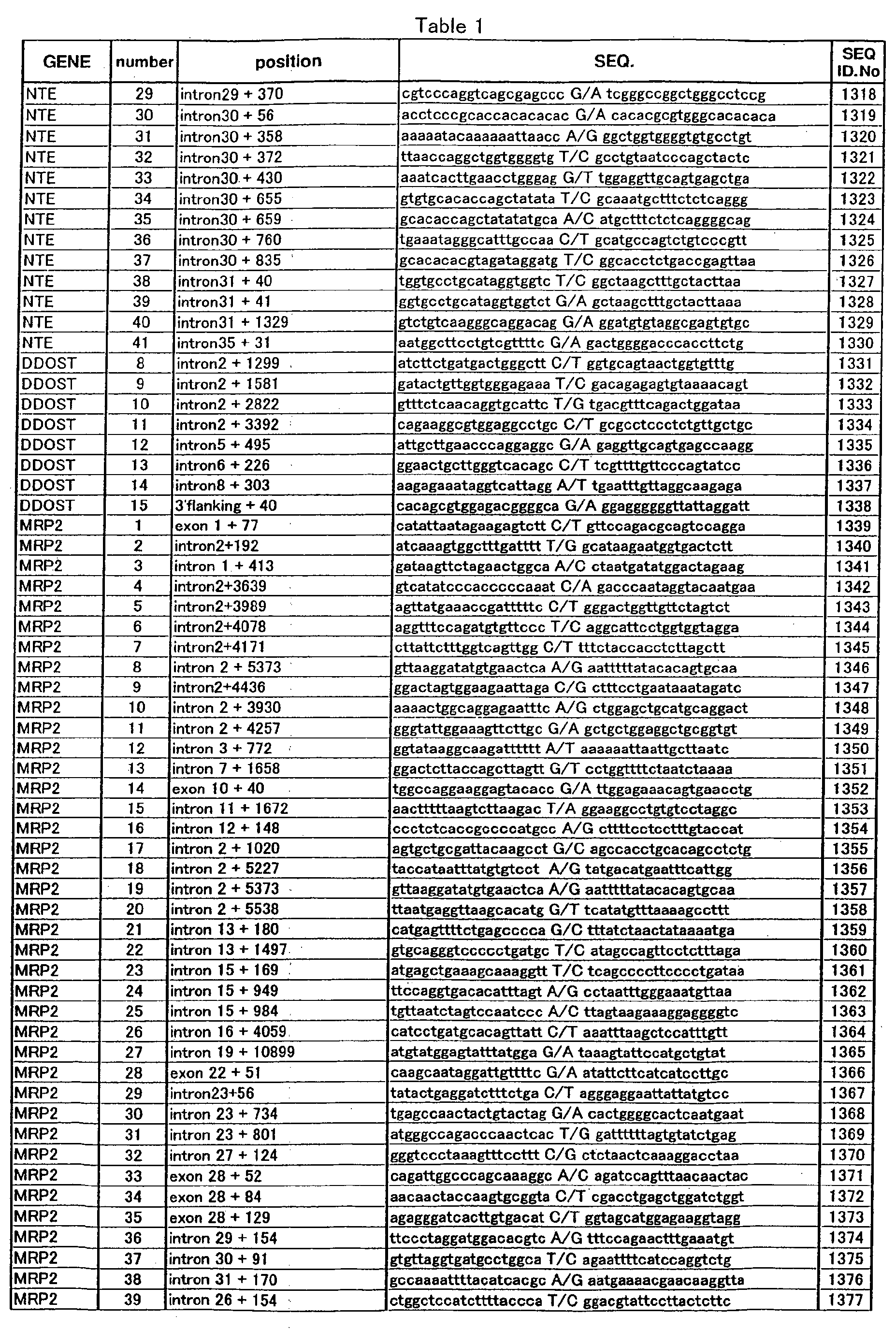

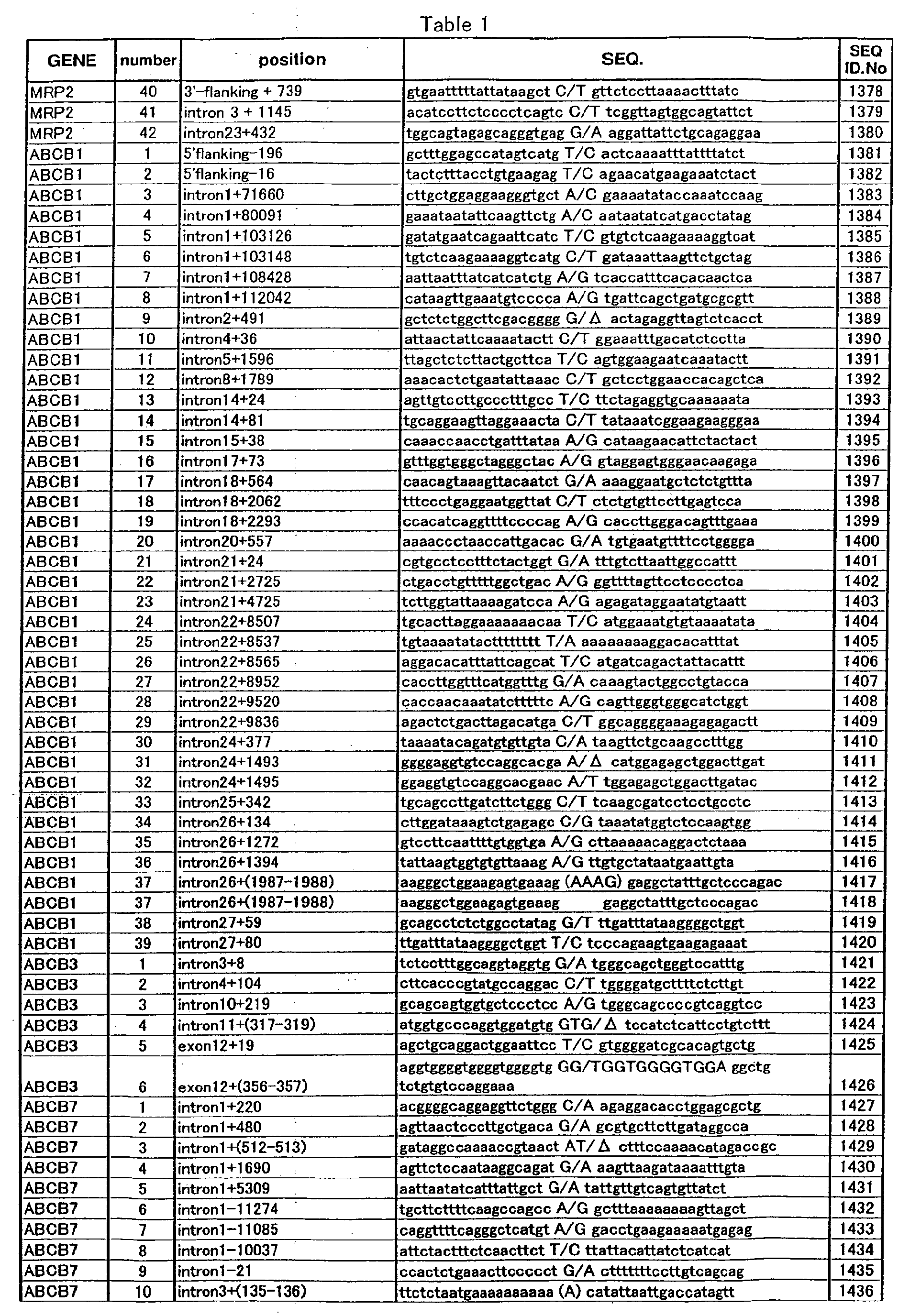

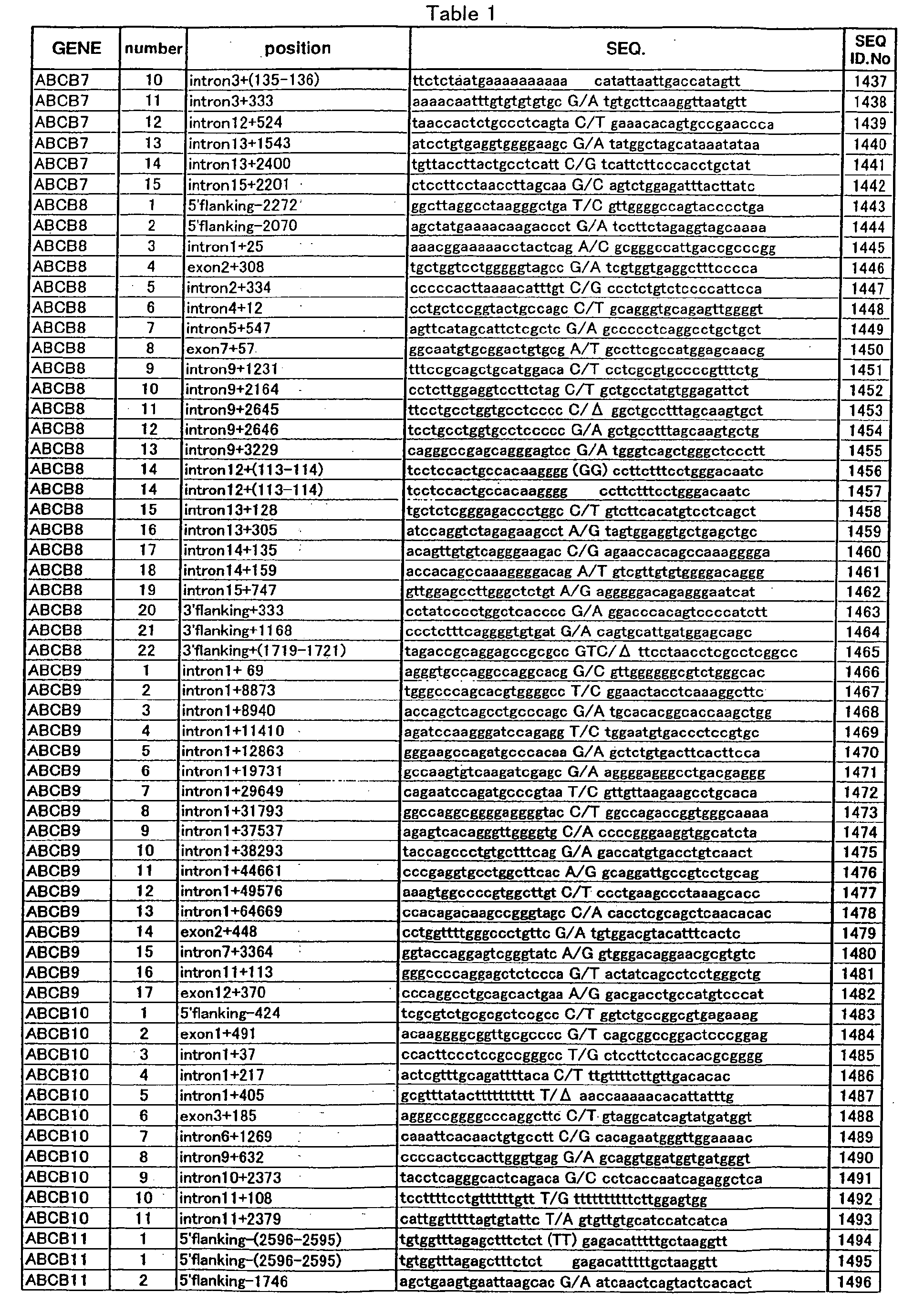

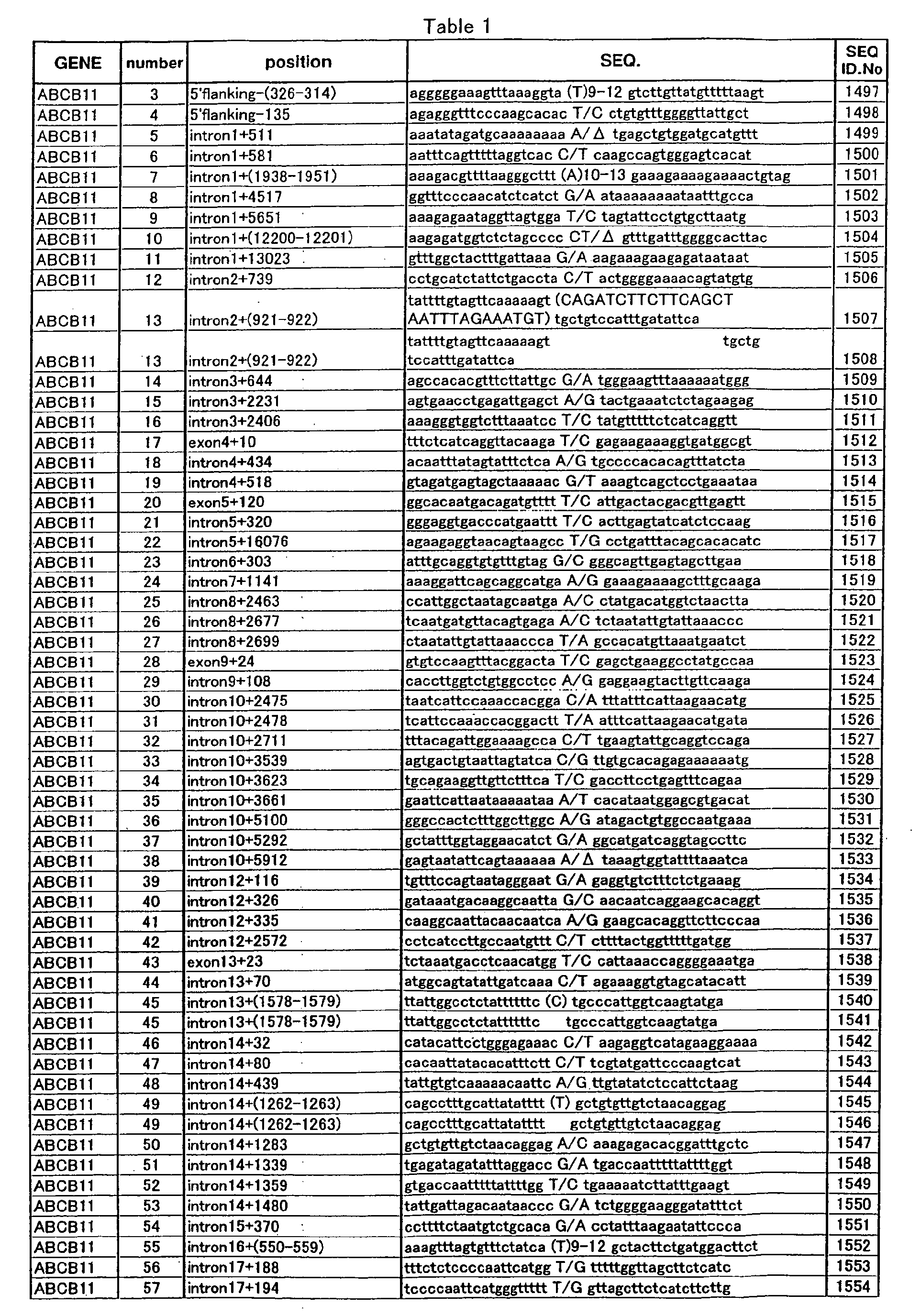

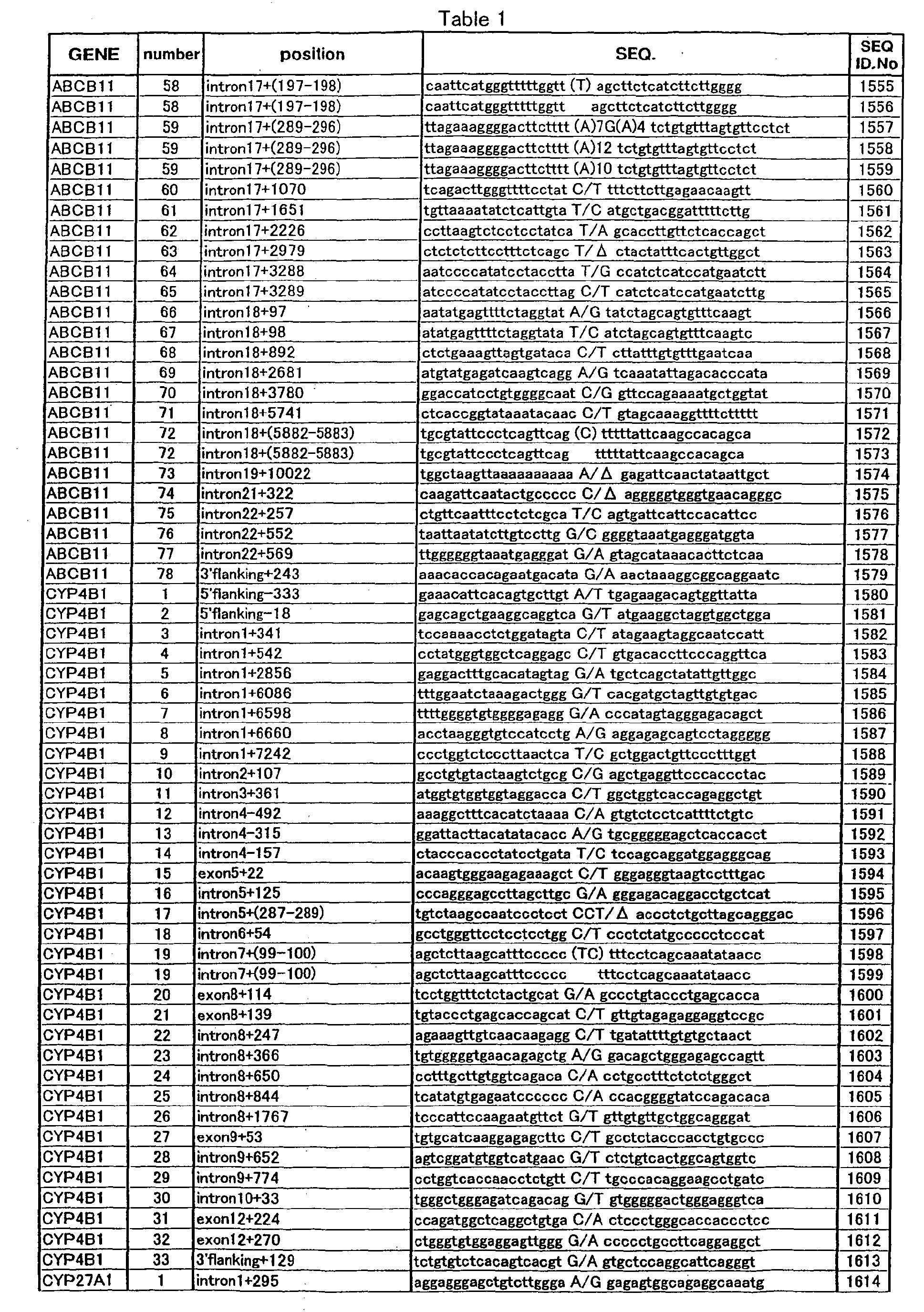

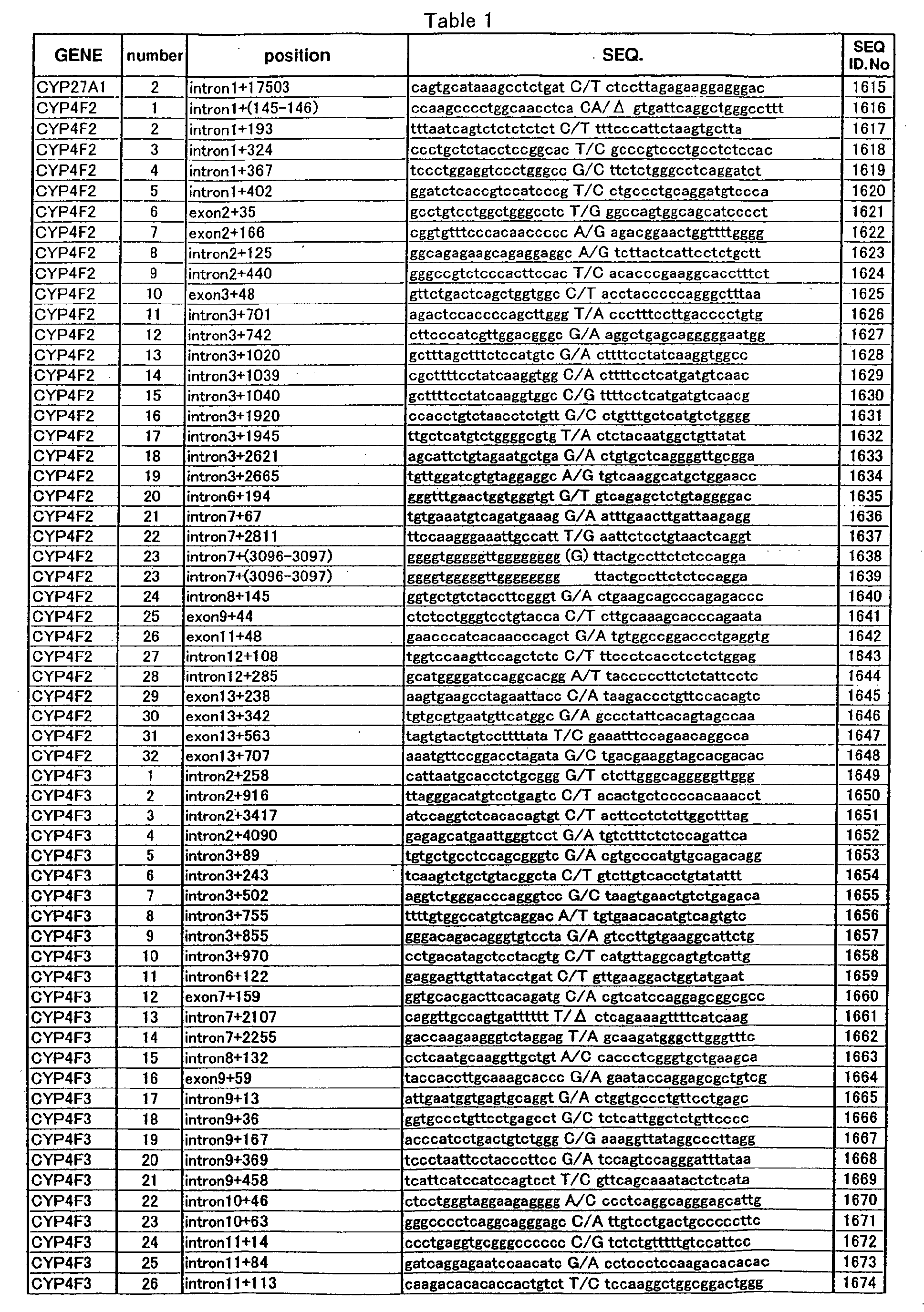

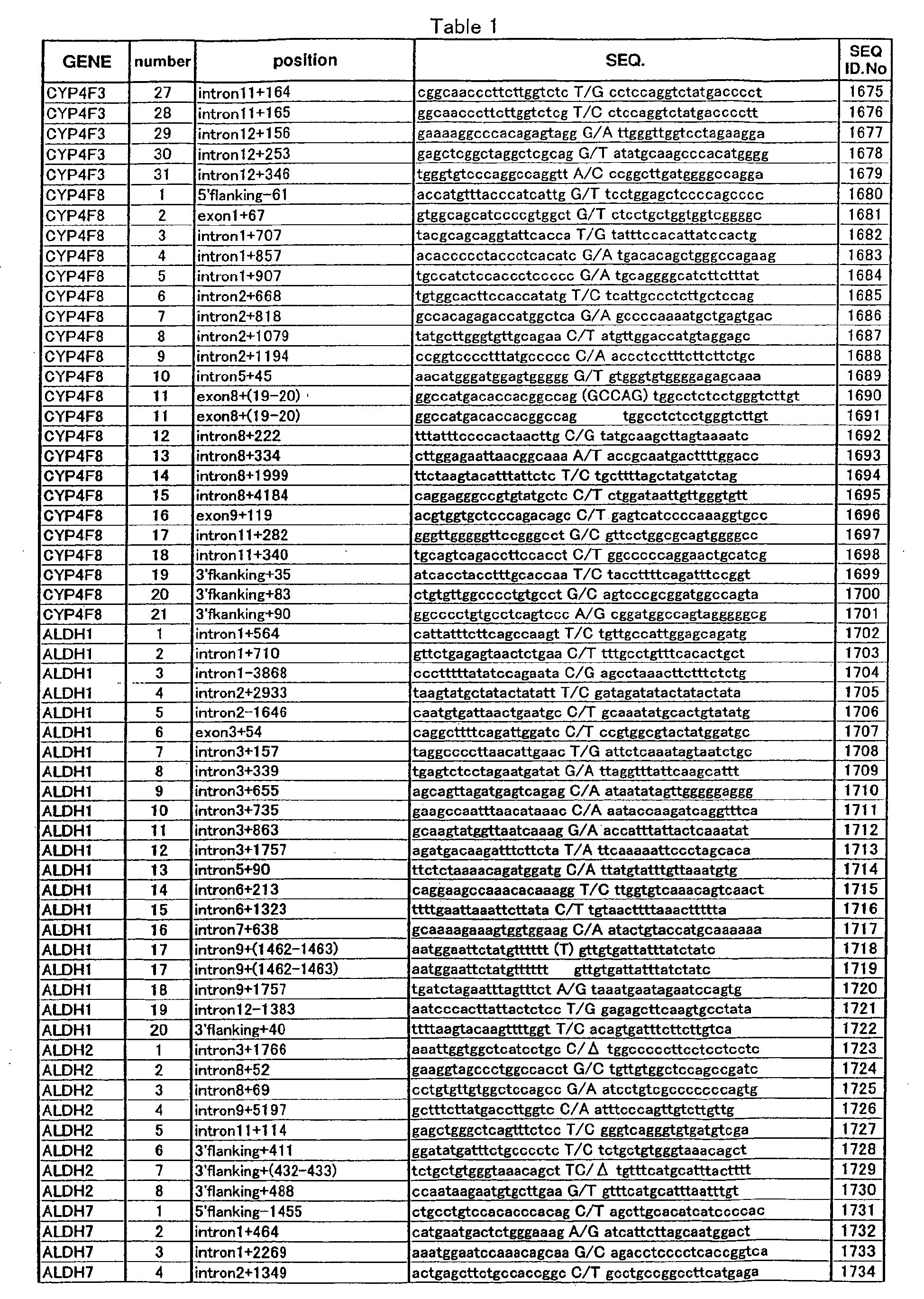

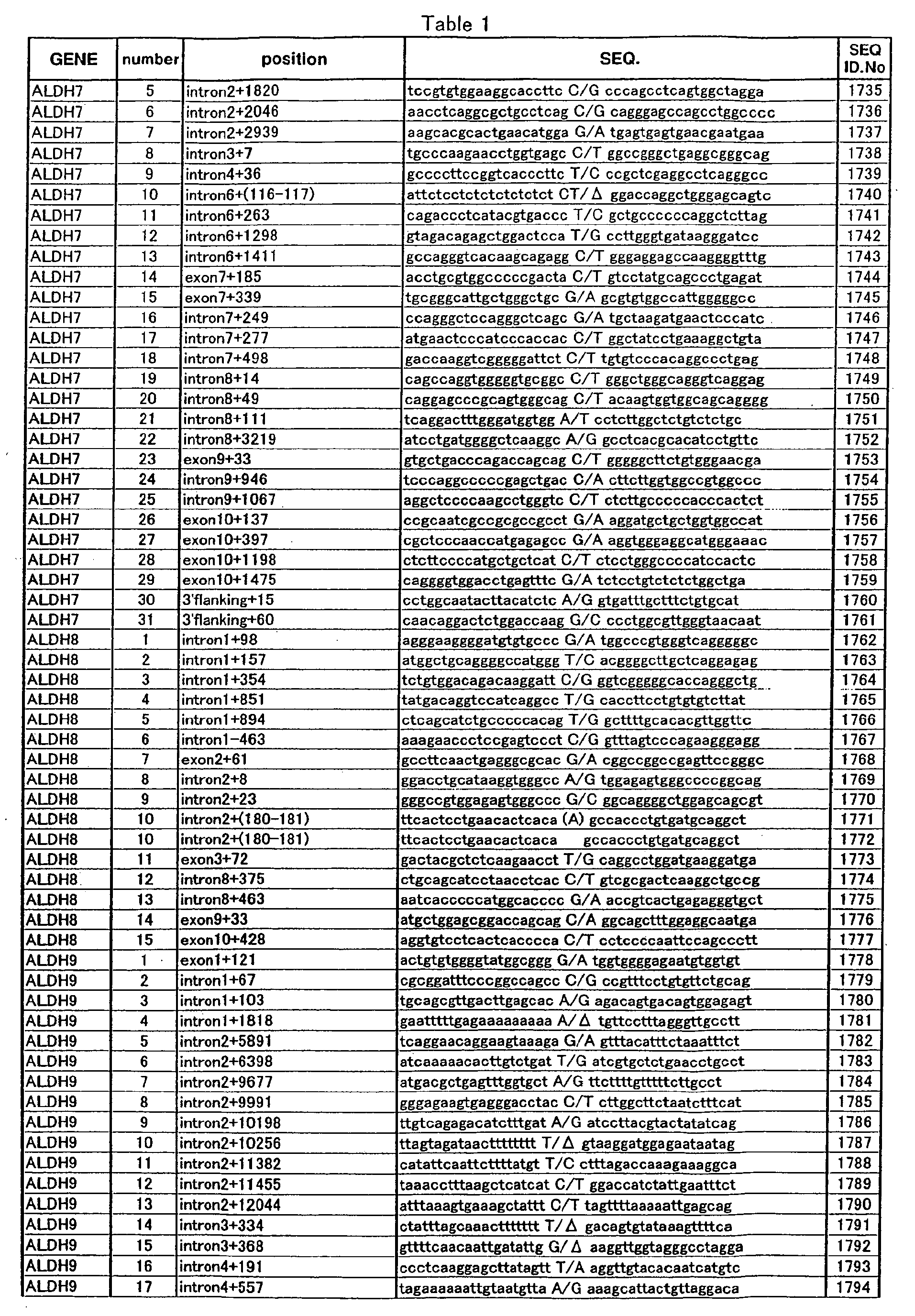

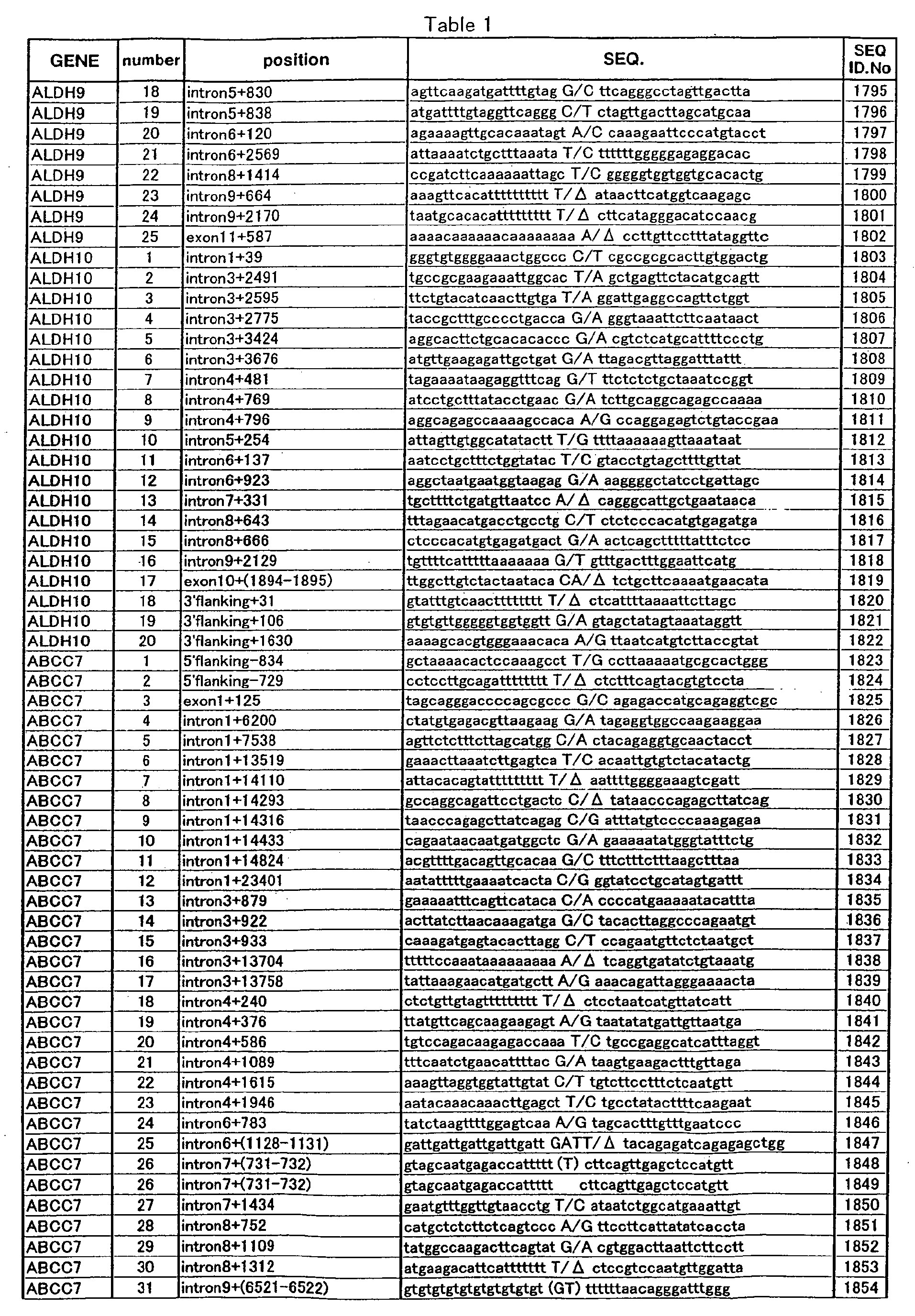

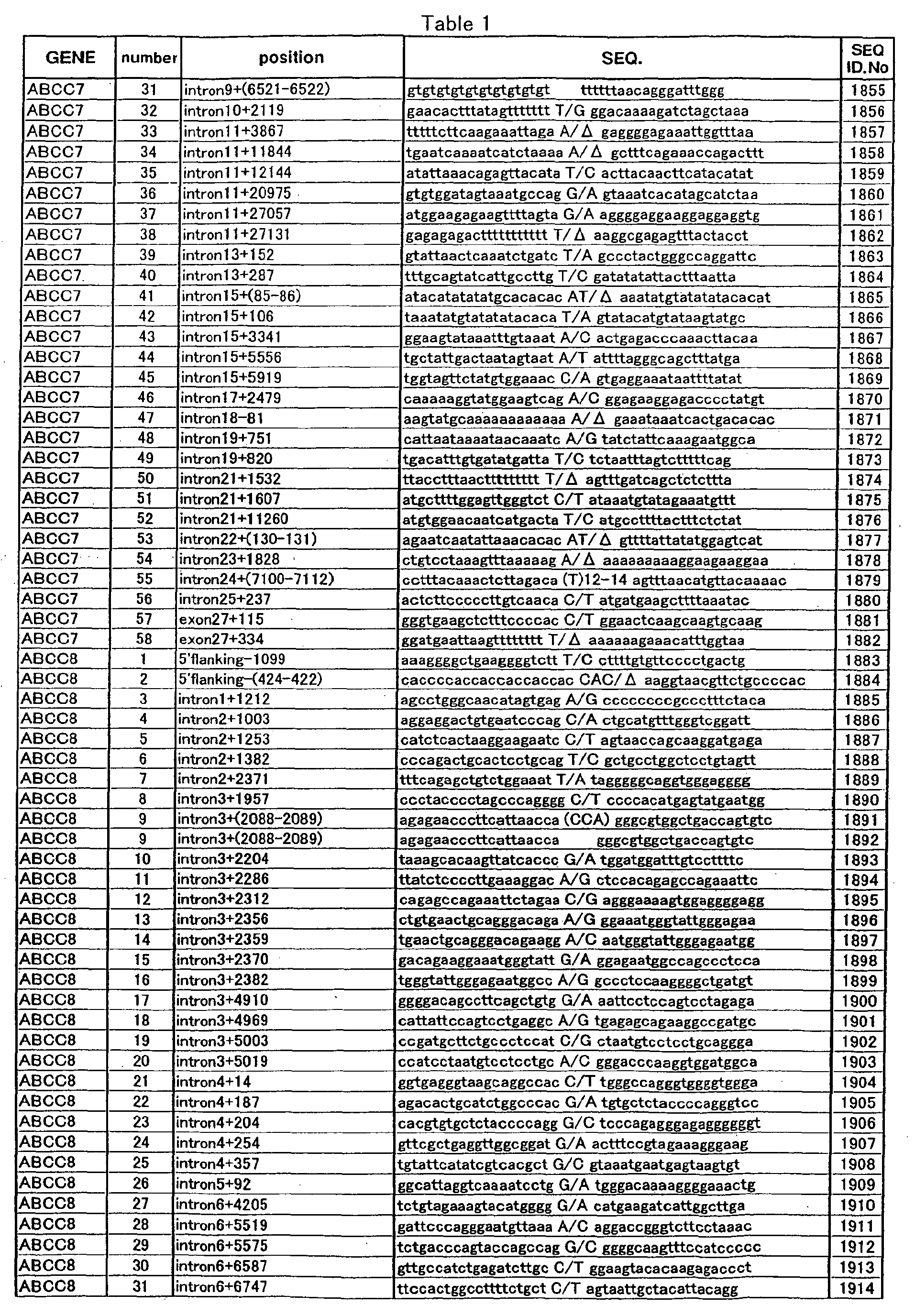

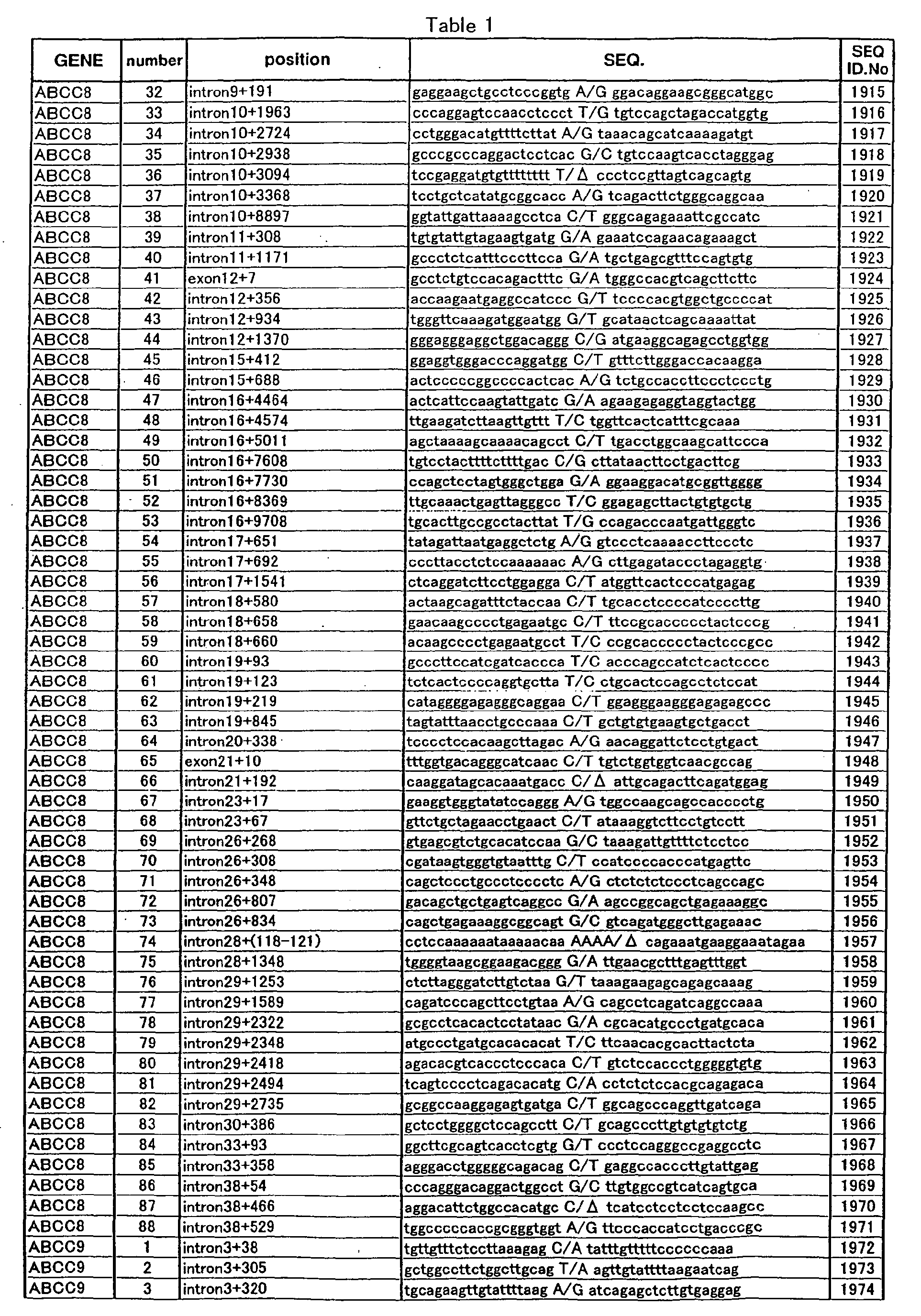

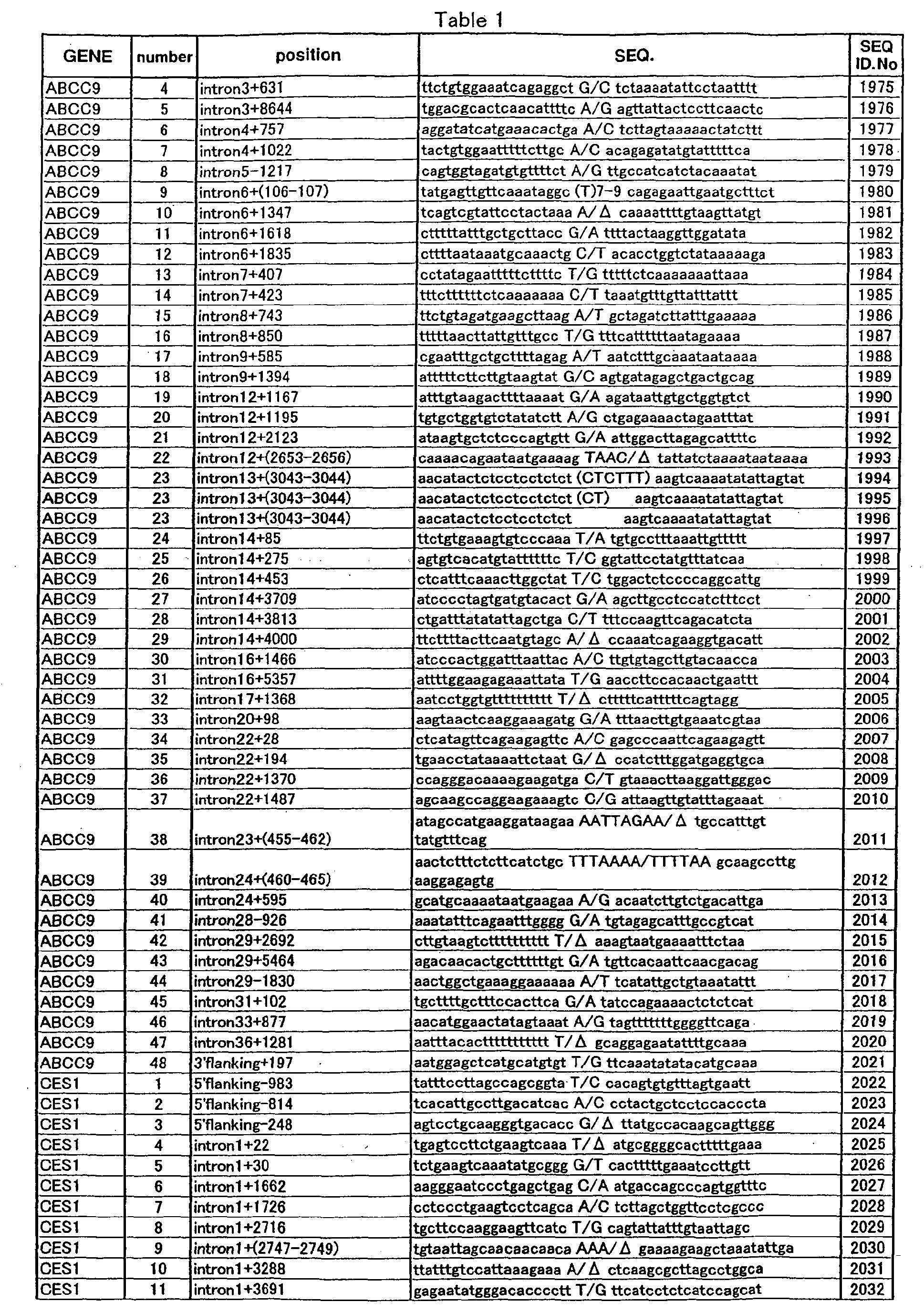

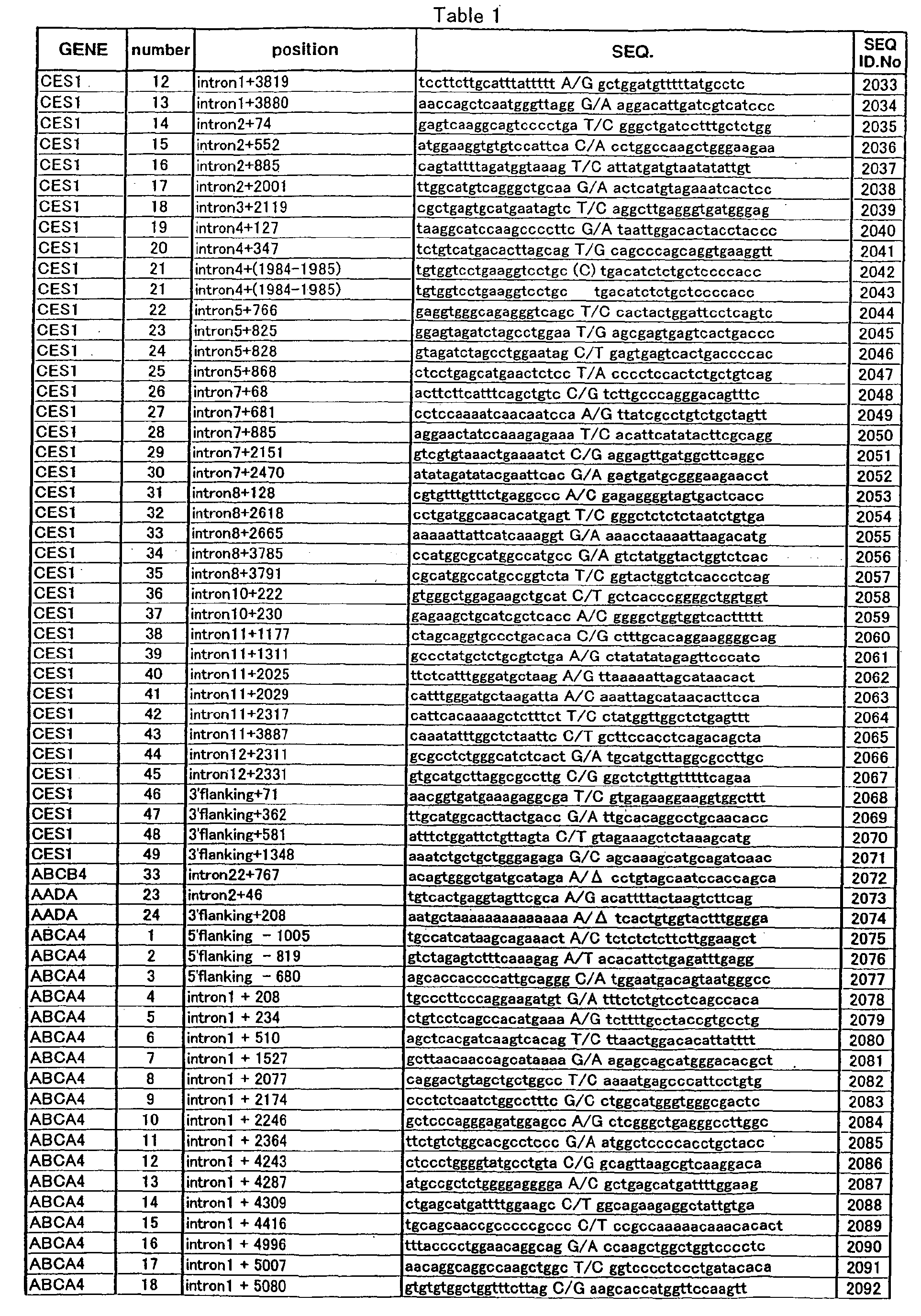

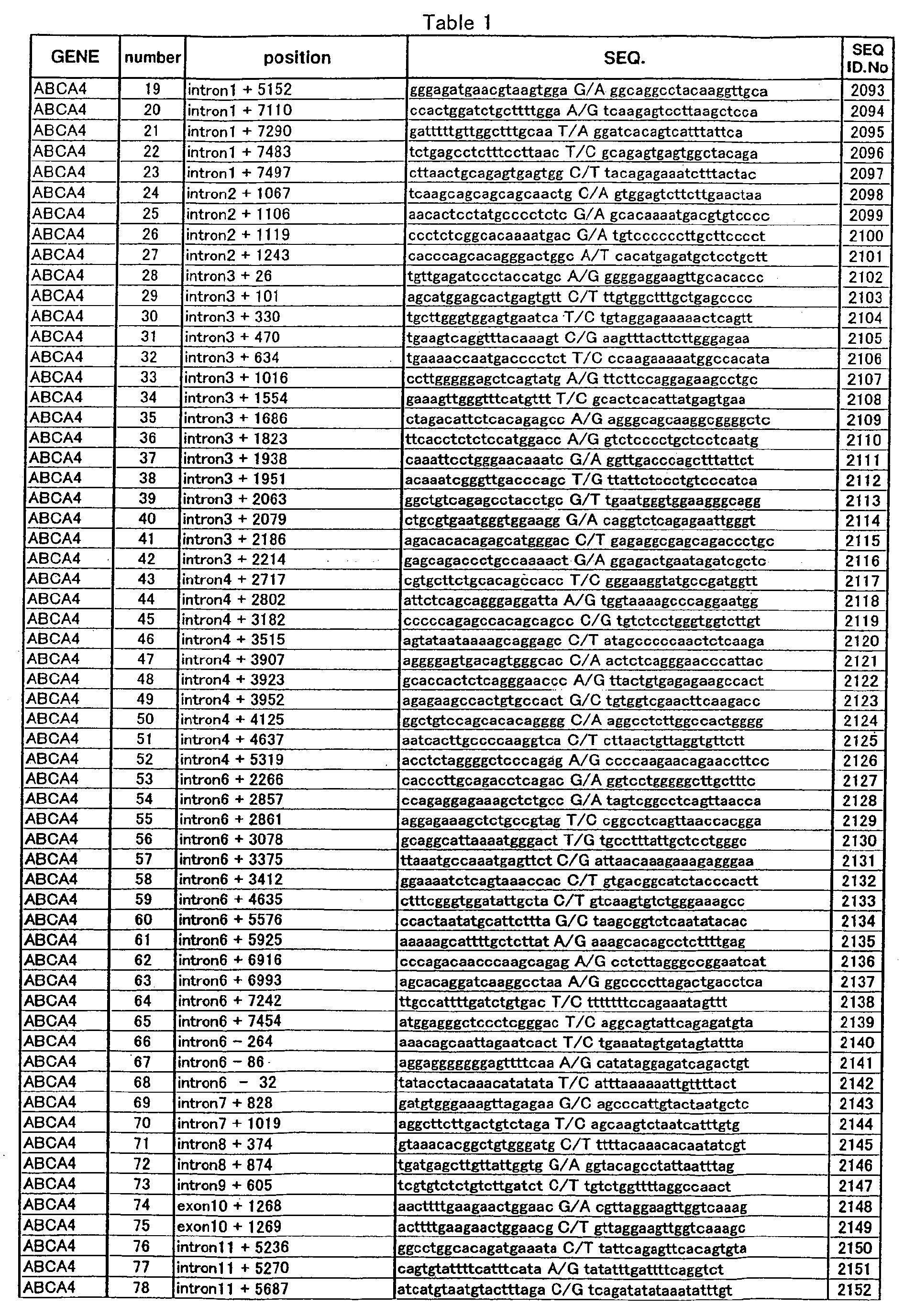

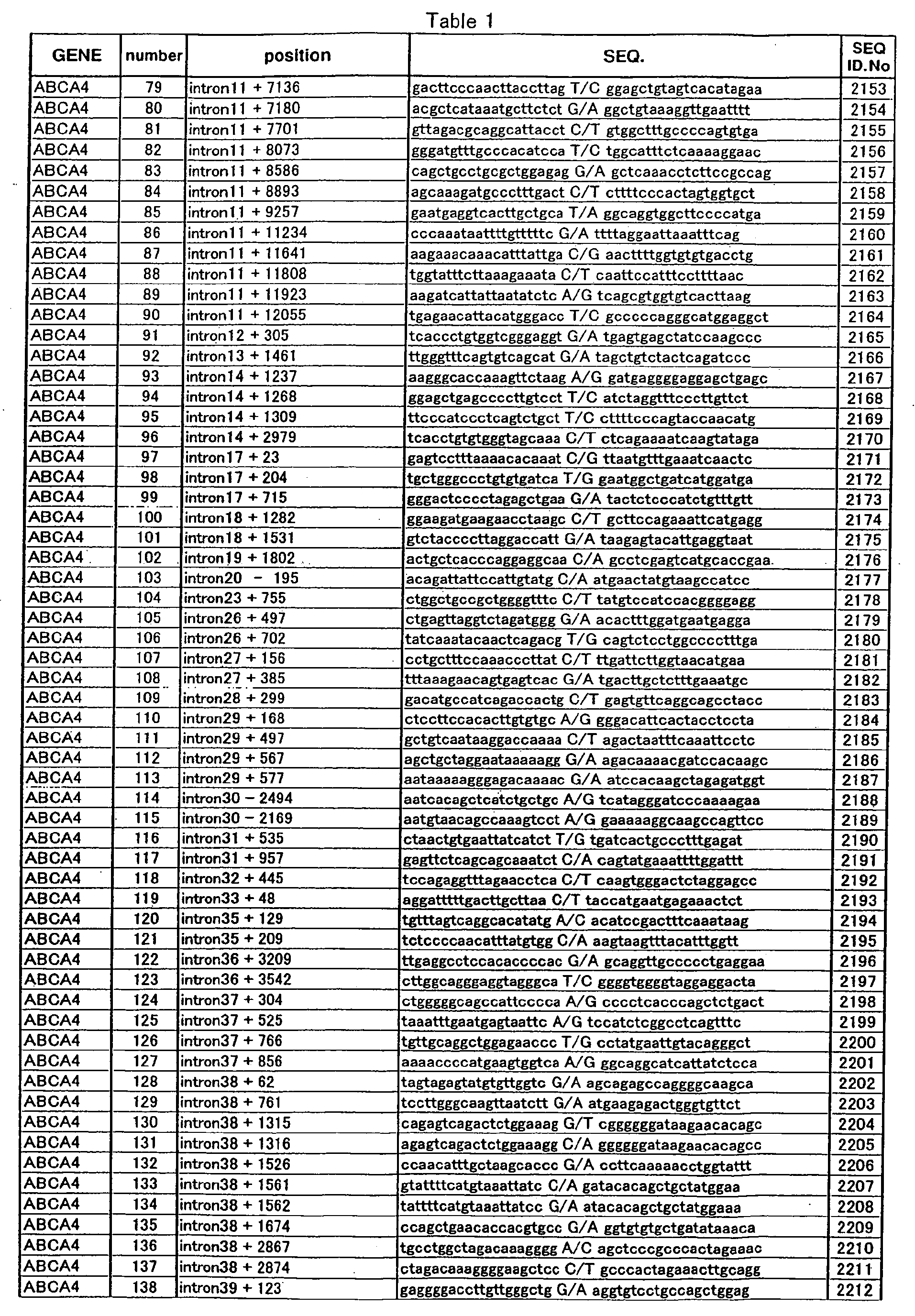

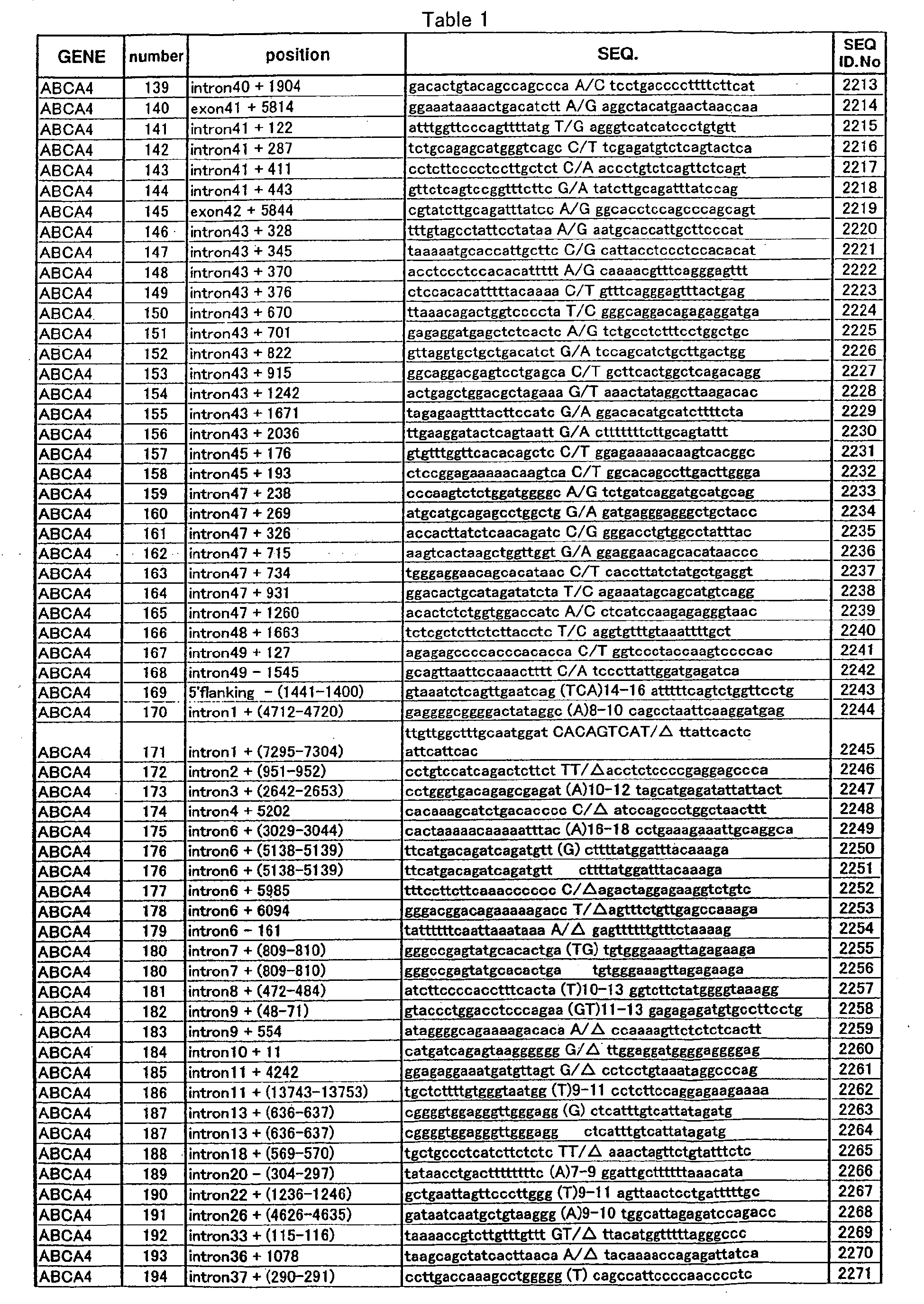

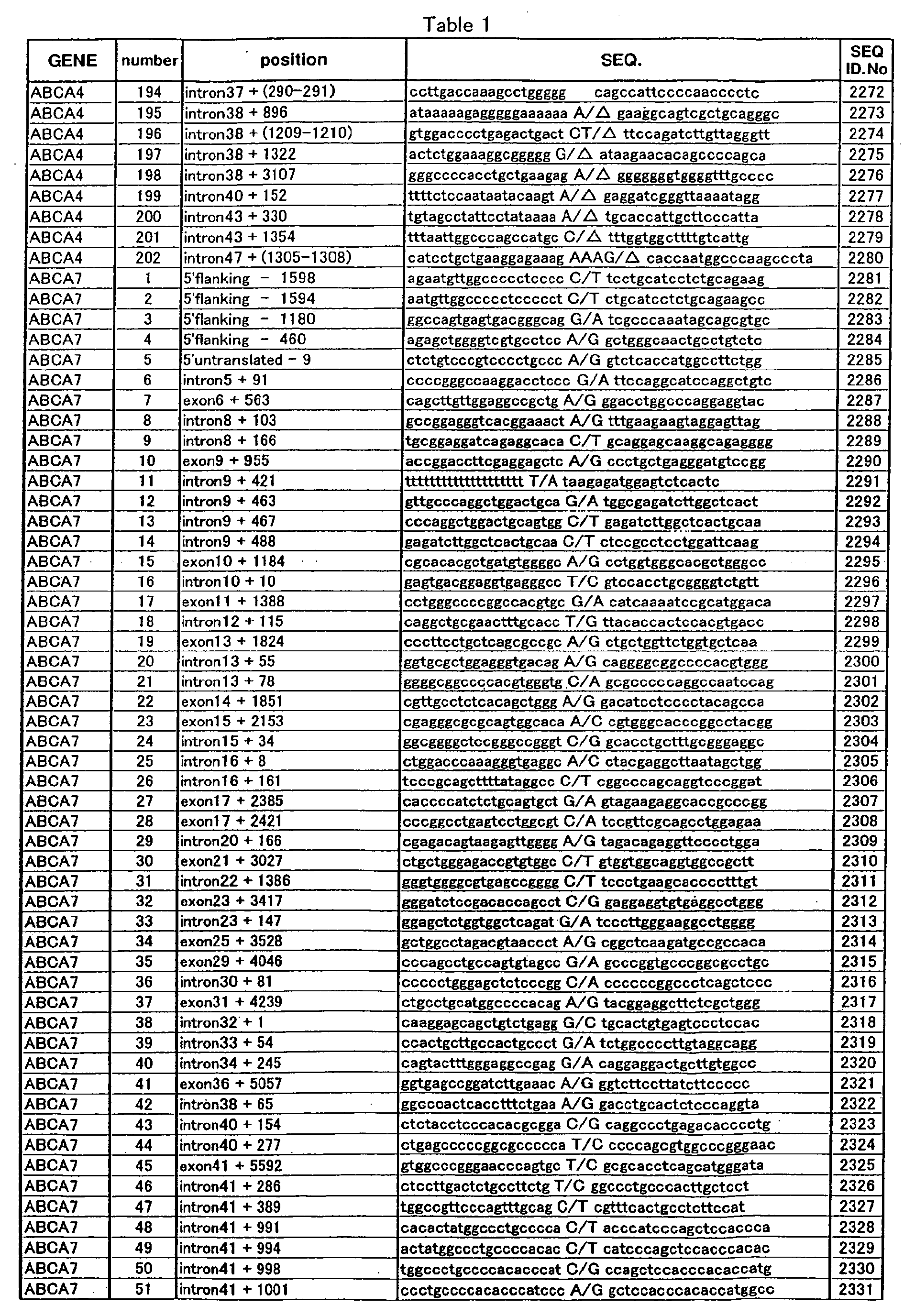

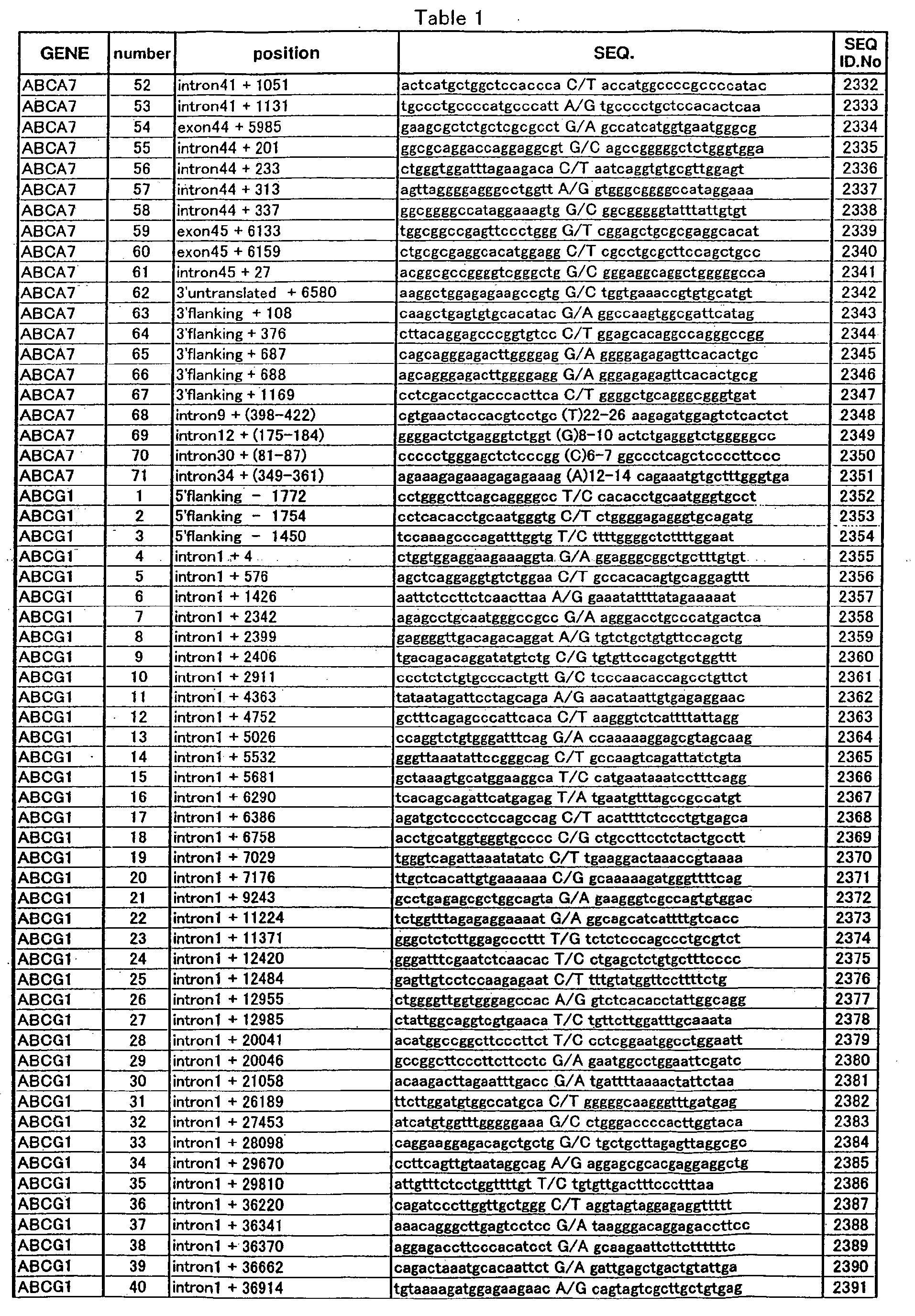

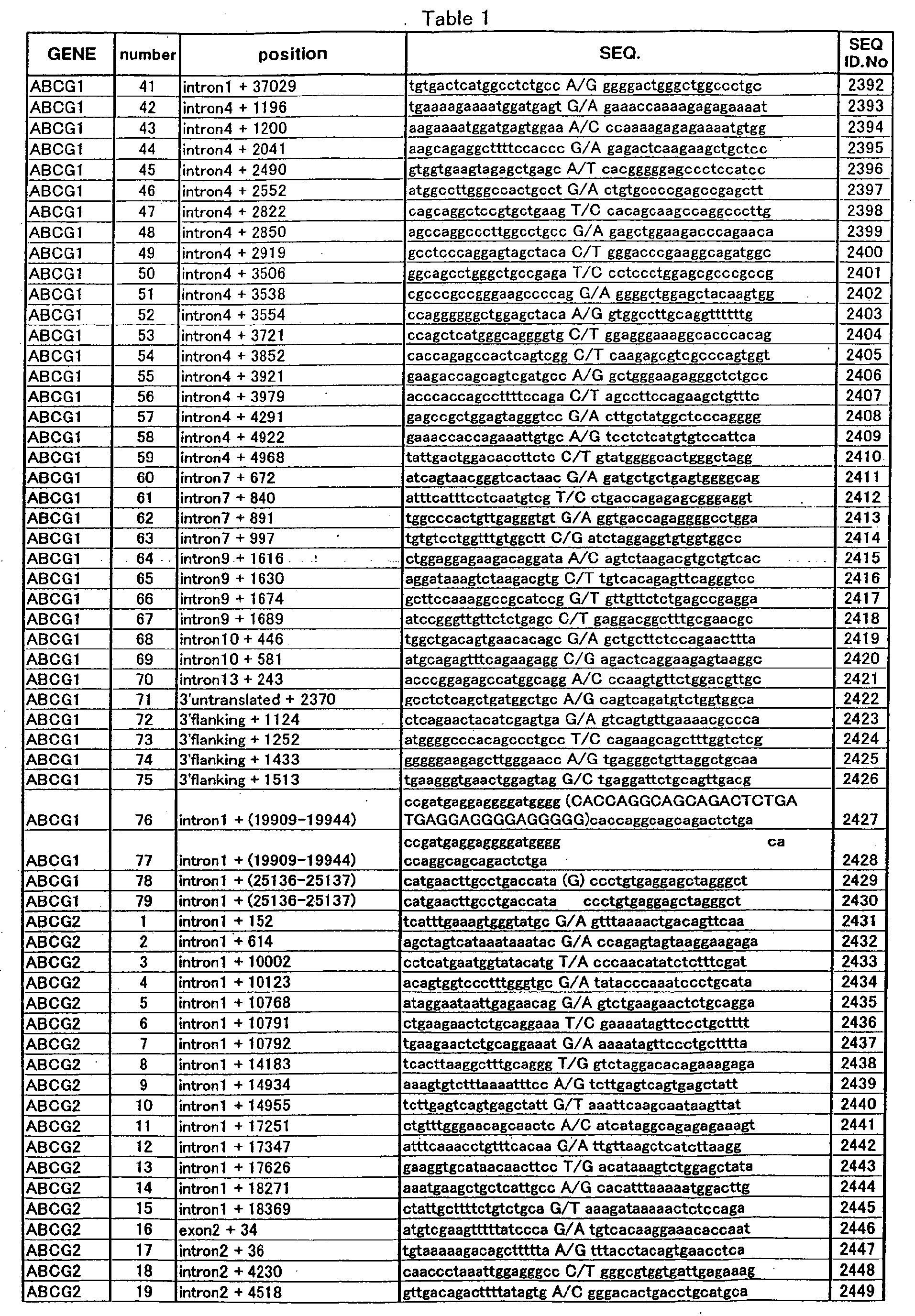

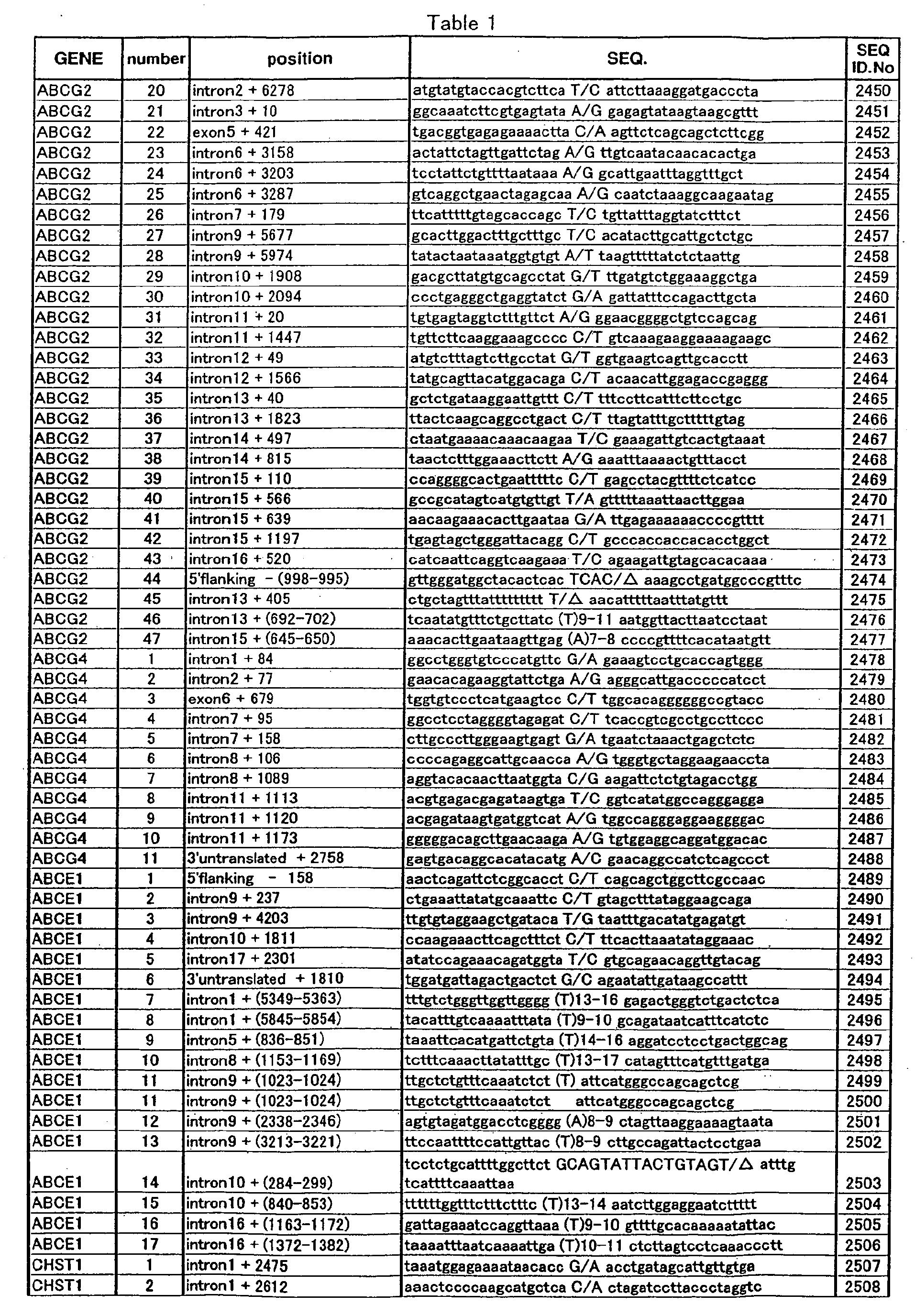

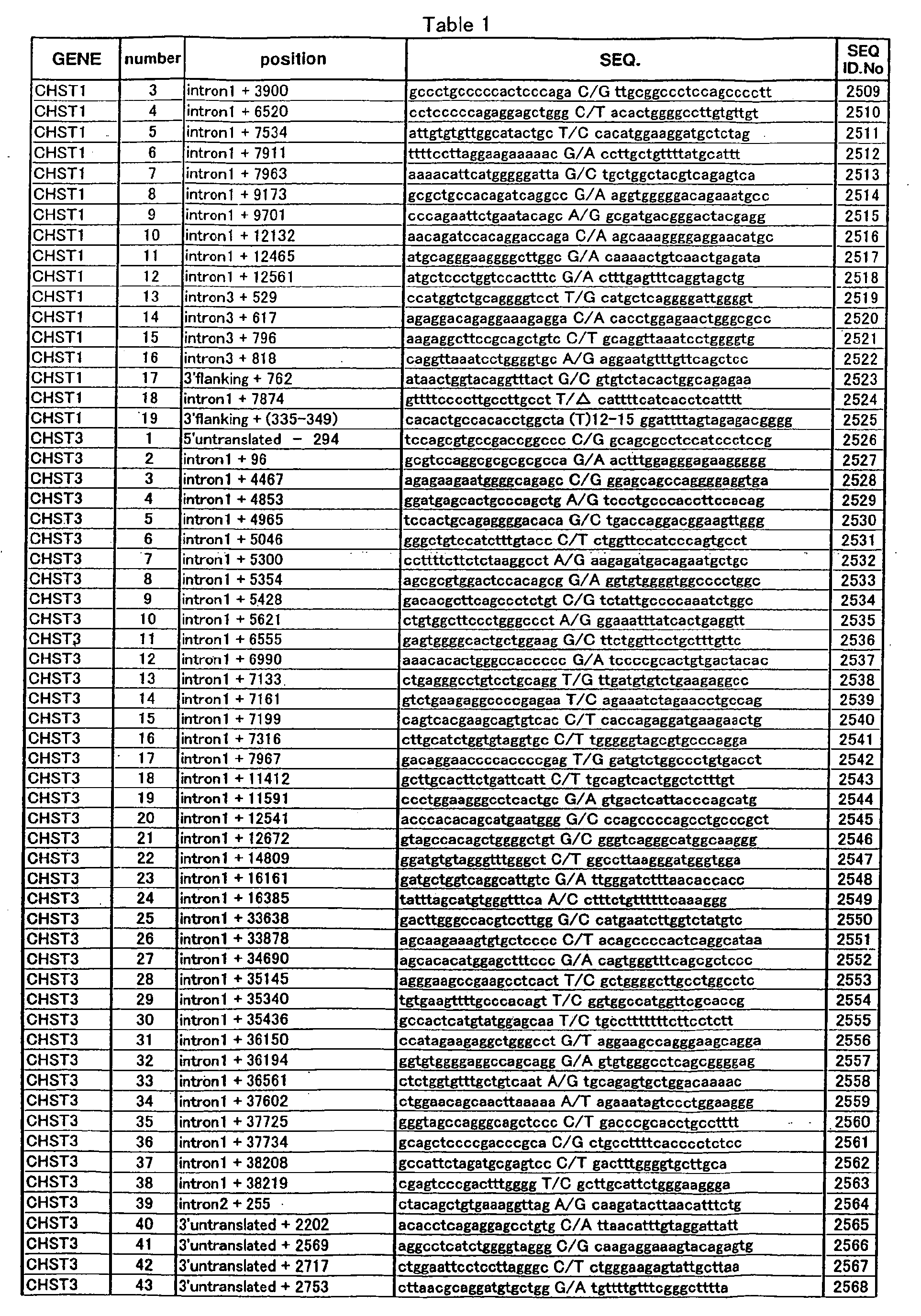

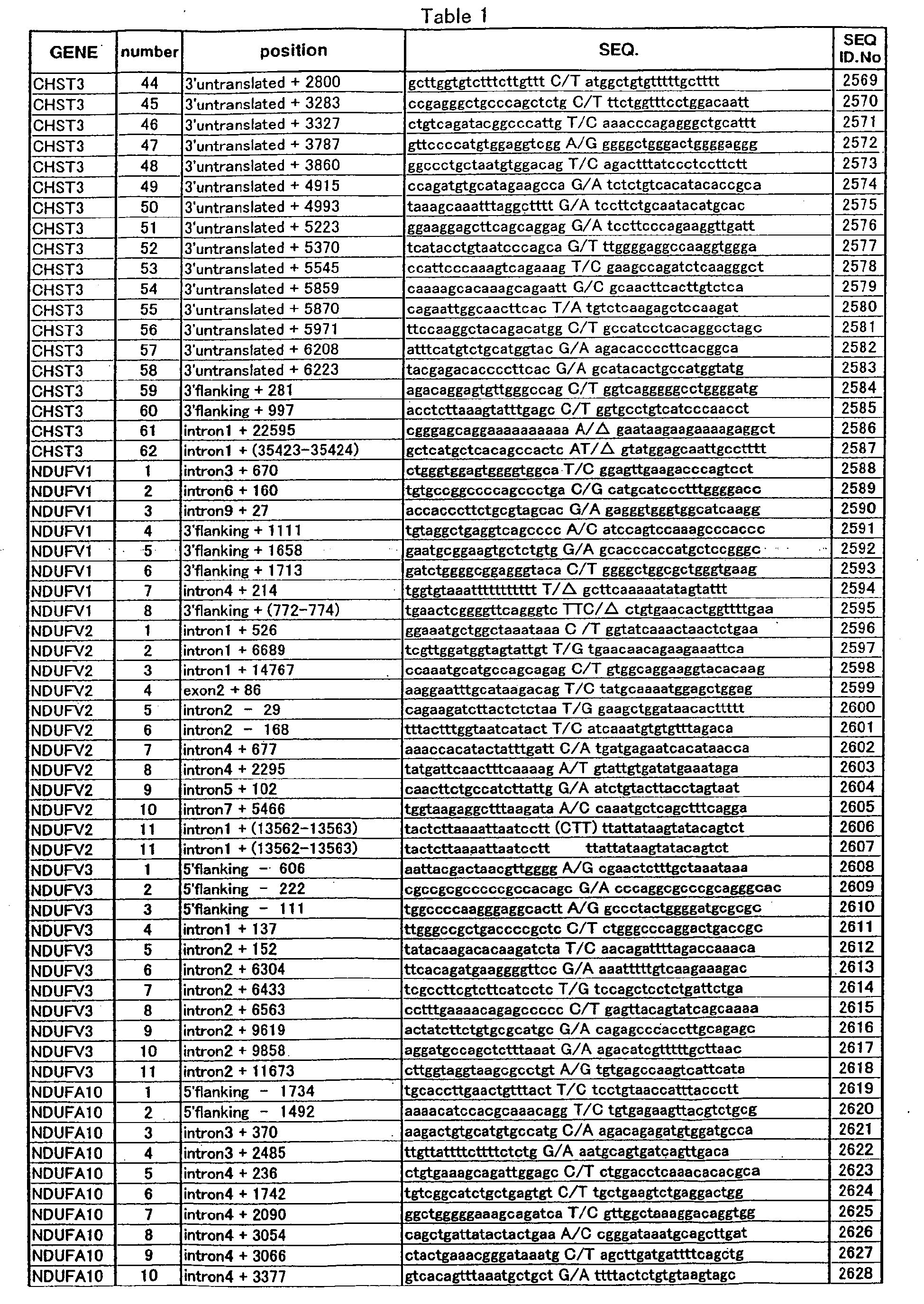

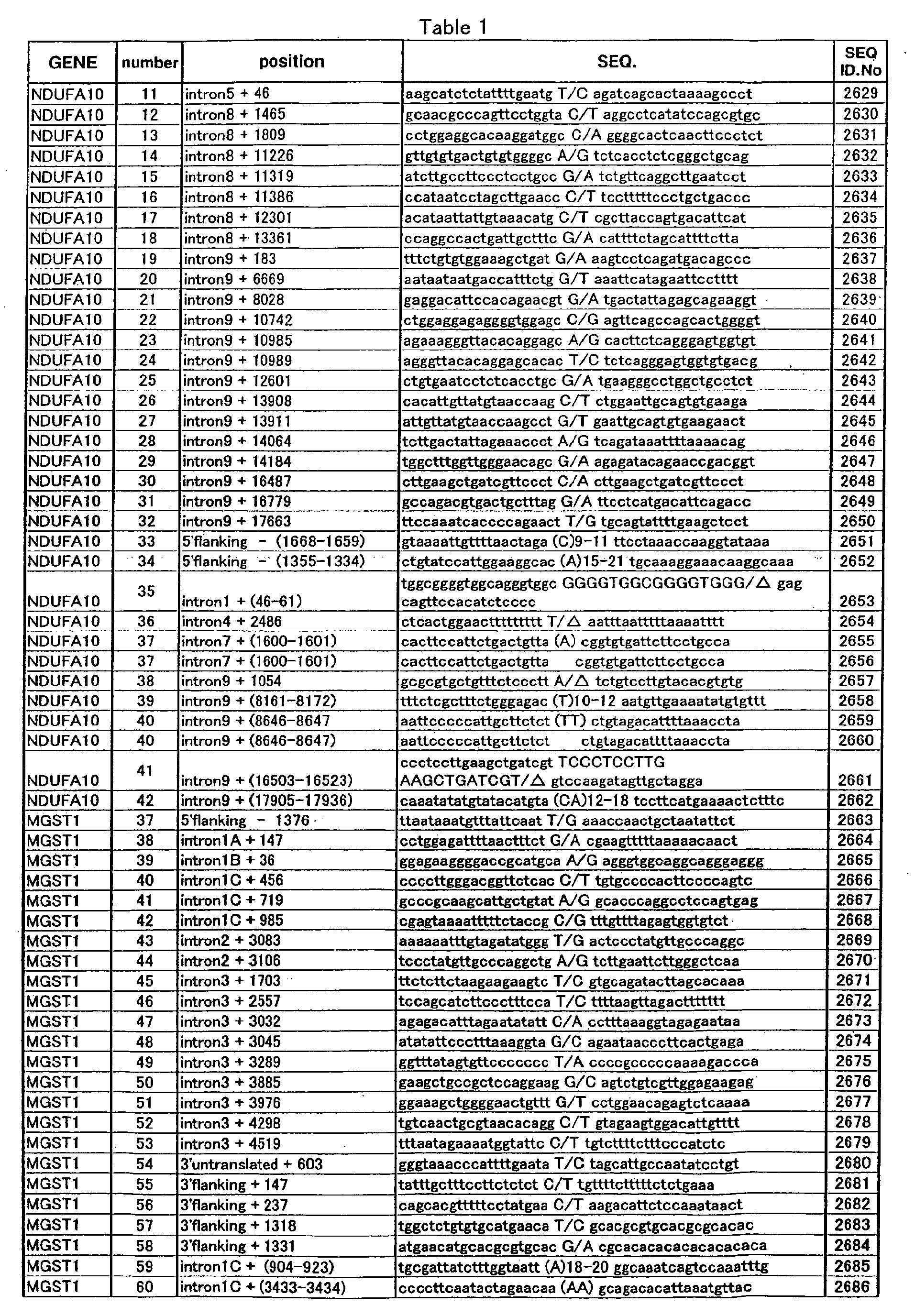

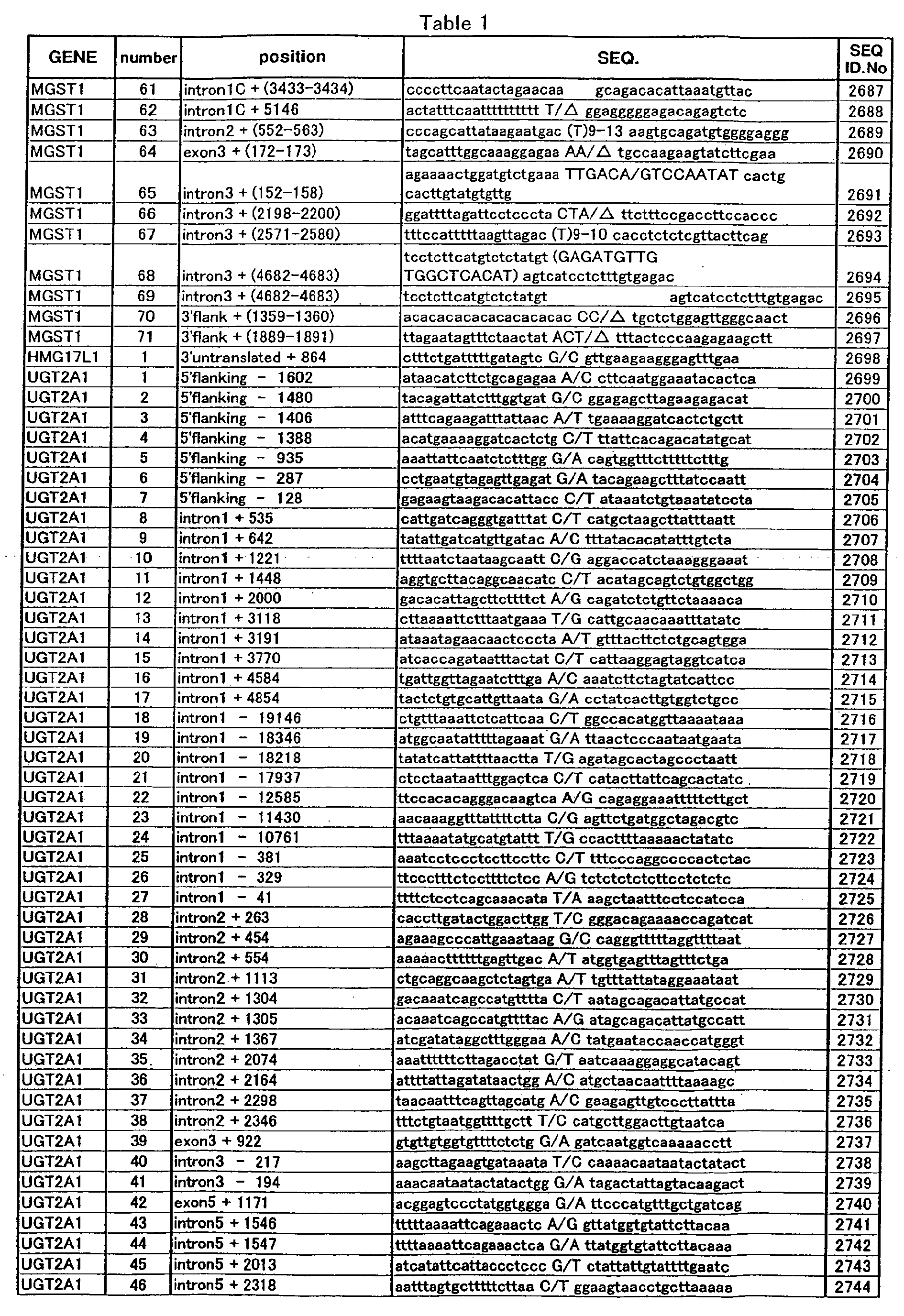

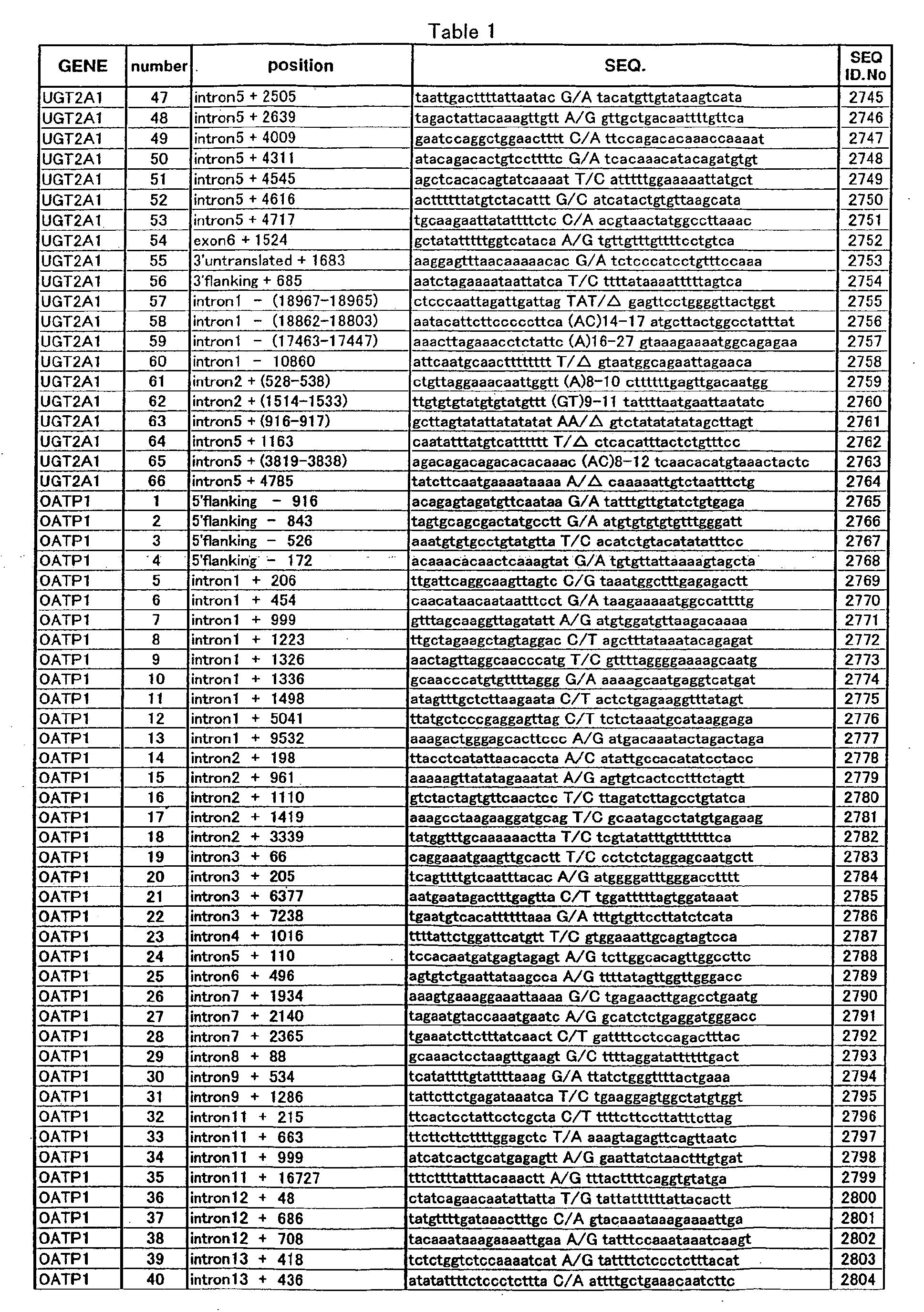

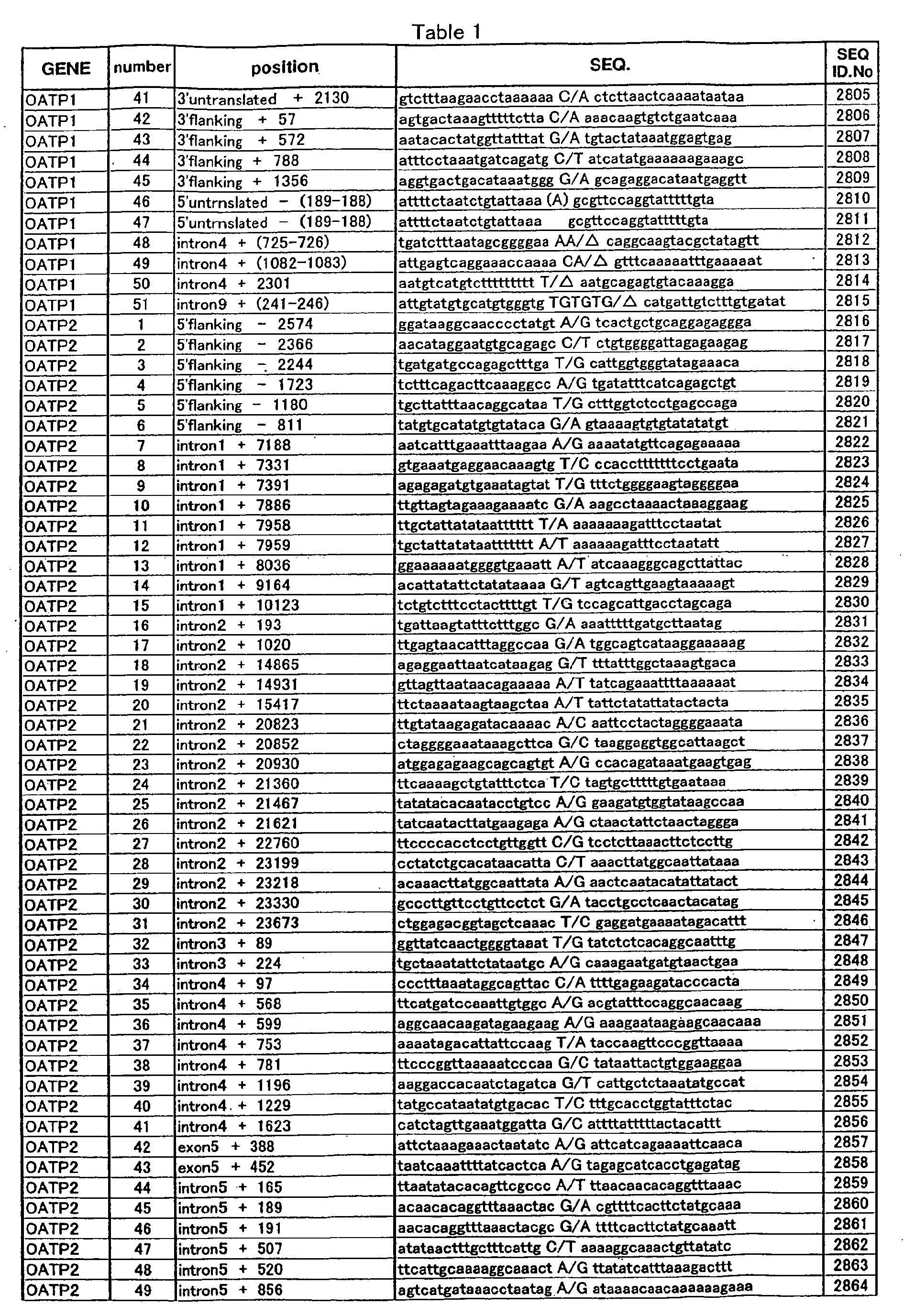

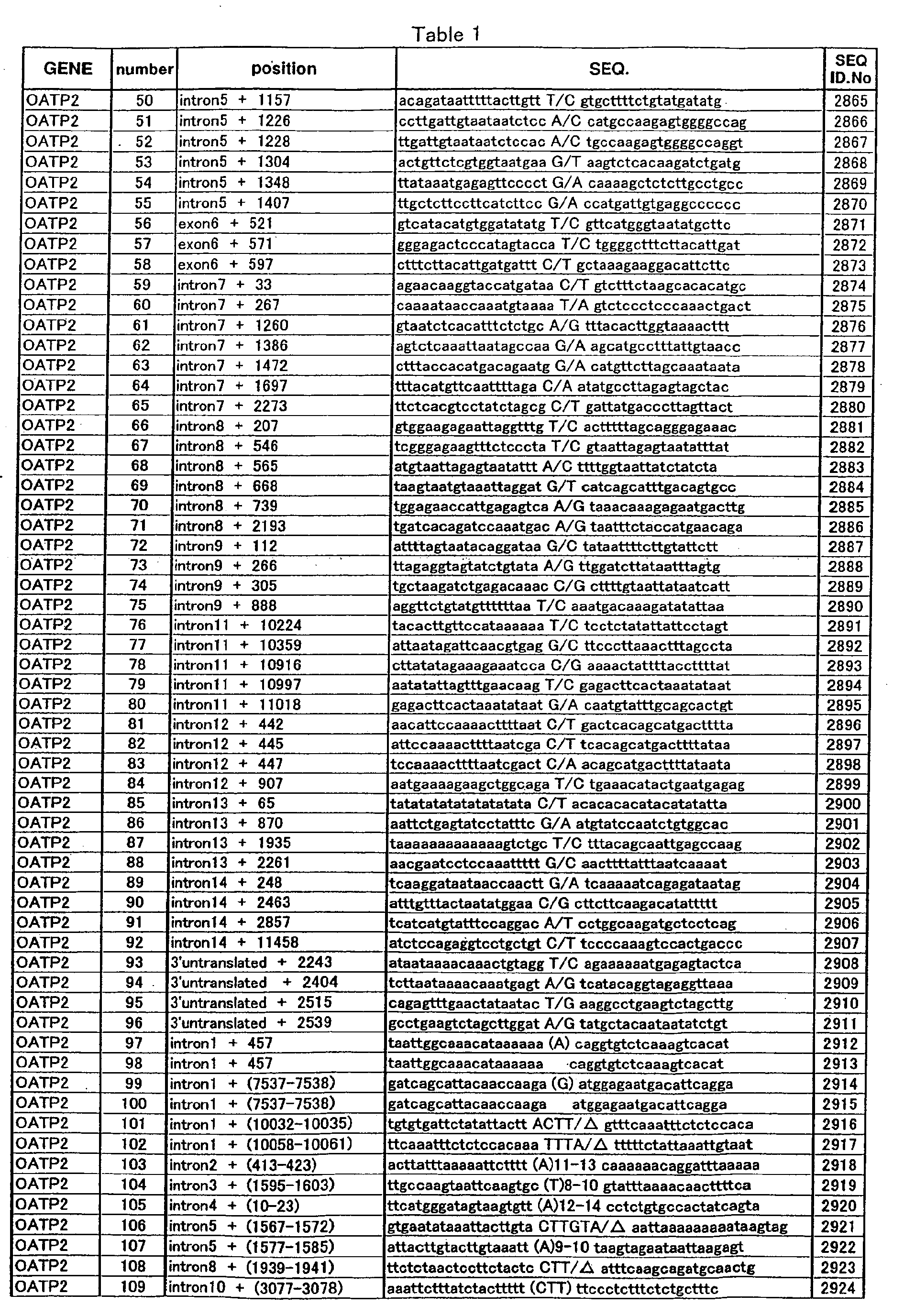

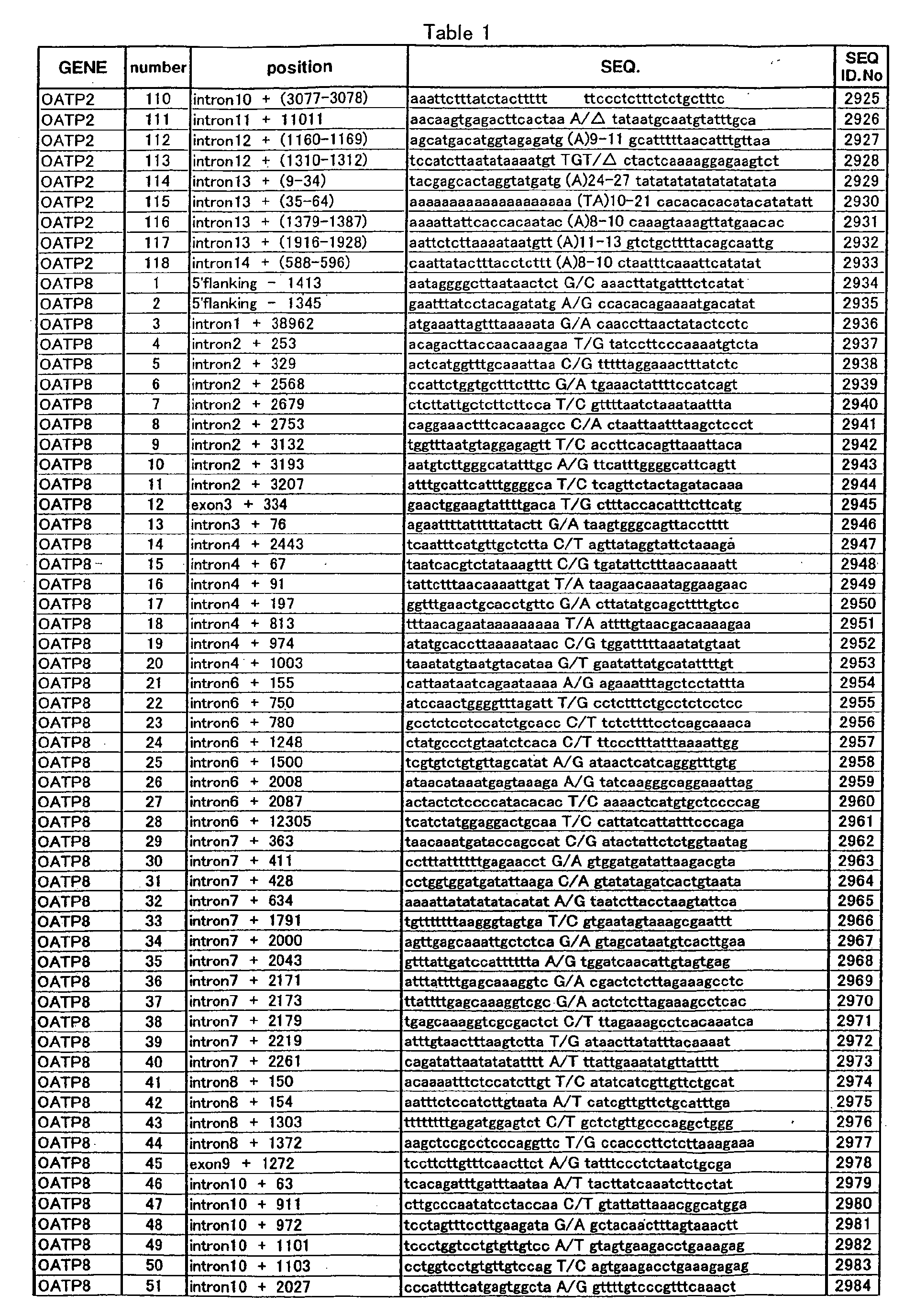

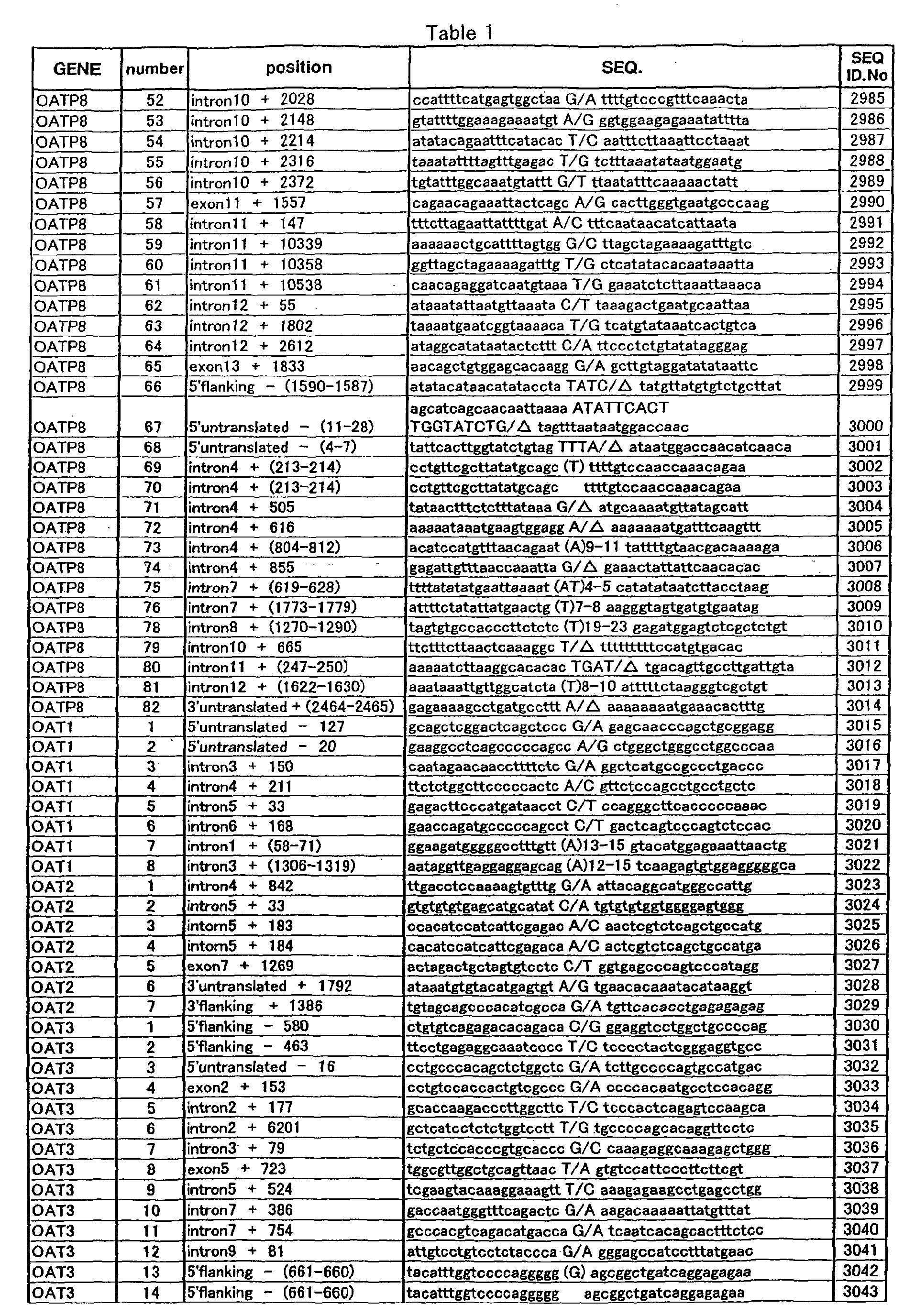

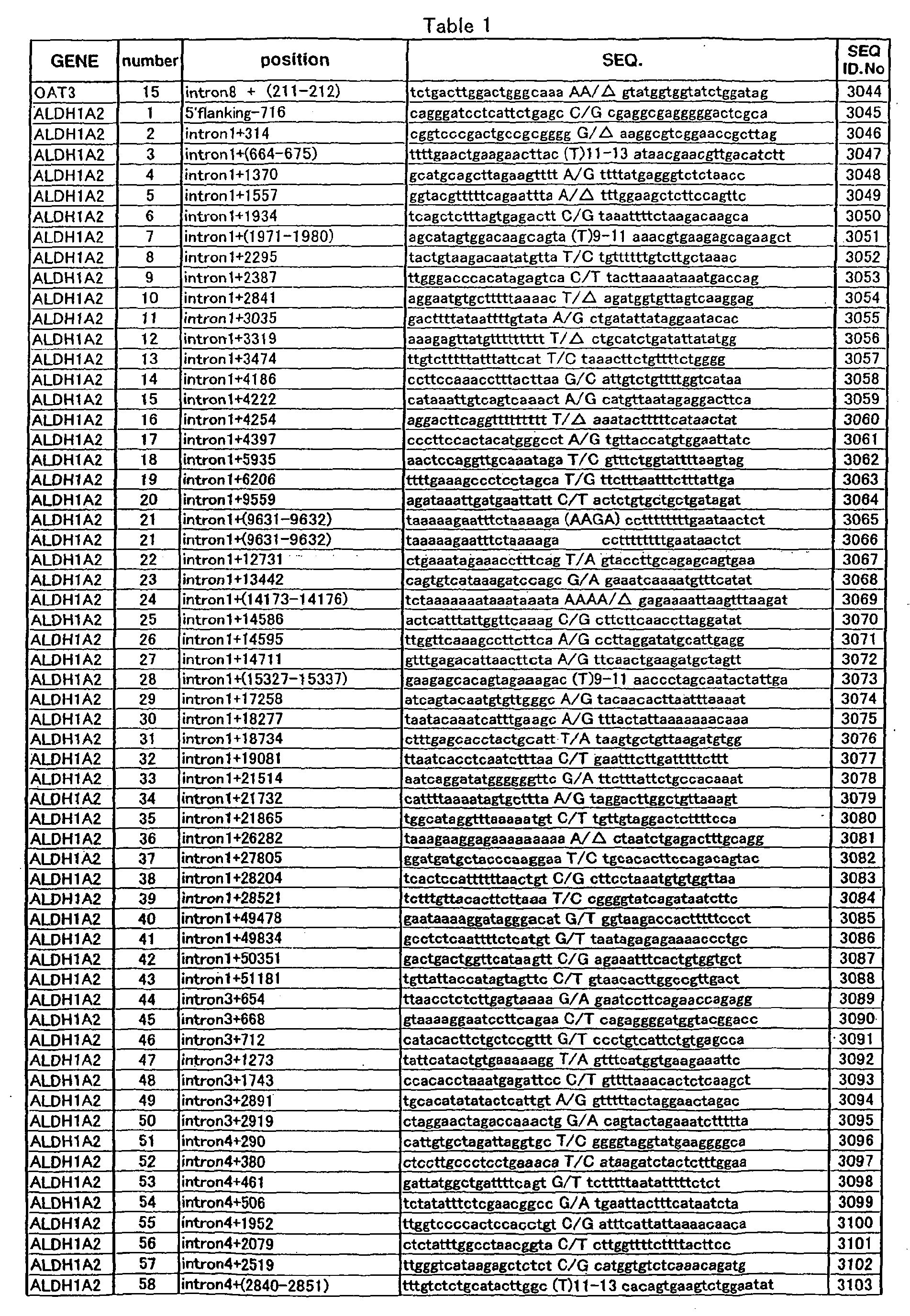

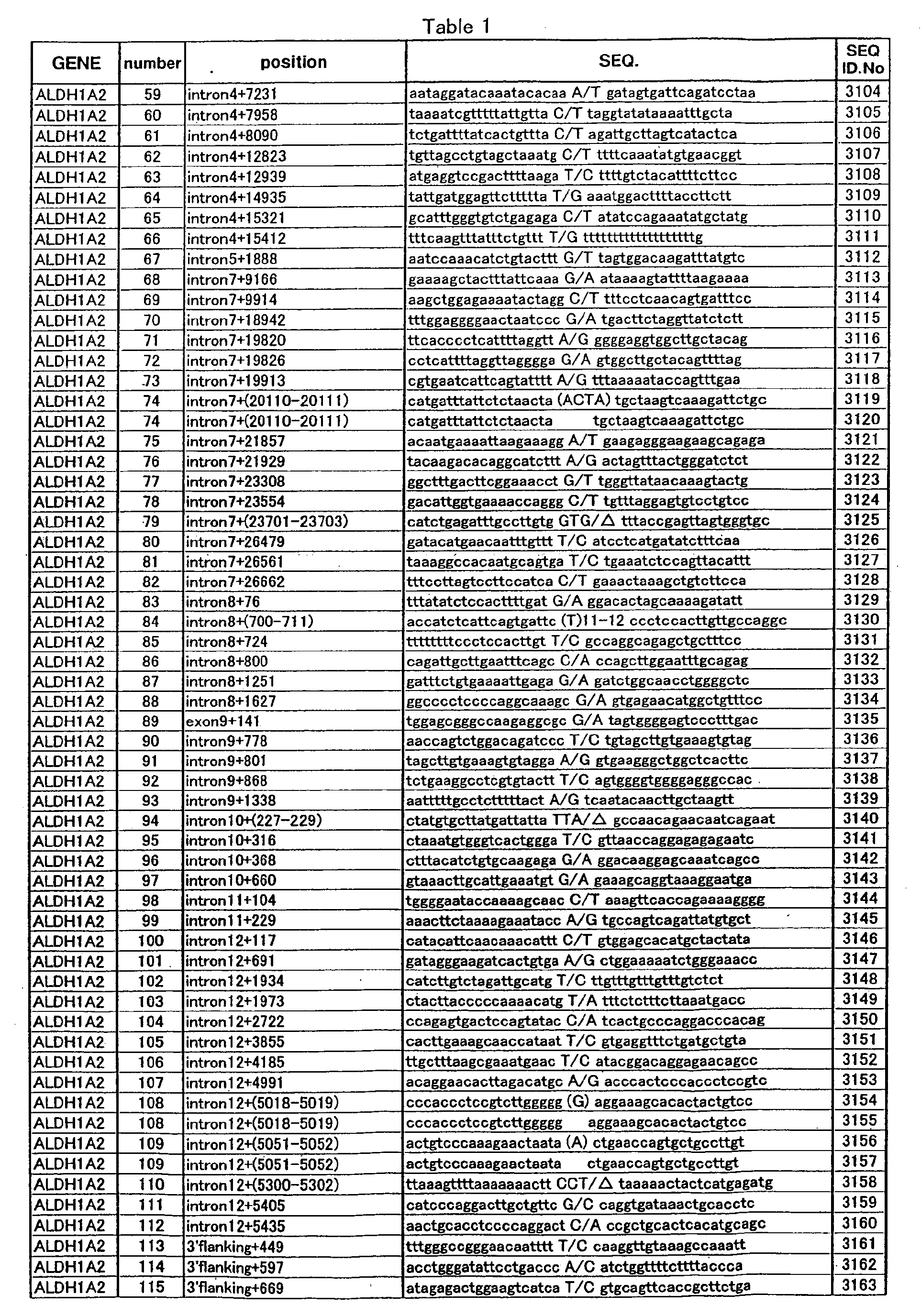

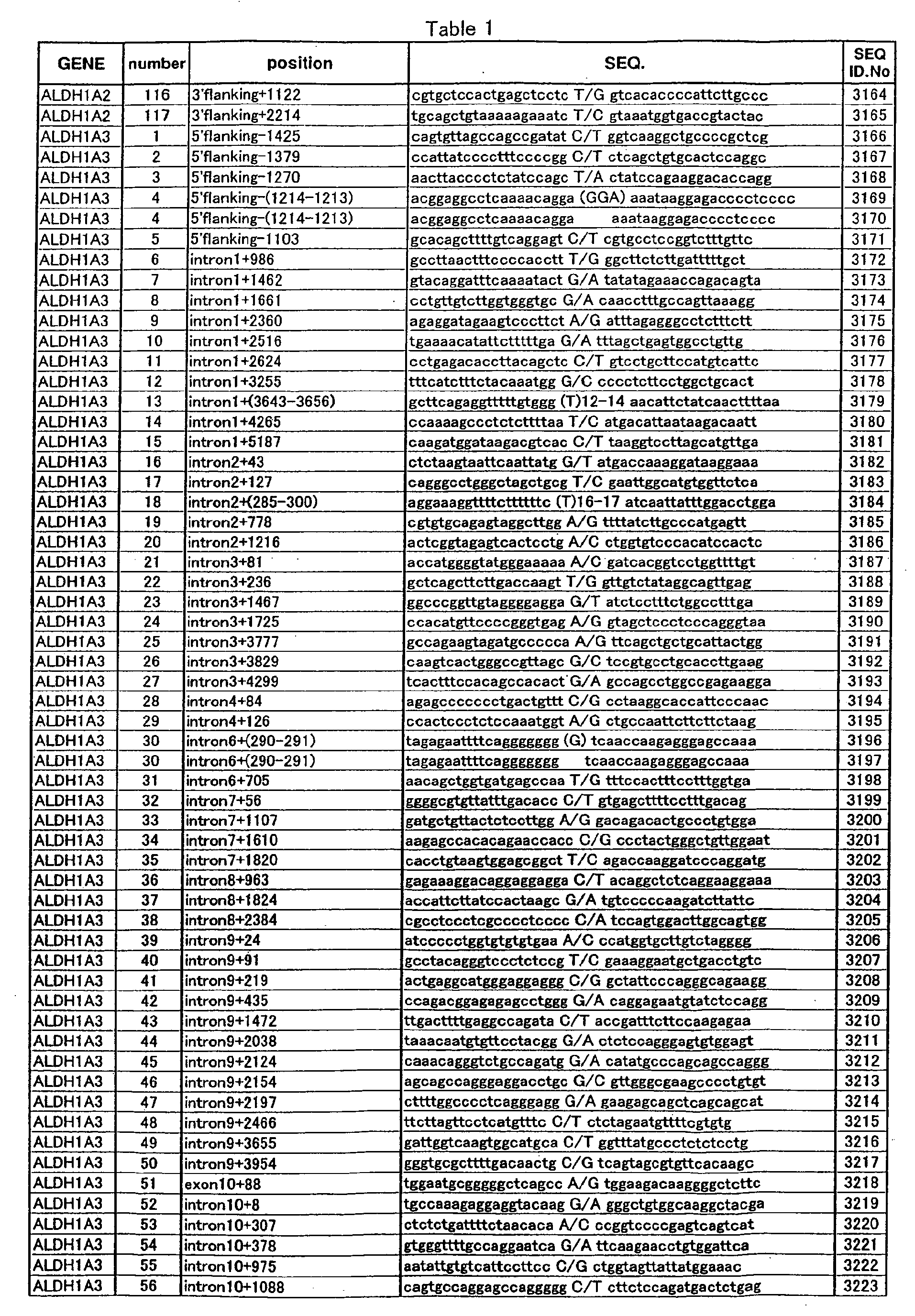

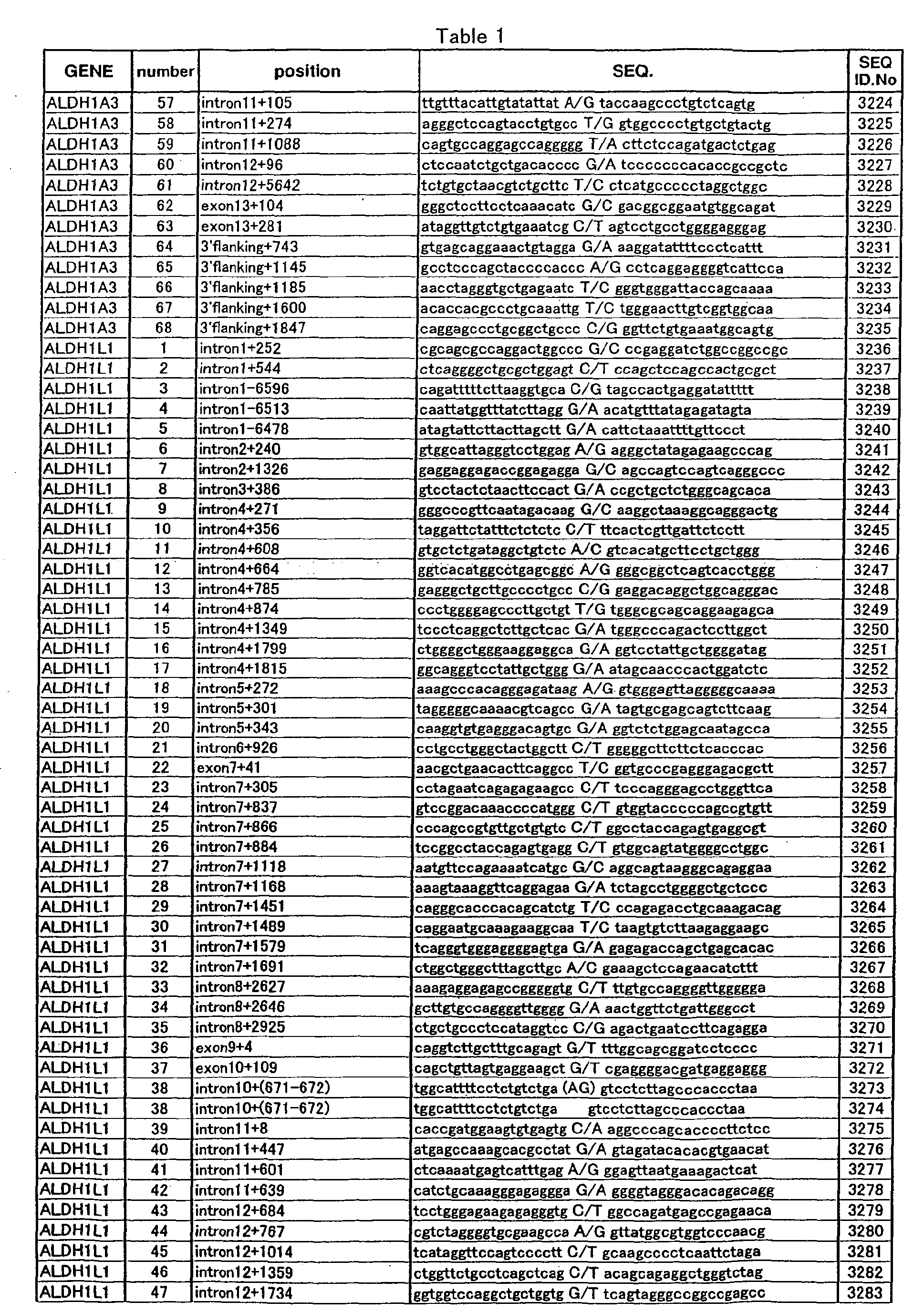

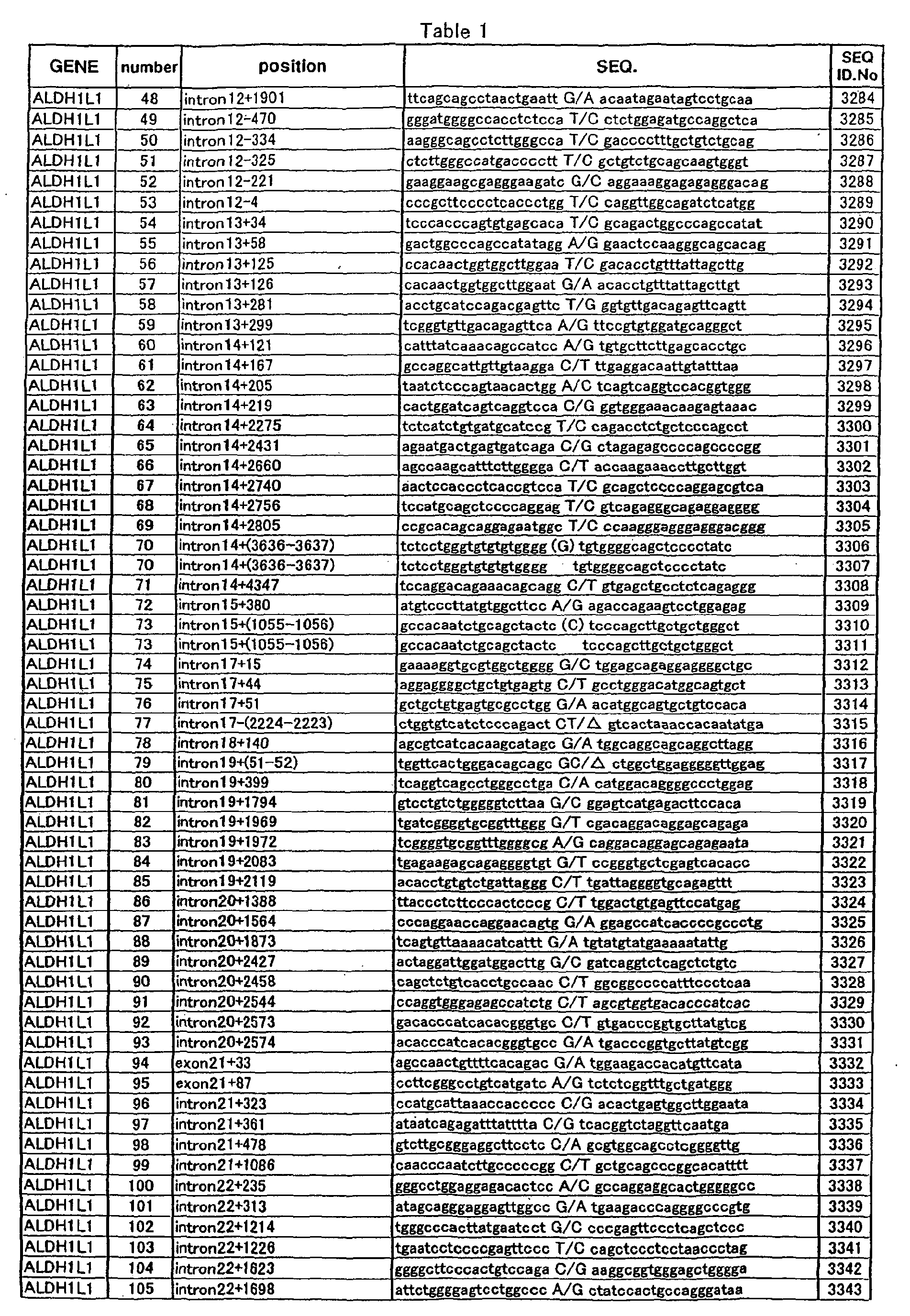

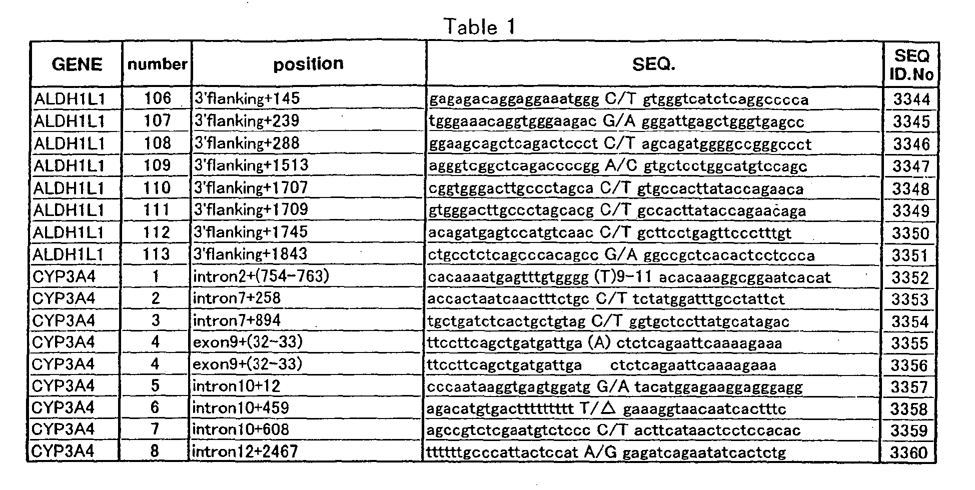

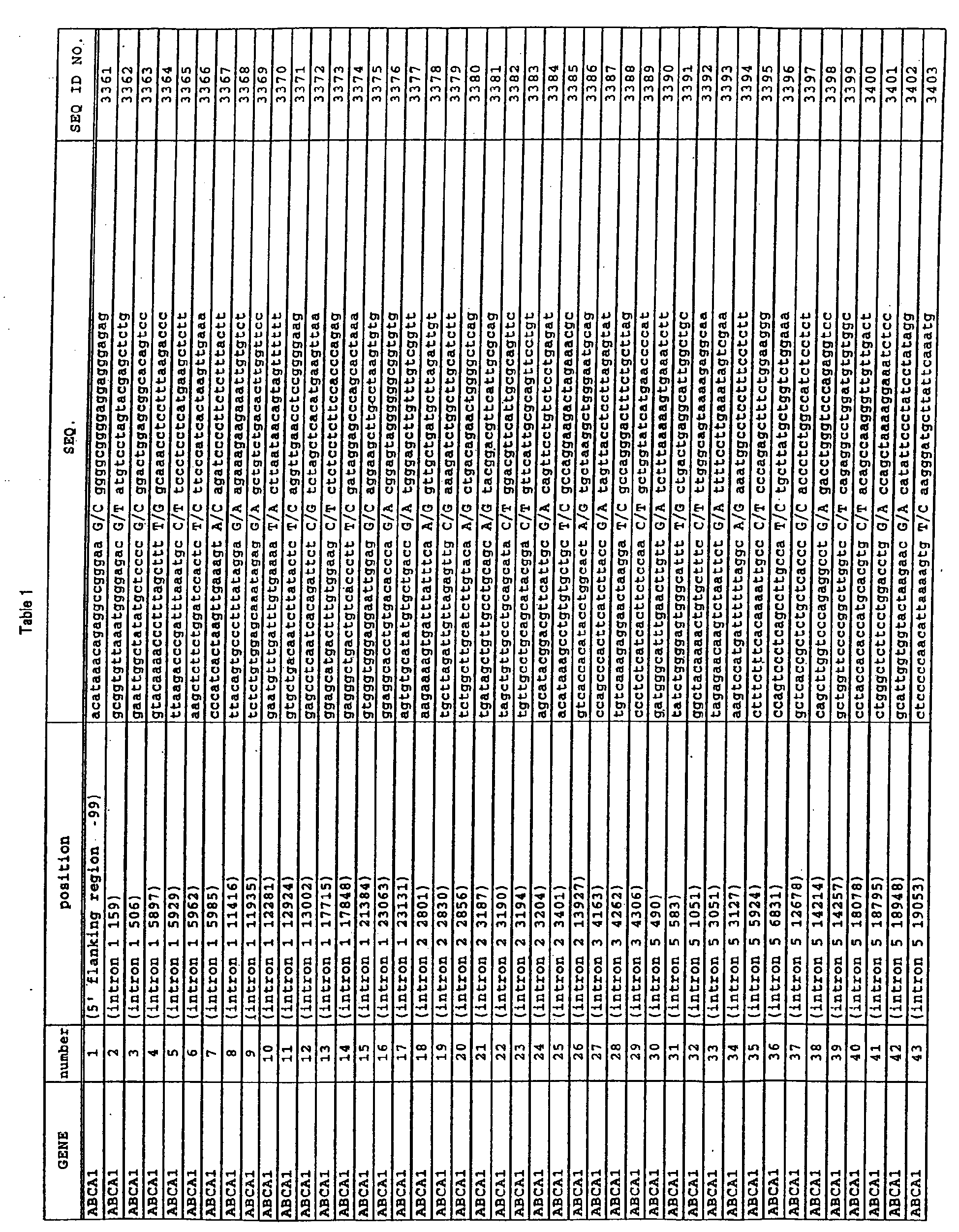

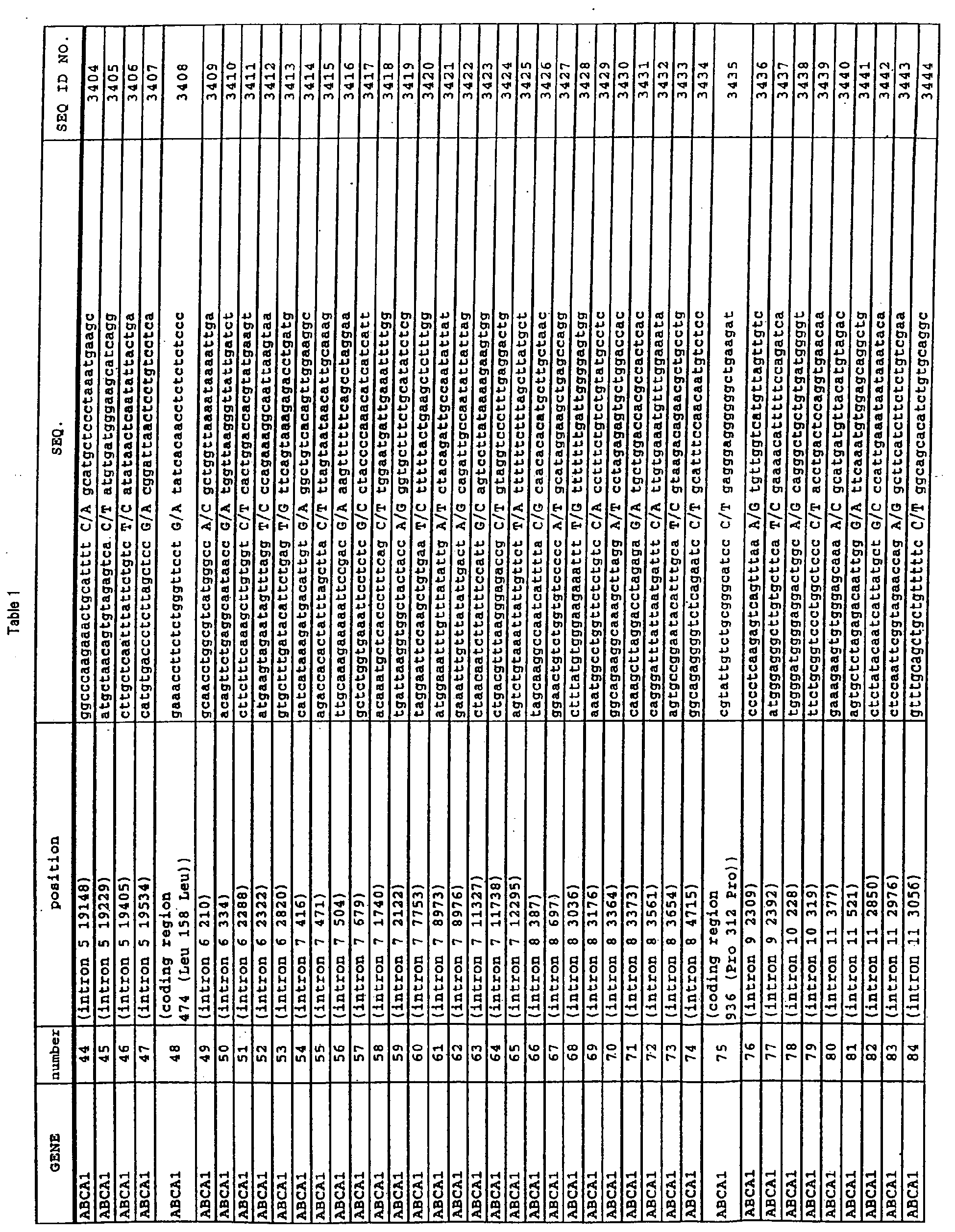

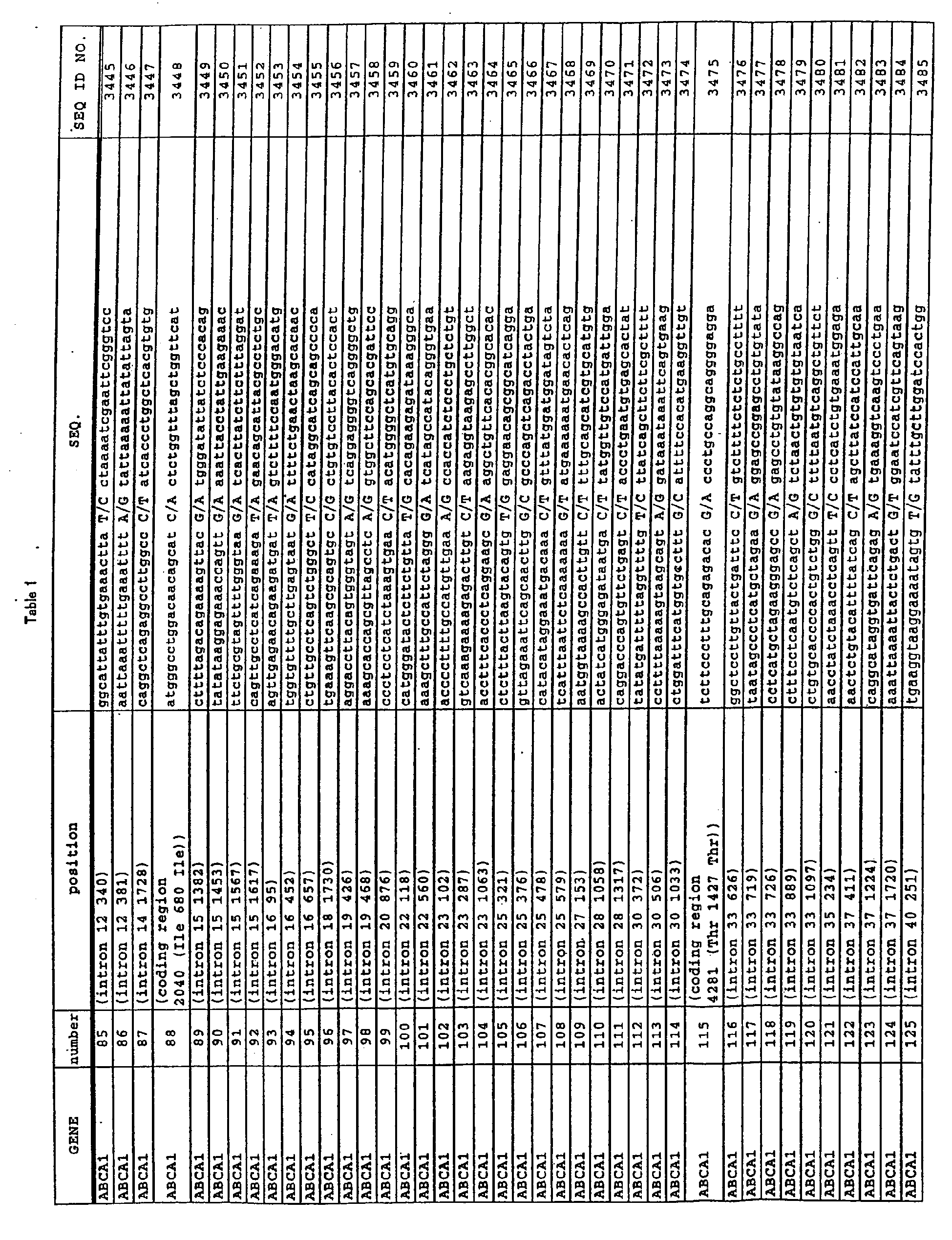

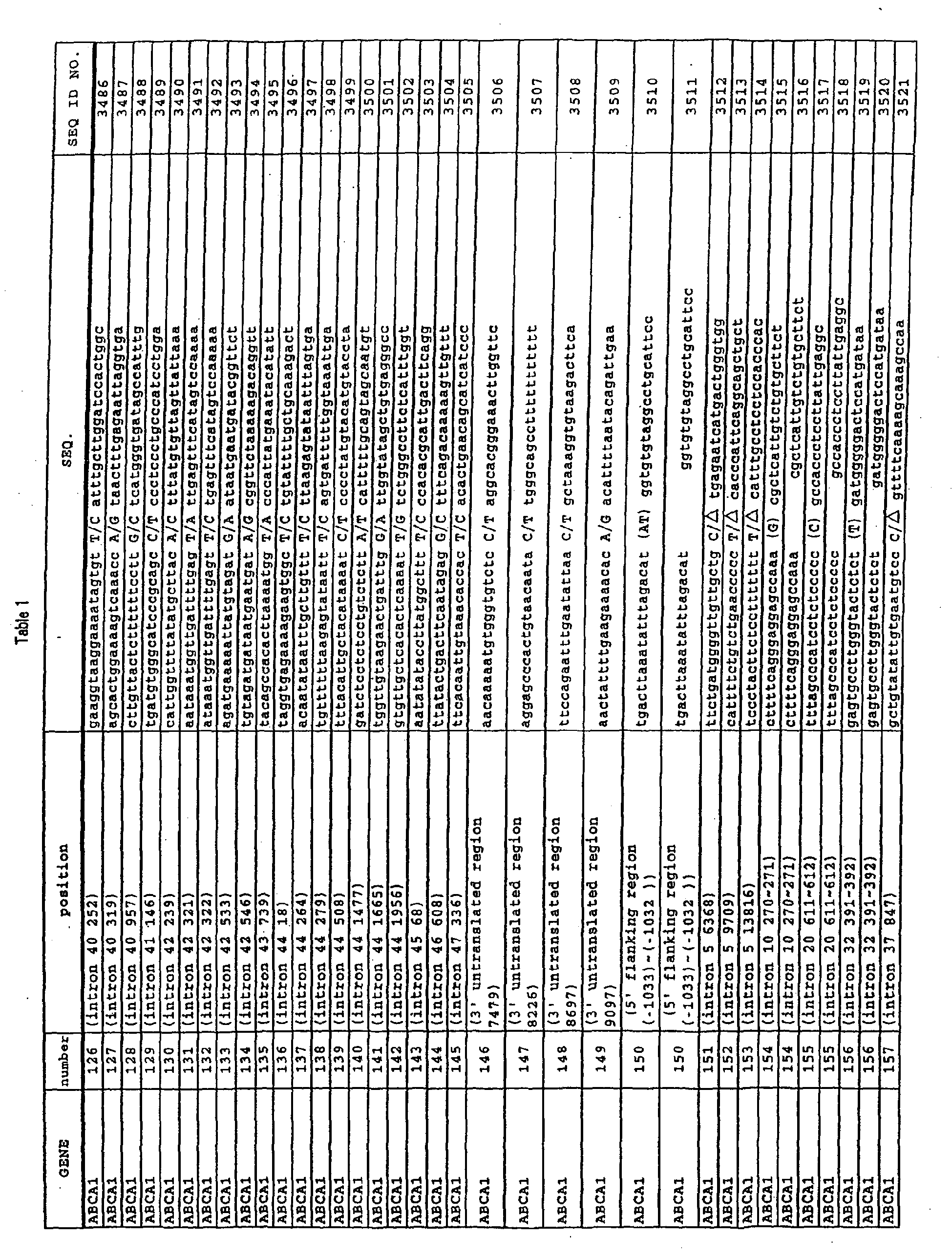

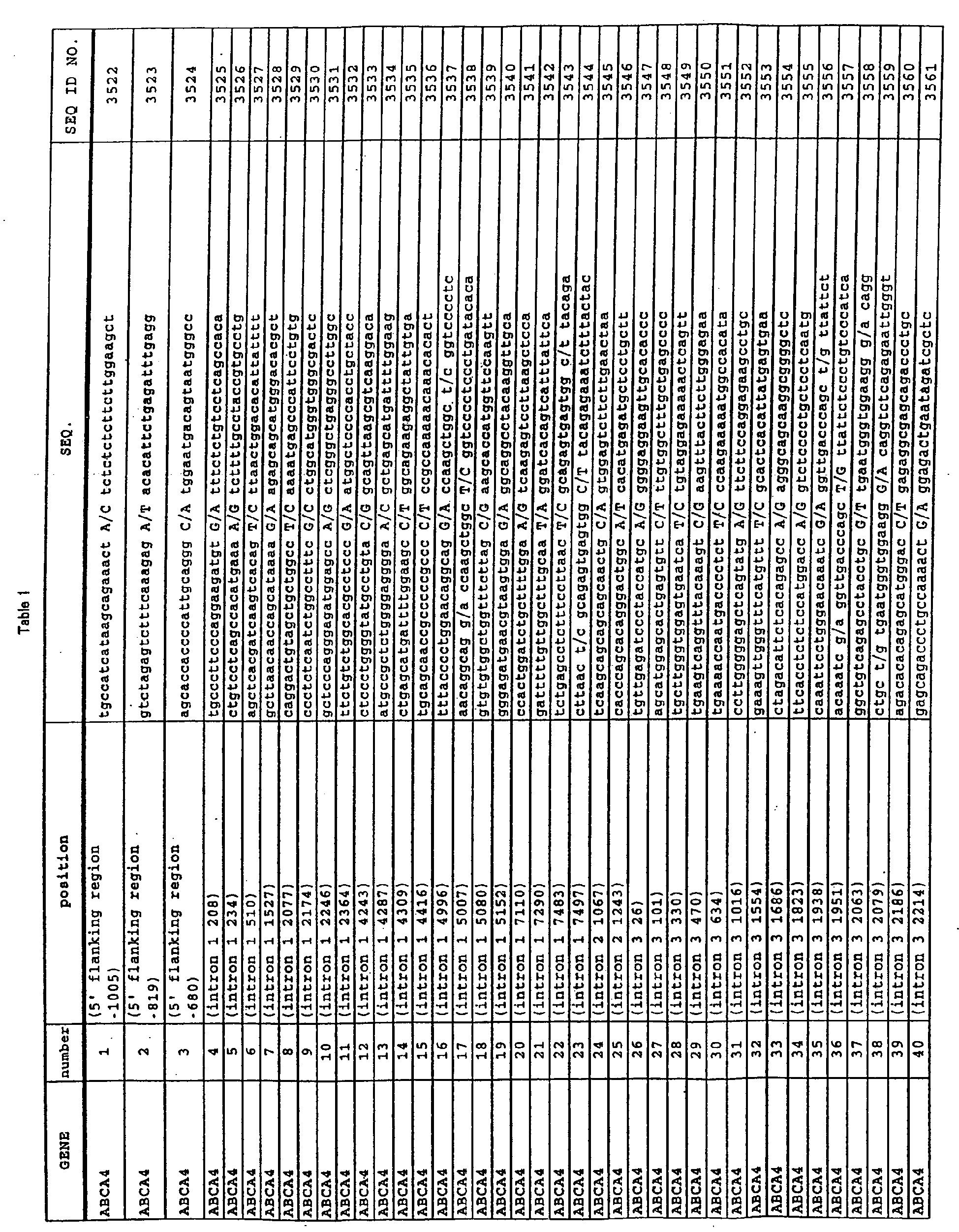

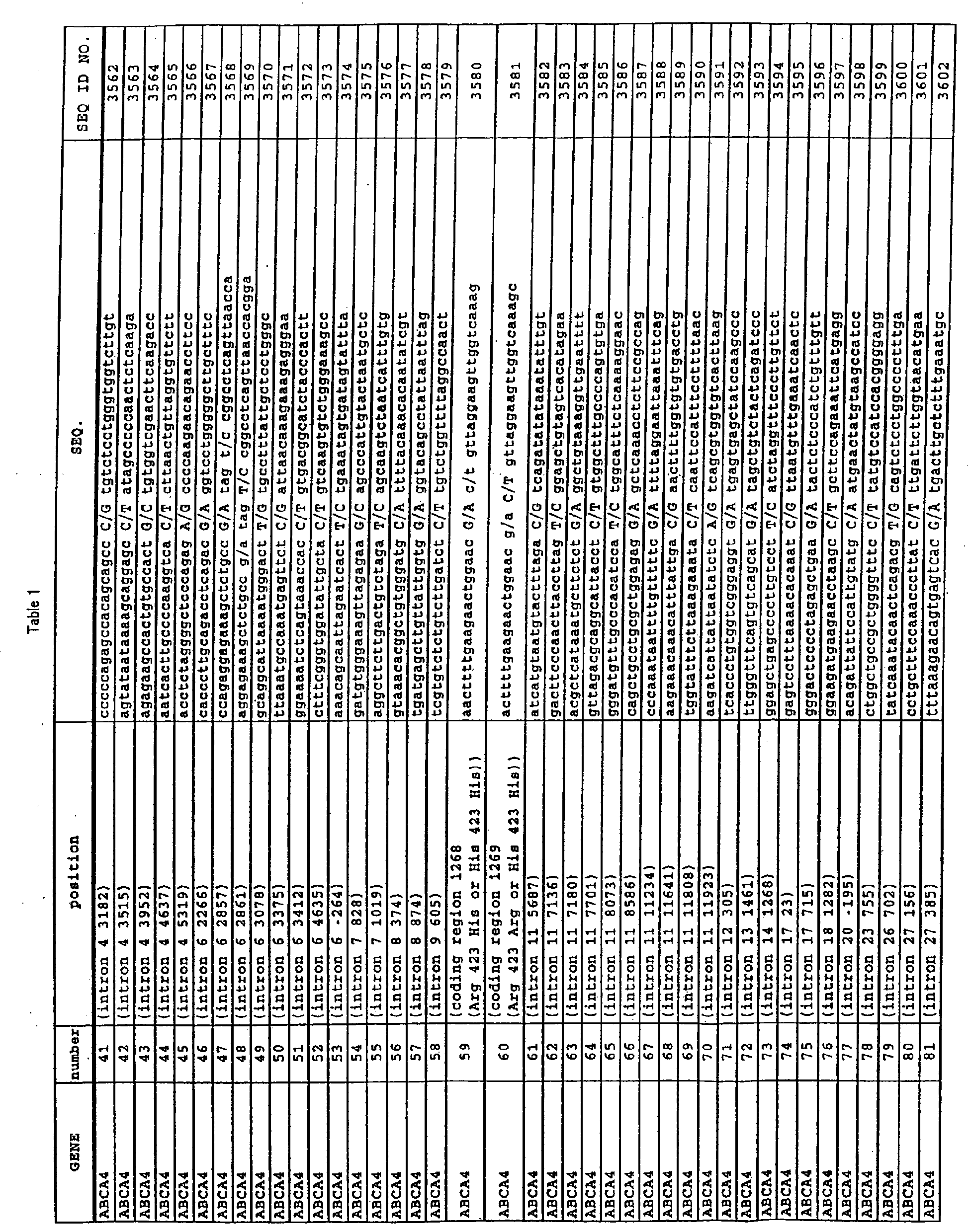

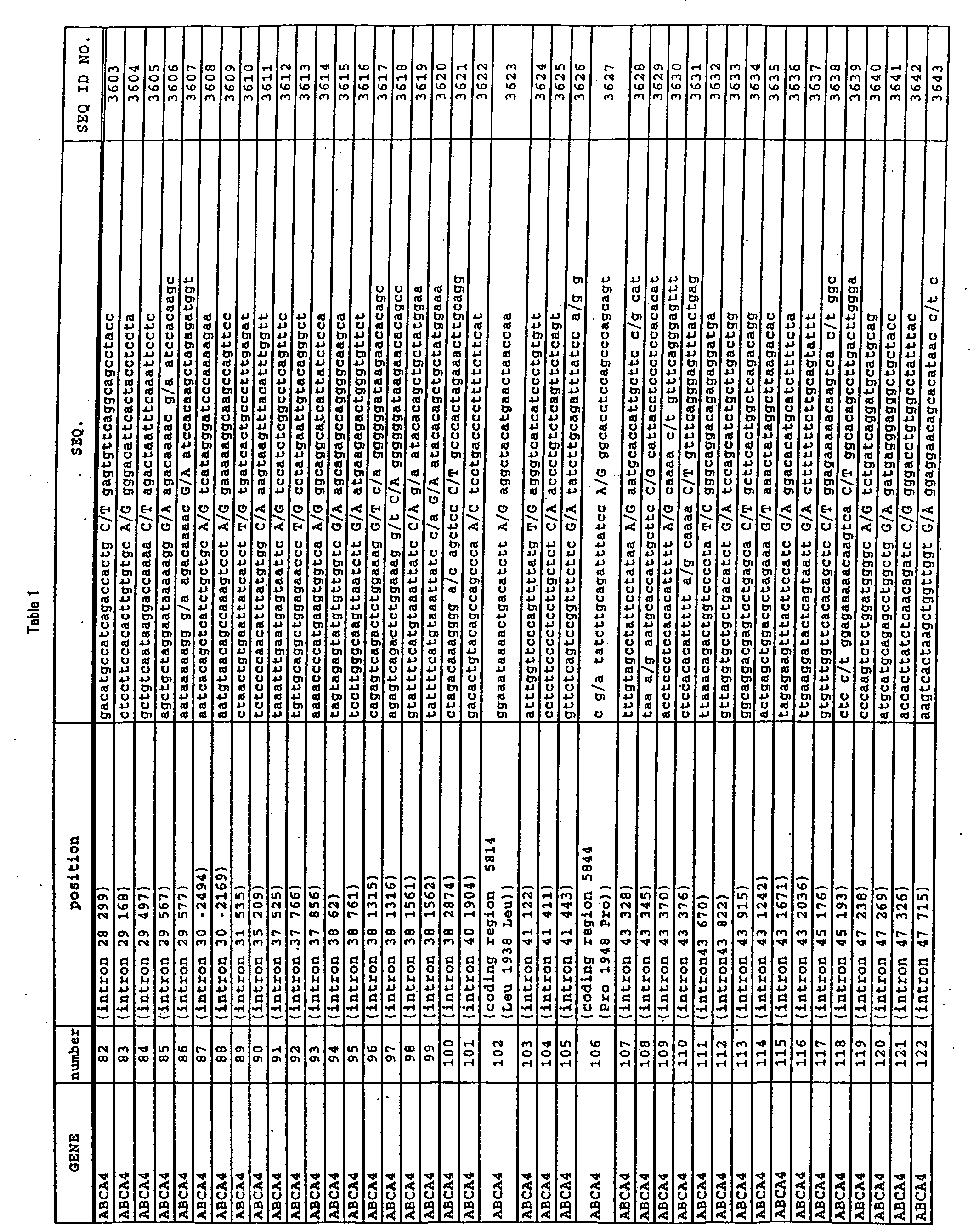

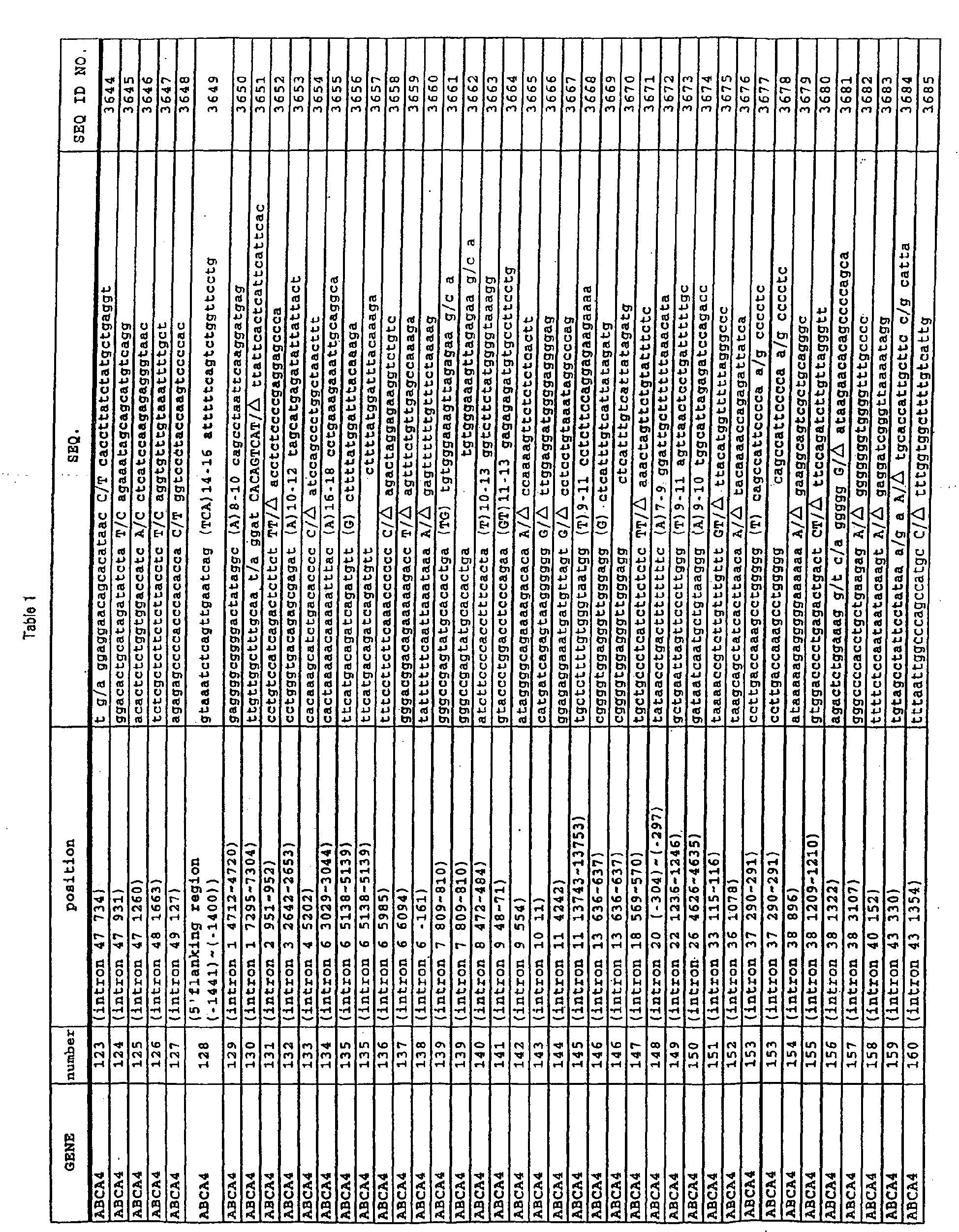

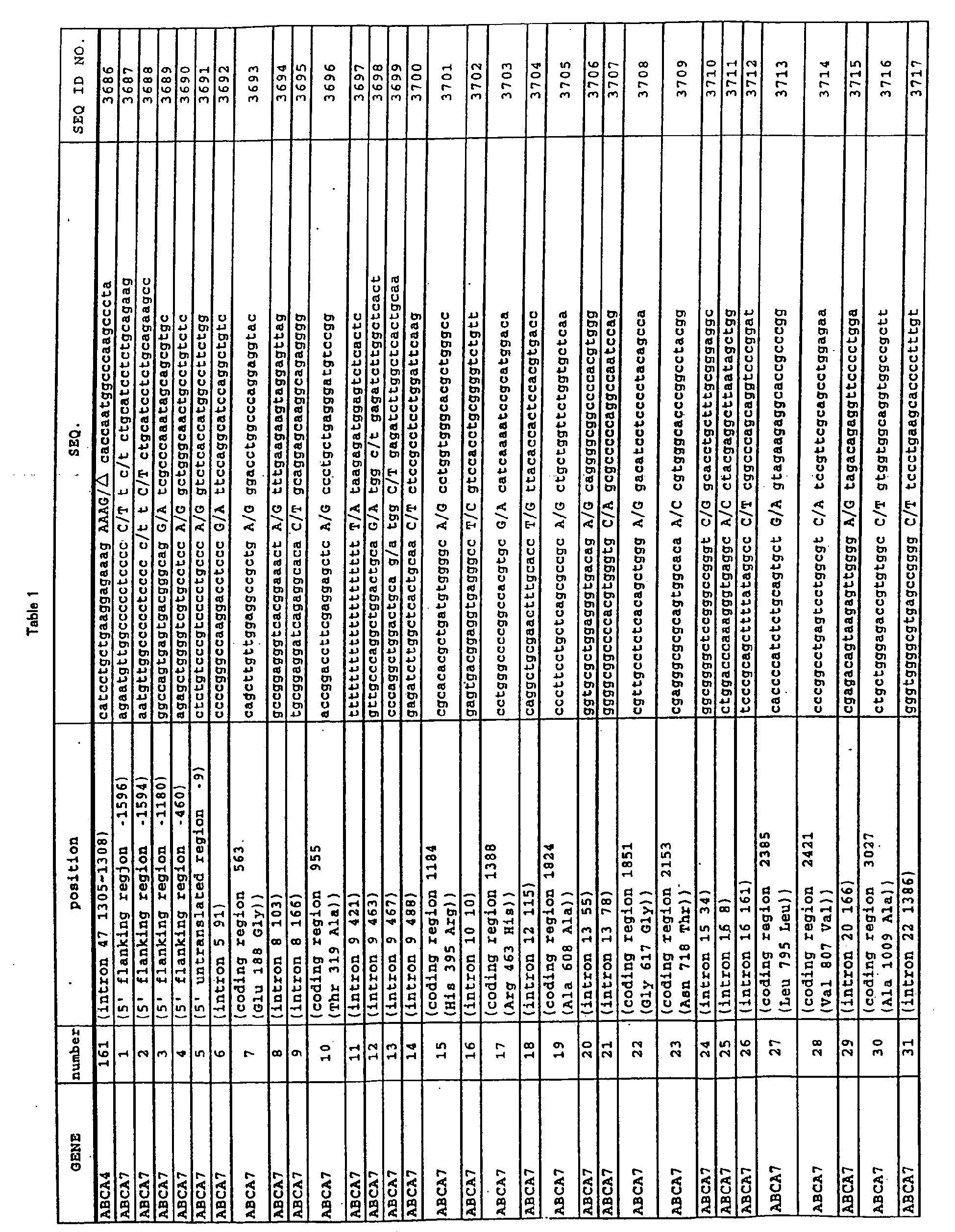

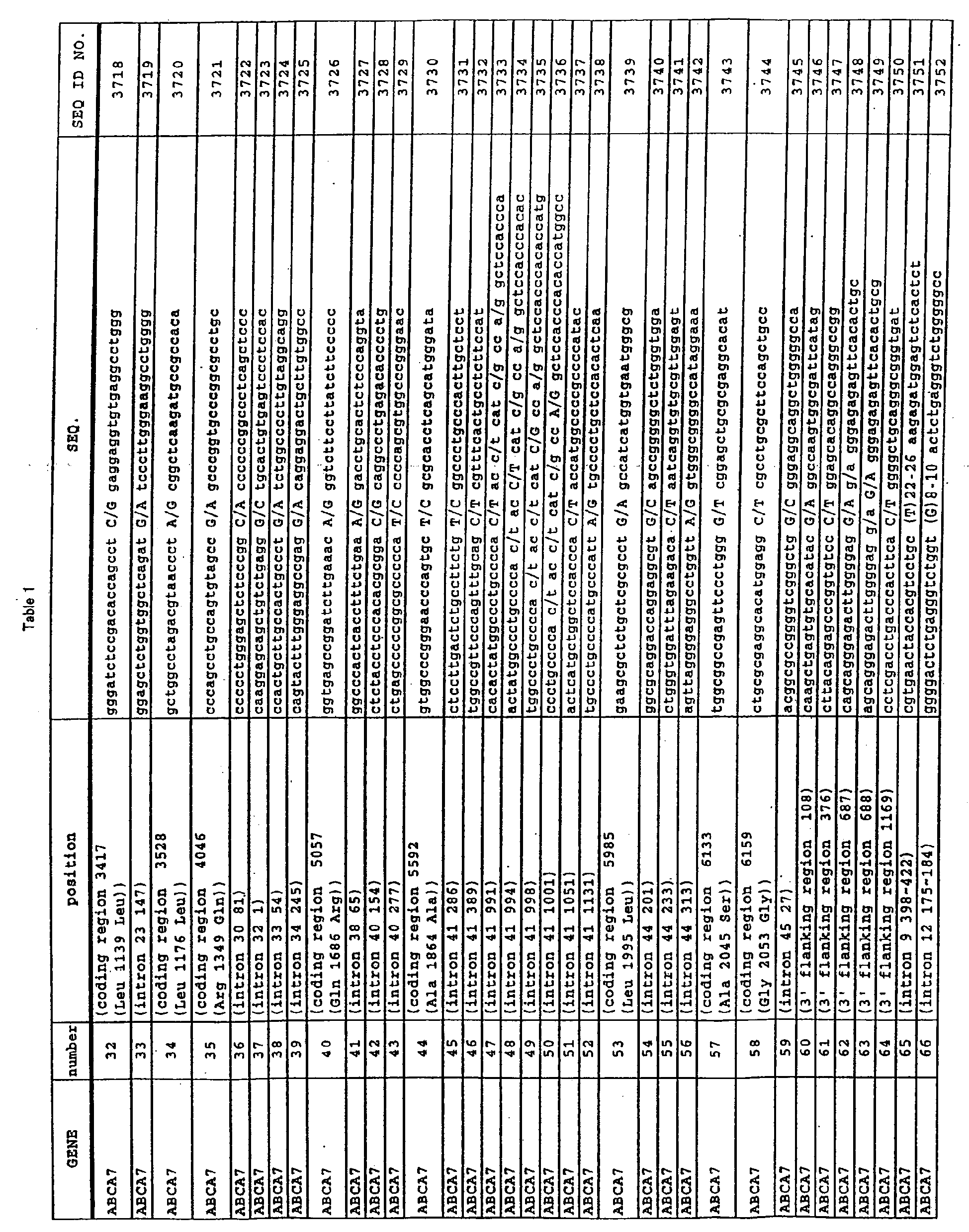

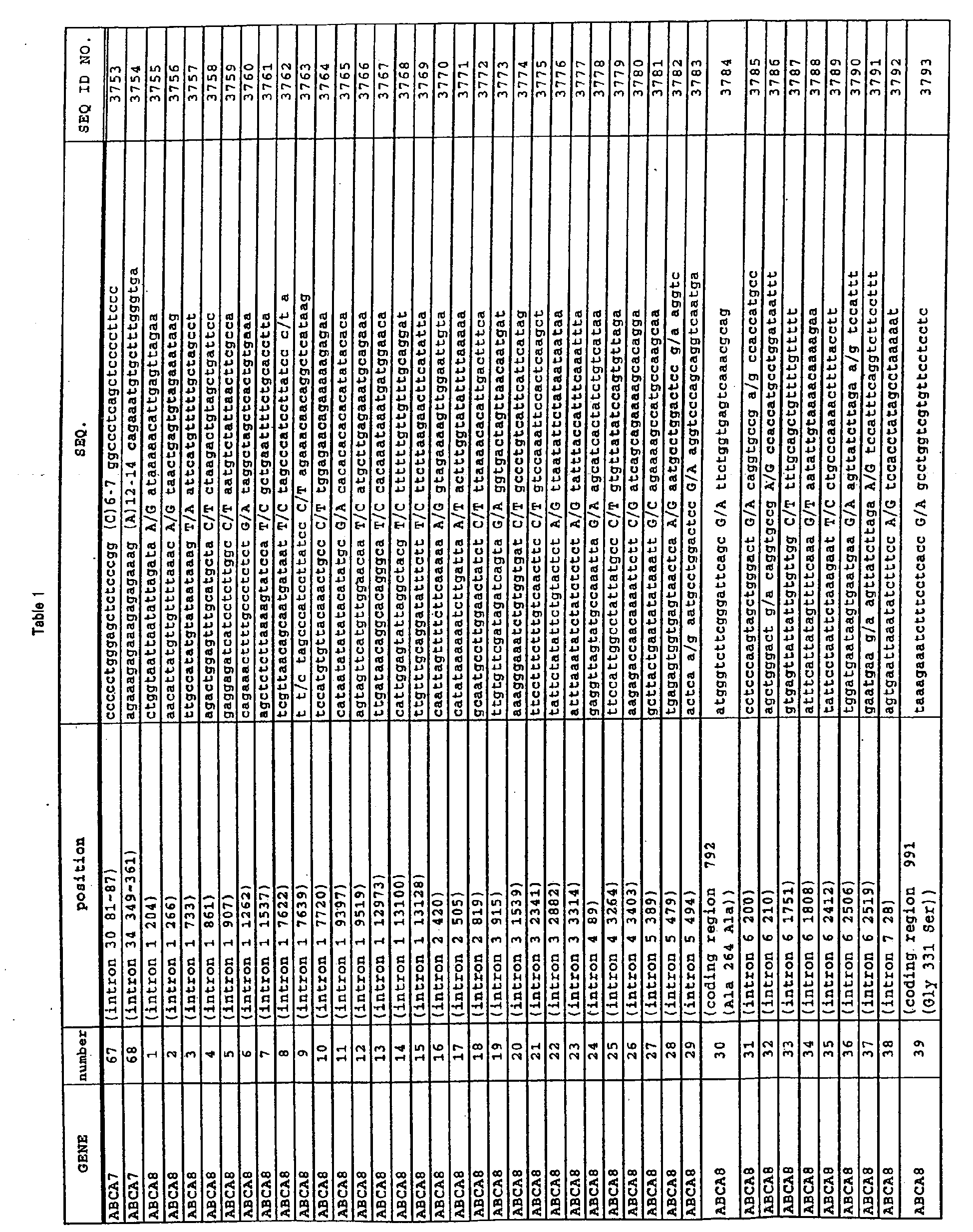

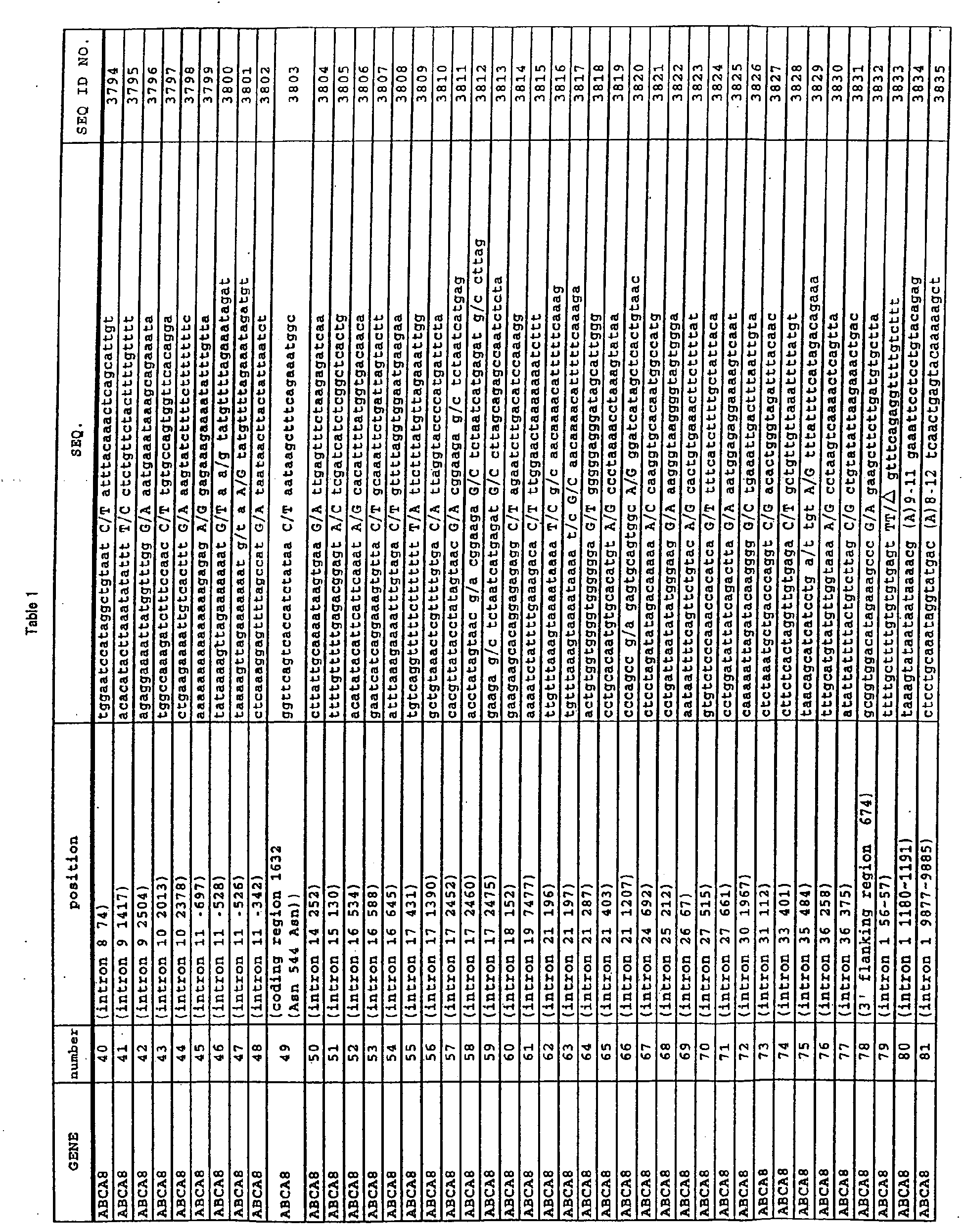

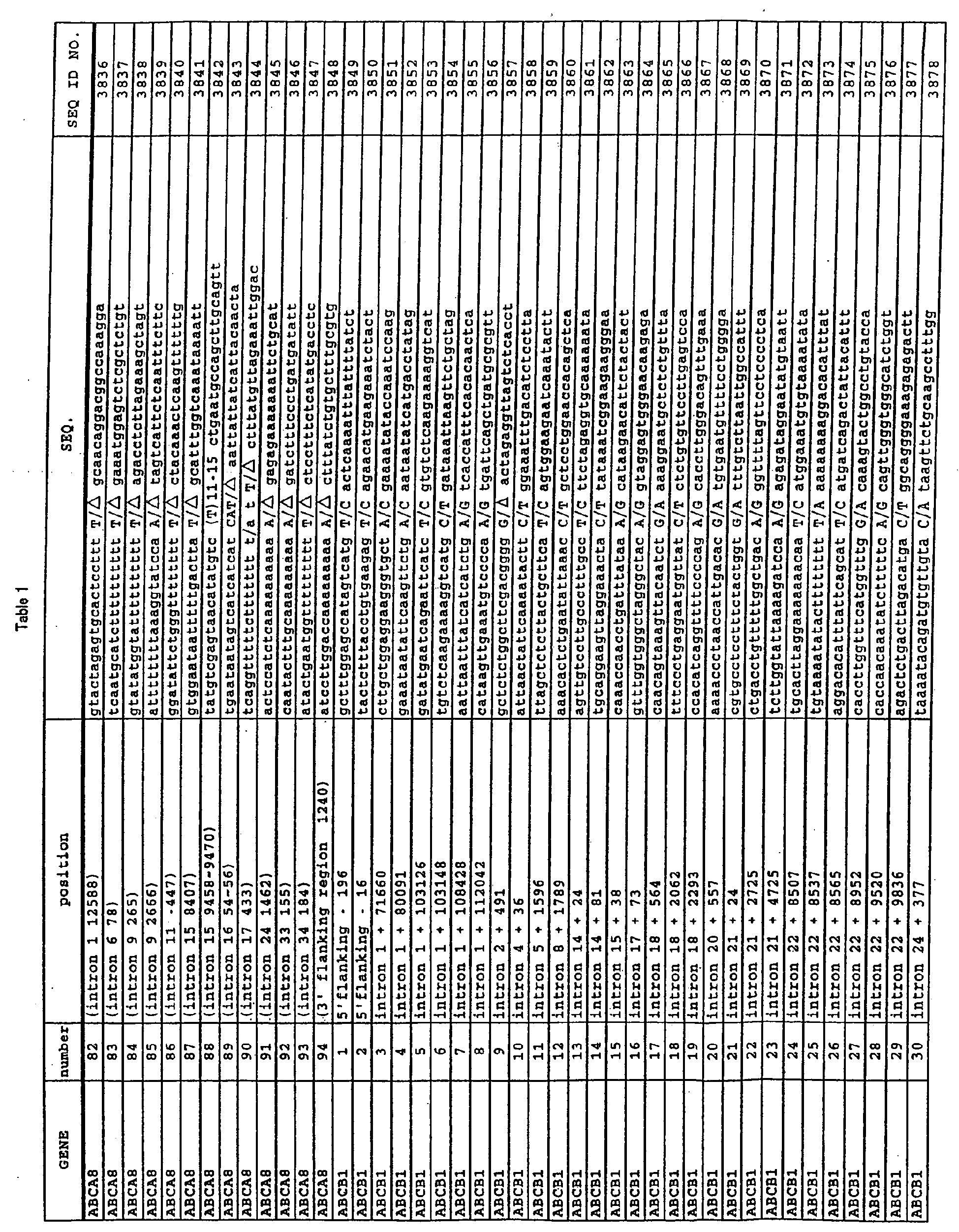

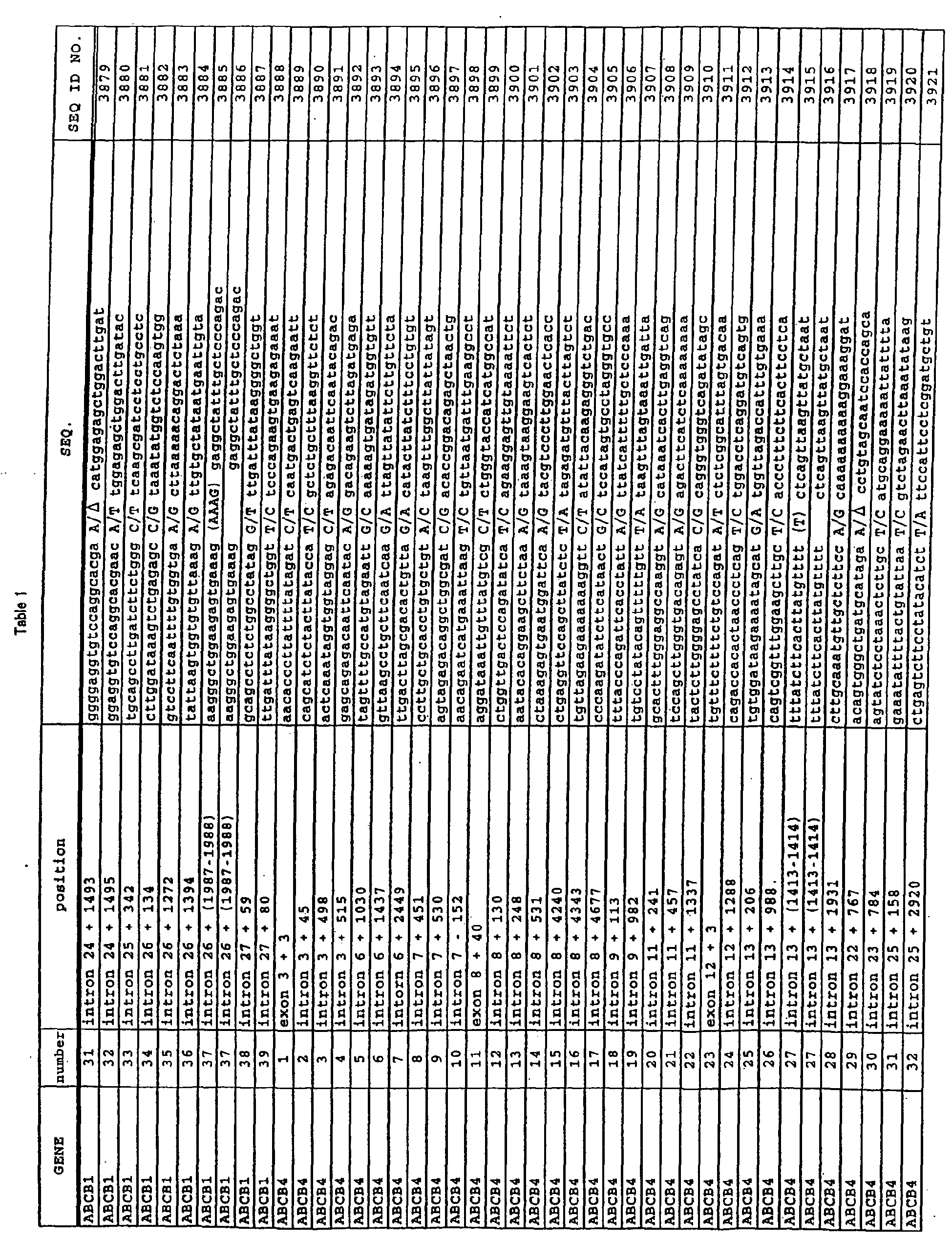

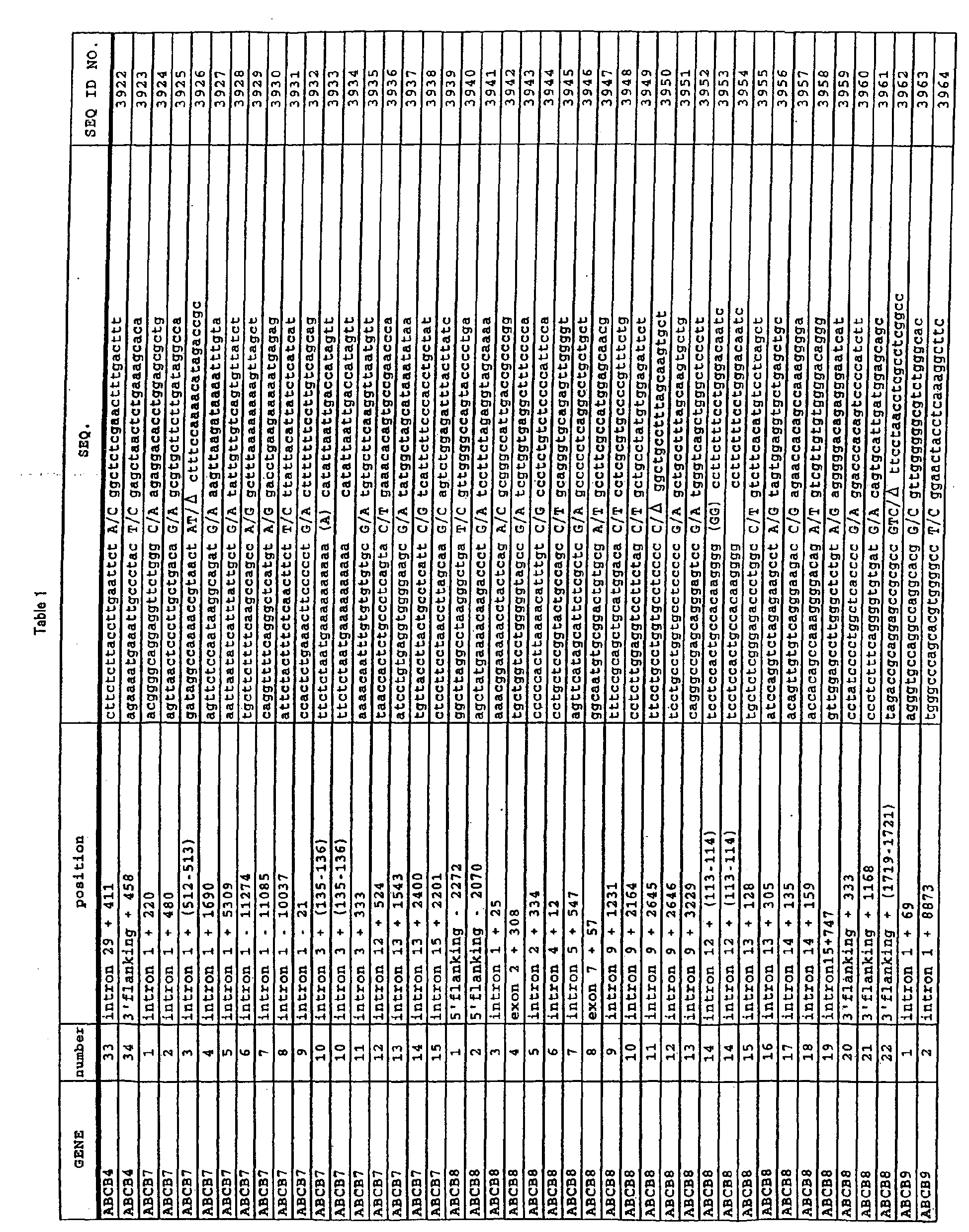

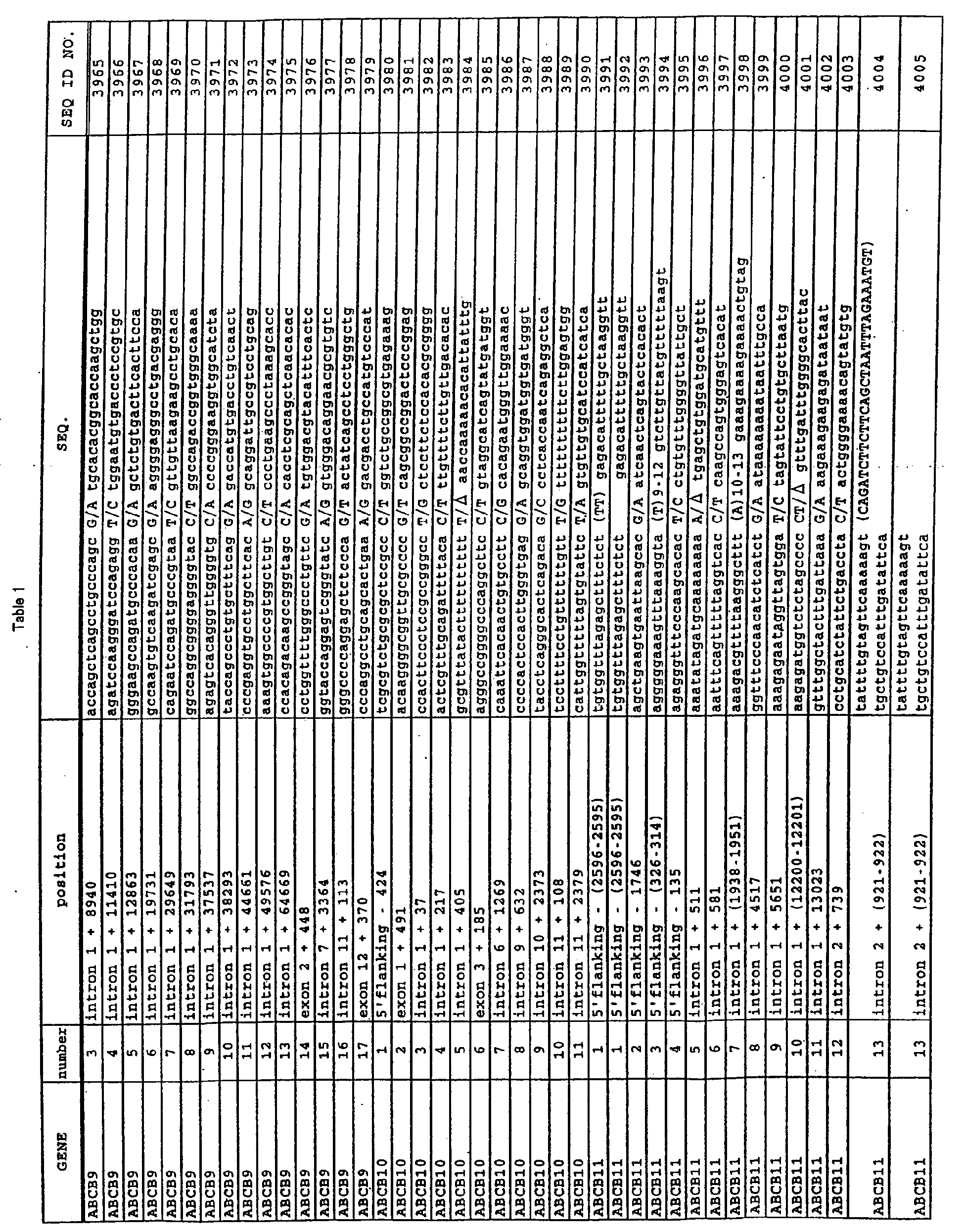

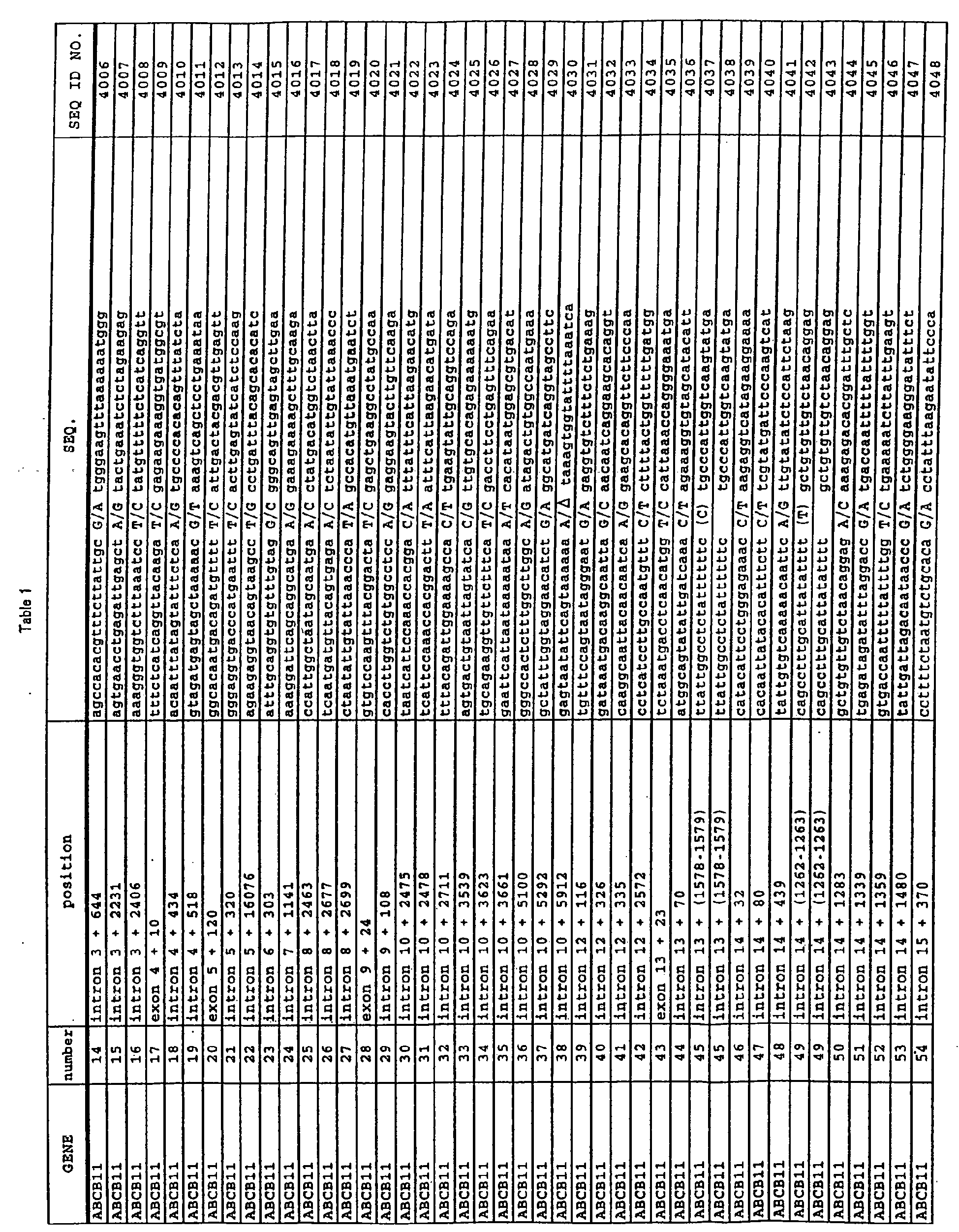

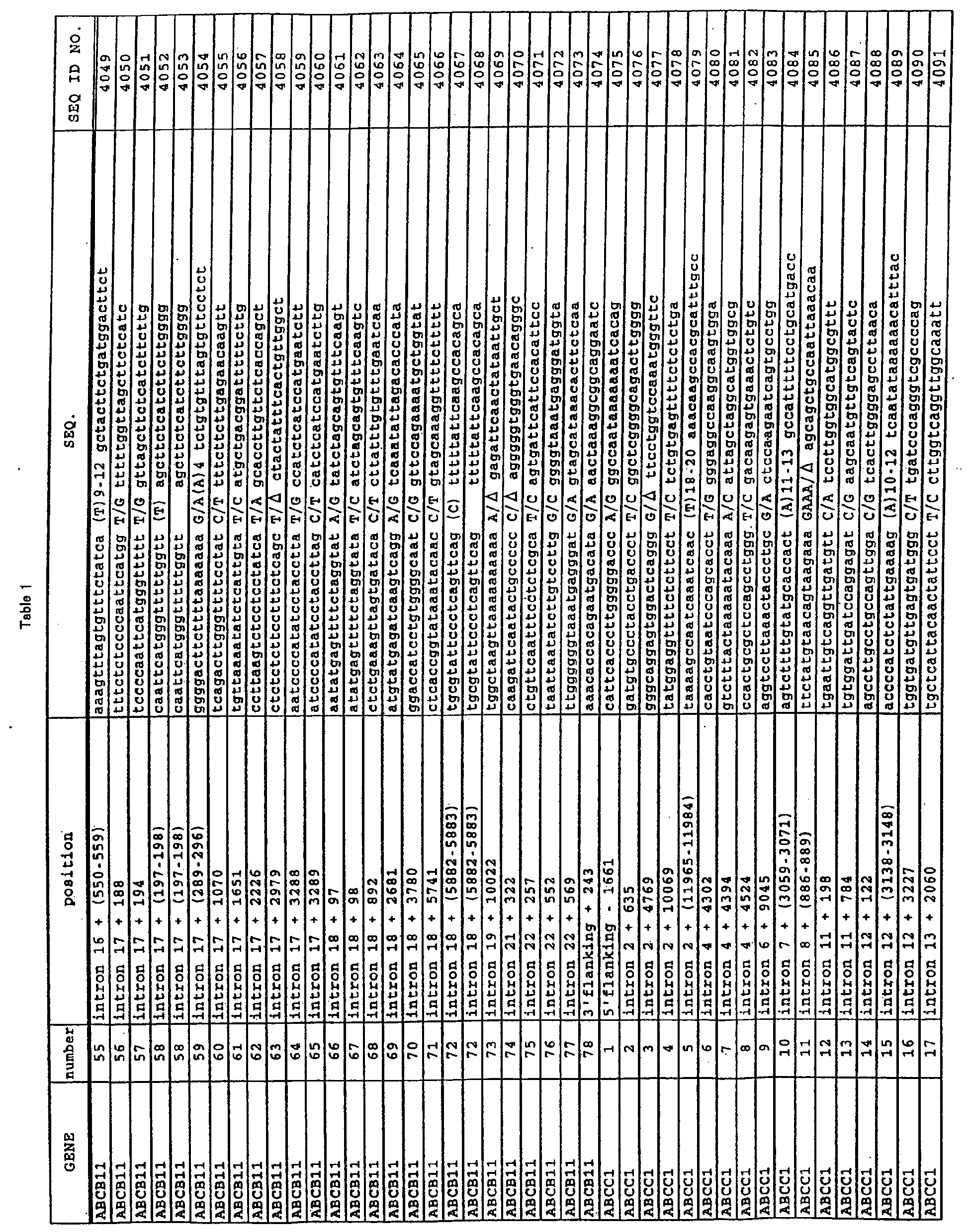

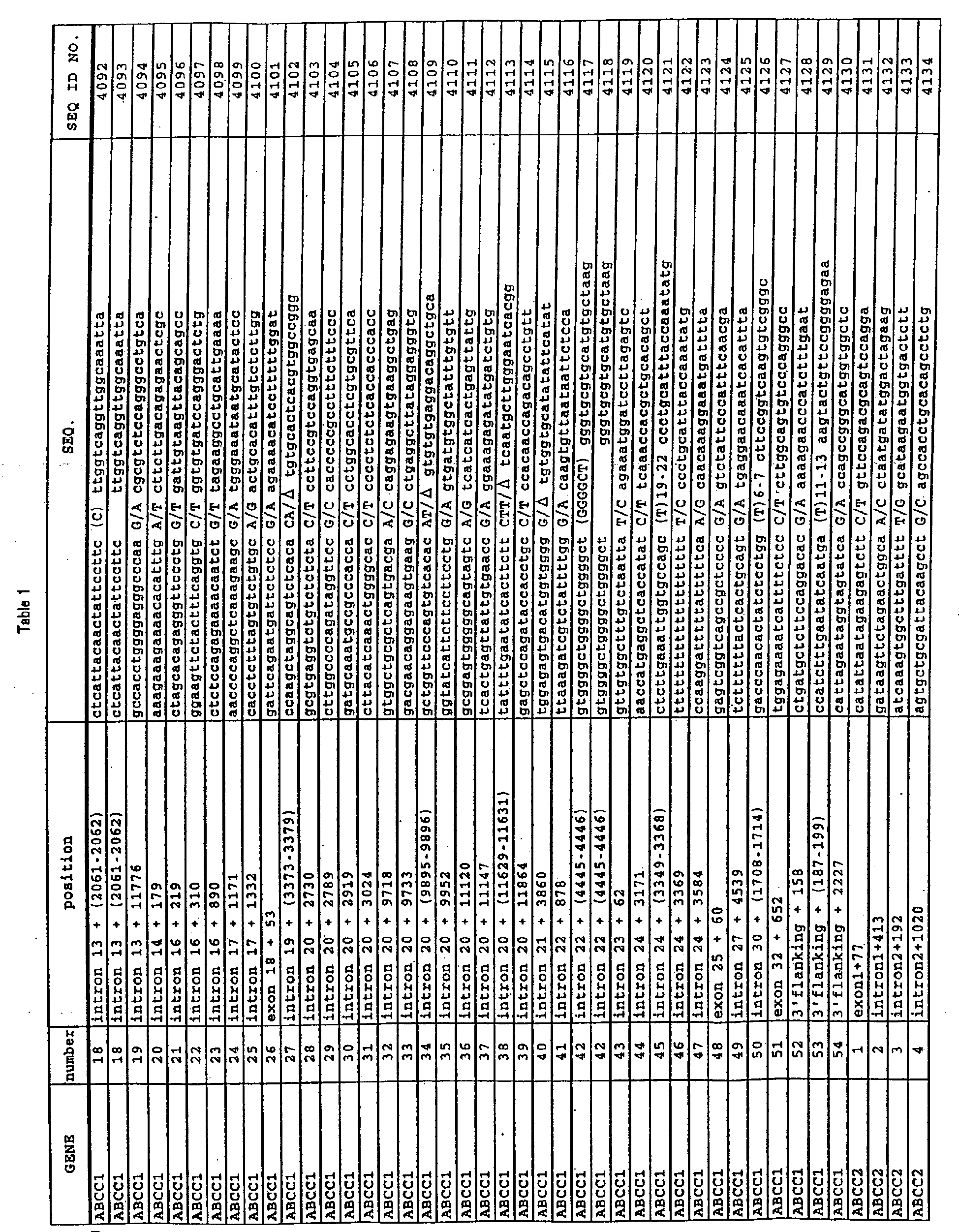

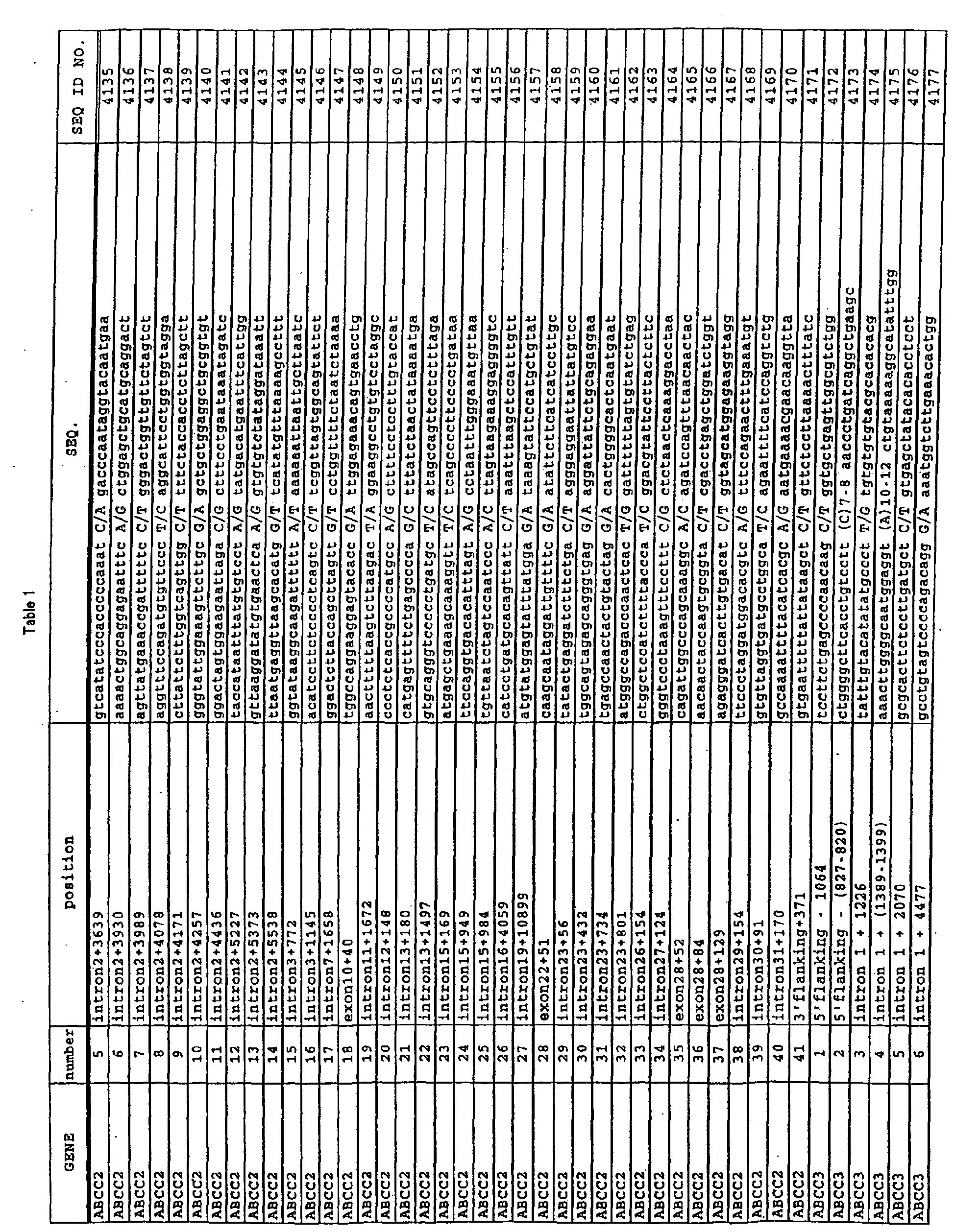

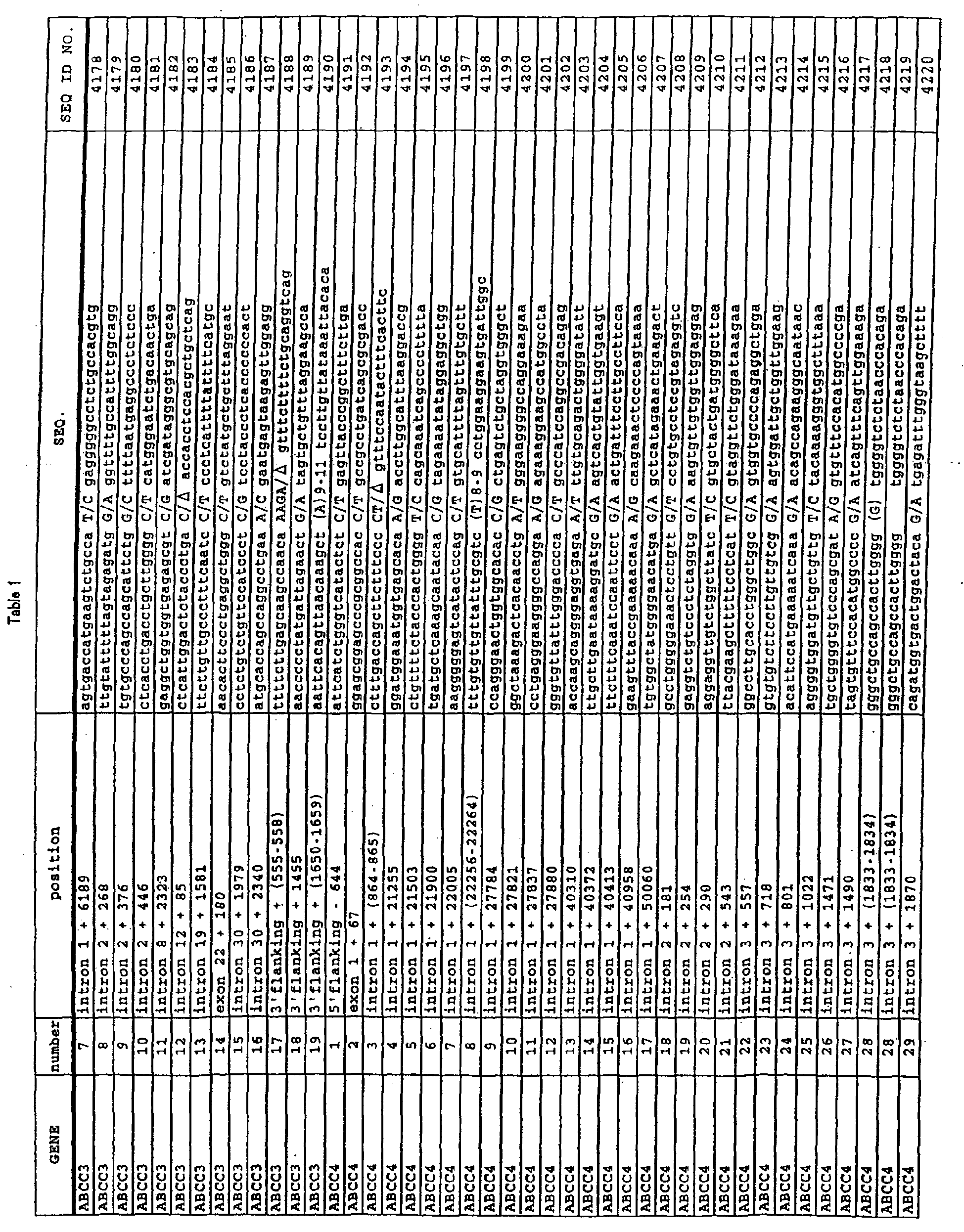

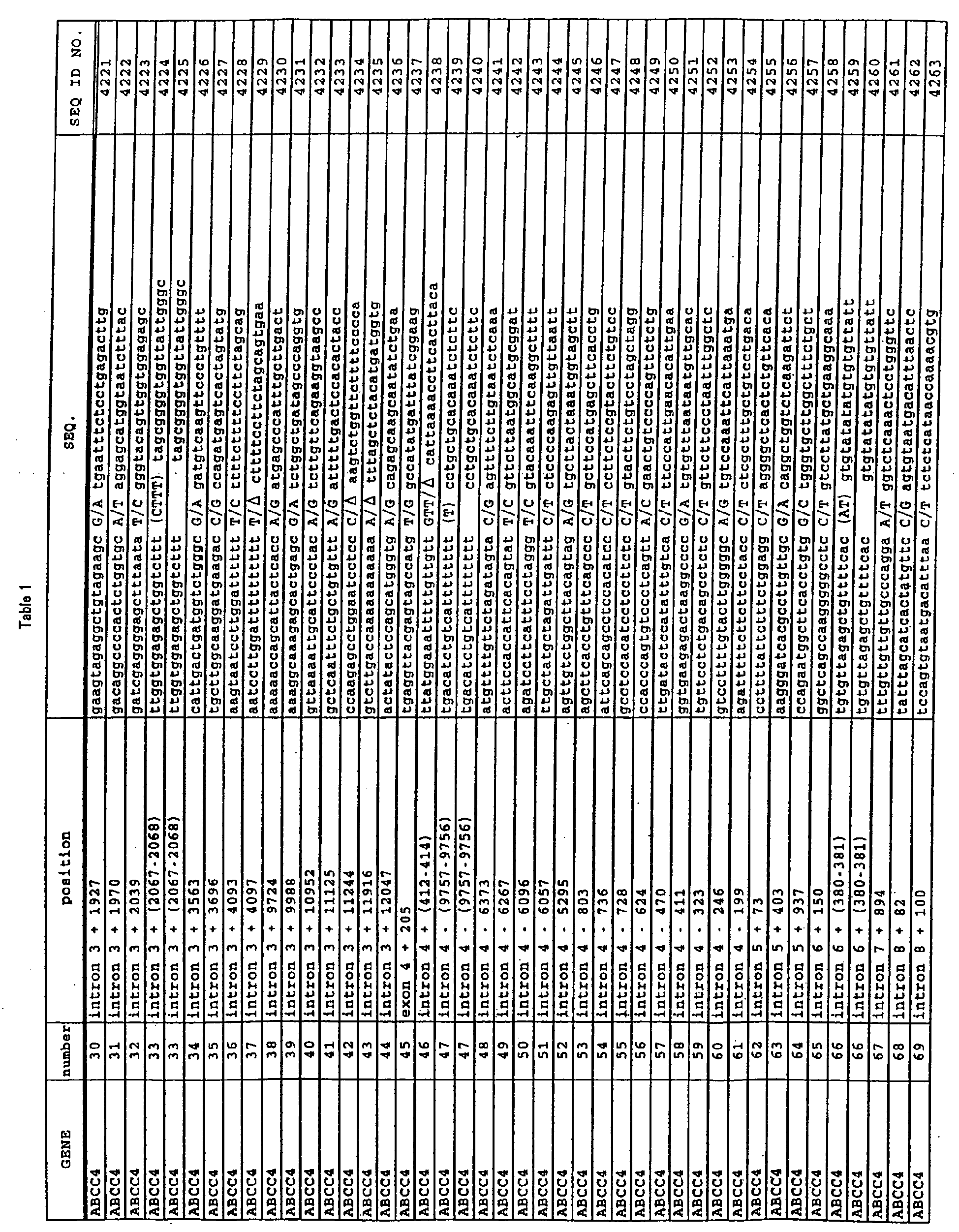

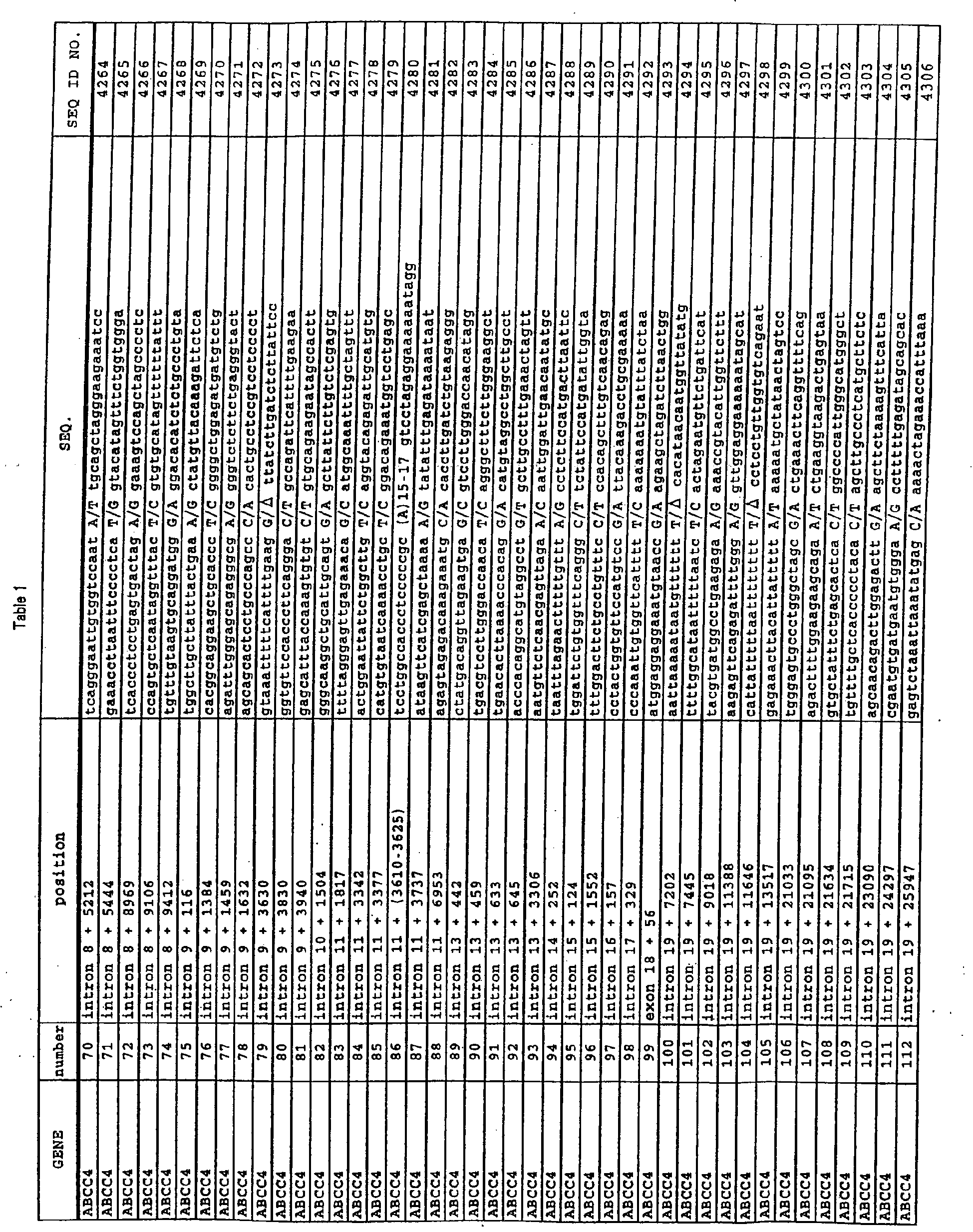

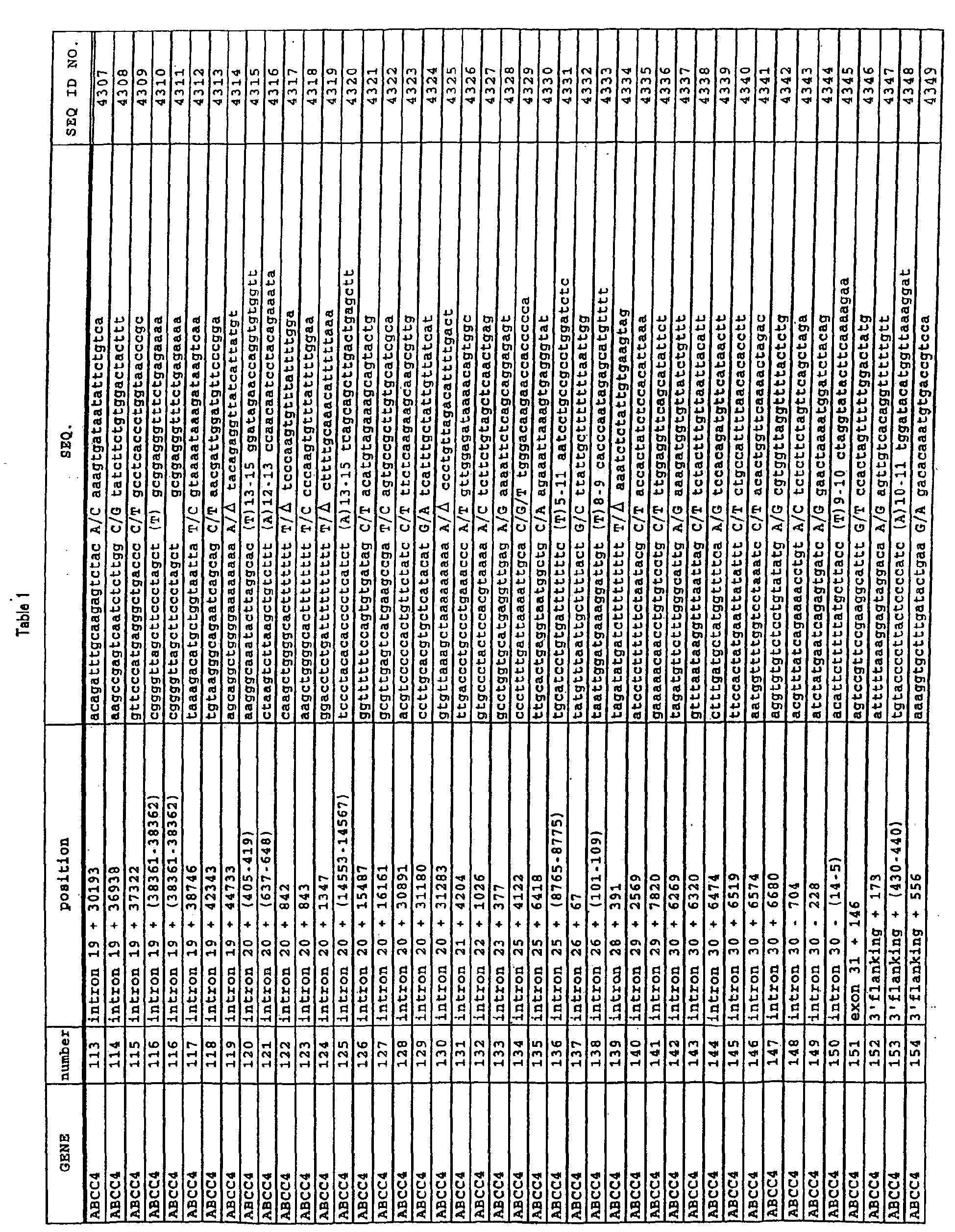

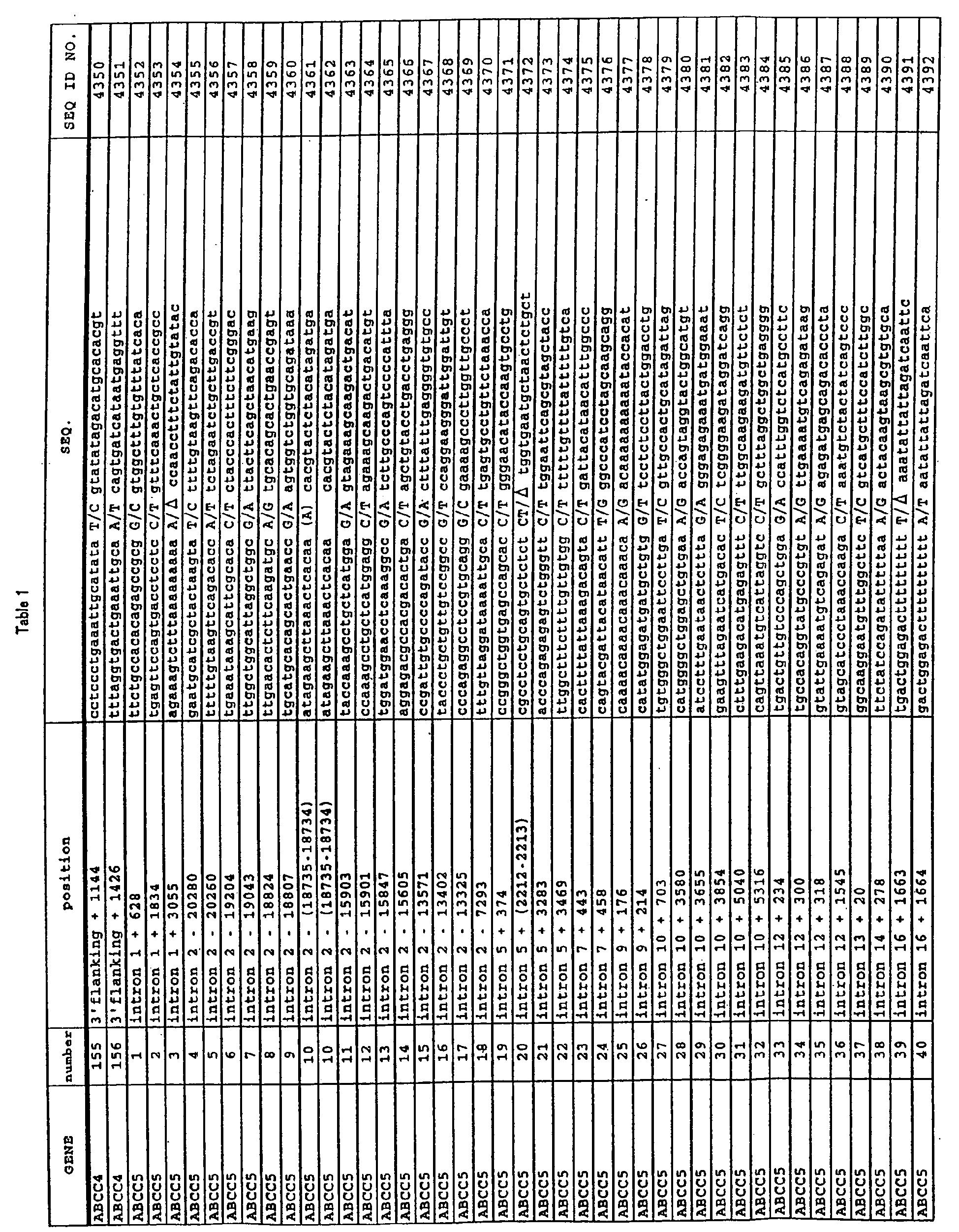

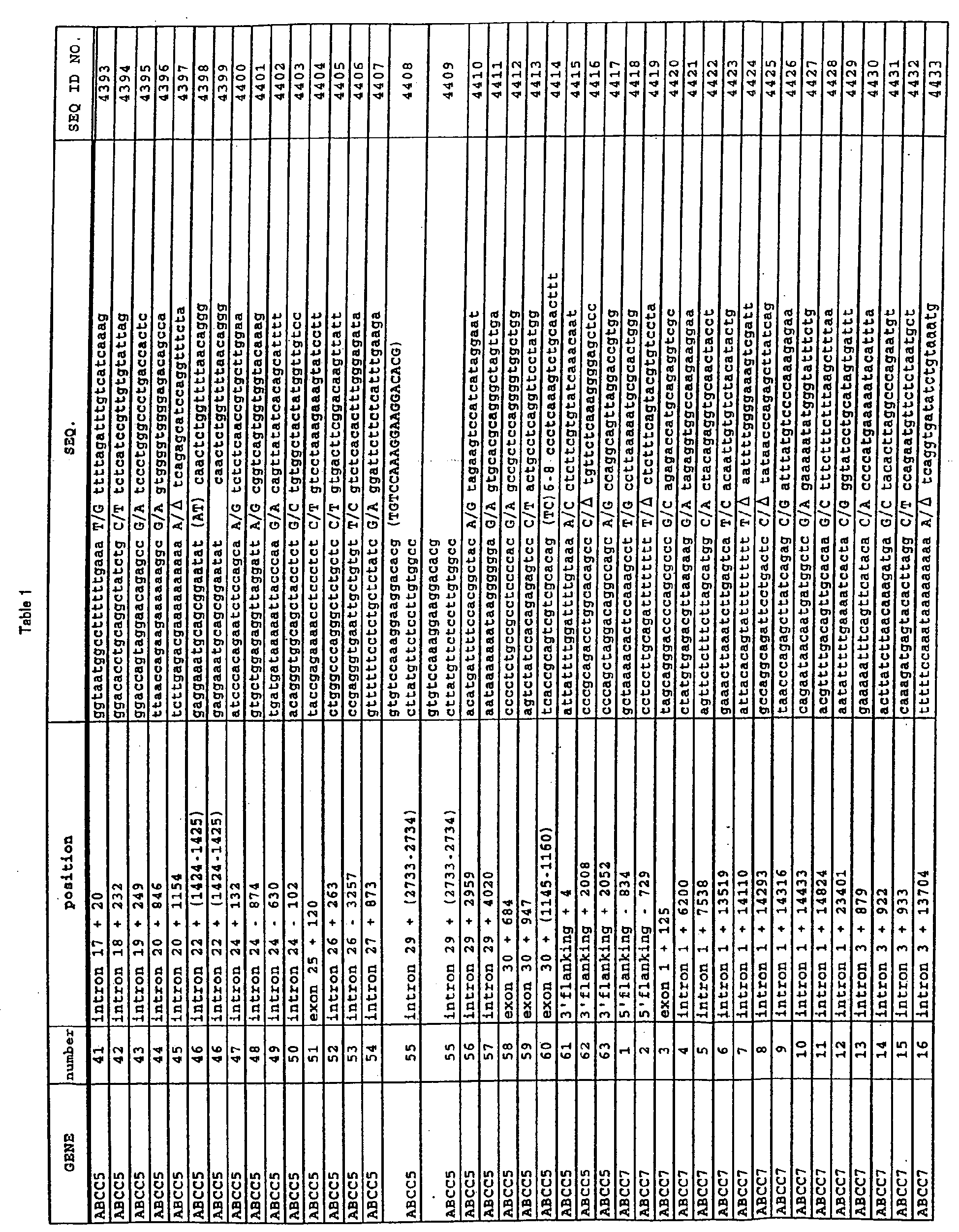

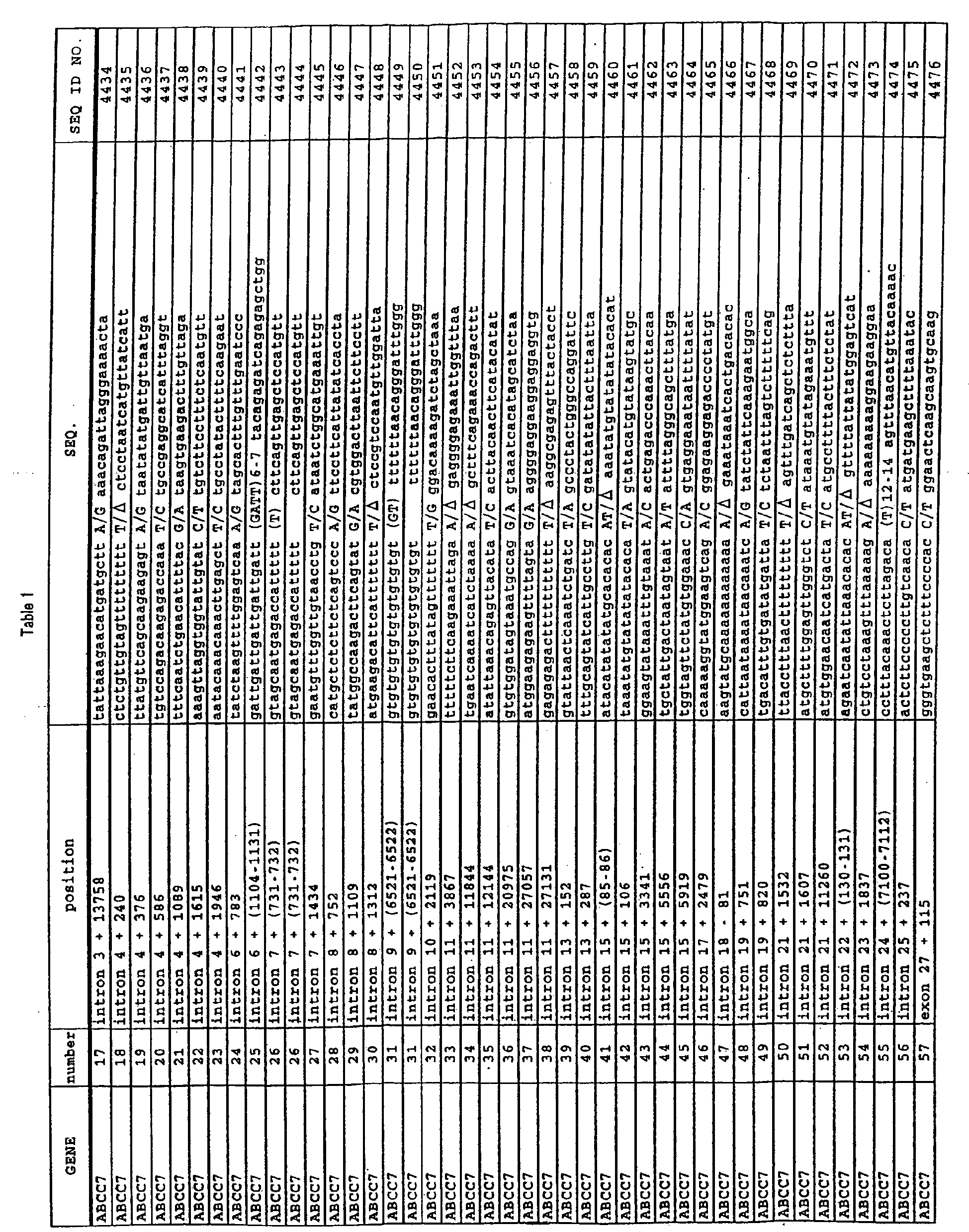

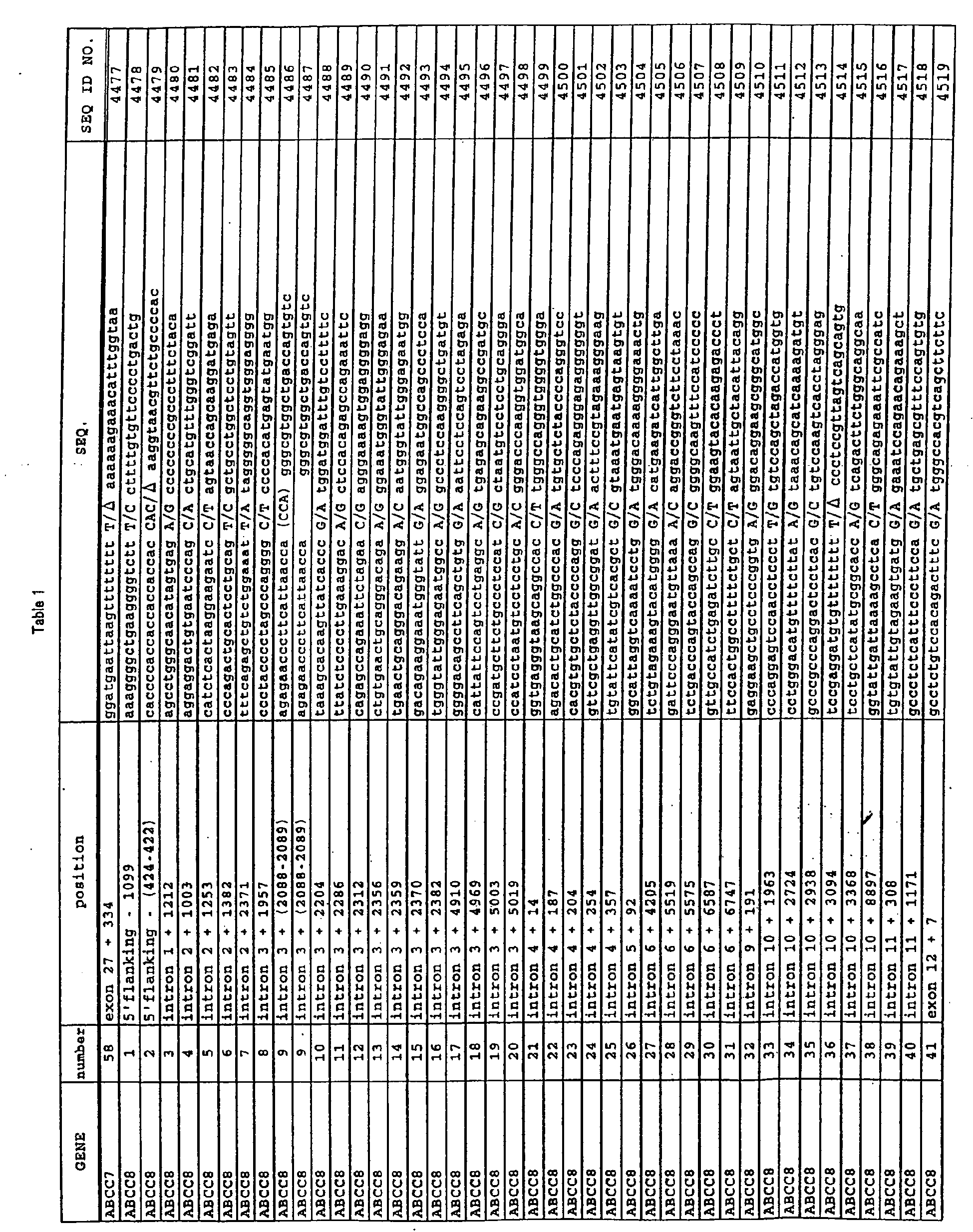

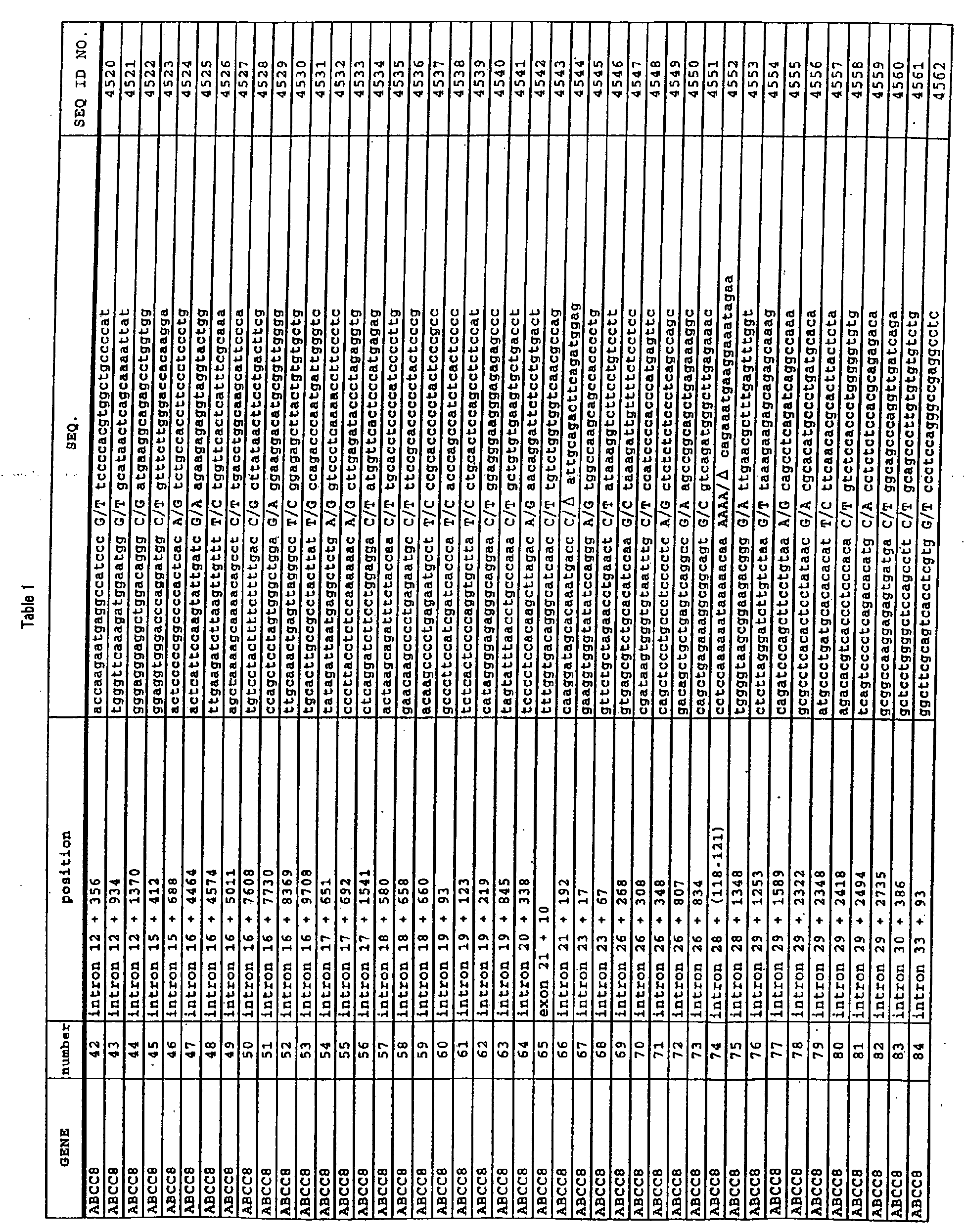

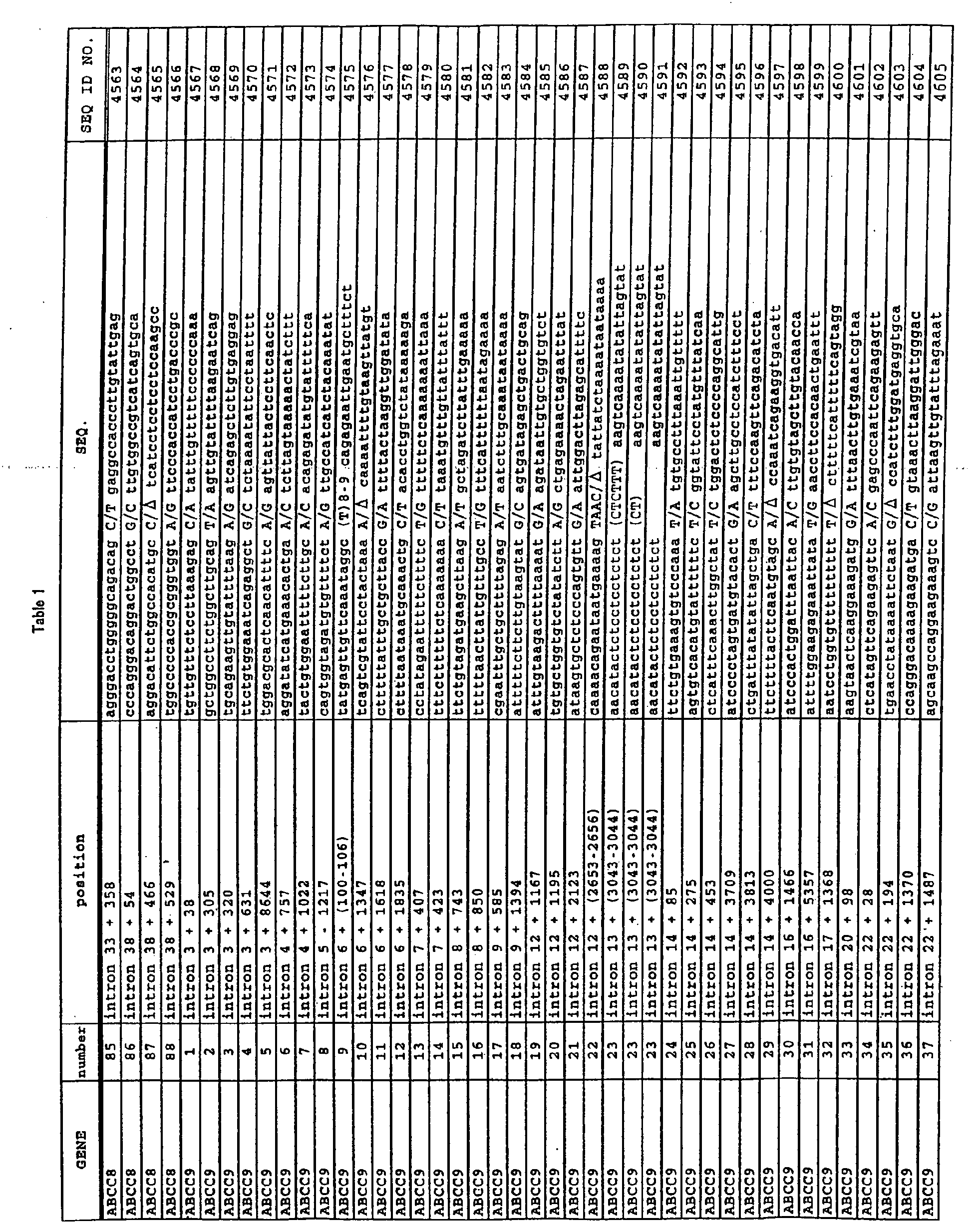

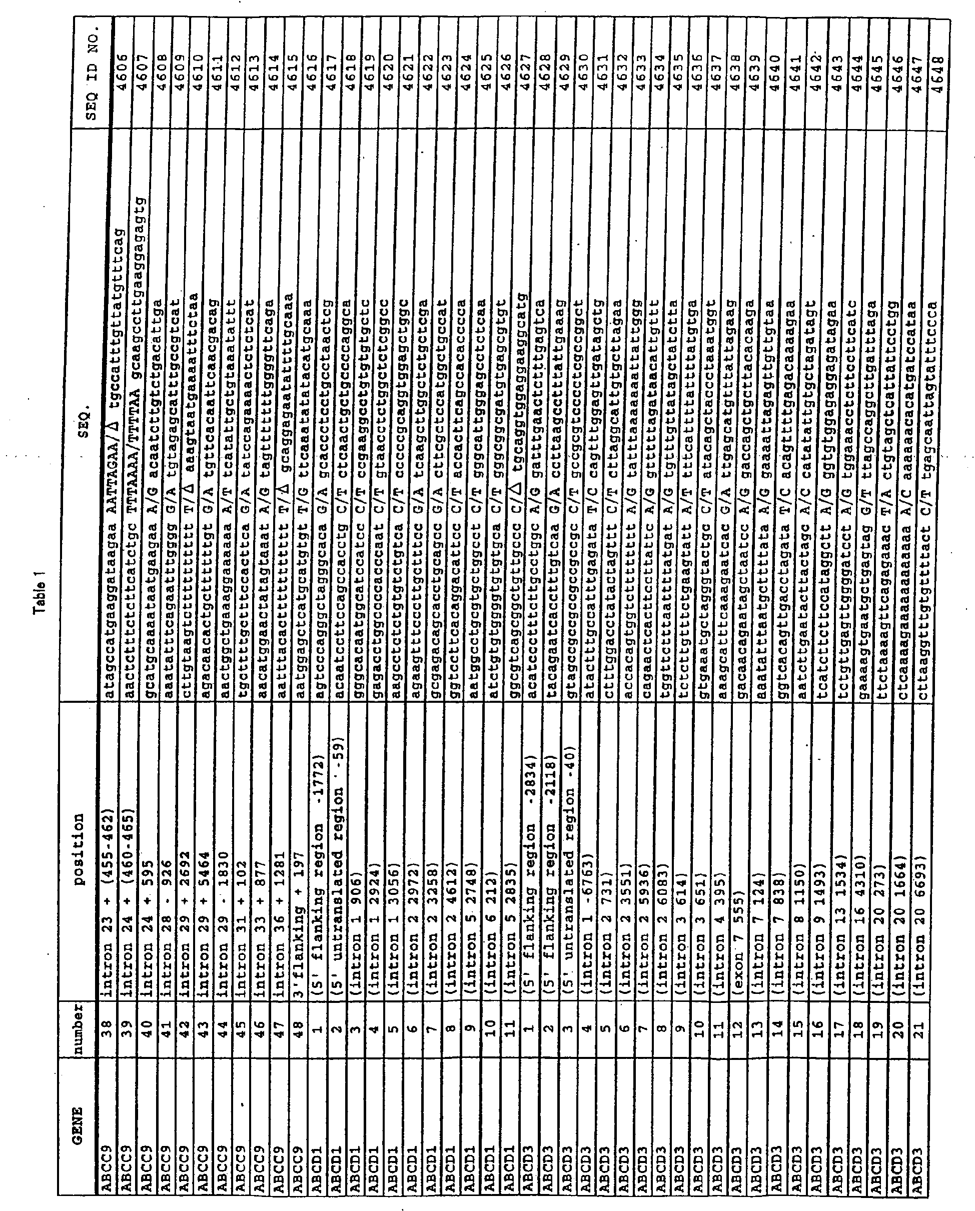

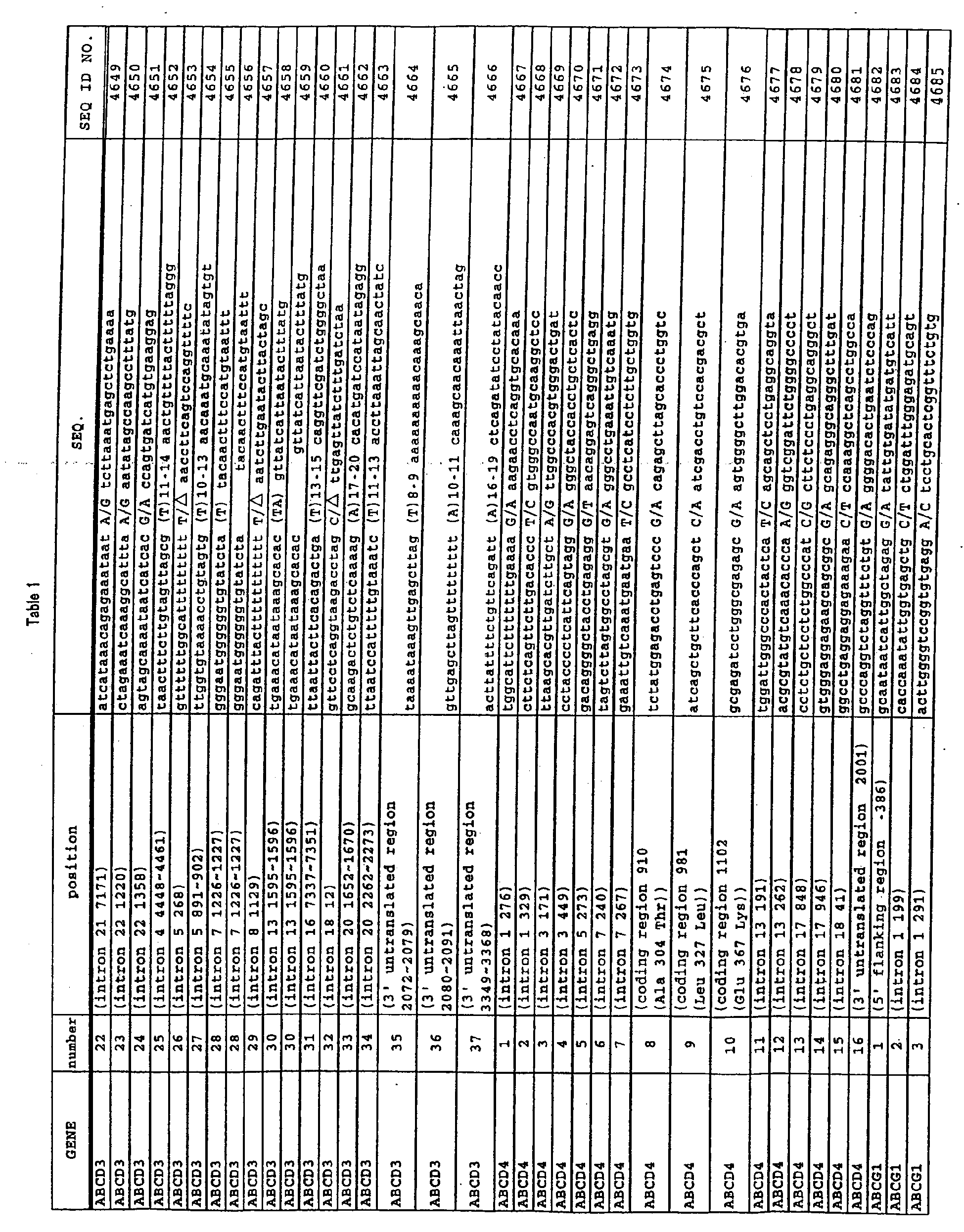

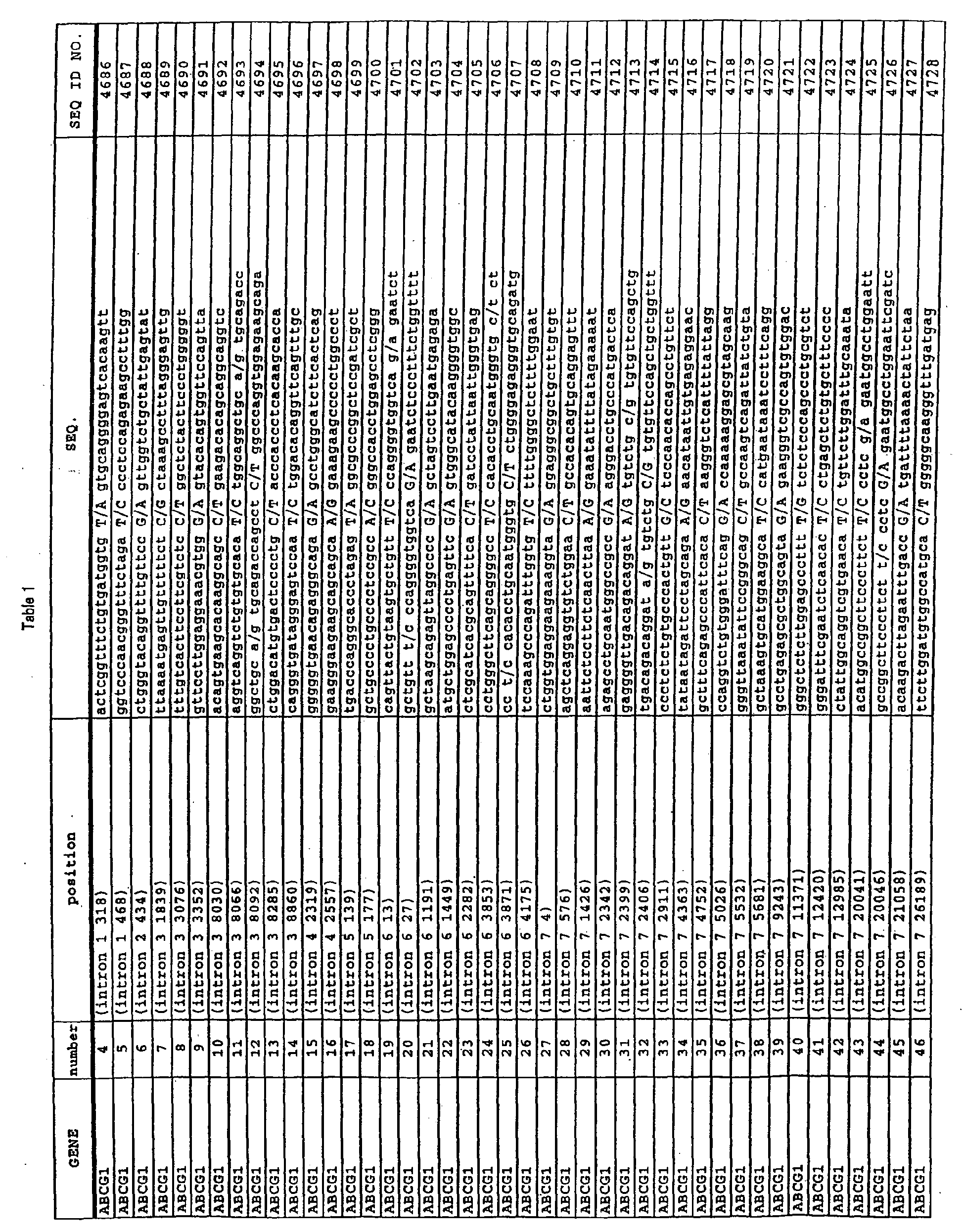

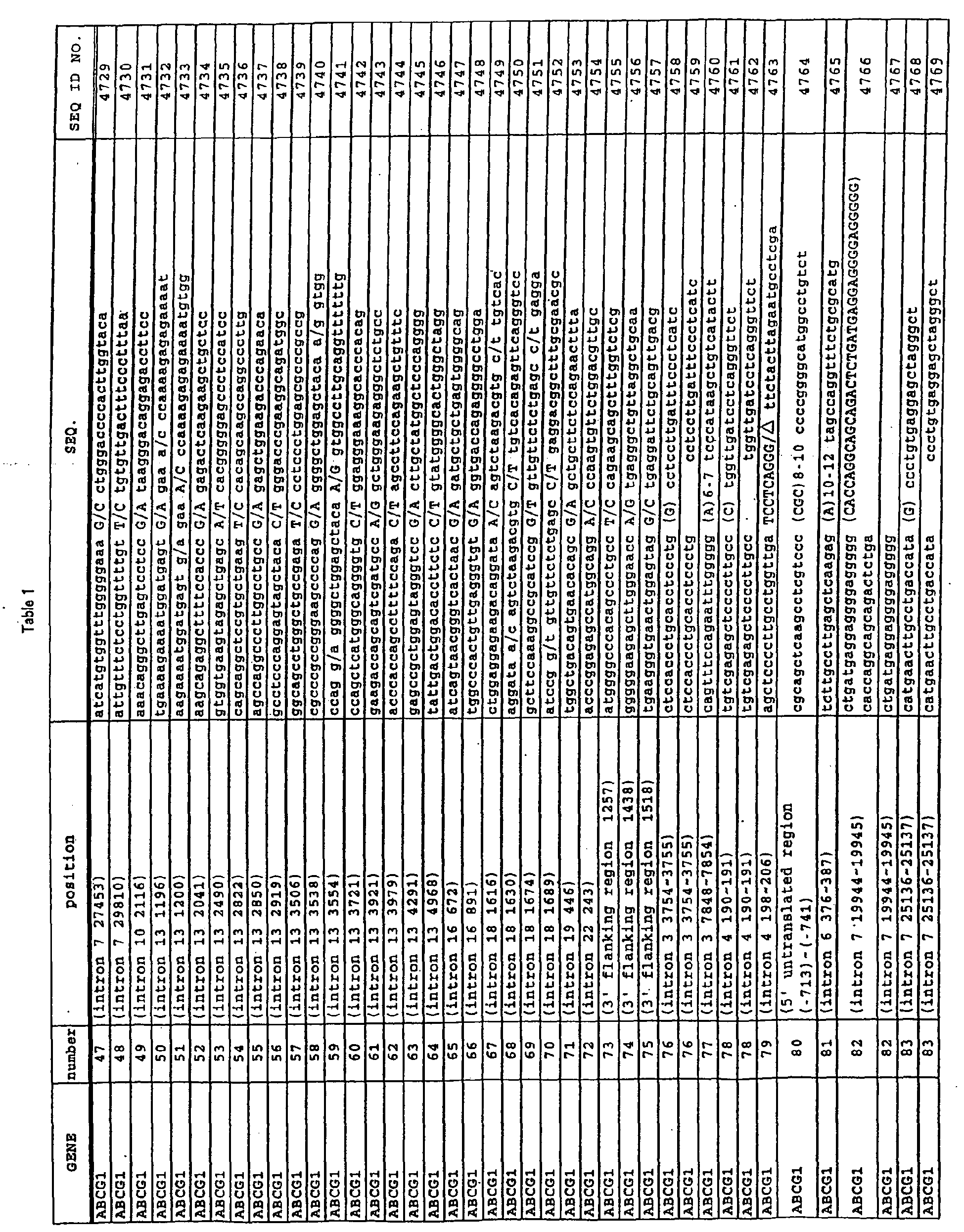

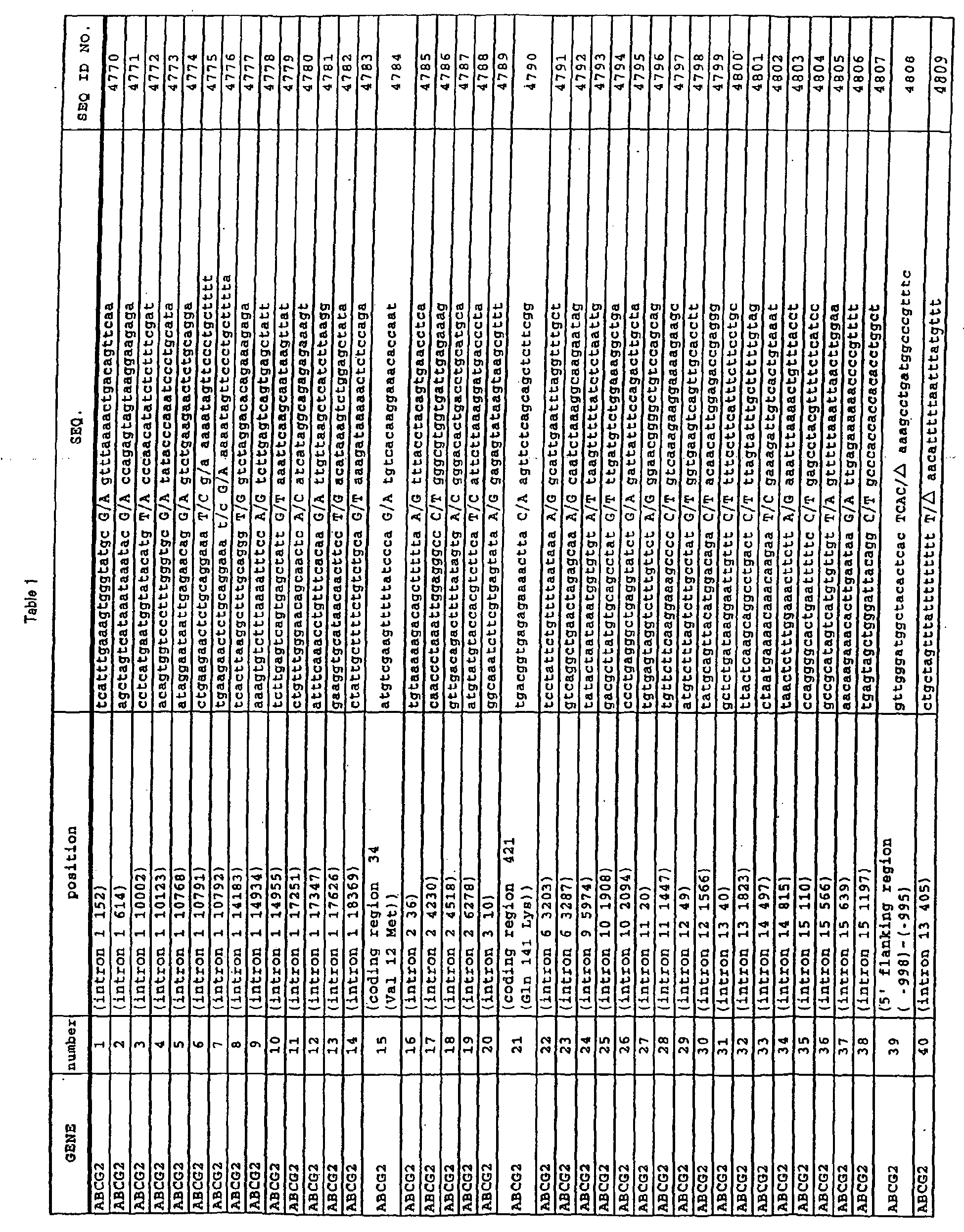

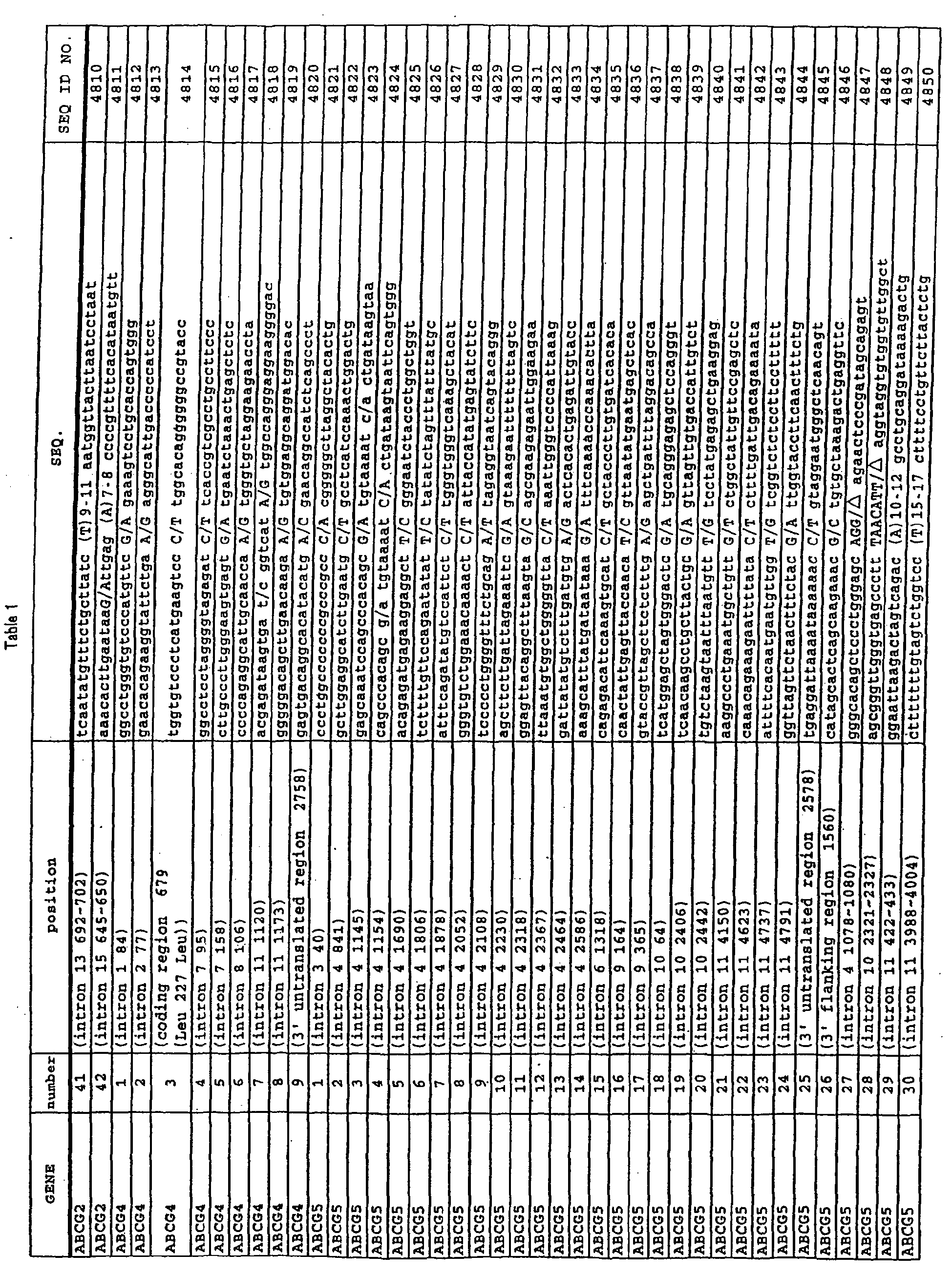

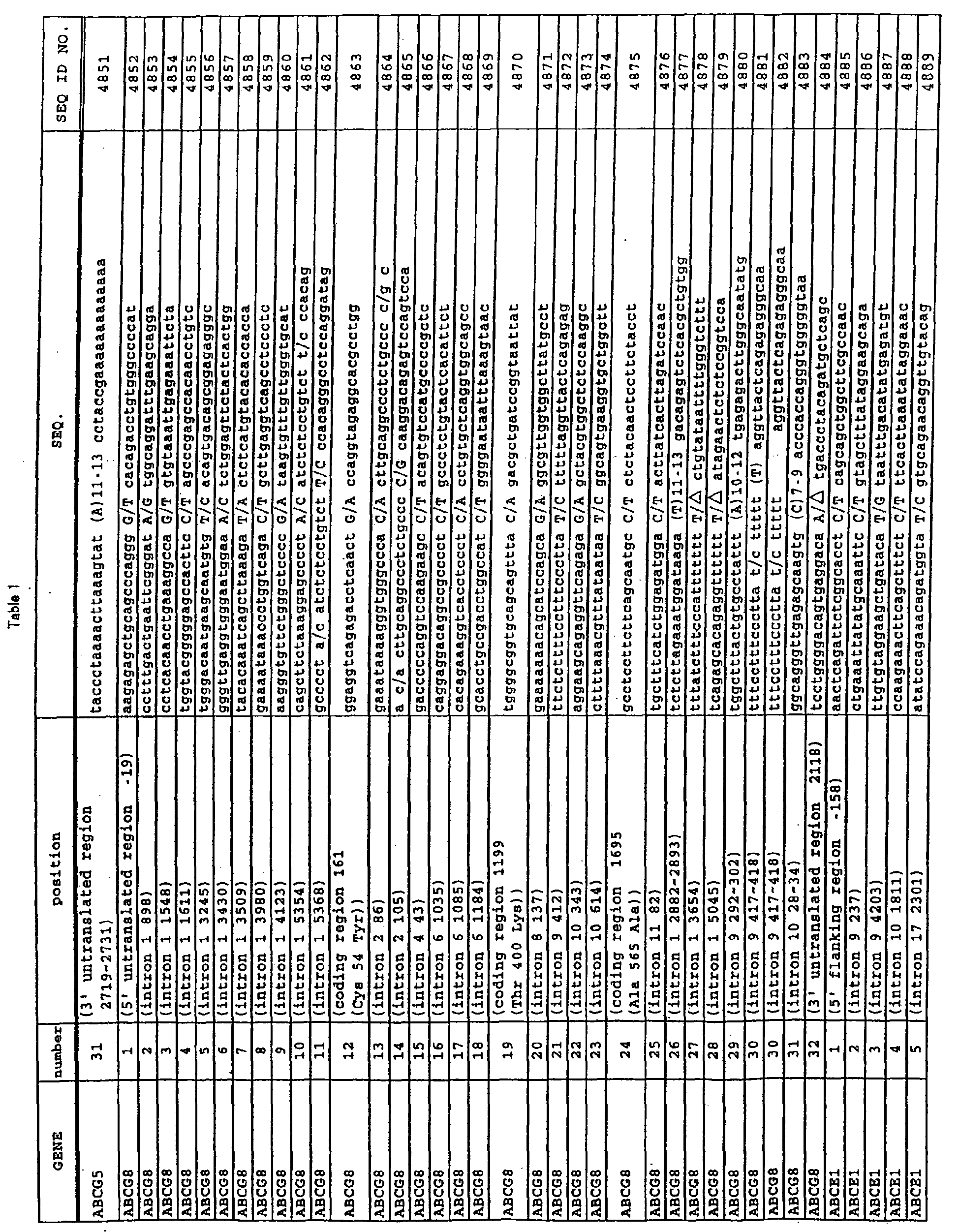

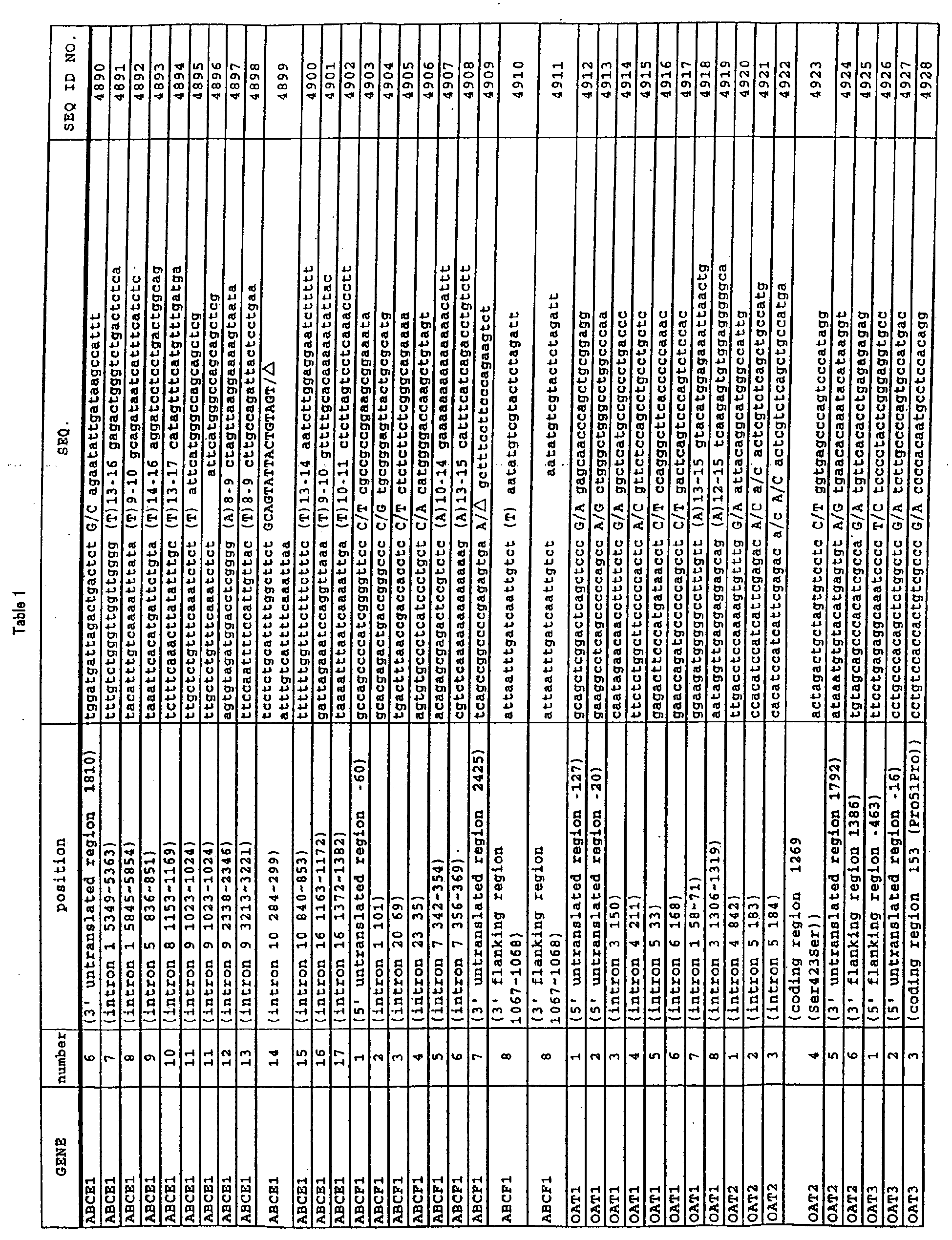

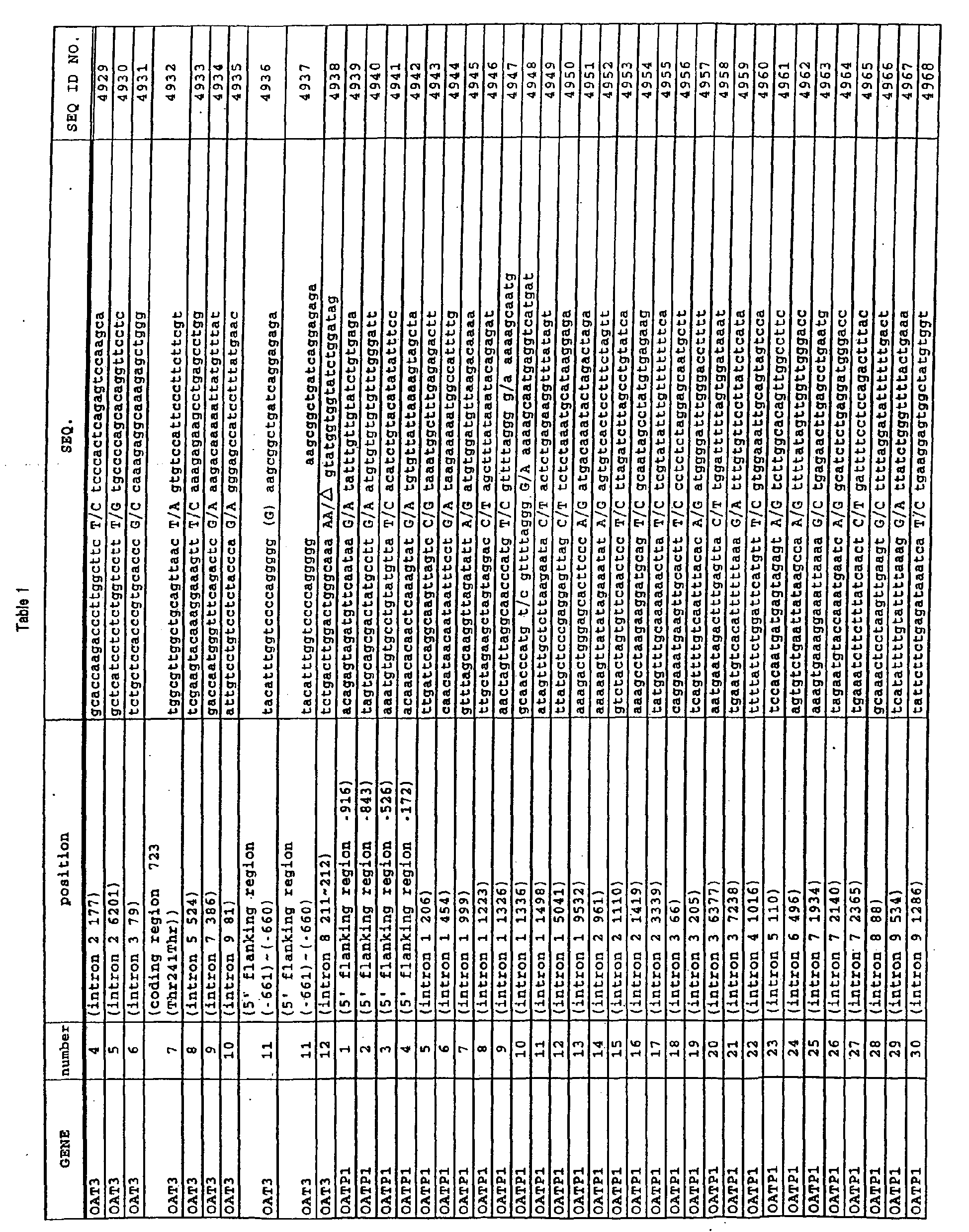

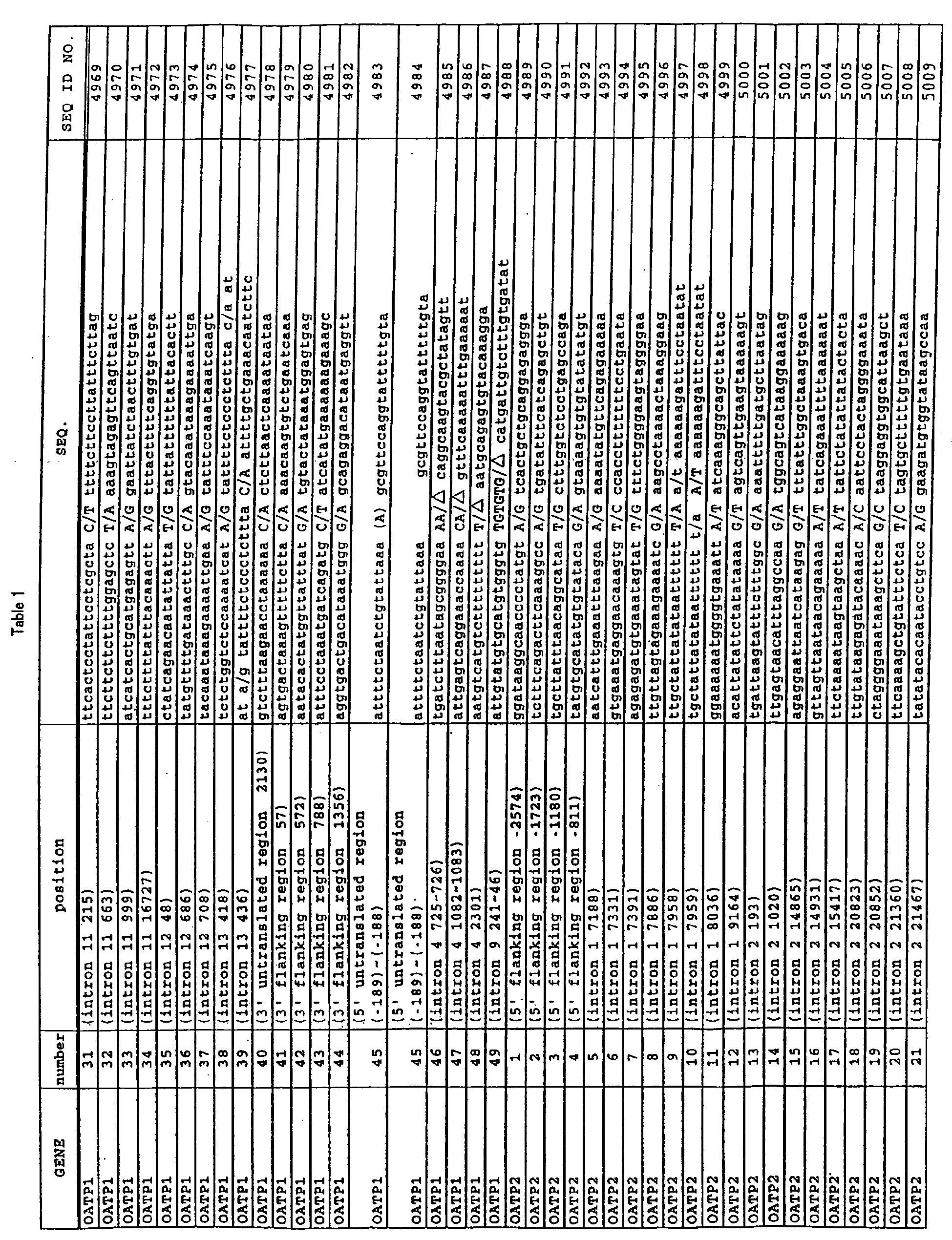

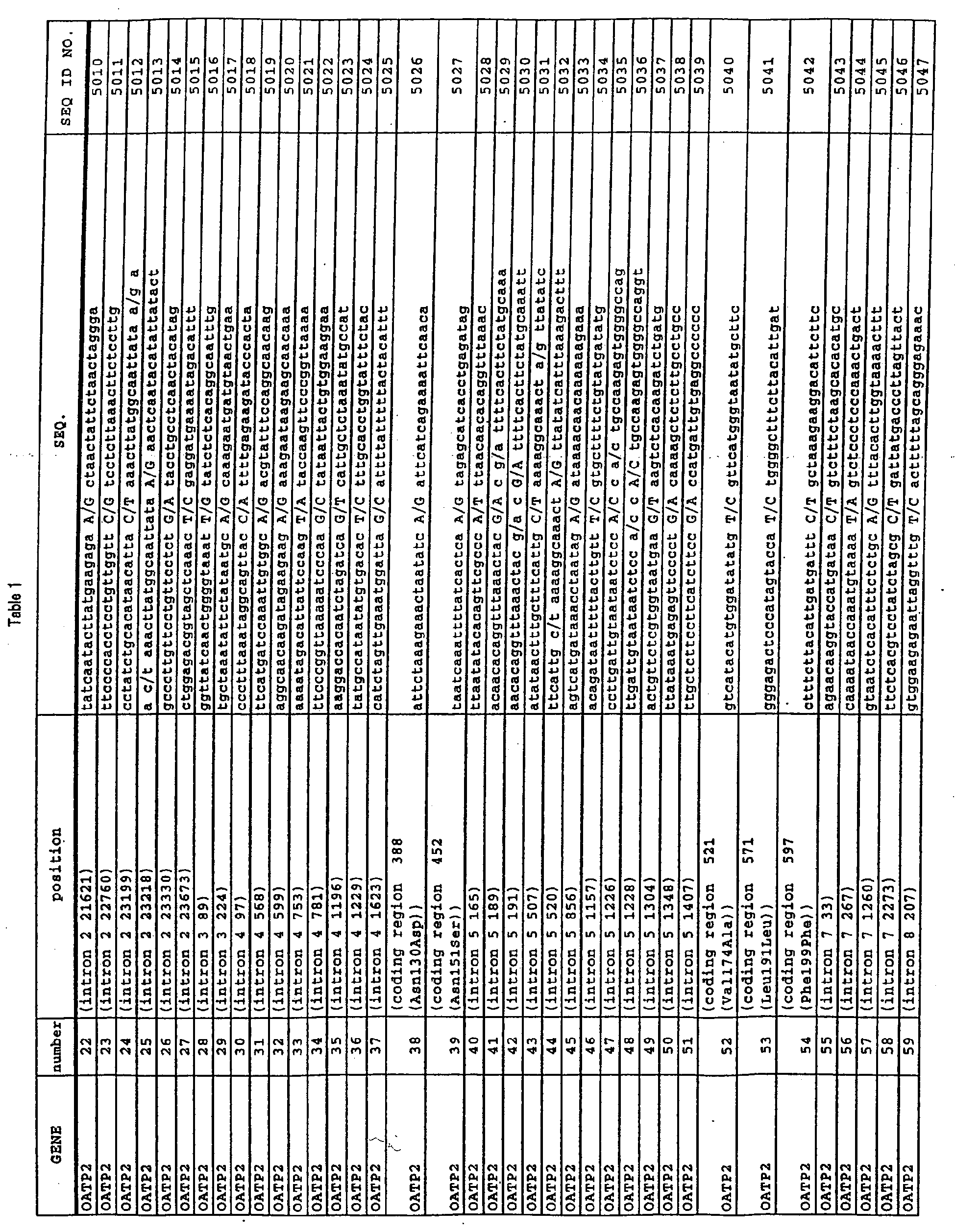

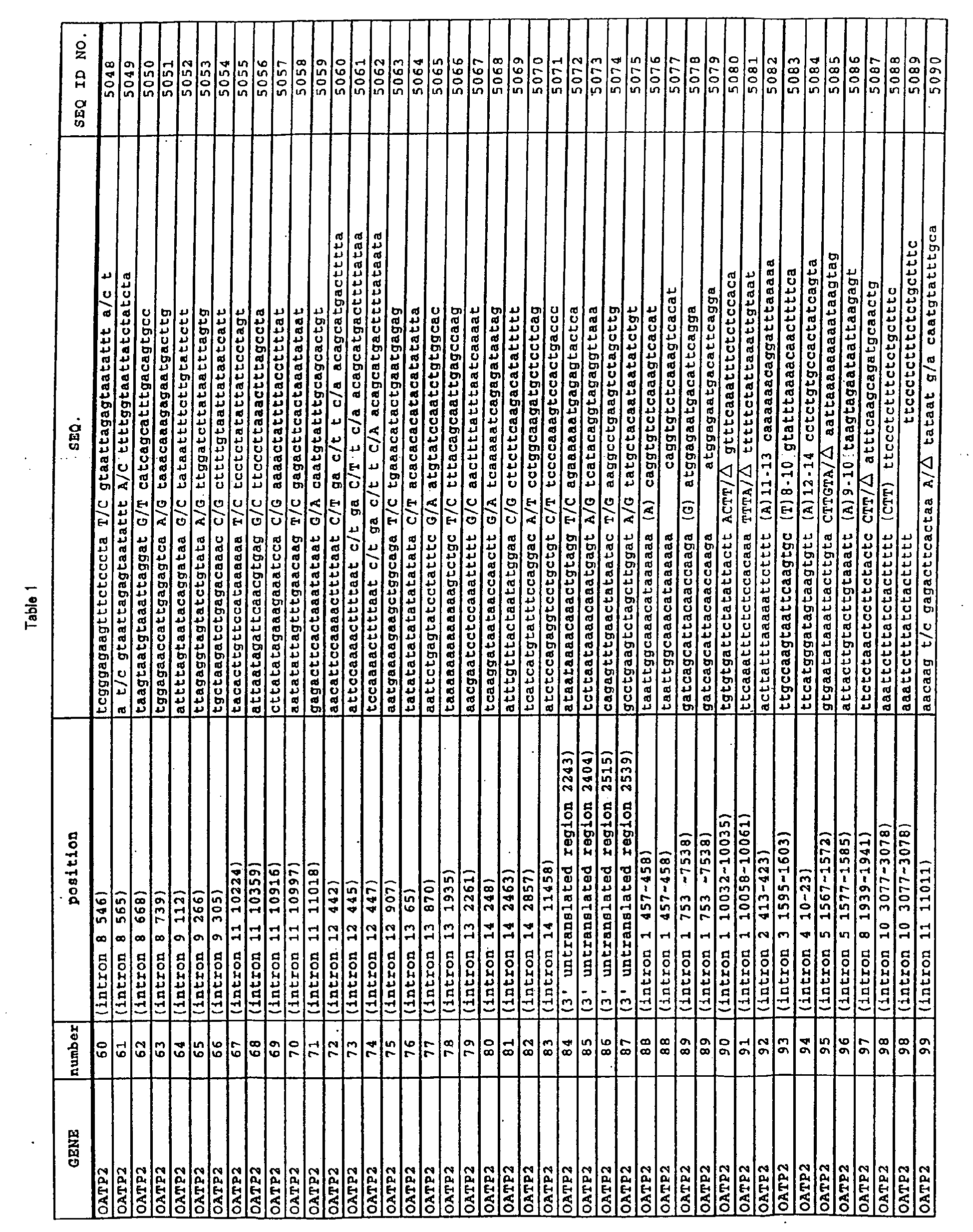

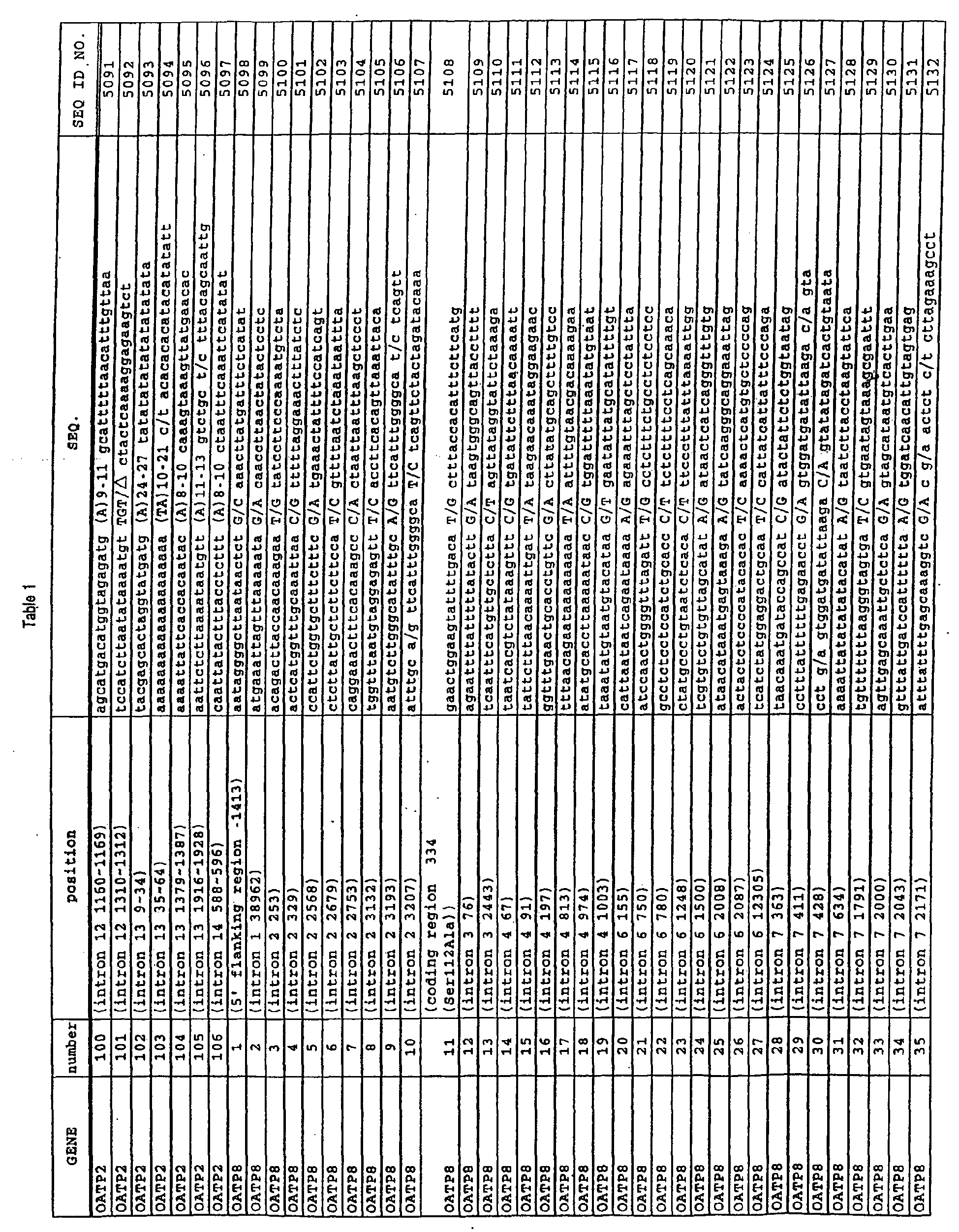

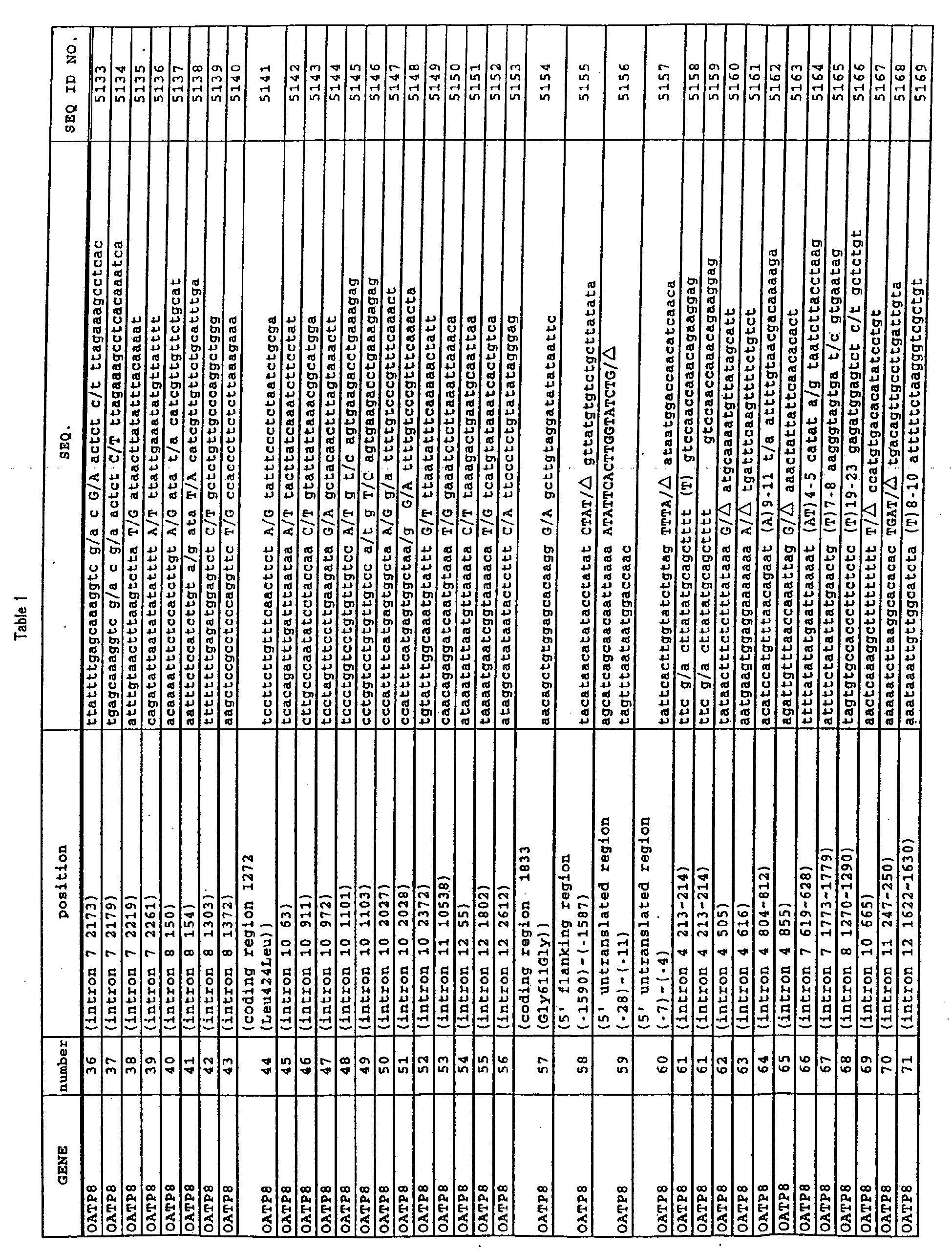

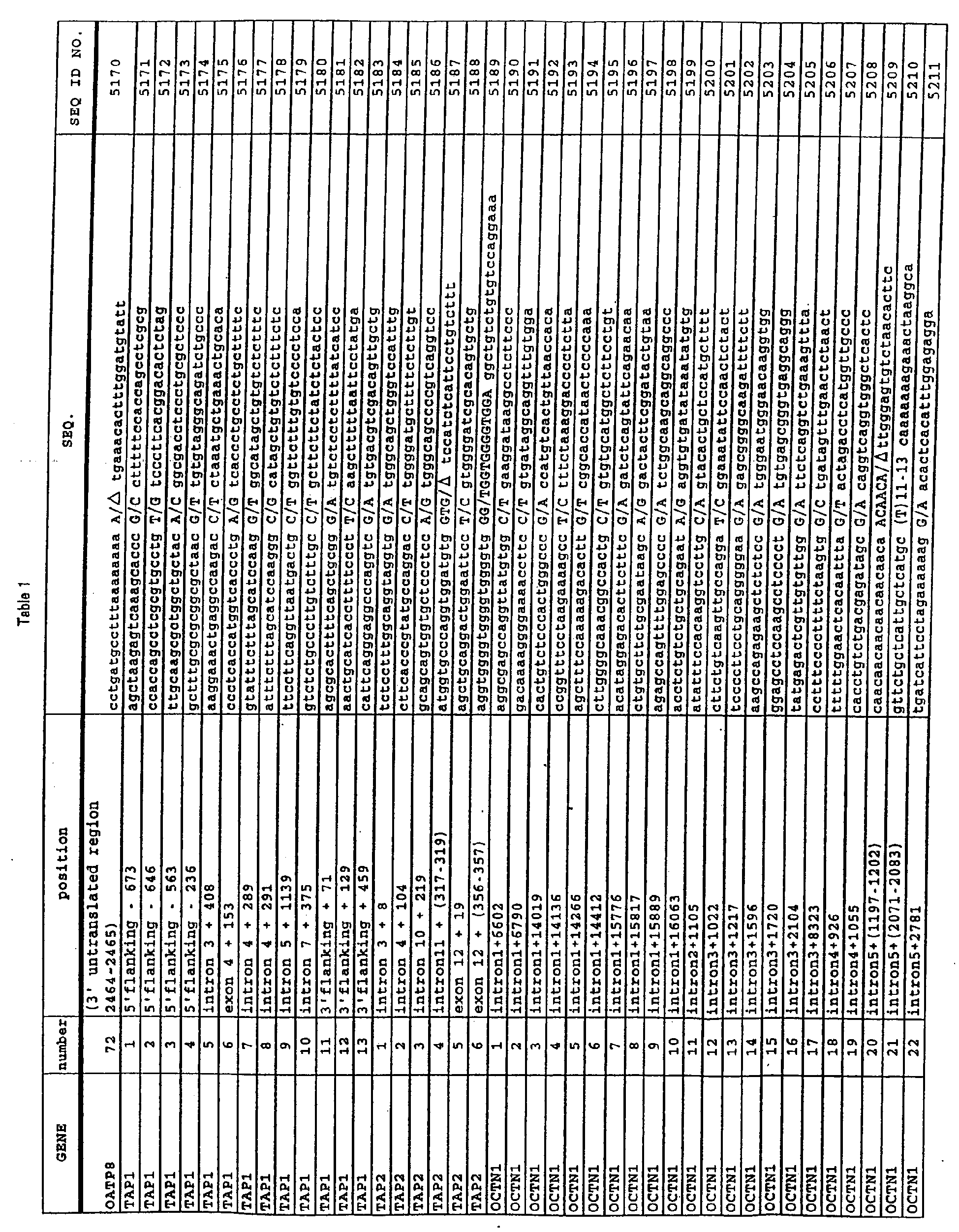

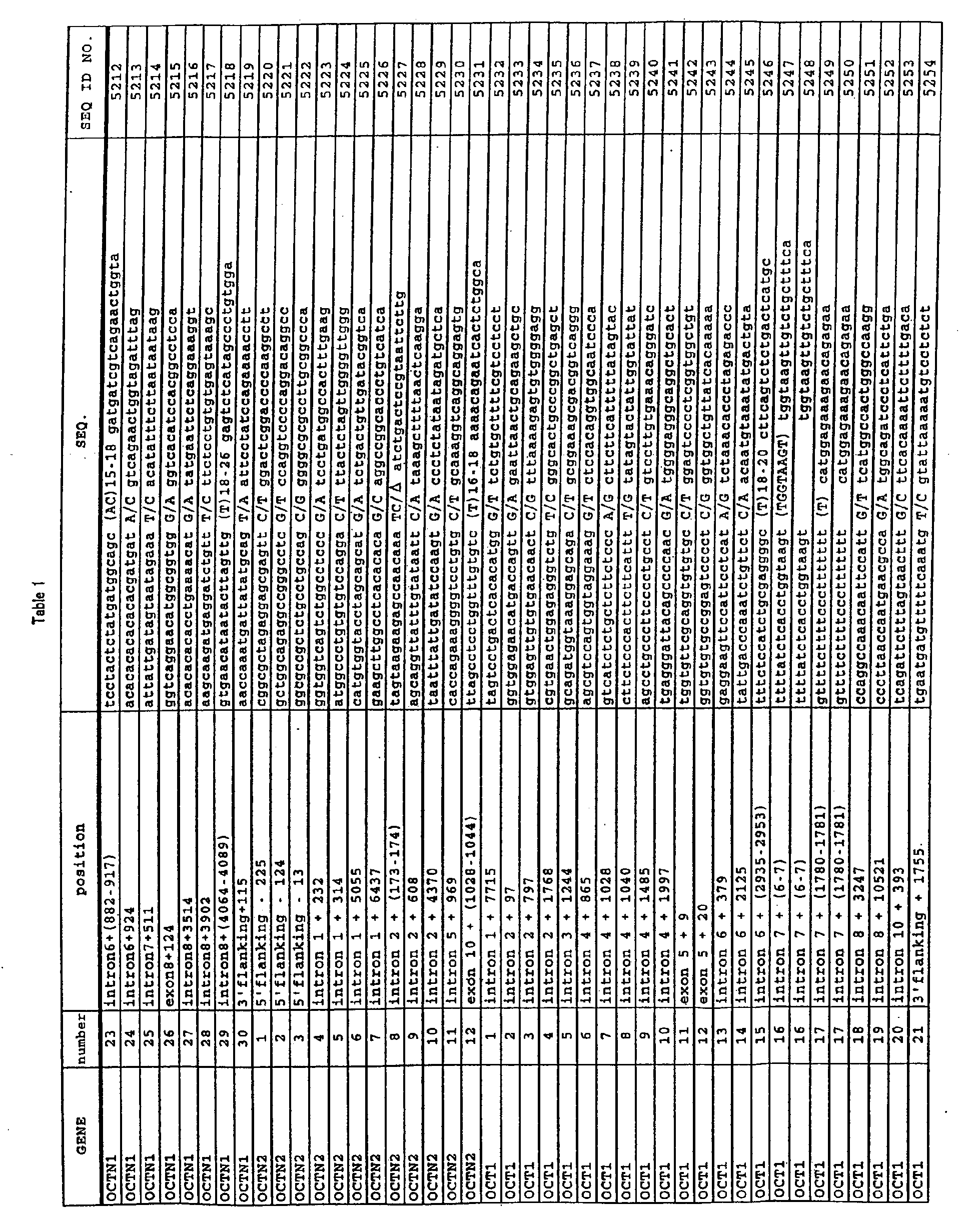

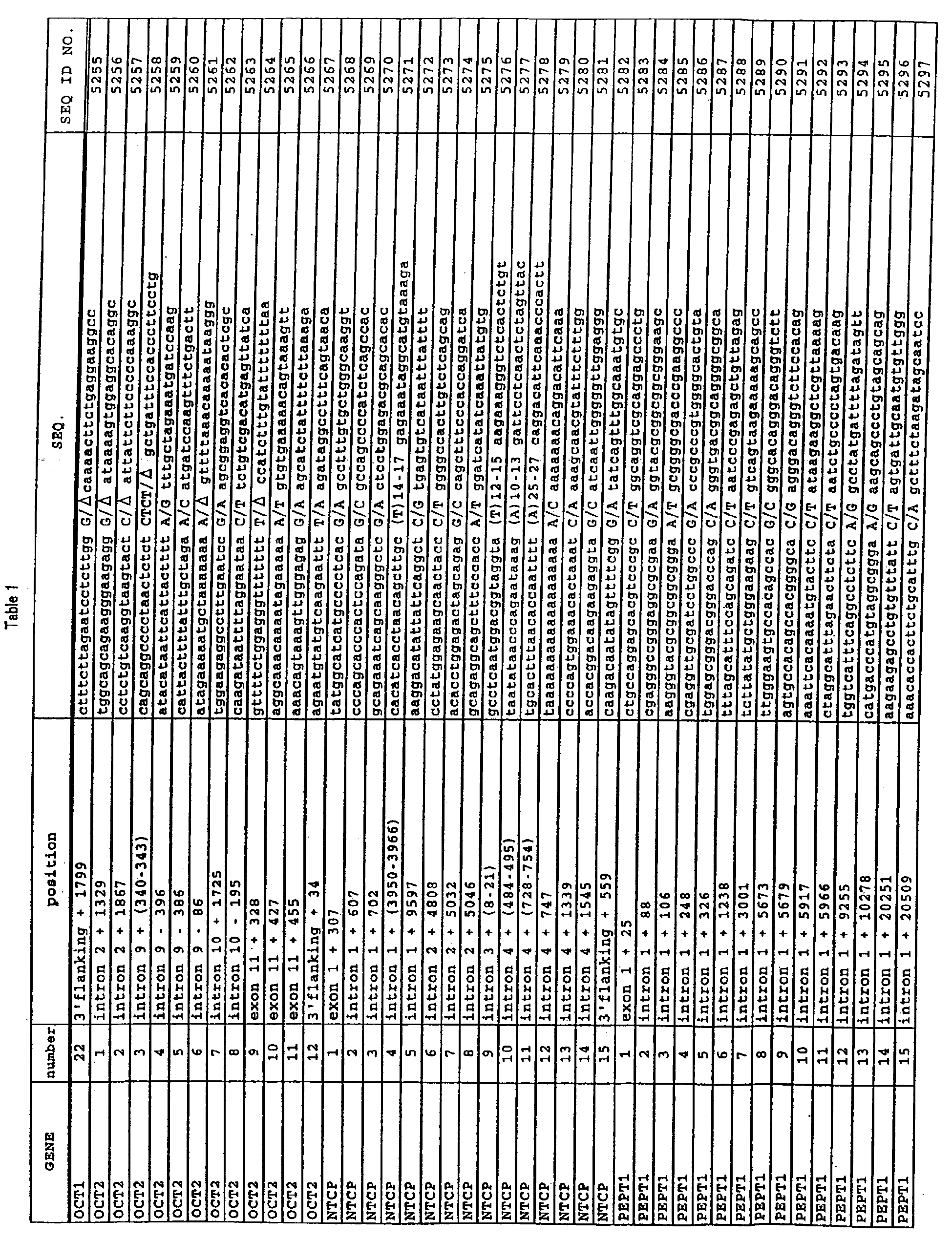

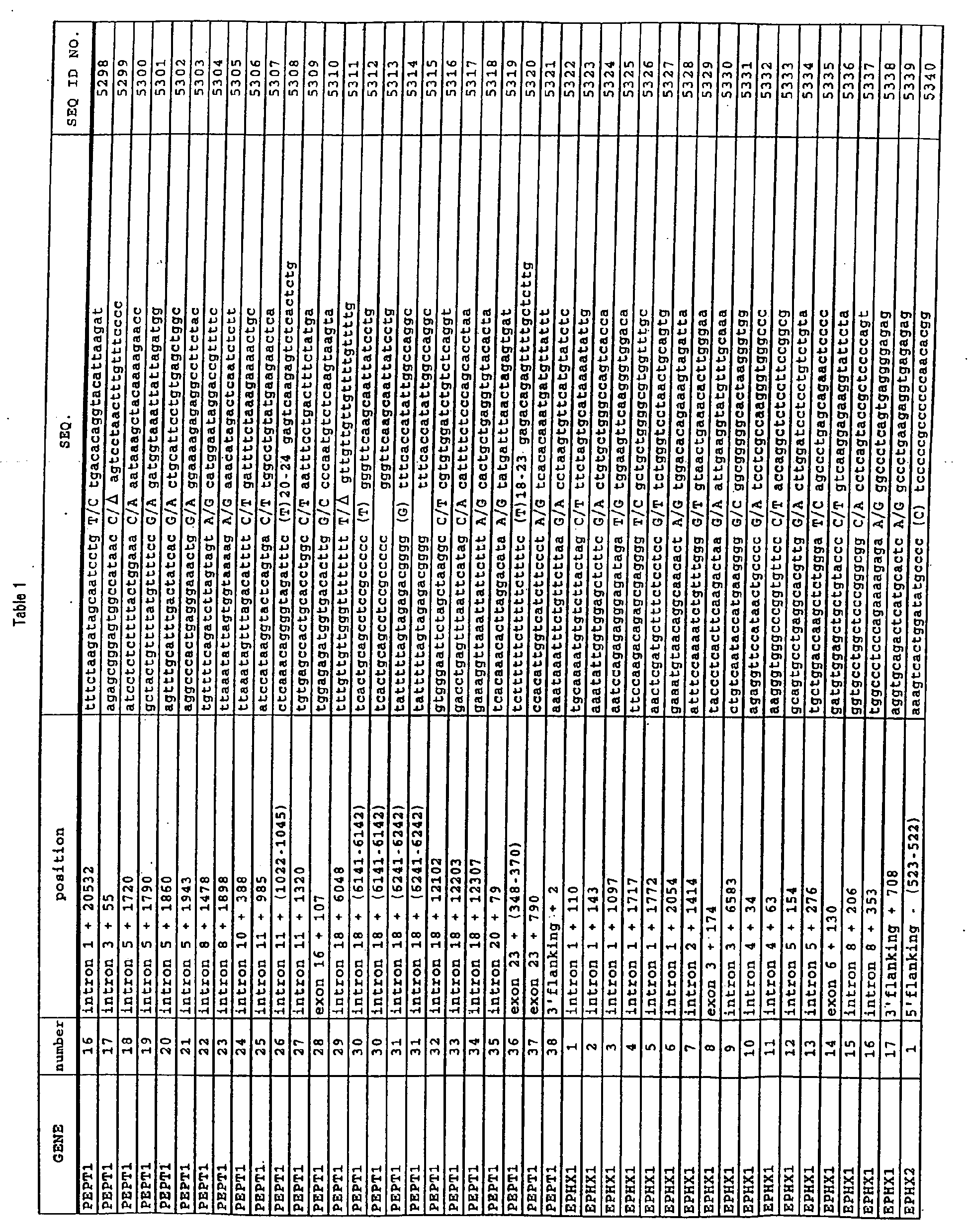

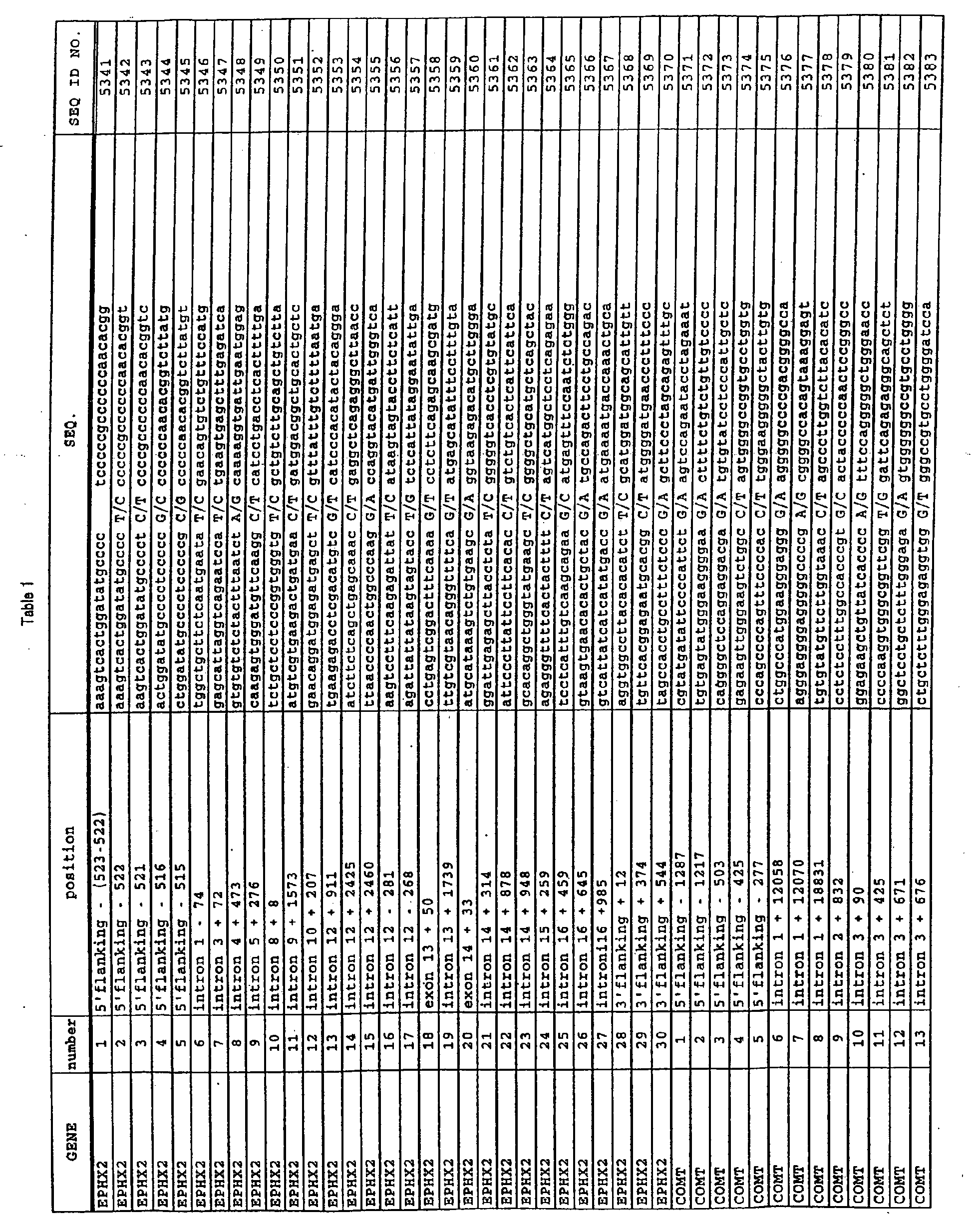

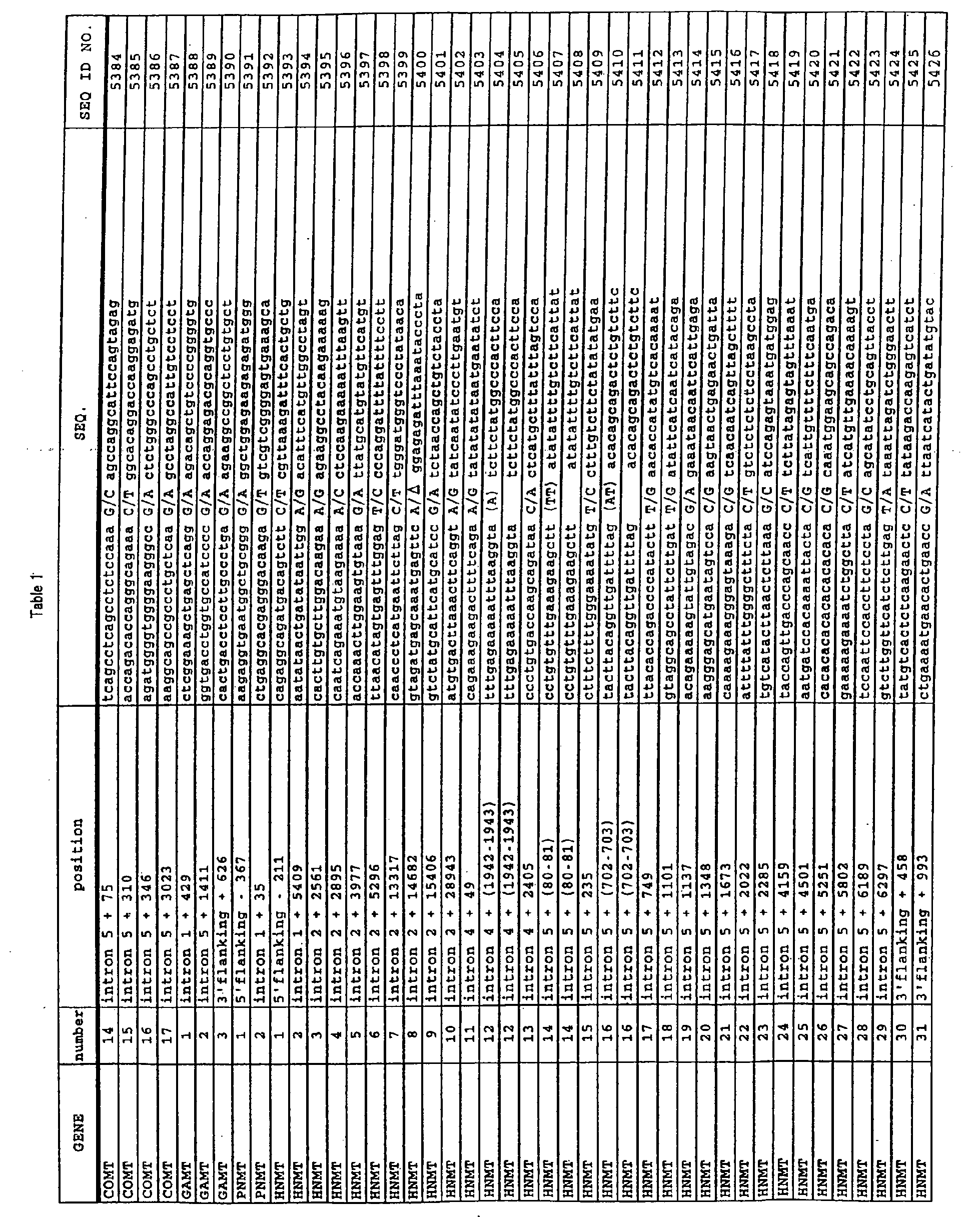

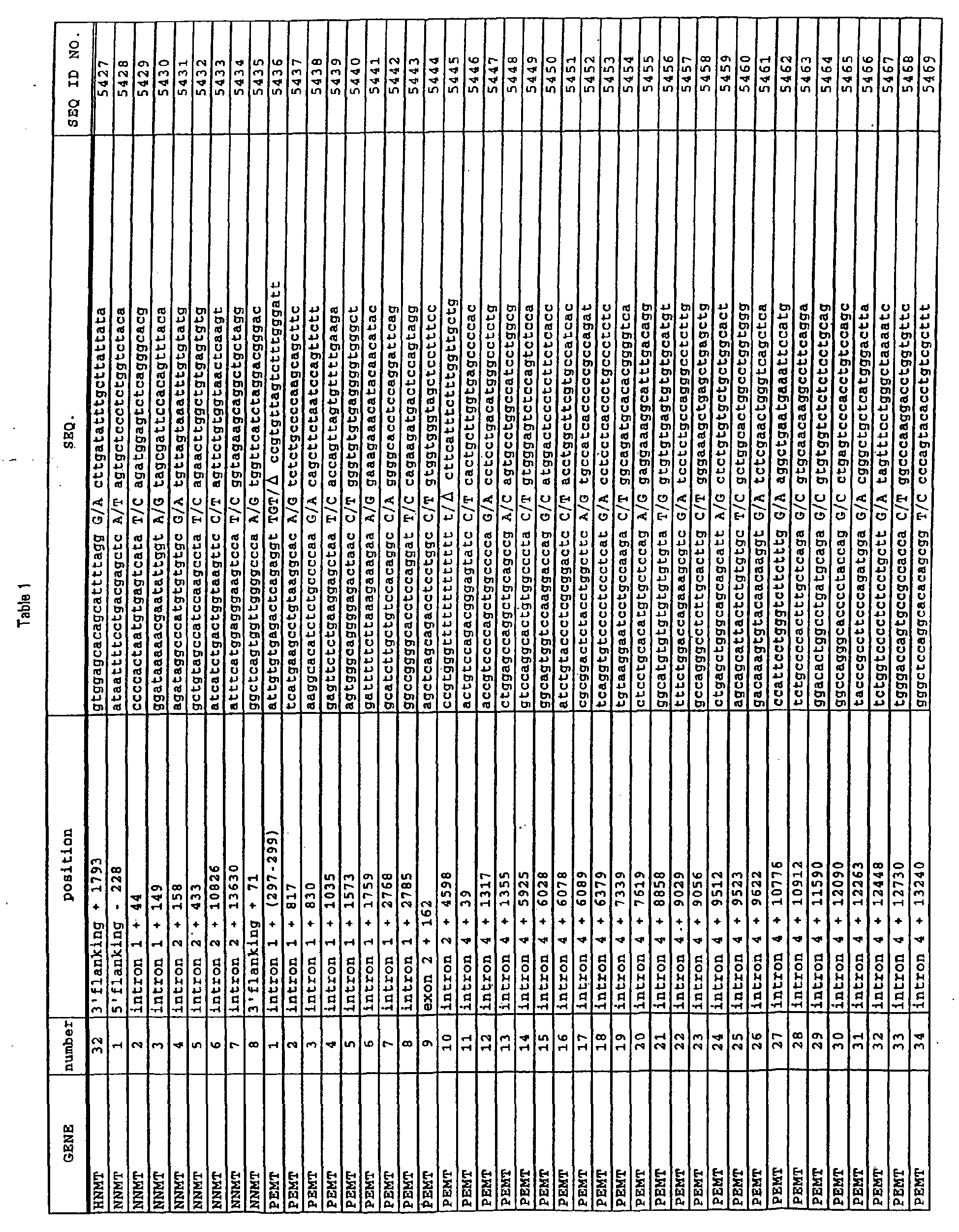

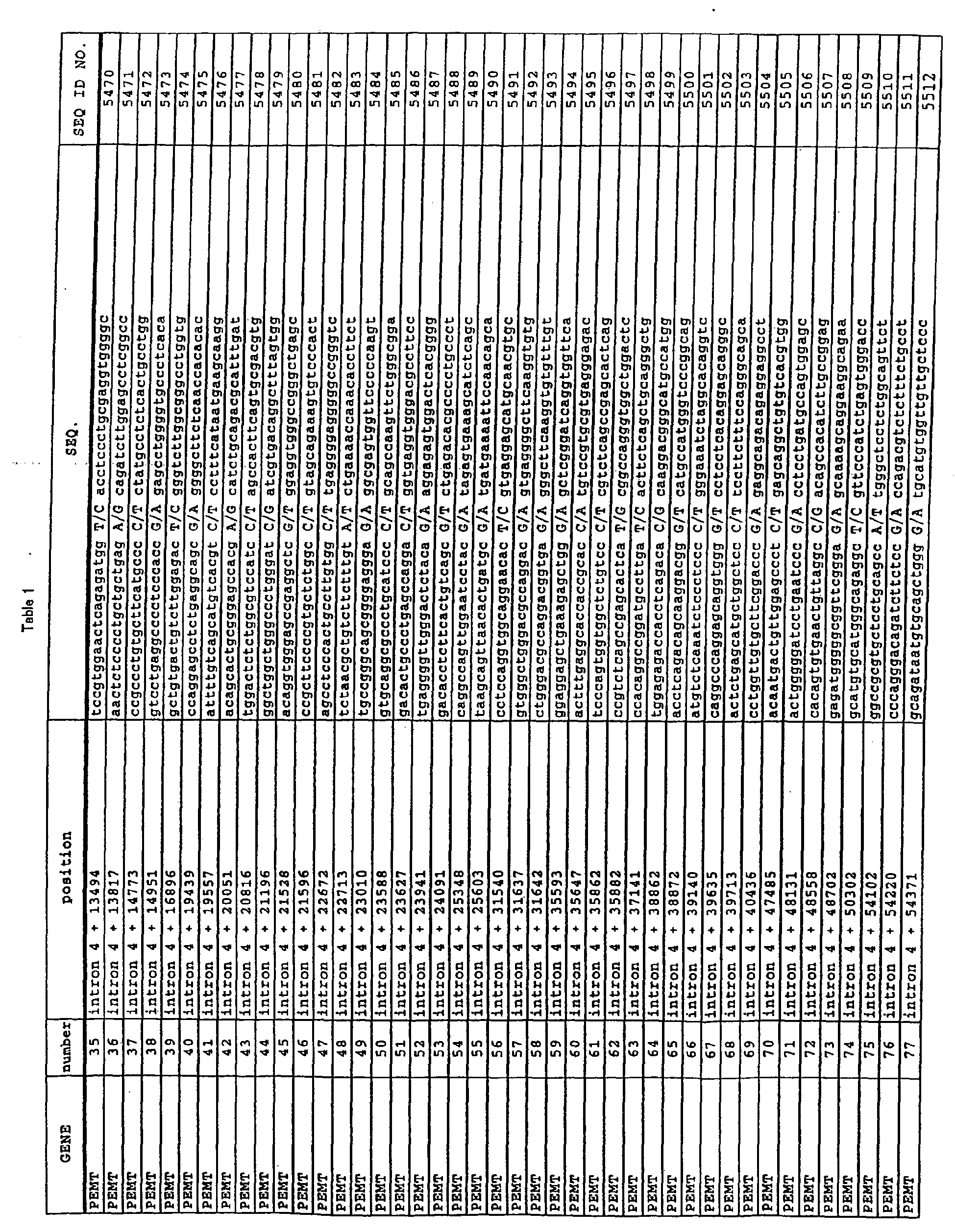

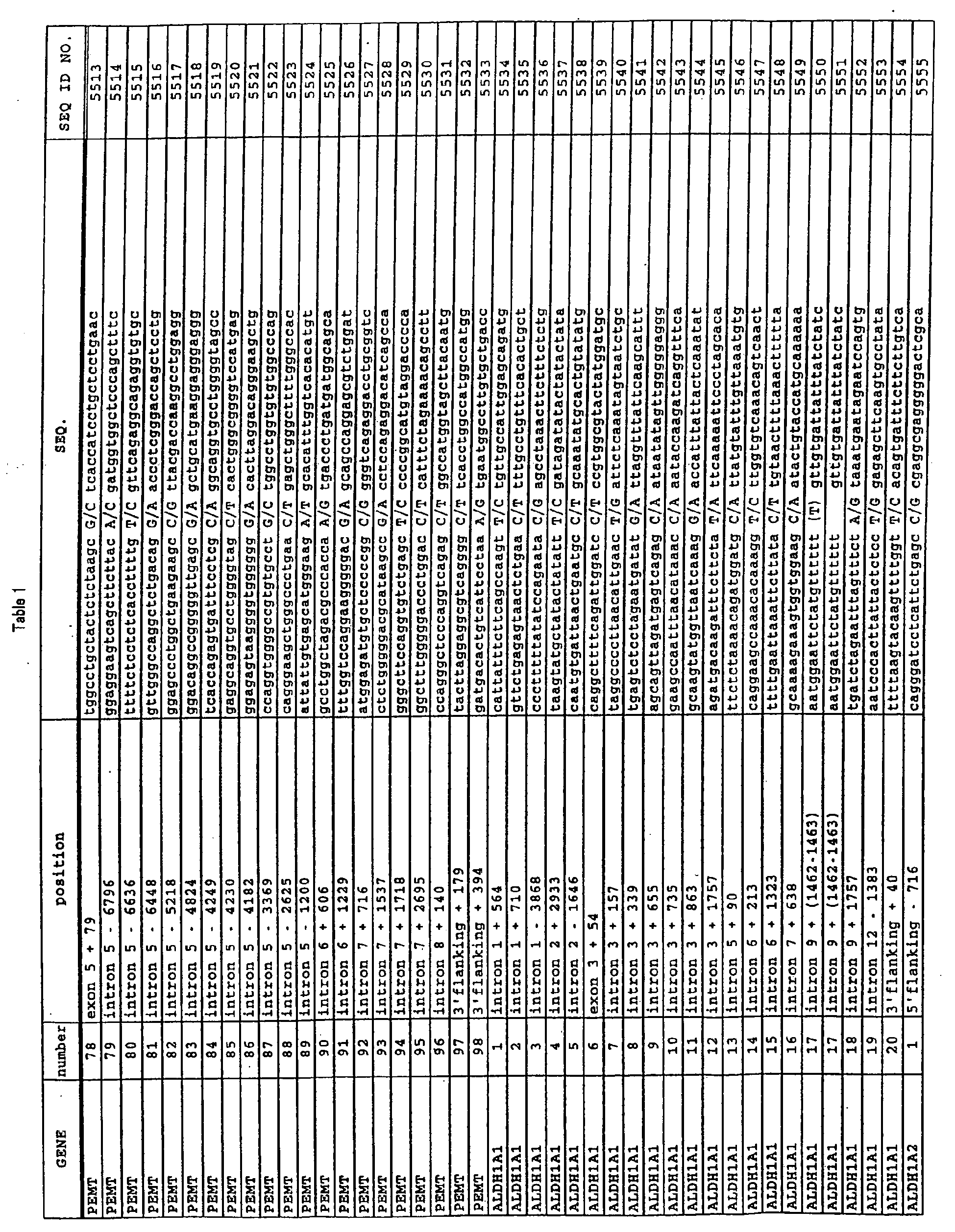

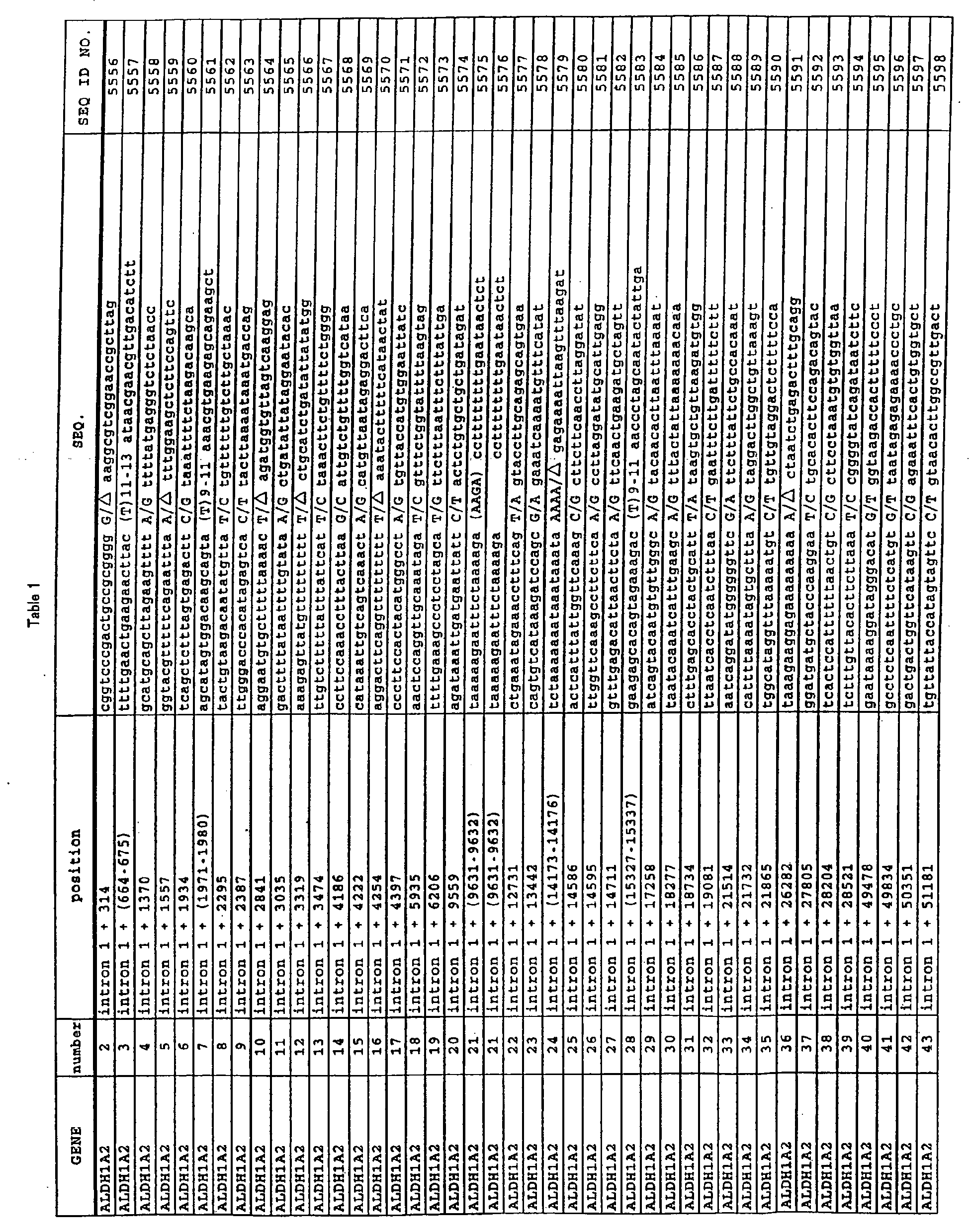

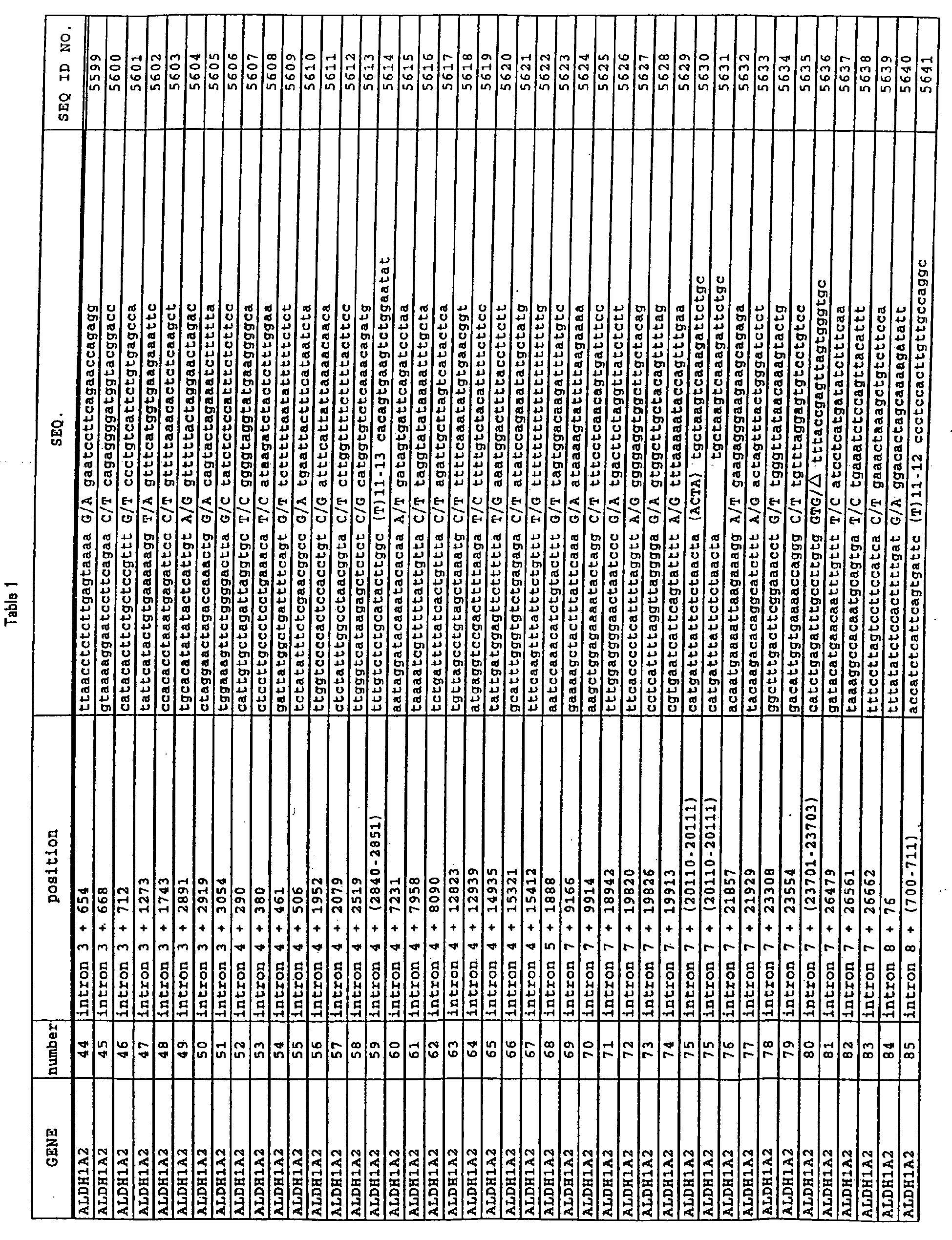

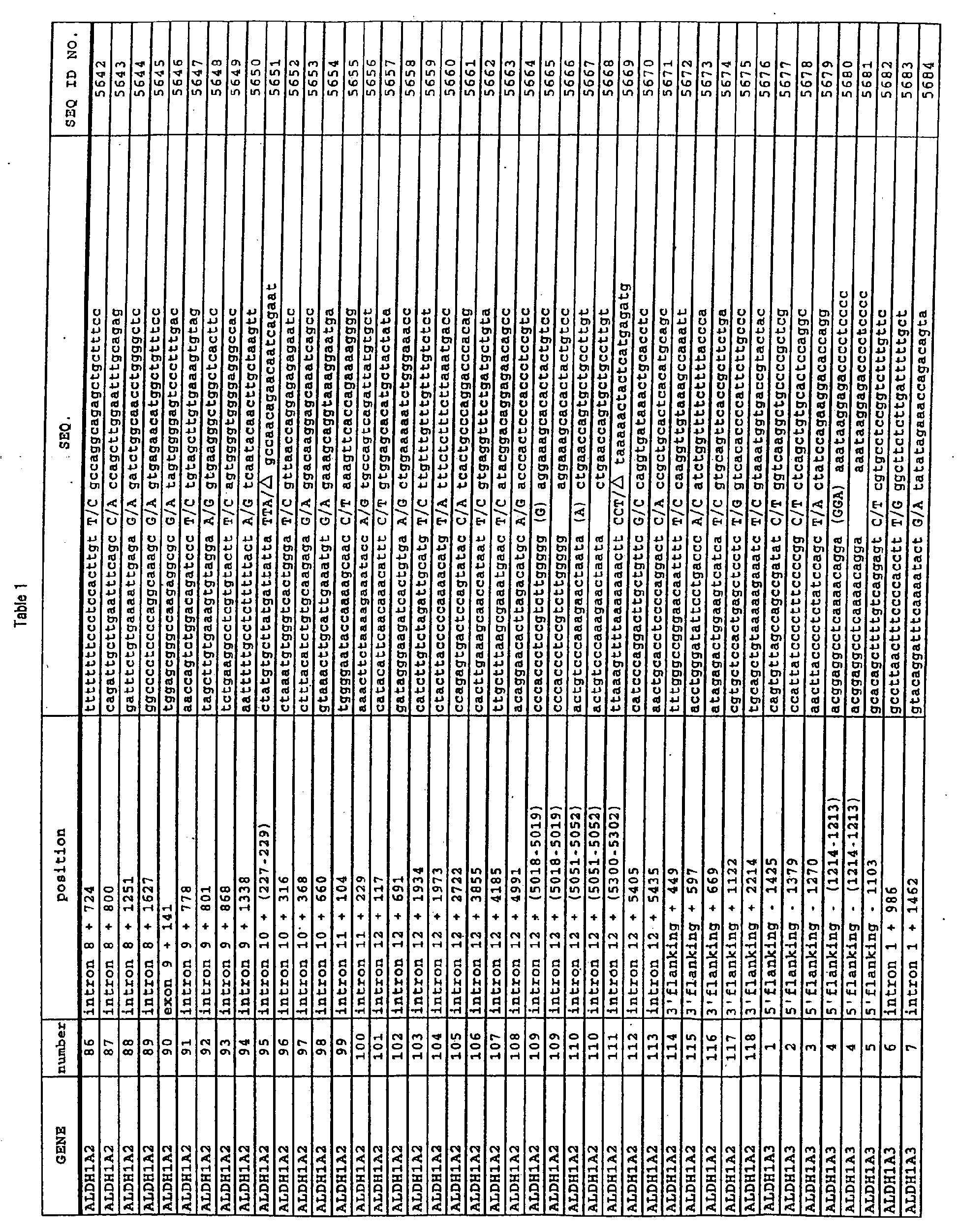

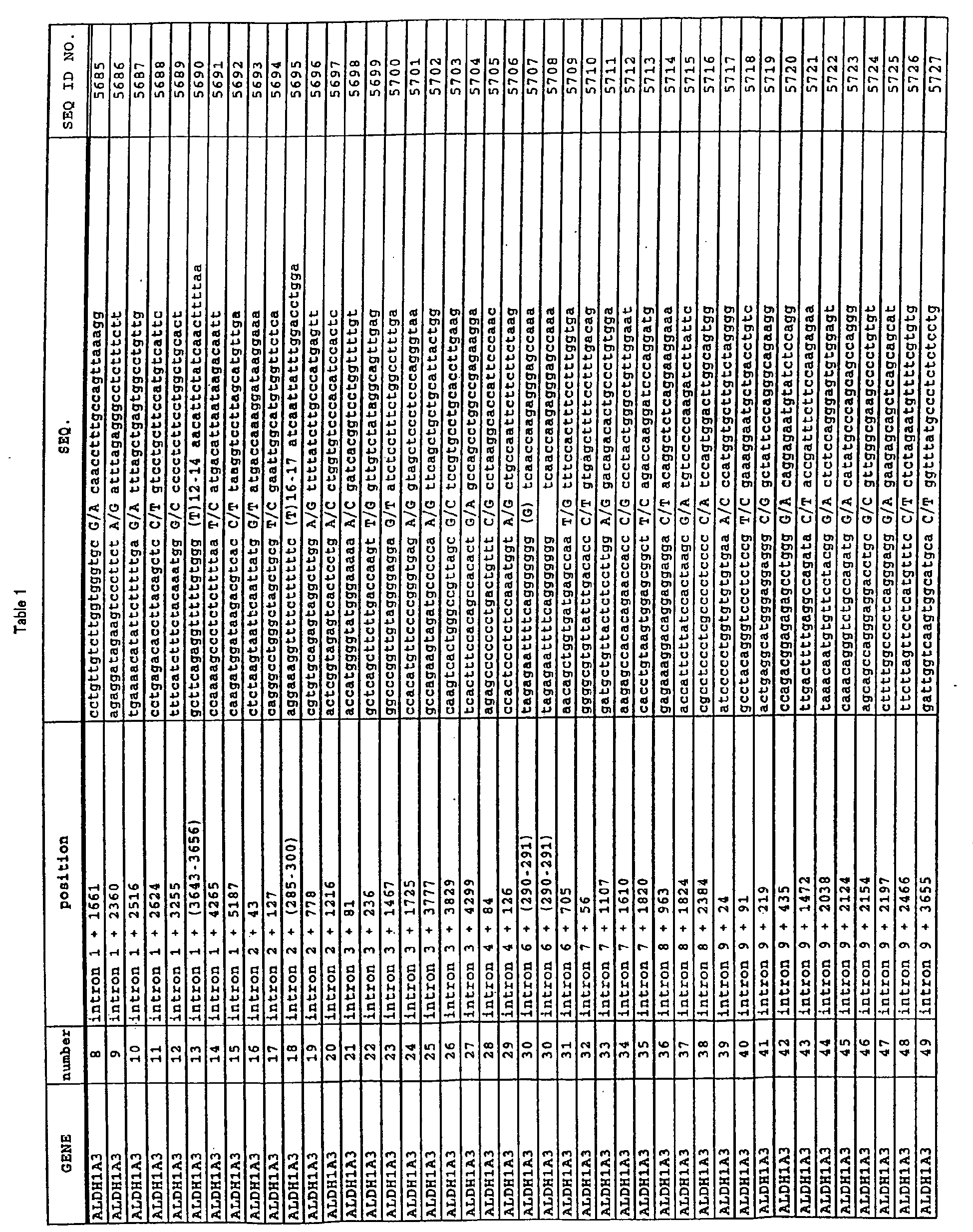

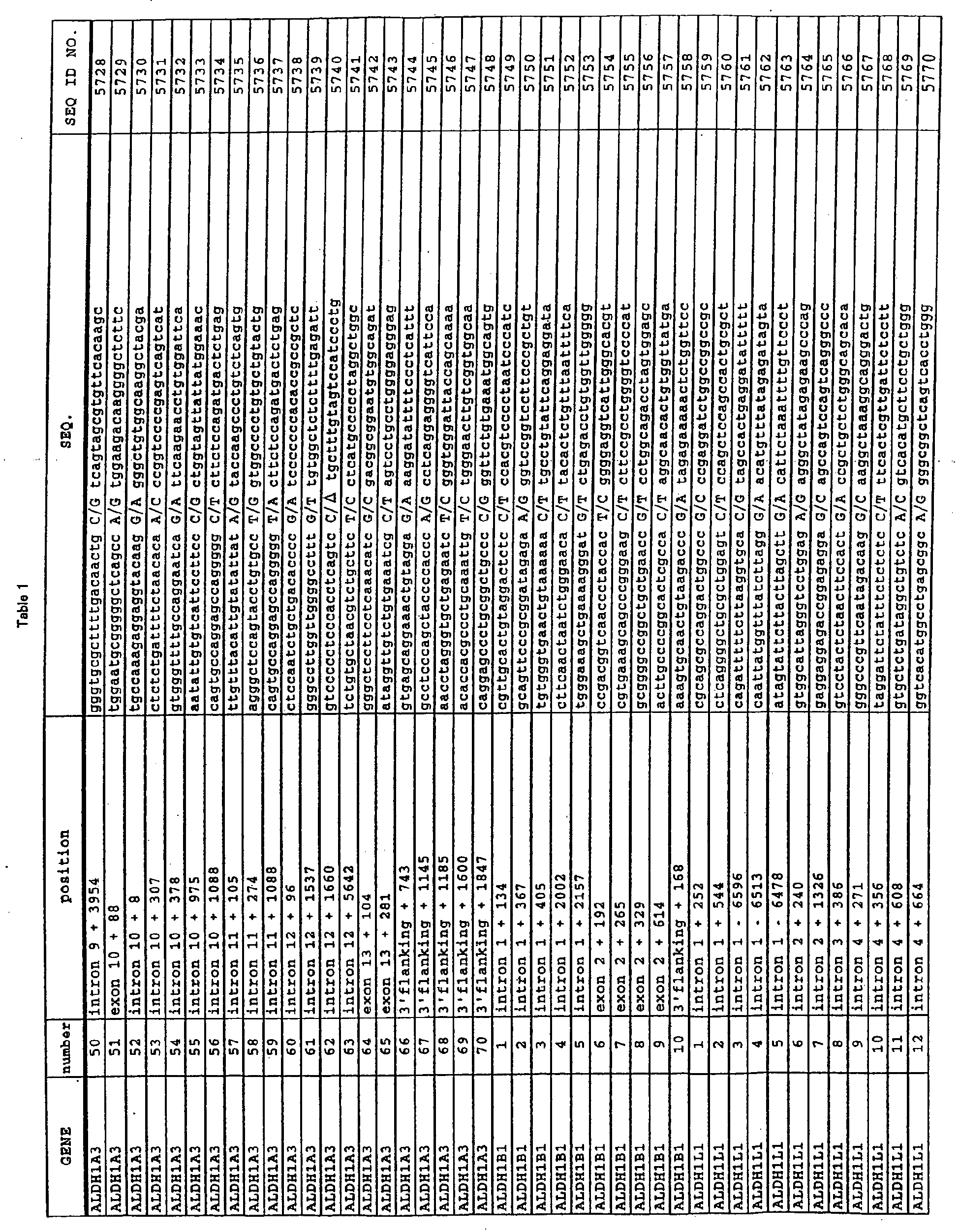

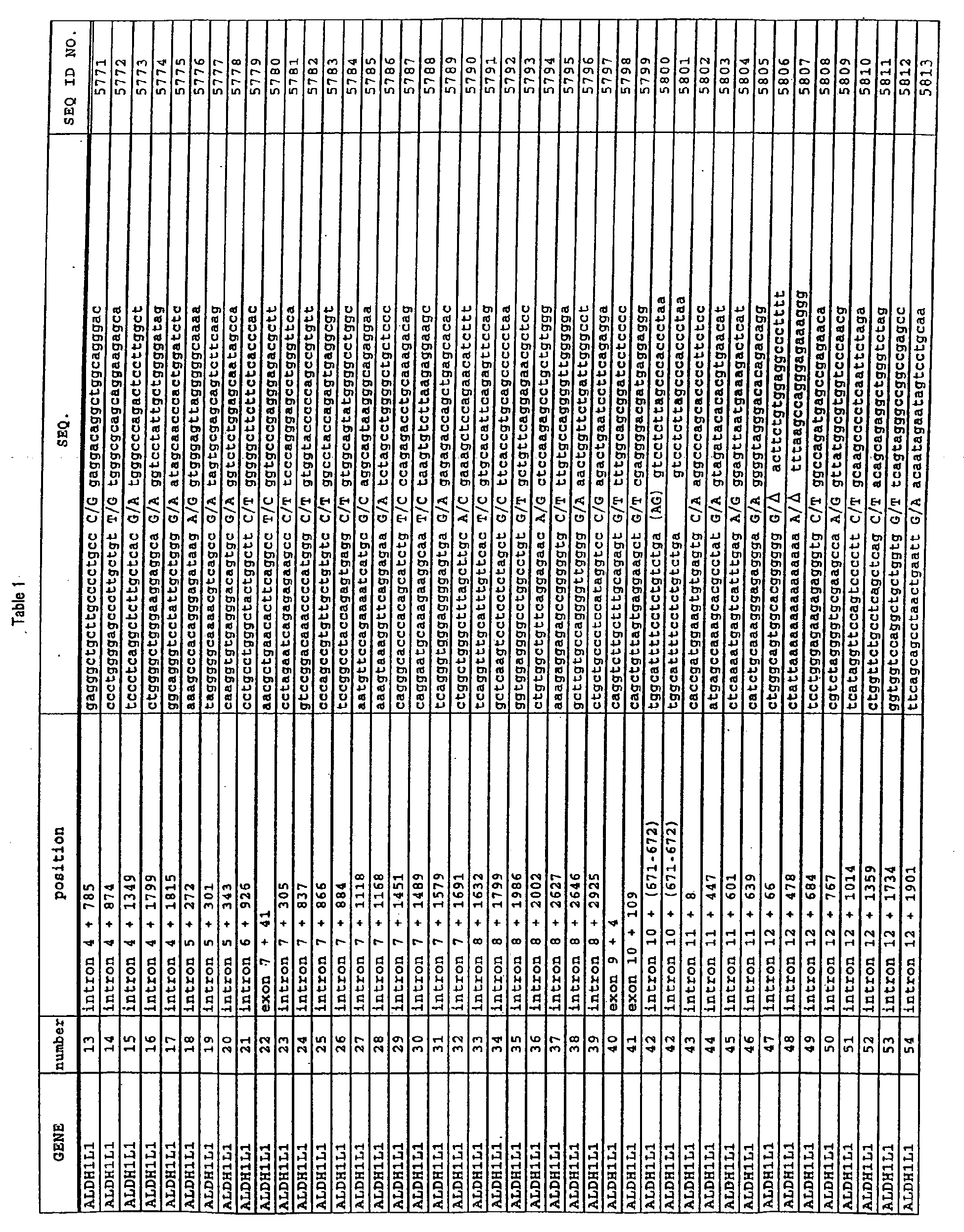

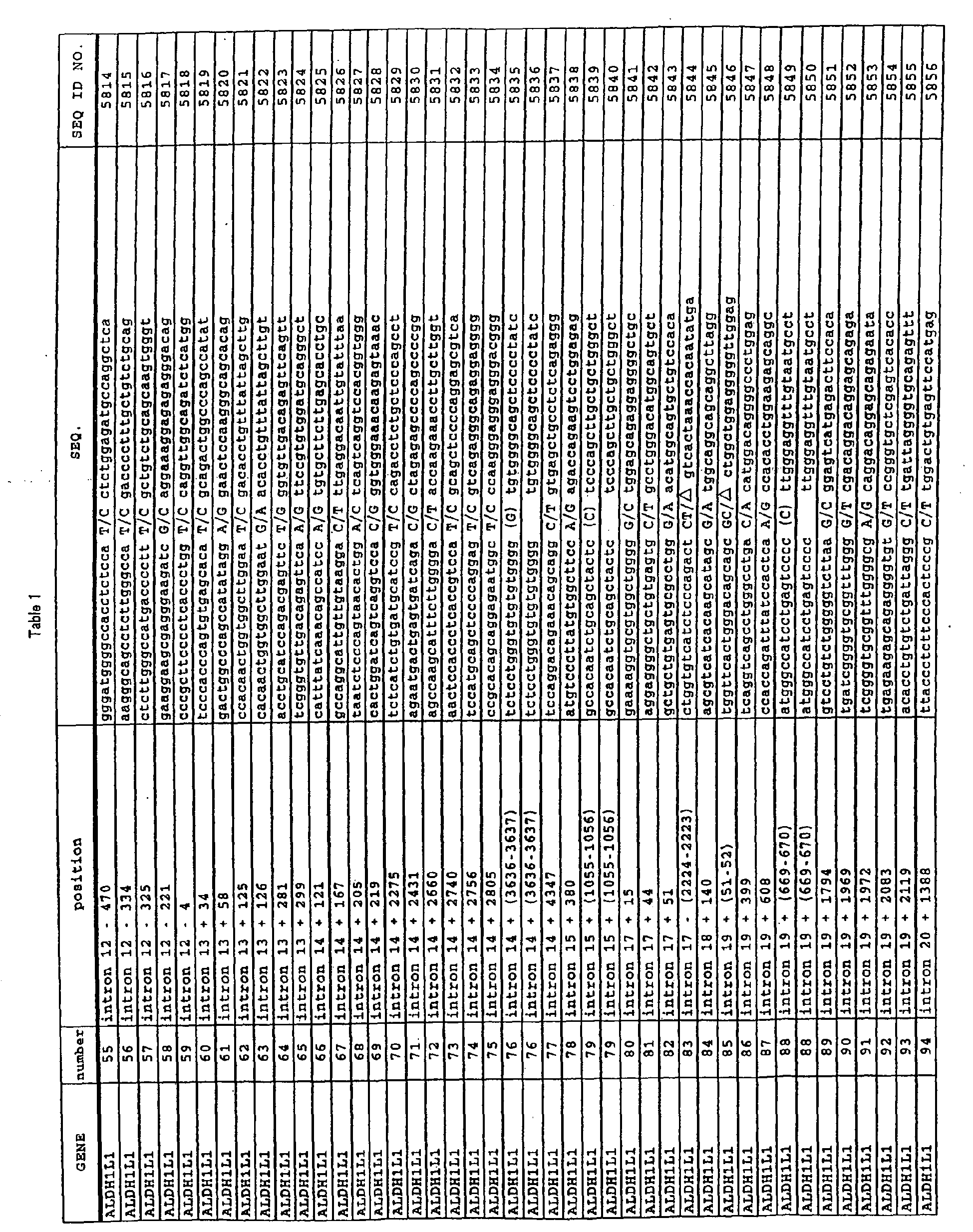

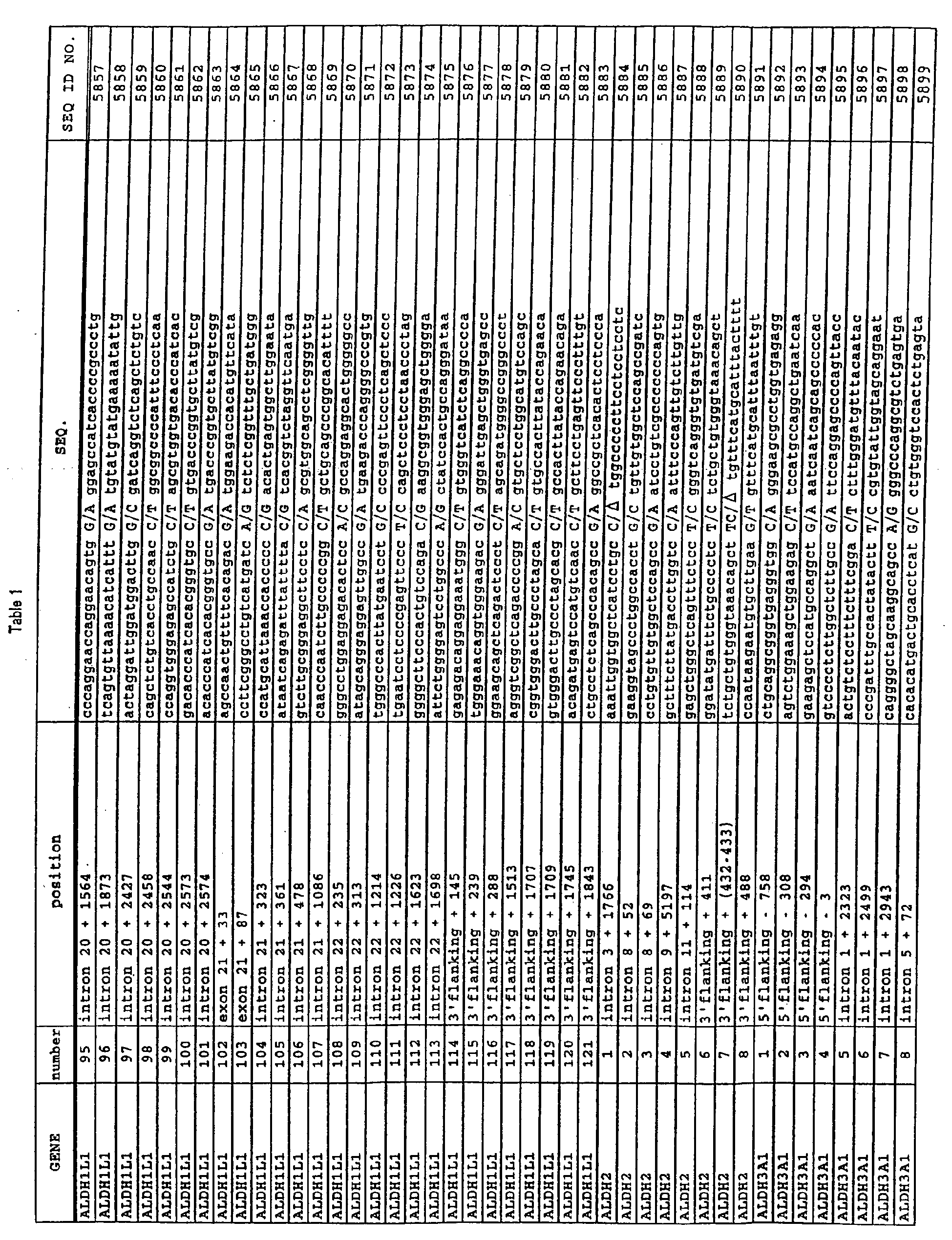

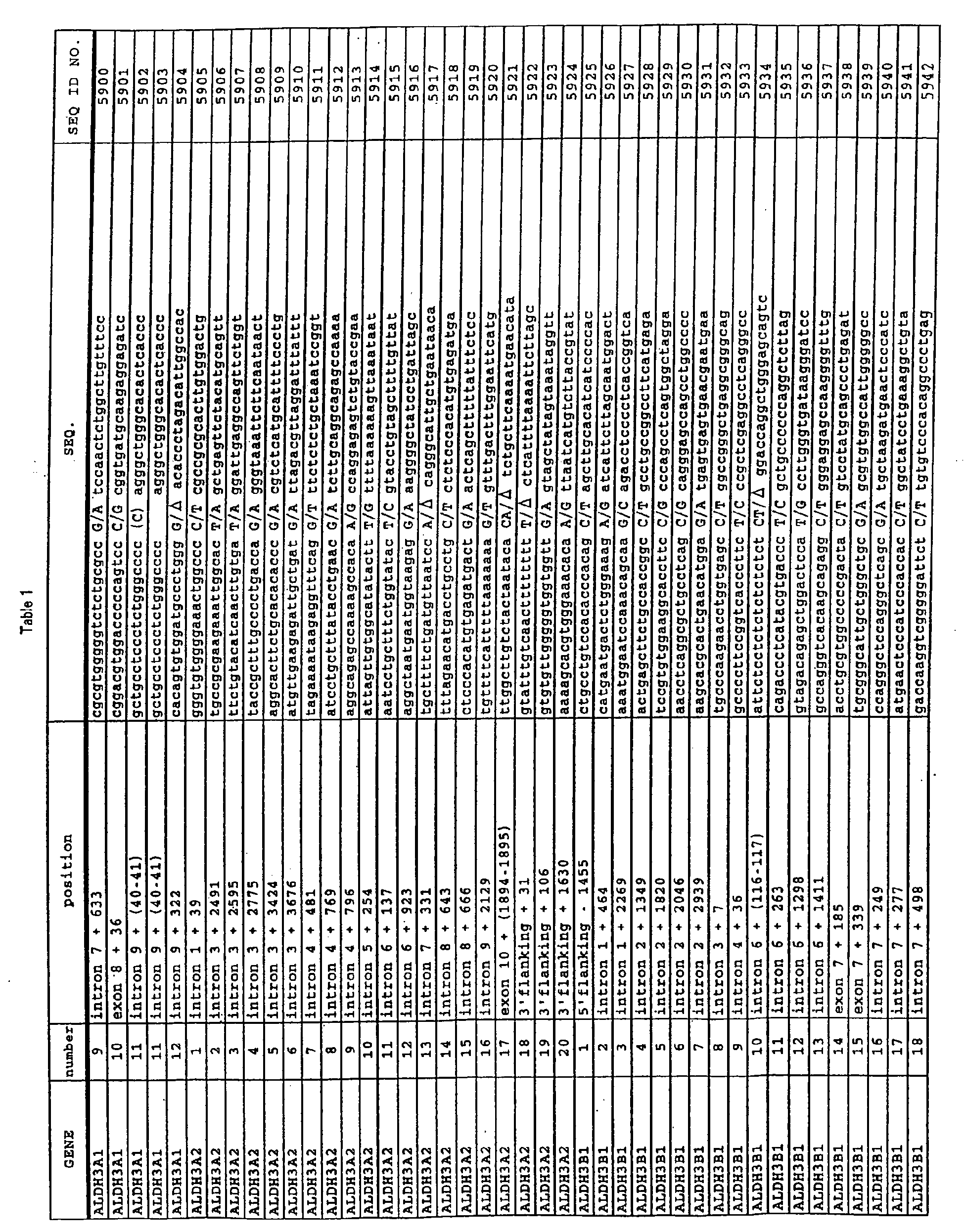

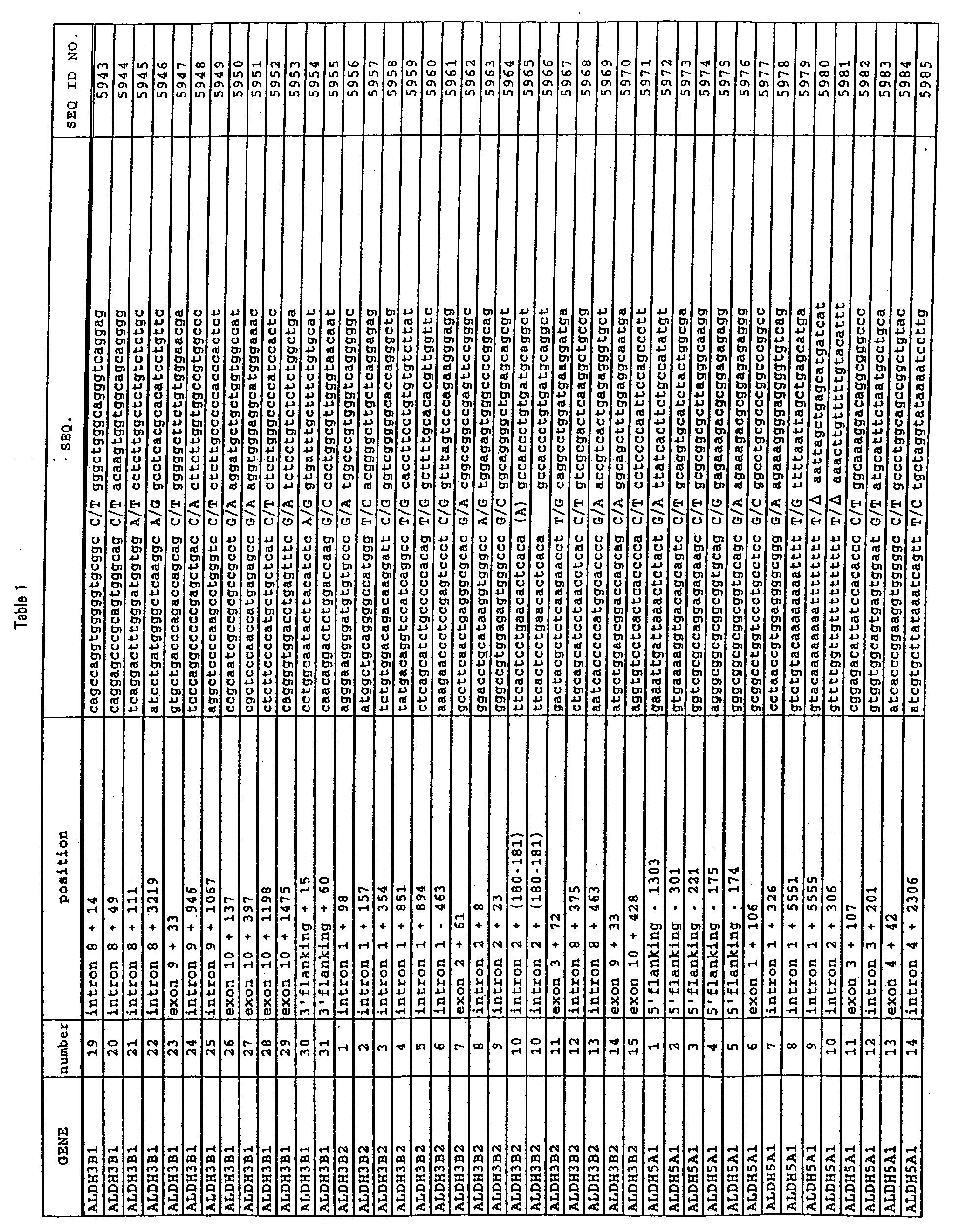

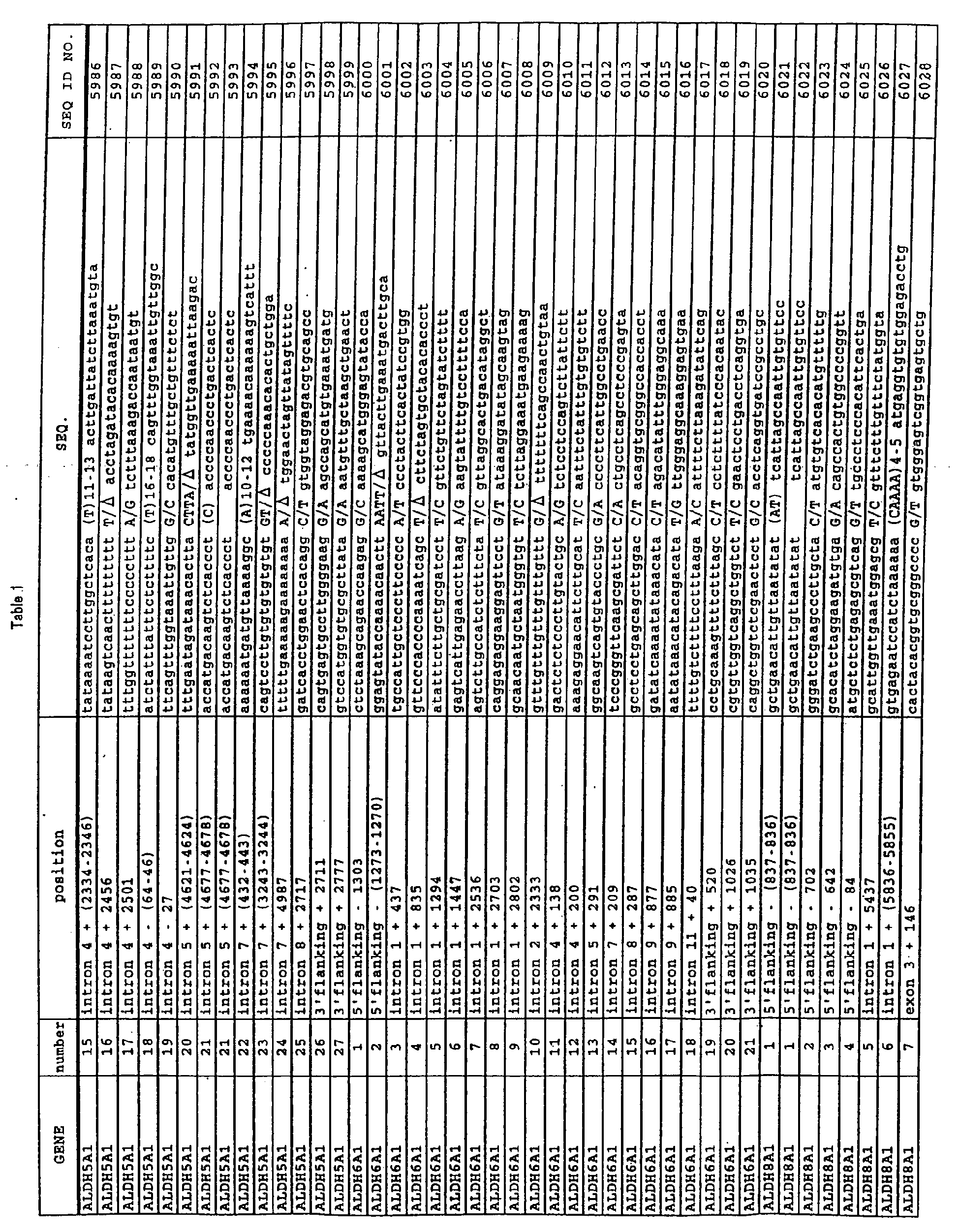

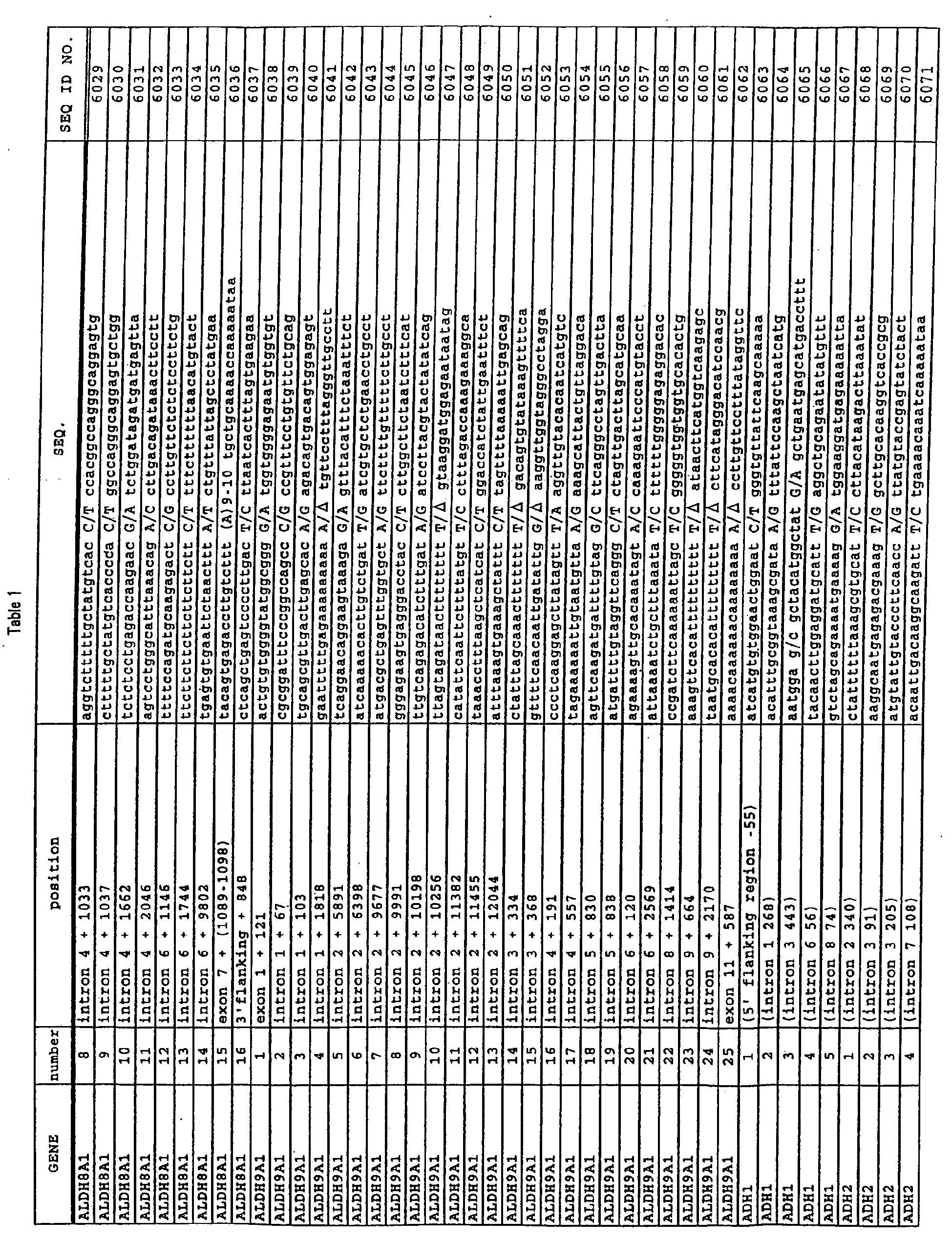

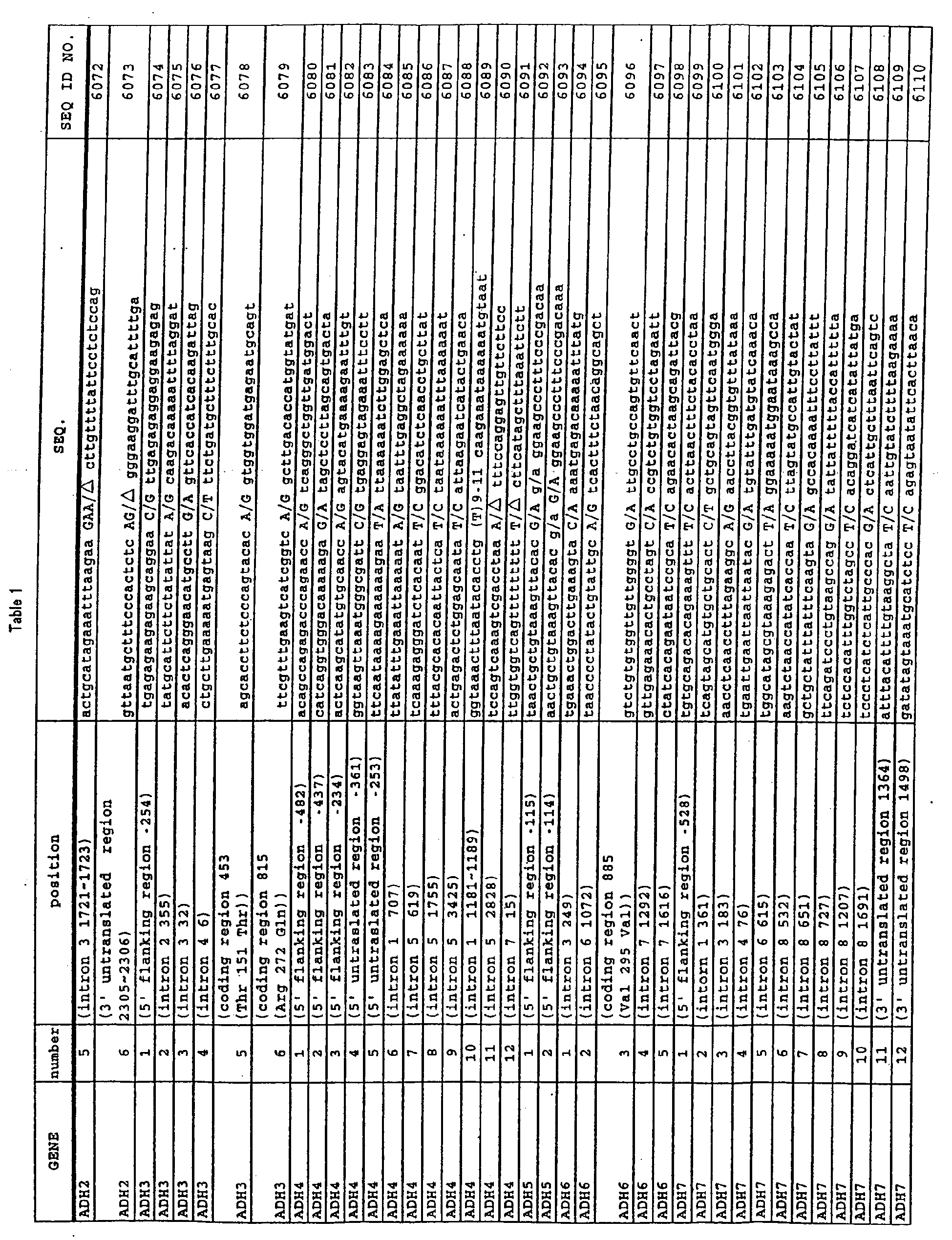

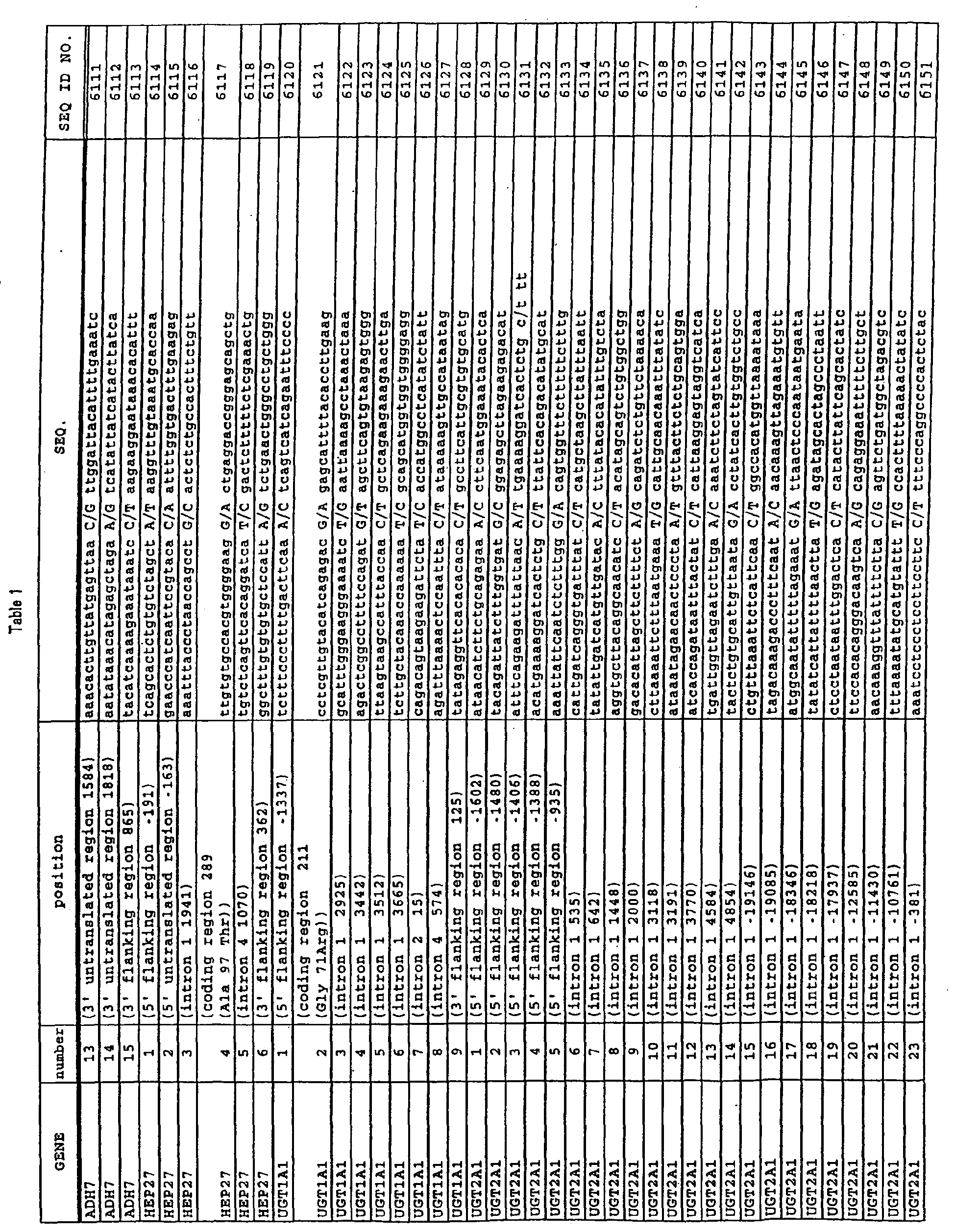

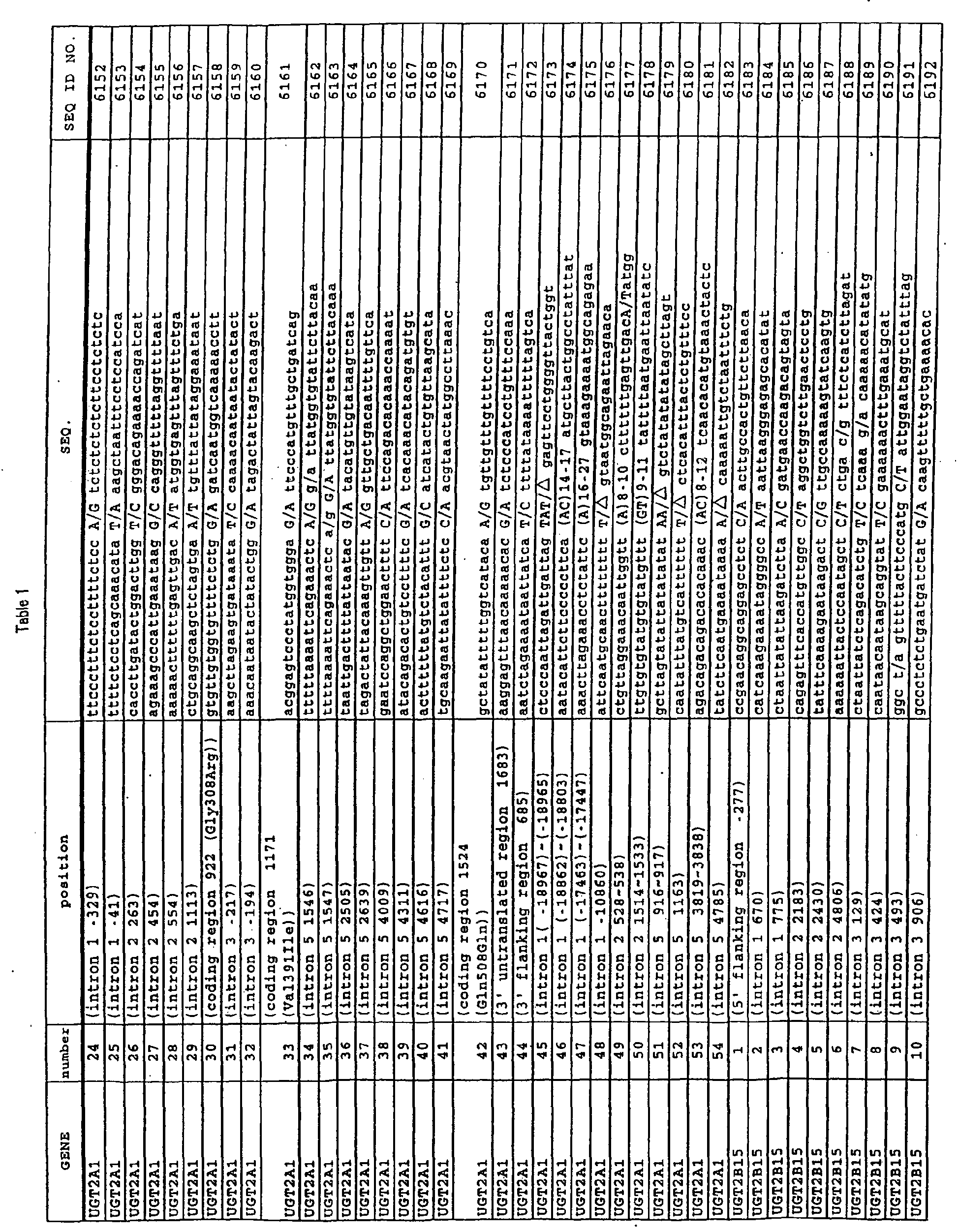

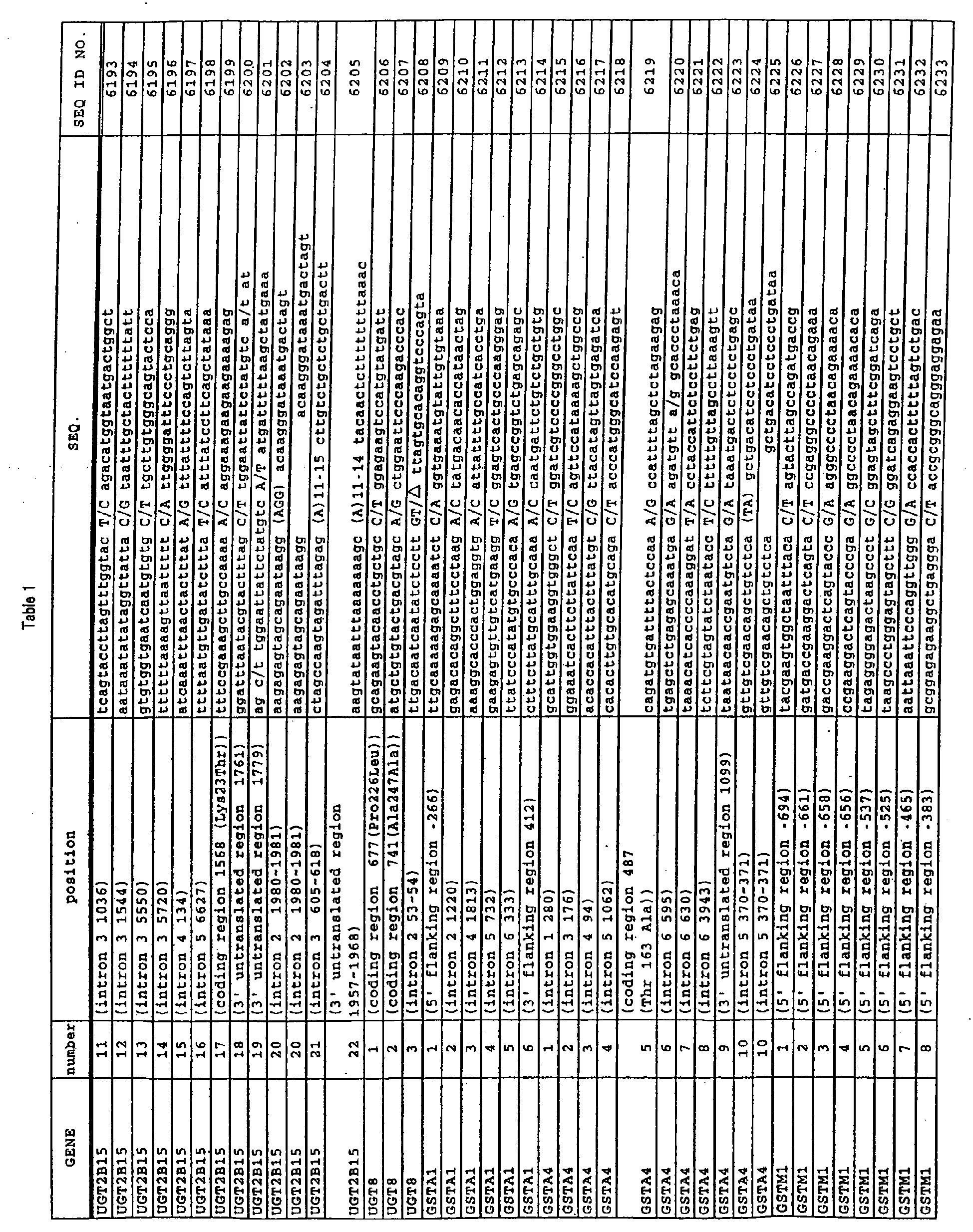

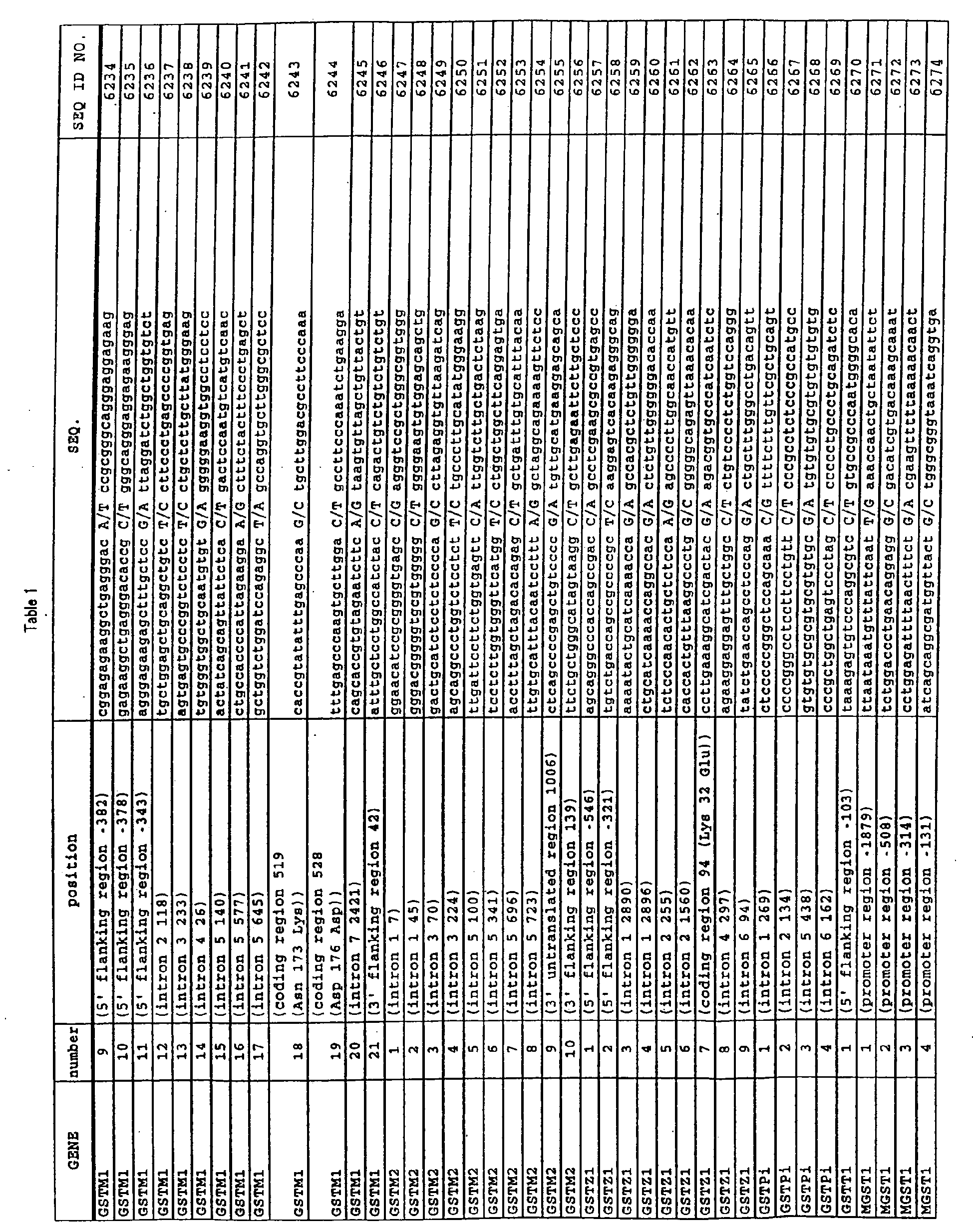

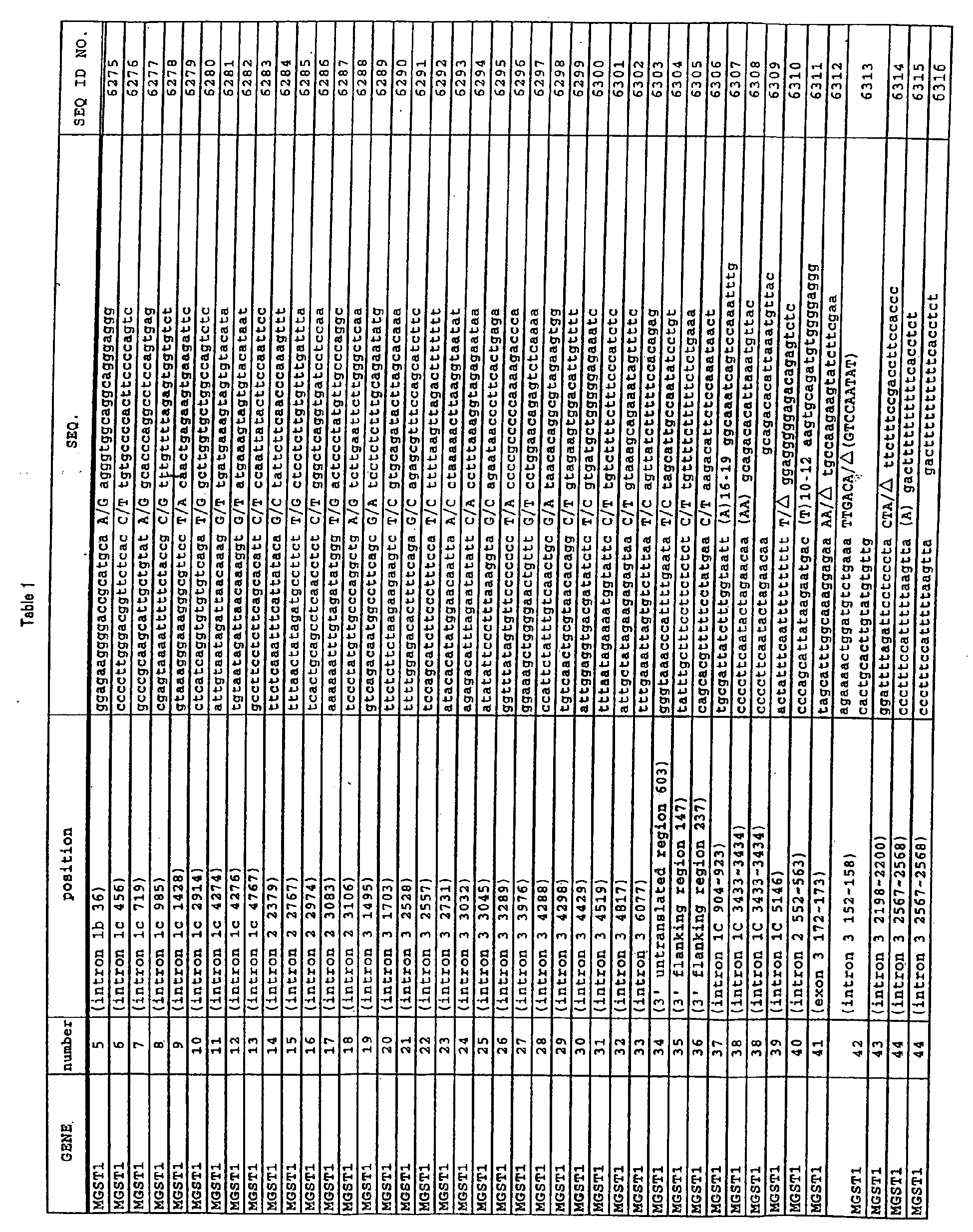

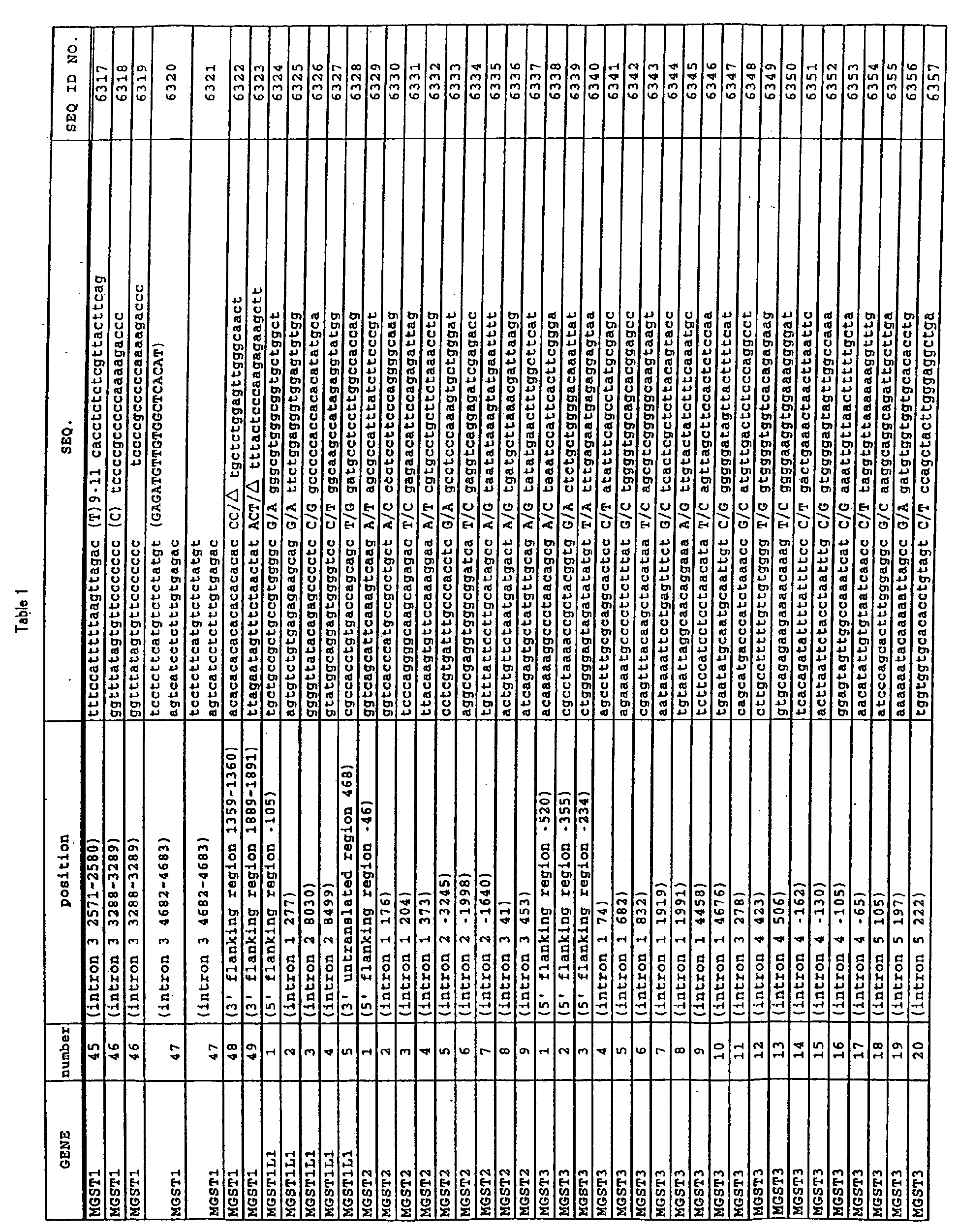

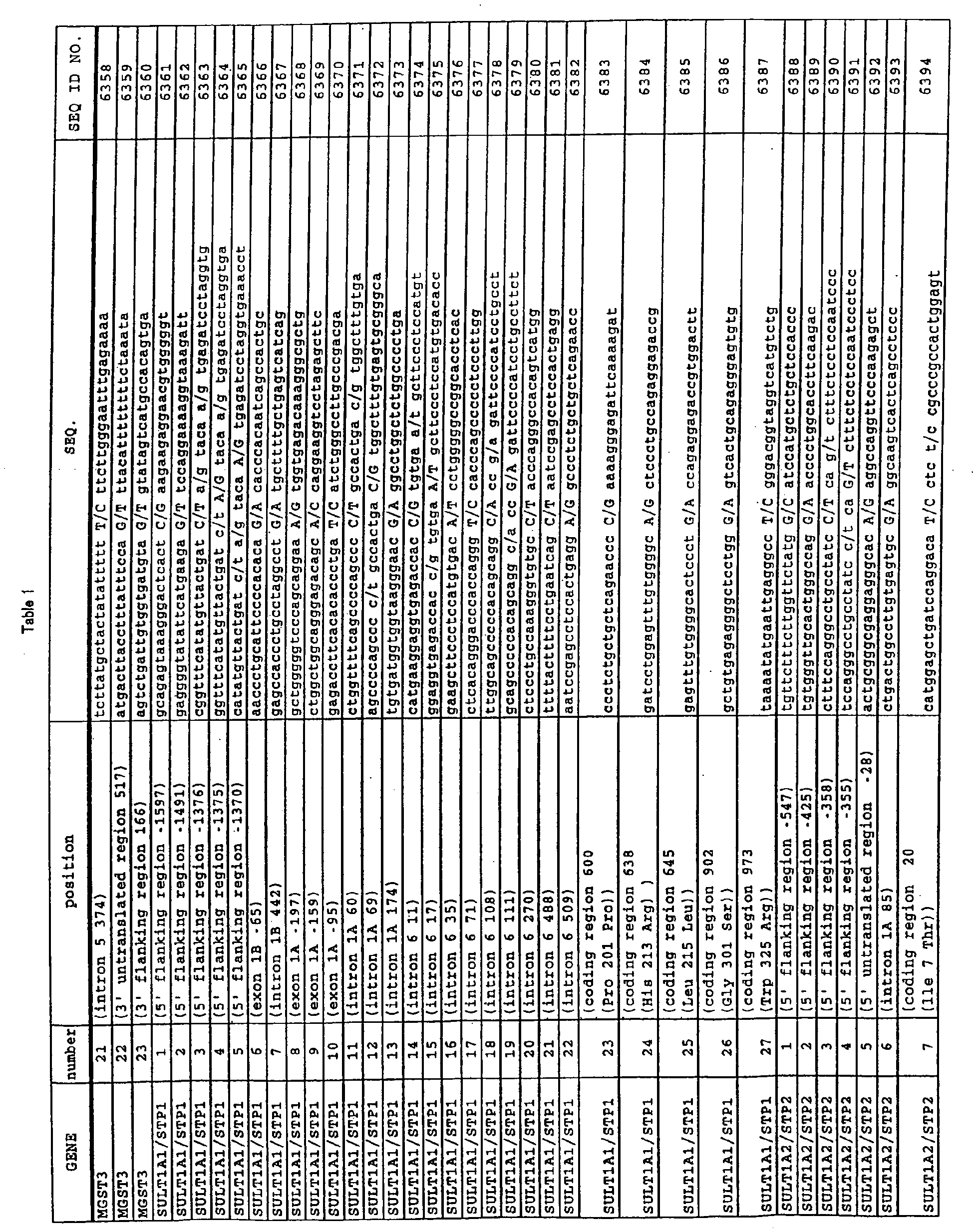

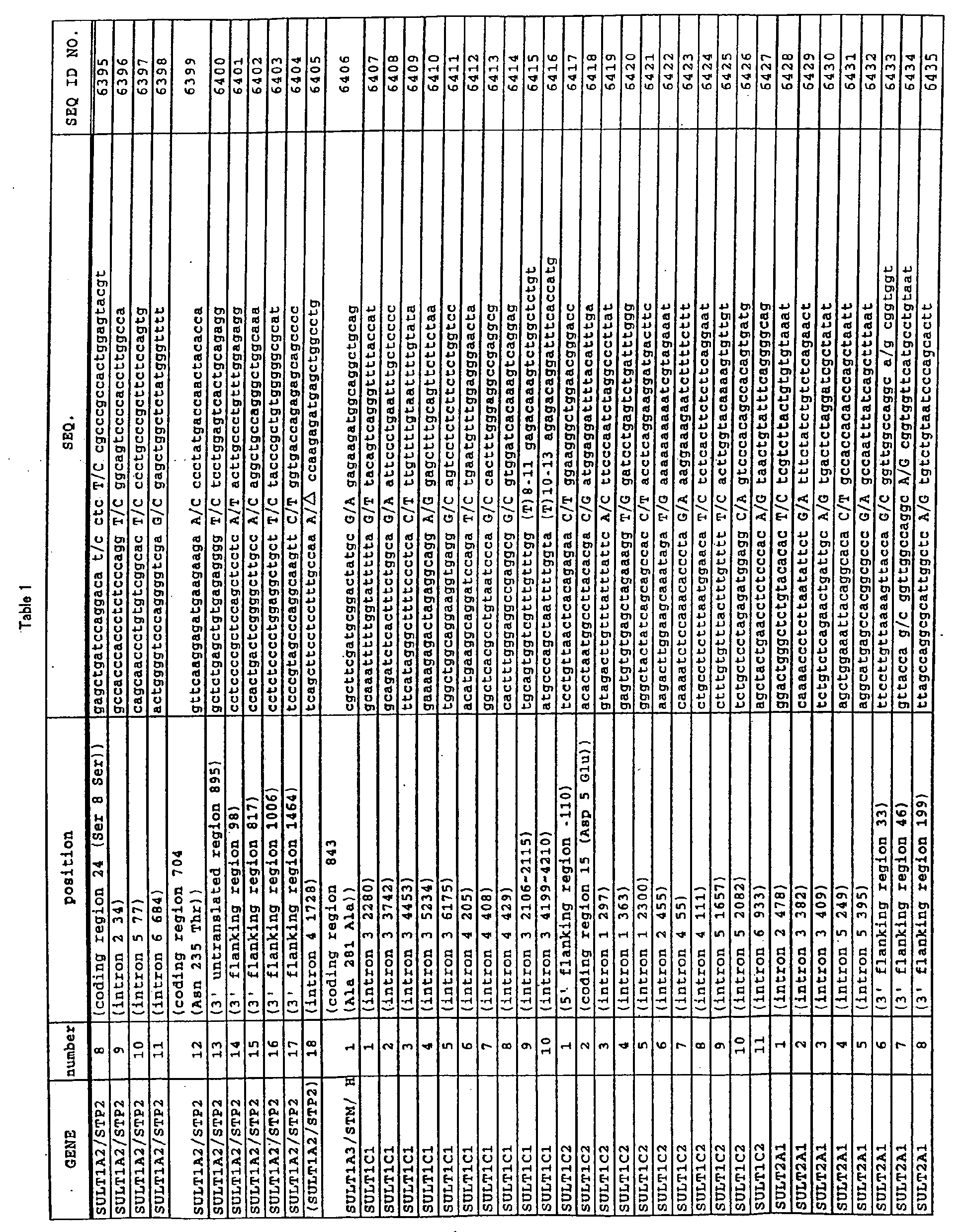

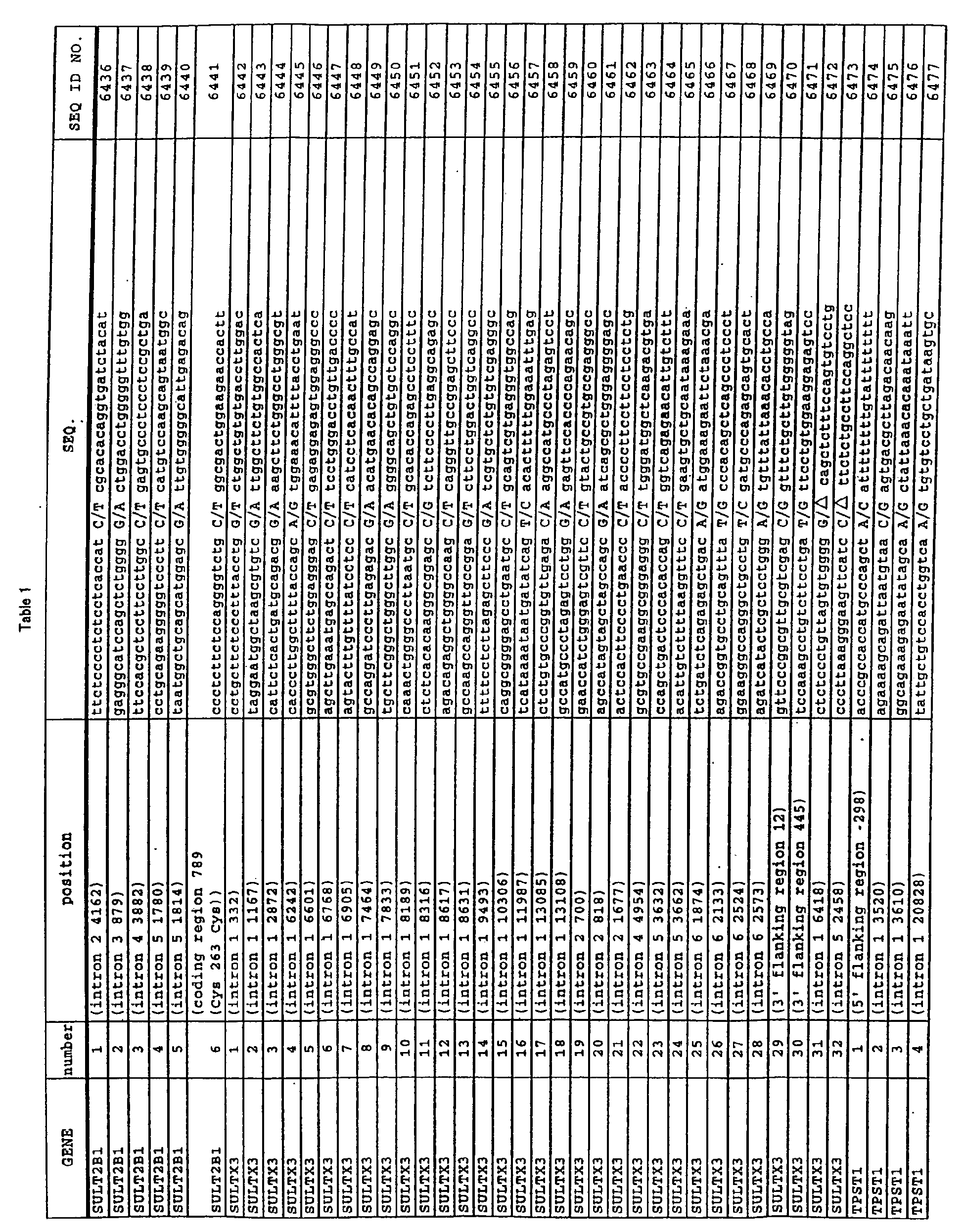

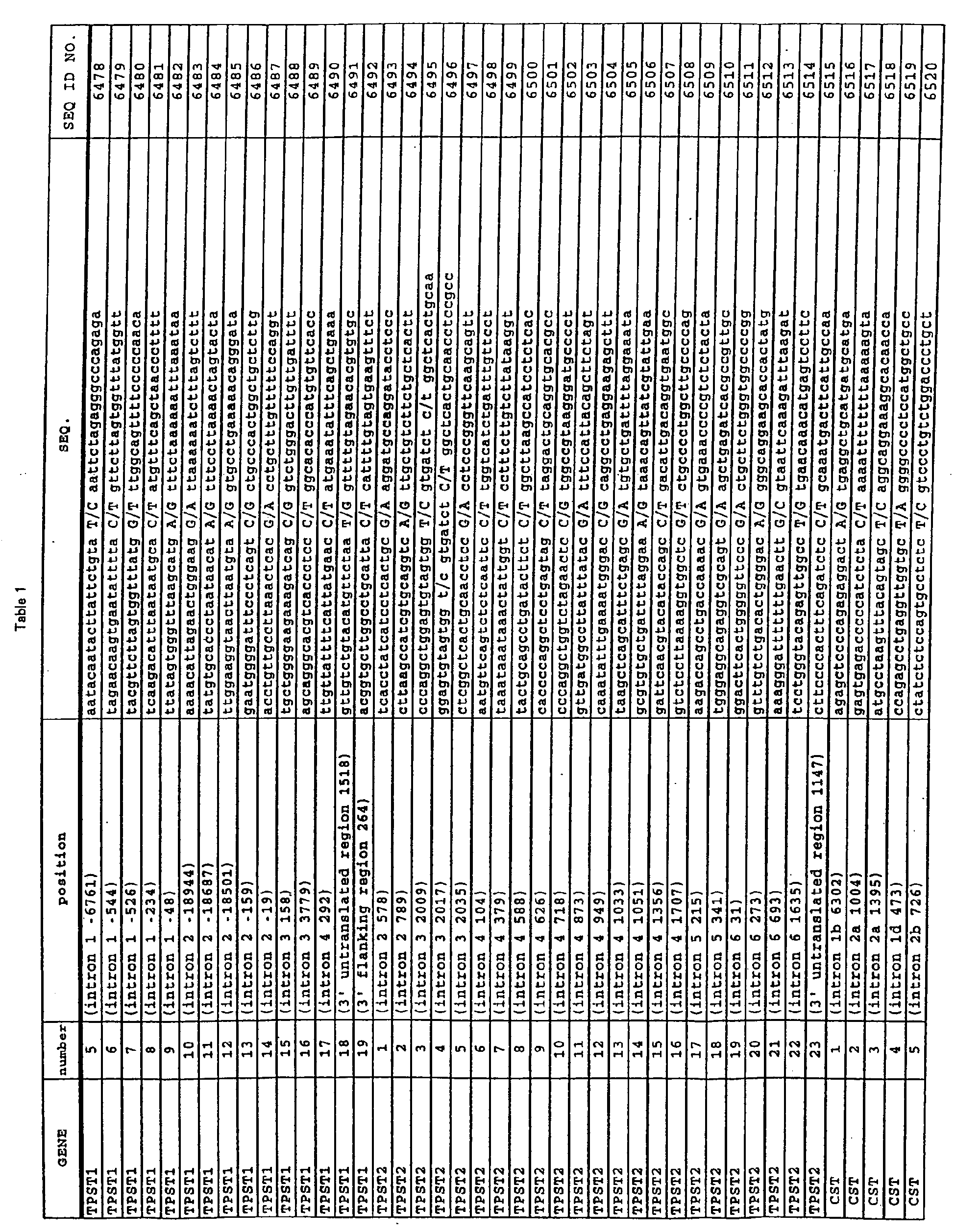

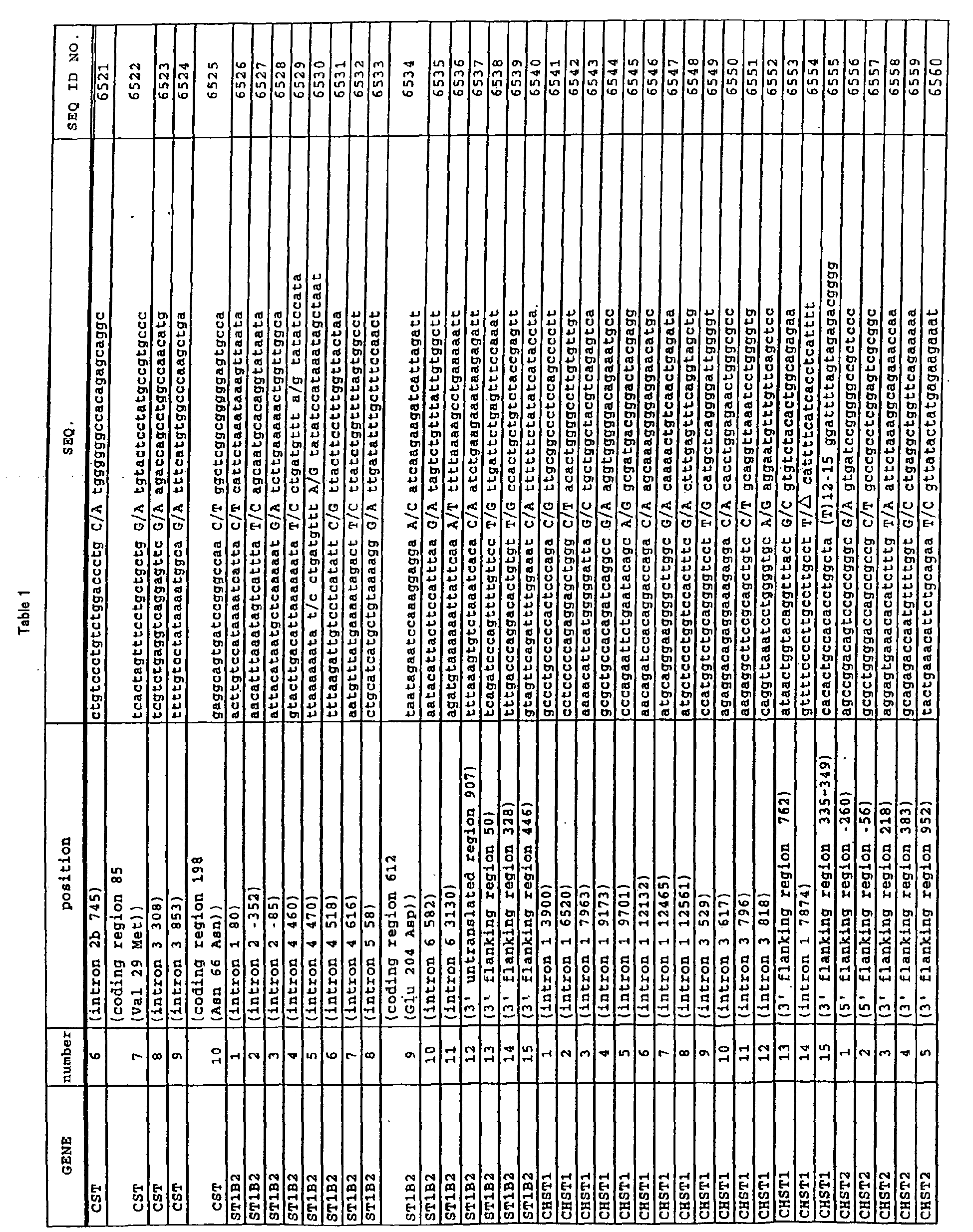

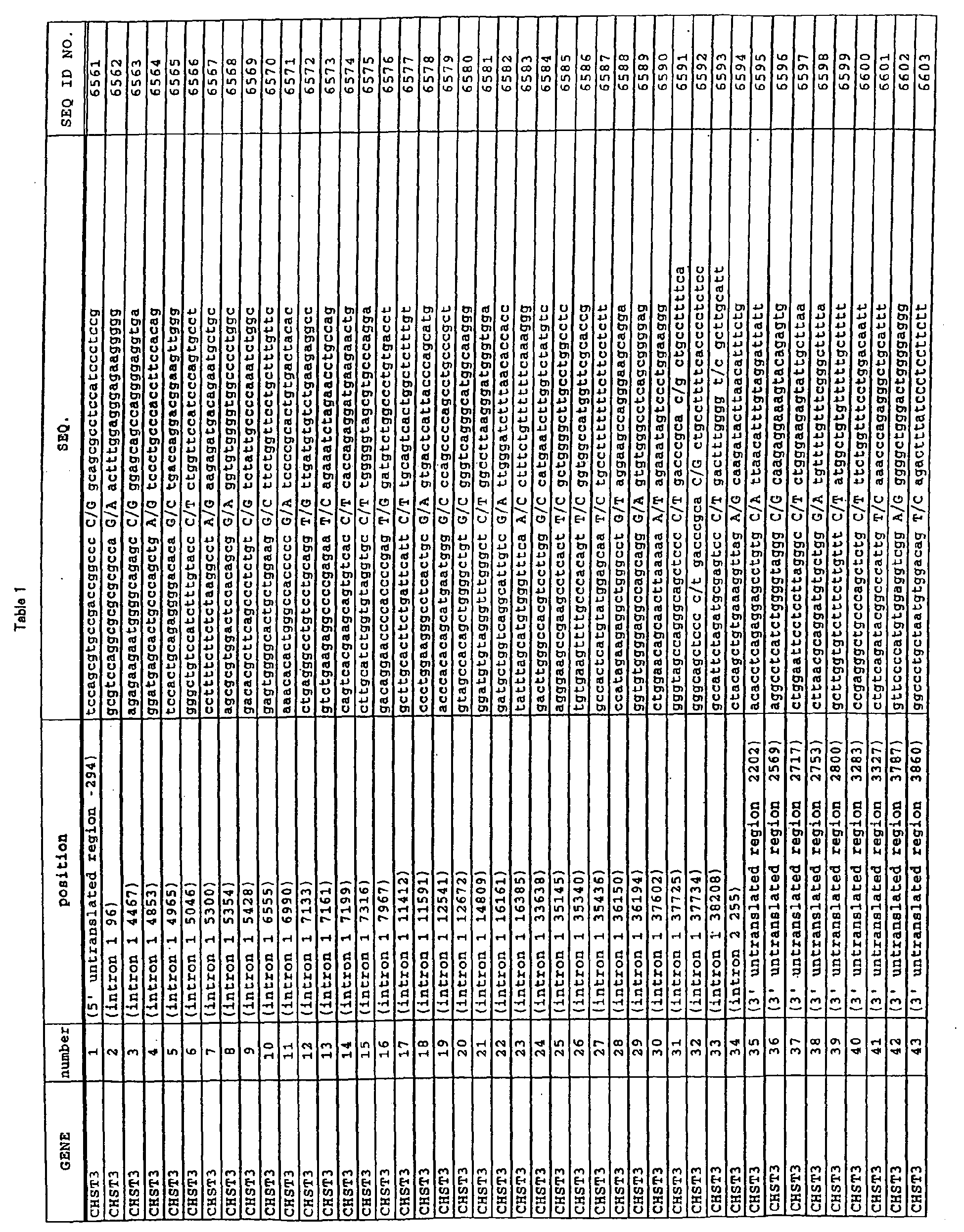

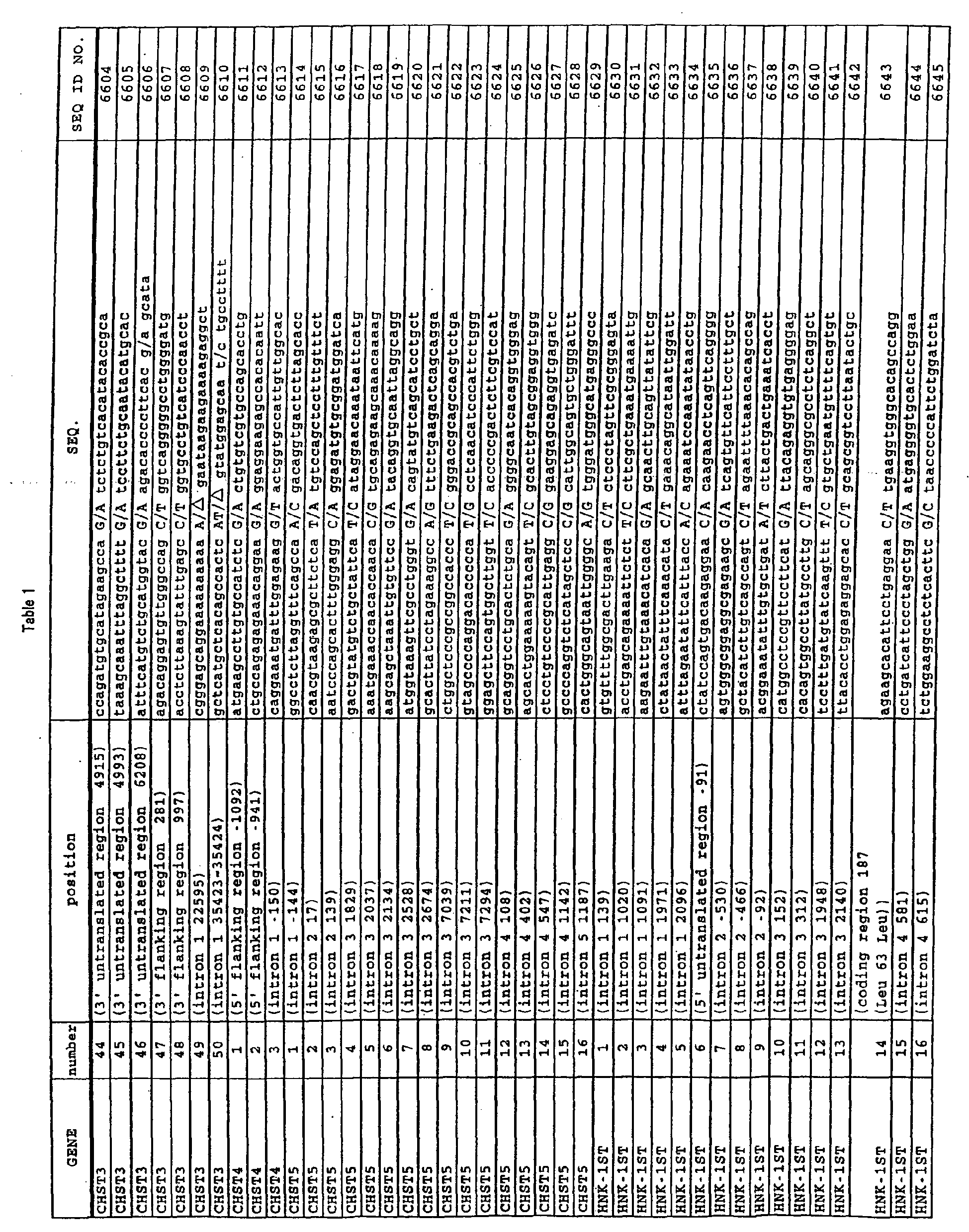

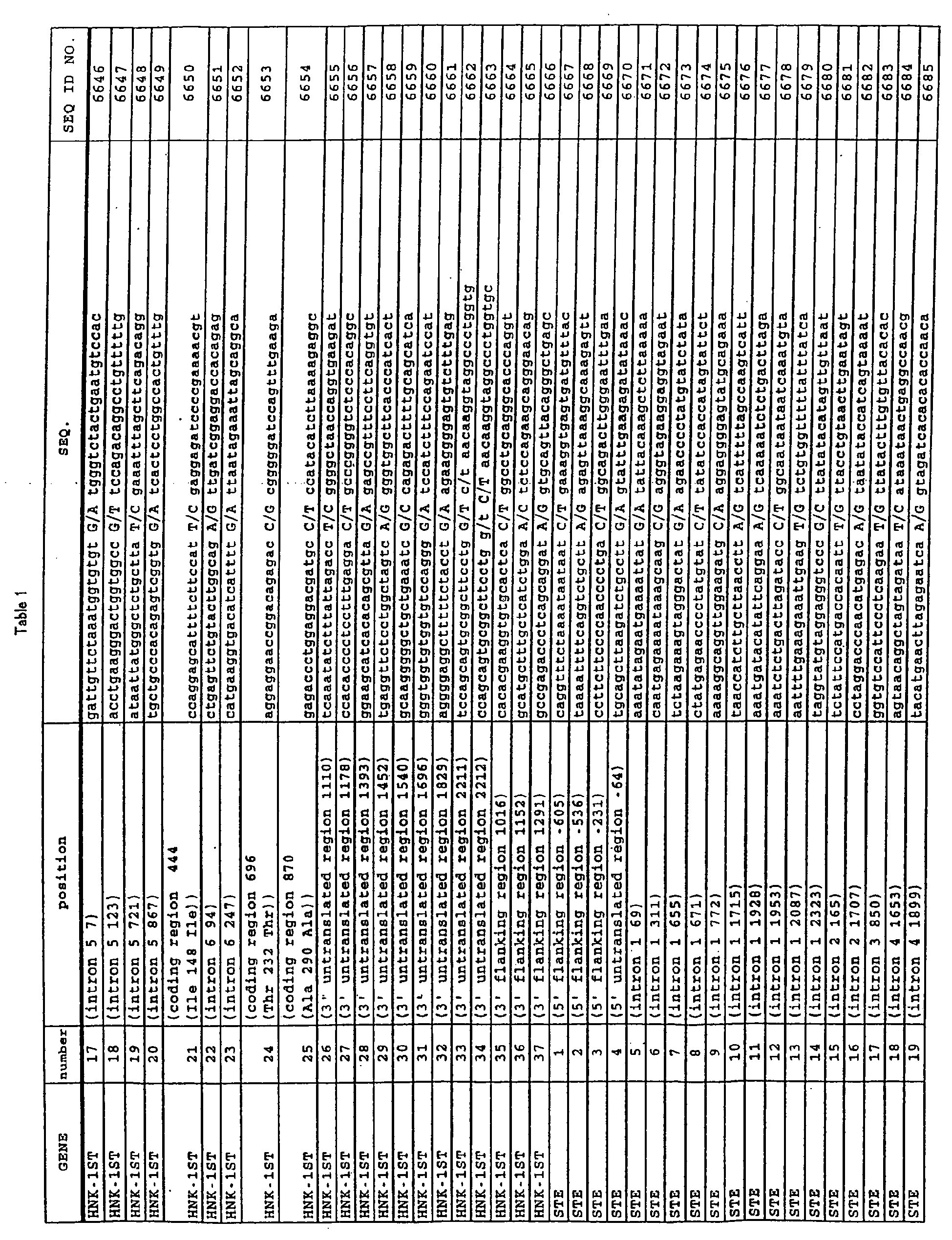

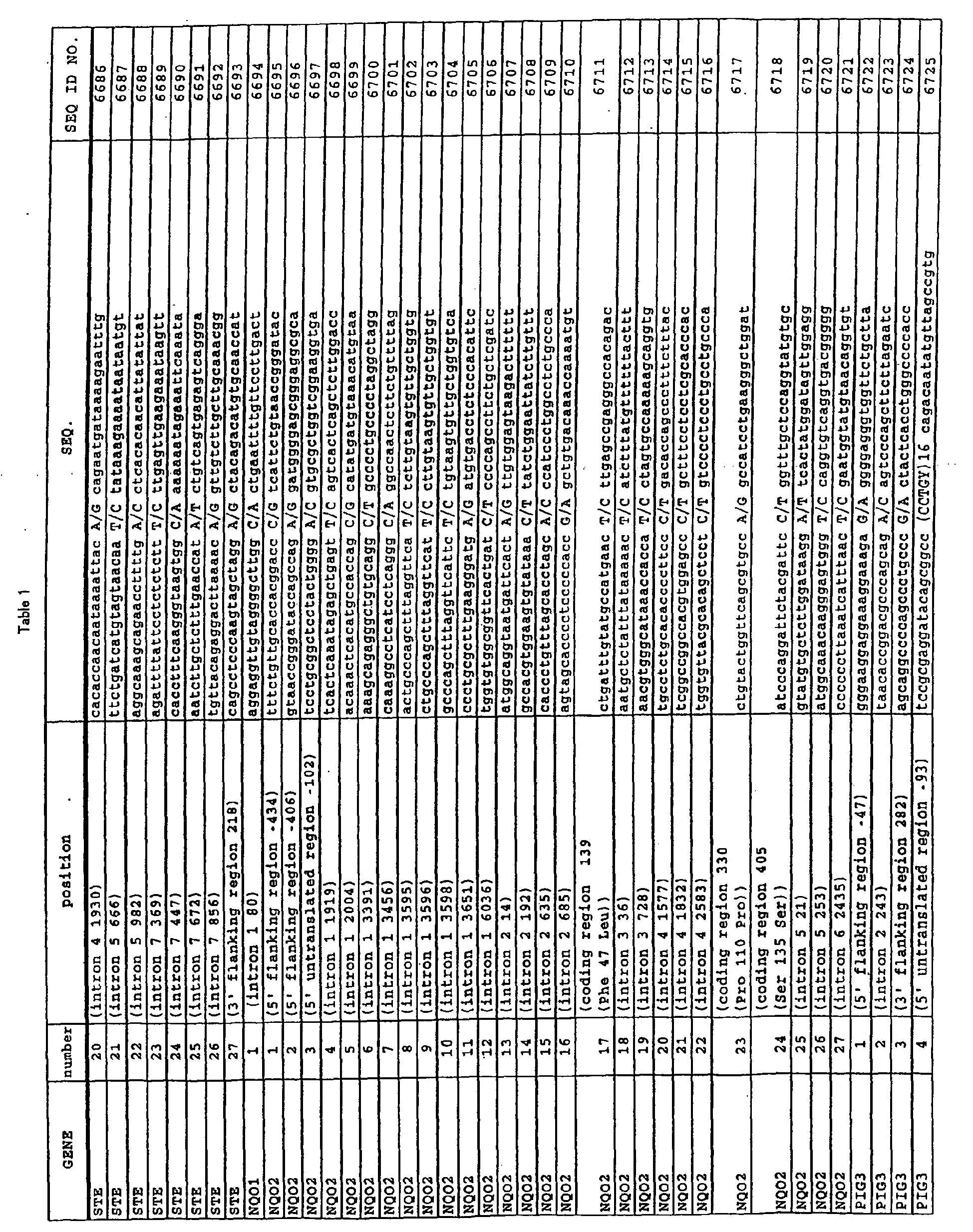

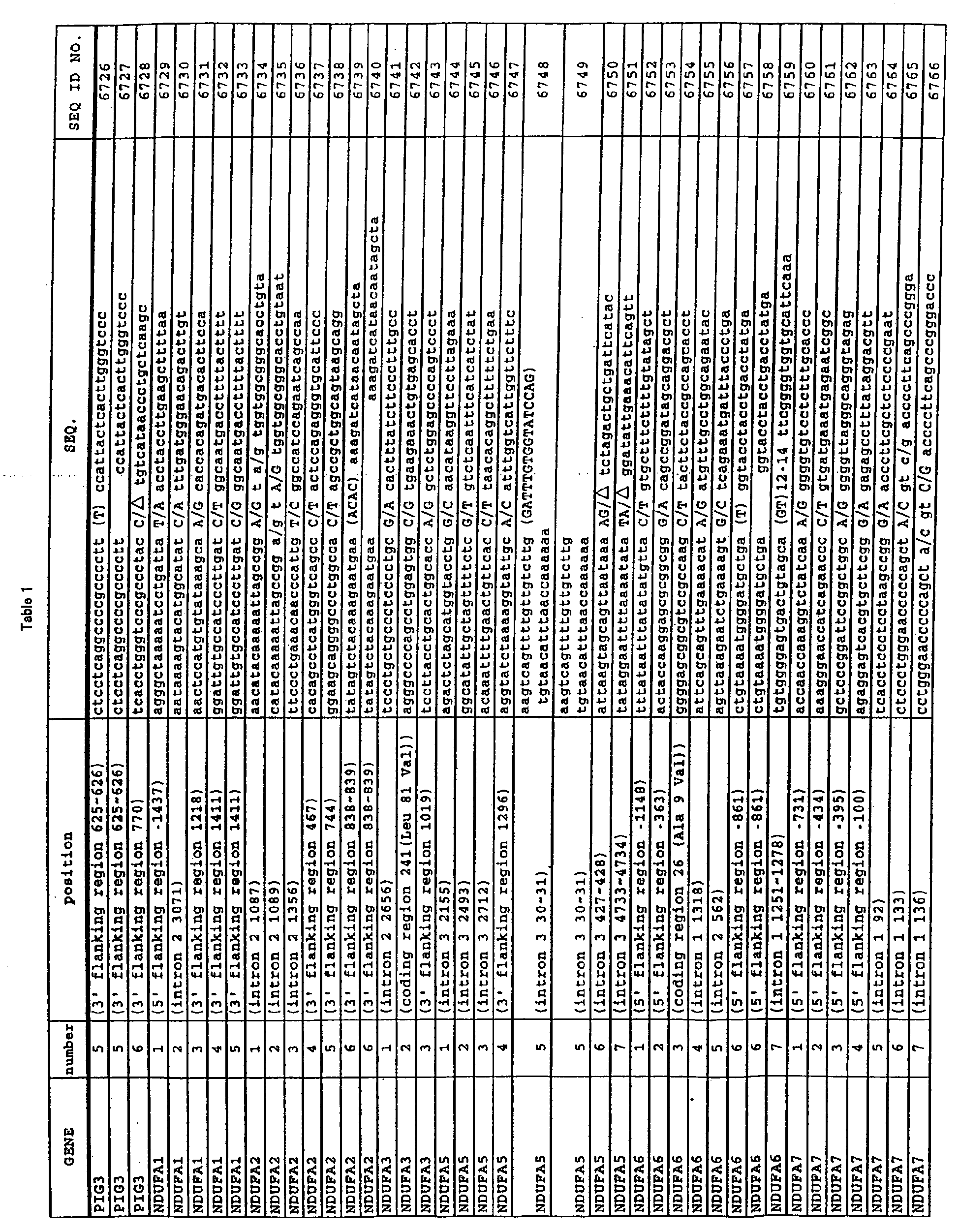

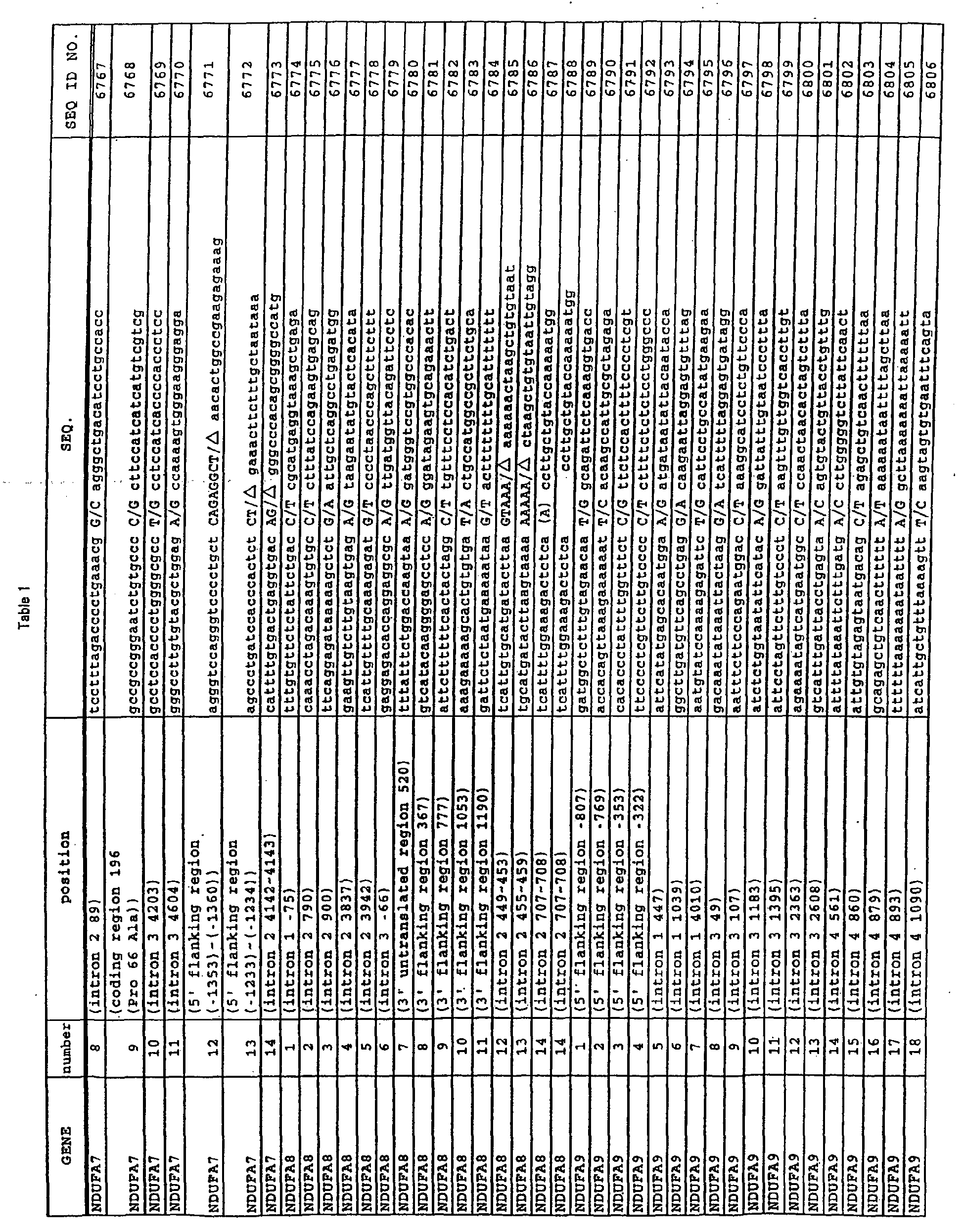

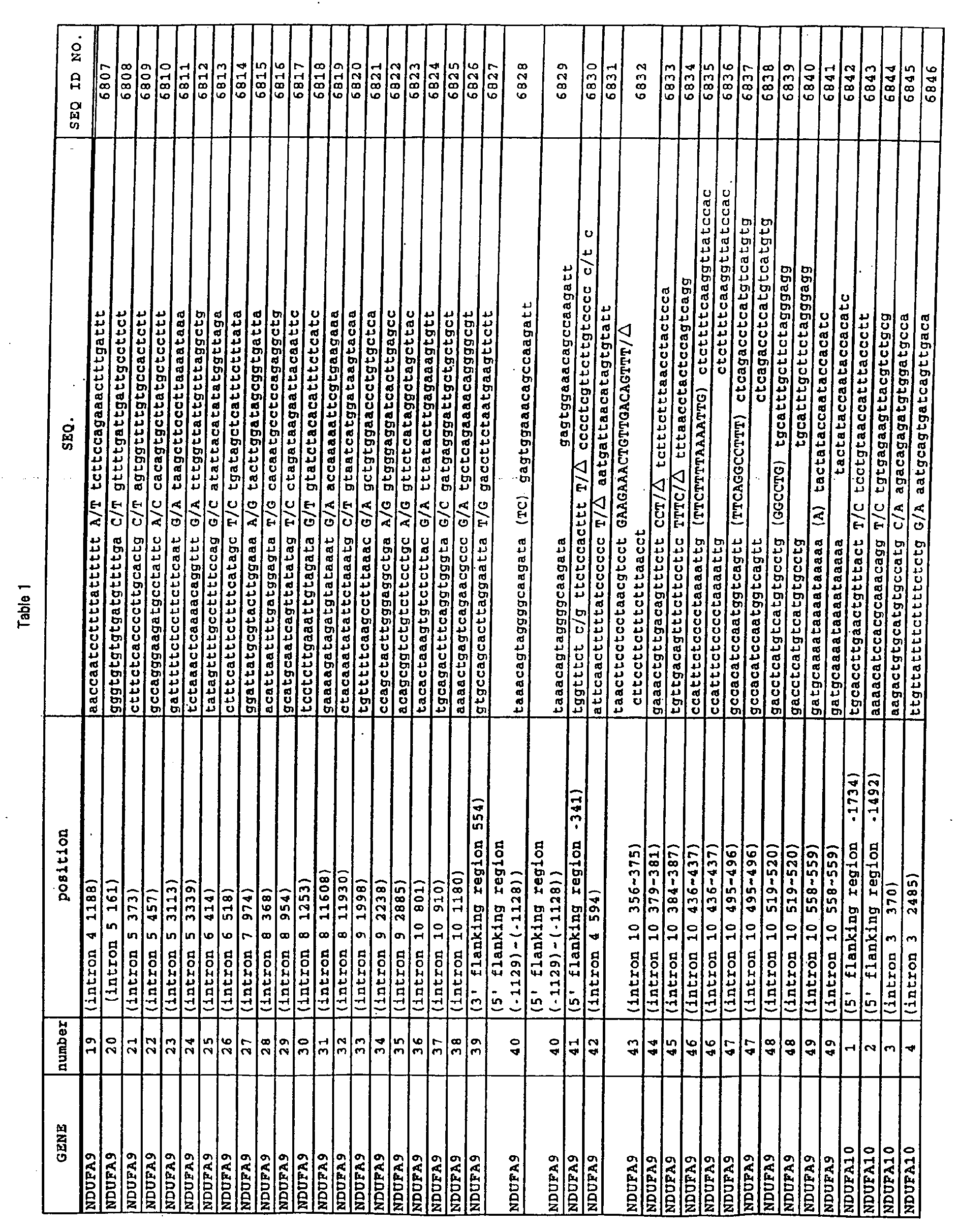

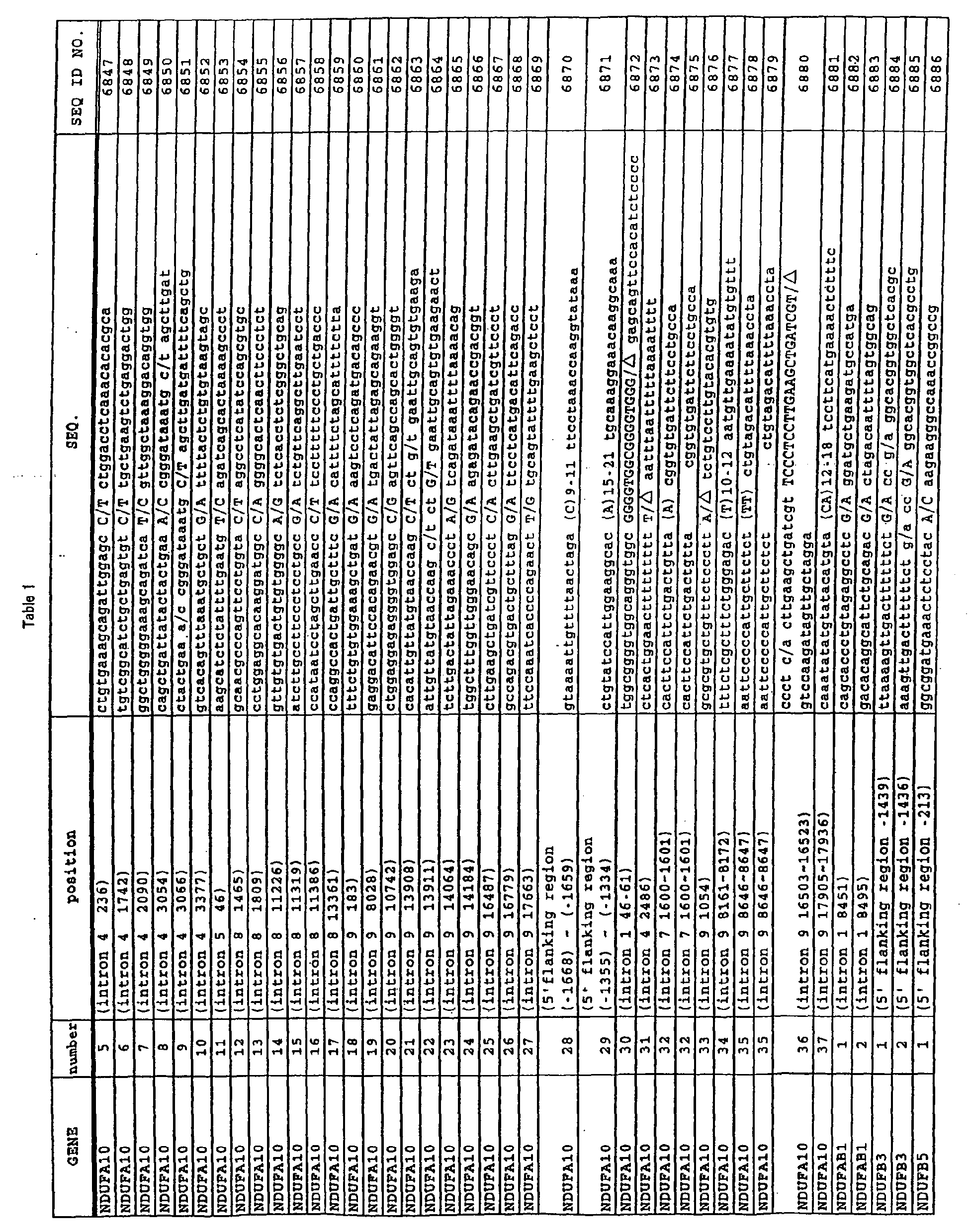

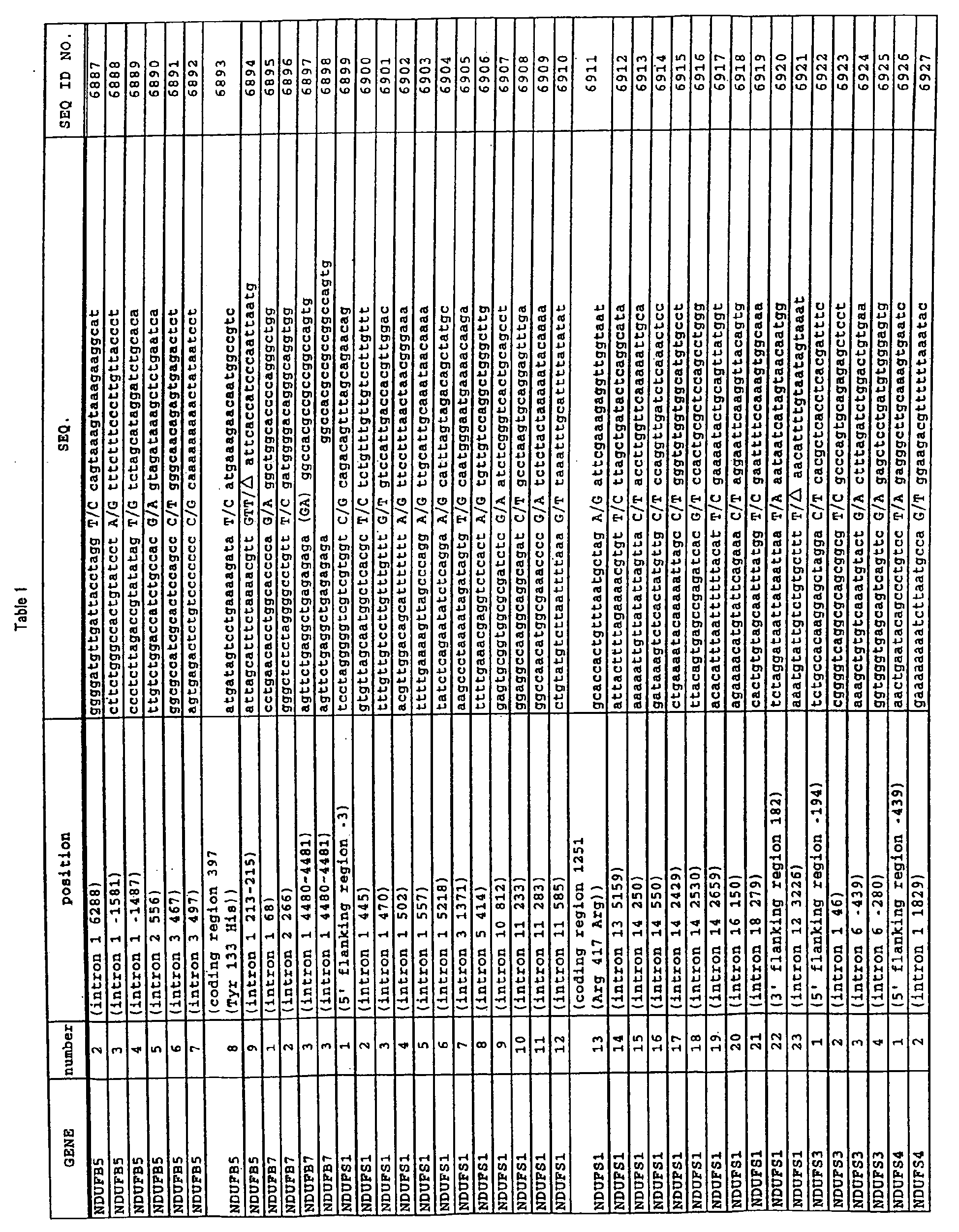

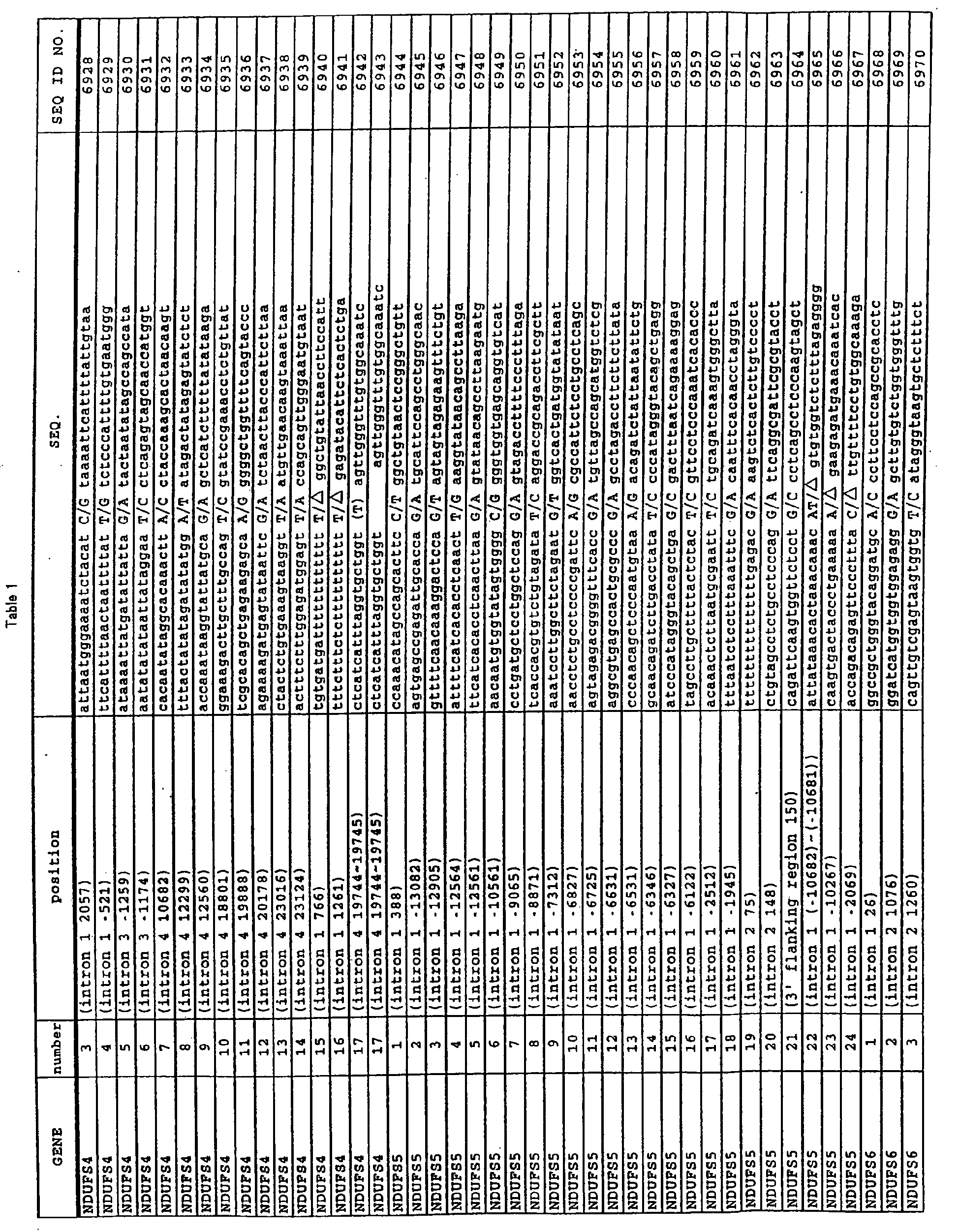

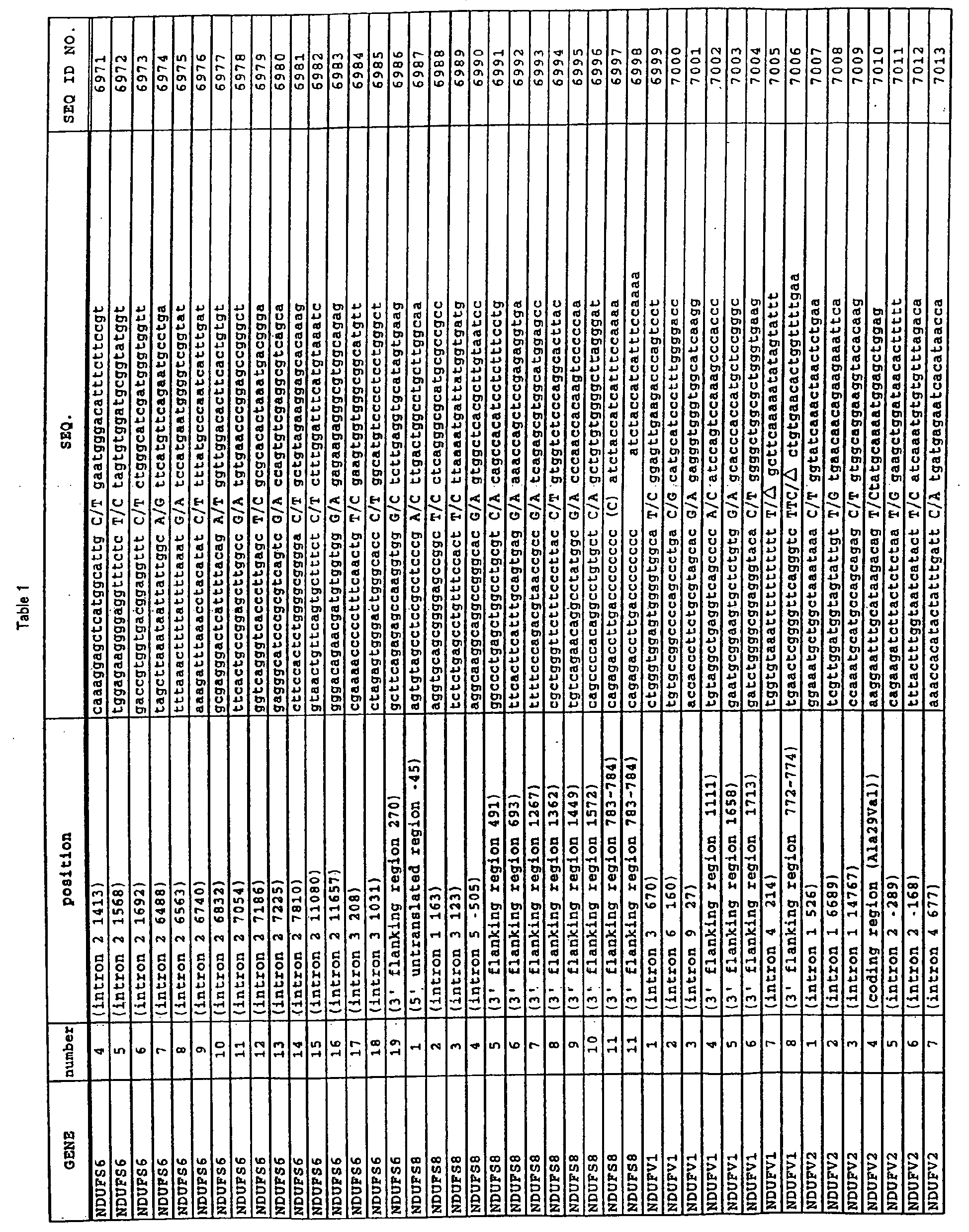

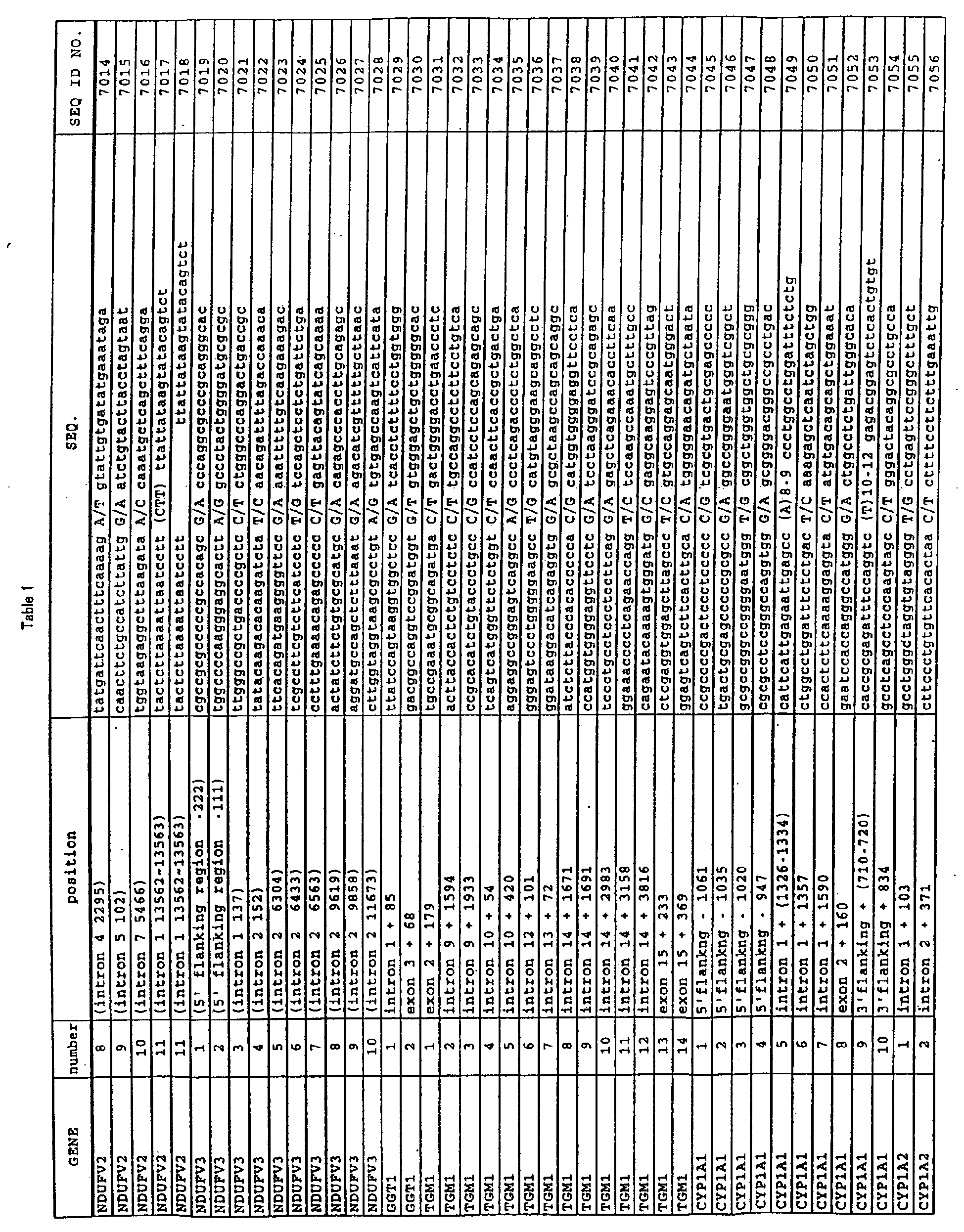

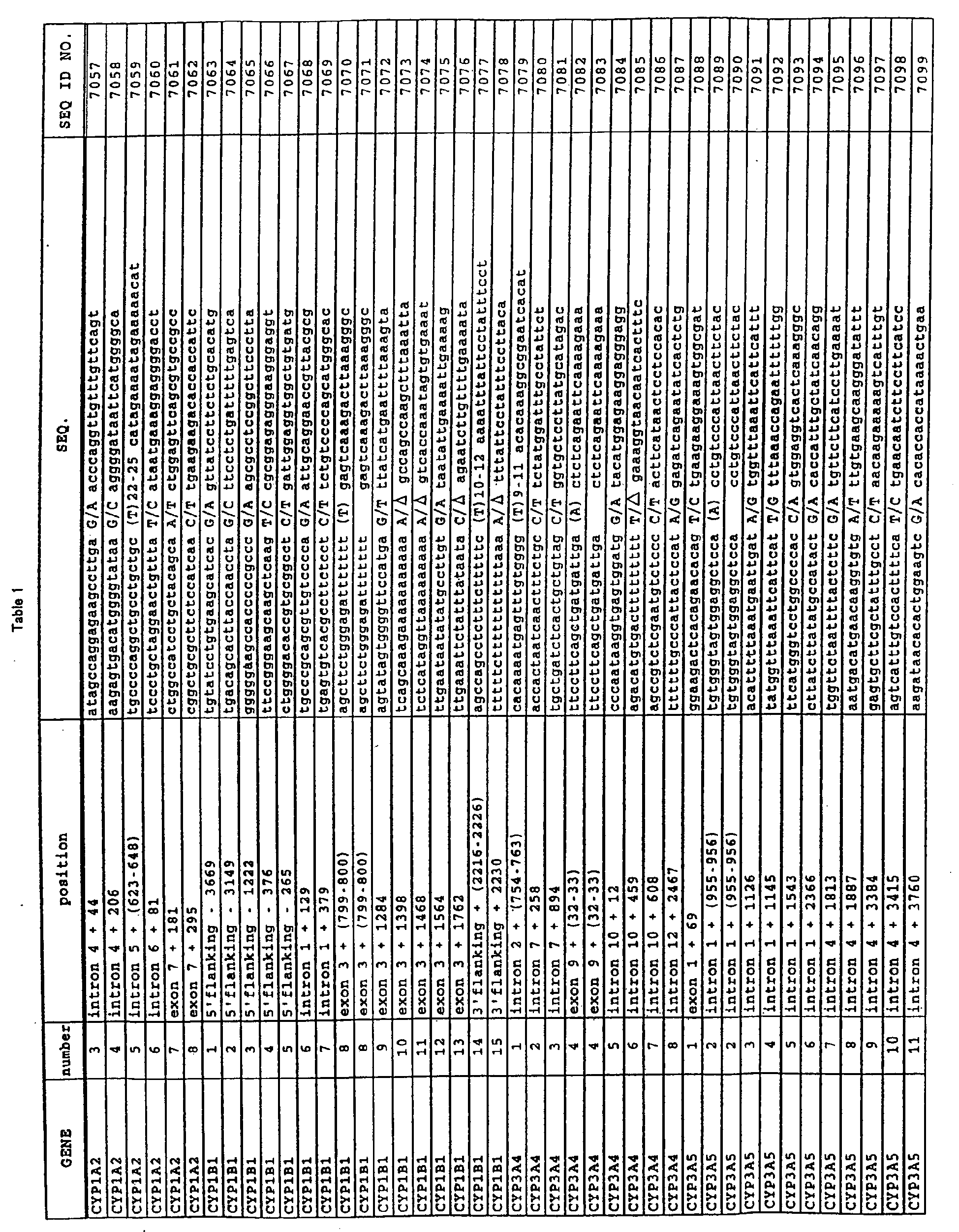

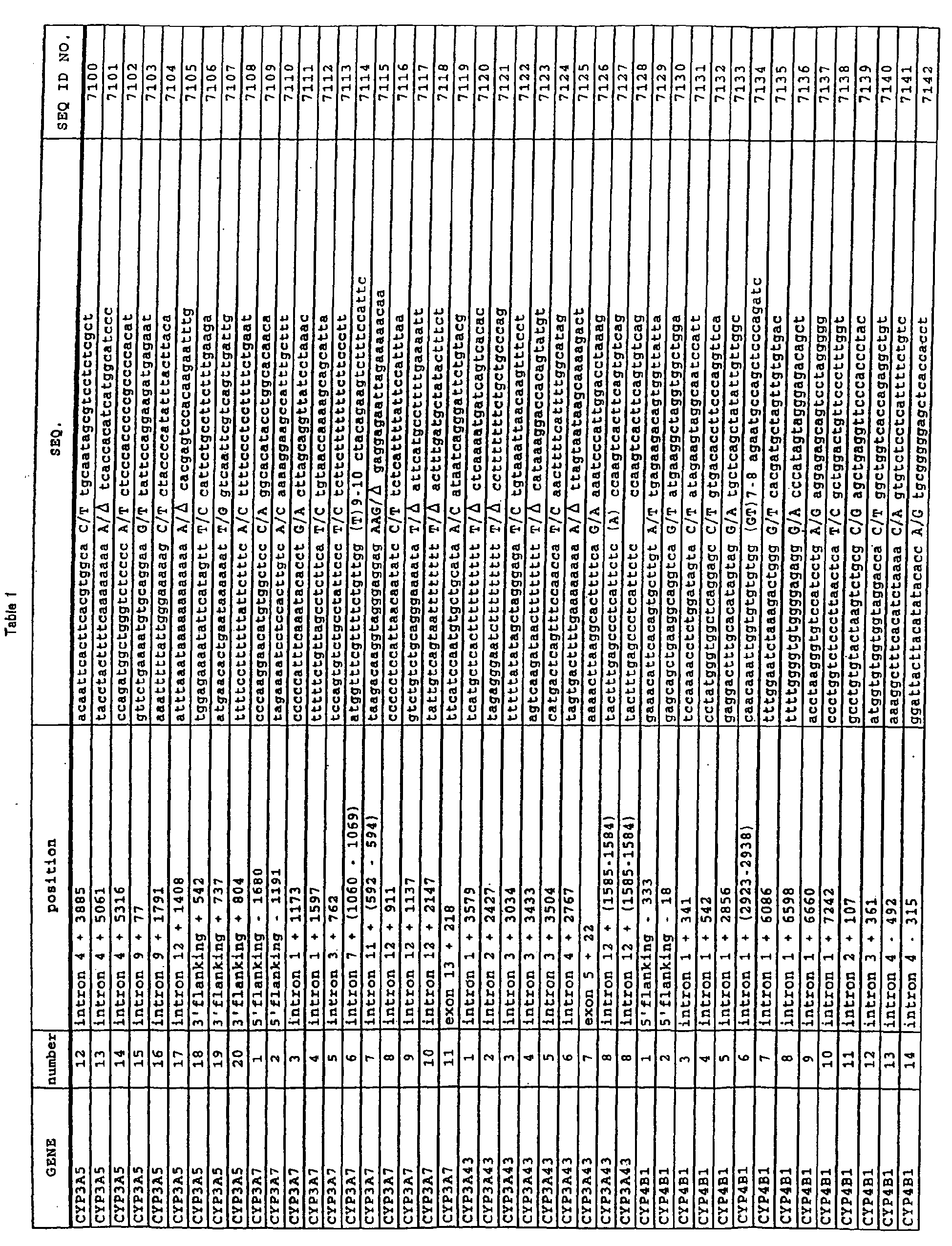

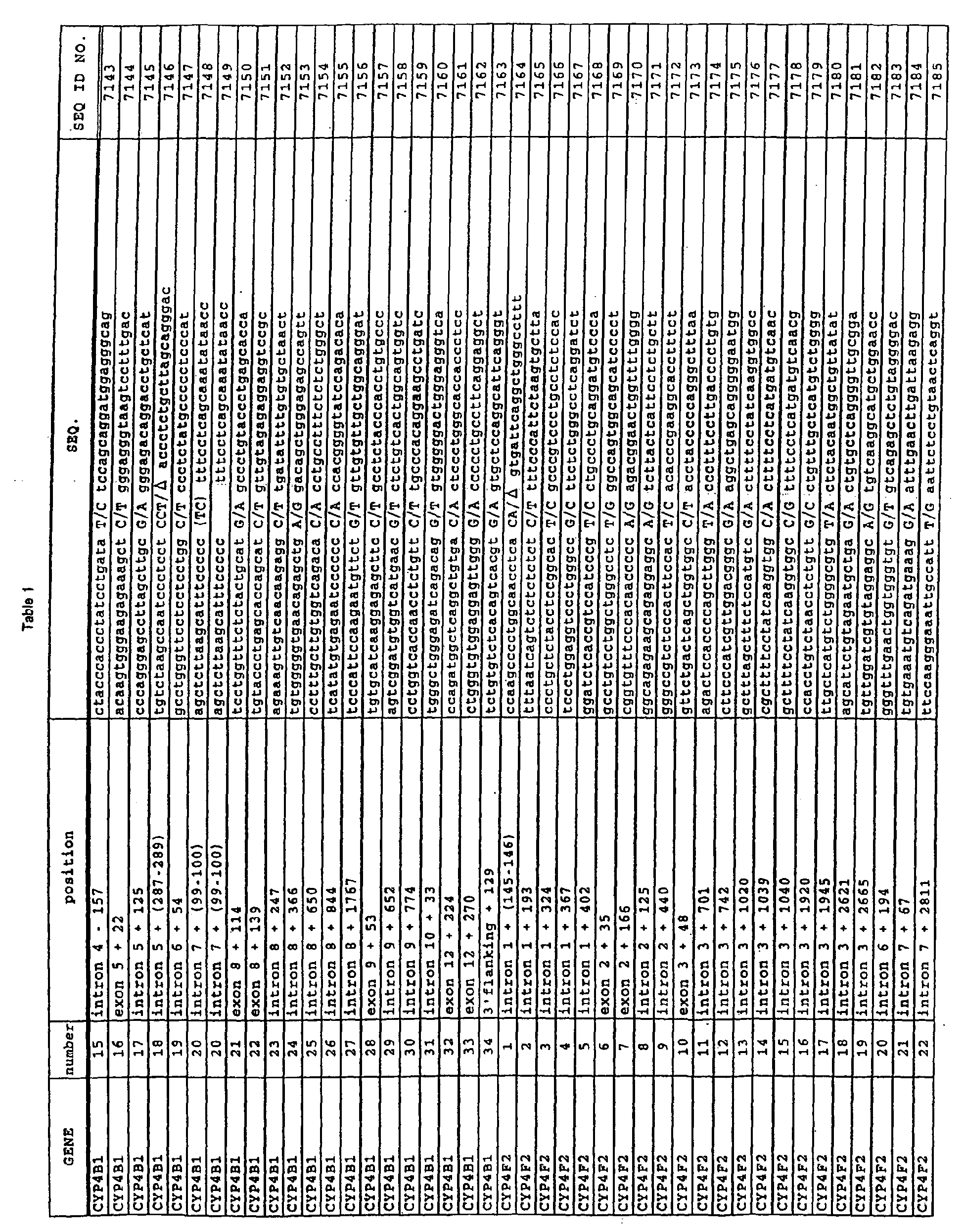

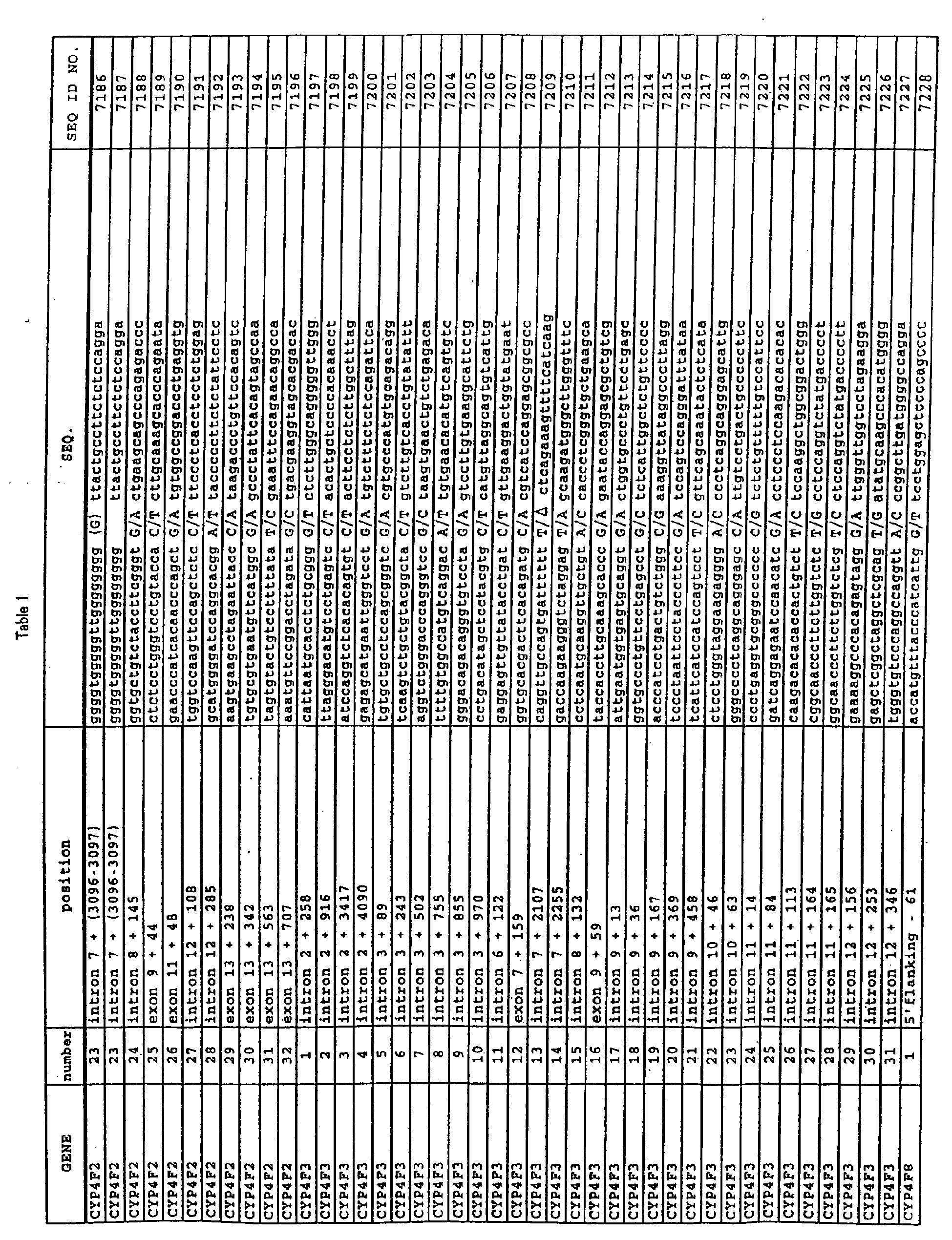

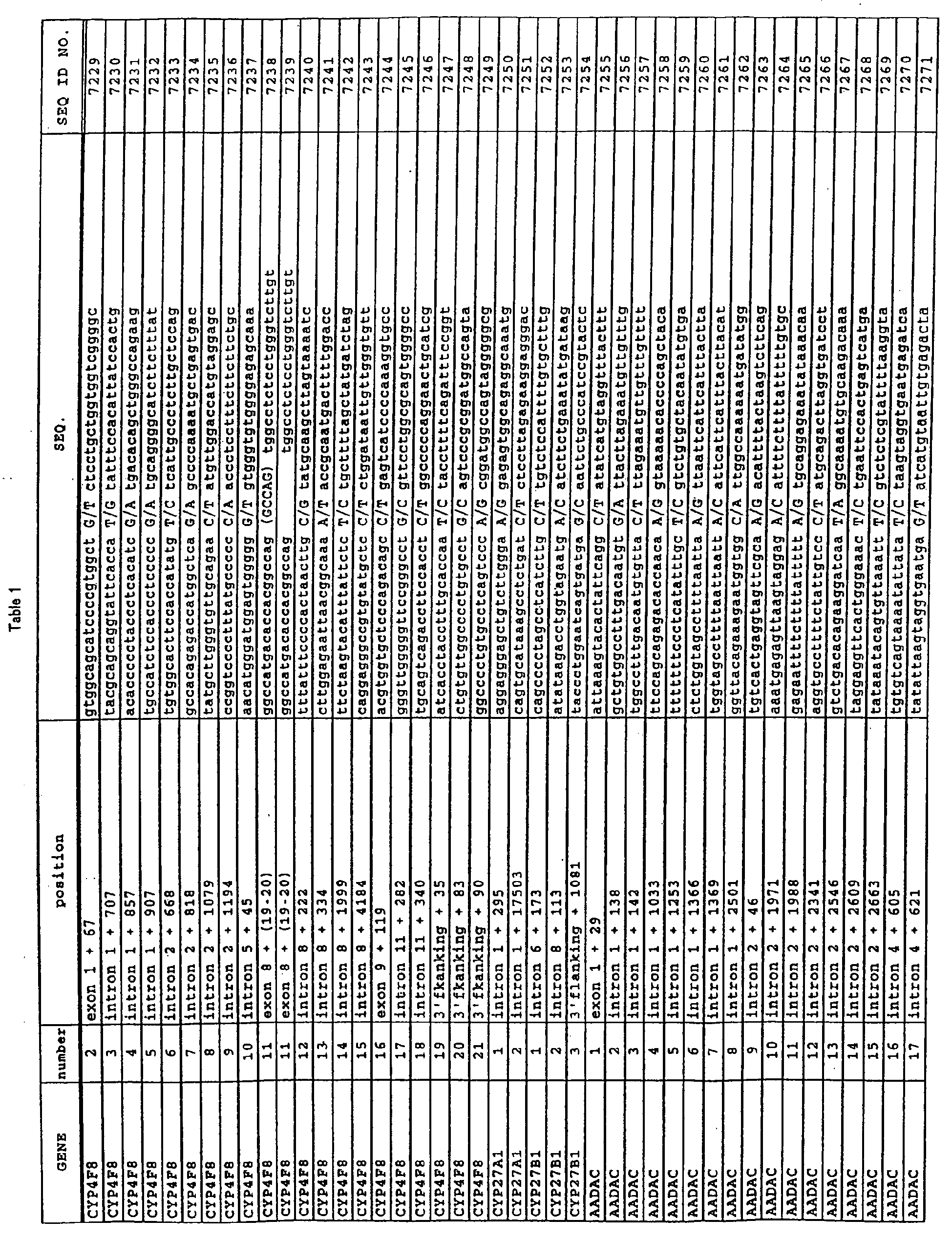

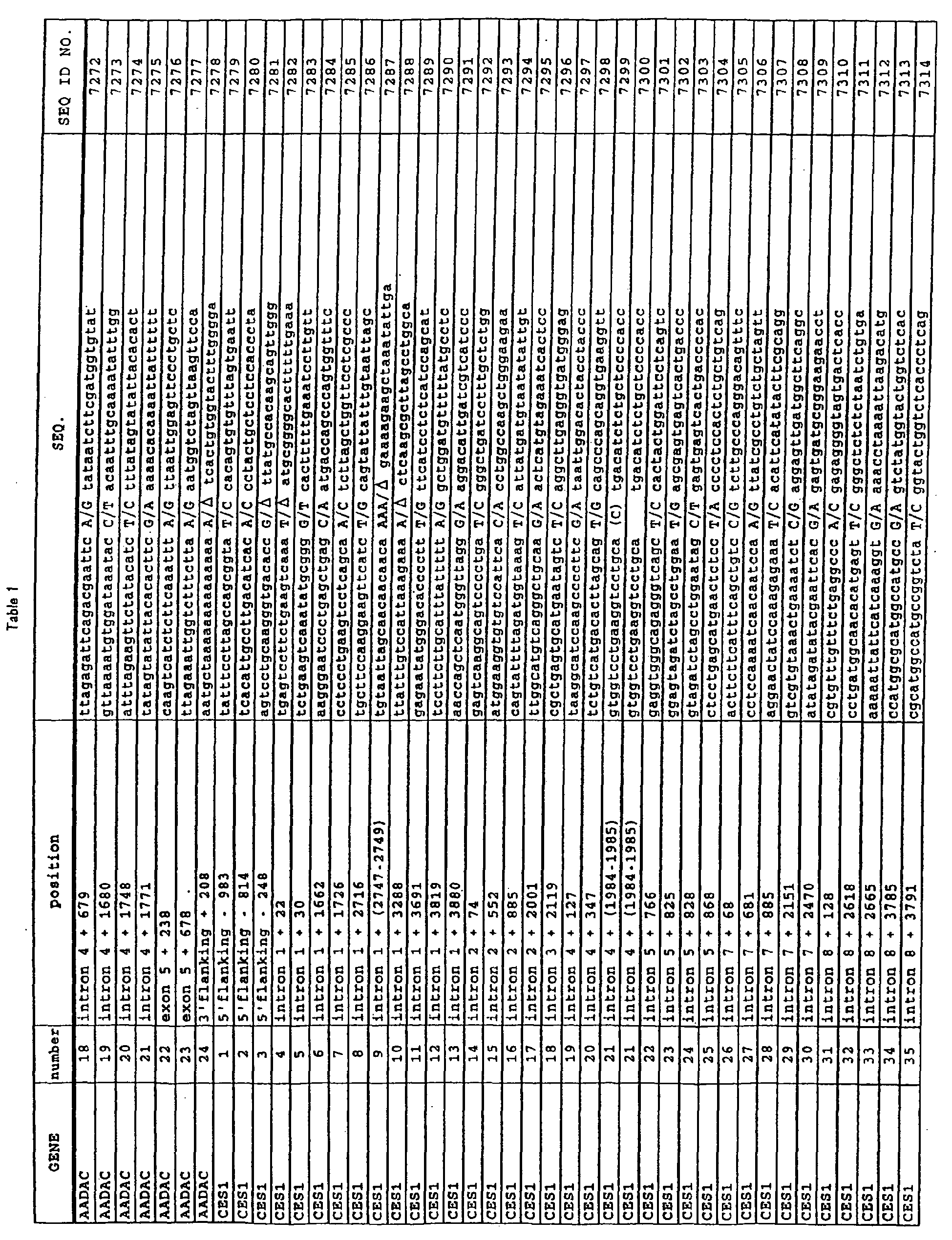

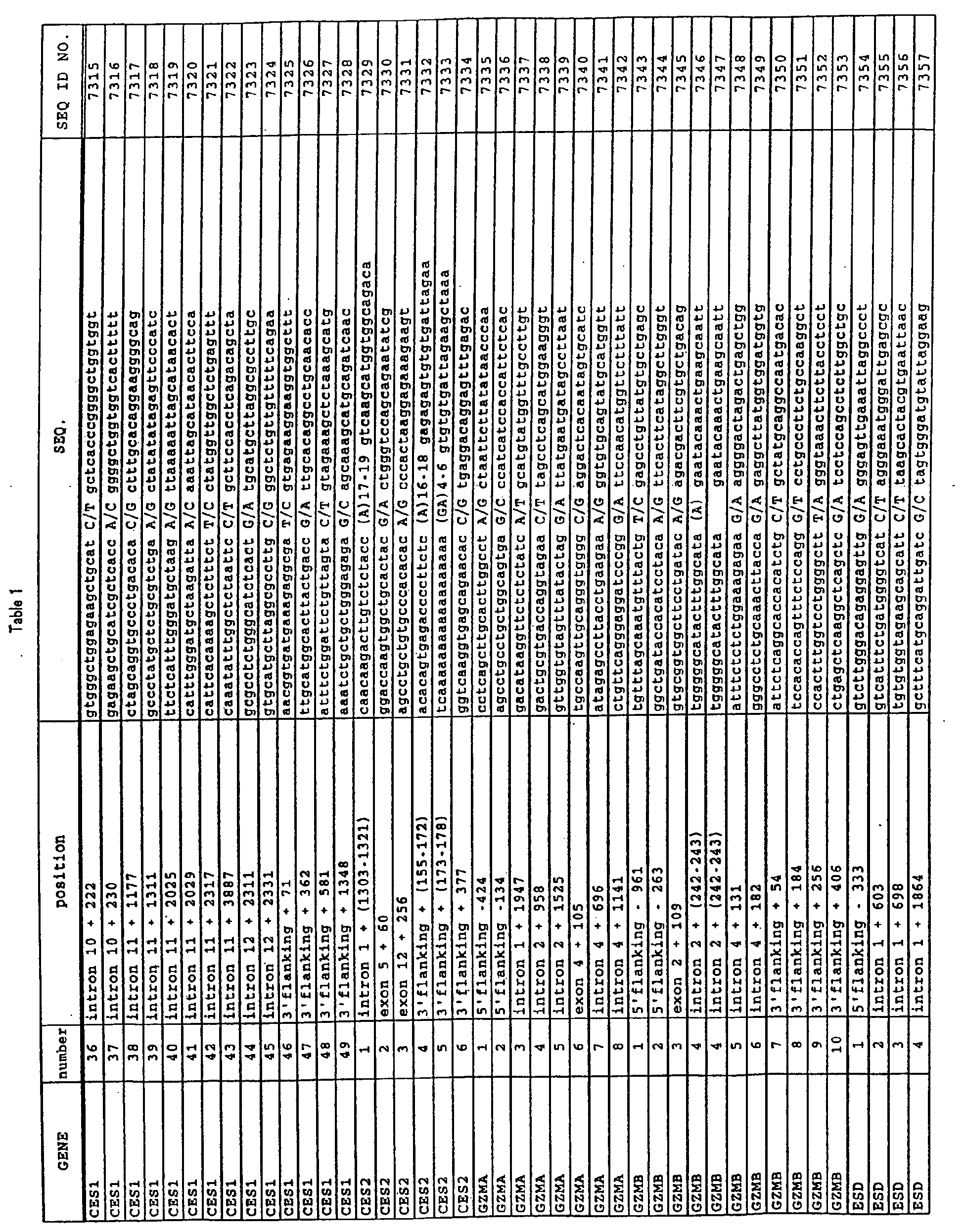

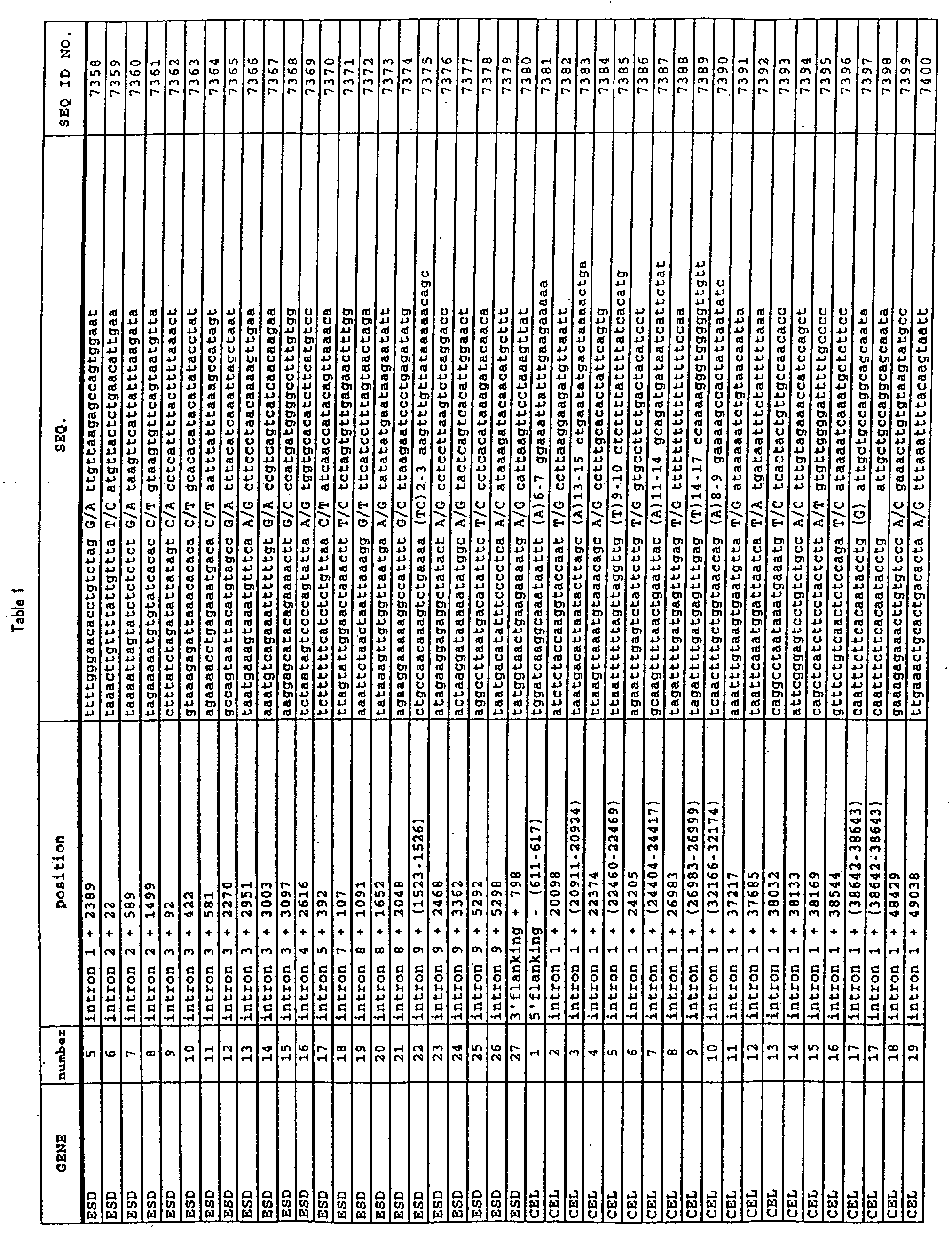

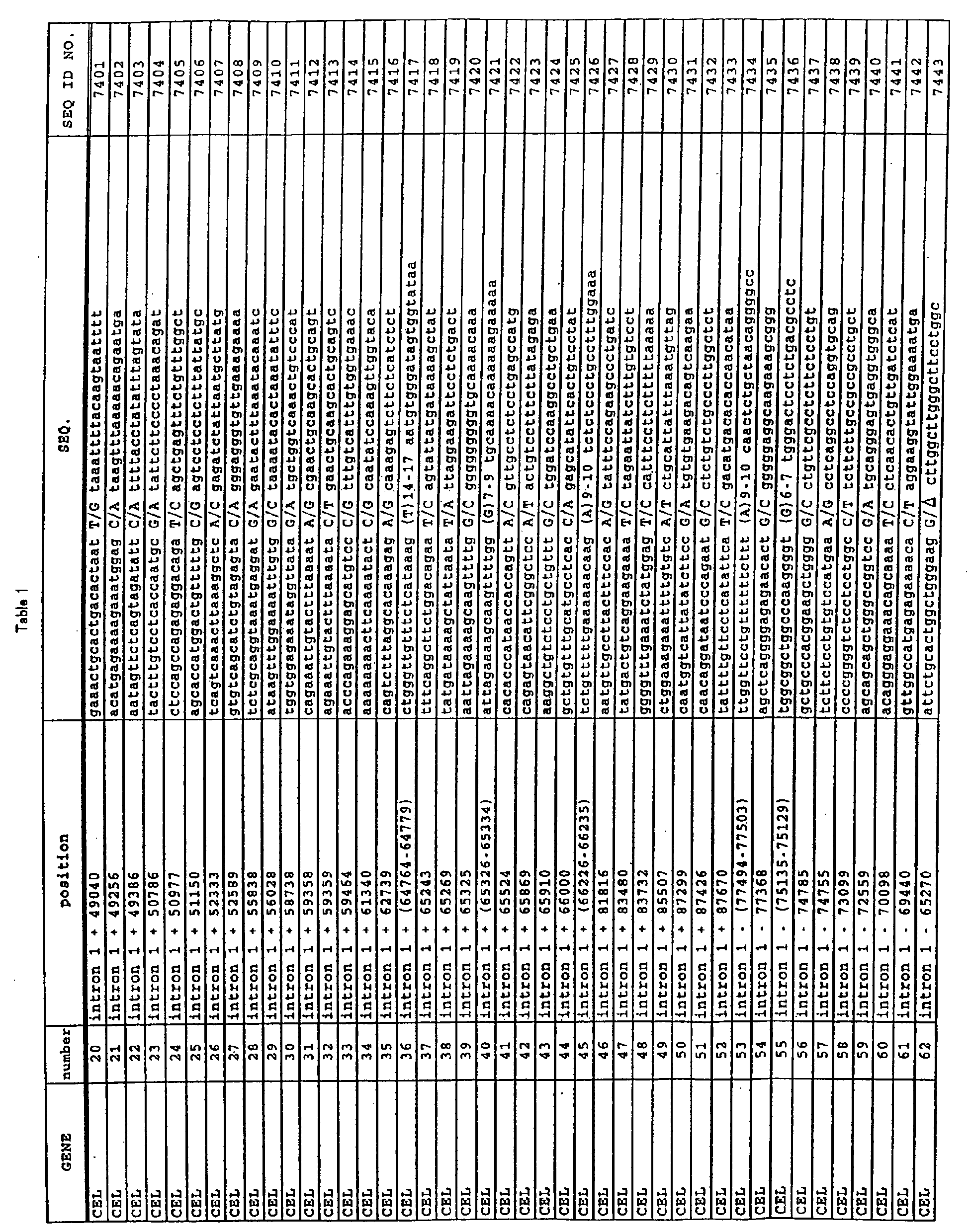

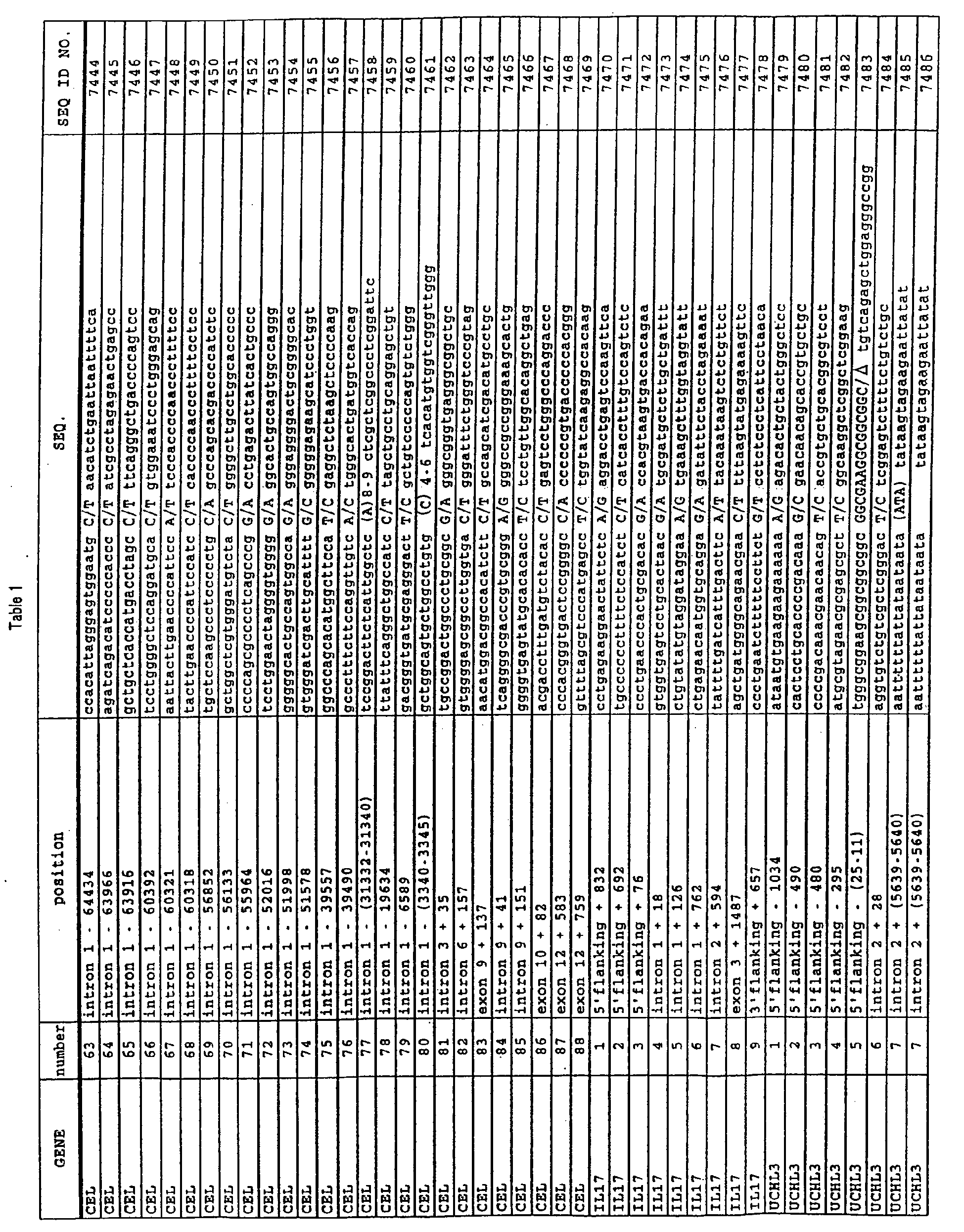

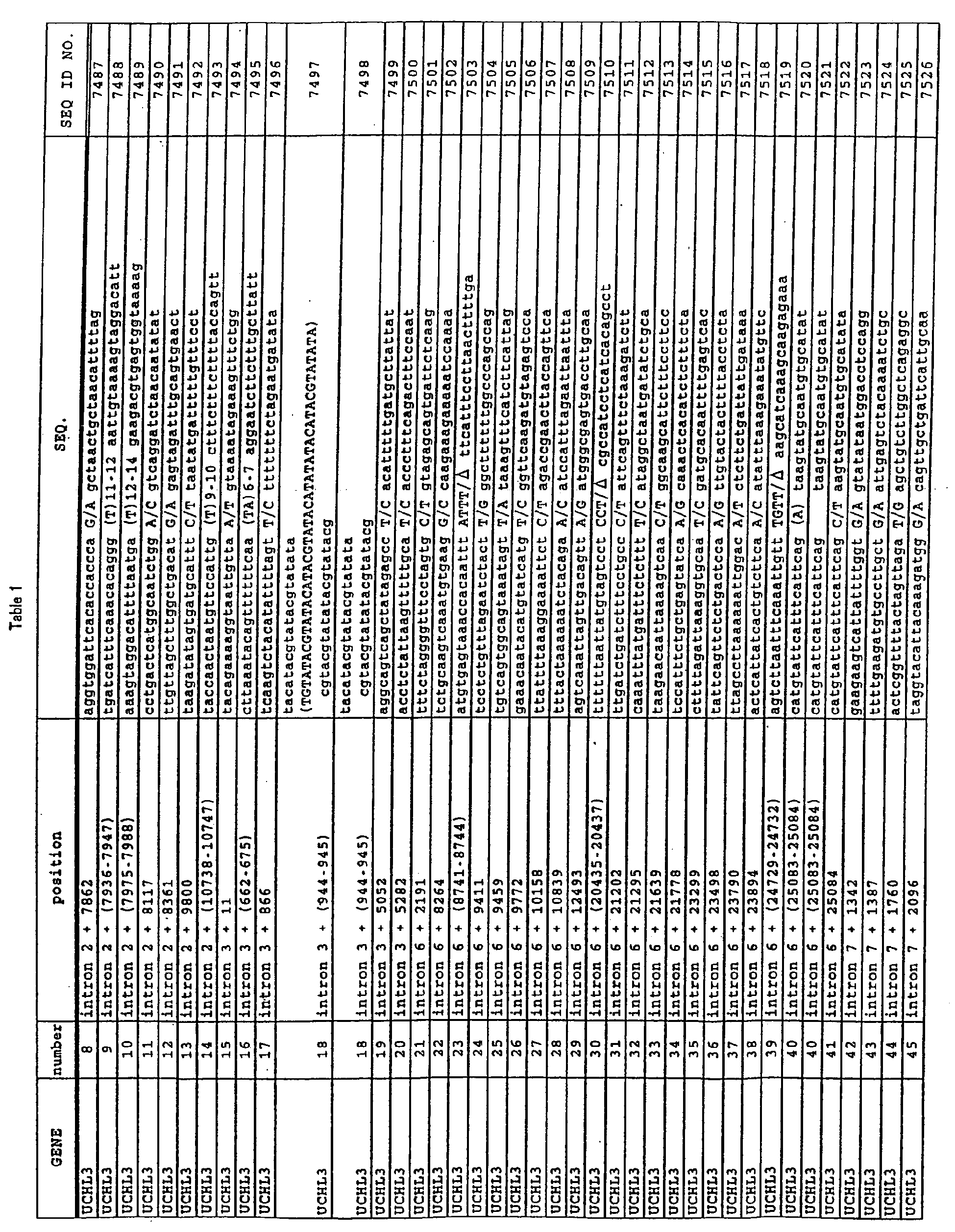

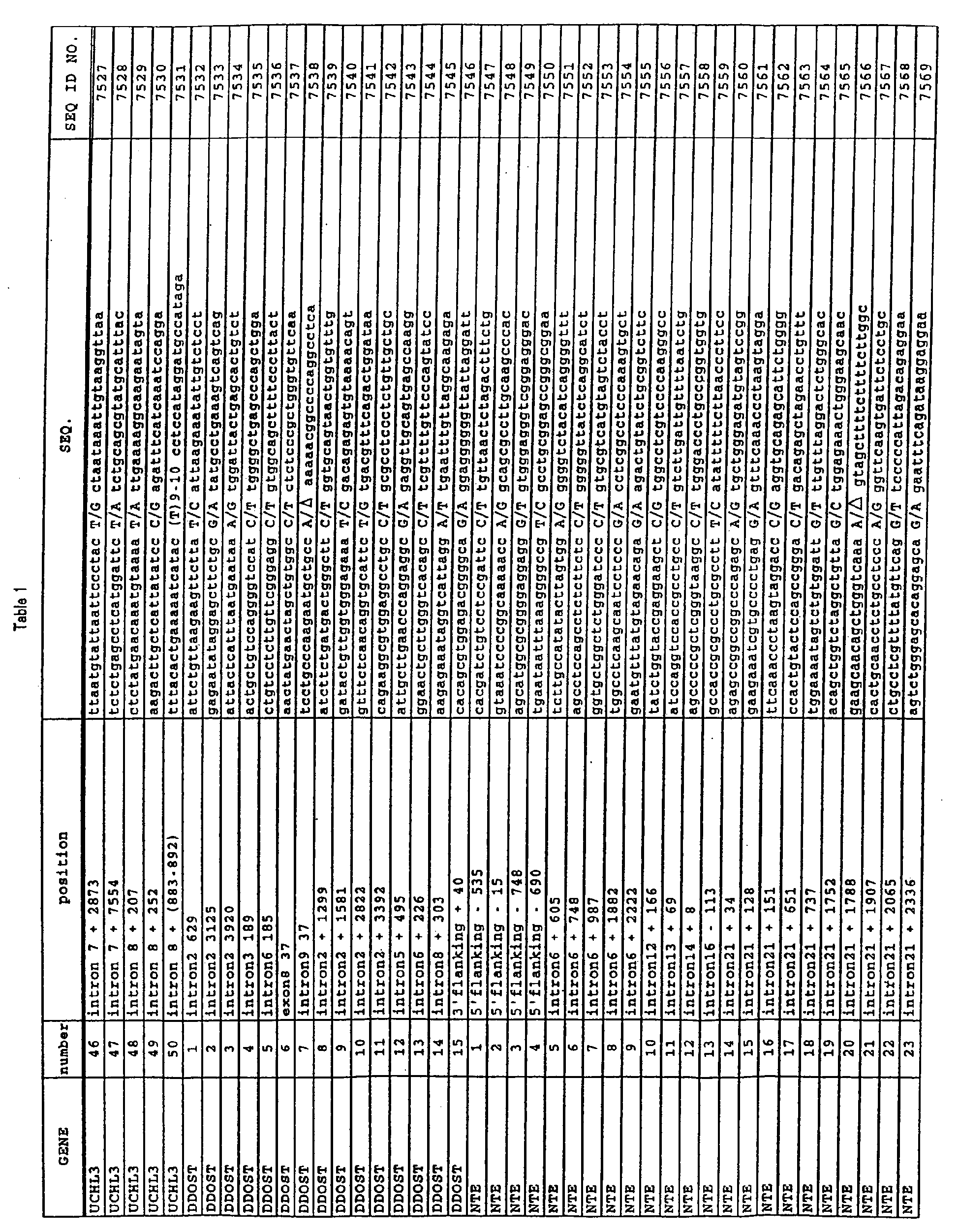

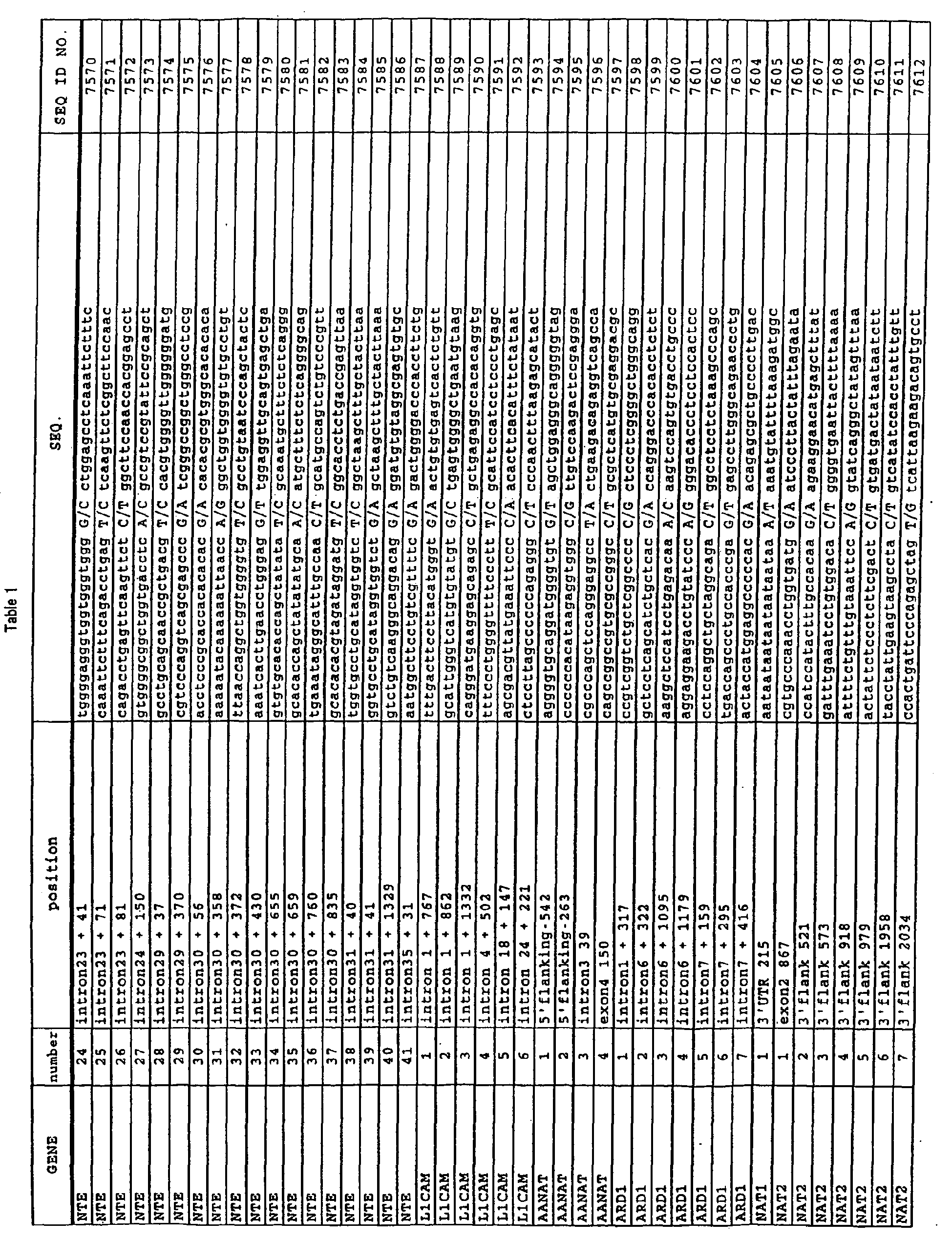

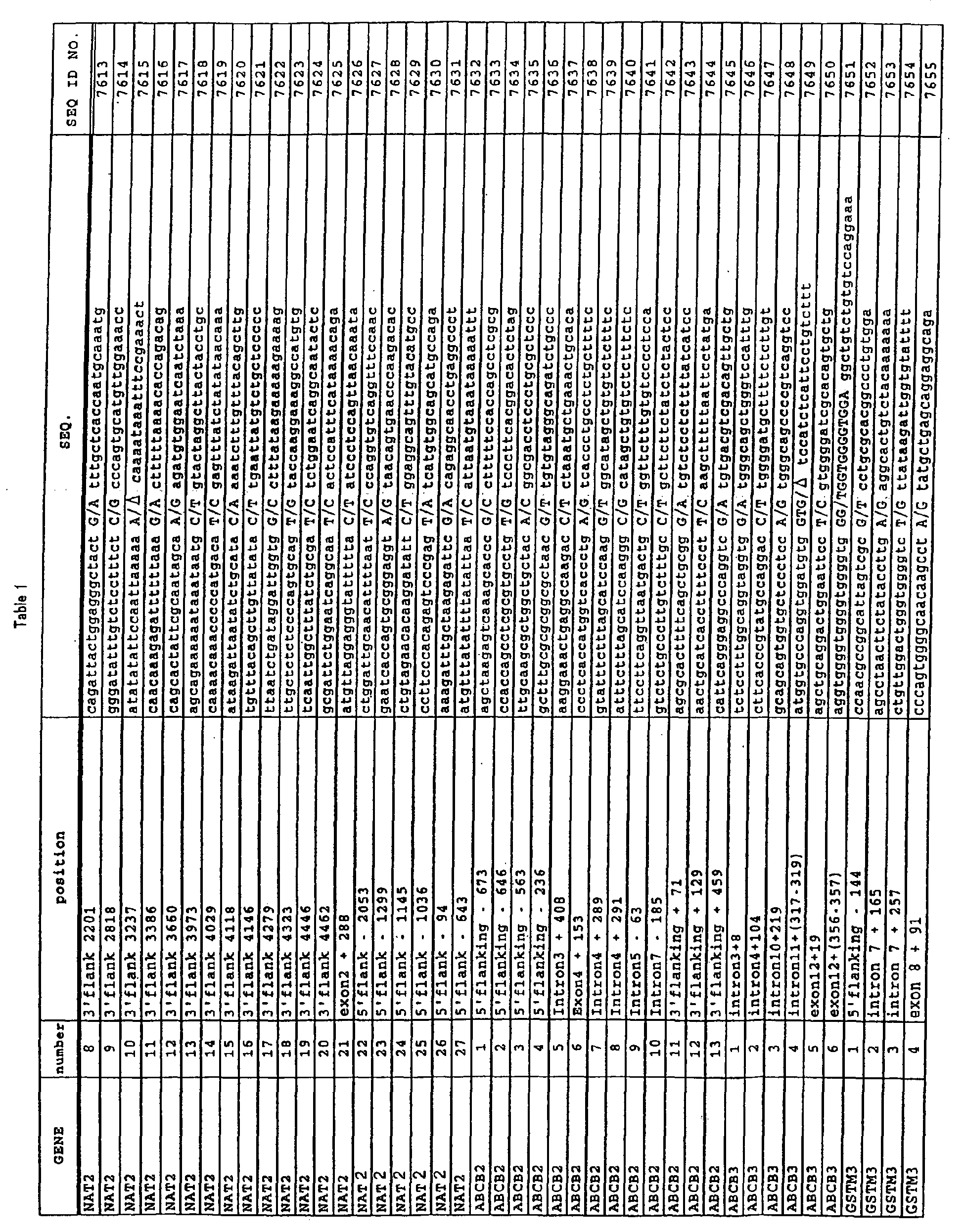

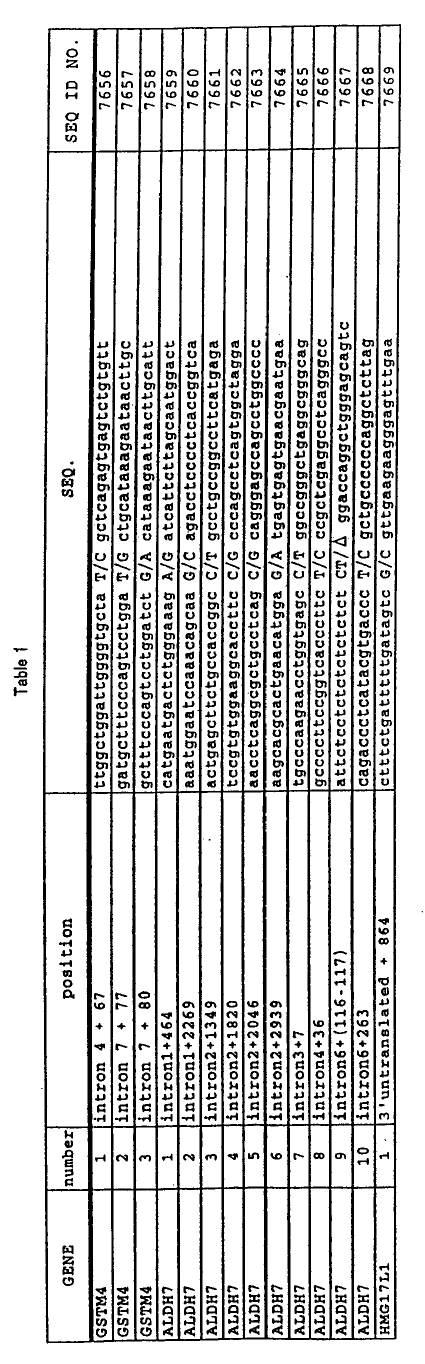

In Table 1, the name of the gene encoding the drug metabolizing enzyme is recorded in the gene name column. The base in capital letters is the SNP data in the sequence column. Two bases separated by a forward slash indicate the SNP of homo and hetero bases. For example, A/G indicates a homo allele A/A and G/G as well as a hetero allele A/G. The sequences in this table have 20 bases before and after the SNP. Here, the base in parentheses, for example the 26th (T) in ABCB4, indicates a polymorphism with an inserted base, and D, such as the 10th spot in NAT2, indicates a polymorphism with a deleted base. In Sequence No. 674, n is VNTR and (cctgy)x, where x is an integer between 1 and 50, indicates a repeated sequence. The bases with numbers in parentheses indicate the number of times they are repeated. For example, "(T) 9-12" in Sequence No. 1552 (ABCB11 No. 55 in Table 1) indicates T is repeated 9 to 12 times.

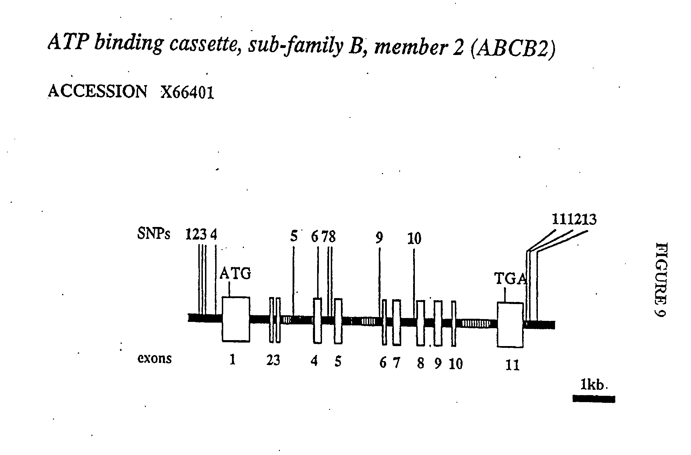

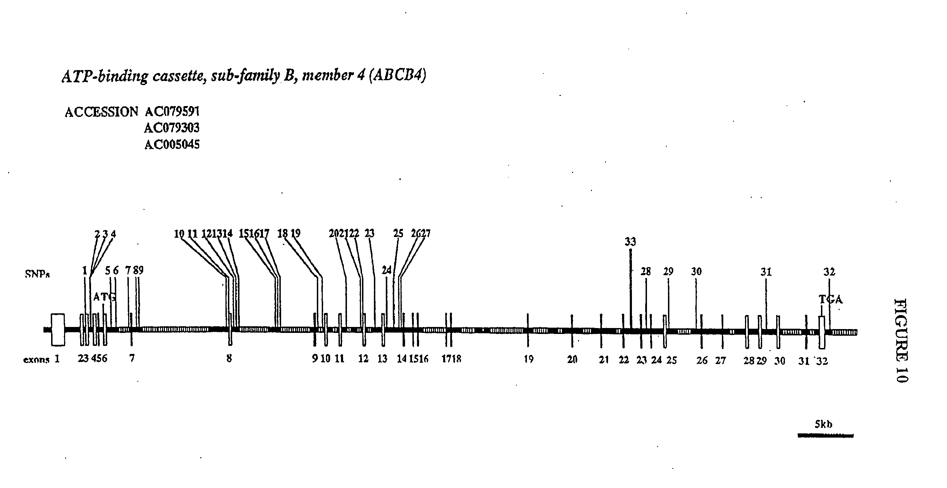

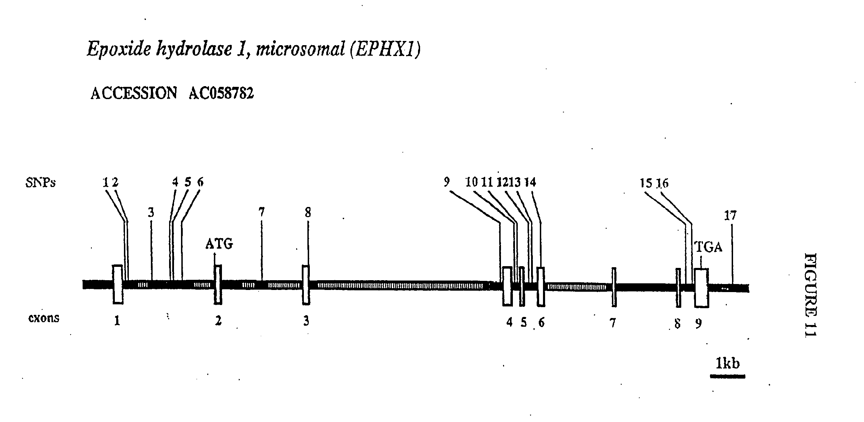

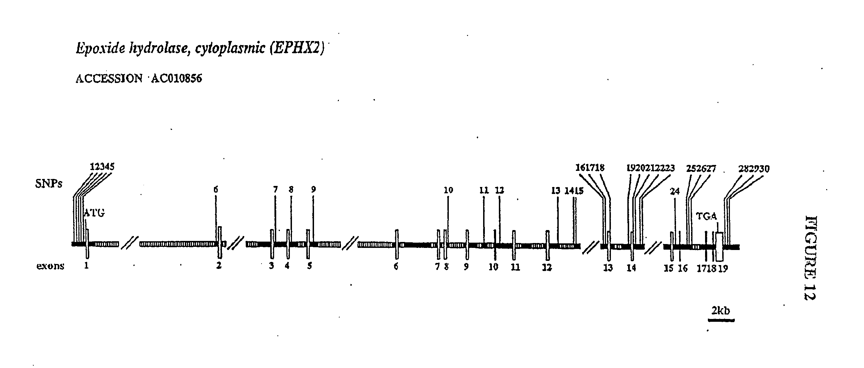

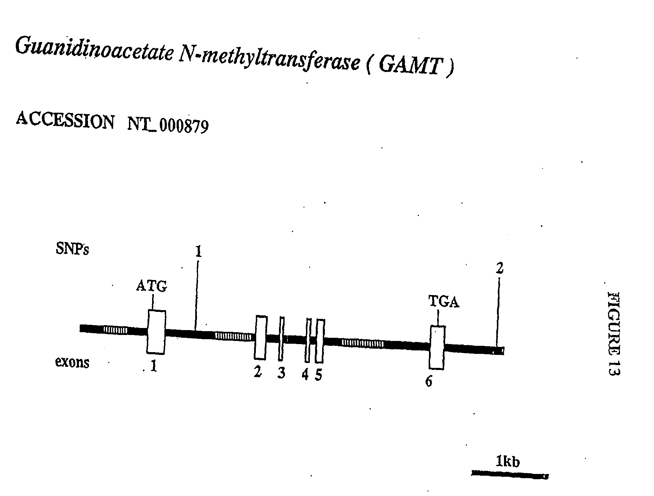

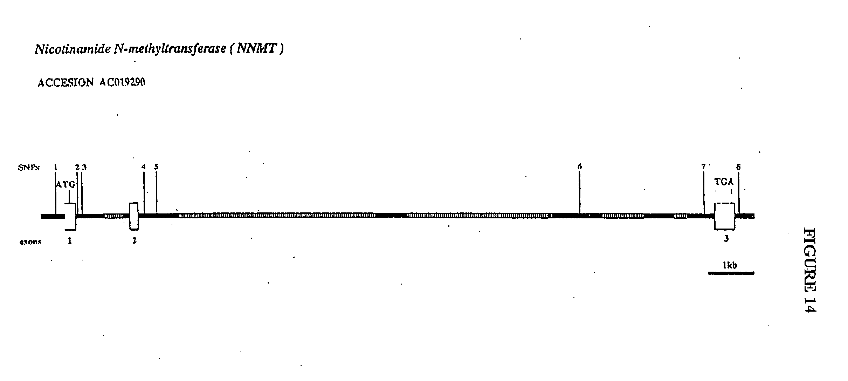

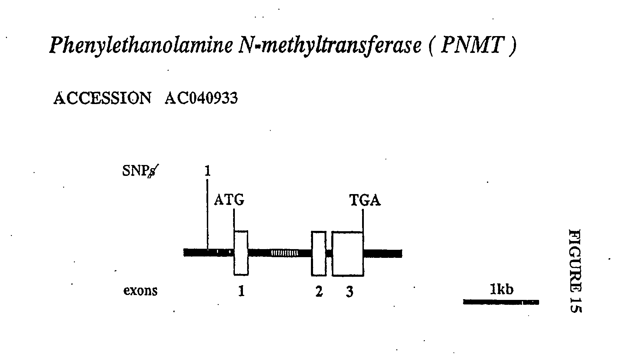

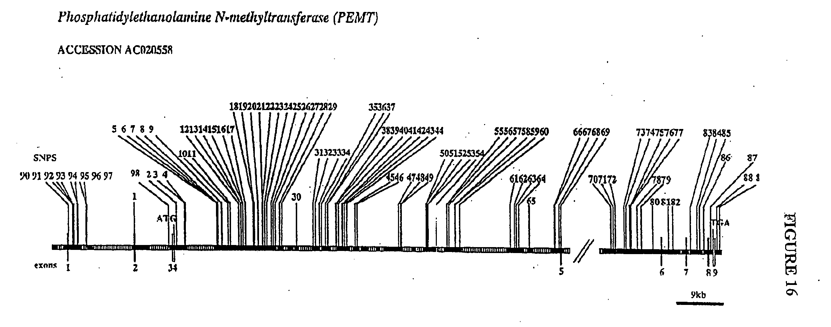

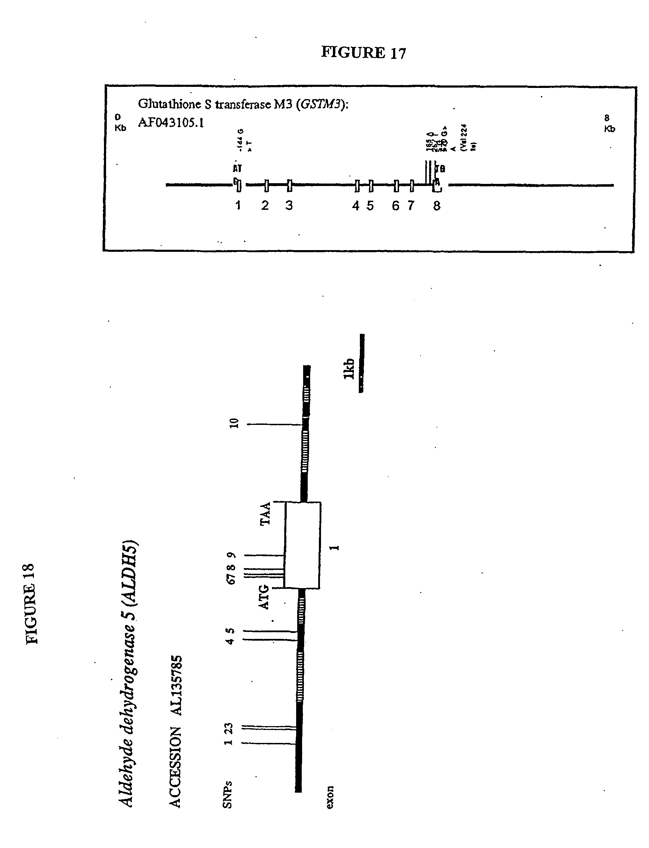

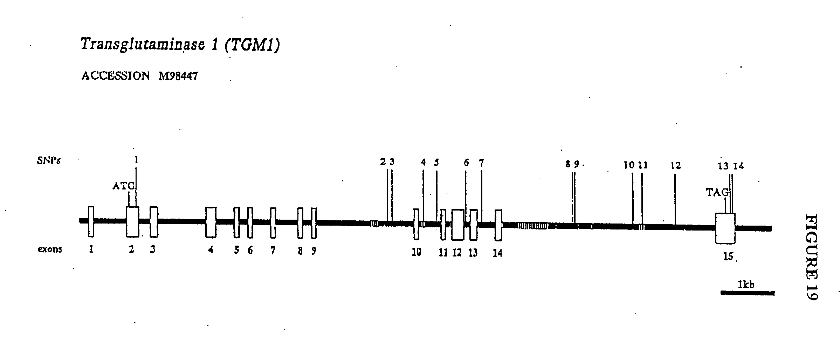

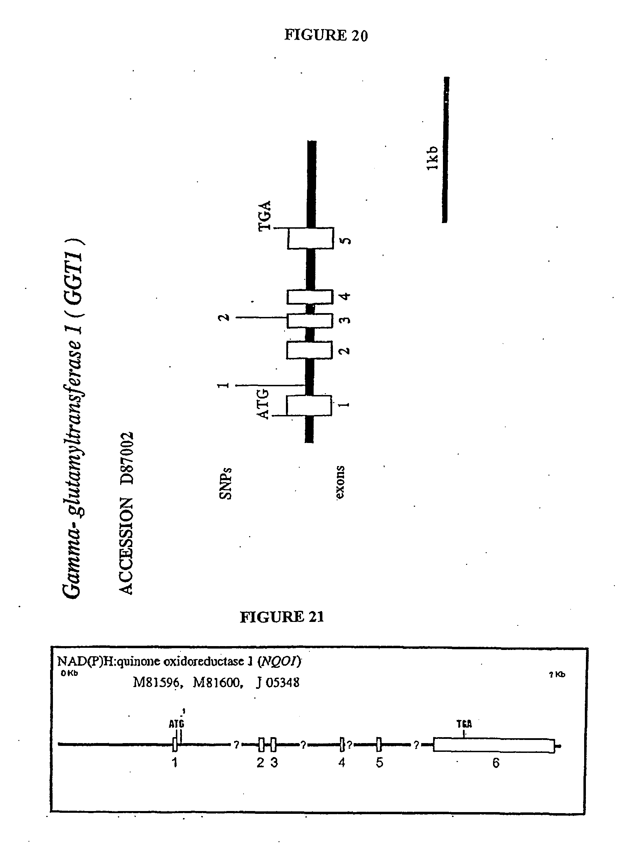

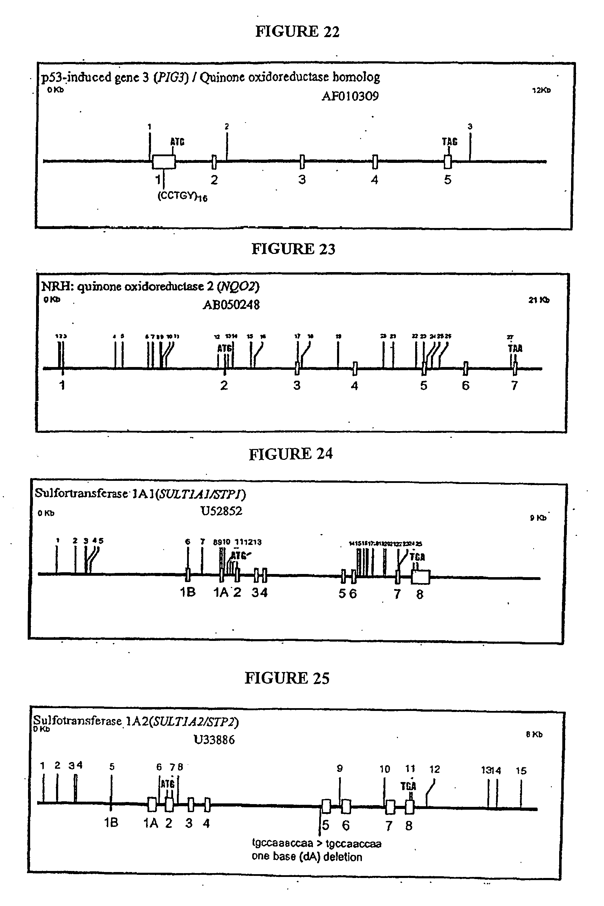

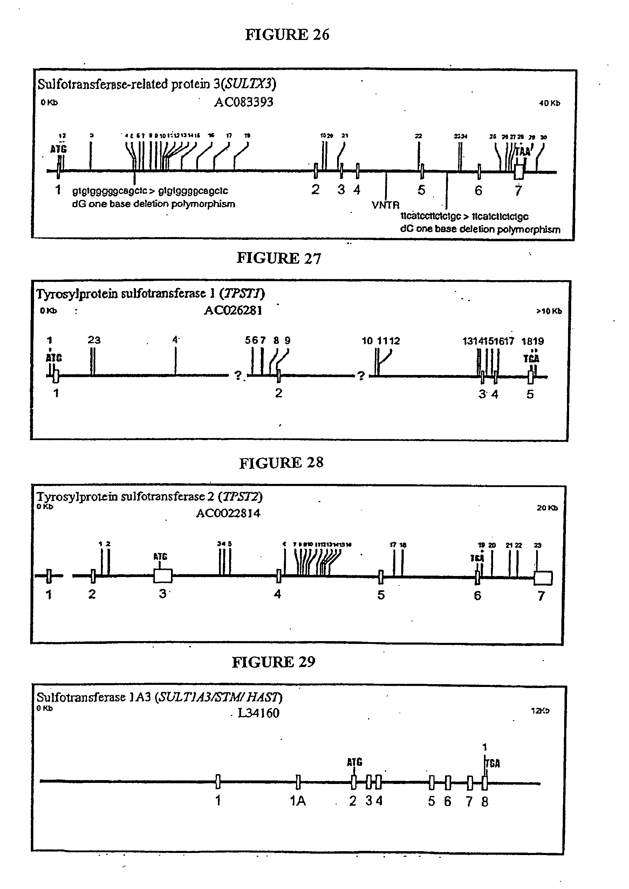

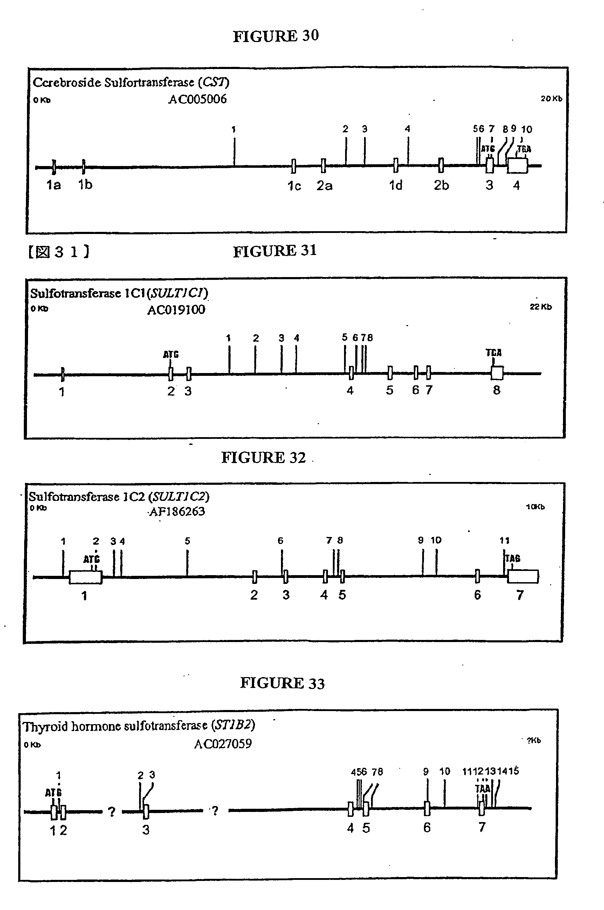

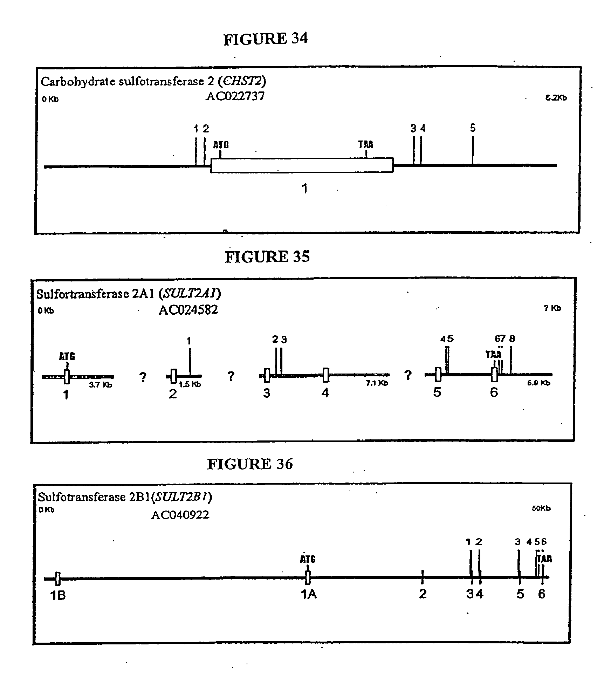

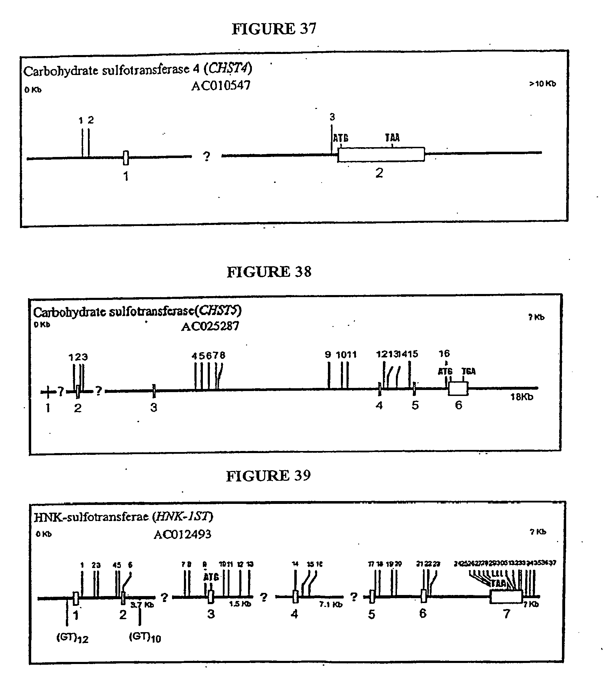

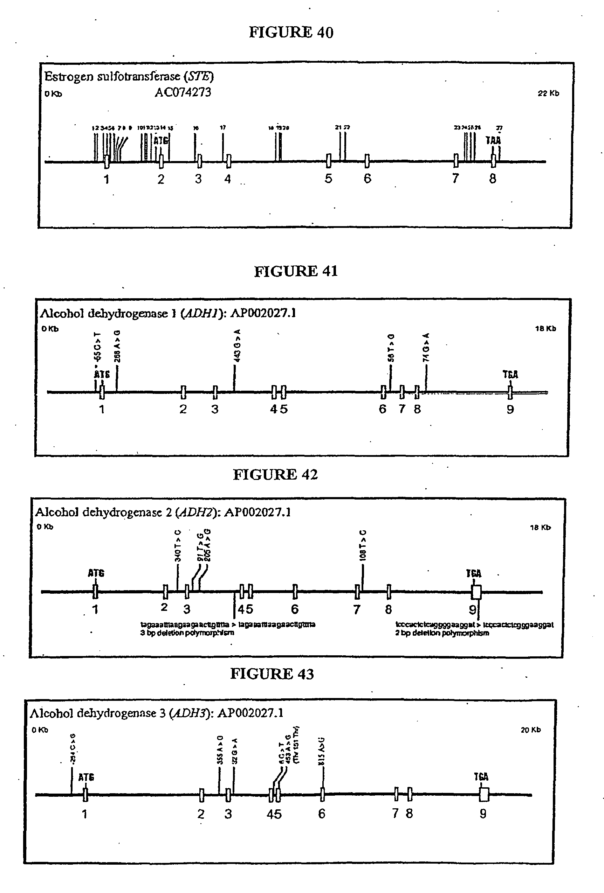

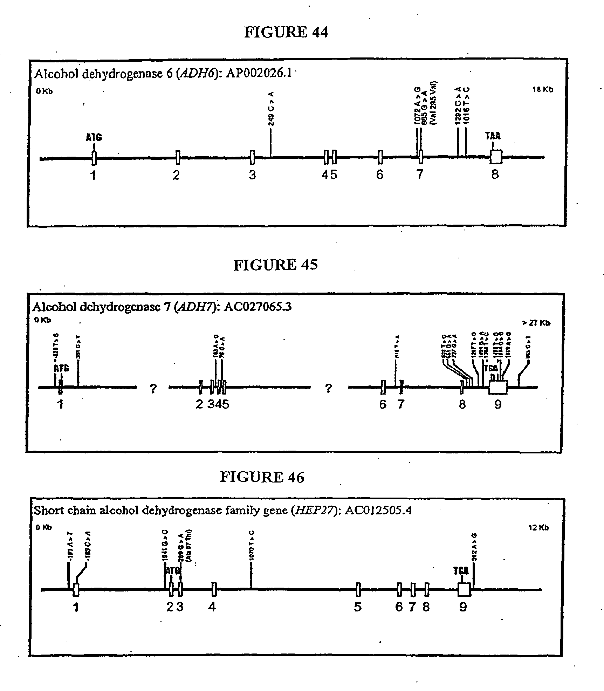

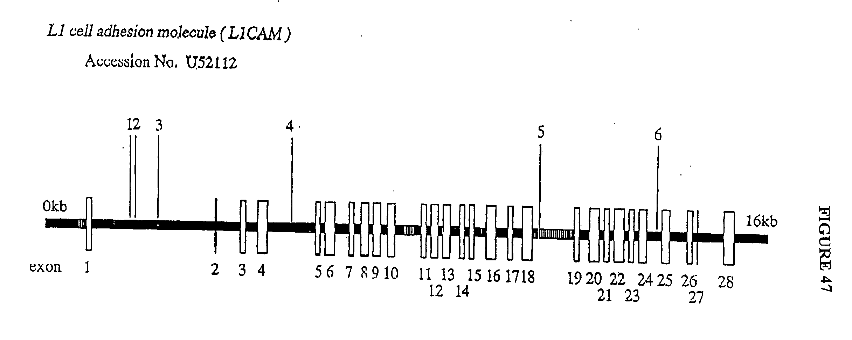

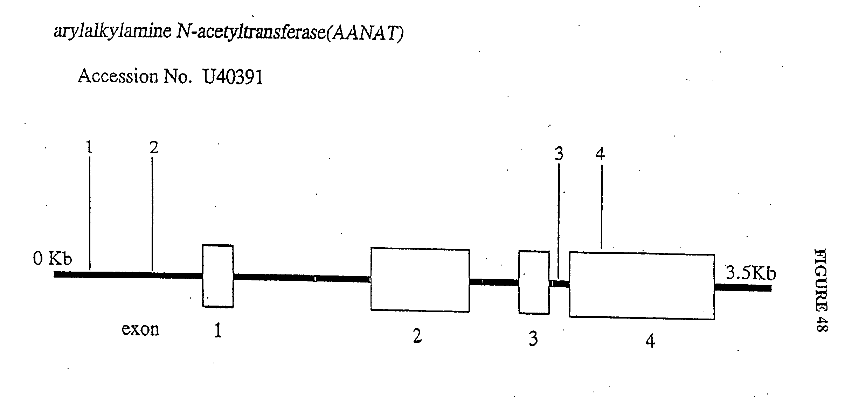

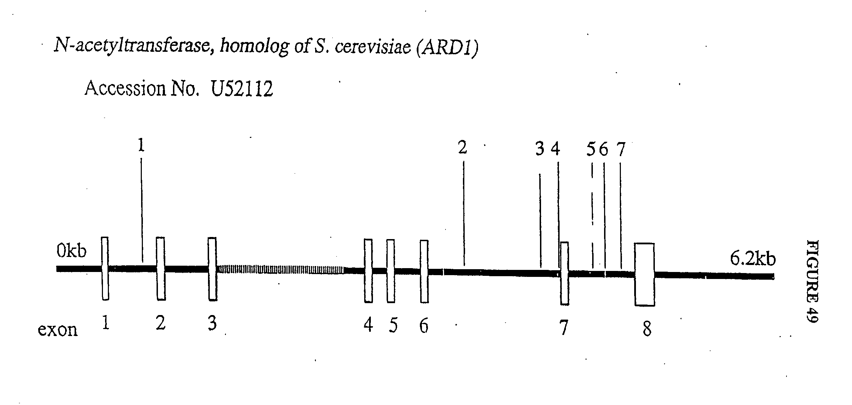

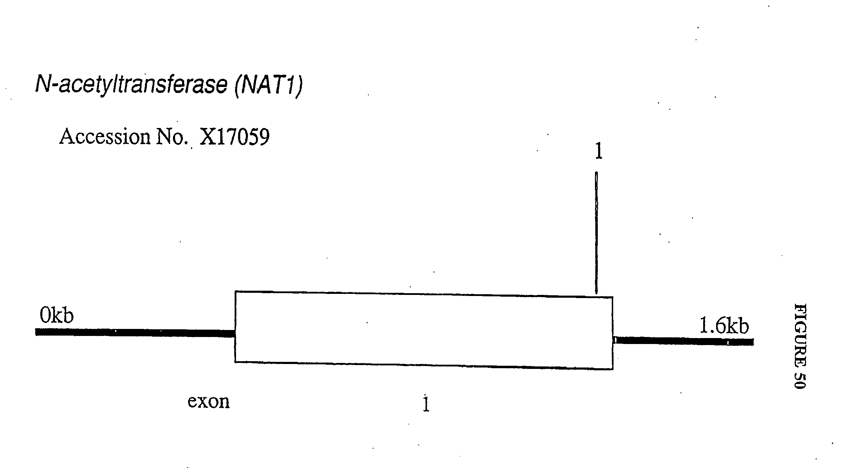

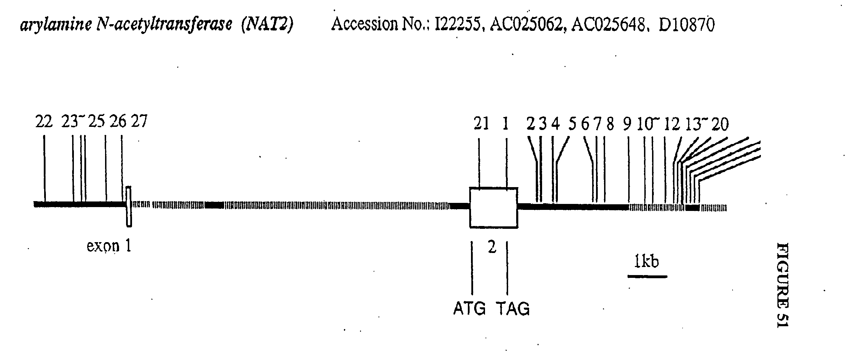

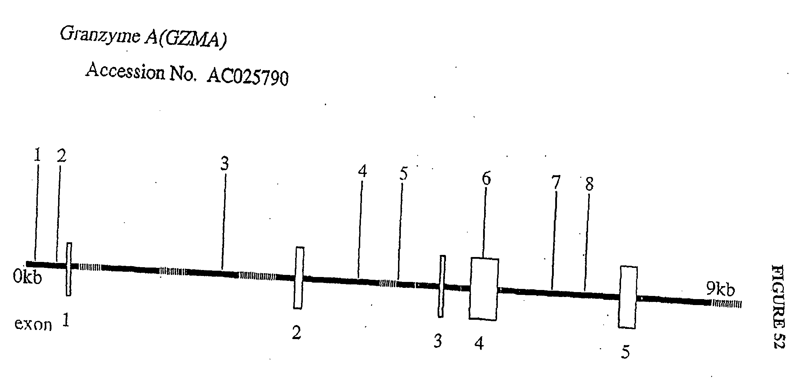

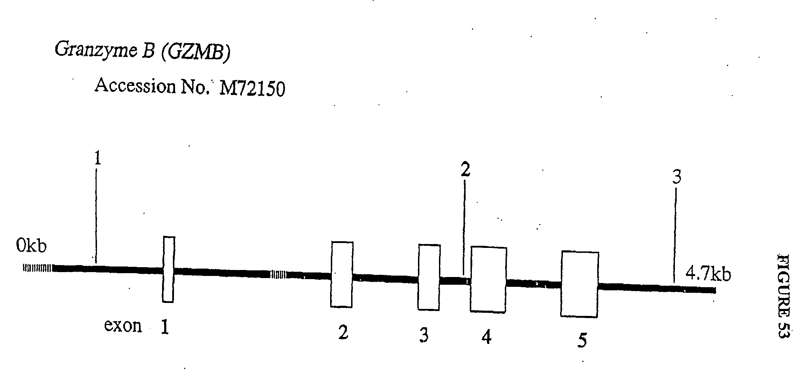

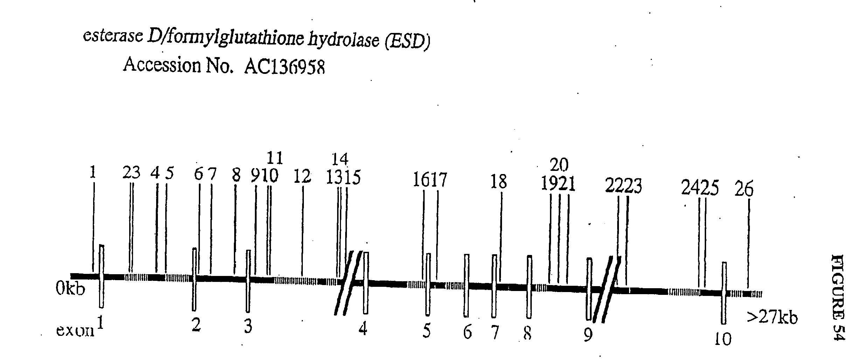

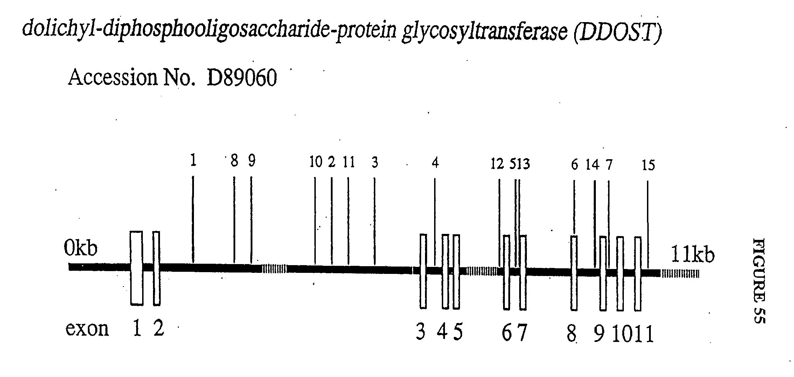

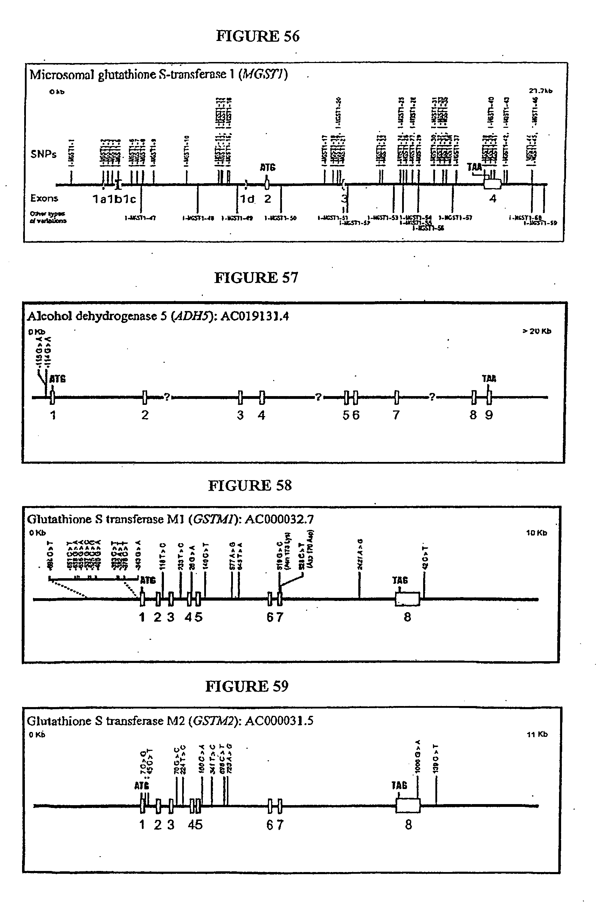

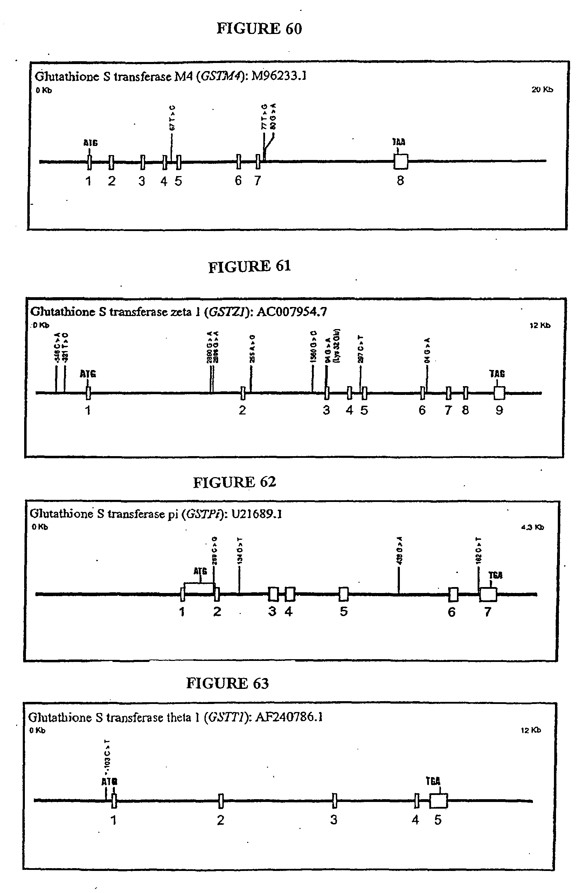

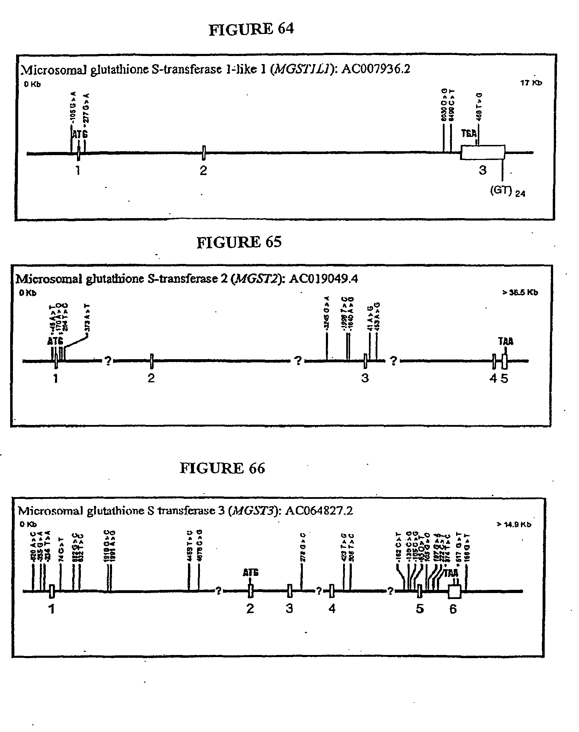

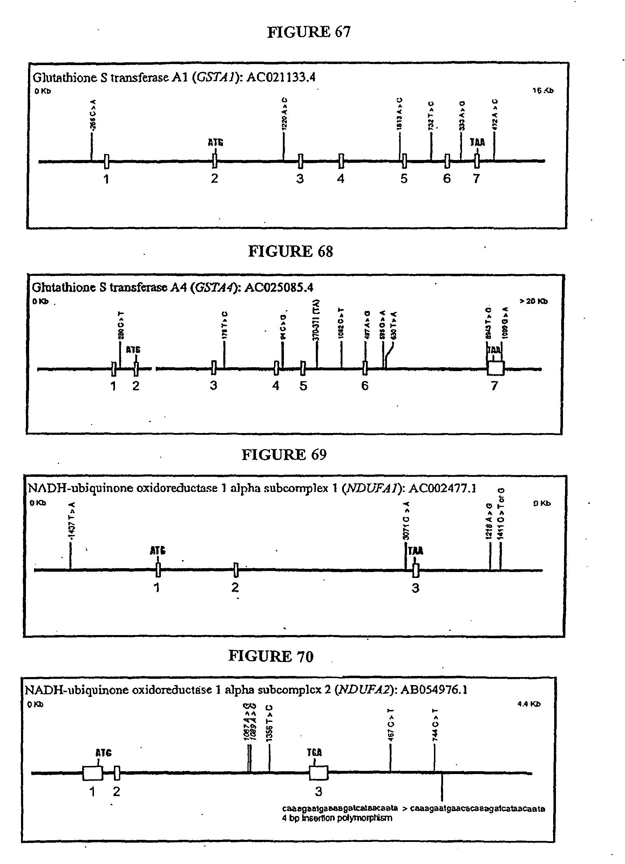

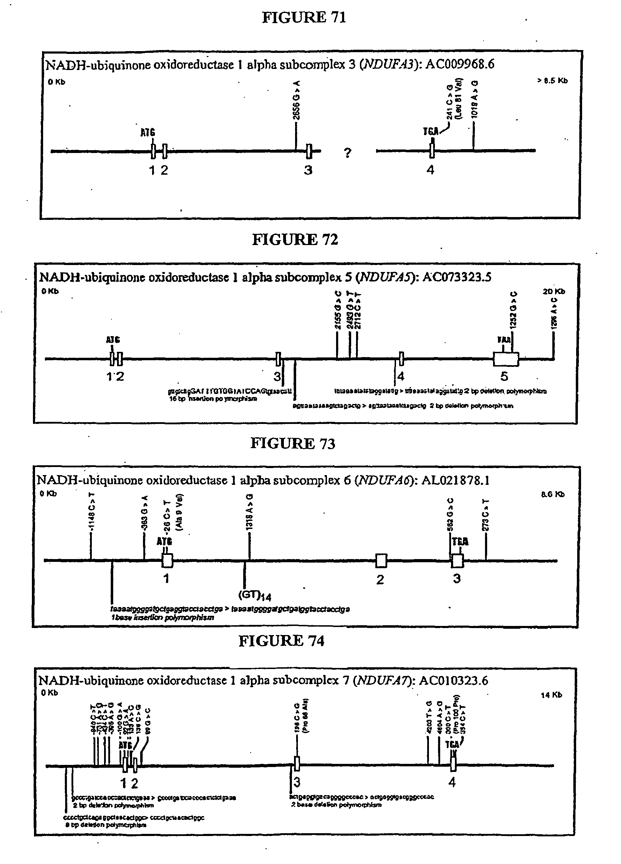

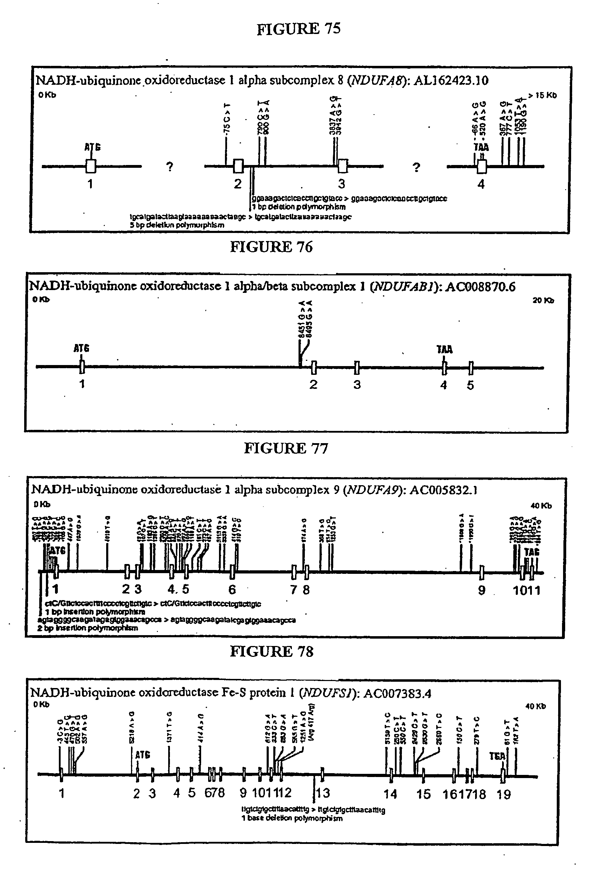

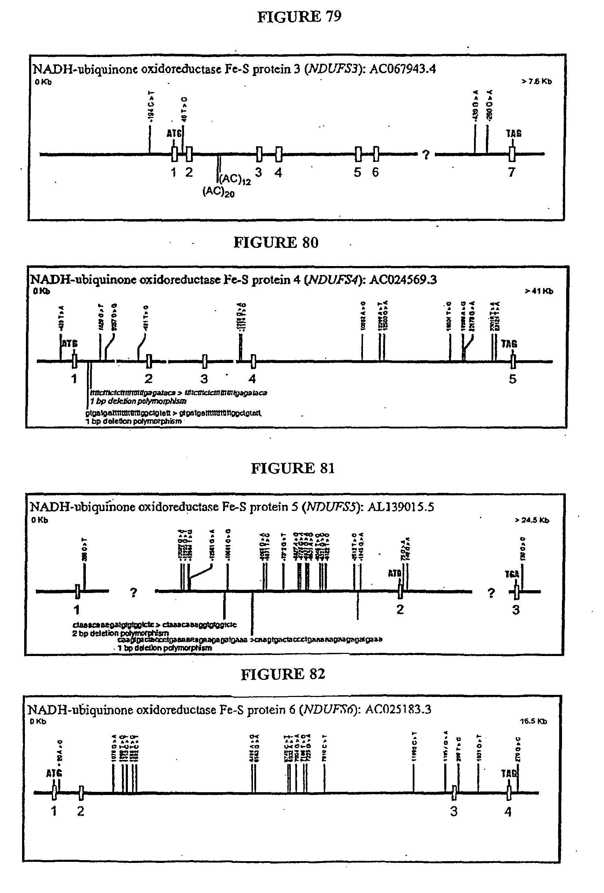

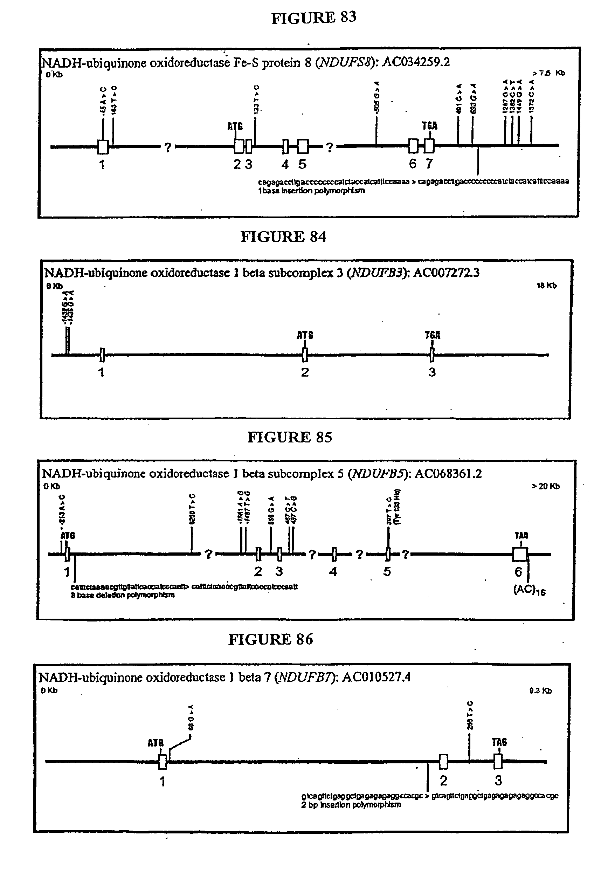

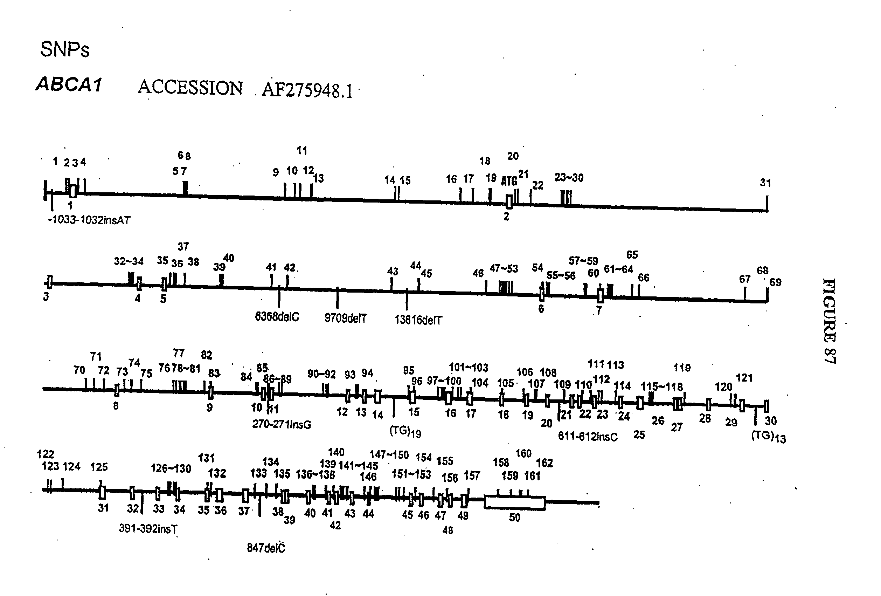

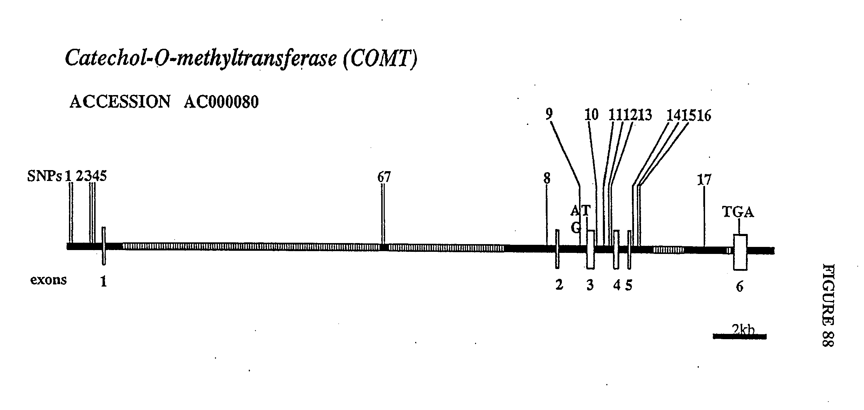

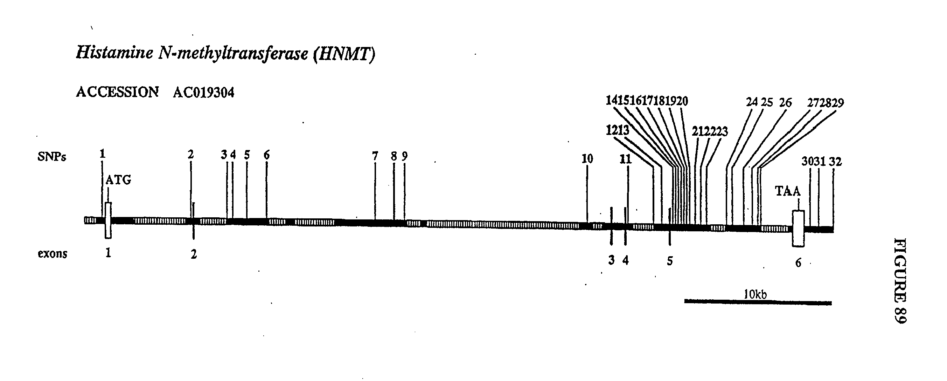

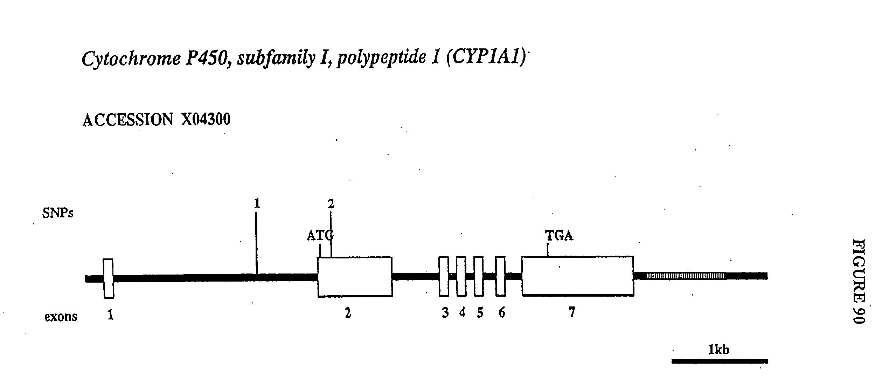

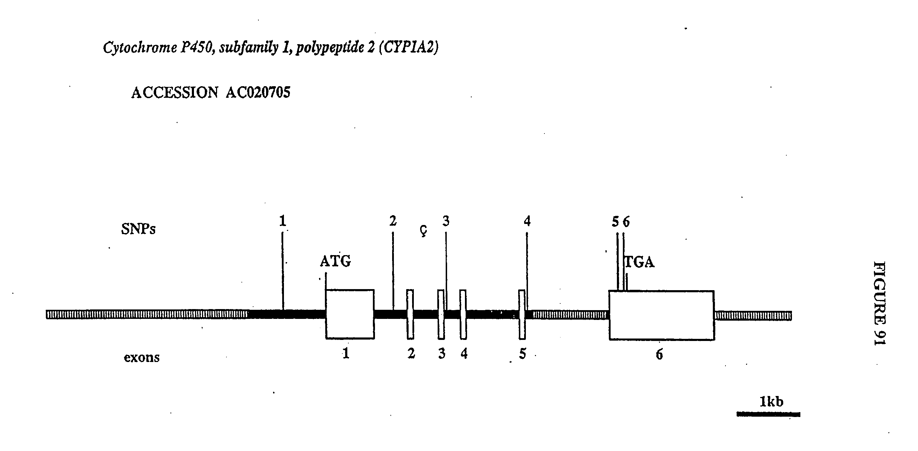

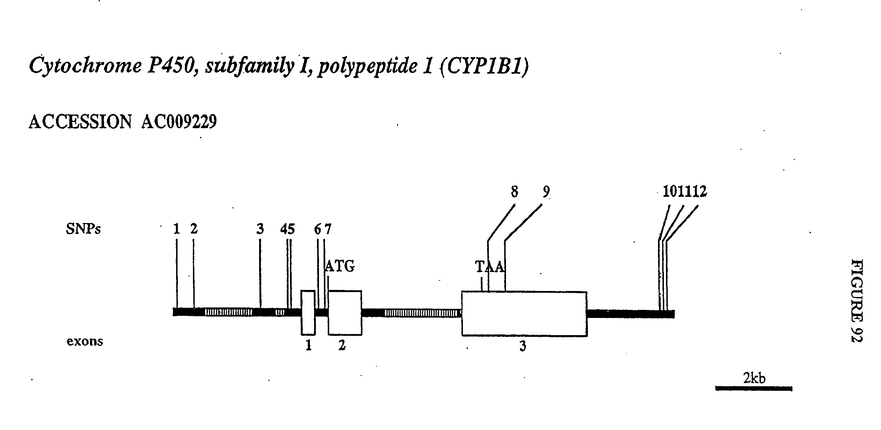

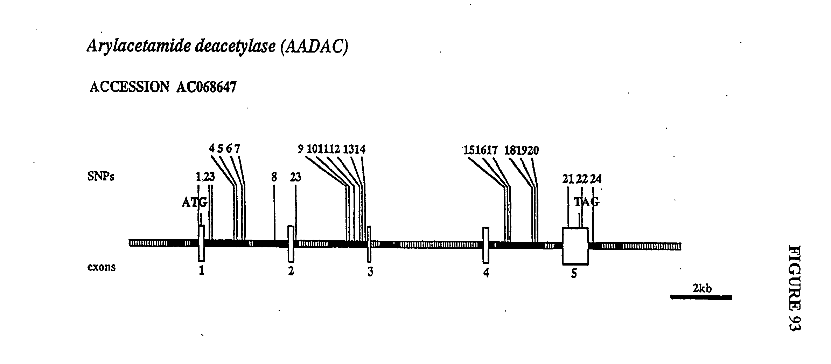

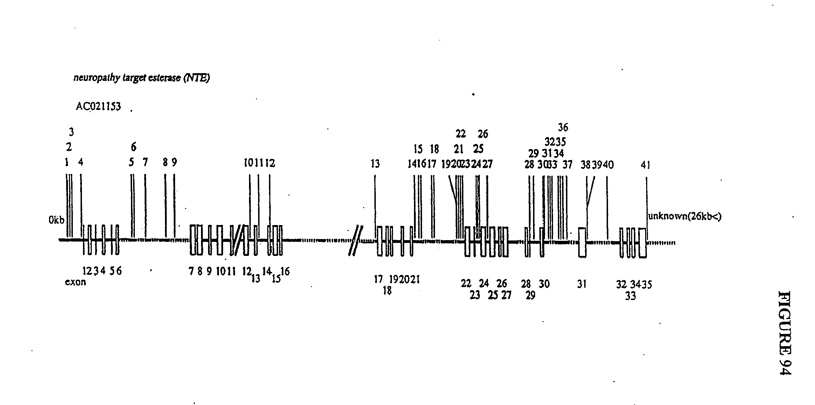

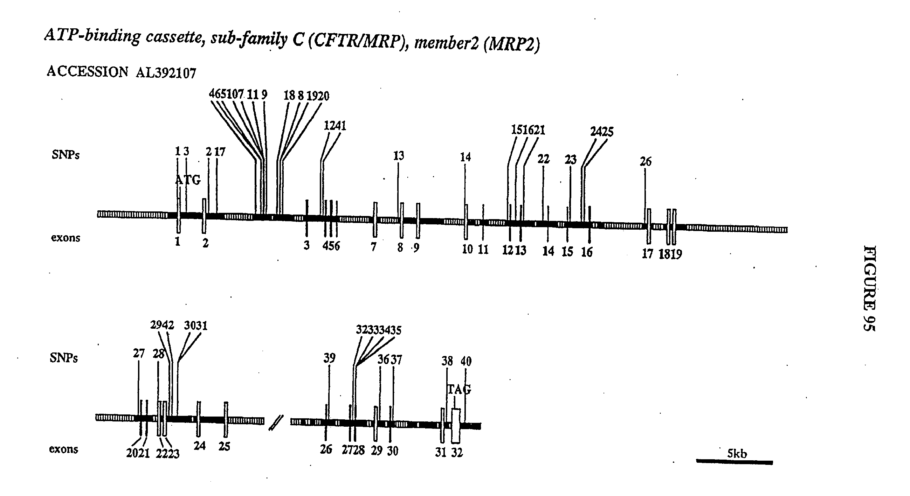

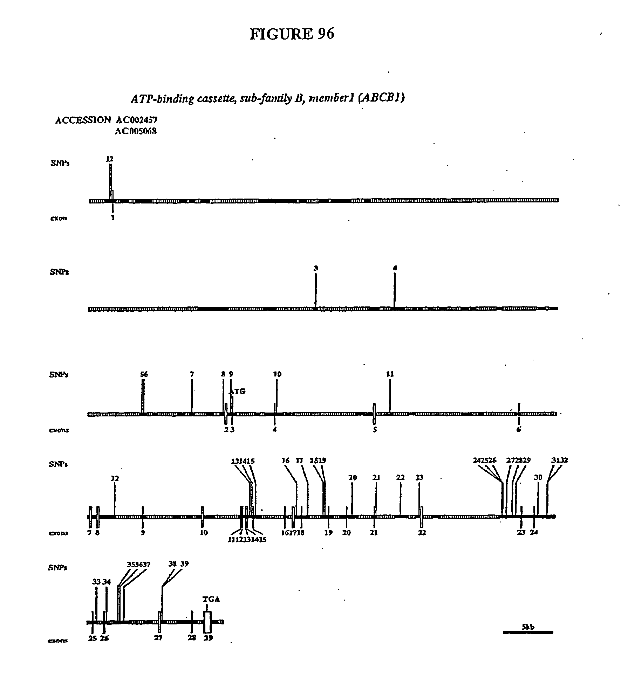

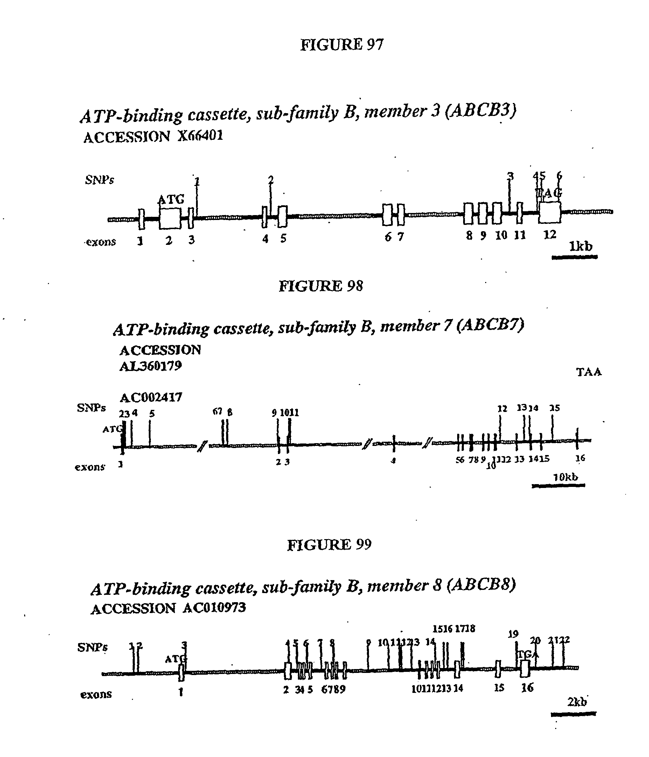

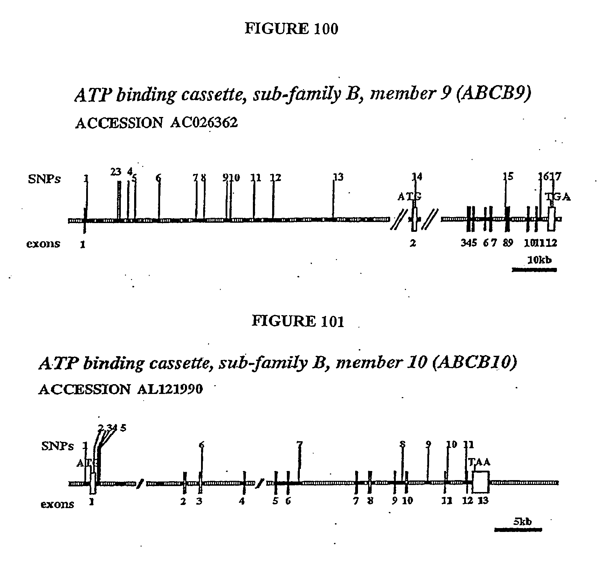

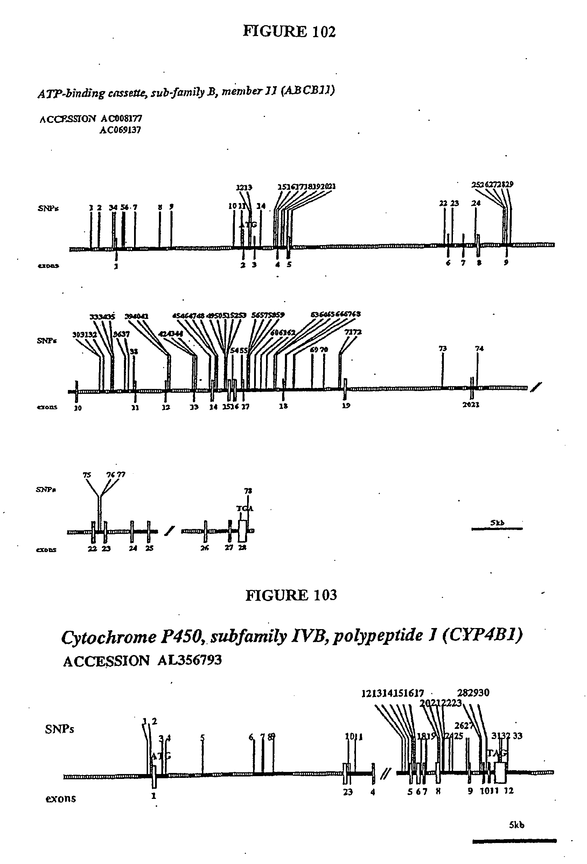

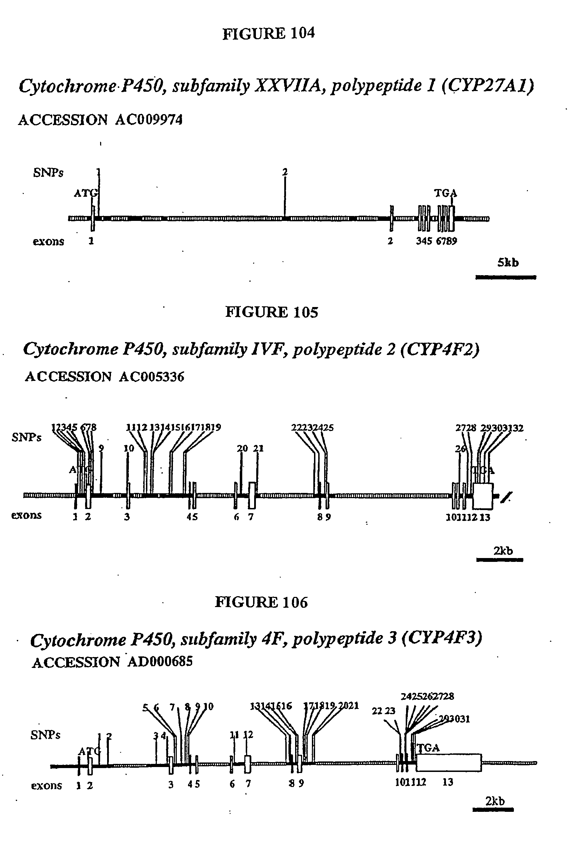

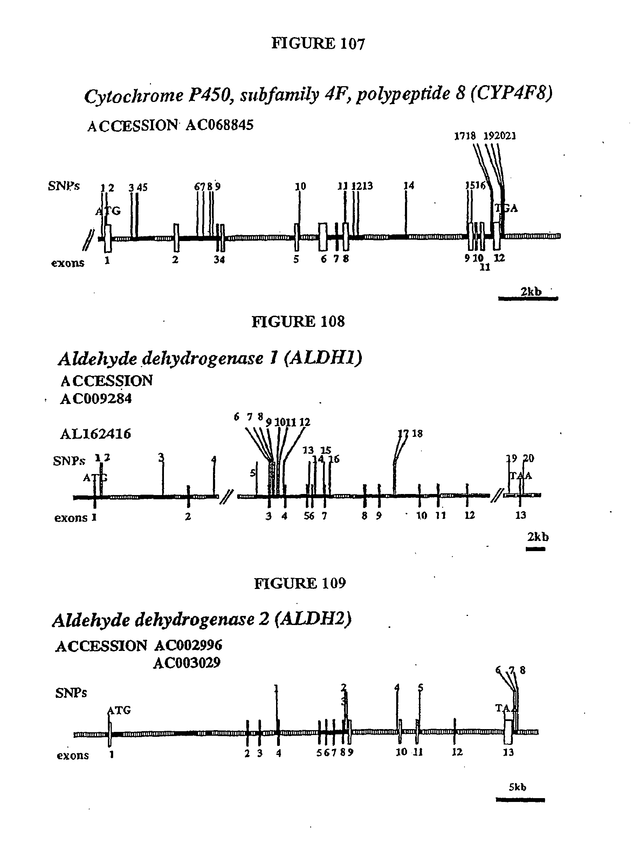

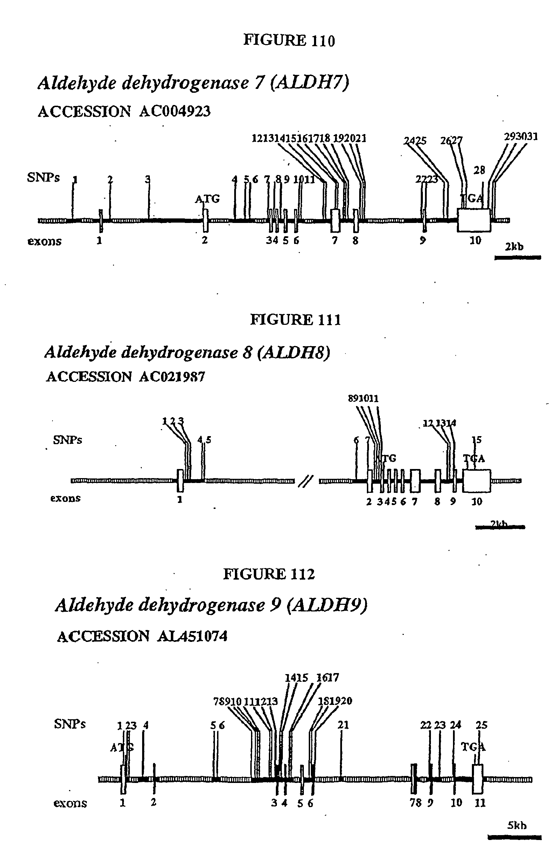

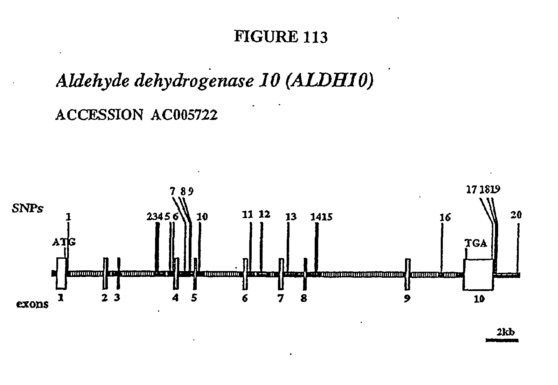

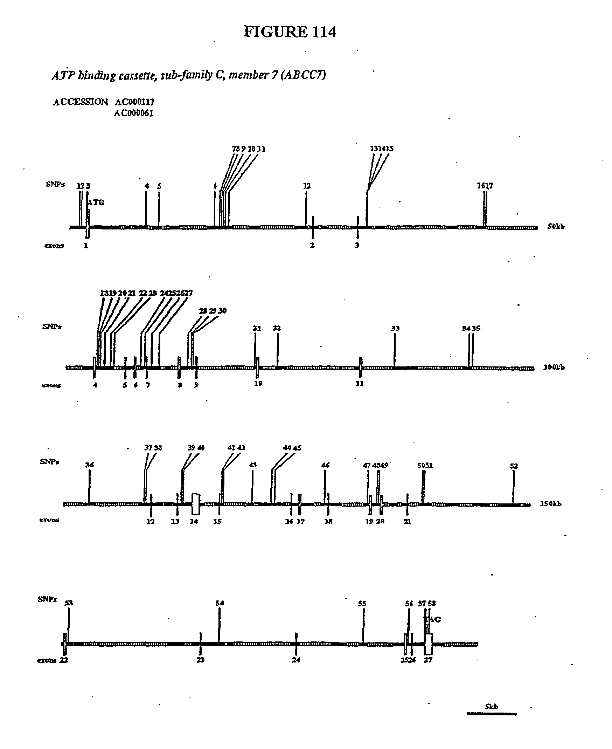

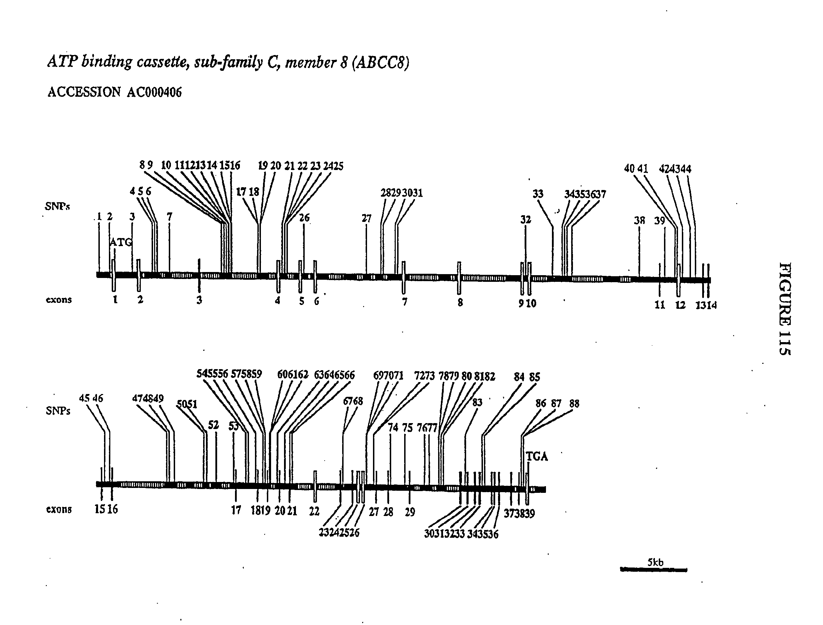

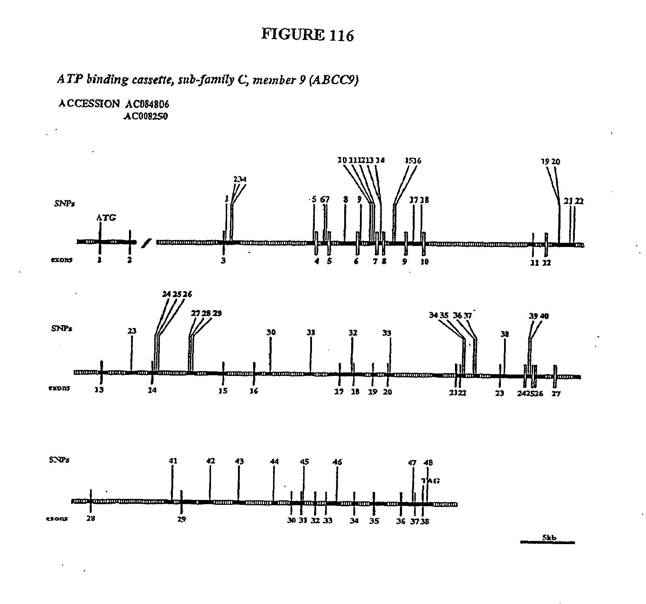

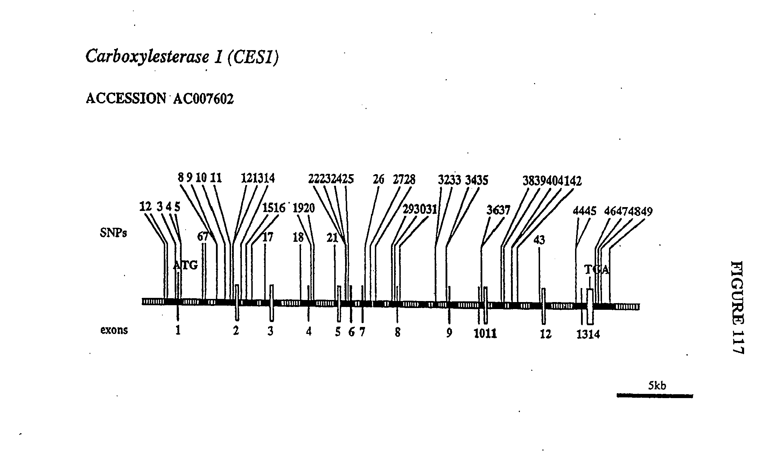

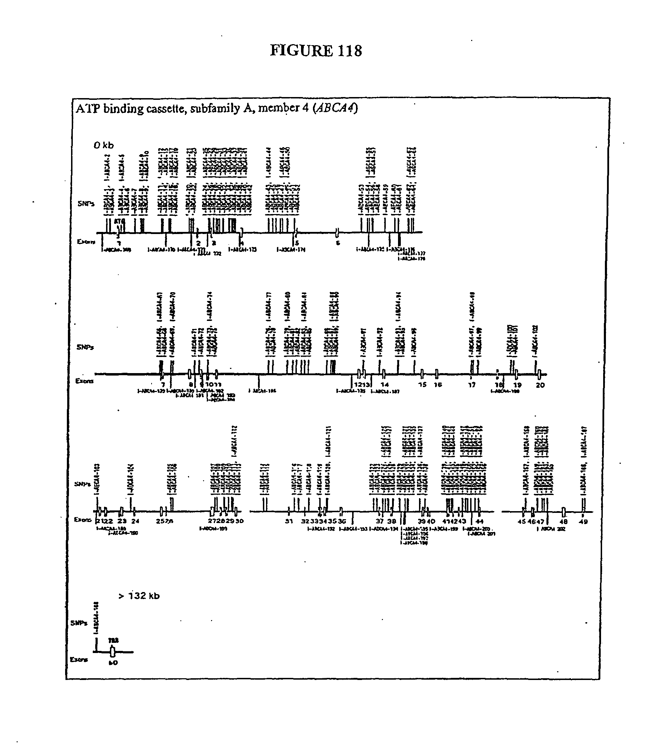

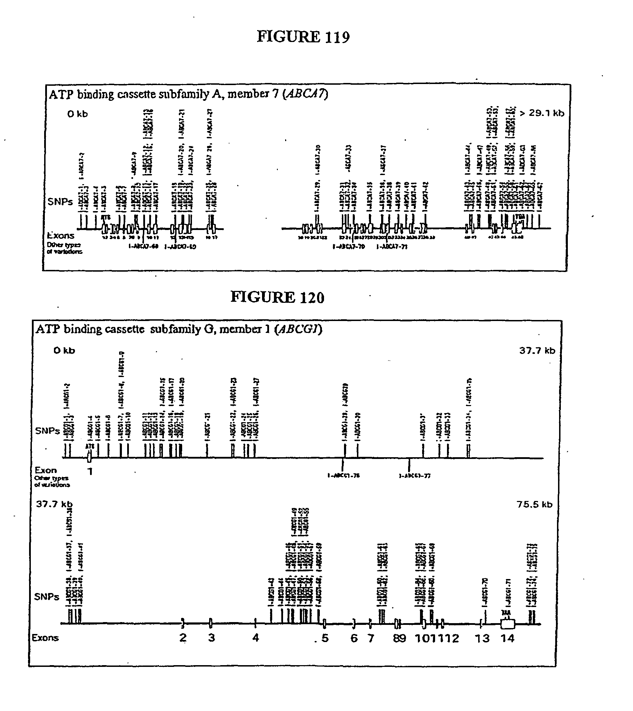

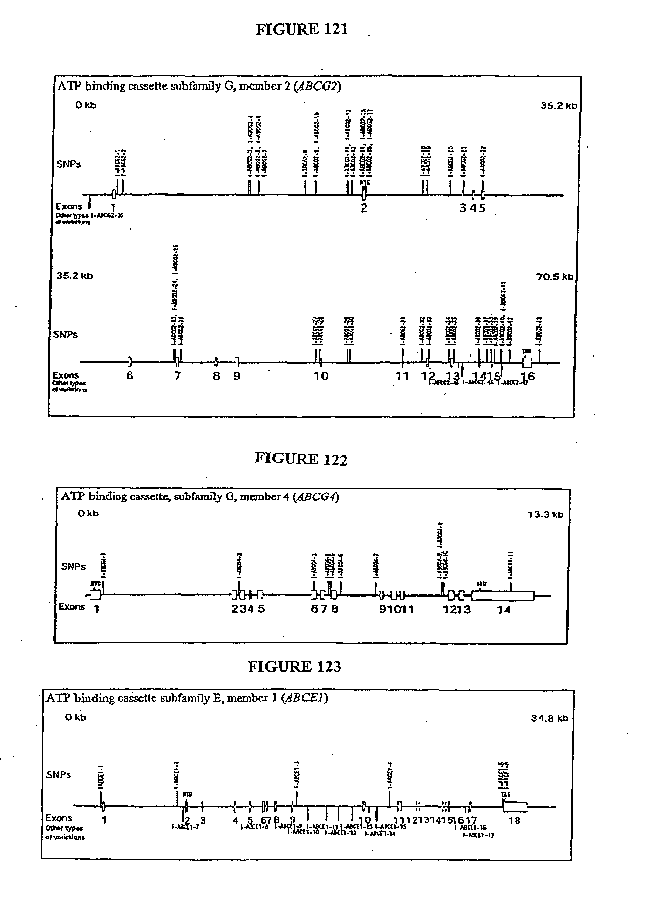

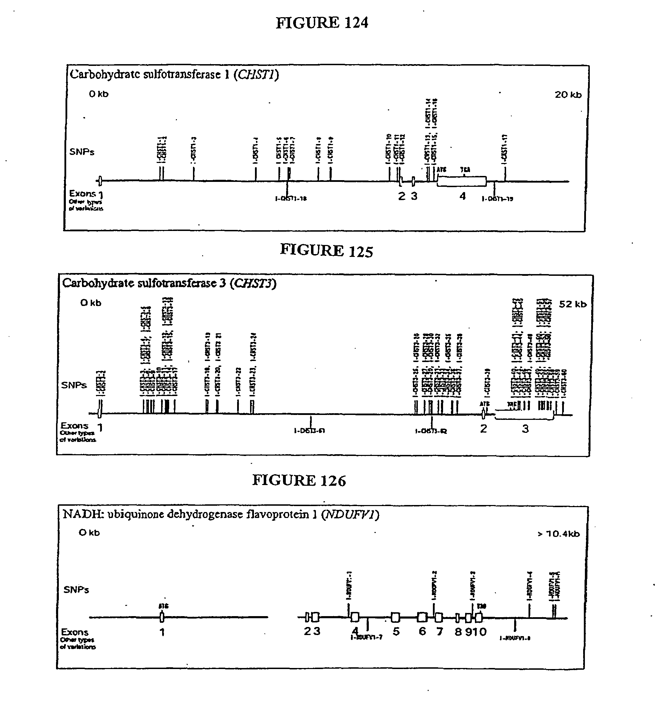

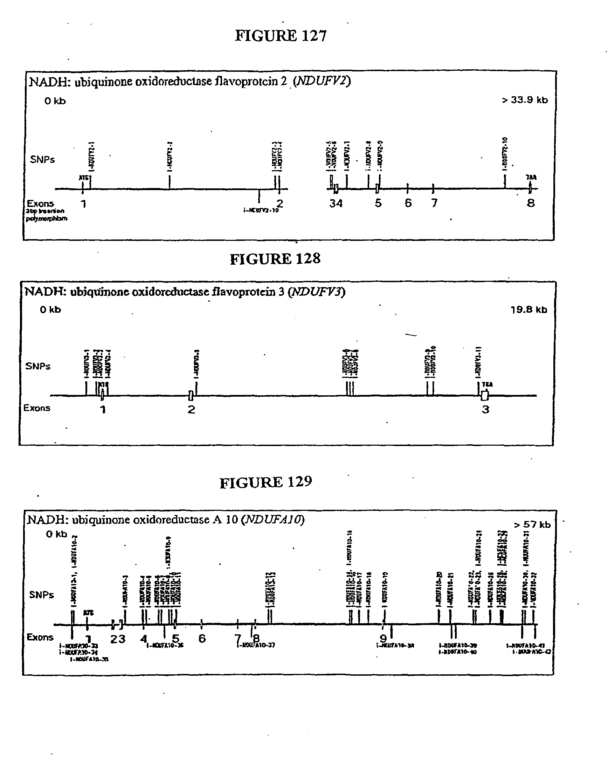

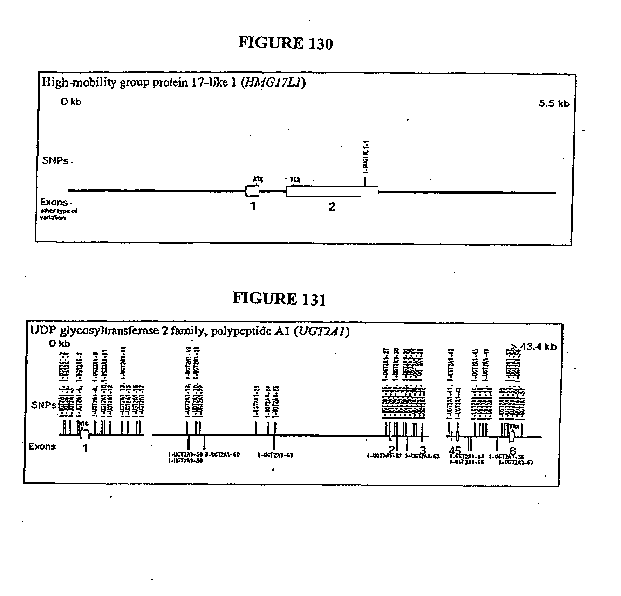

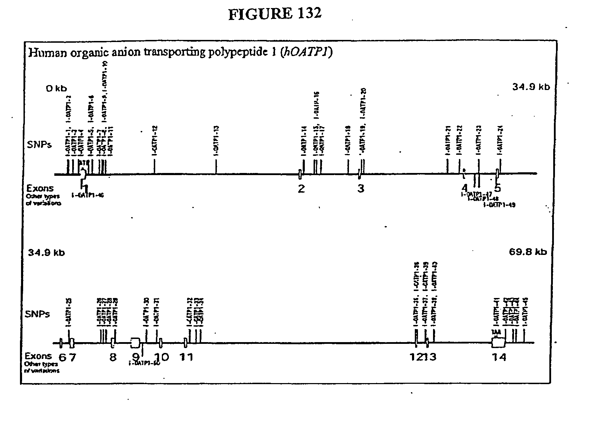

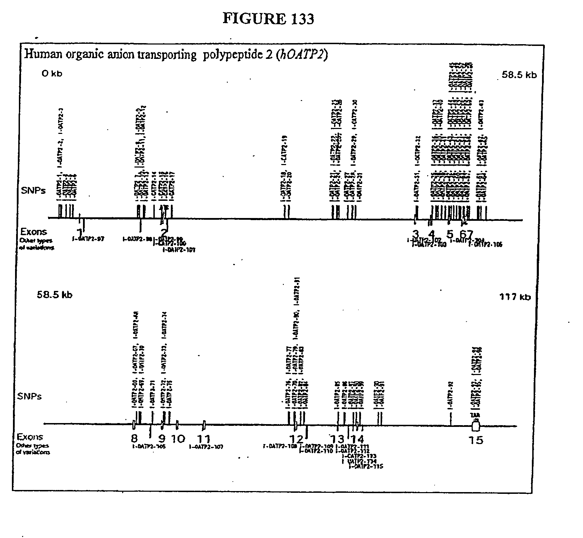

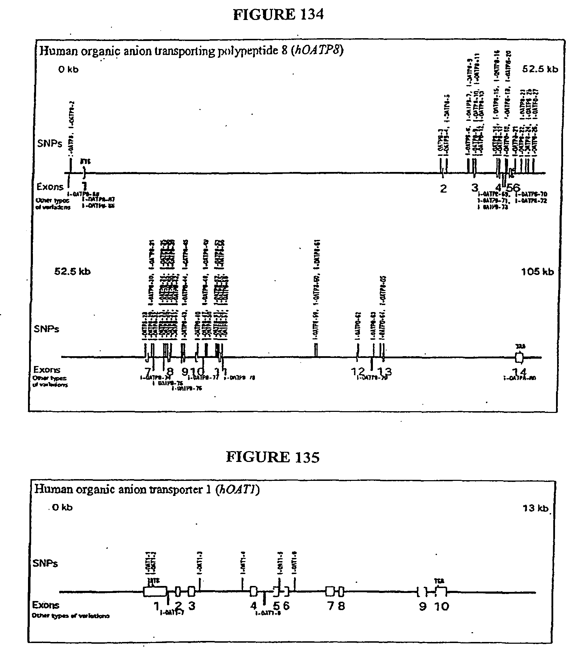

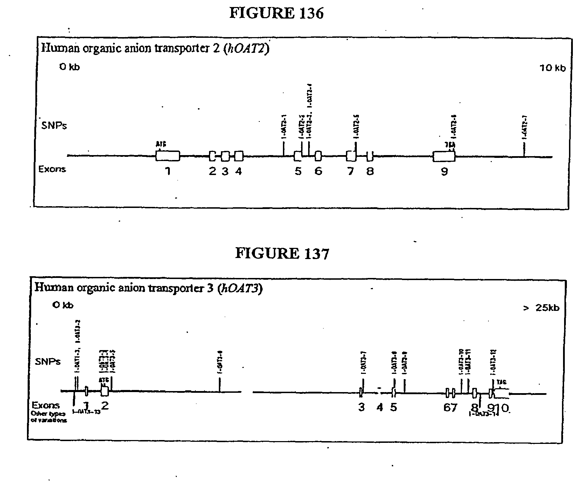

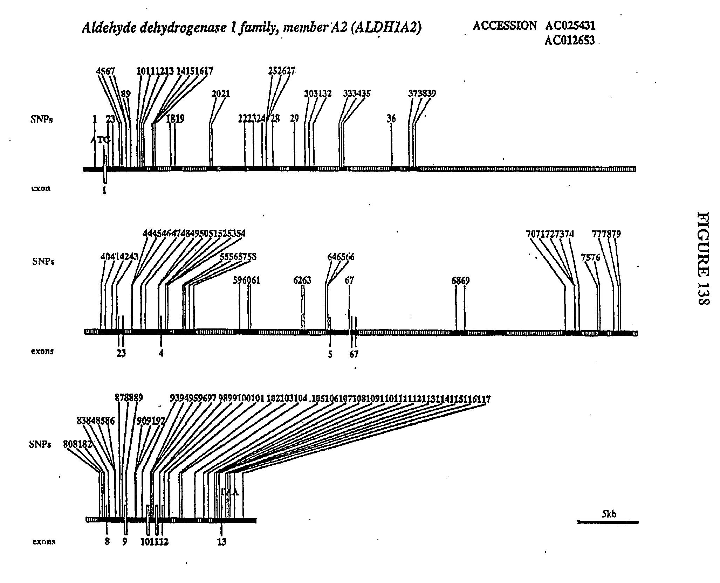

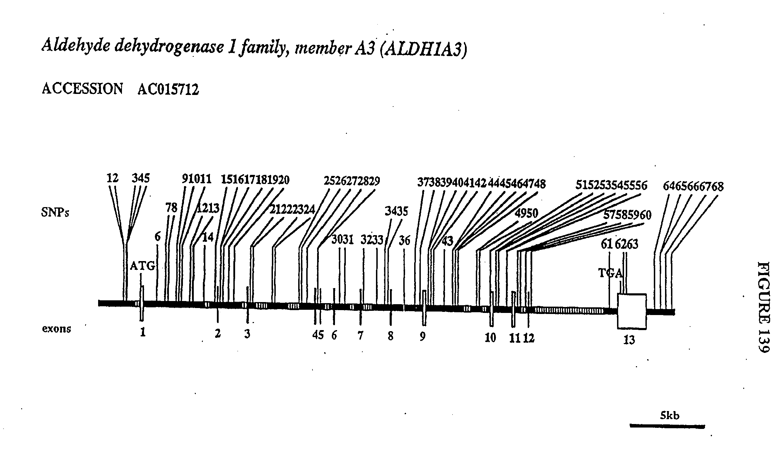

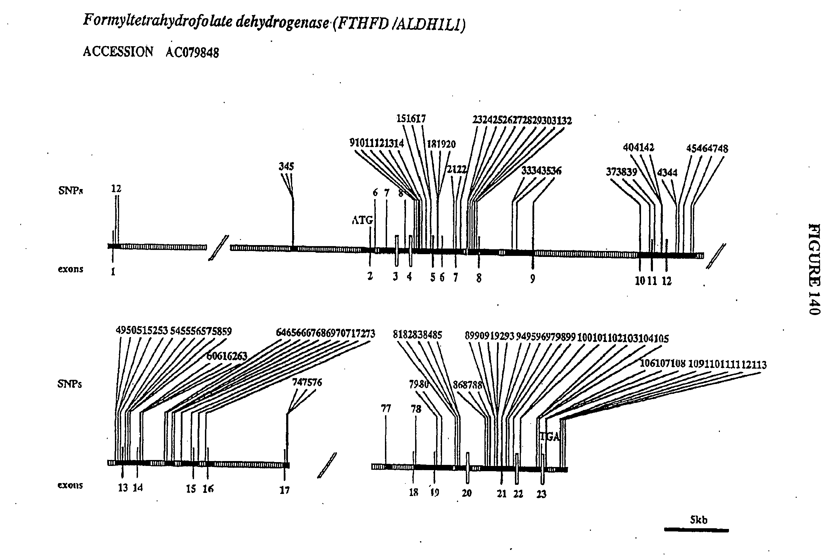

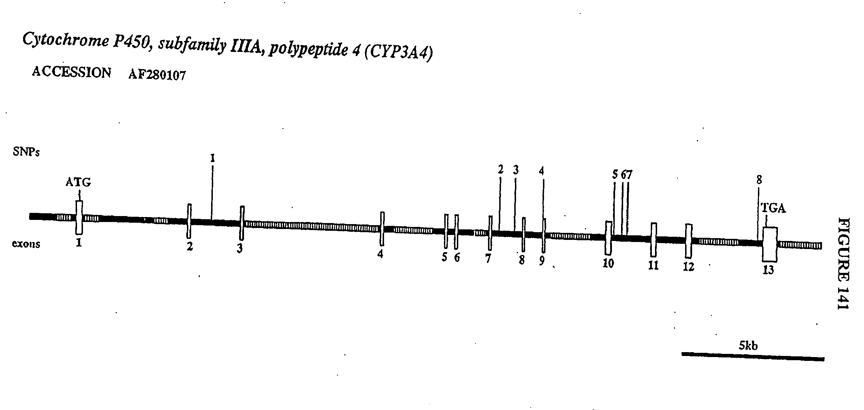

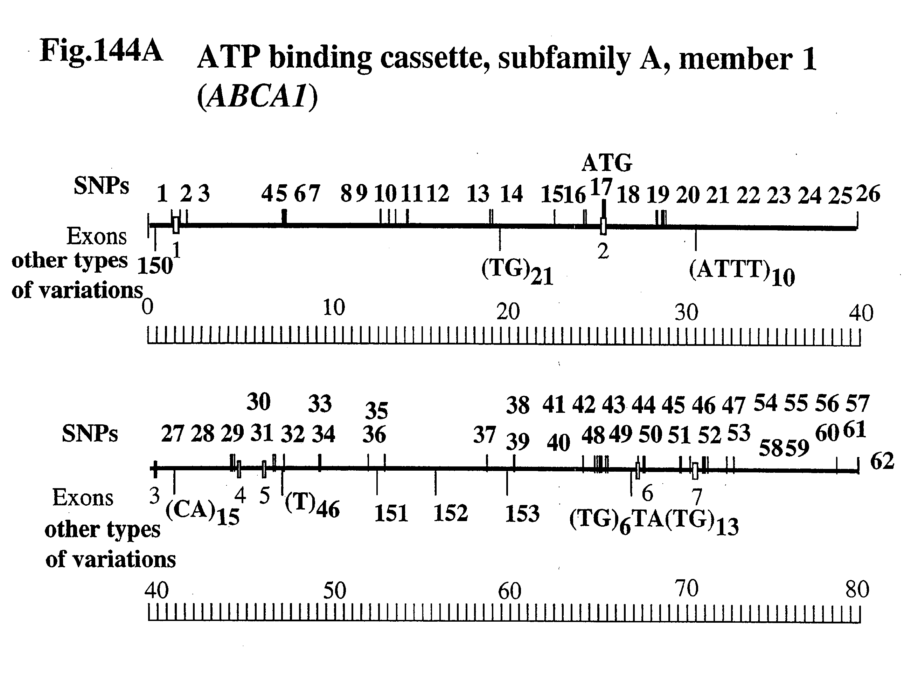

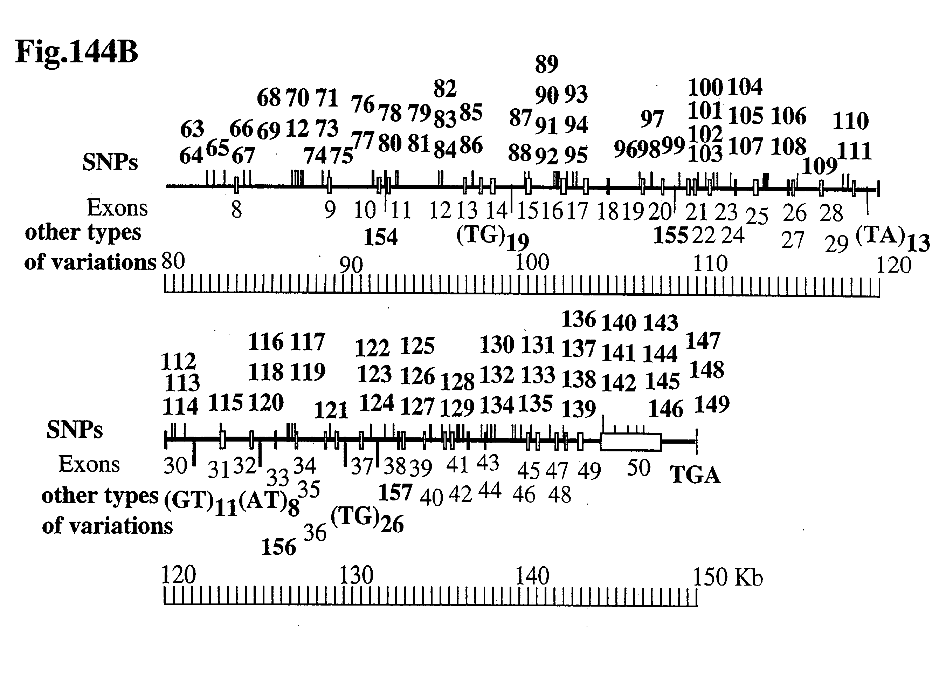

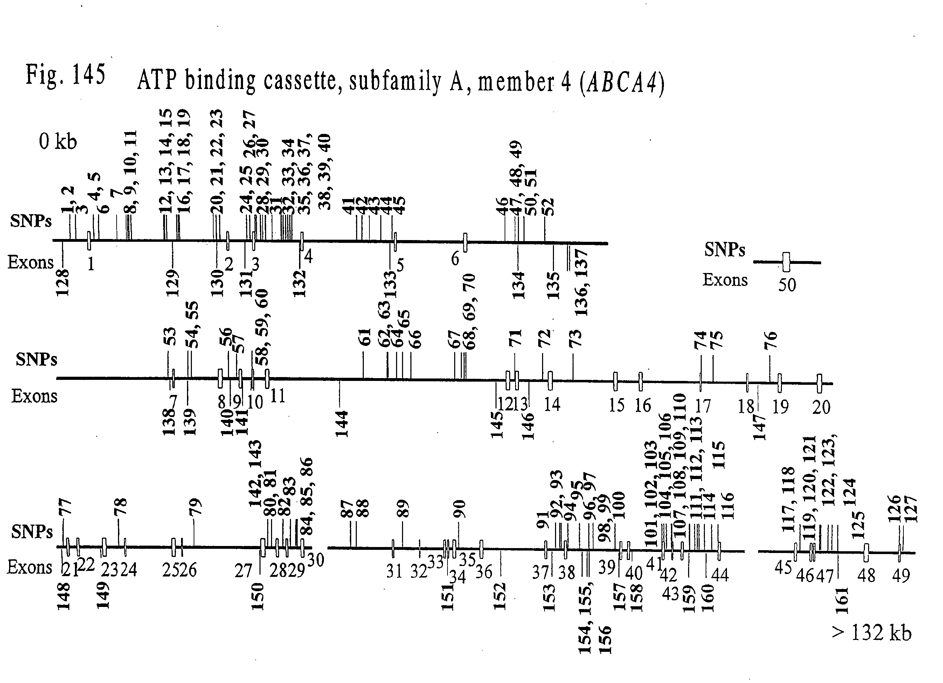

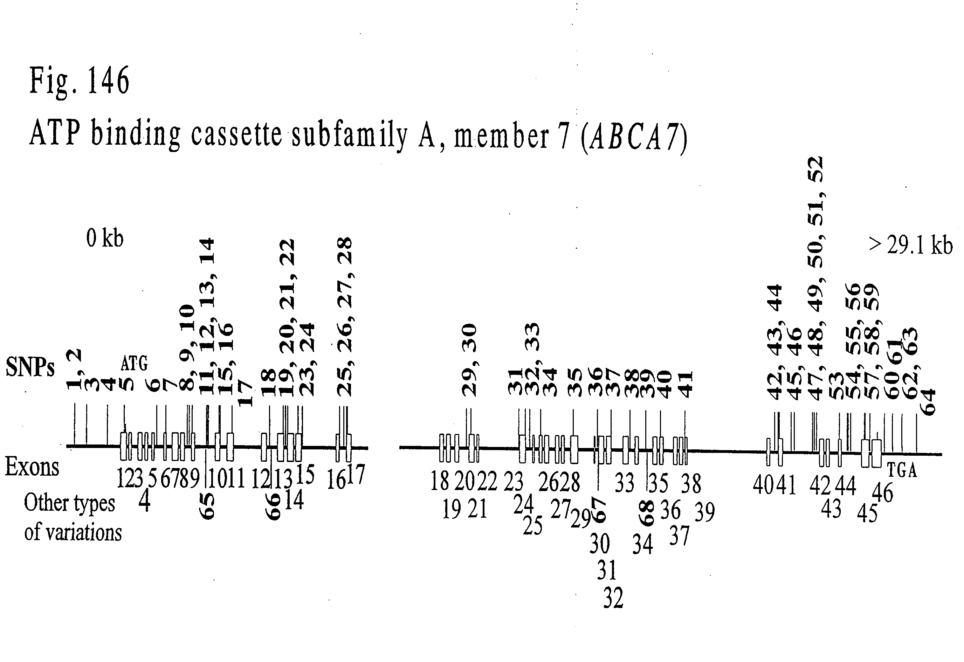

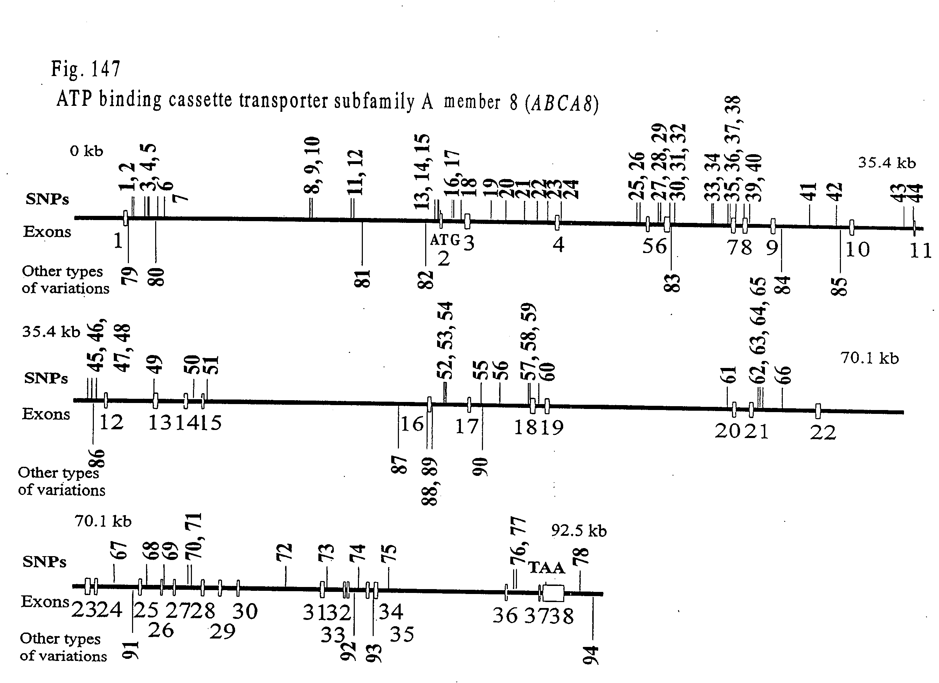

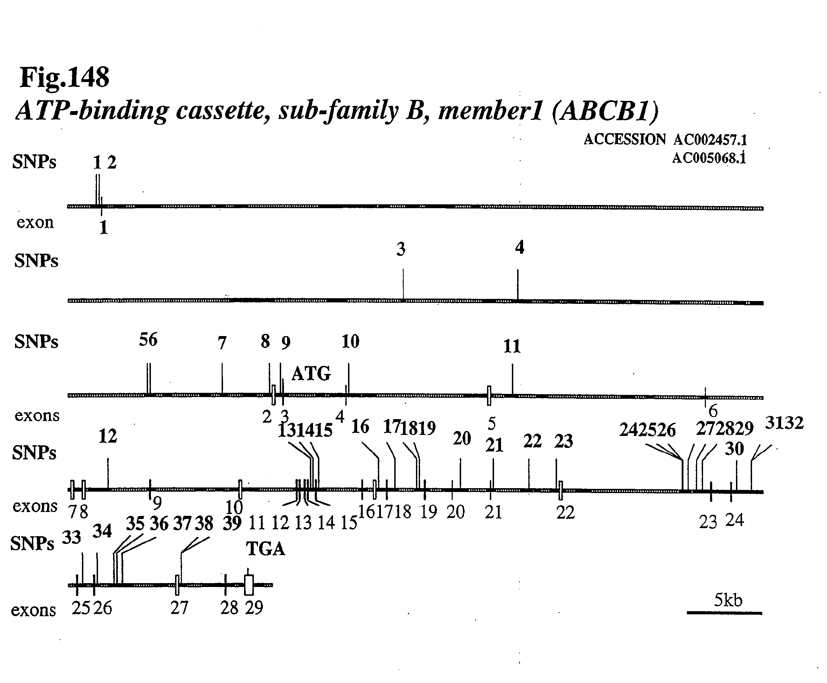

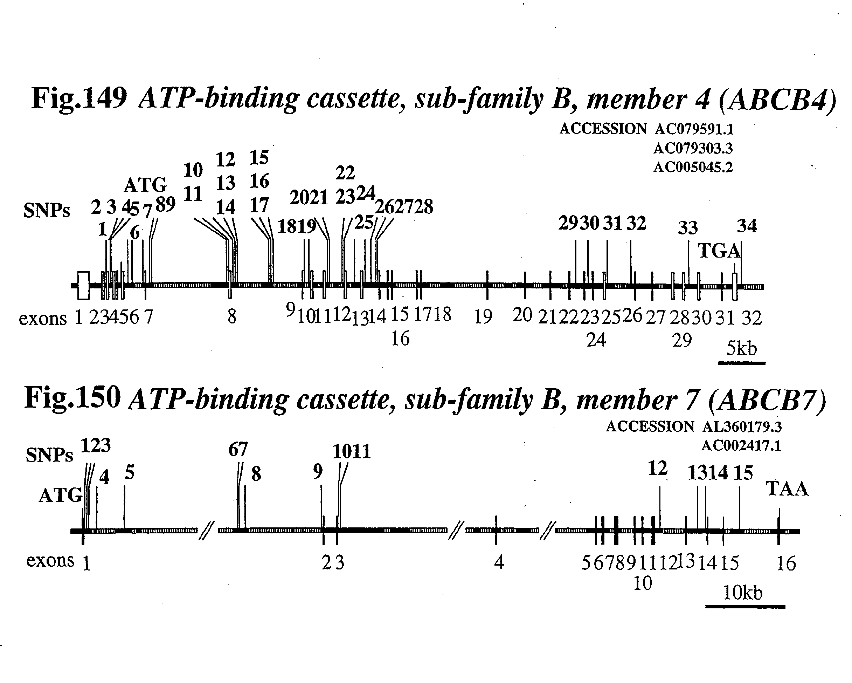

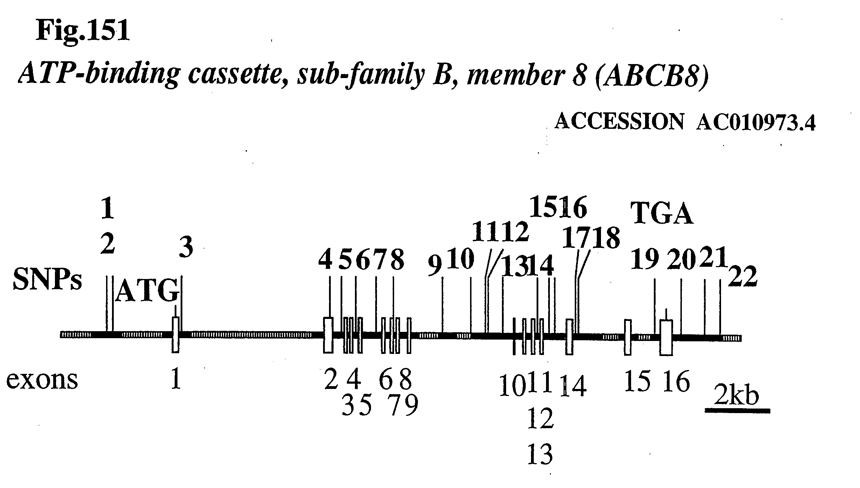

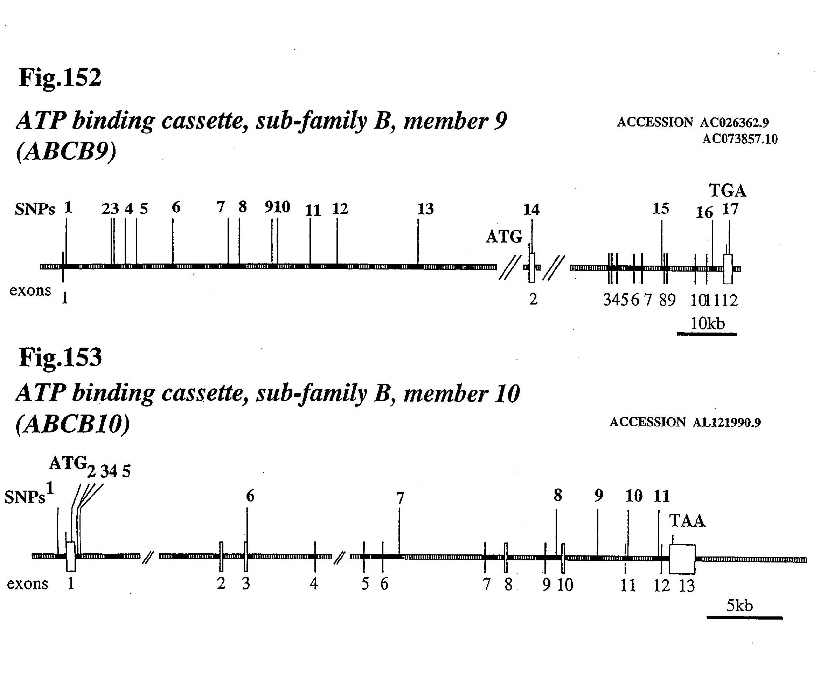

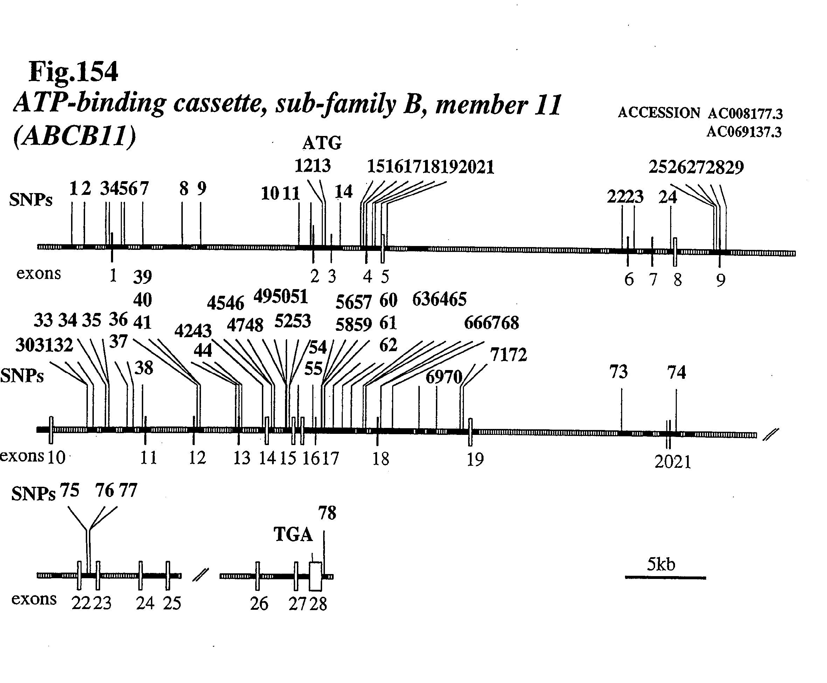

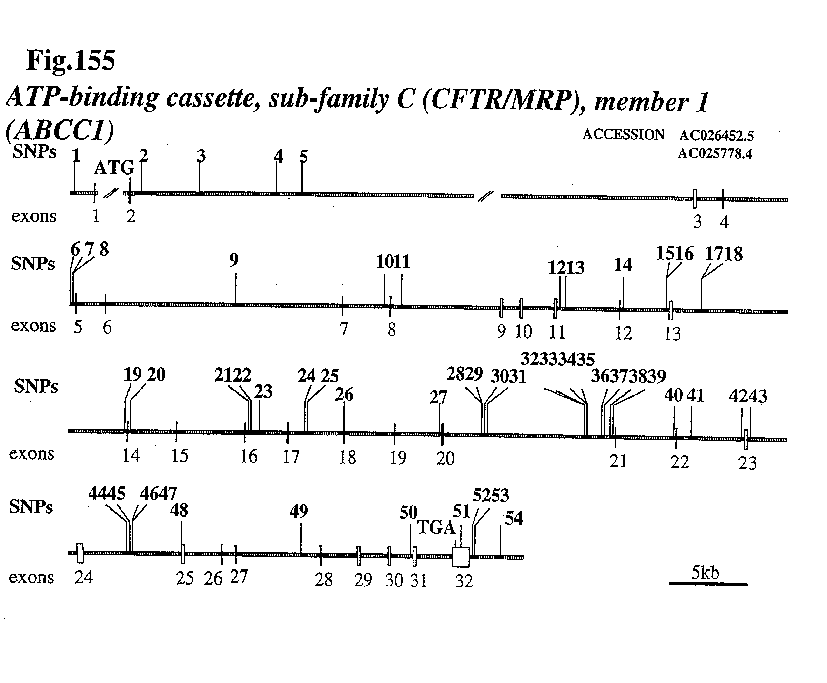

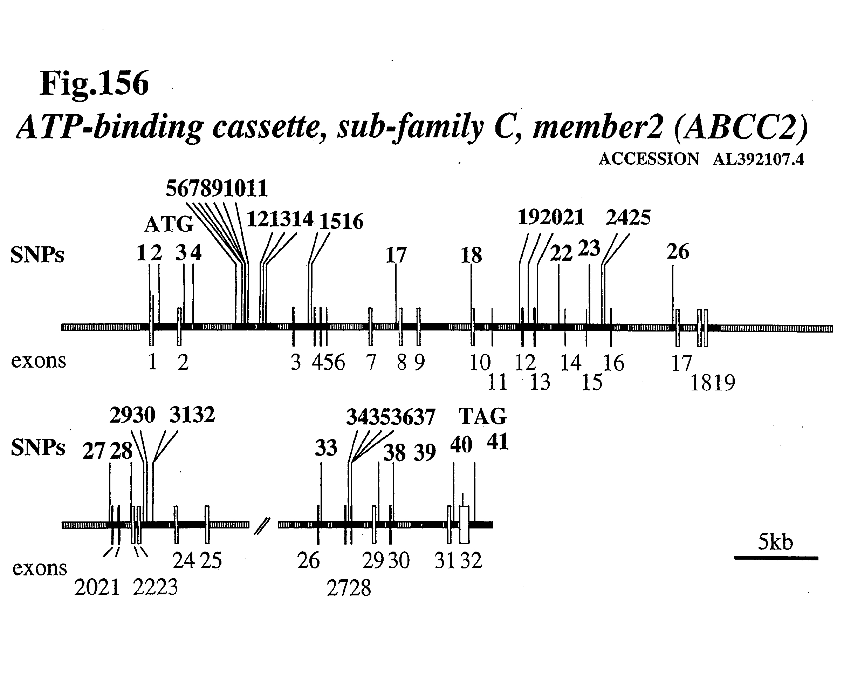

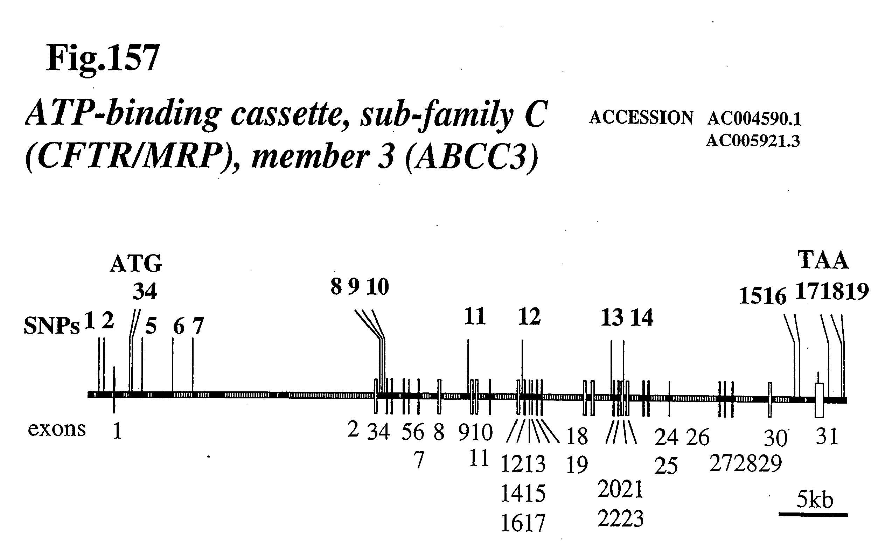

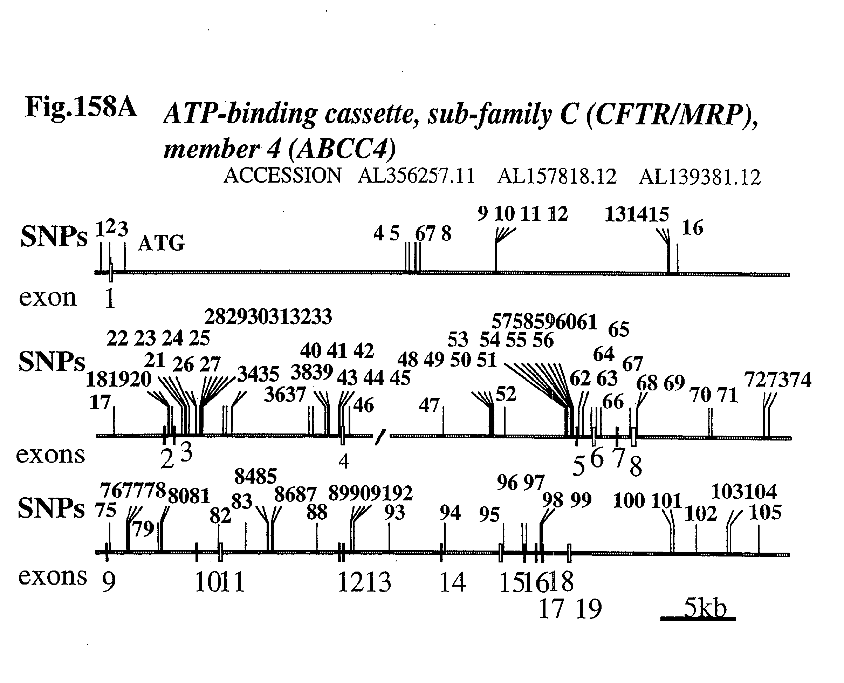

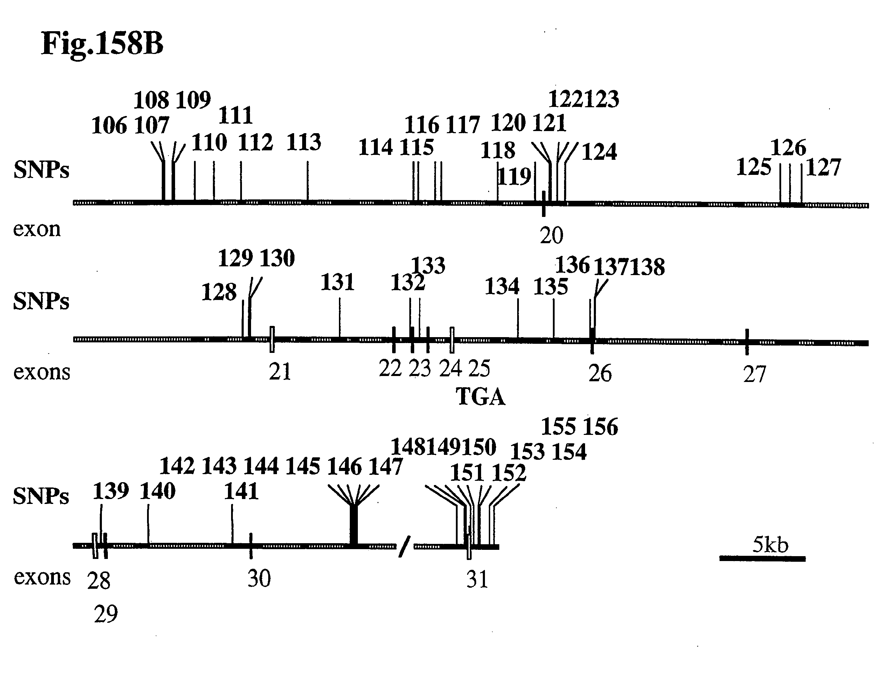

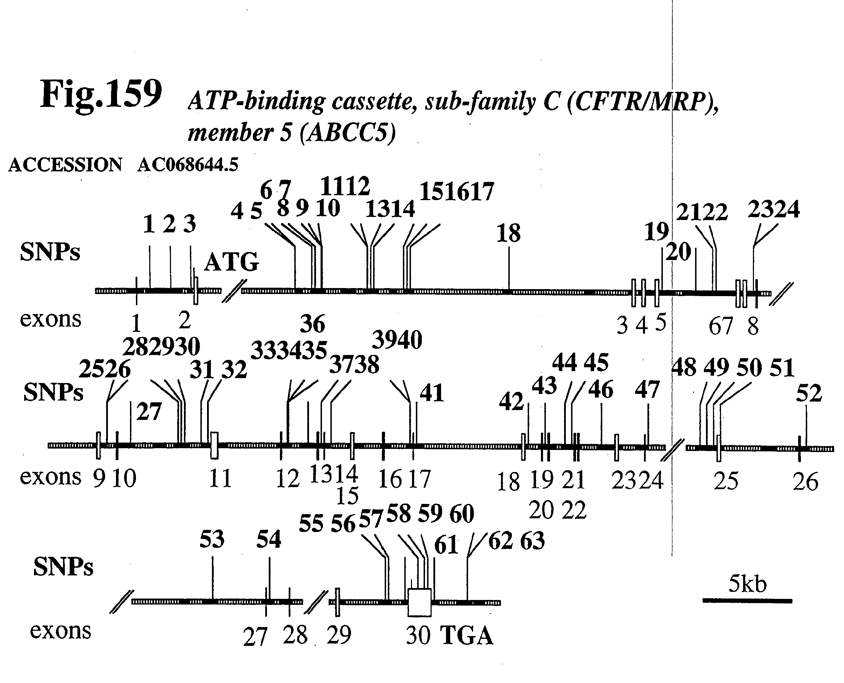

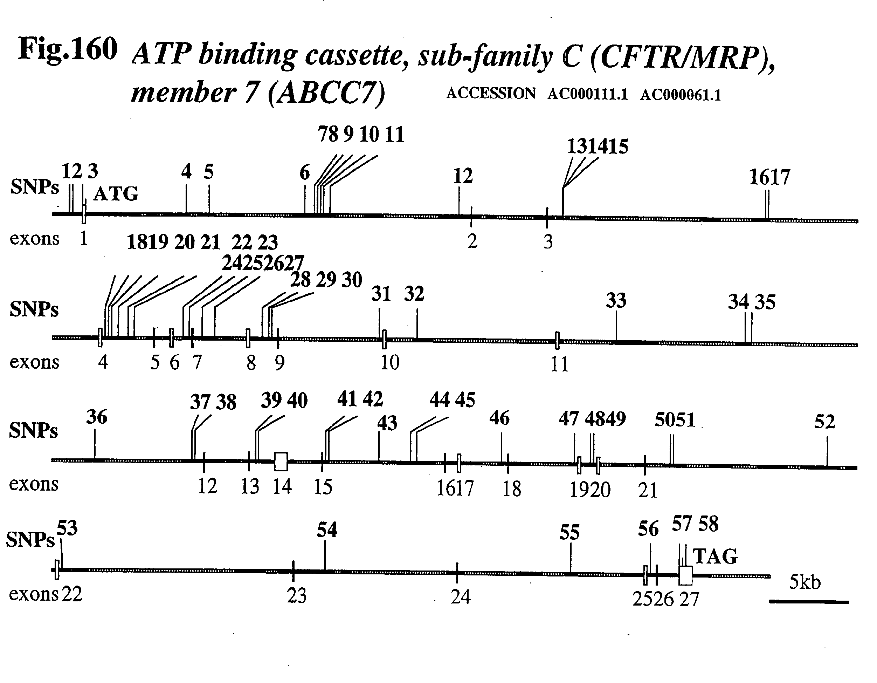

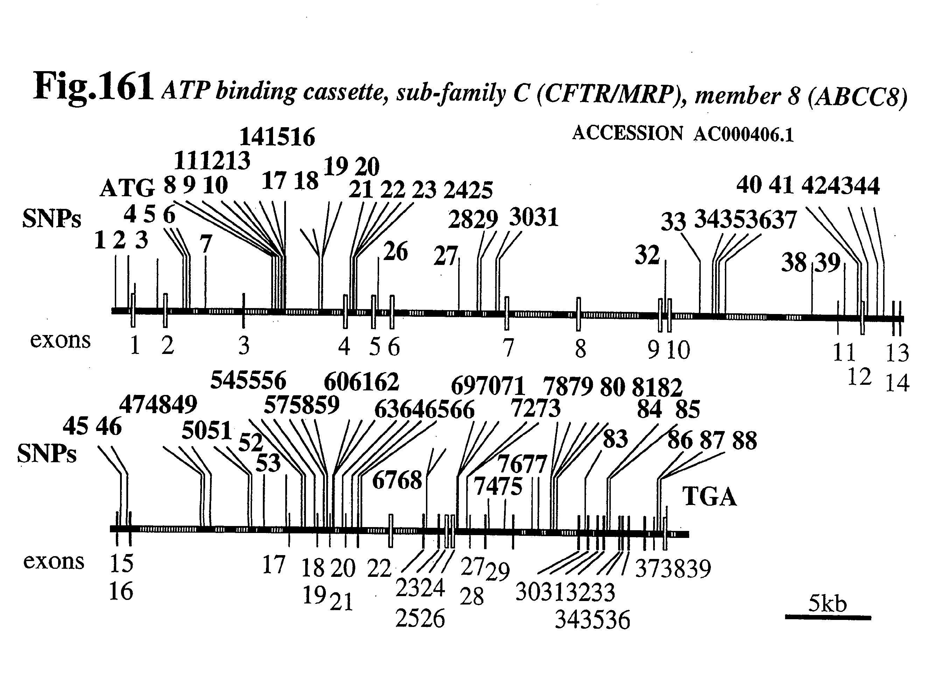

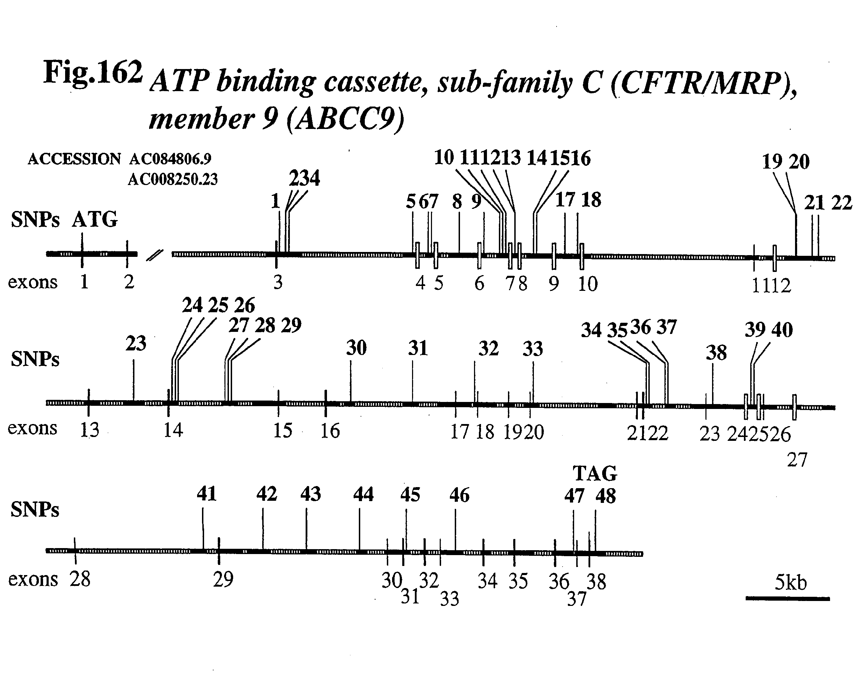

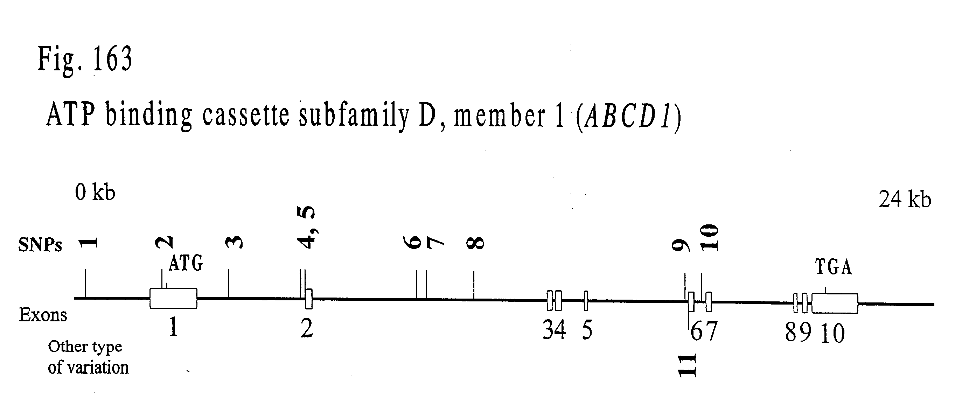

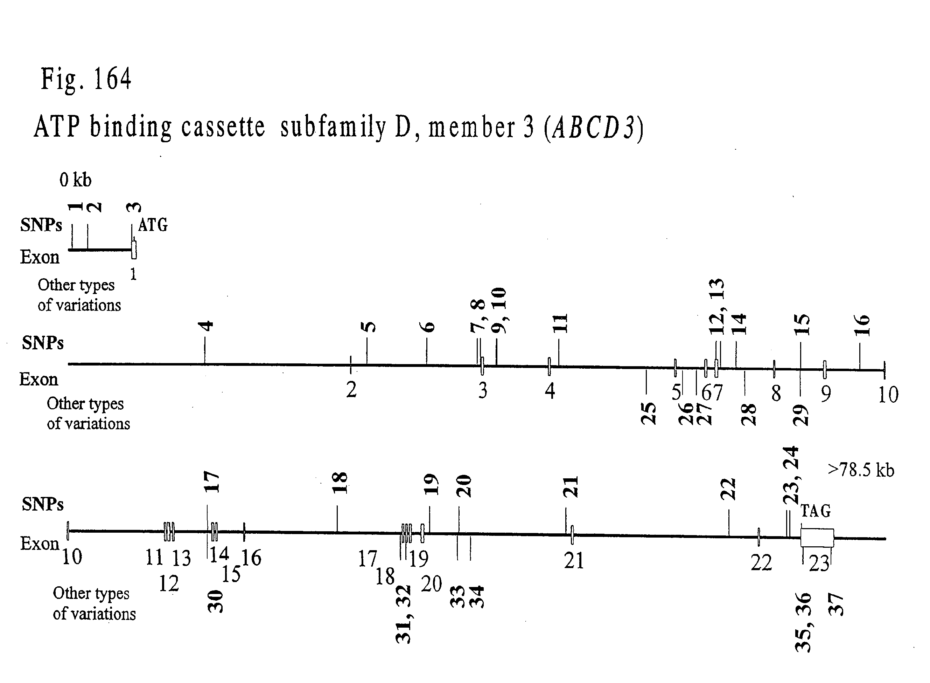

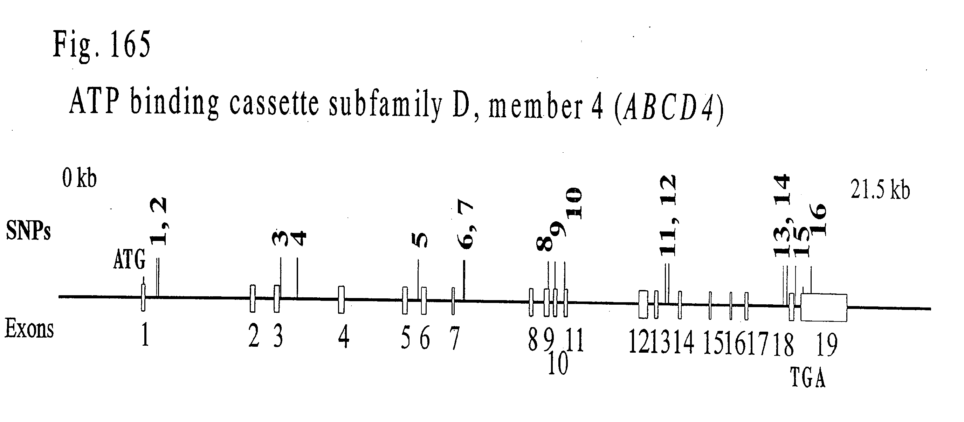

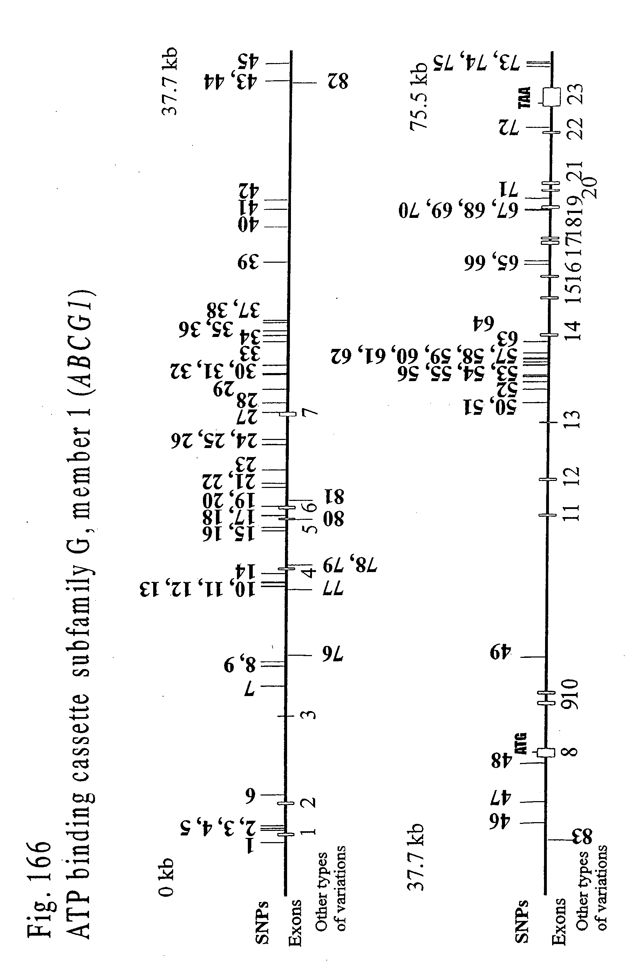

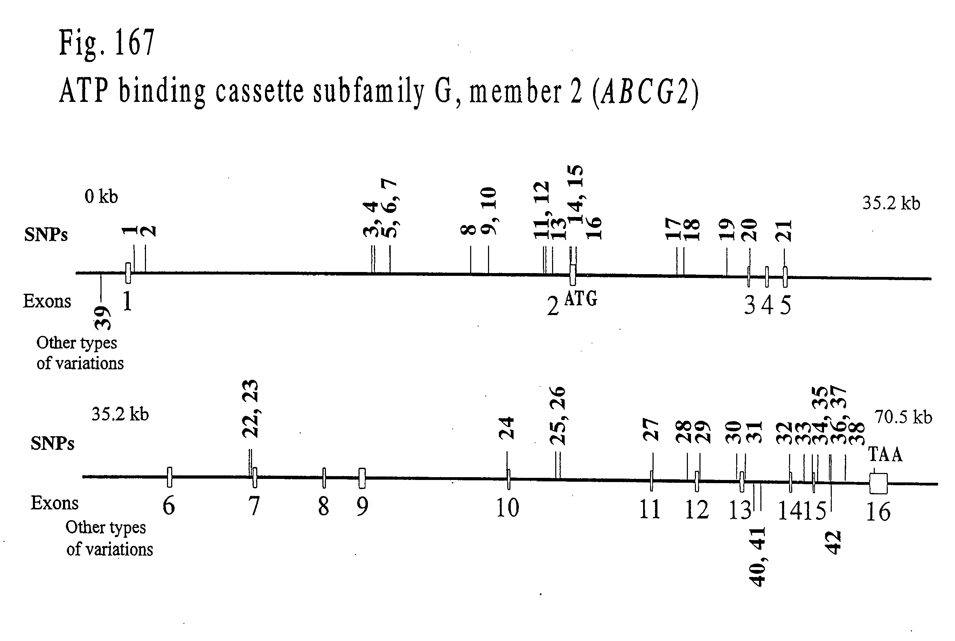

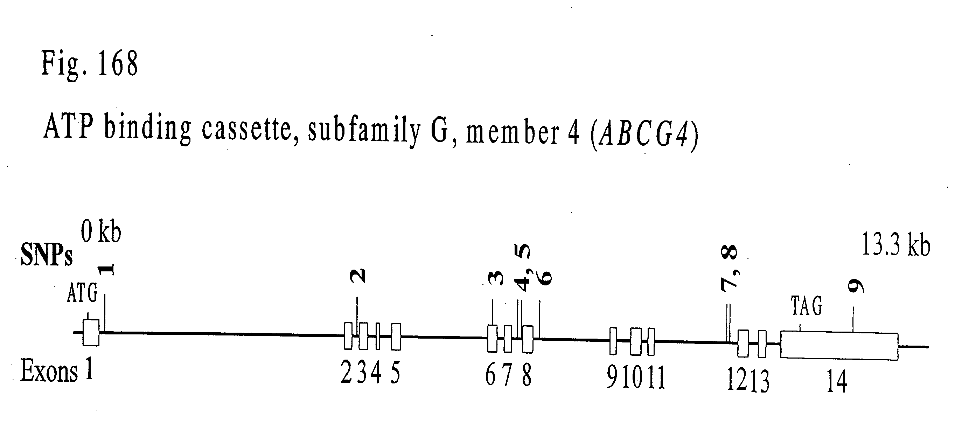

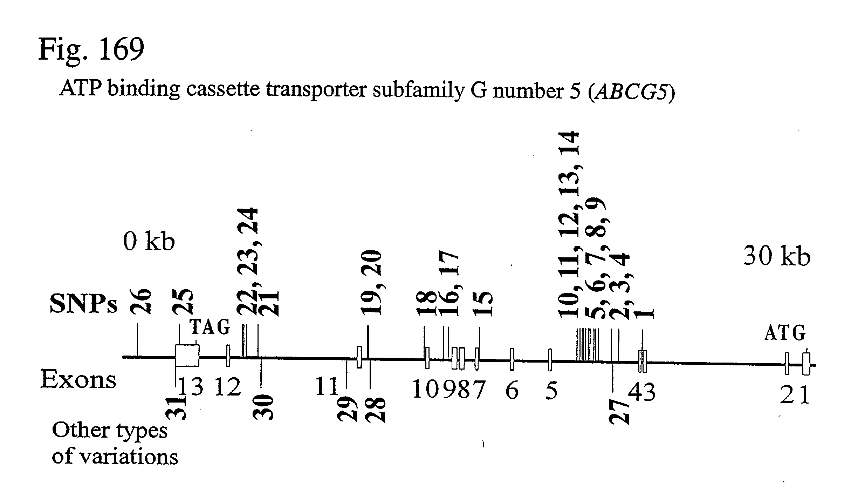

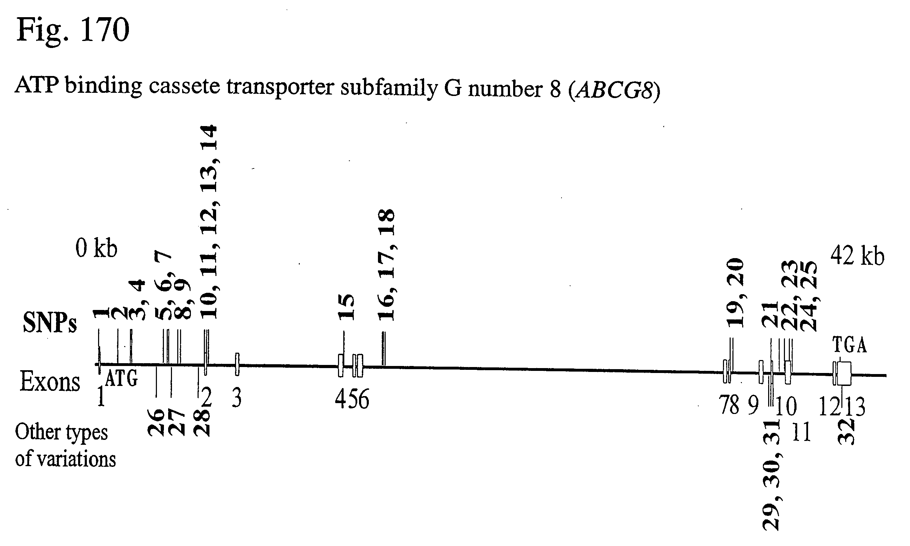

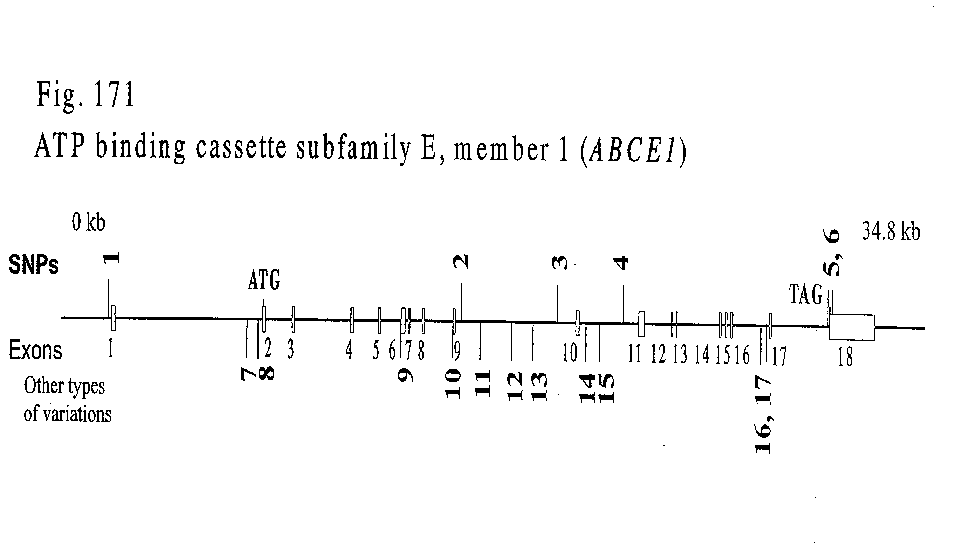

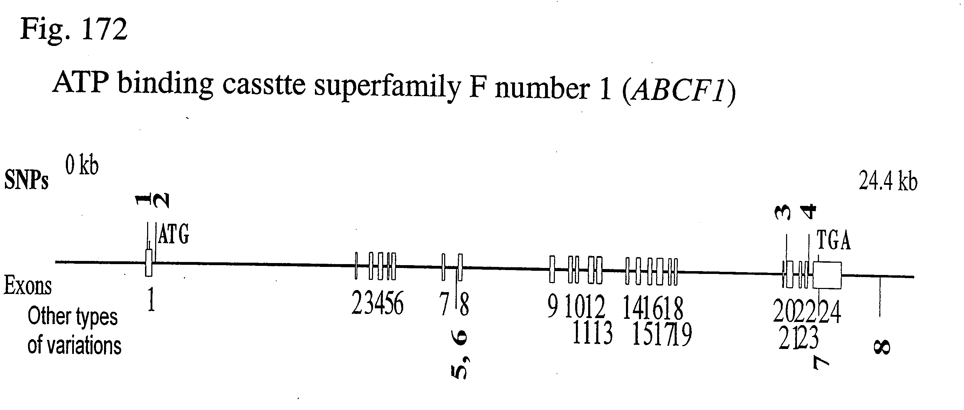

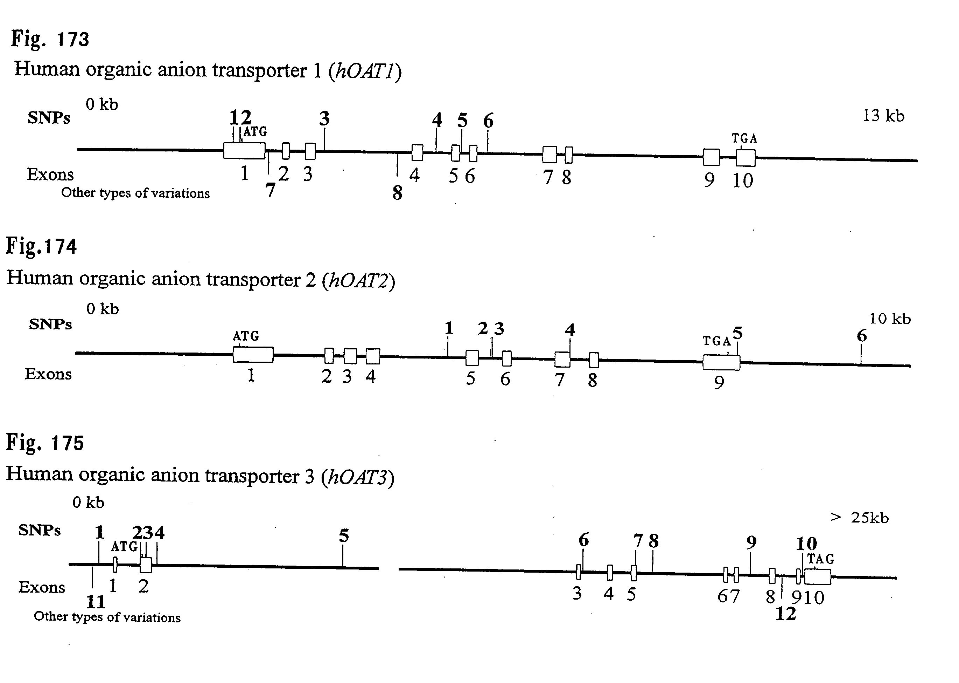

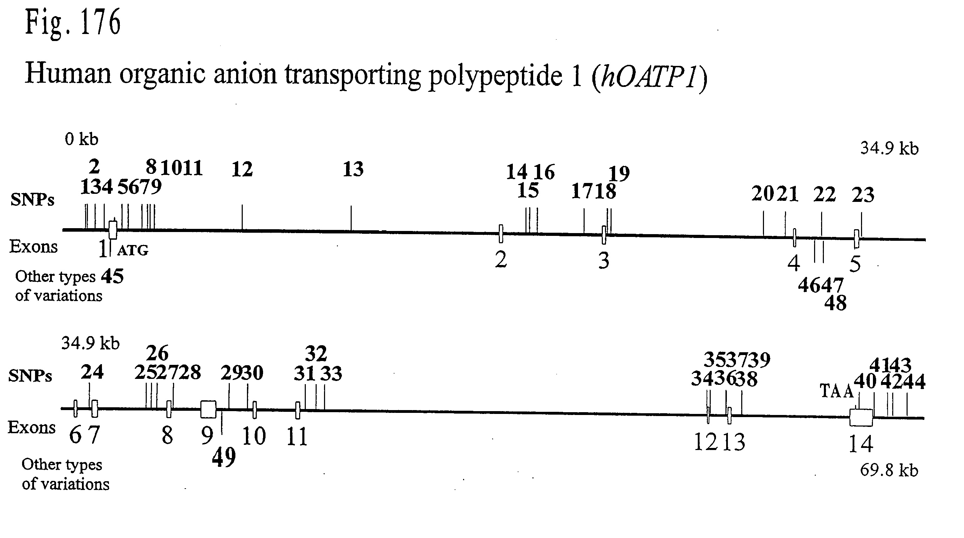

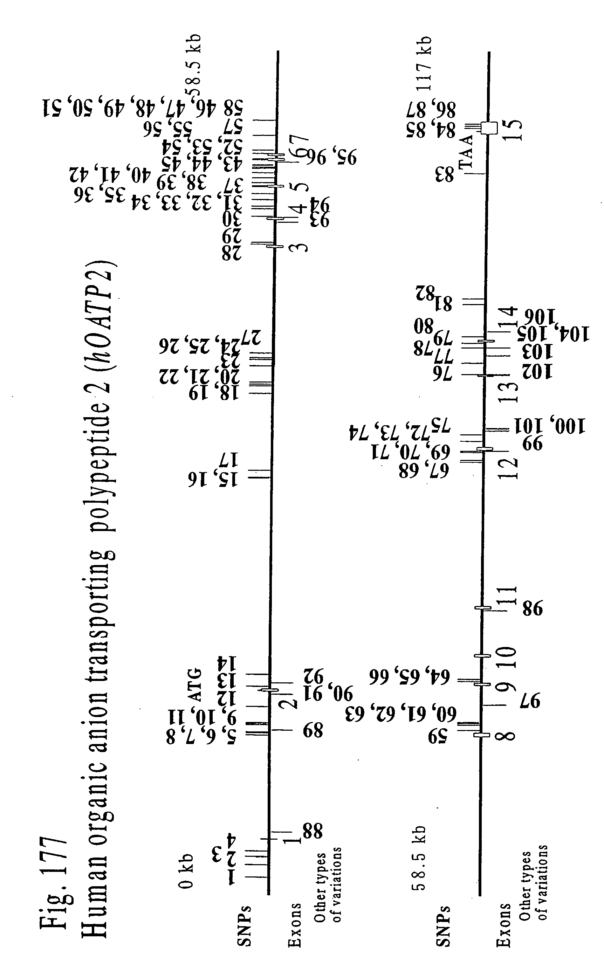

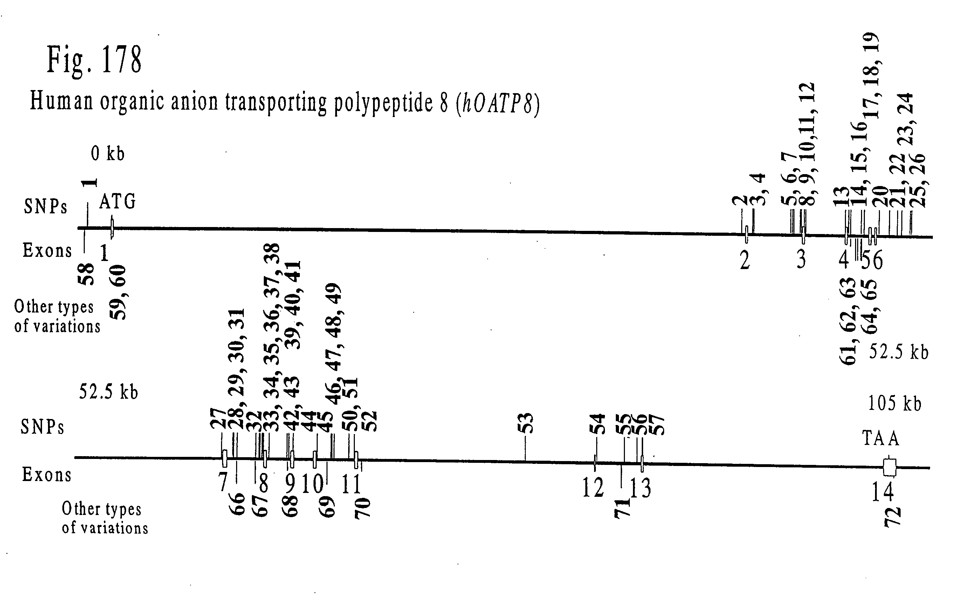

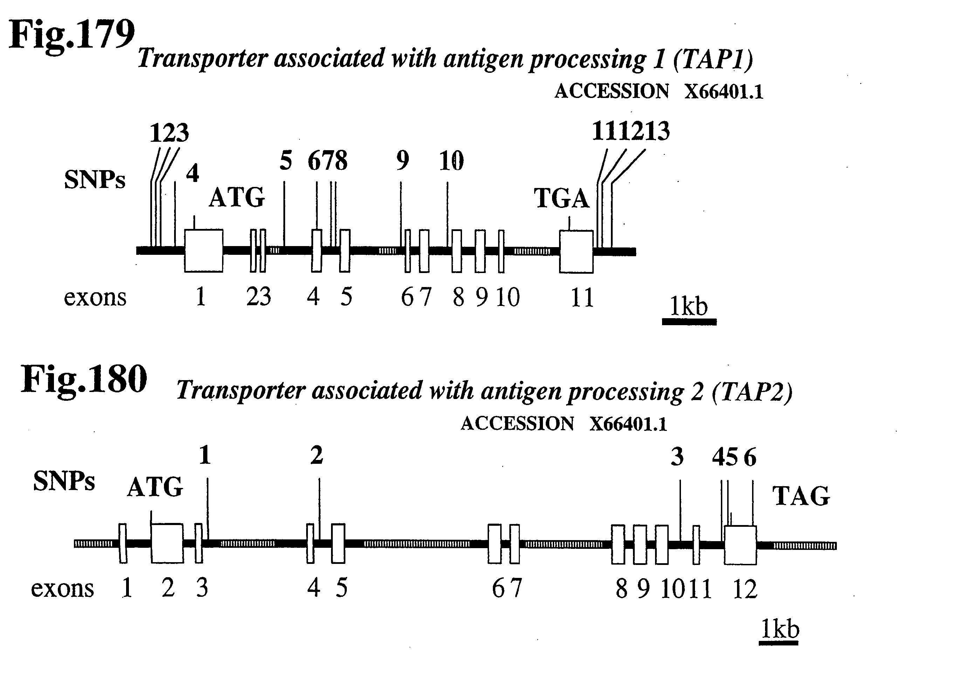

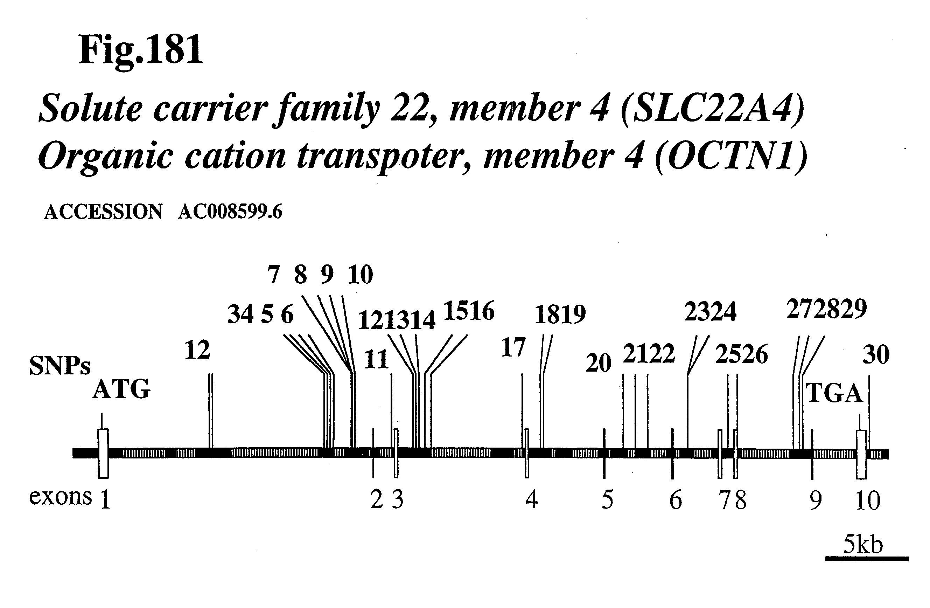

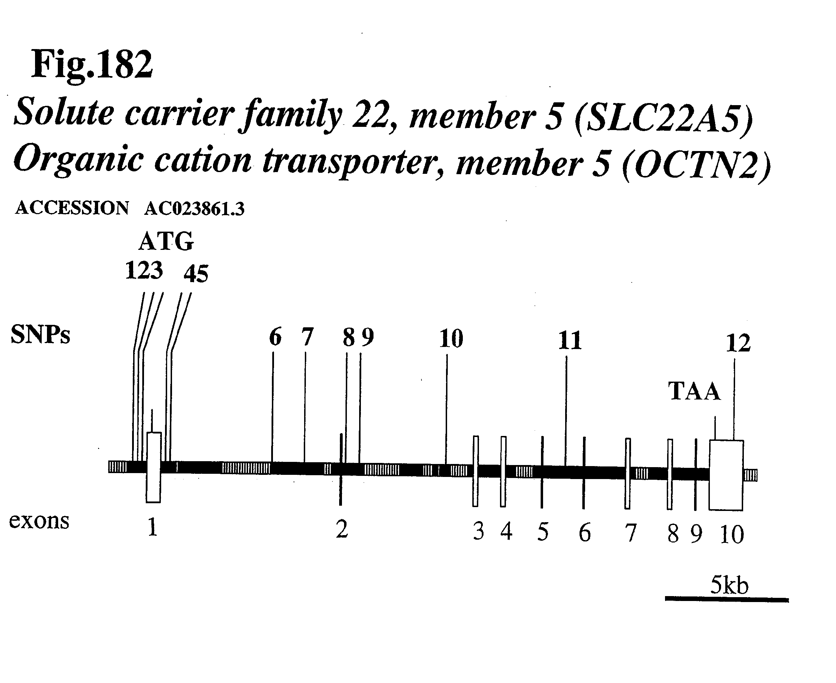

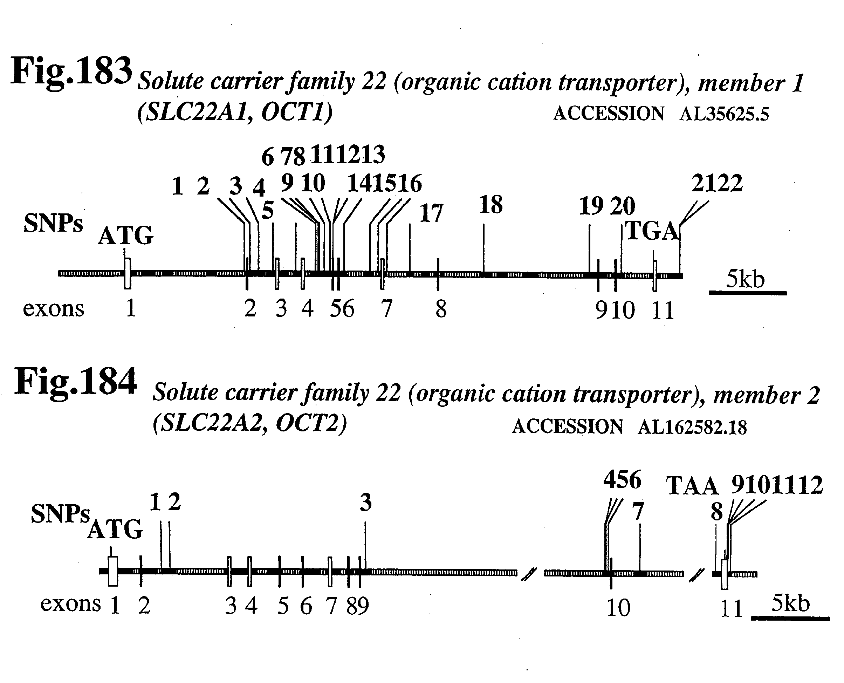

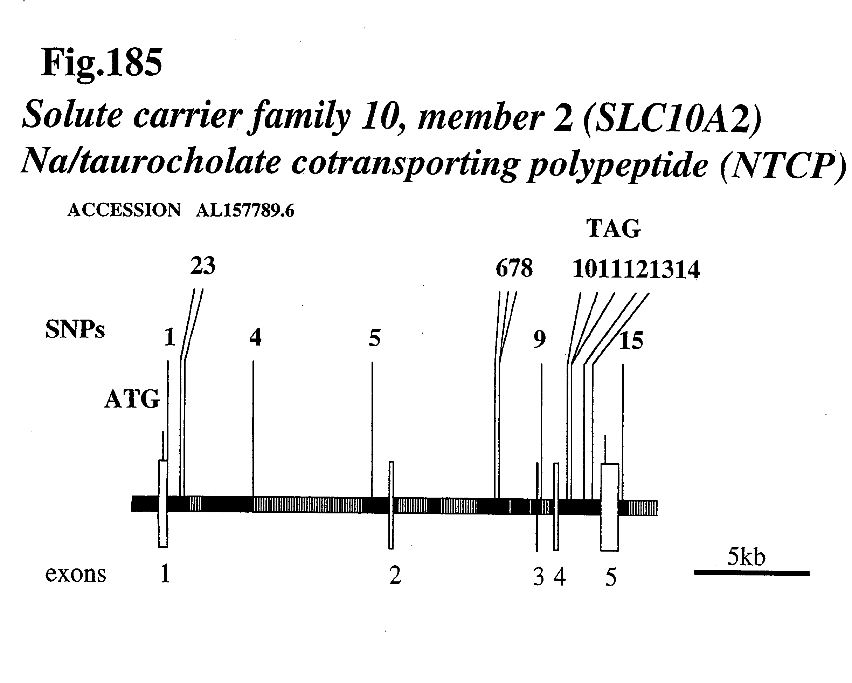

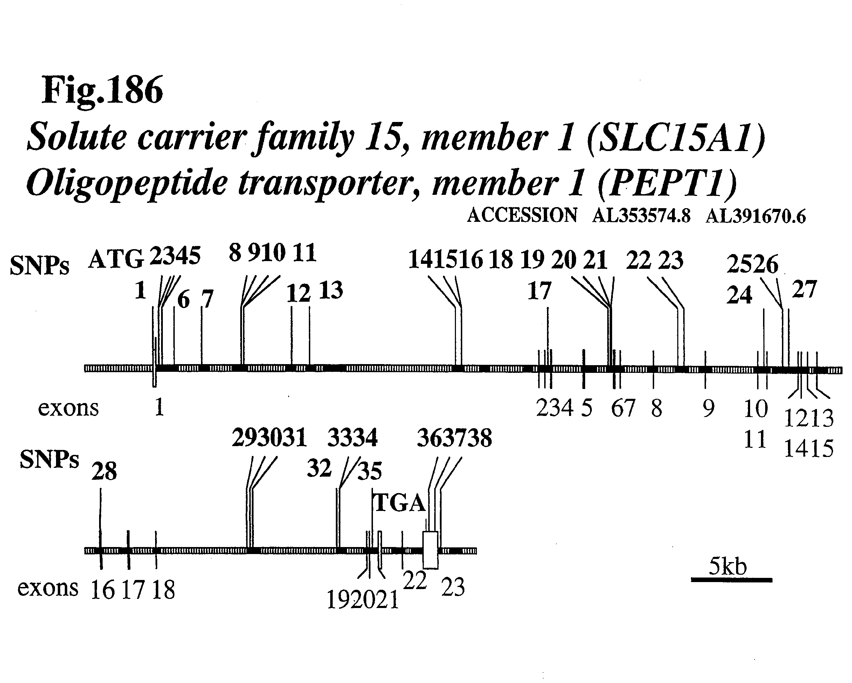

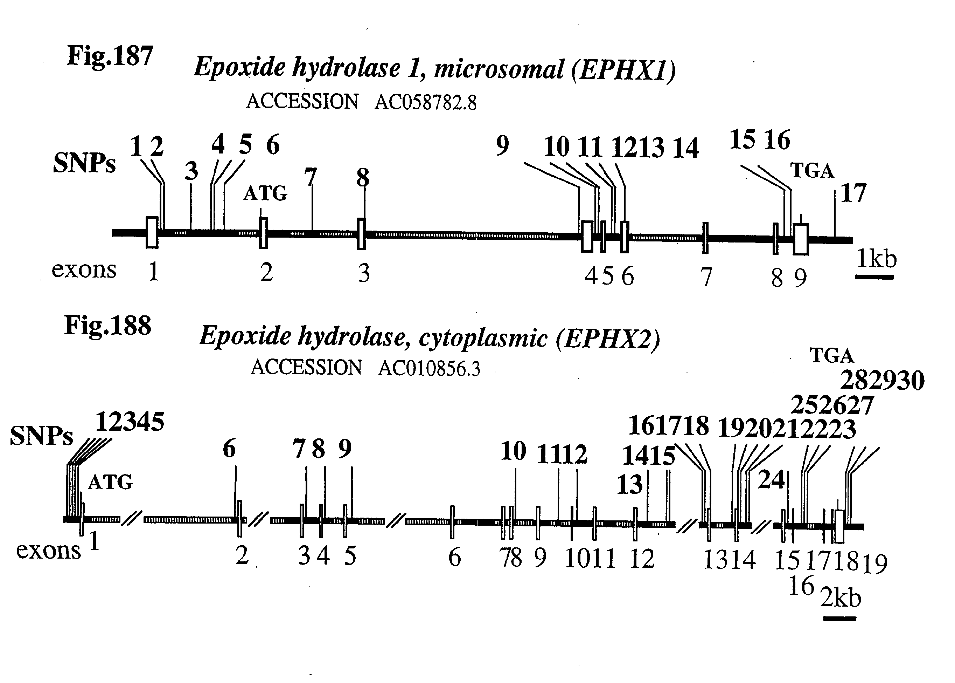

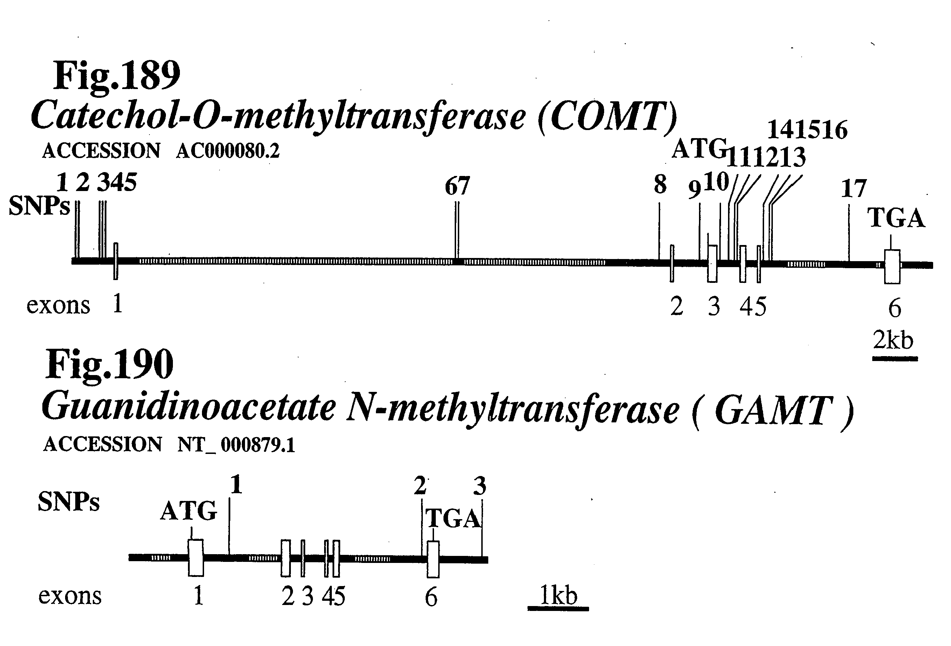

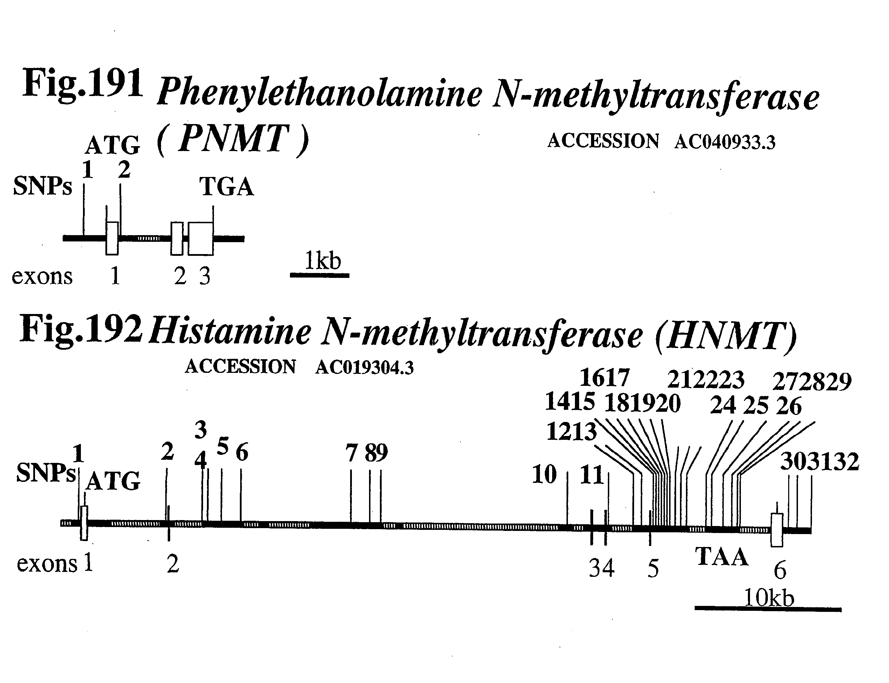

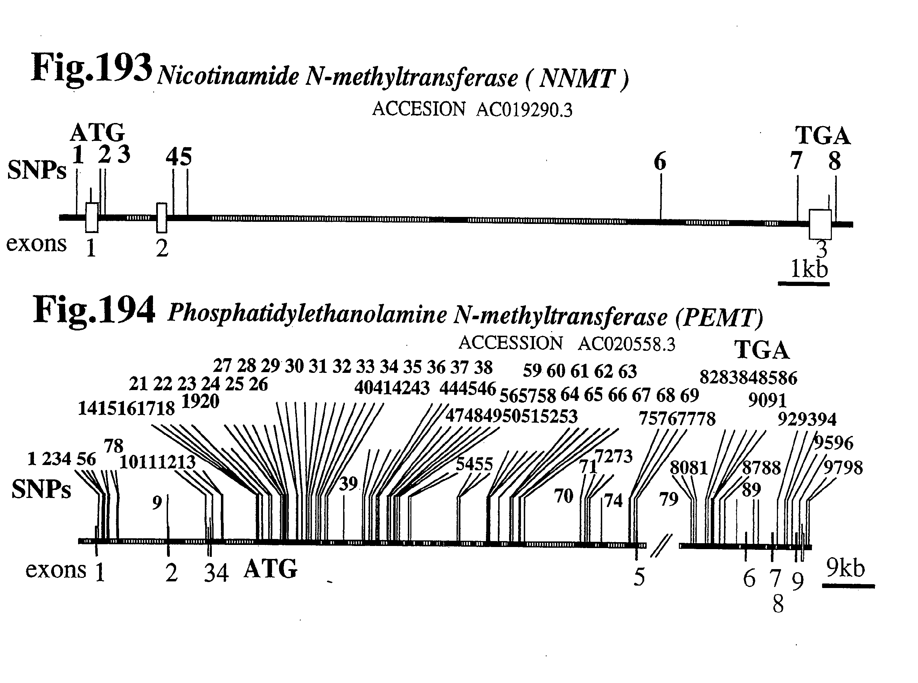

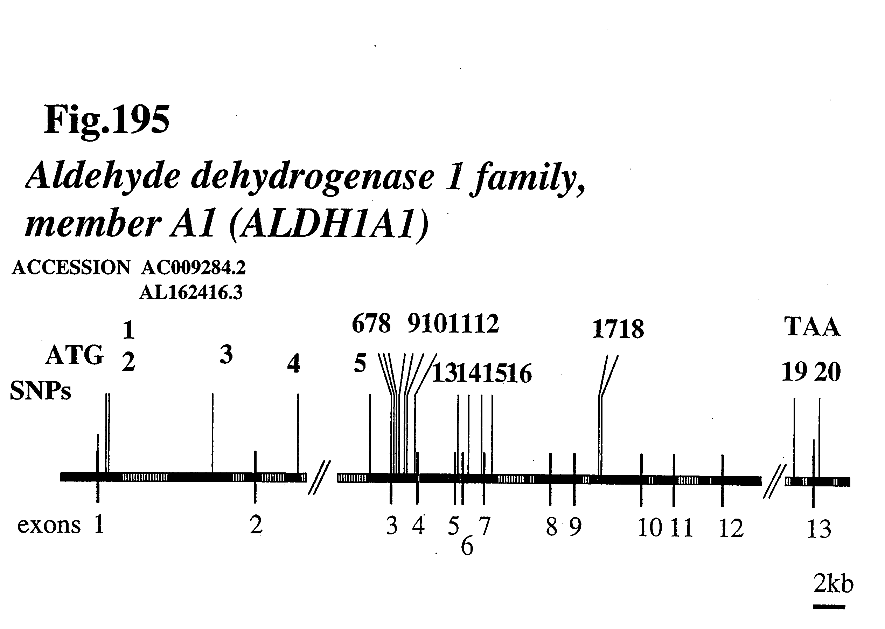

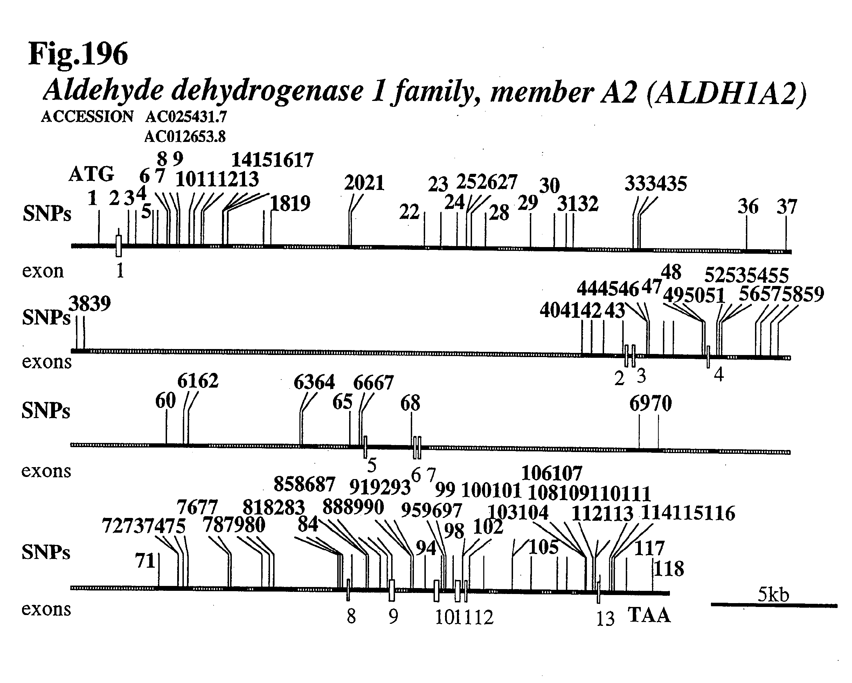

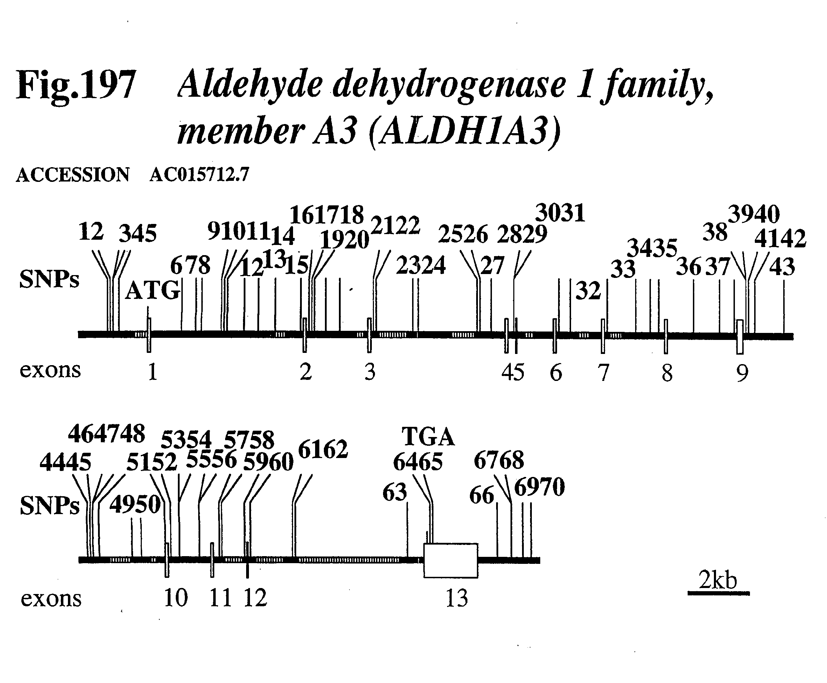

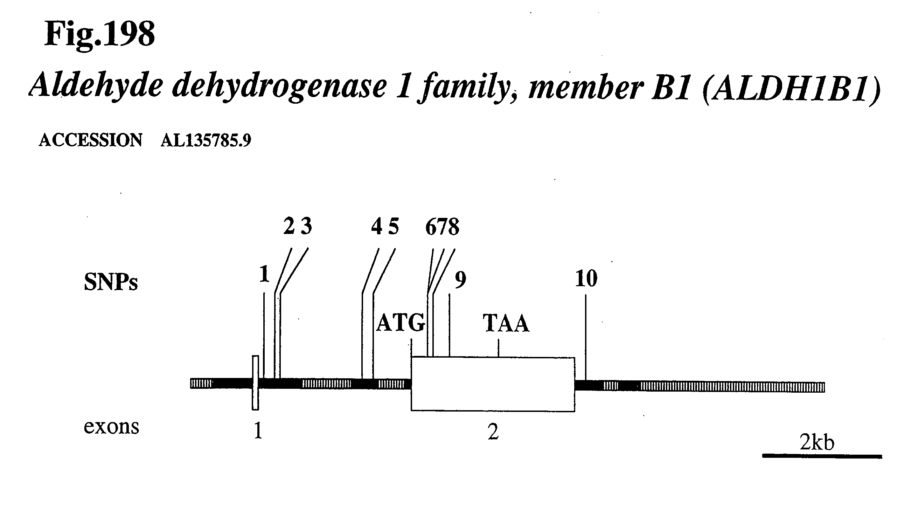

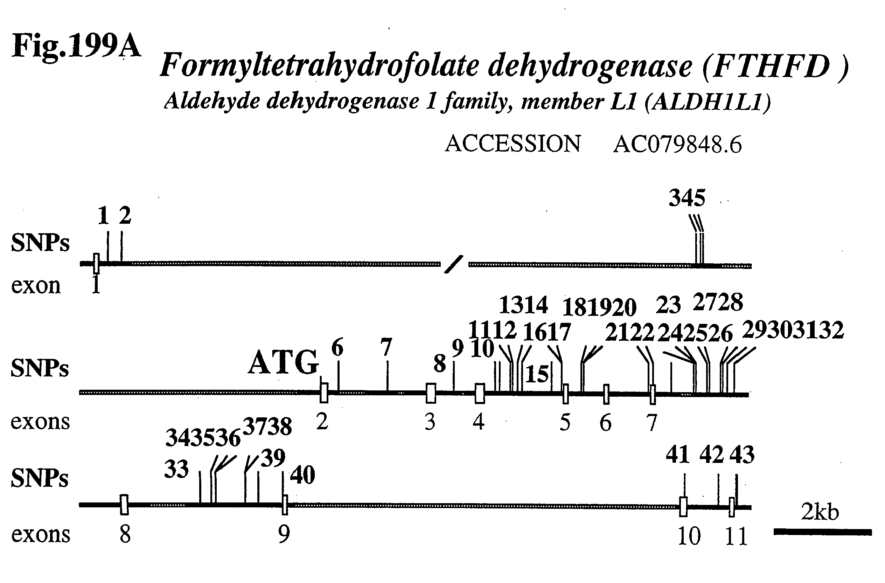

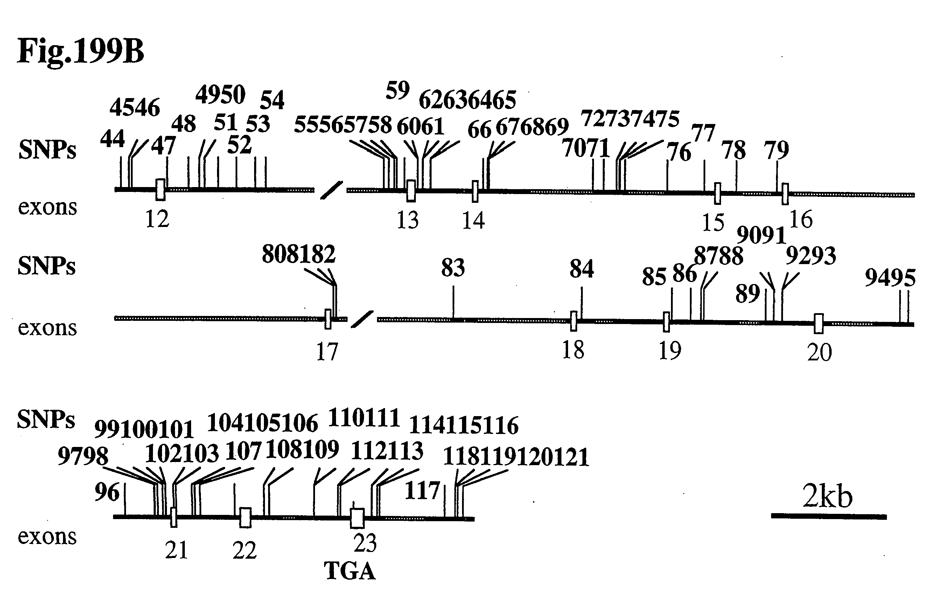

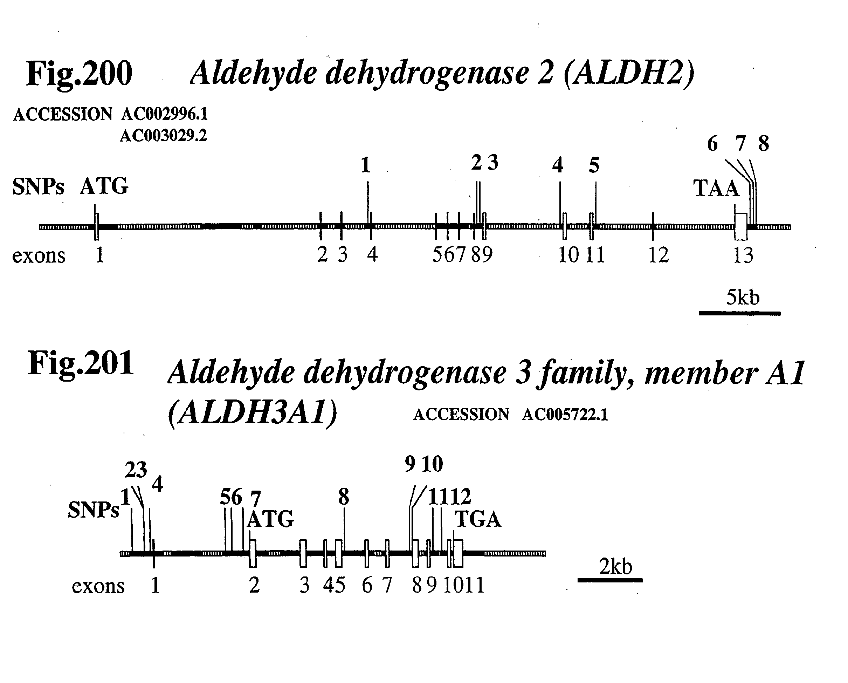

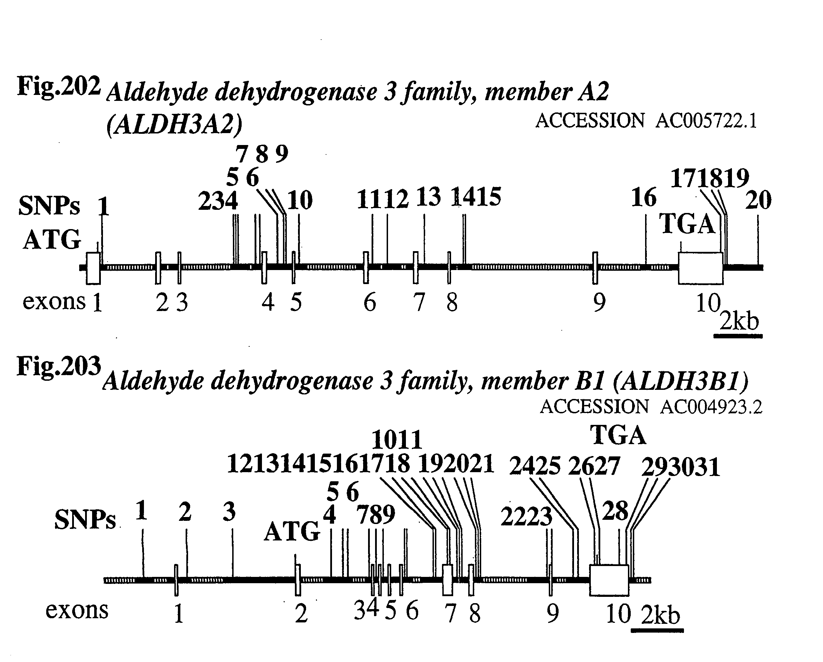

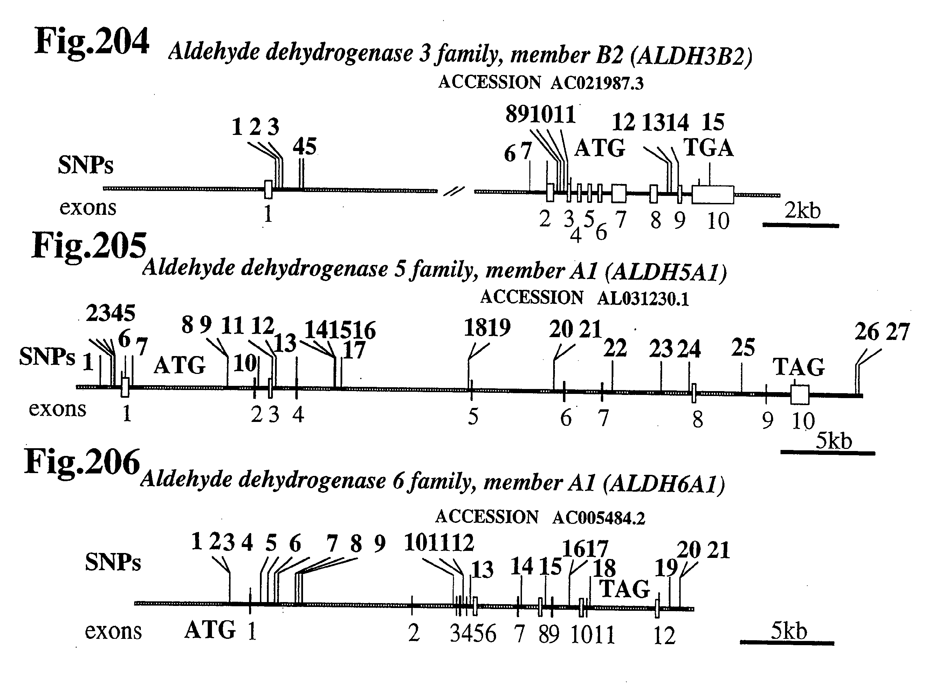

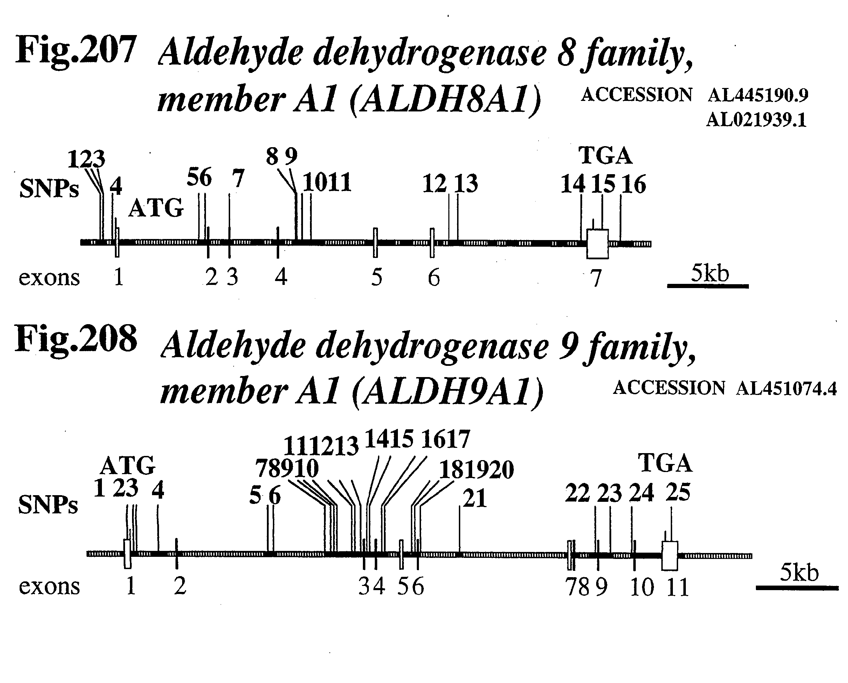

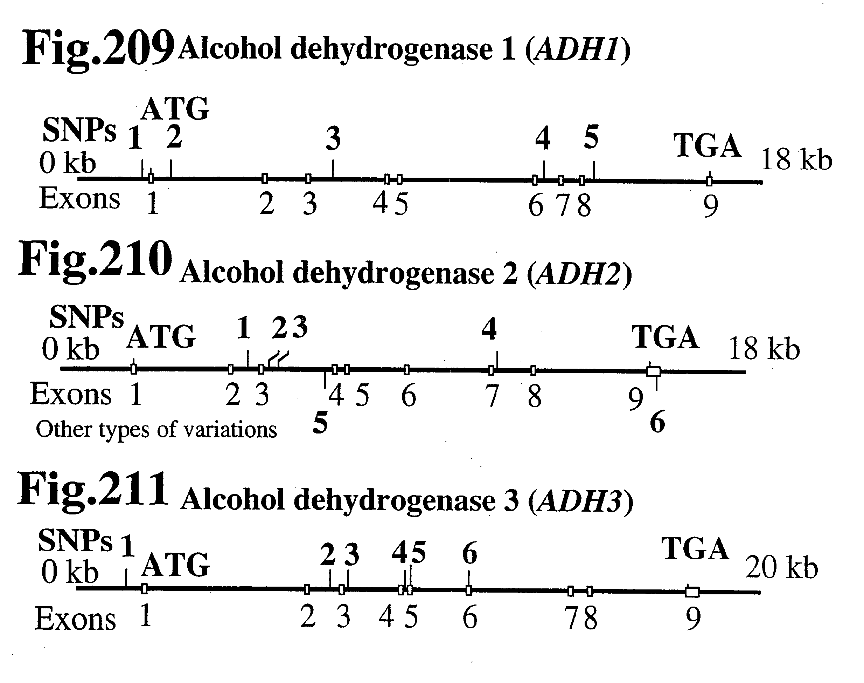

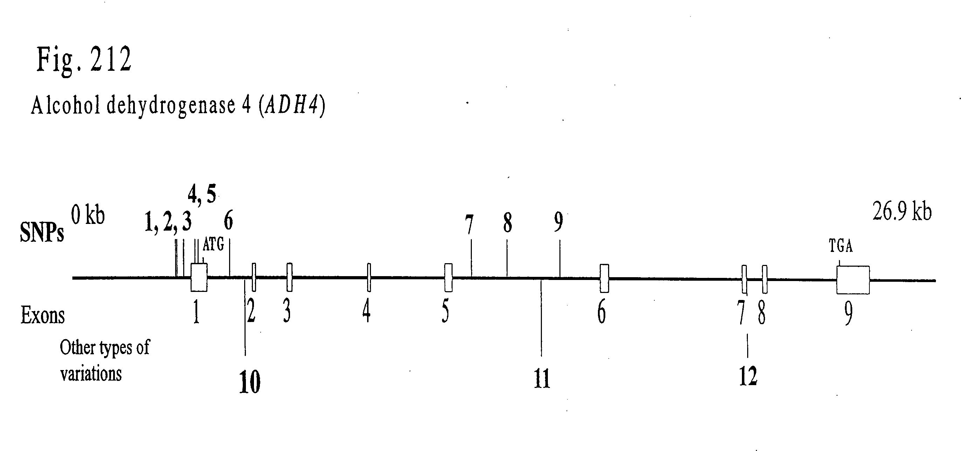

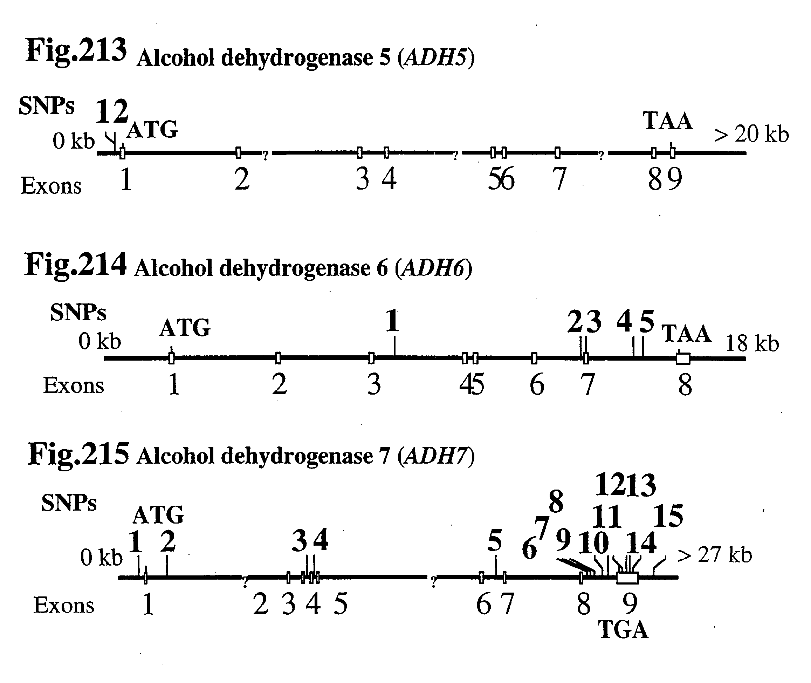

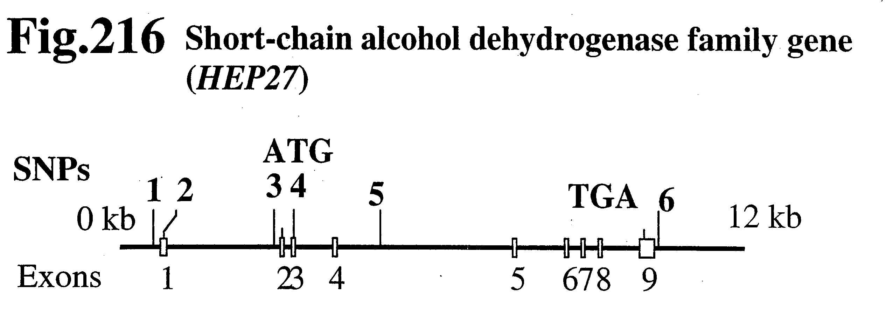

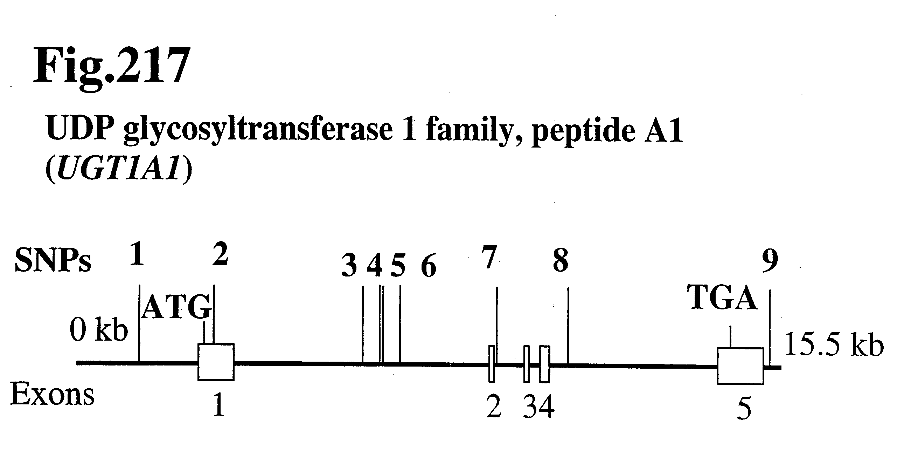

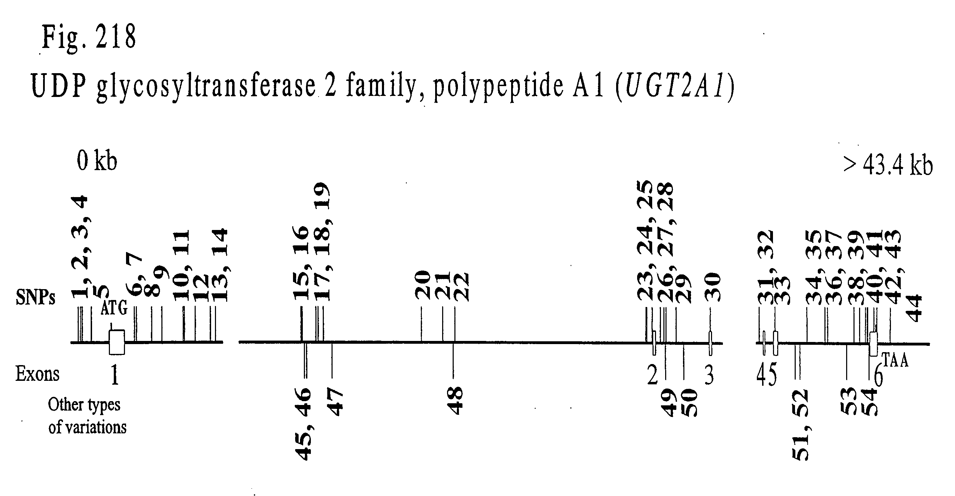

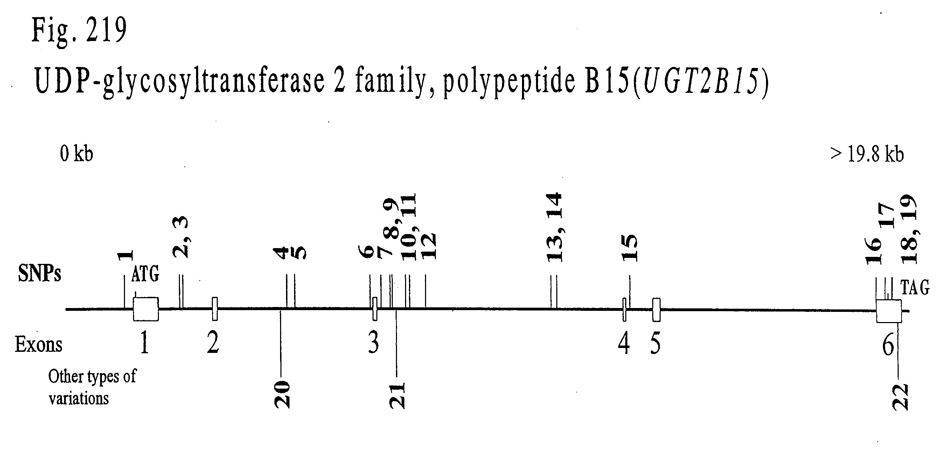

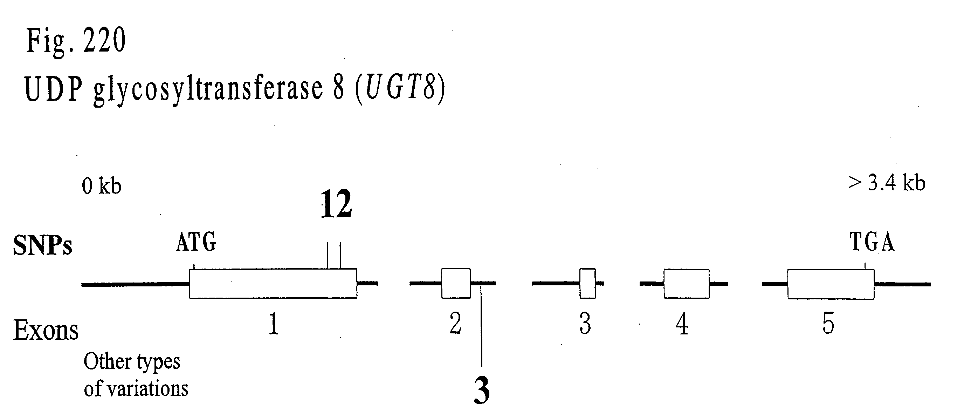

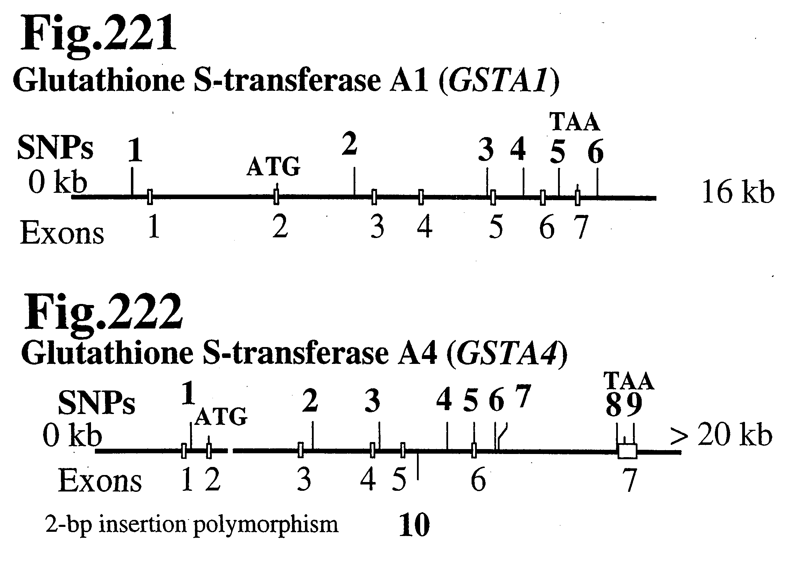

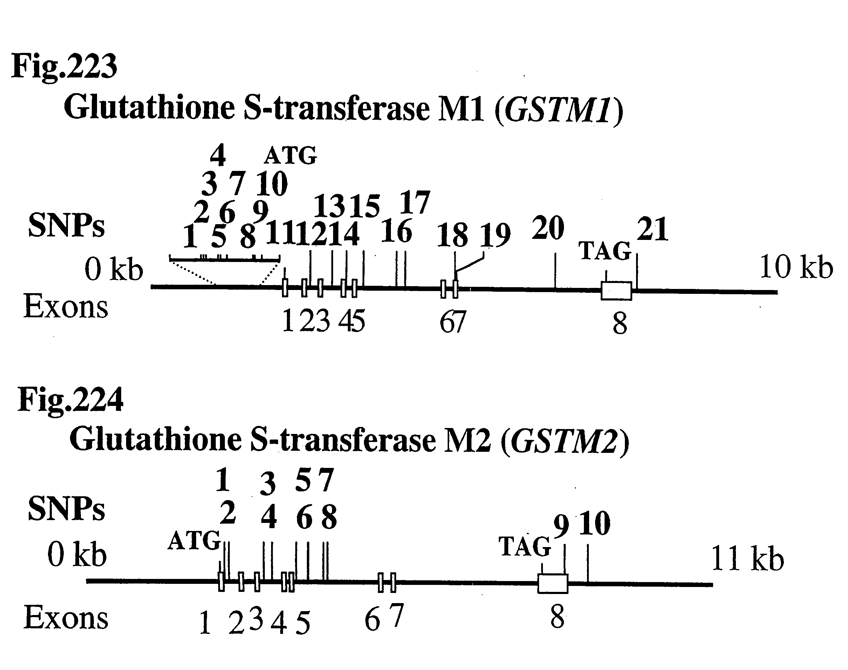

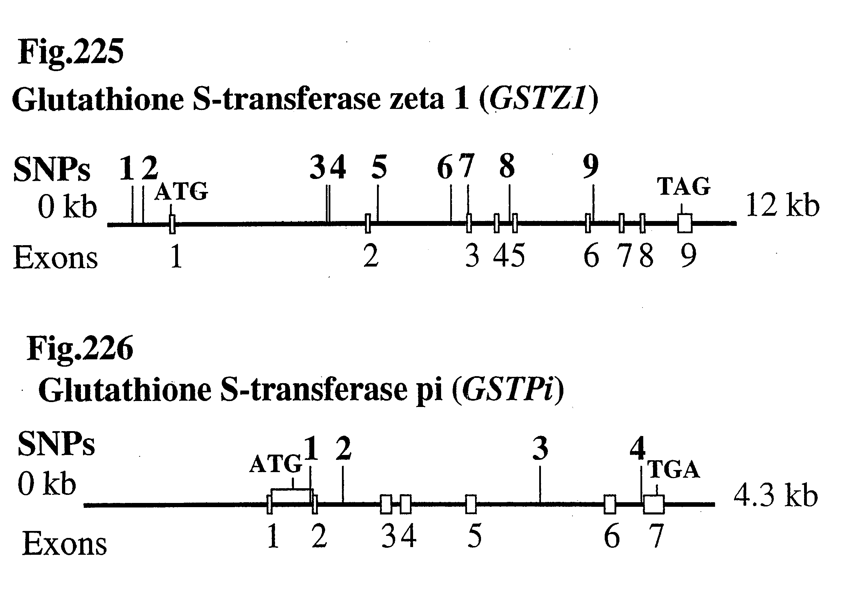

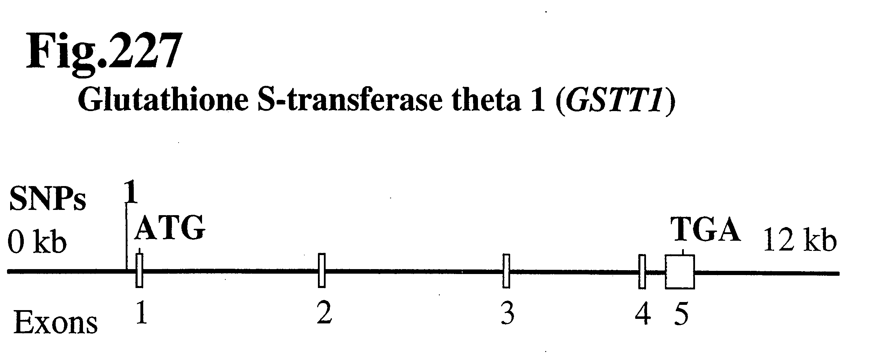

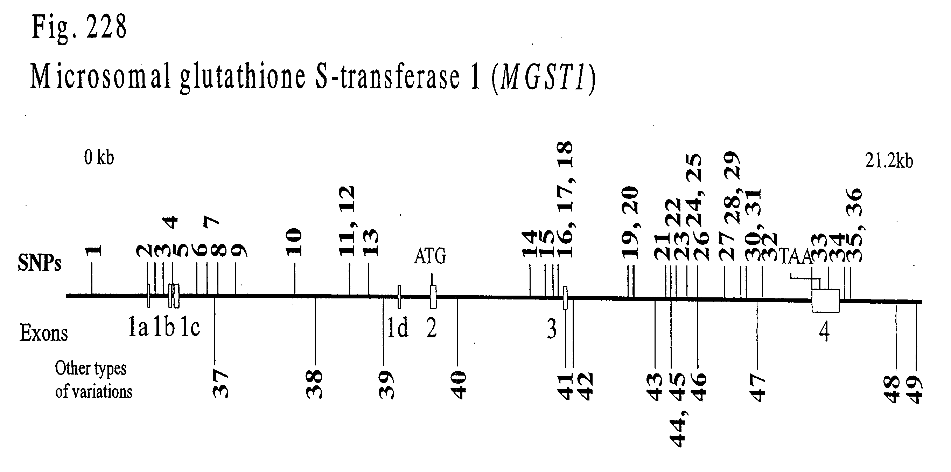

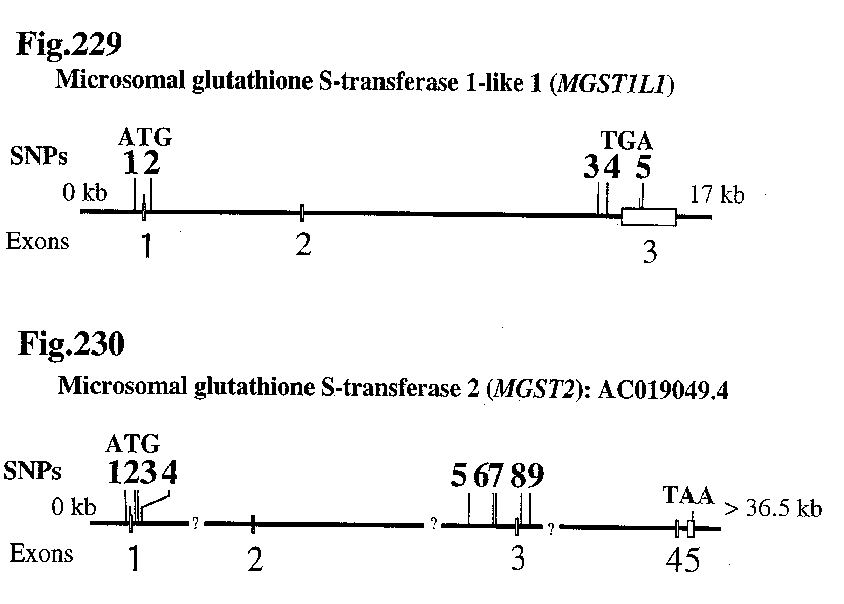

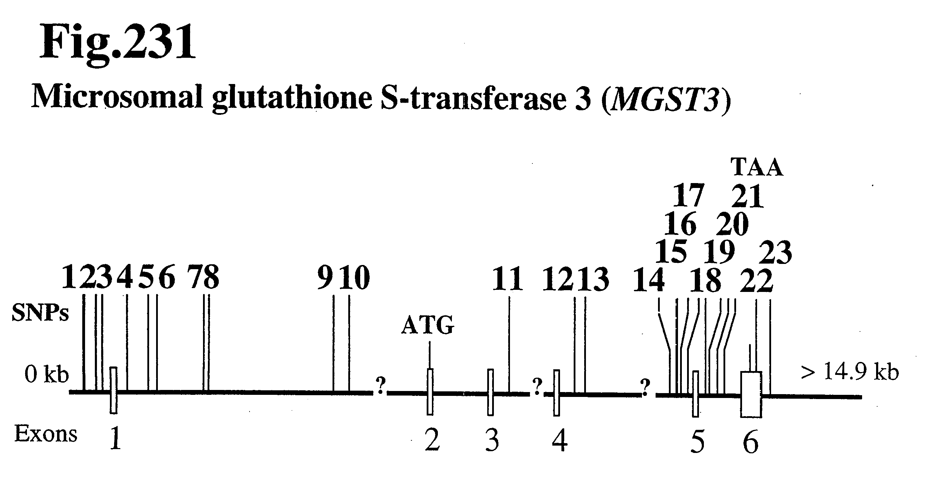

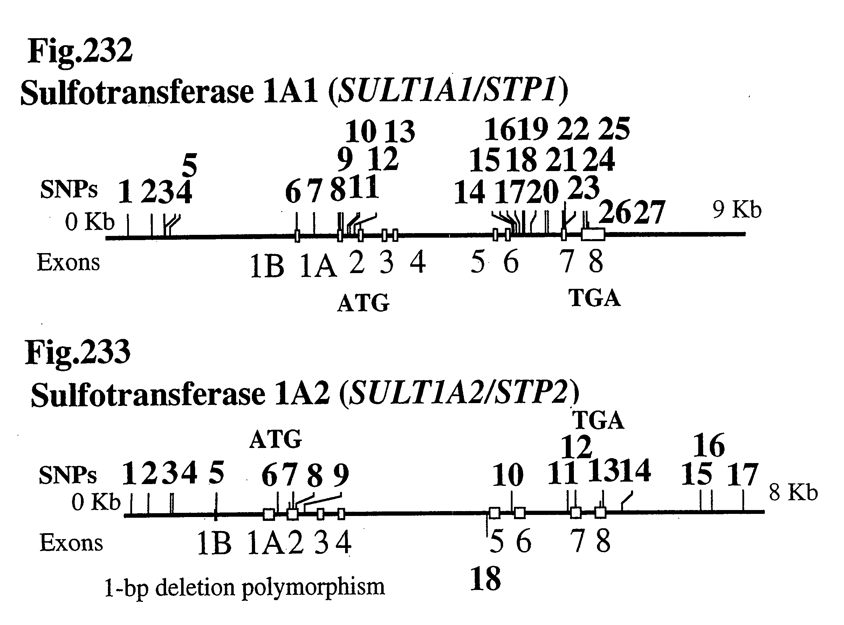

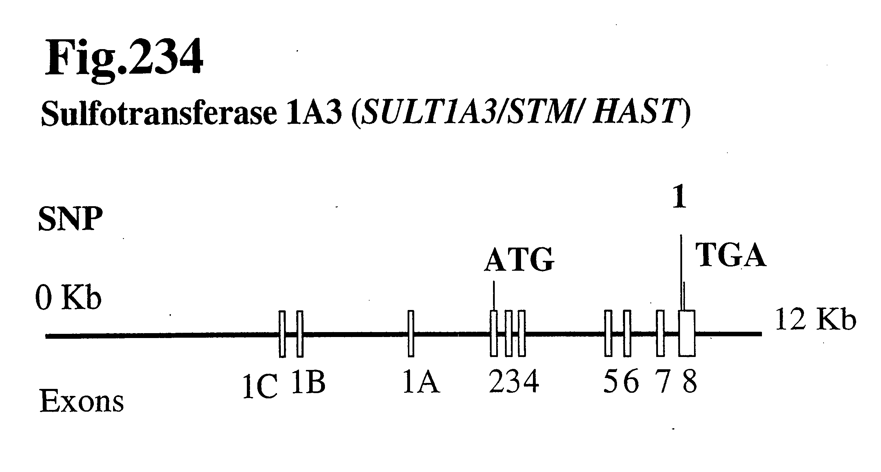

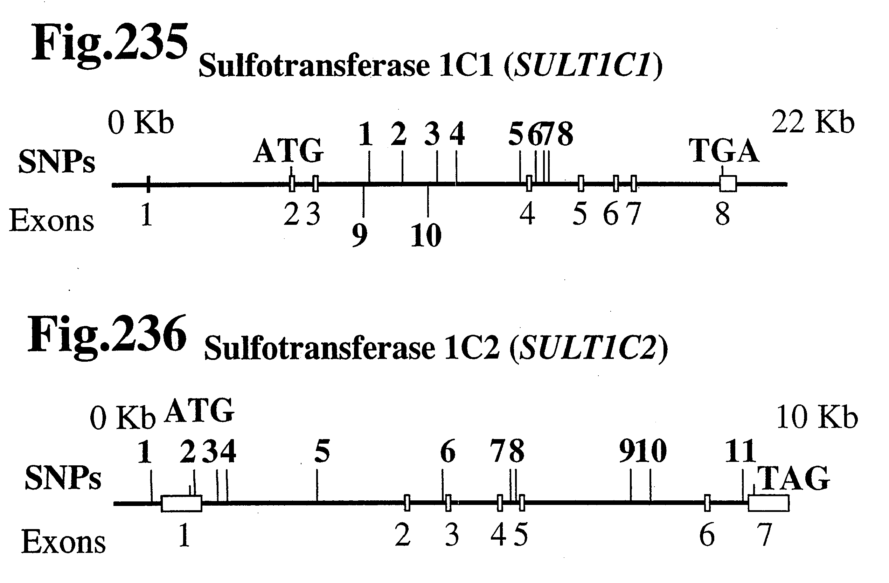

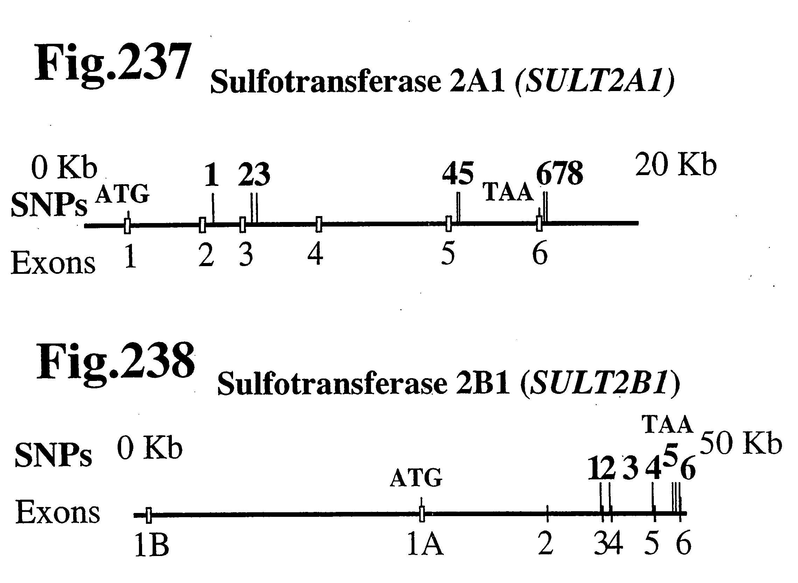

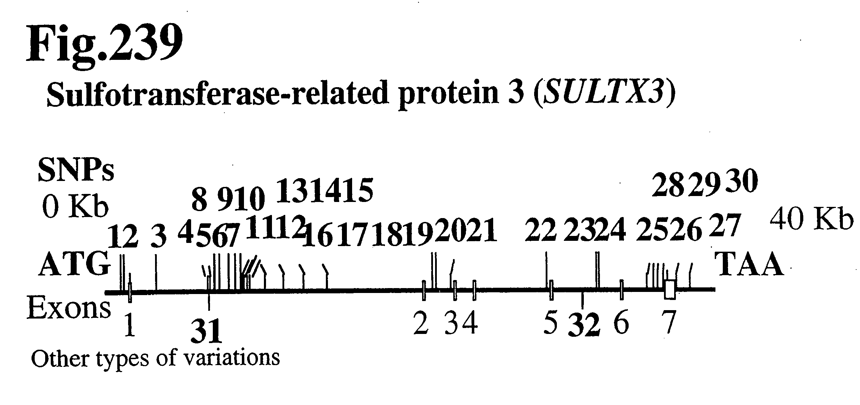

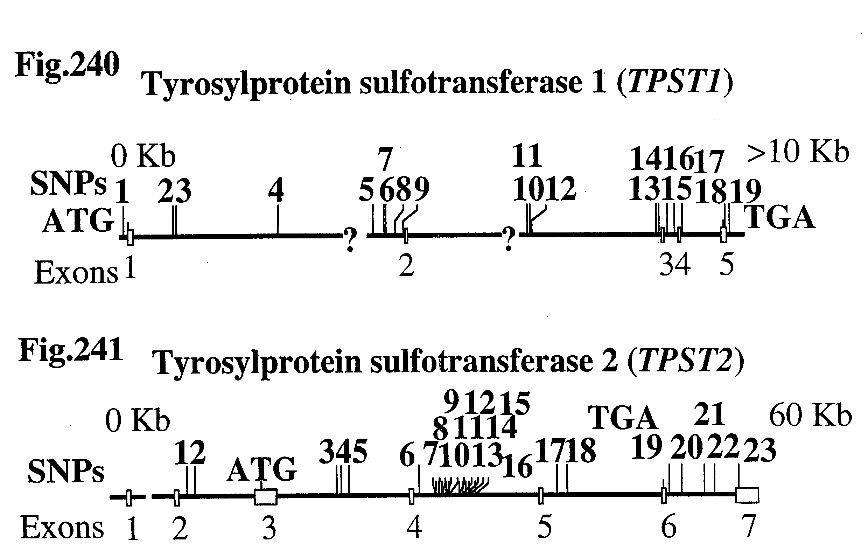

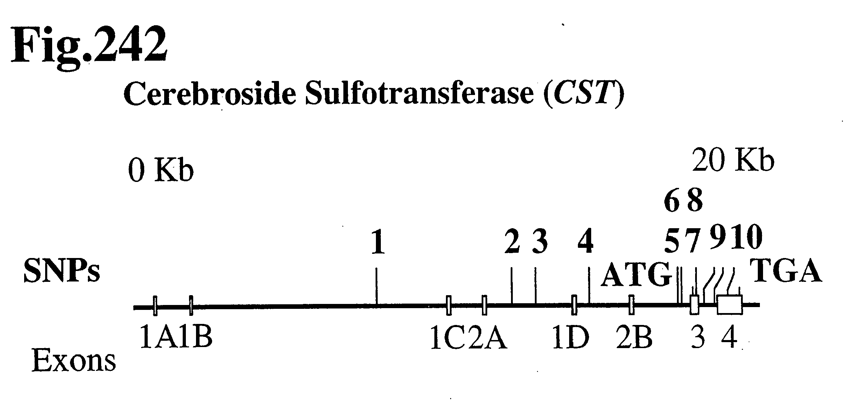

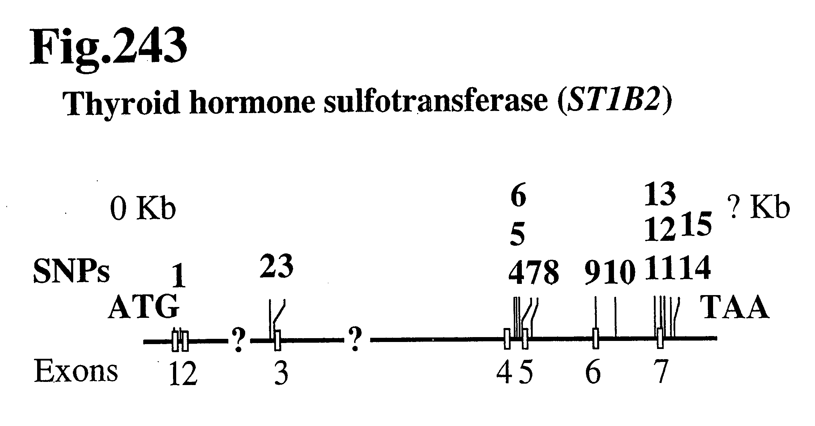

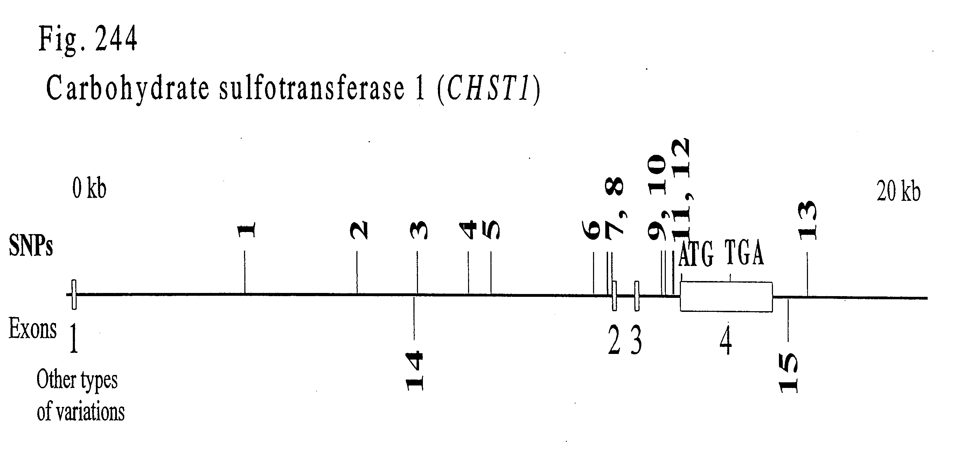

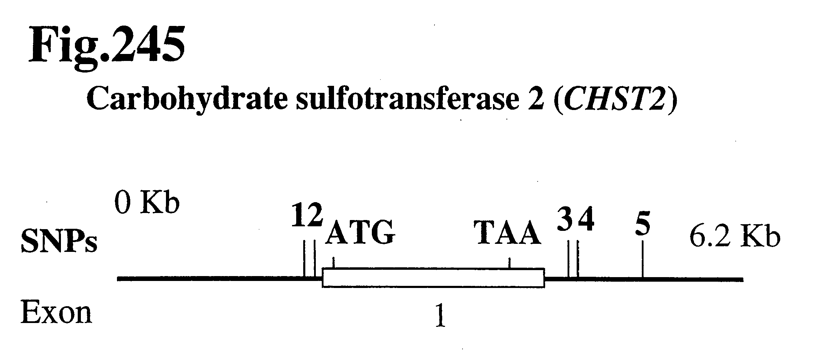

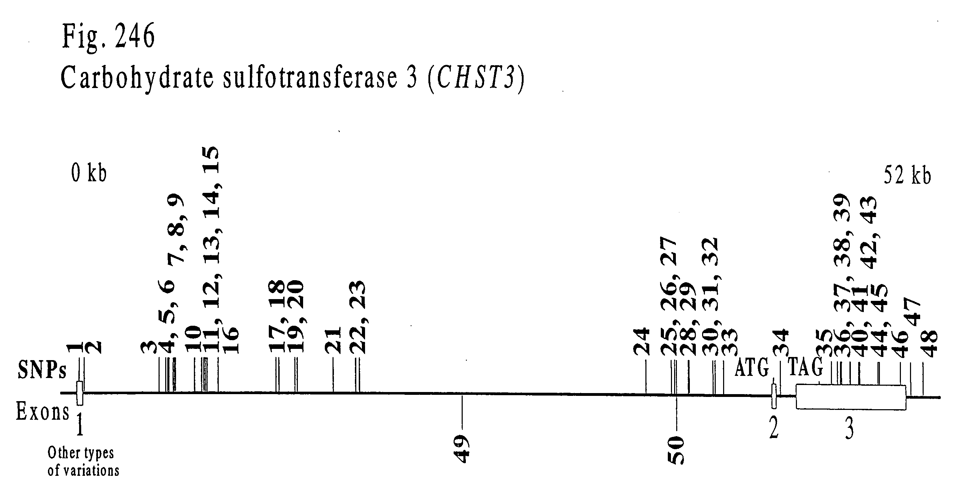

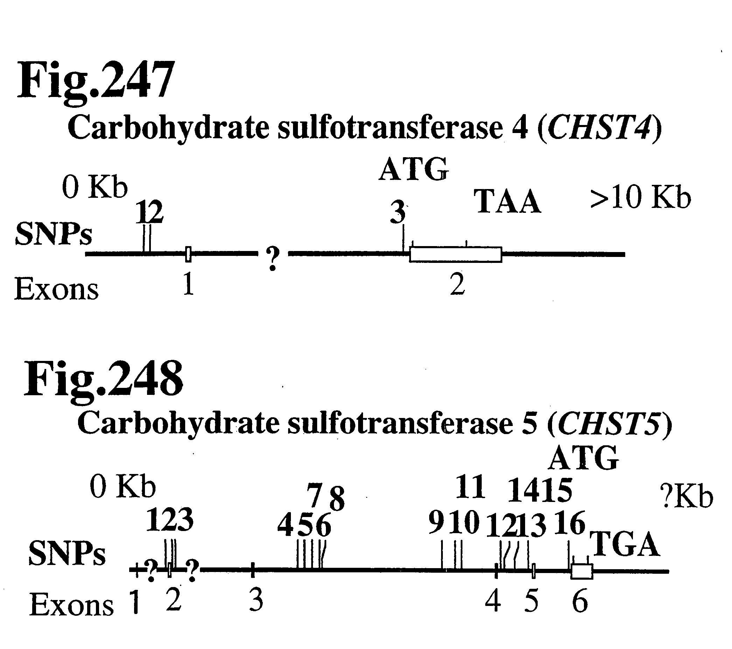

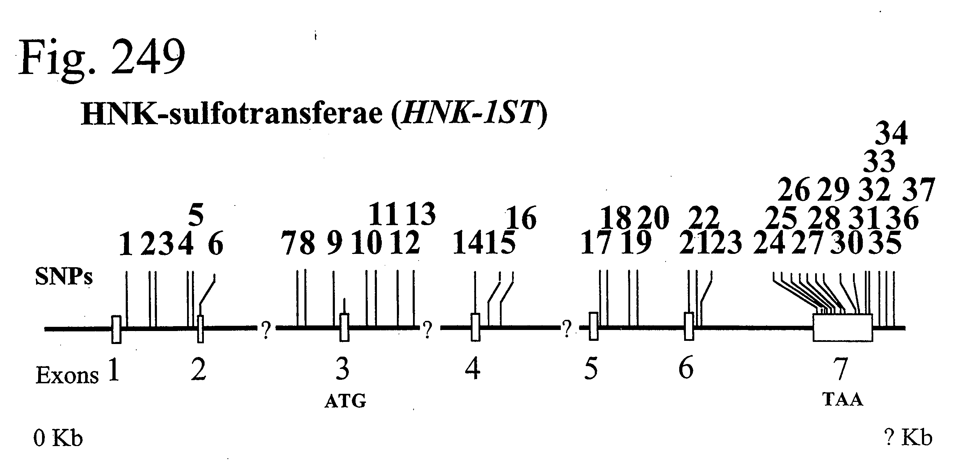

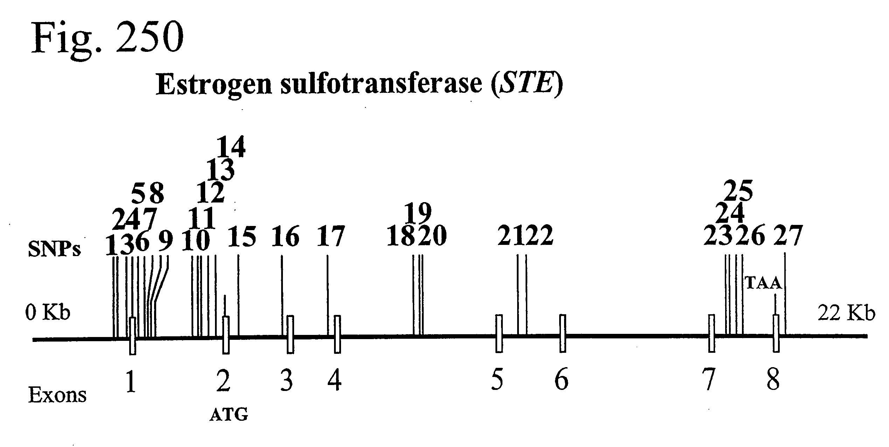

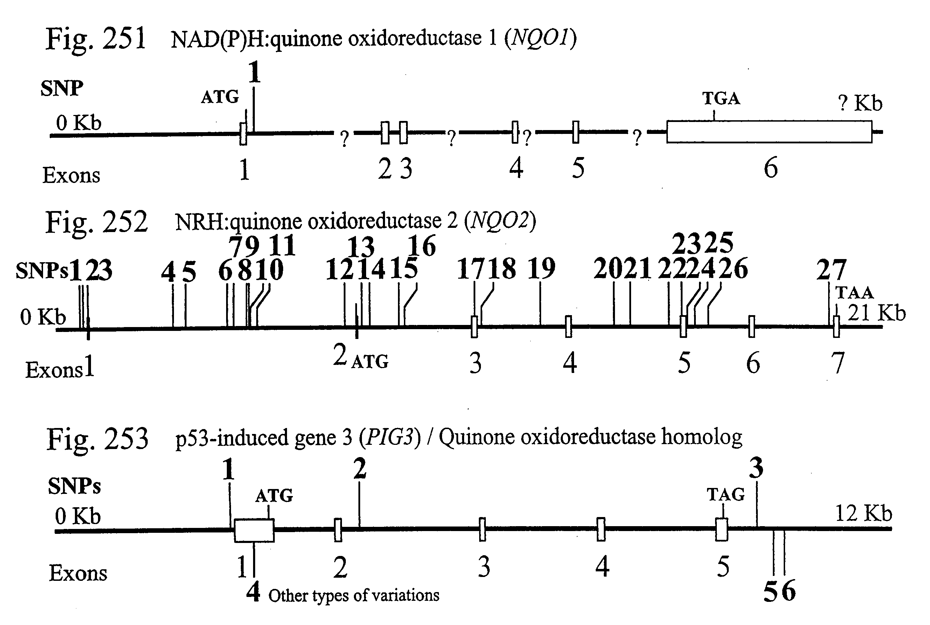

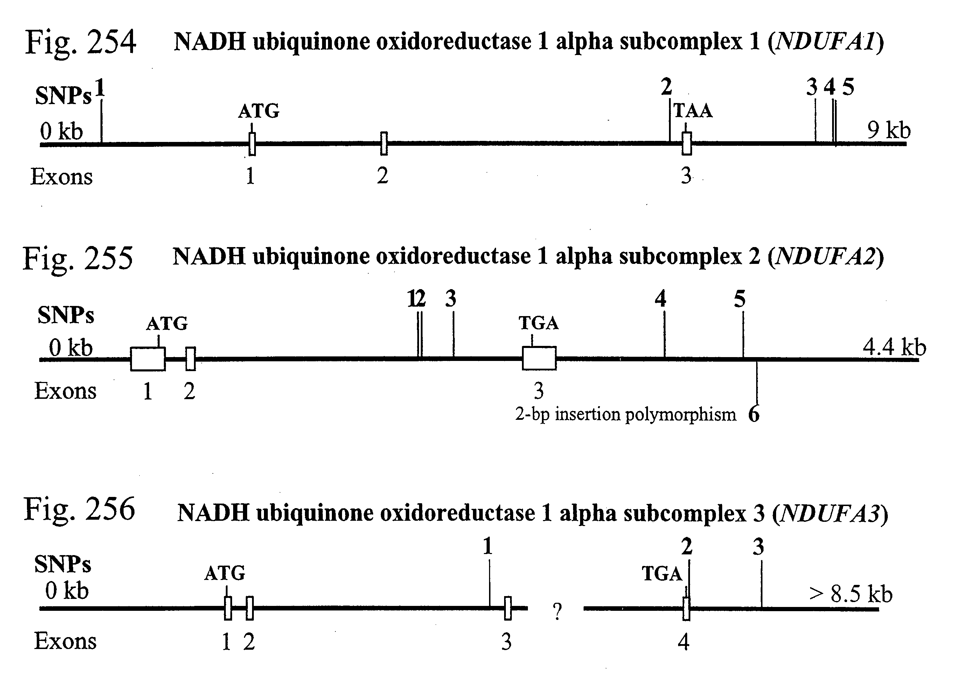

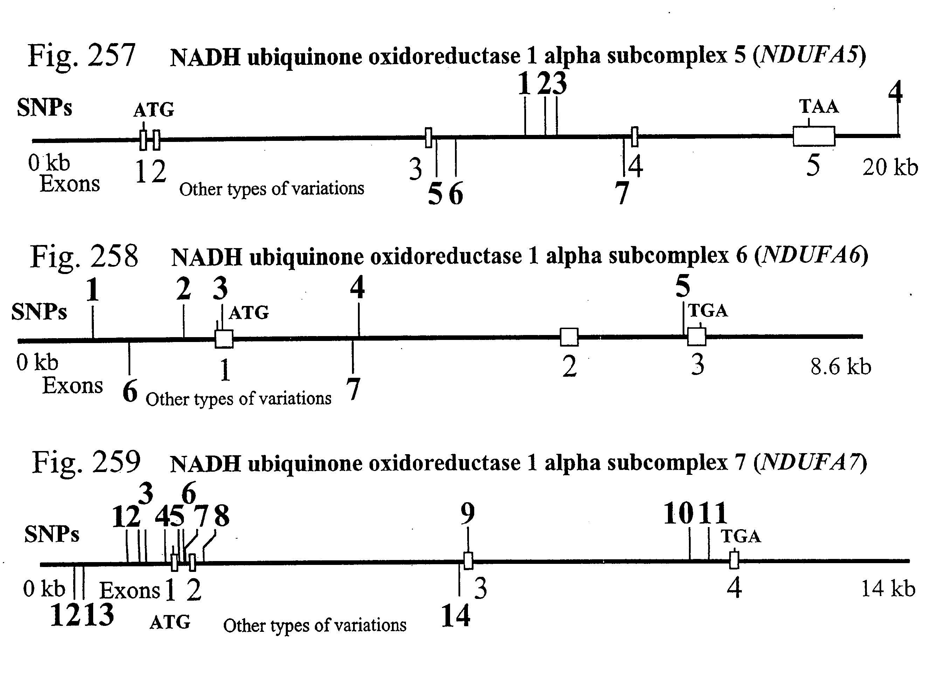

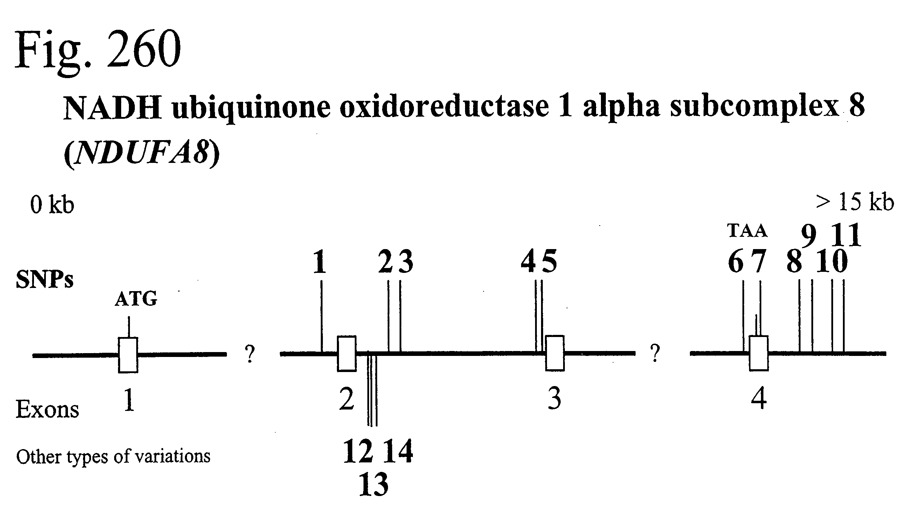

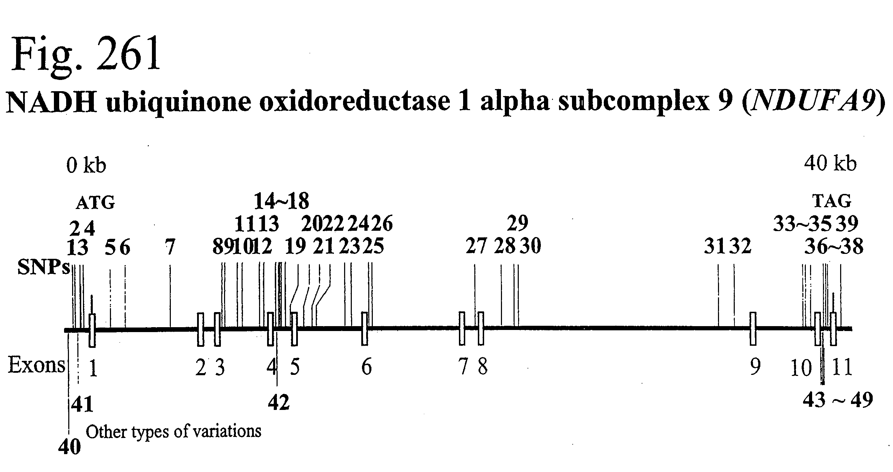

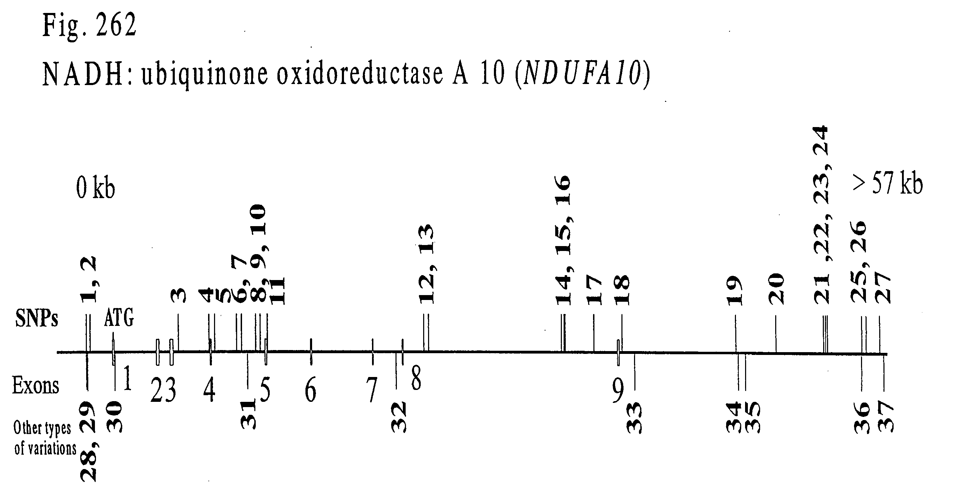

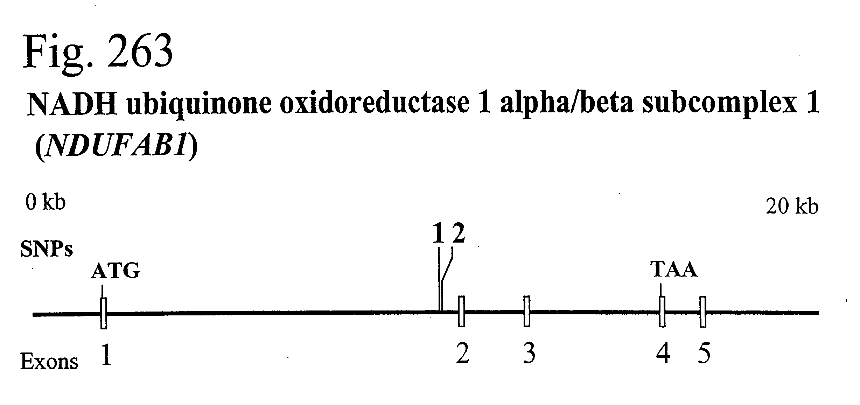

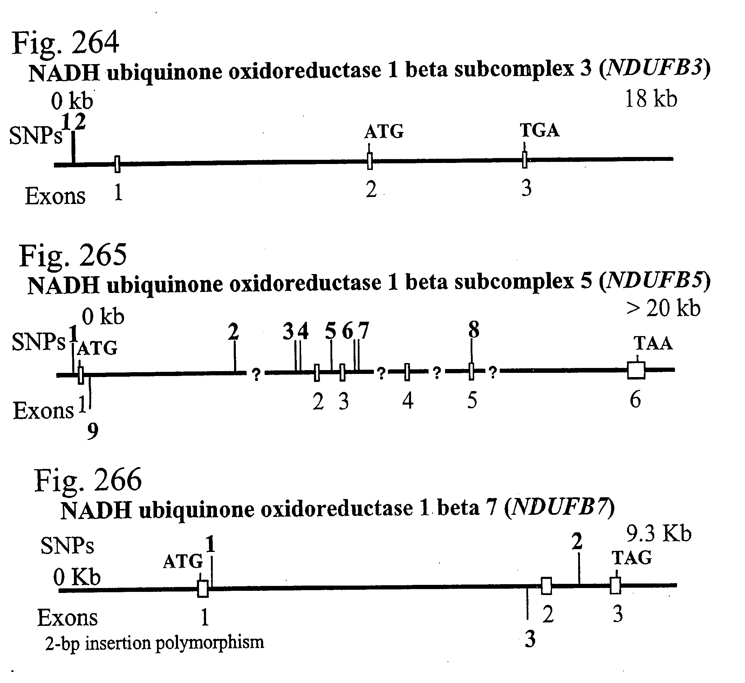

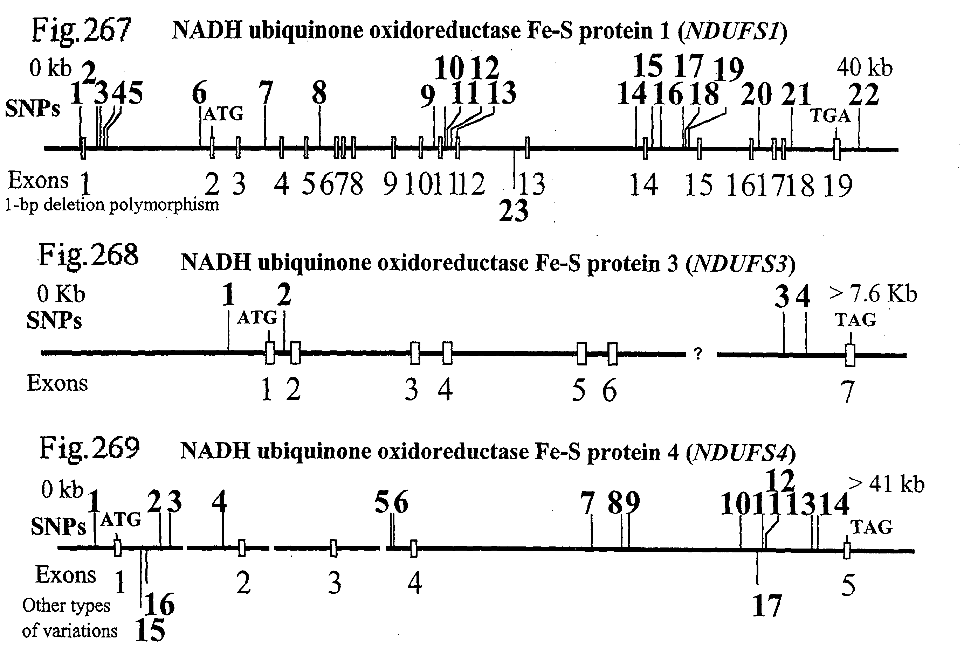

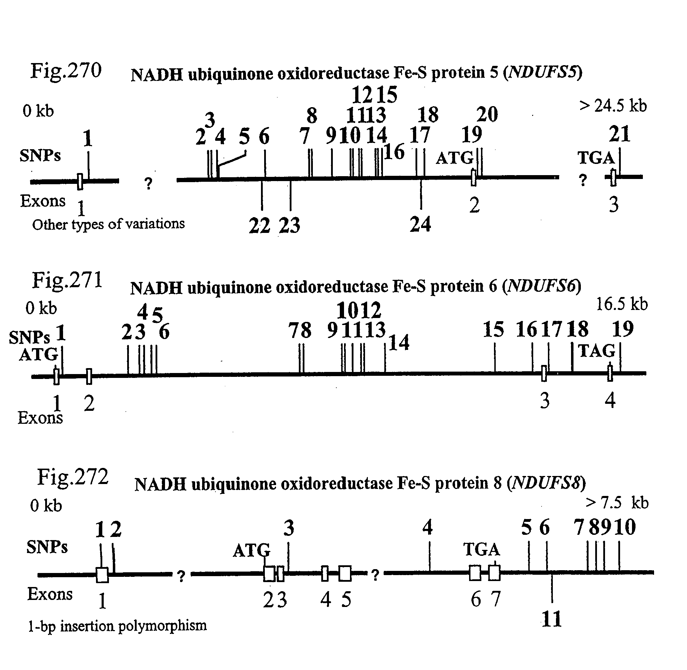

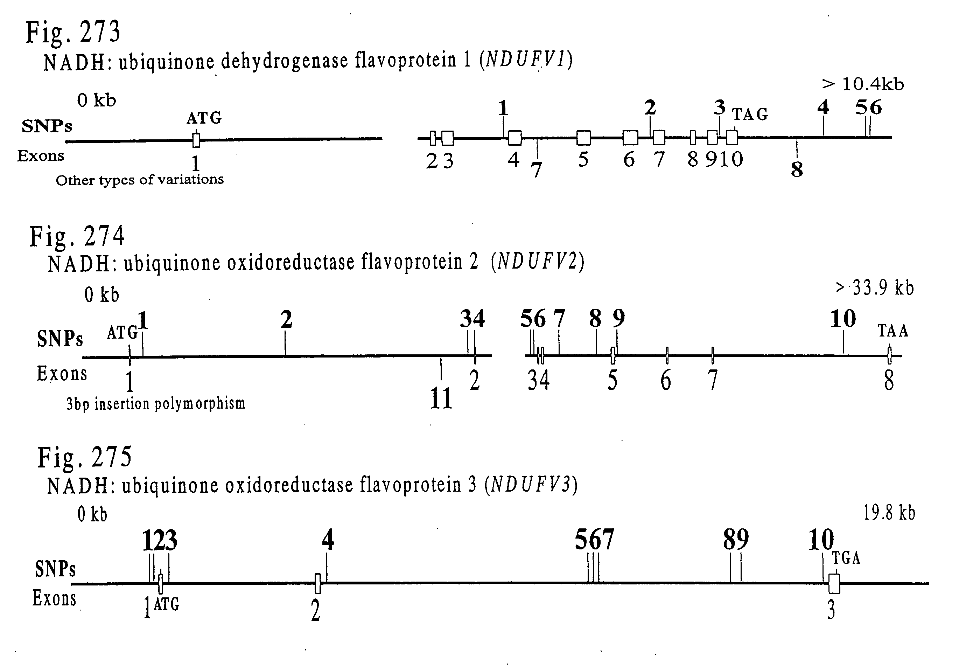

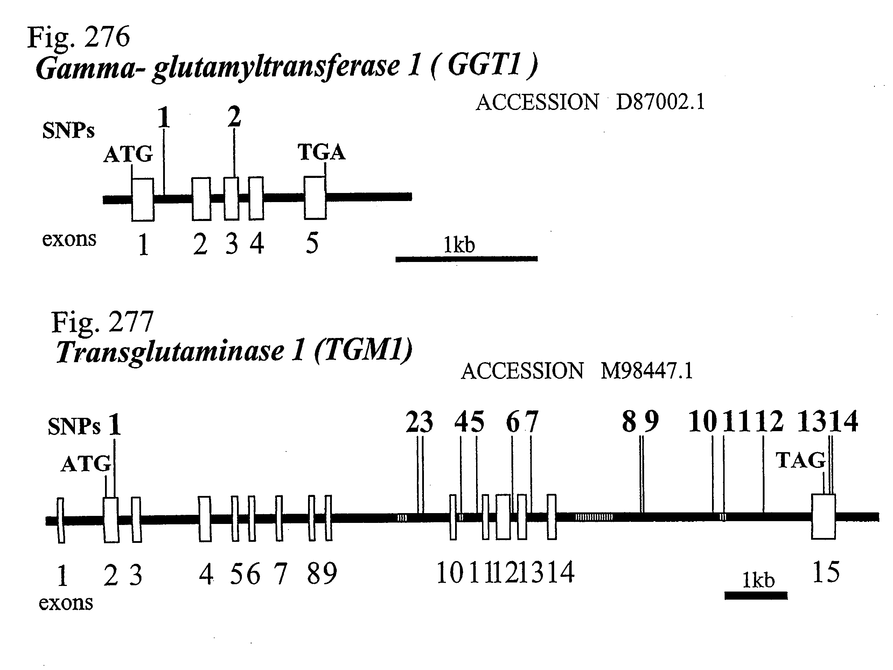

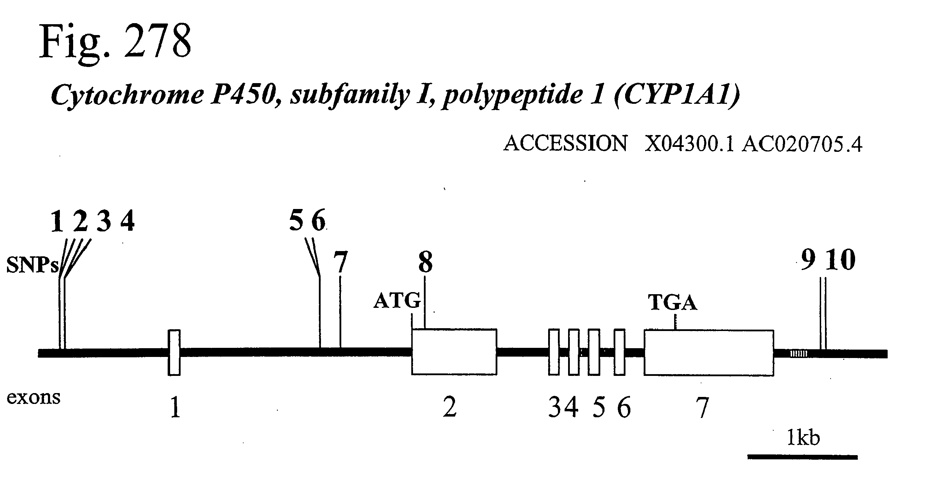

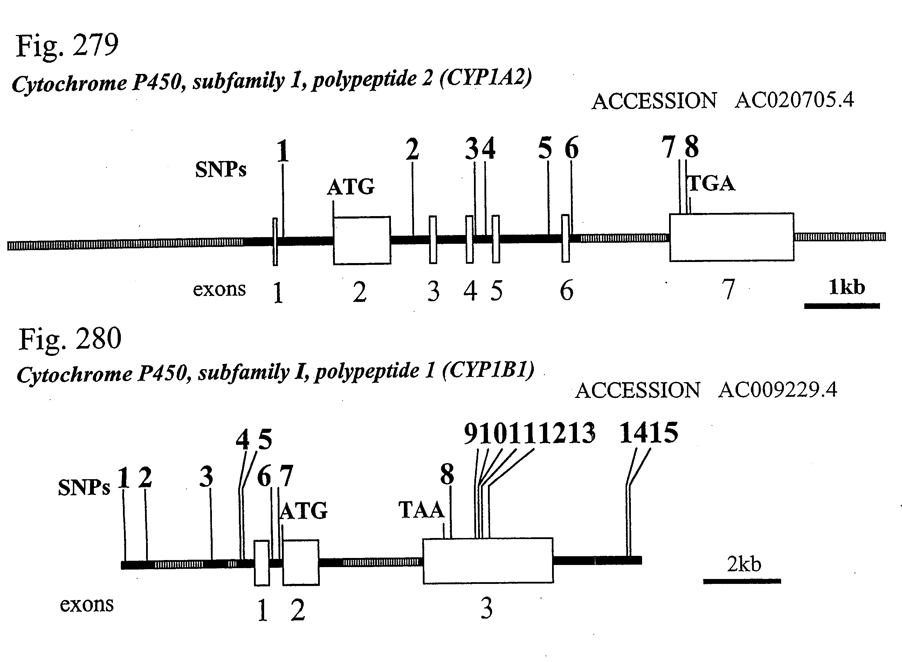

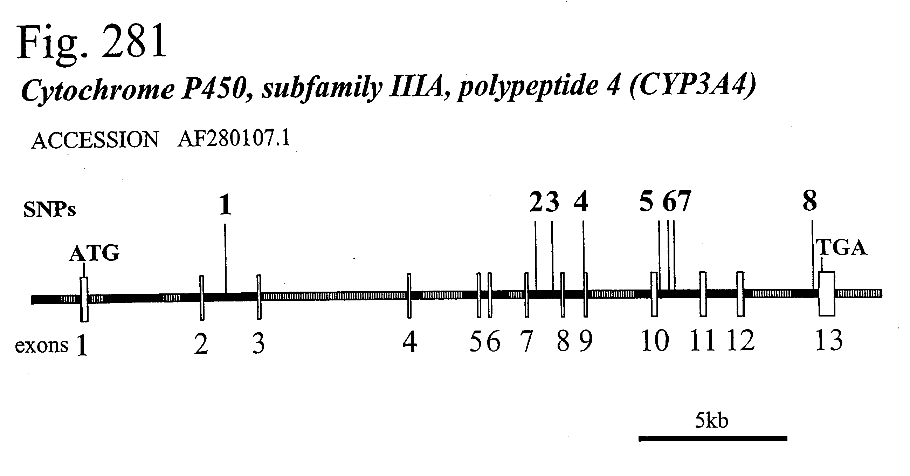

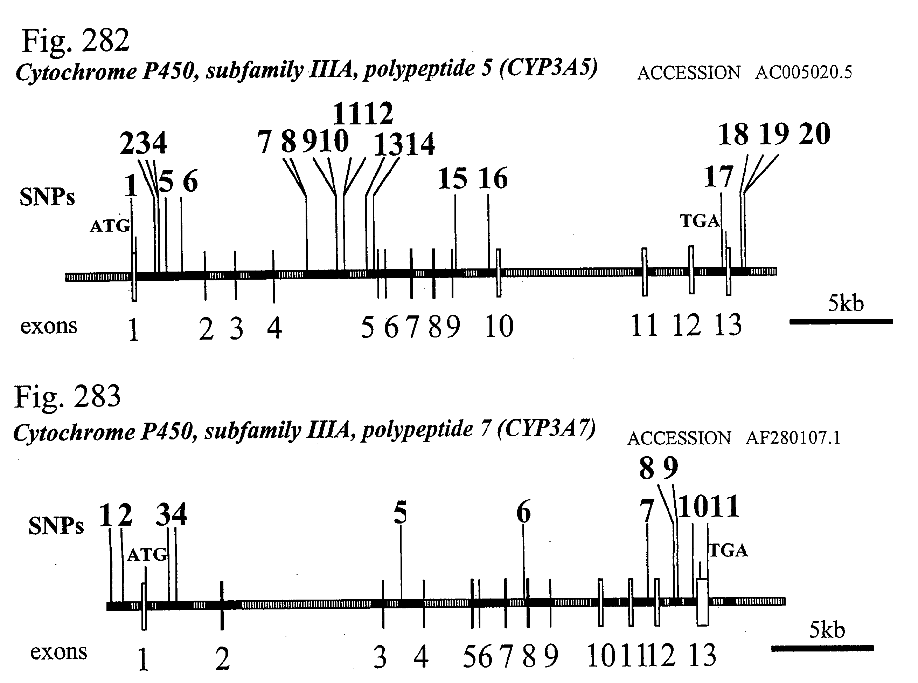

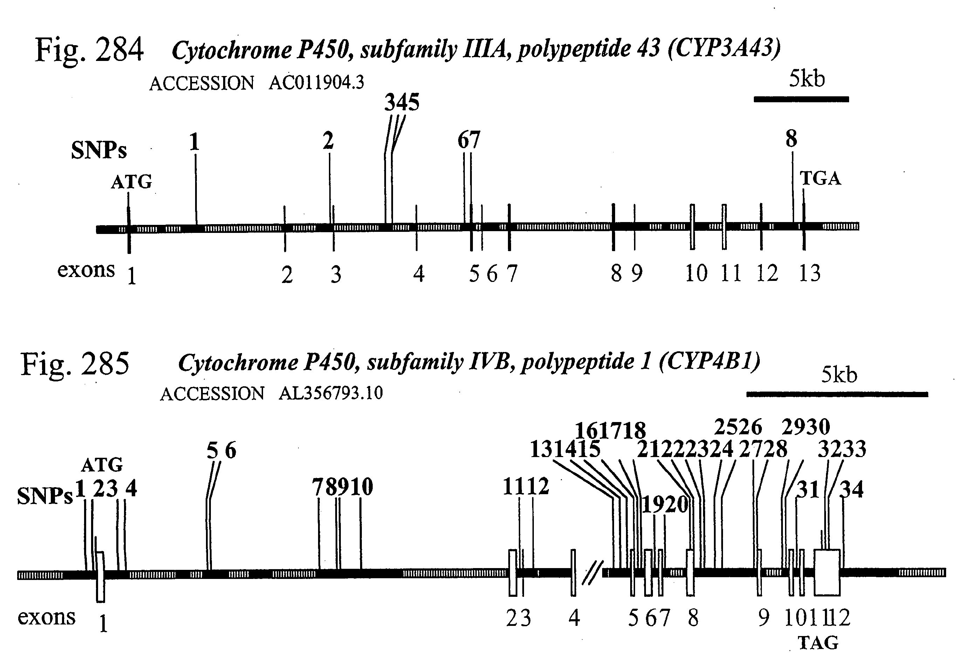

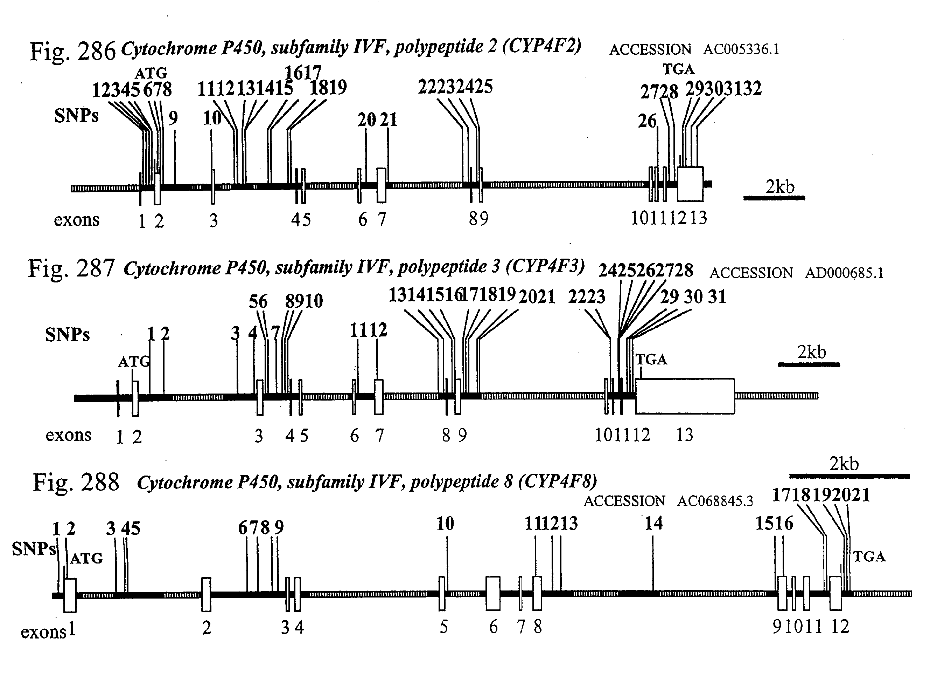

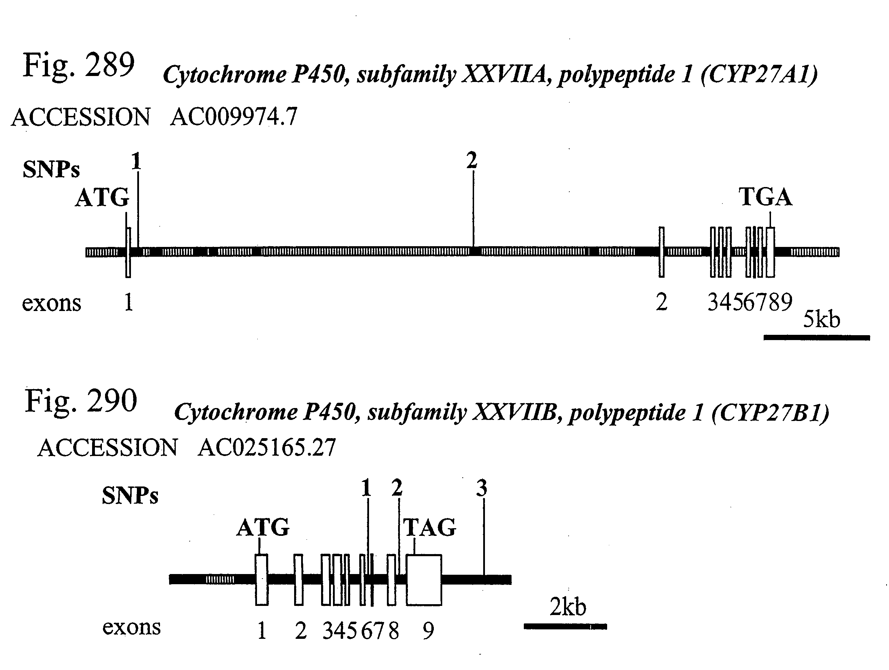

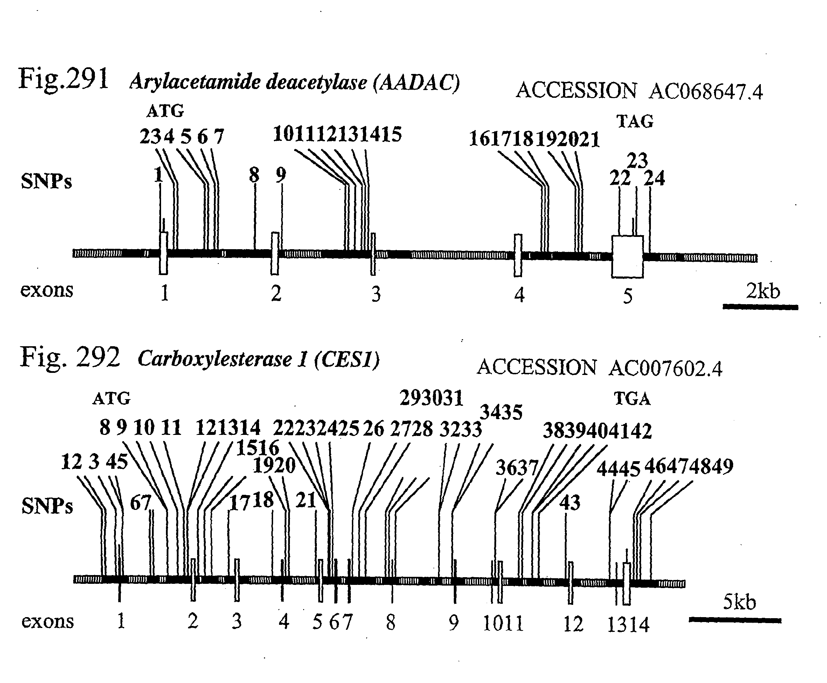

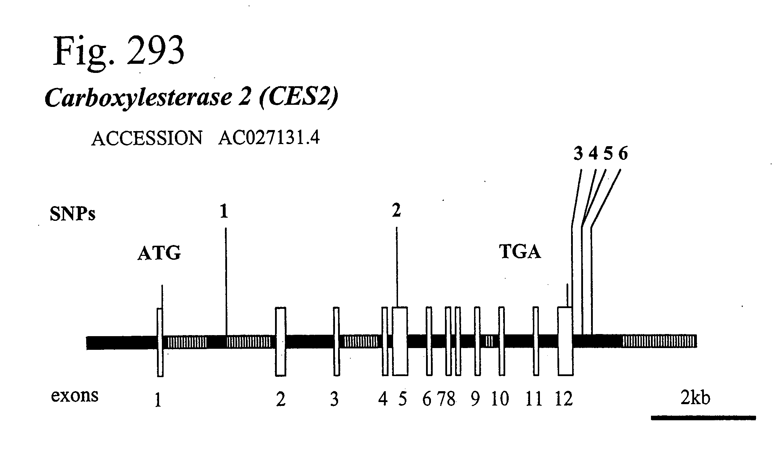

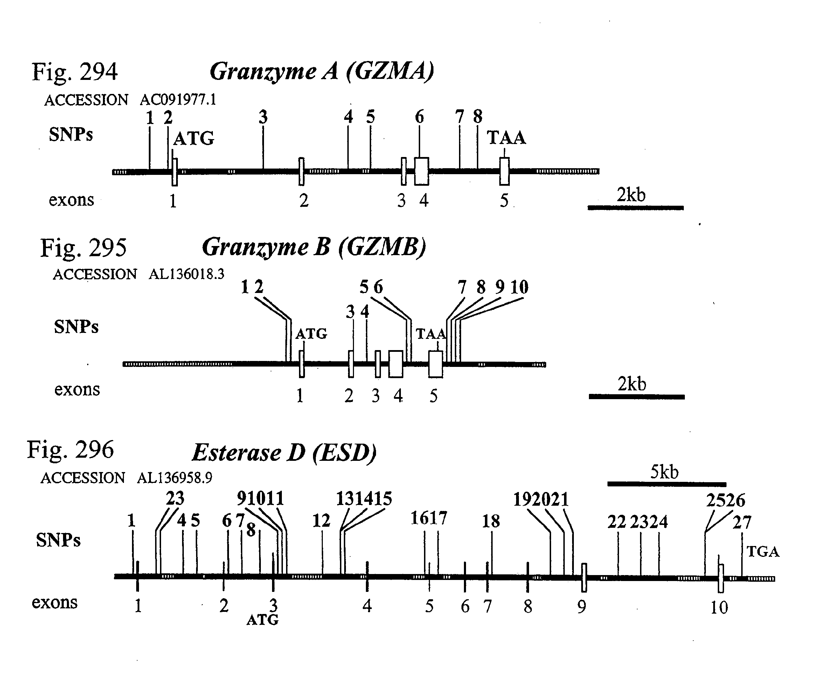

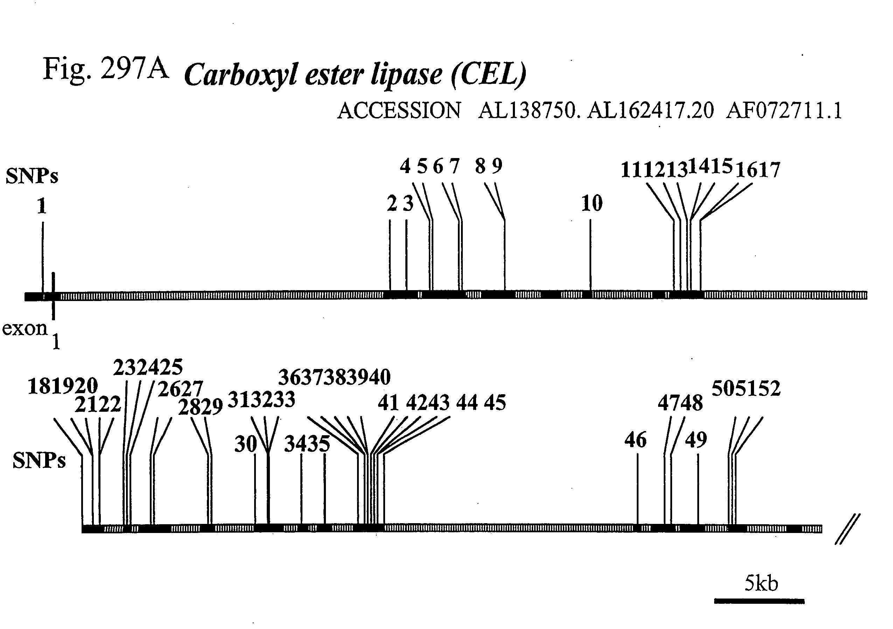

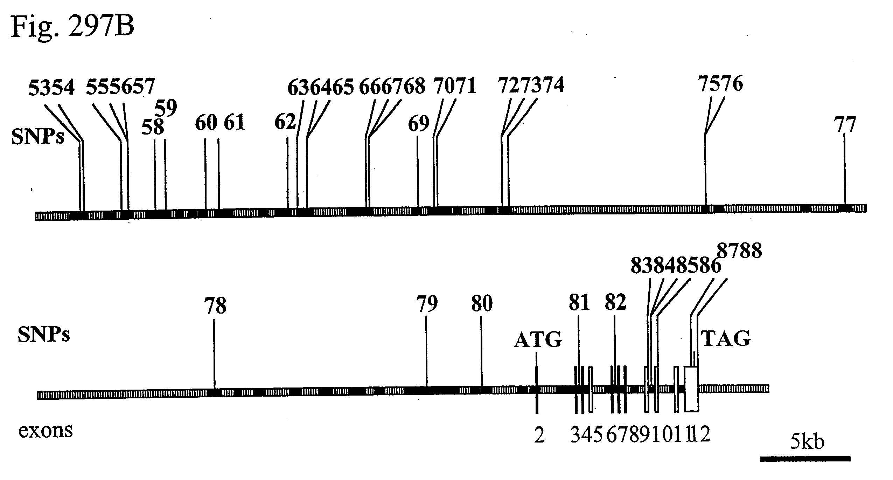

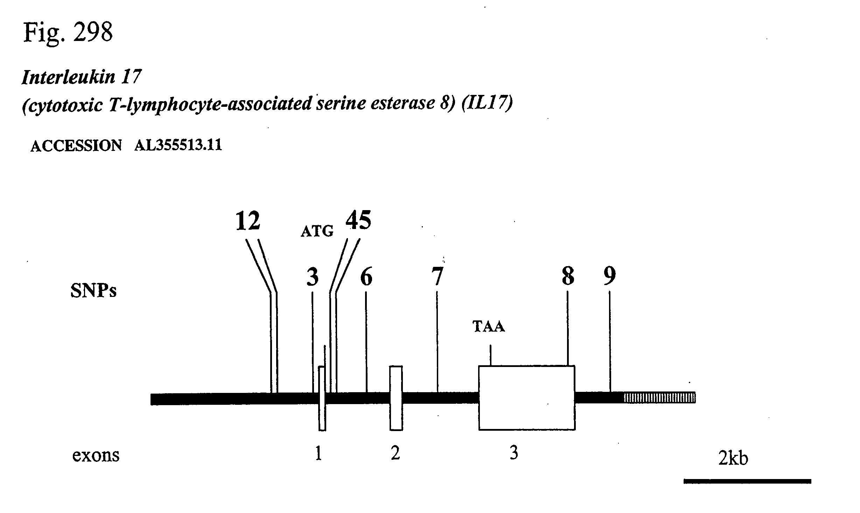

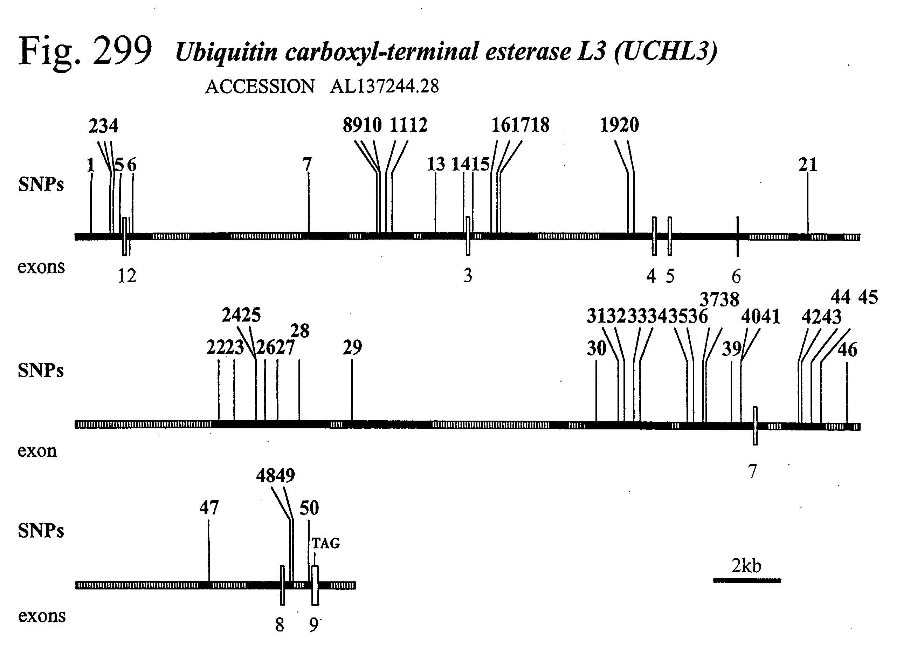

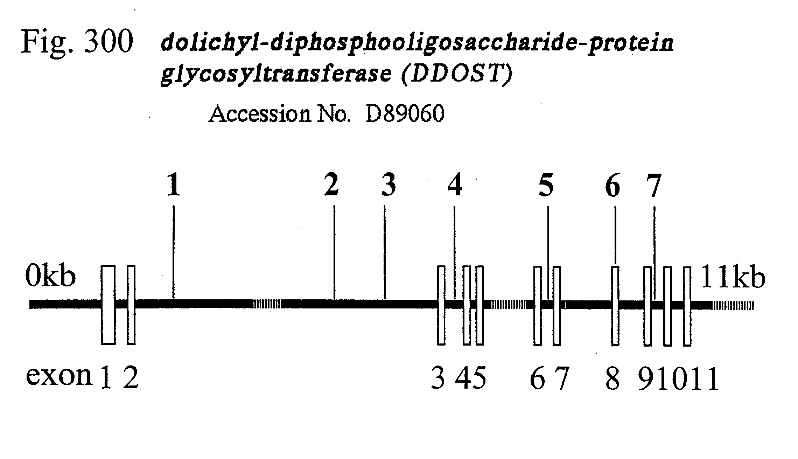

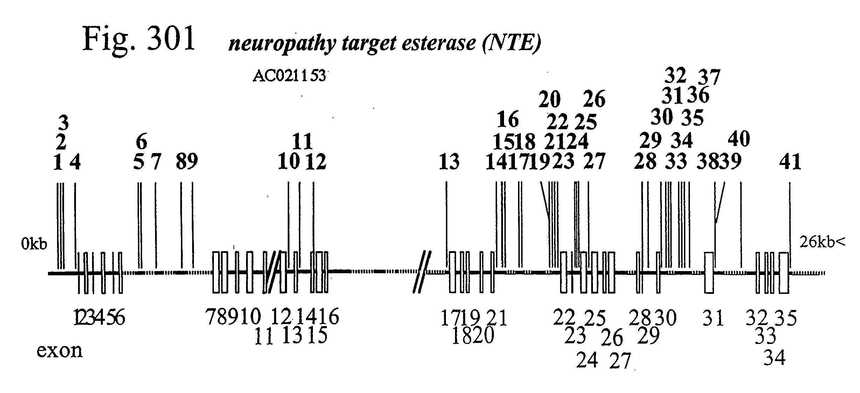

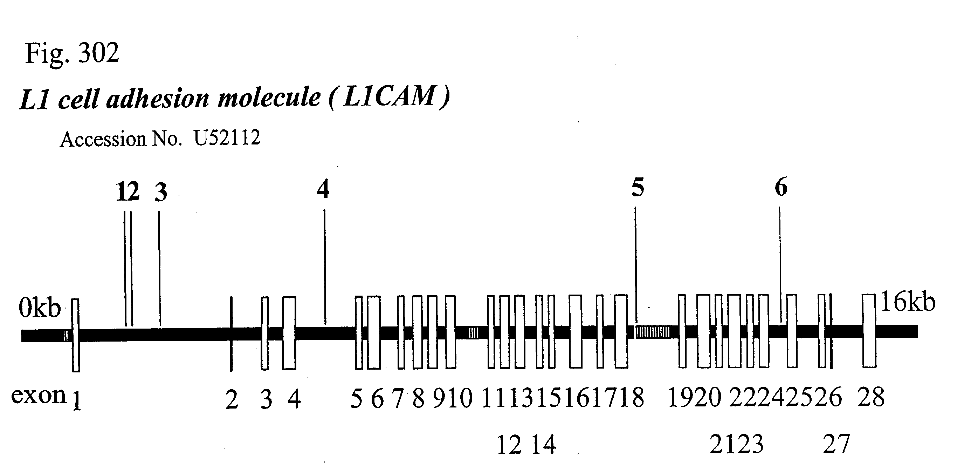

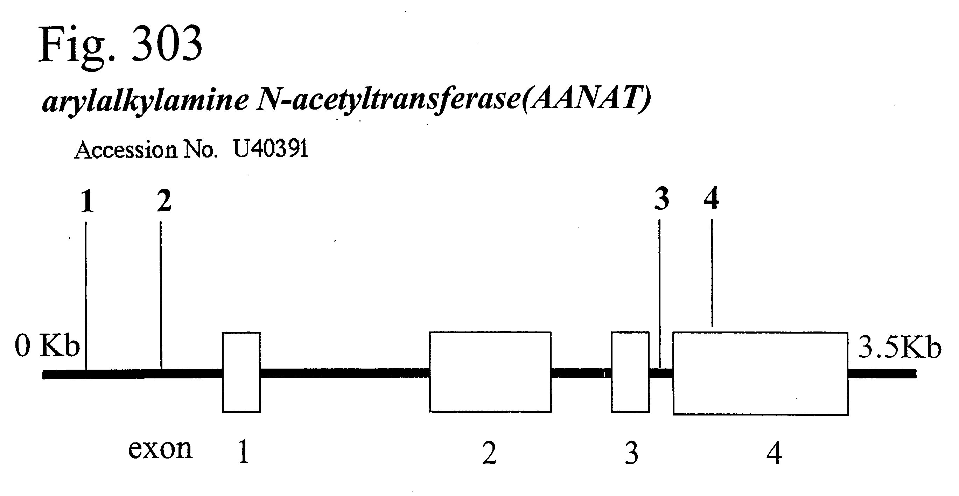

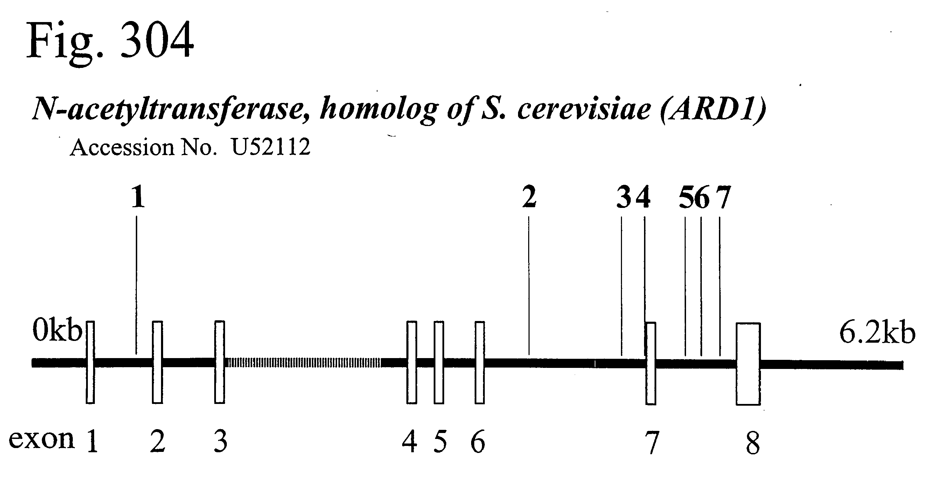

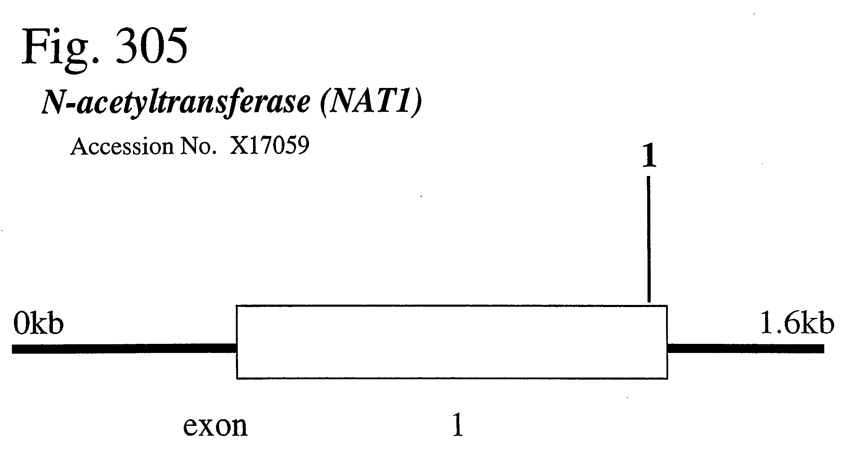

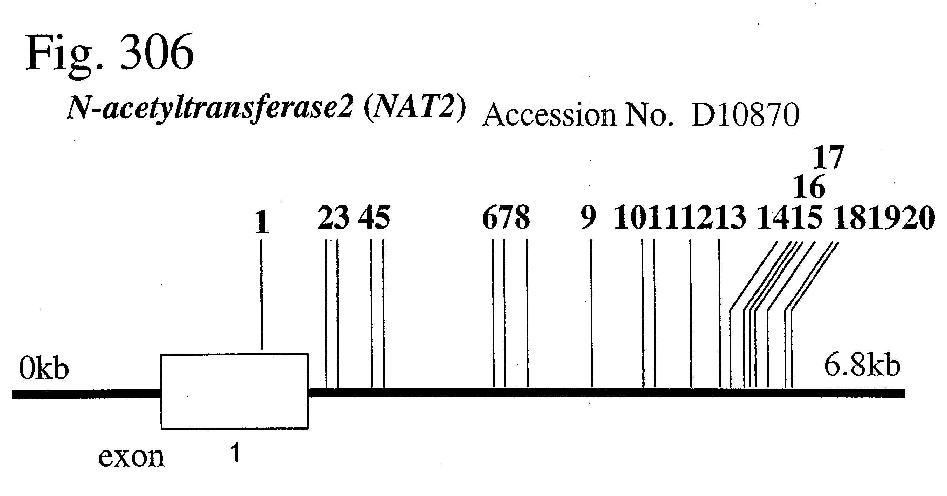

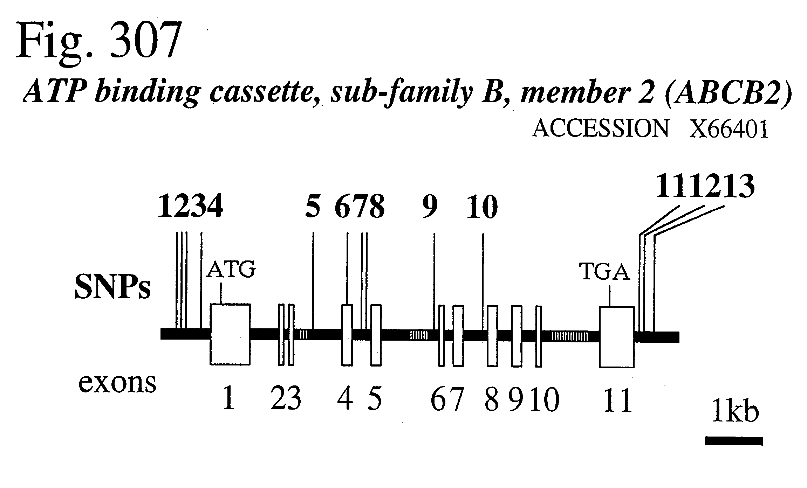

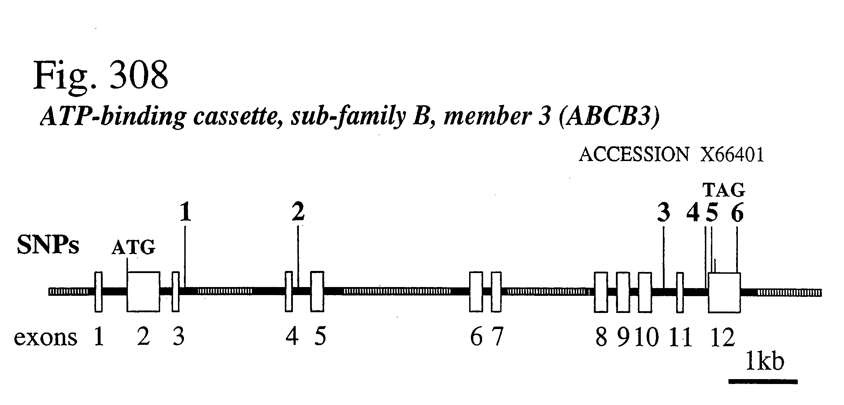

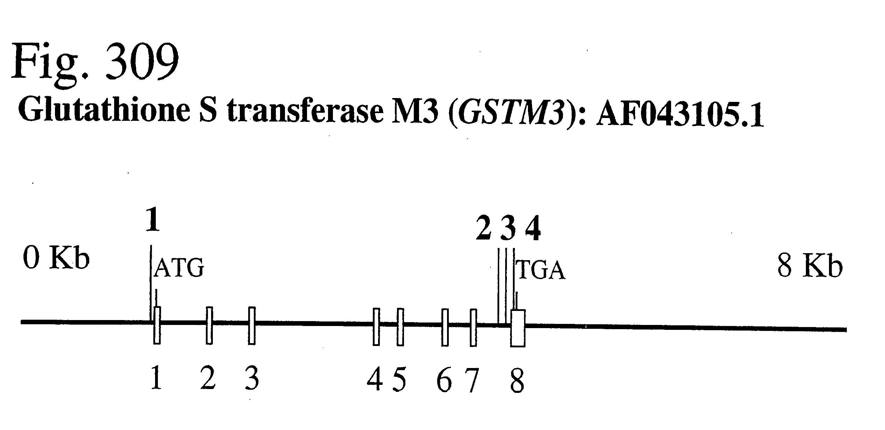

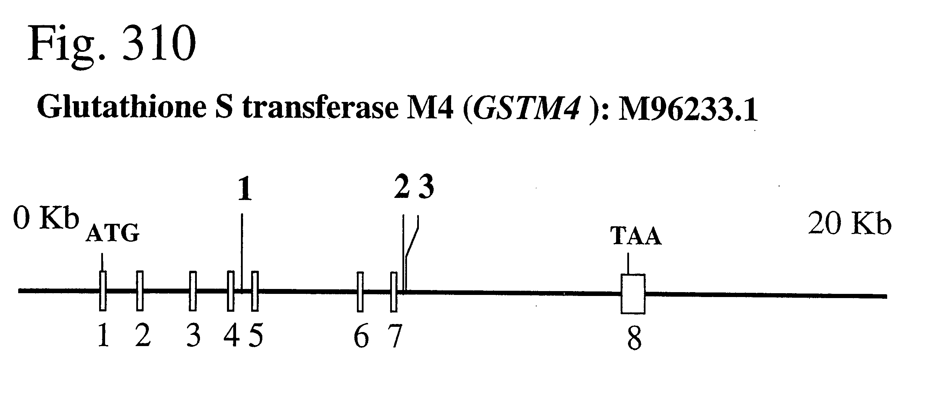

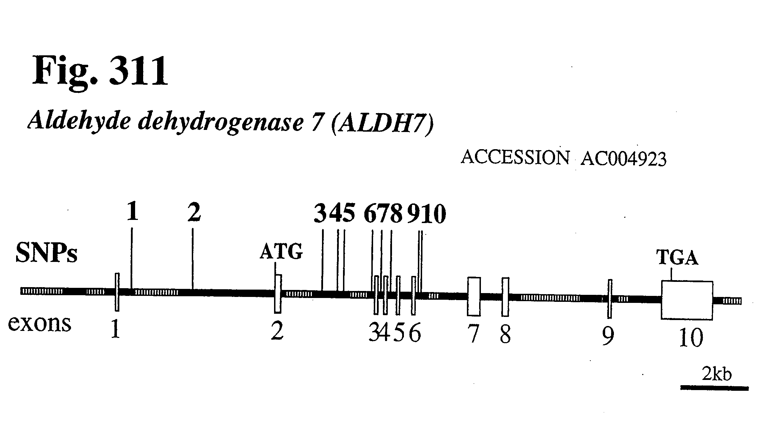

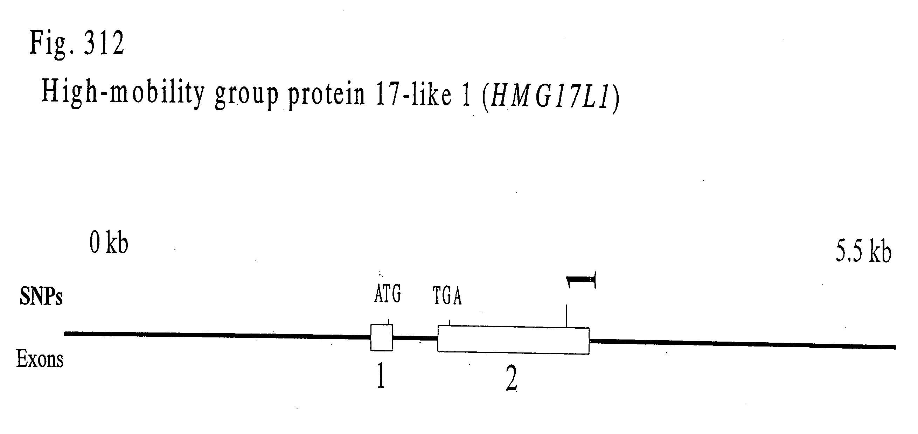

Here, "position" indicates the position of the SNP genome. The position of SNPs in the 5' flanking region, intron region and 3' flanking region are intron base sequences counted as a single number starting at the exon-intron junction. The position of SNPs in the exon region are exon base sequences counted as a single number starting at the exon-intron junction. Also, (+) or no symbol indicates a number counted in the 3' upstream direction and (-) indicates a number counted in the 5' downstream direction. The number in the "number" column indicates the position of the SNP in the gene maps of the various genes (FIG 9 through FIG 141 and FIG 144 through 312).

The sequence represented by the SEQ ID Nos. 1-3360 and 3361-7669 can readily be associated with the corresponding gene, chromosome, and chromosomal position. Each of the genes shown in Table 1 correlates to a corresponding Figure in the present application. The Figures show a map of the gene with positional identifiers for each of the polymorphisms. The Figures also provide an accession number that correlates to public genome databases, allowing the genetic context of the polymorphism and the gene to be understood. Using the information in Table 1, the Figures, and public genome databases, one skilled in the art is able to identify flanking sequences. This allows, for example, the development of PCR primers that flank the polymorphism. Considerations for PCR primer design are known in the art for both single PCR reactions and multiplex reactions (See e.g., Henegariu et al., BioTechniques 23:504-511 [1997] and PCR Applications, edited by Innis, Gelfand, and Sninsky, Academic Press, San Diego, CA 1999), each of which is herein incorporated by references in its entirety). Examples of primers that find use in the amplification of sequences containing polymorphisms, as well as amplification conditions, are found at the IMS-JST JSNP database website (See, submissions from Laboratory for Genotyping, The SNP Research Center, The Institute of Physical and Chemical Research (RTKEN)).

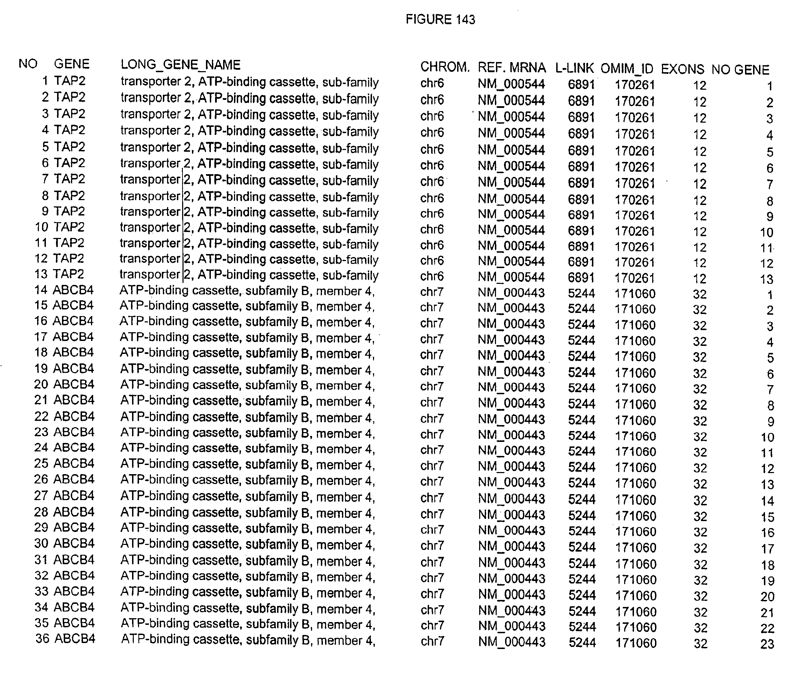

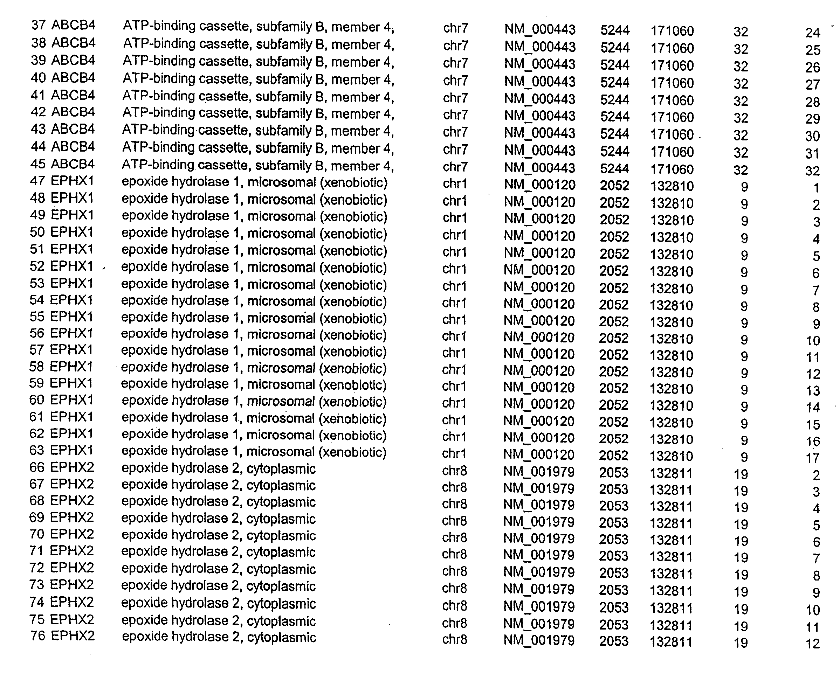

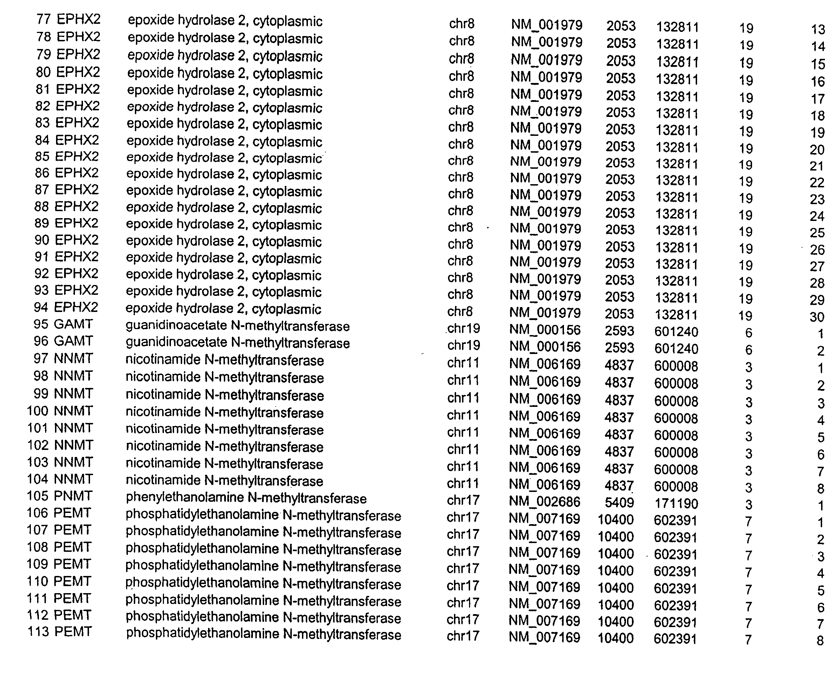

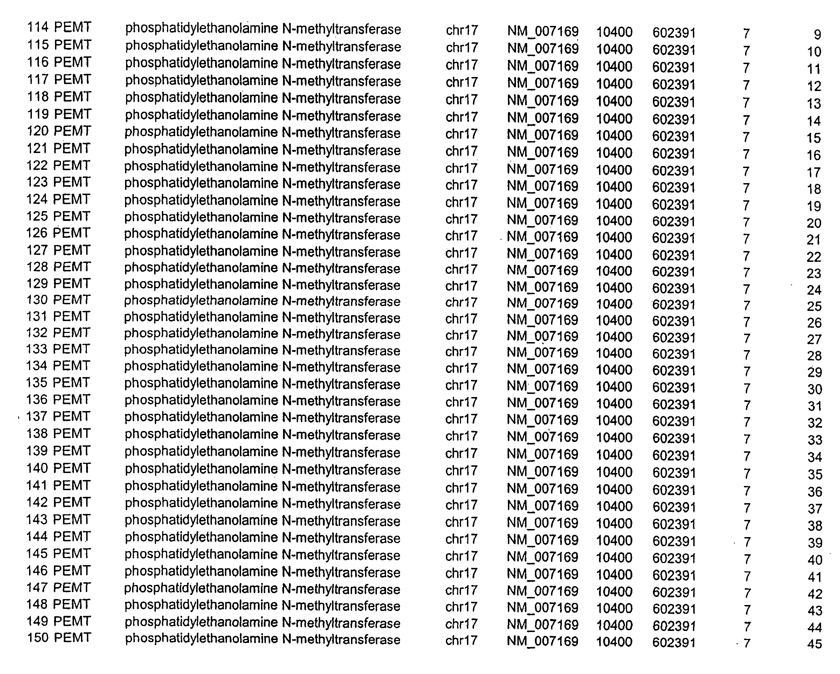

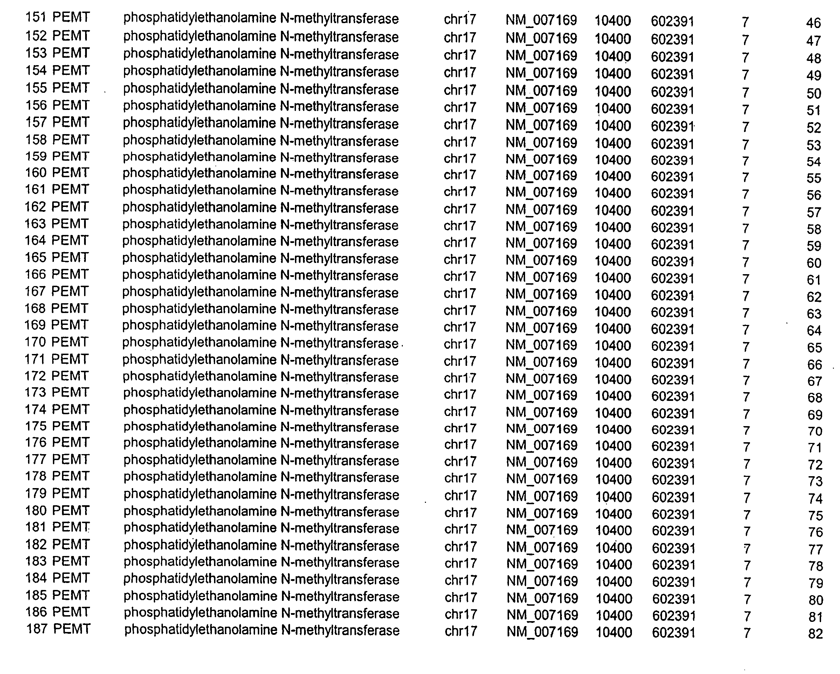

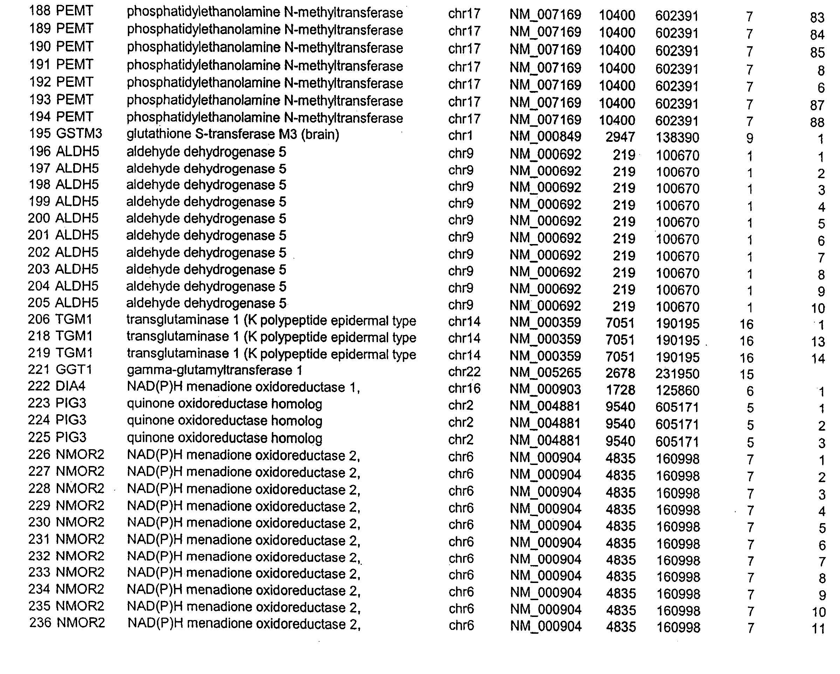

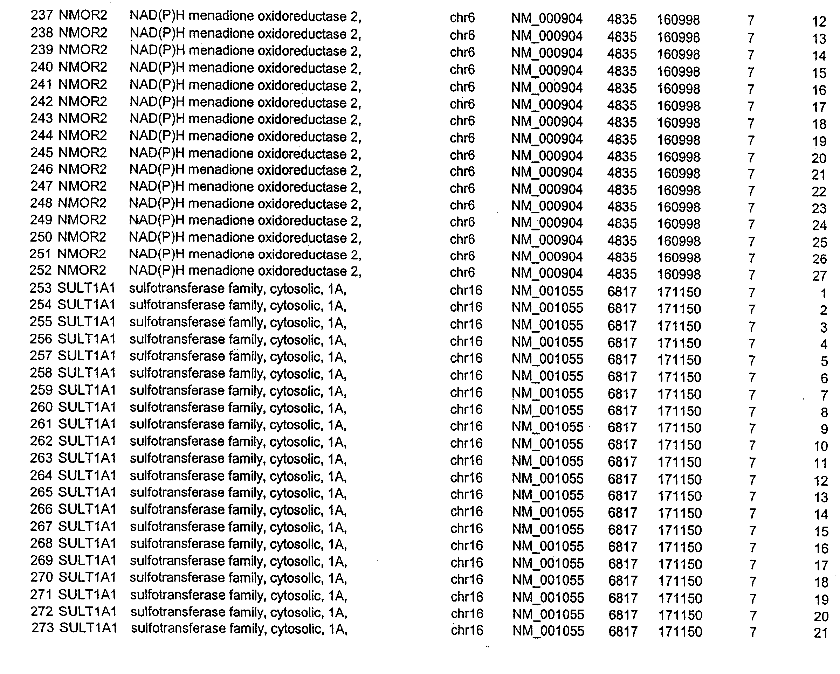

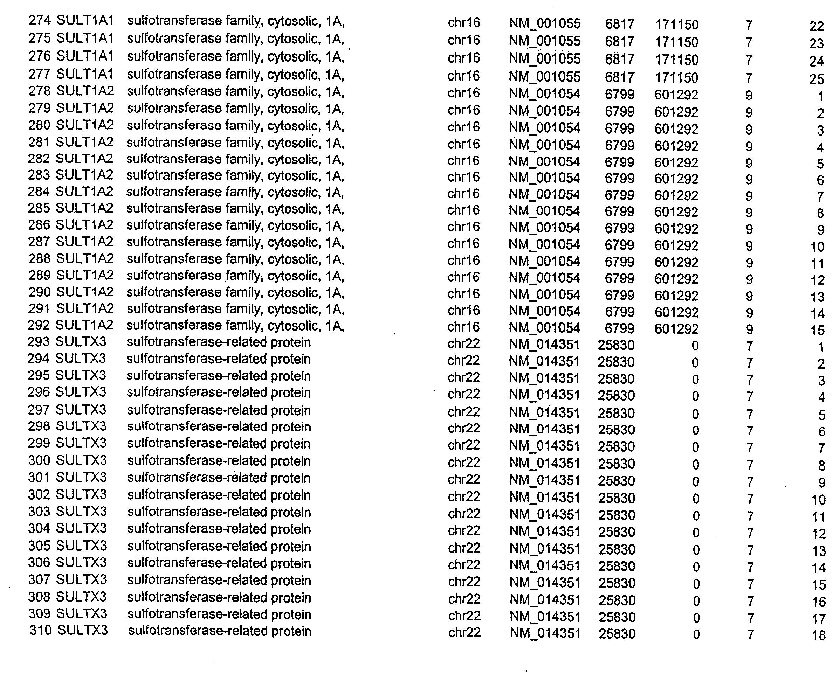

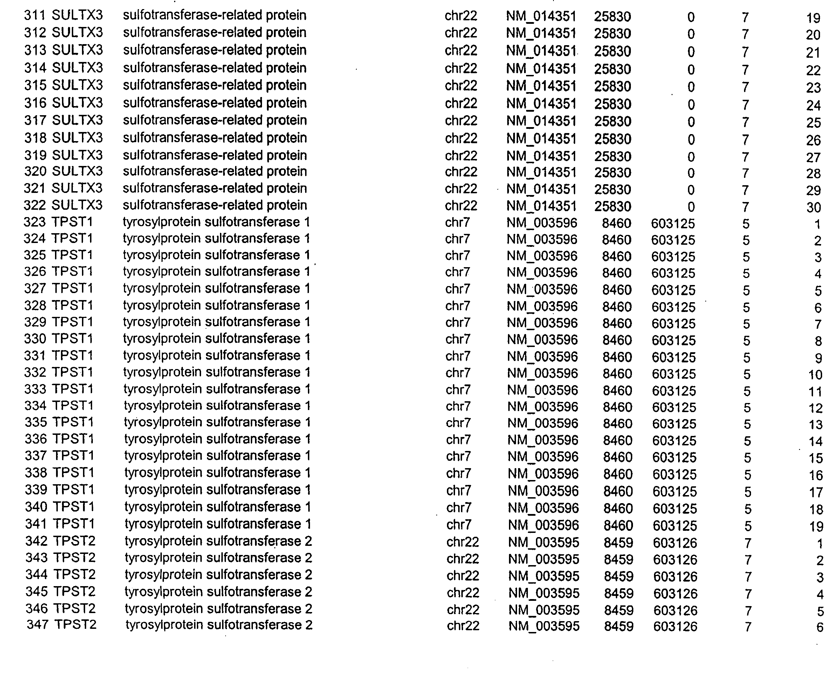

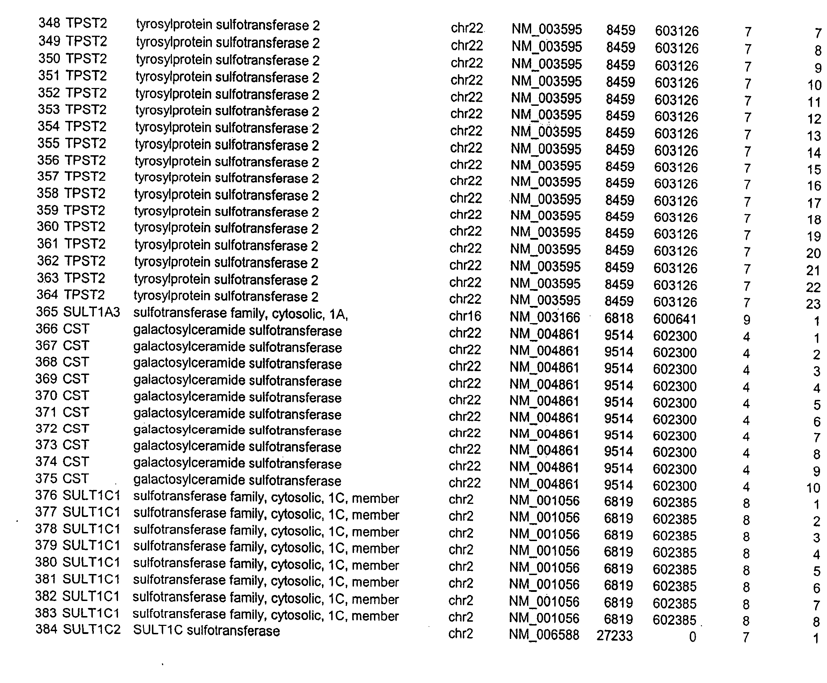

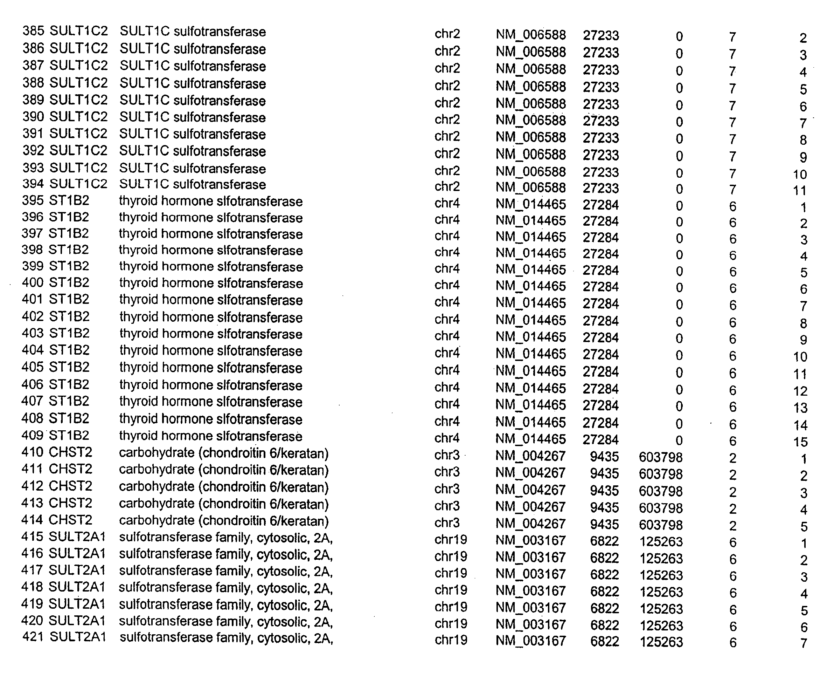

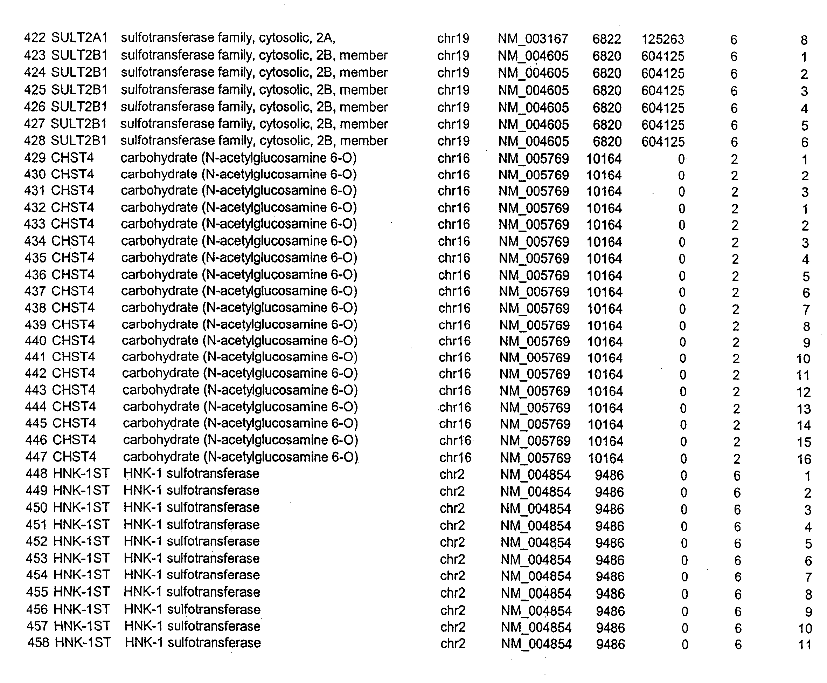

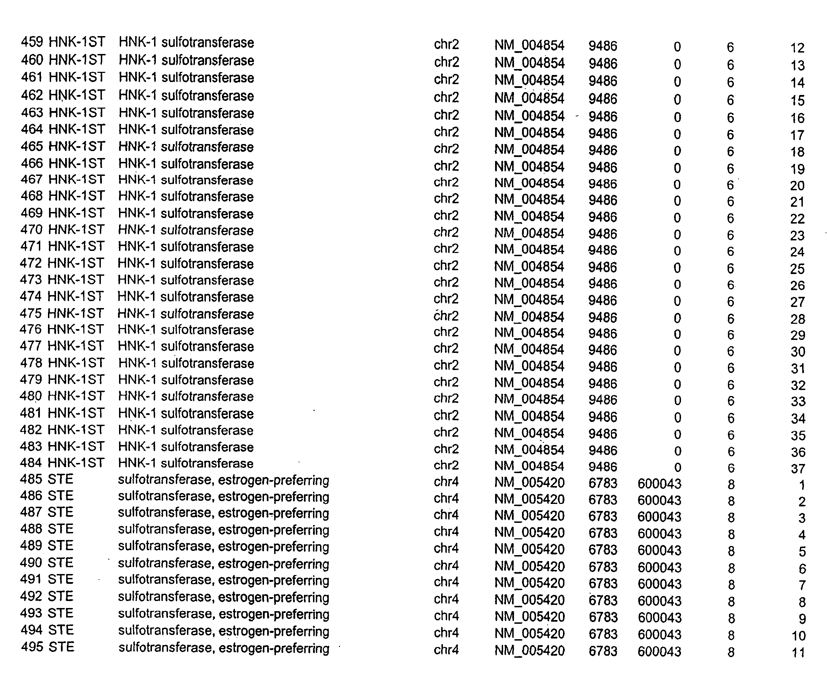

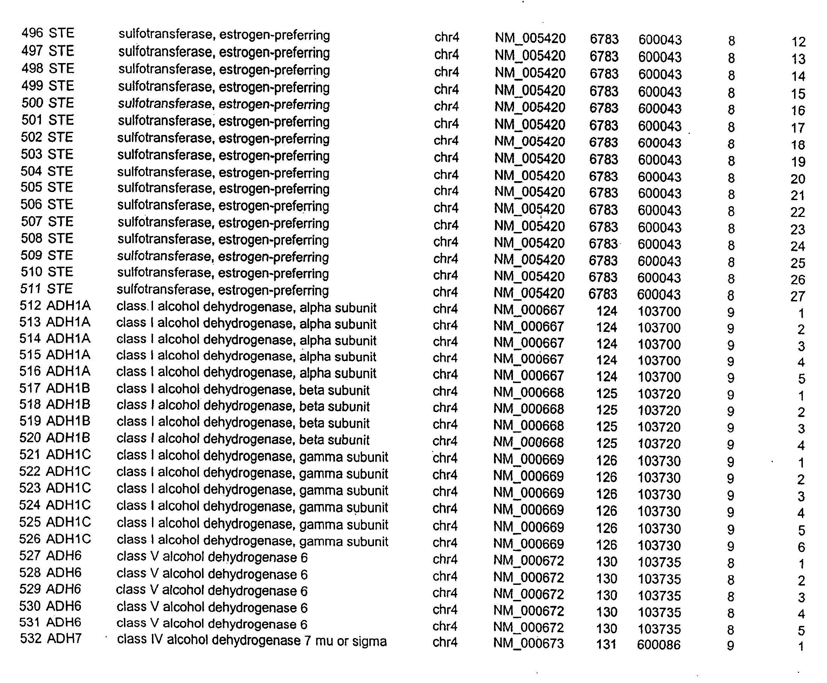

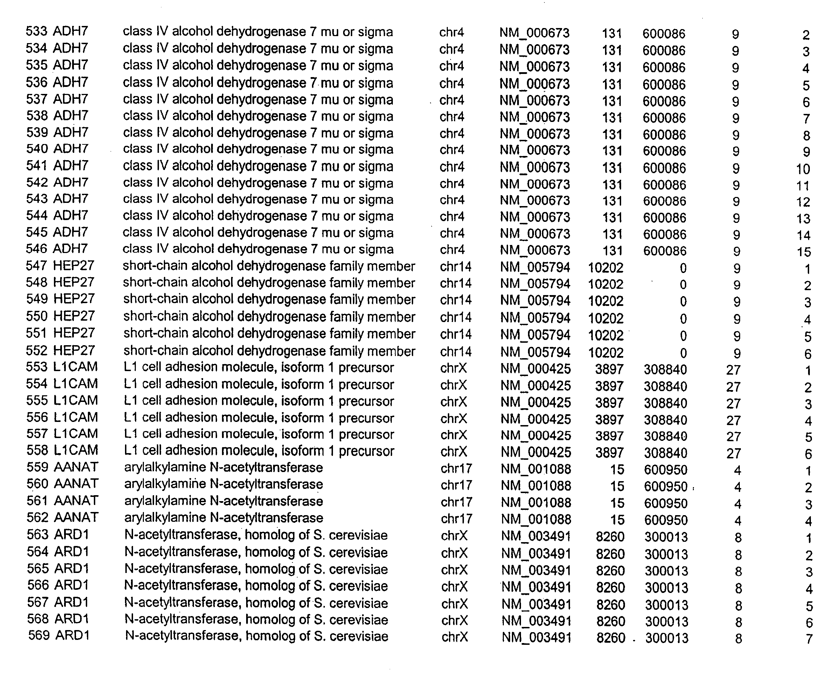

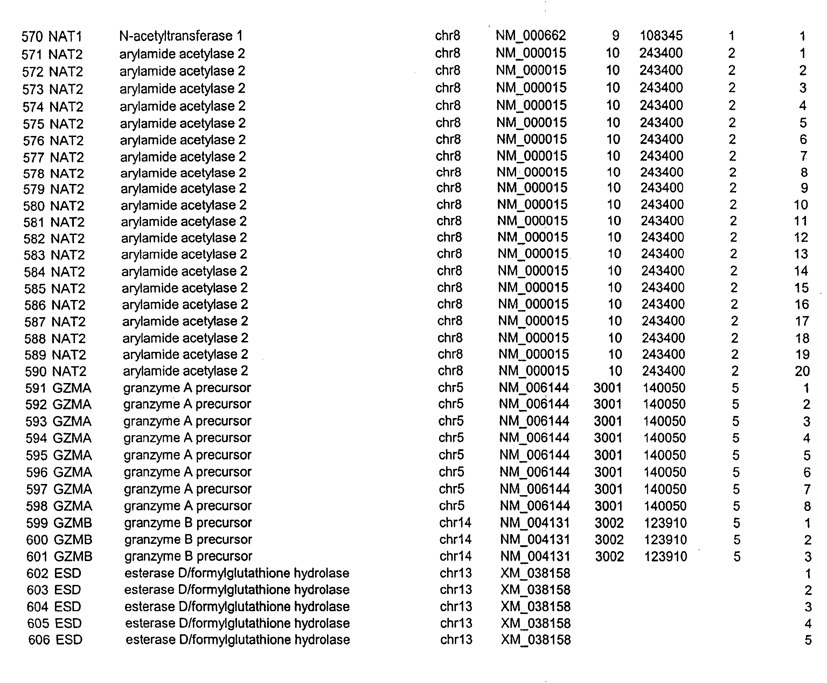

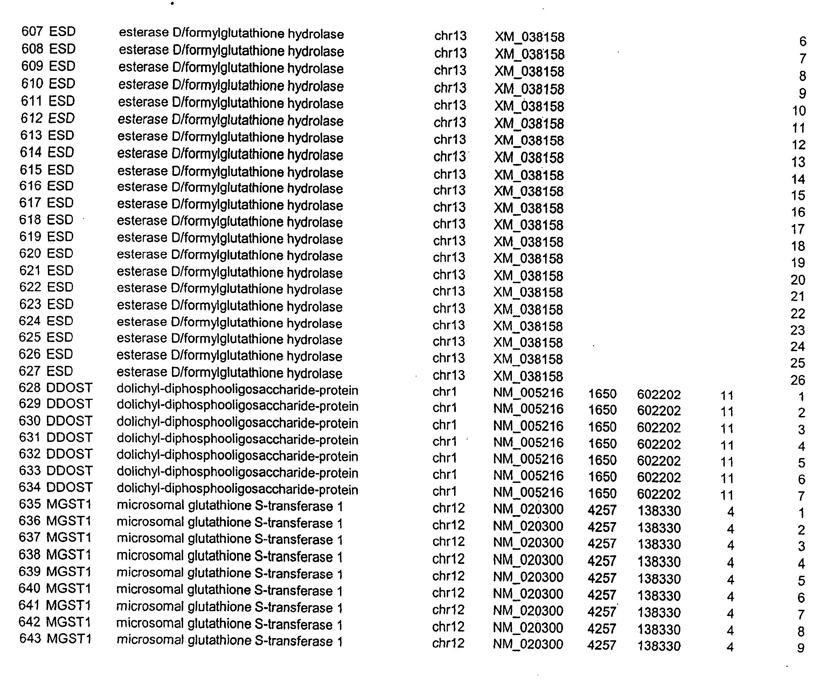

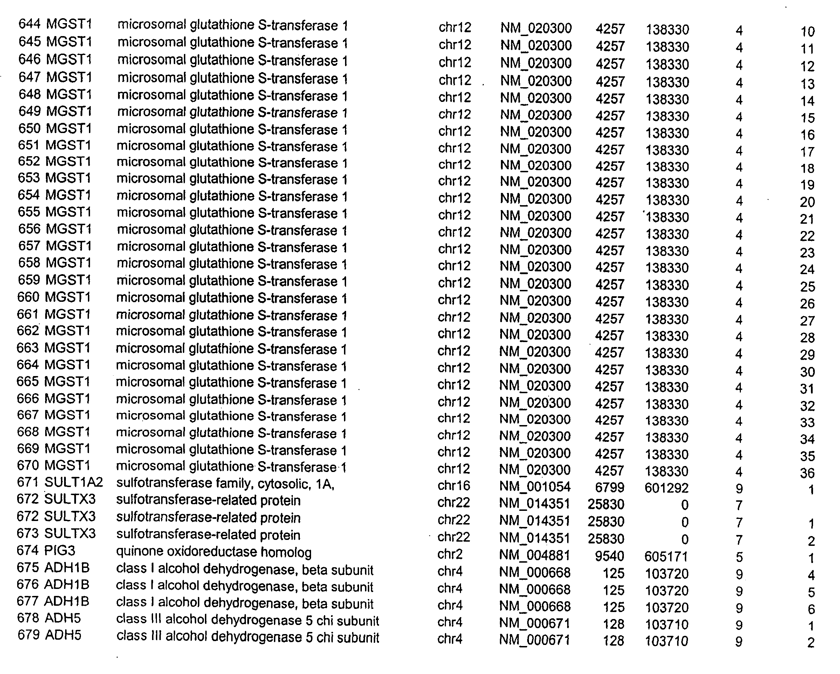

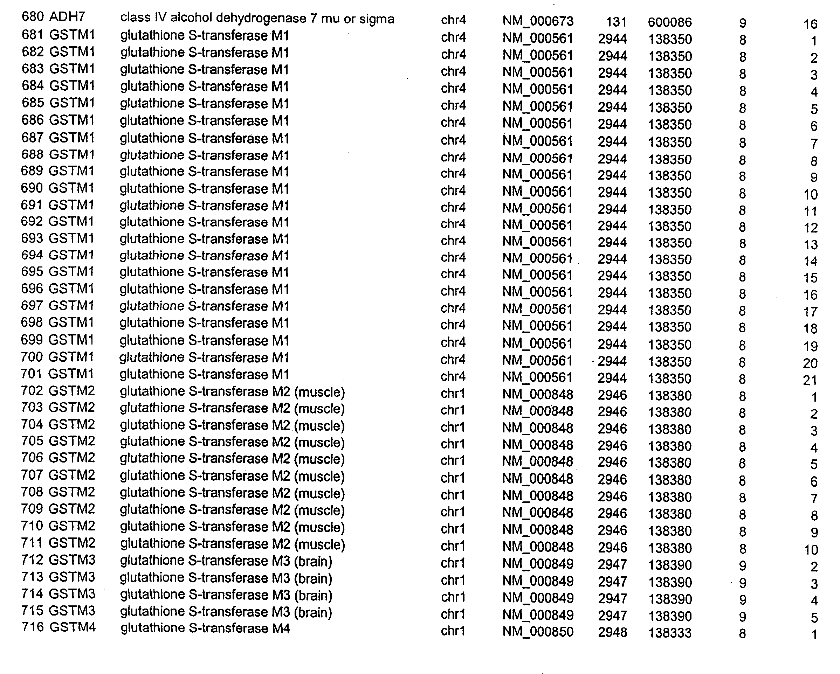

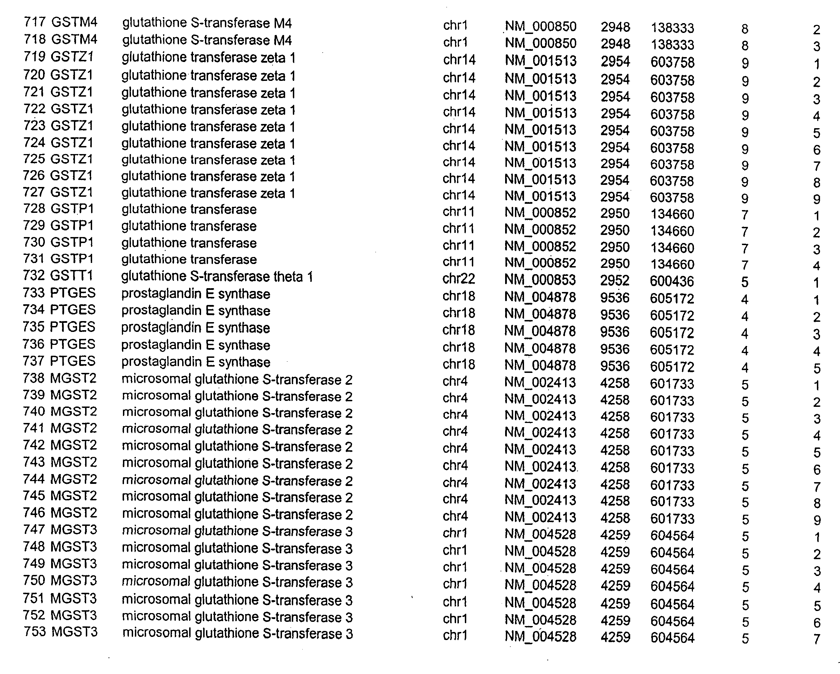

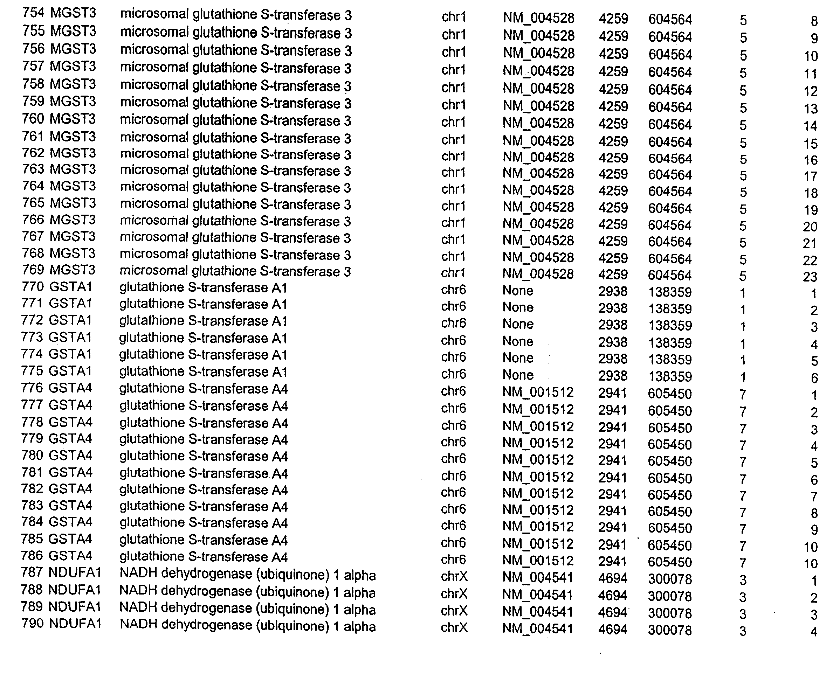

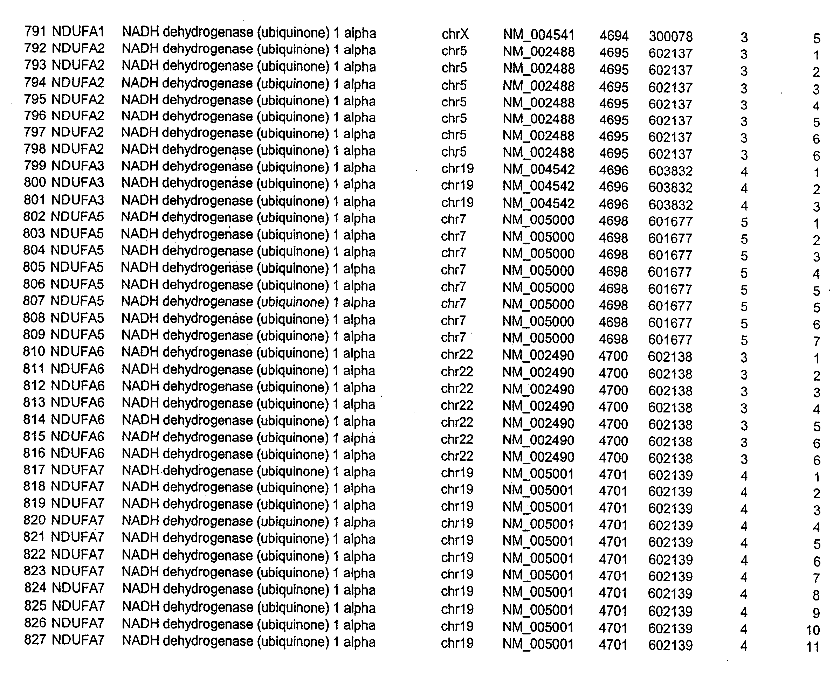

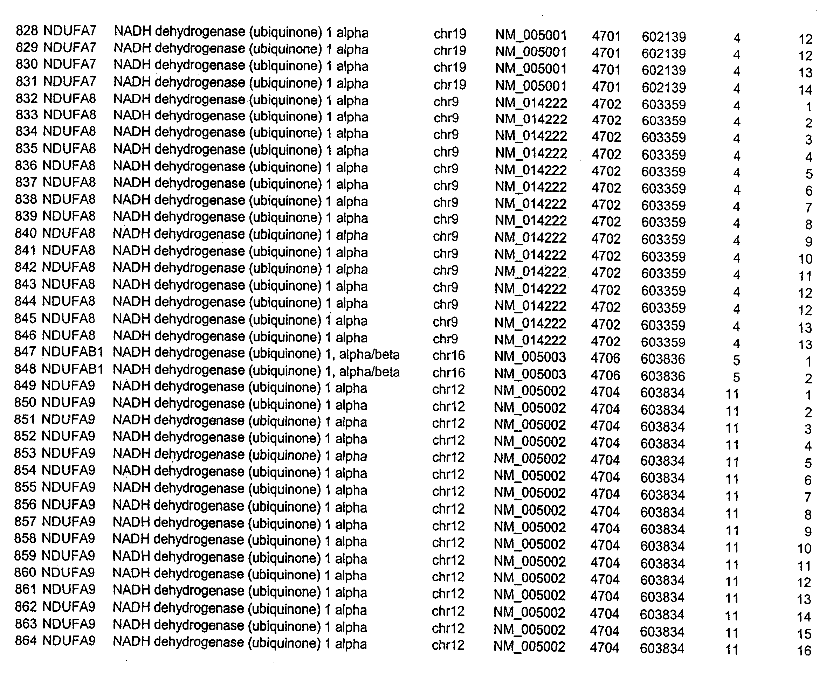

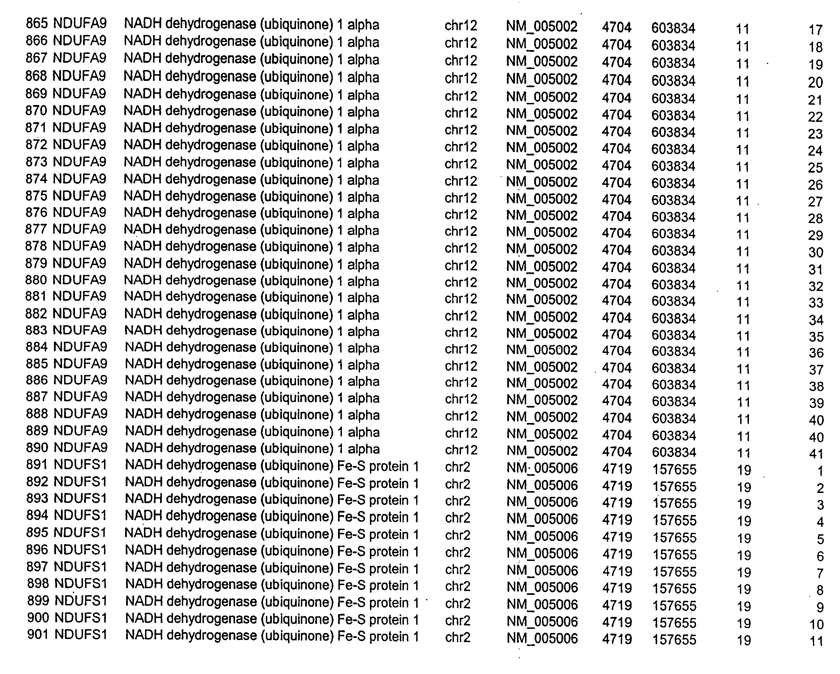

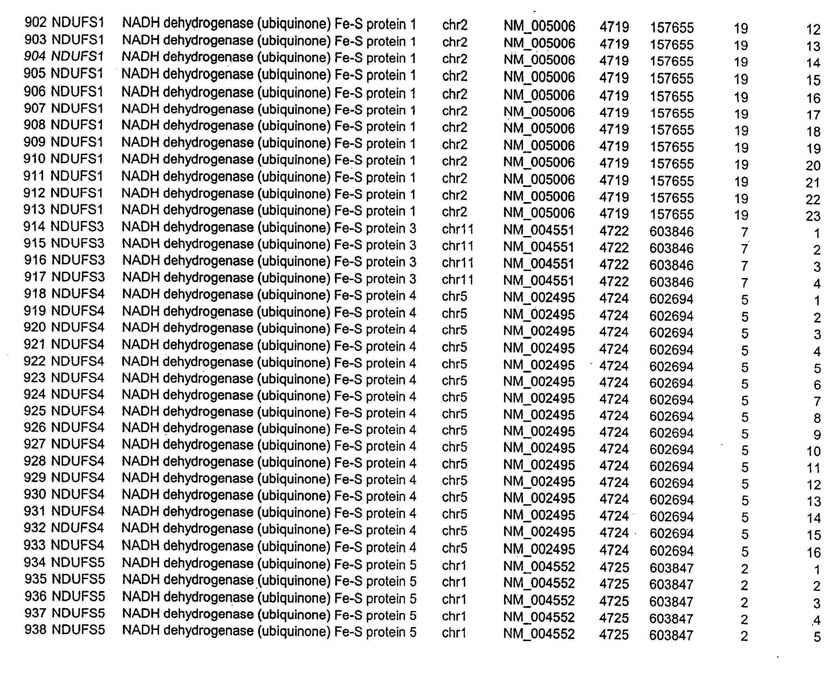

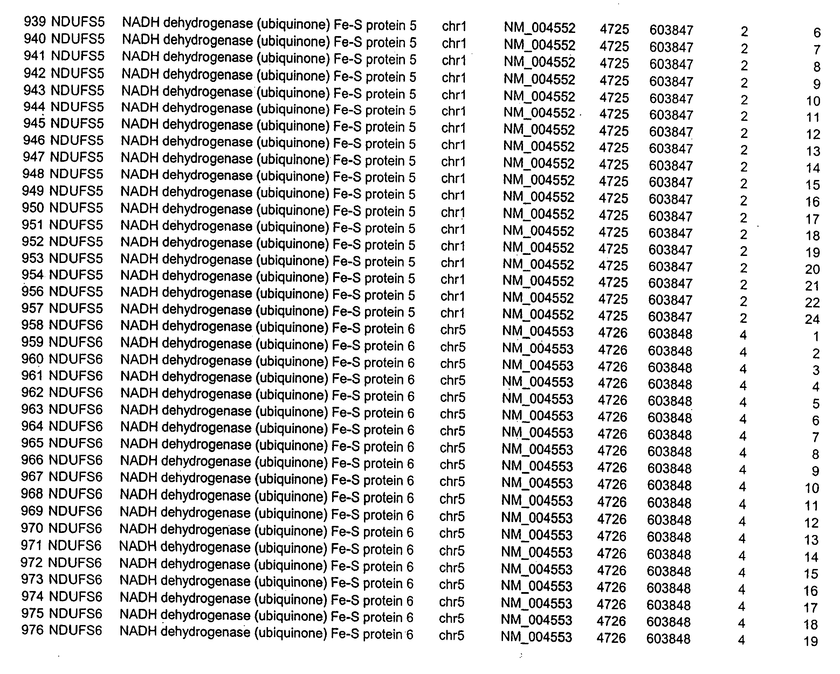

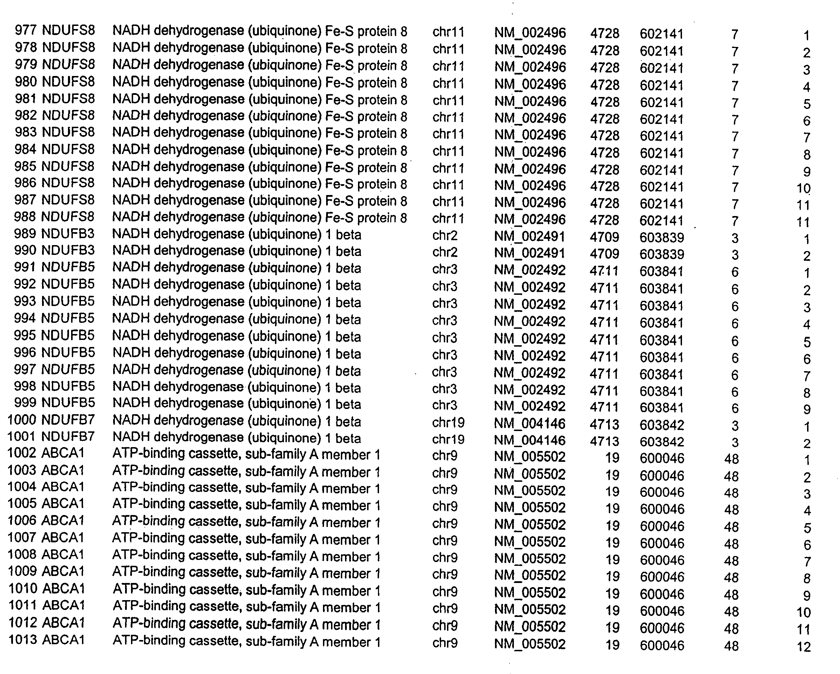

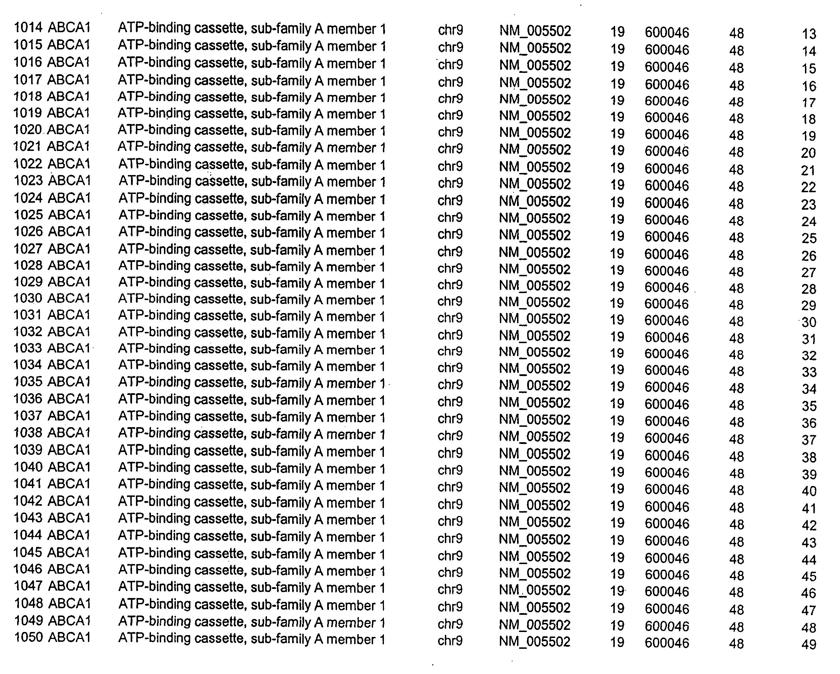

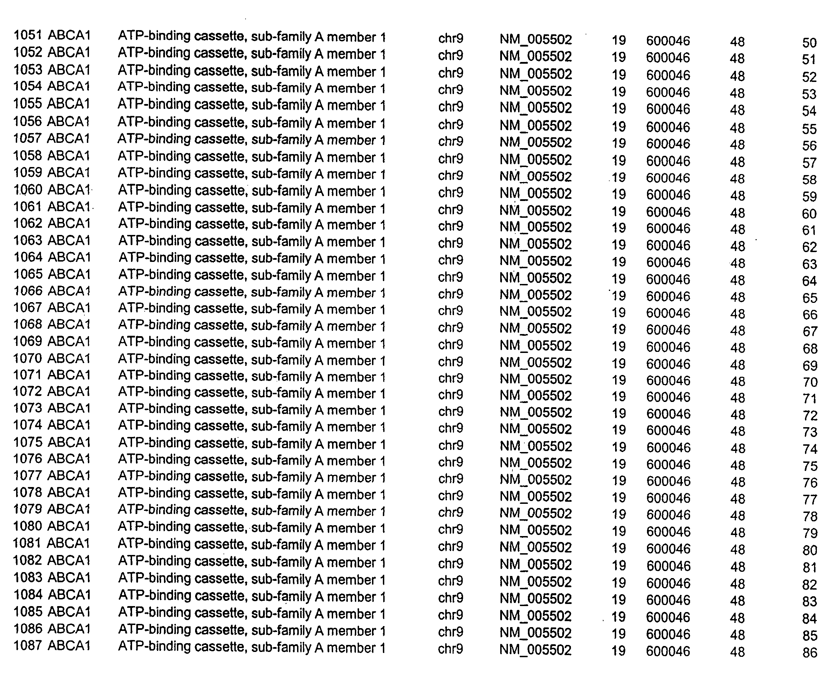

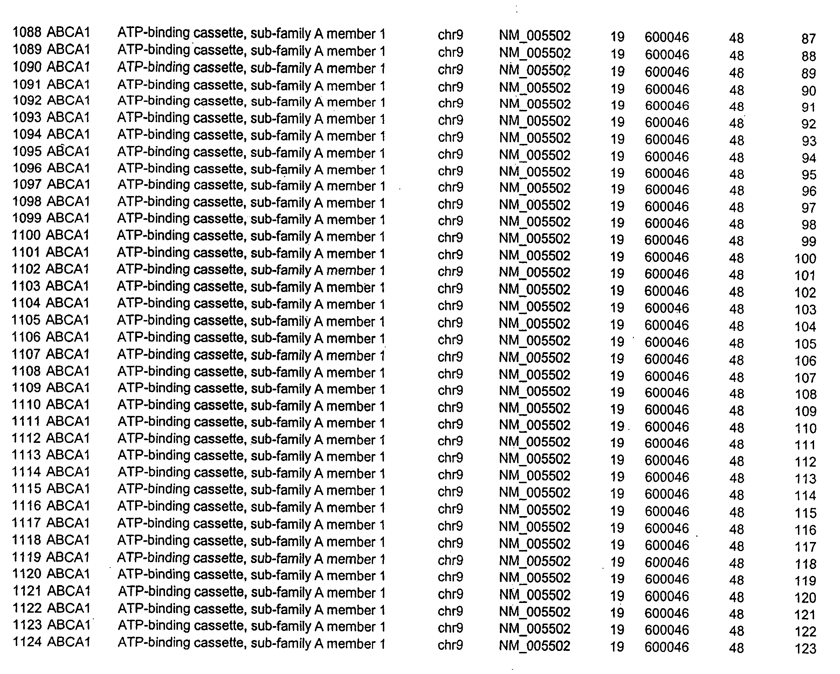

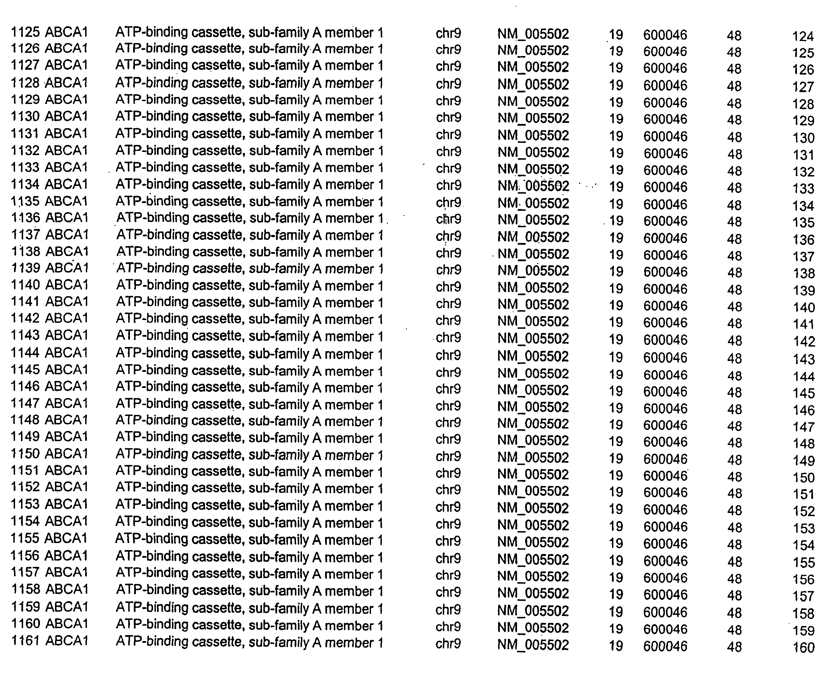

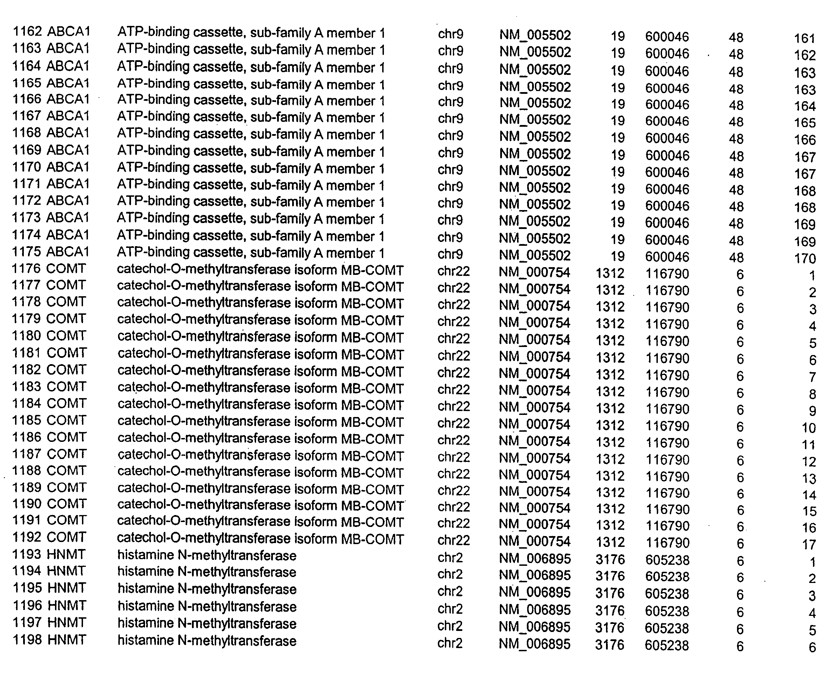

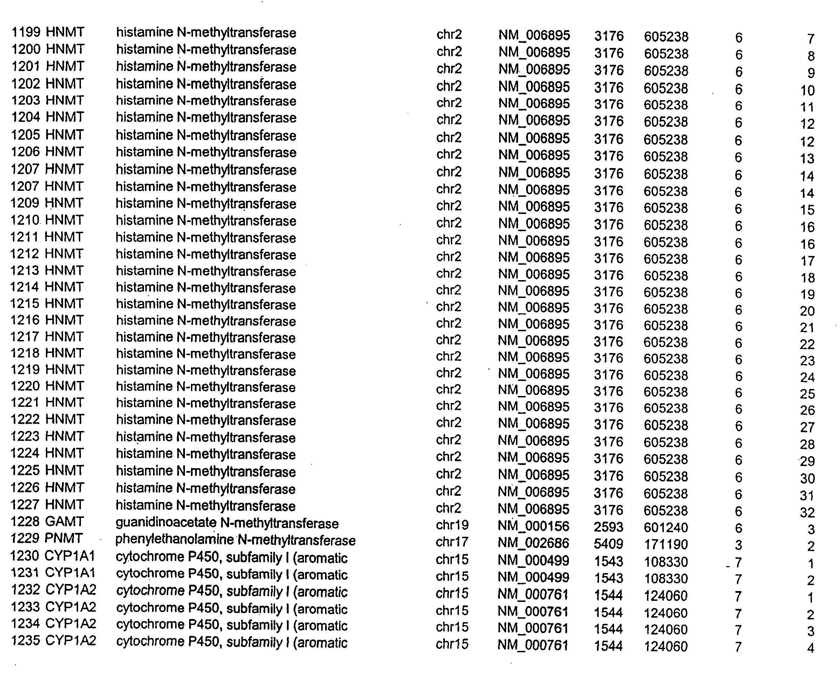

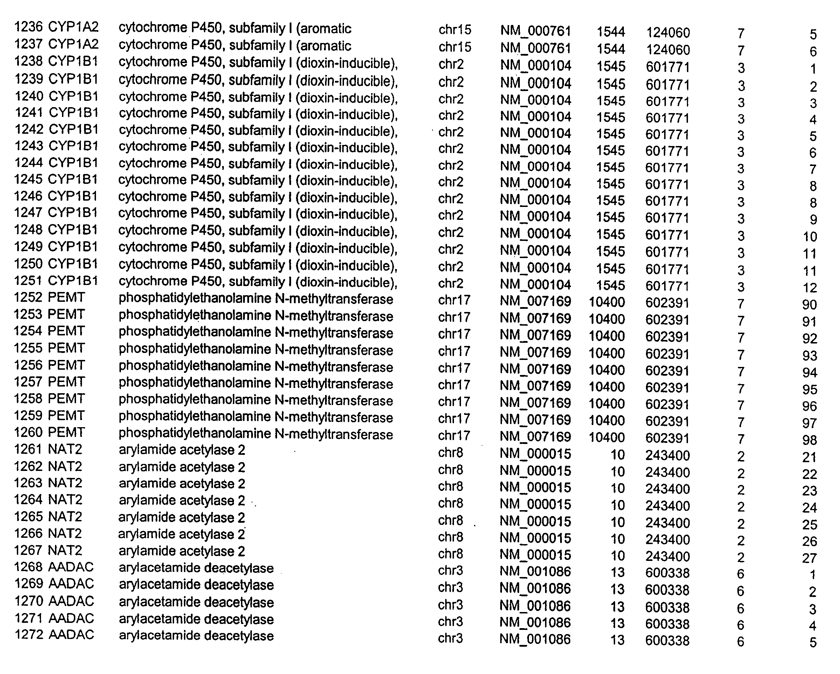

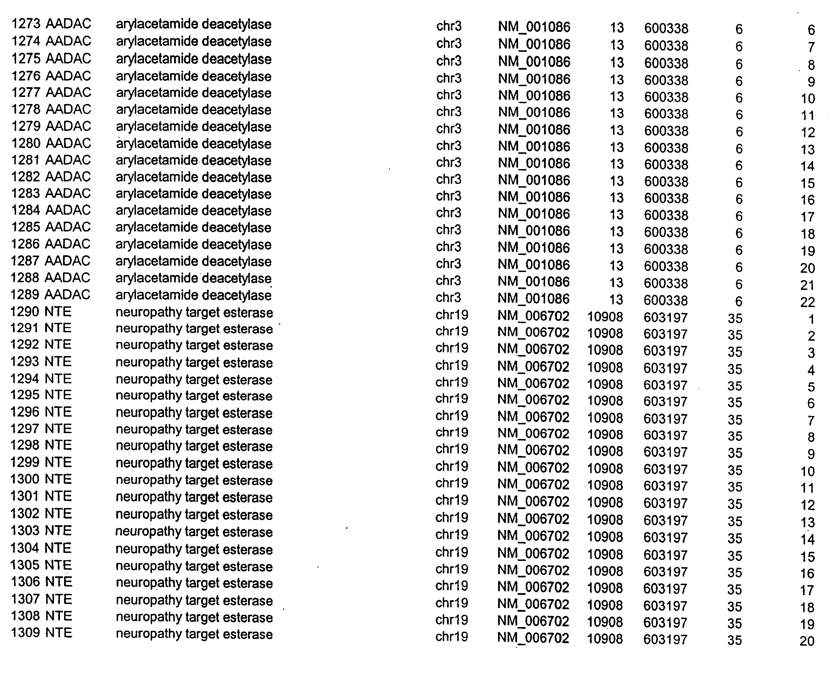

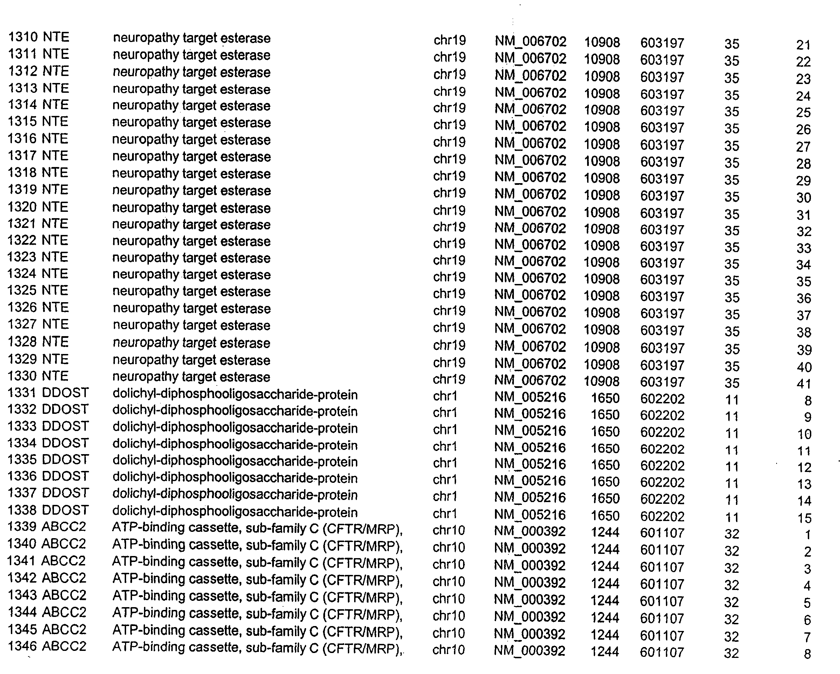

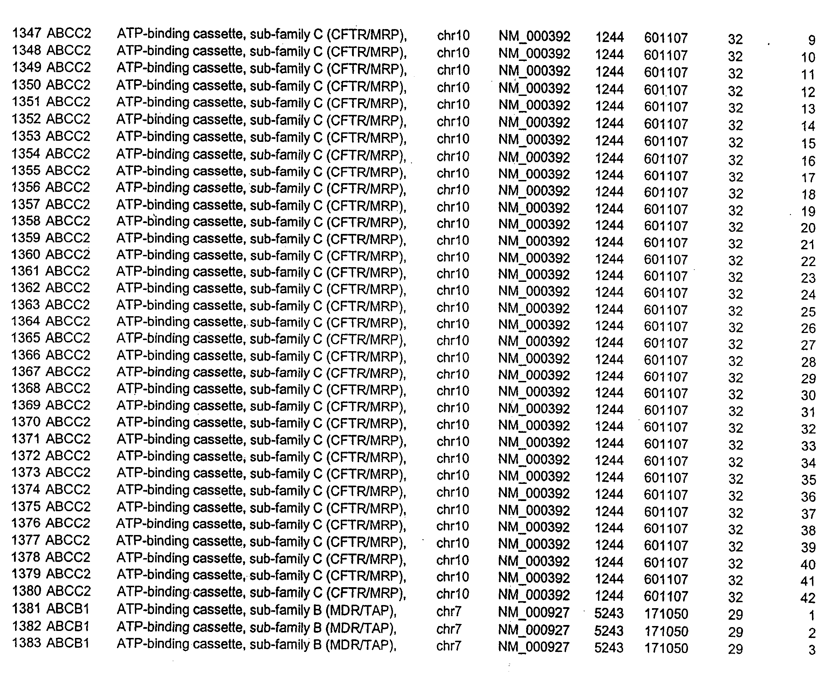

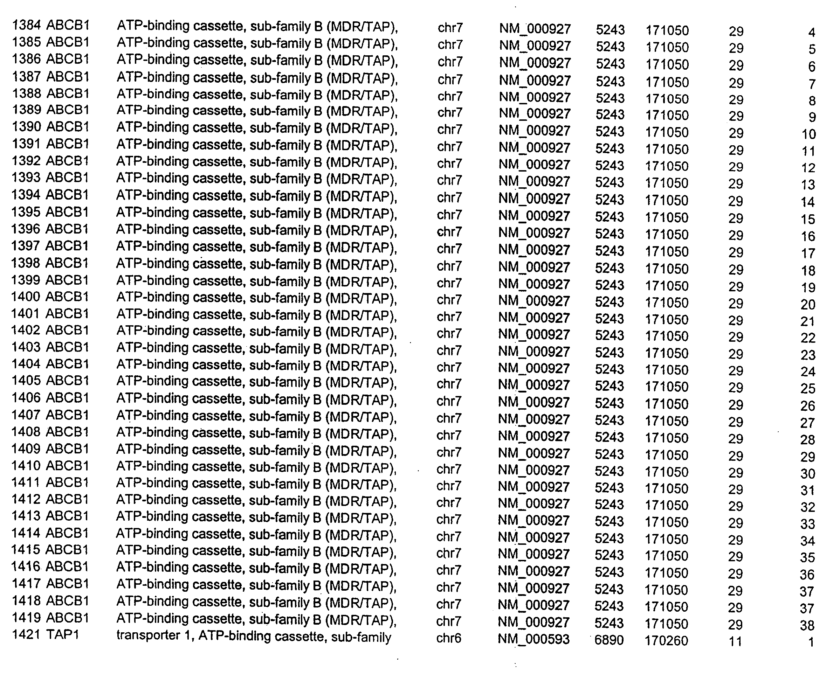

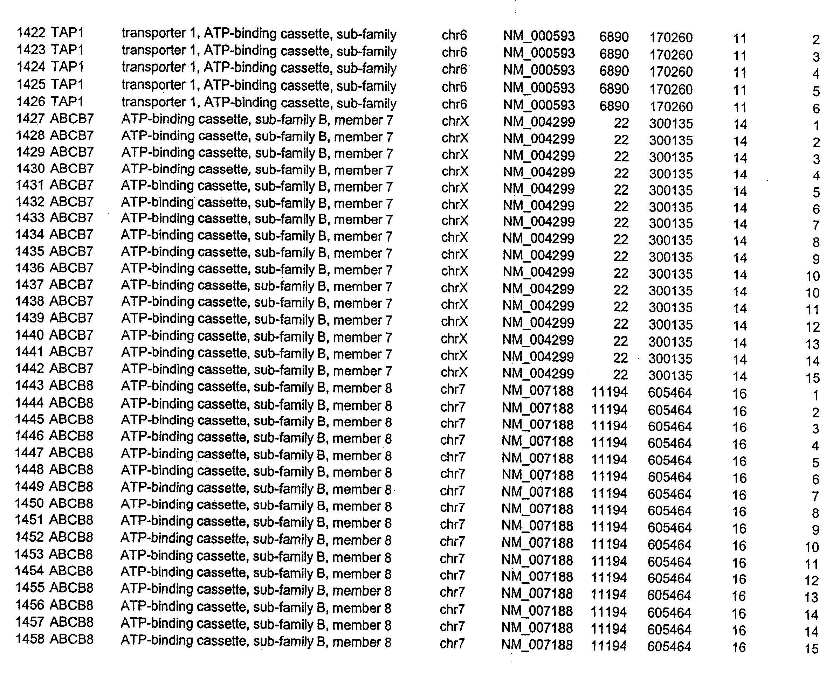

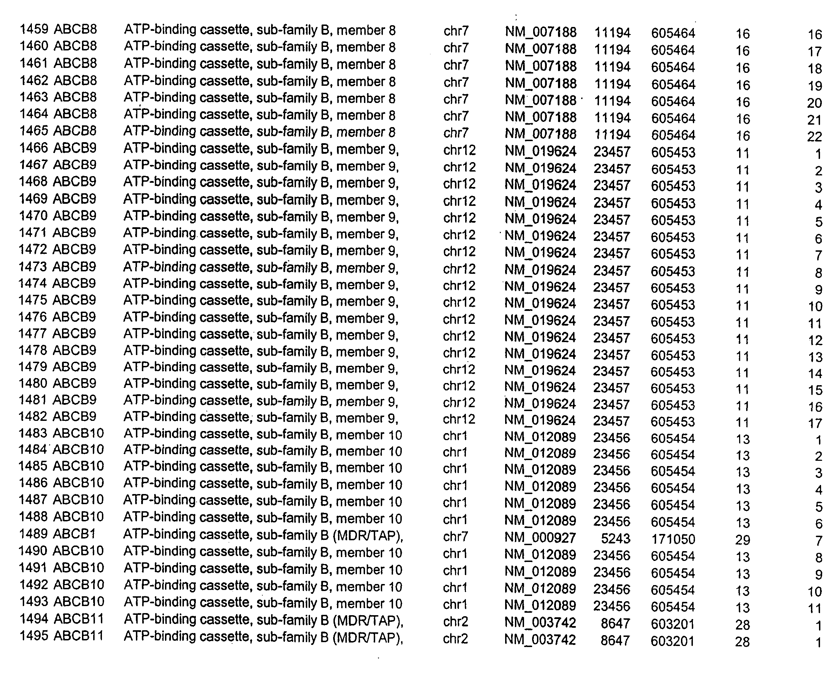

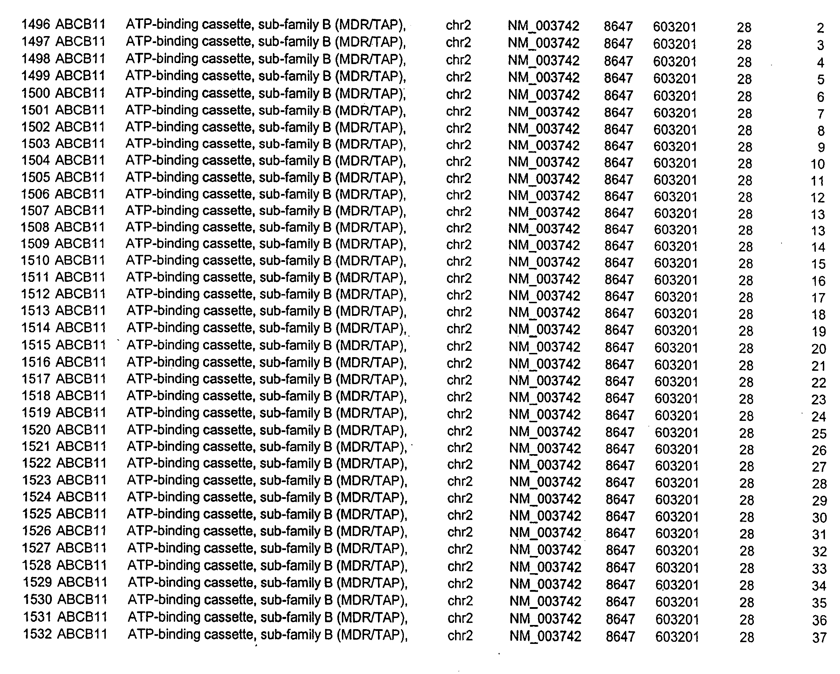

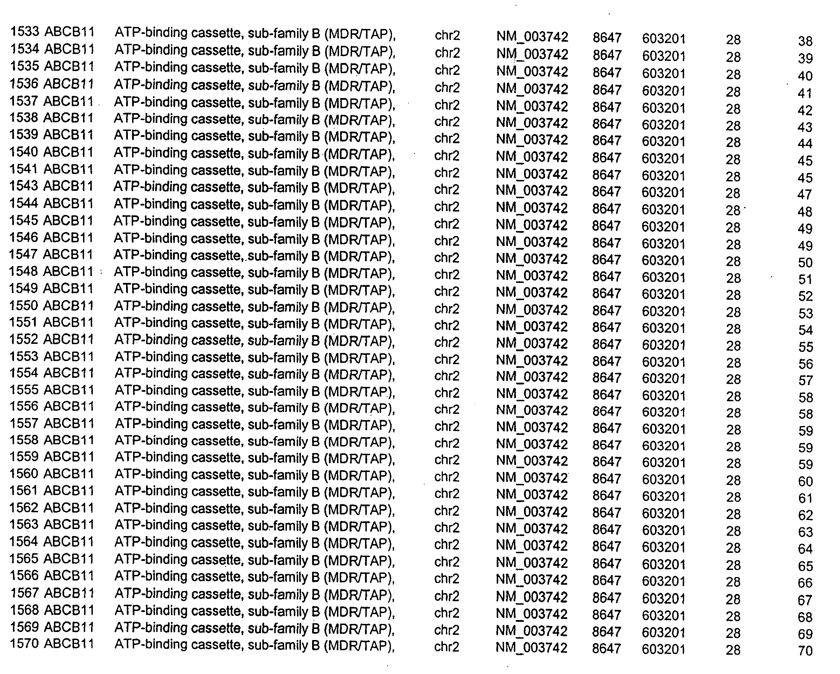

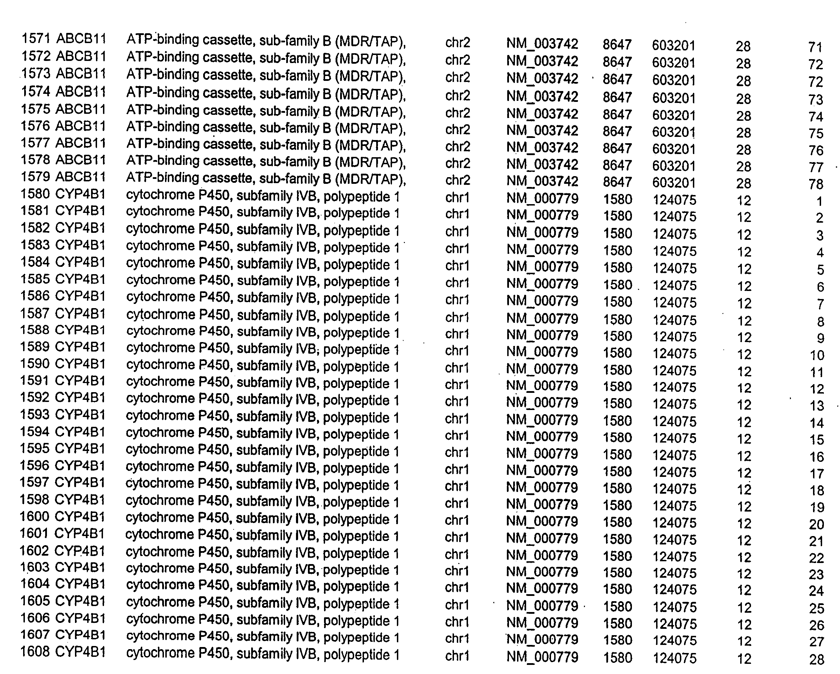

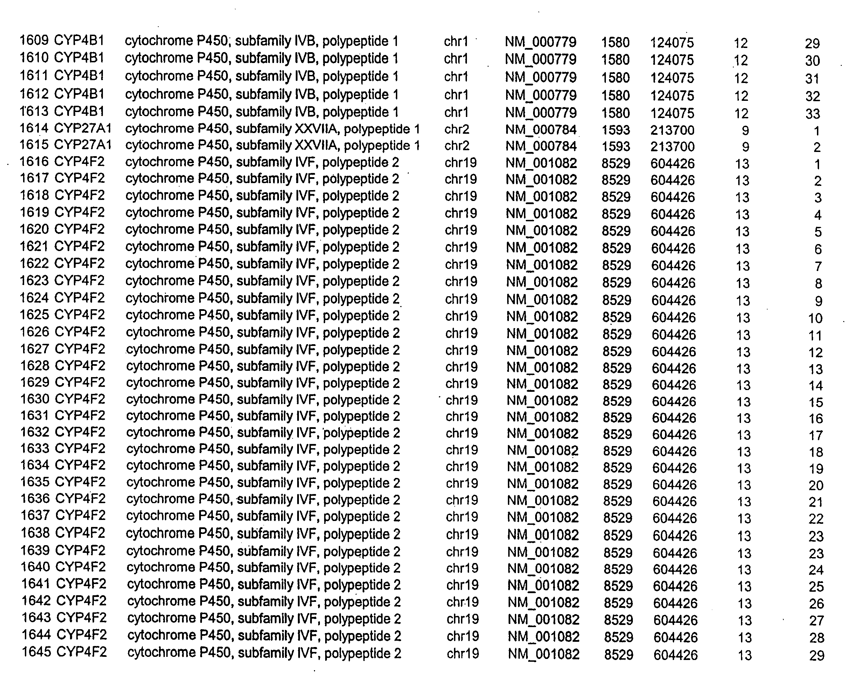

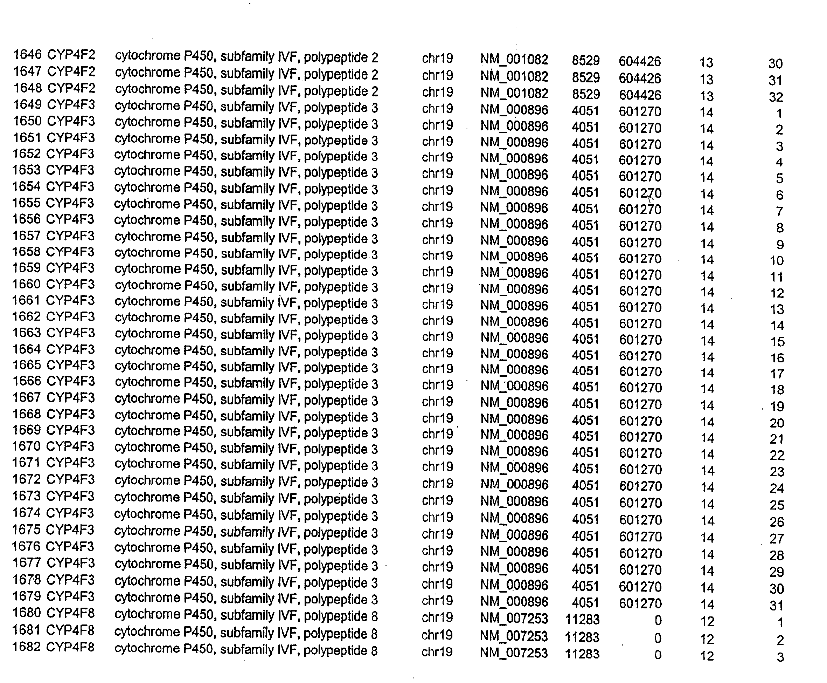

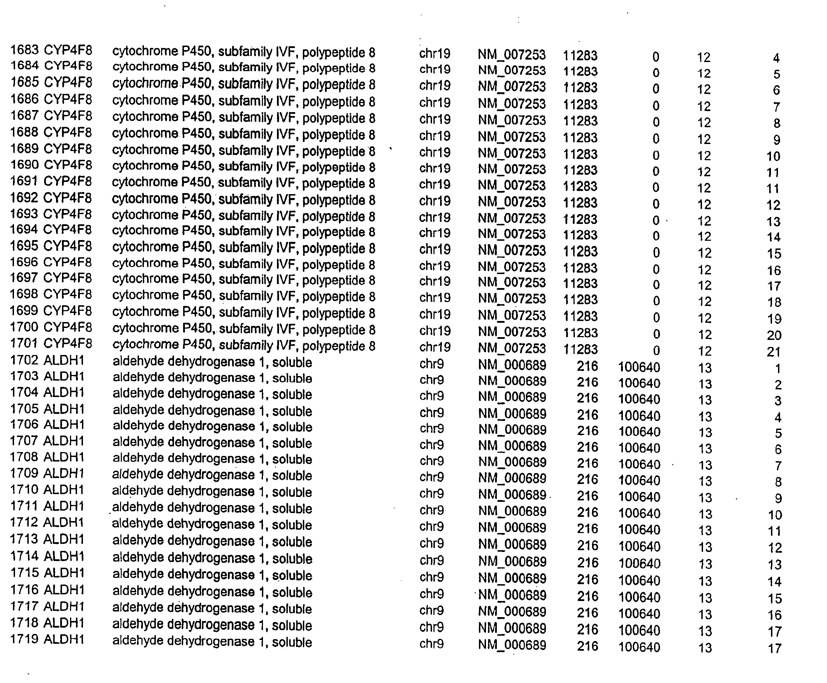

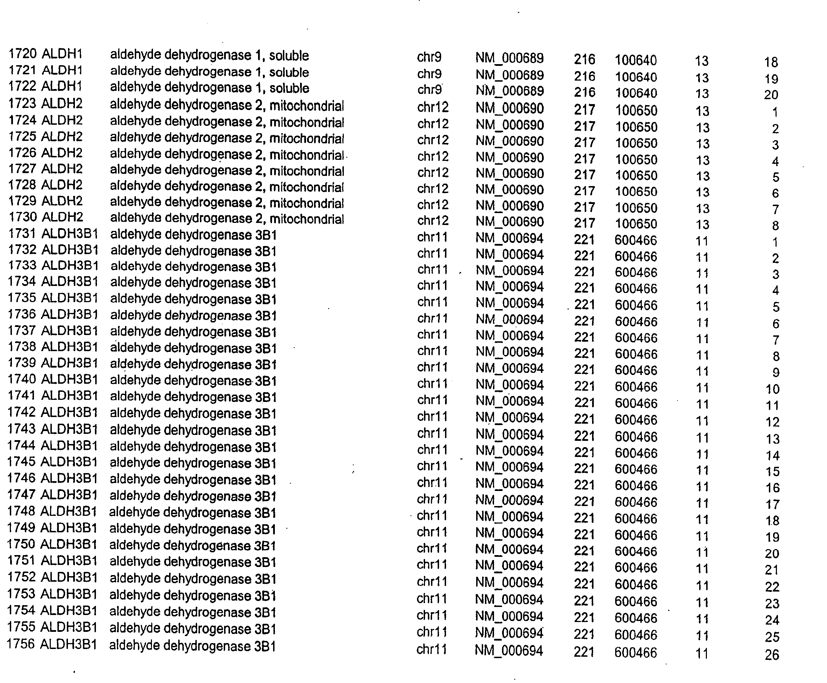

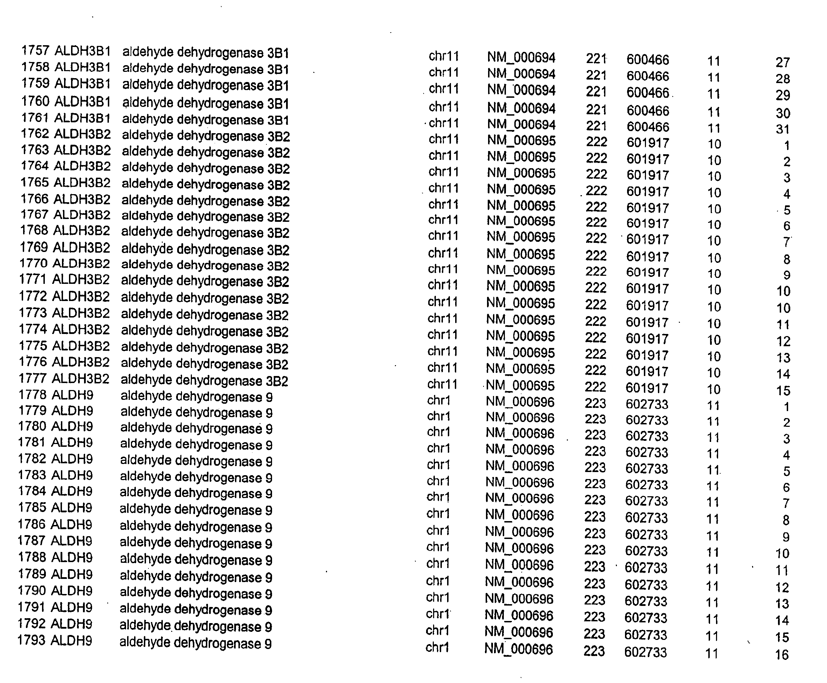

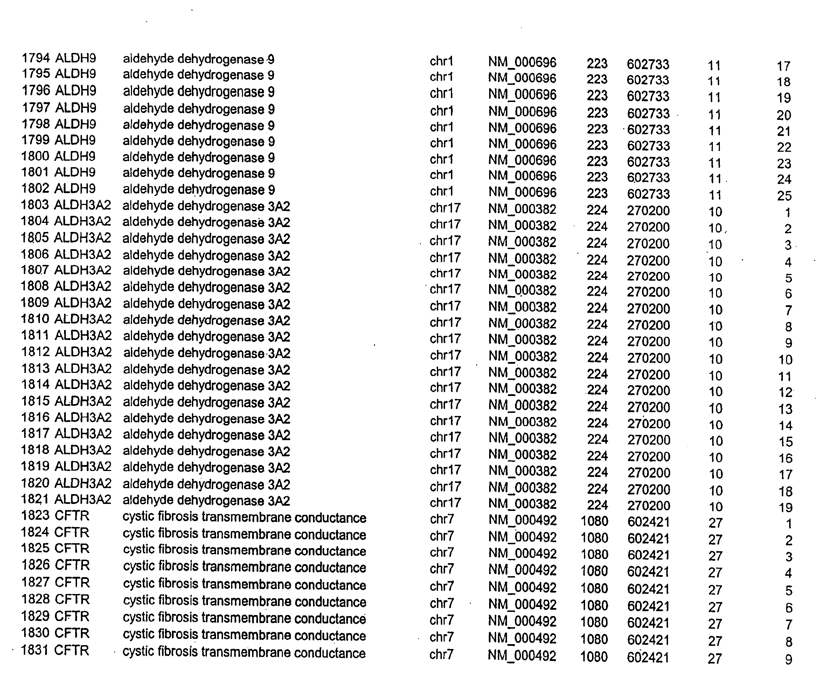

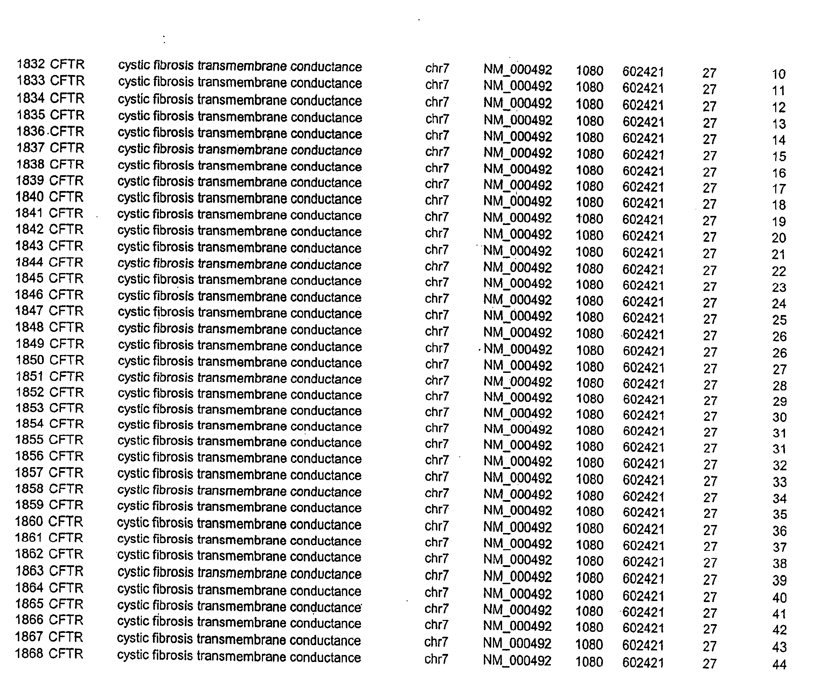

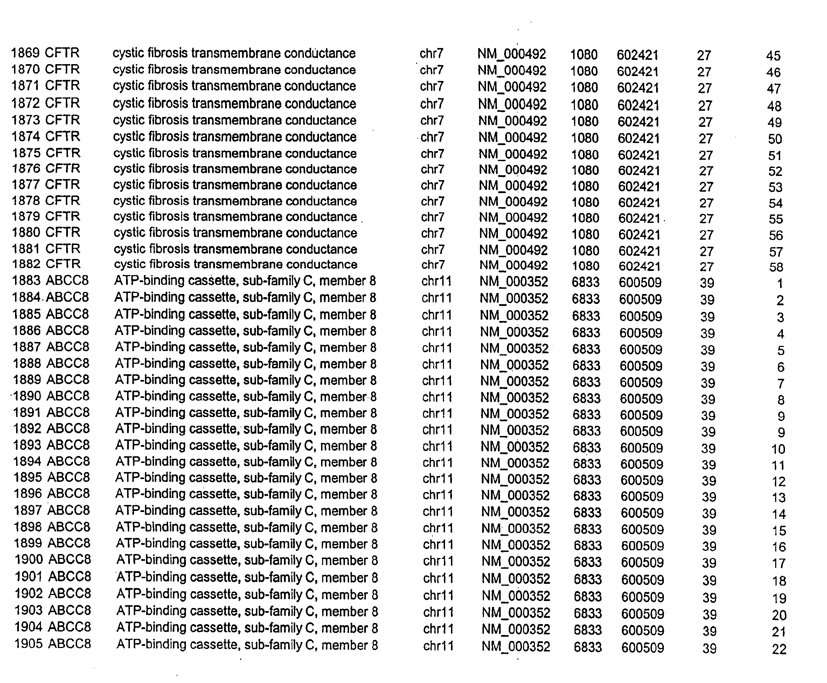

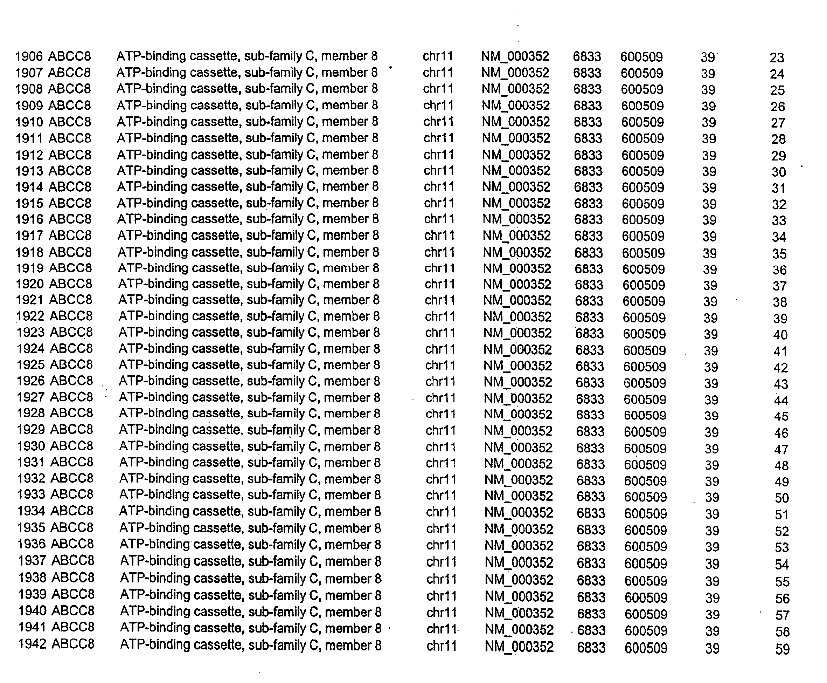

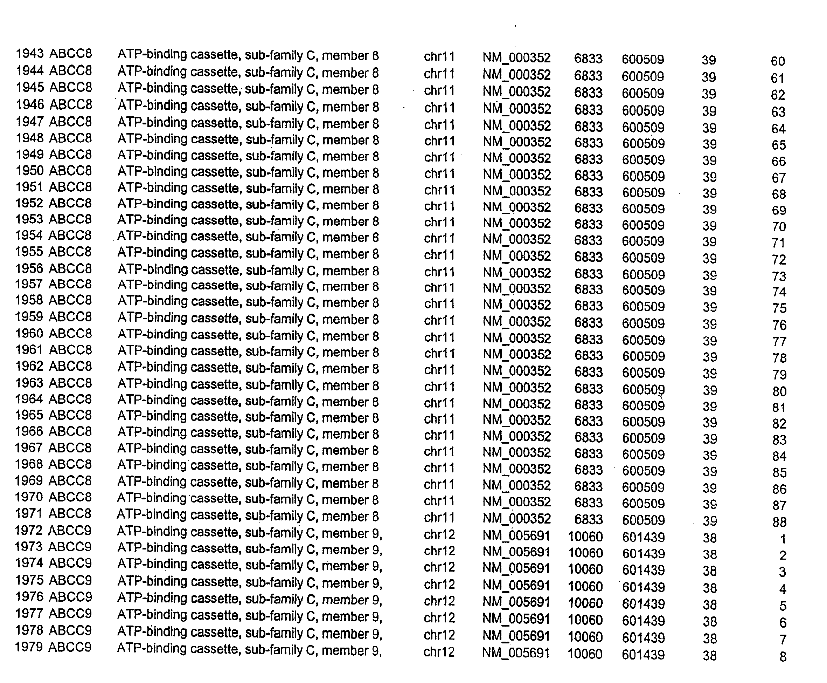

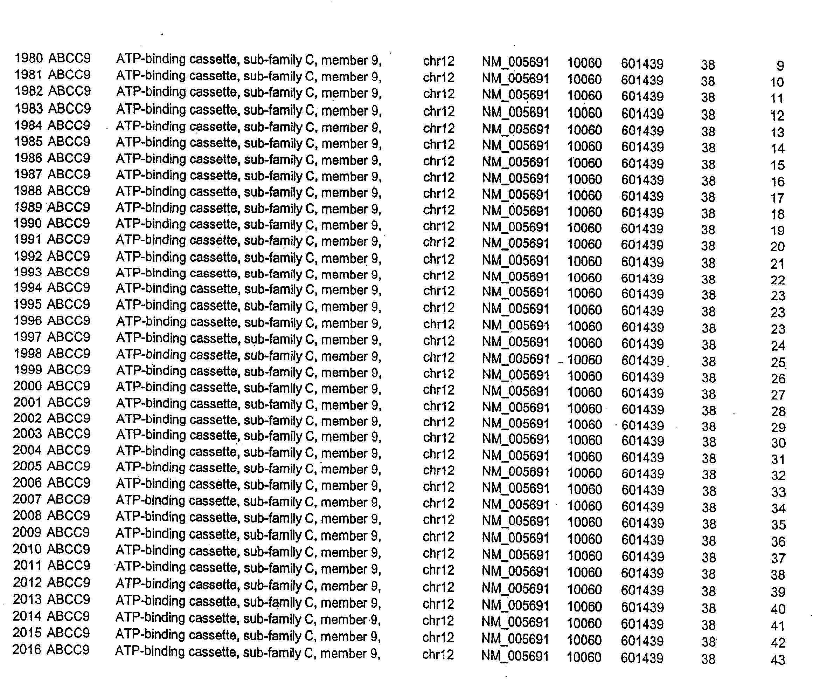

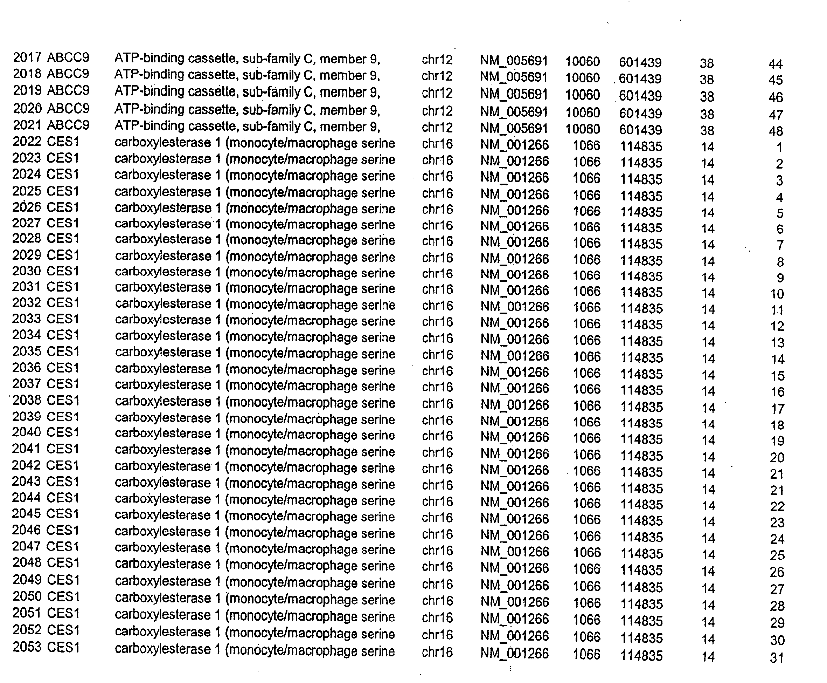

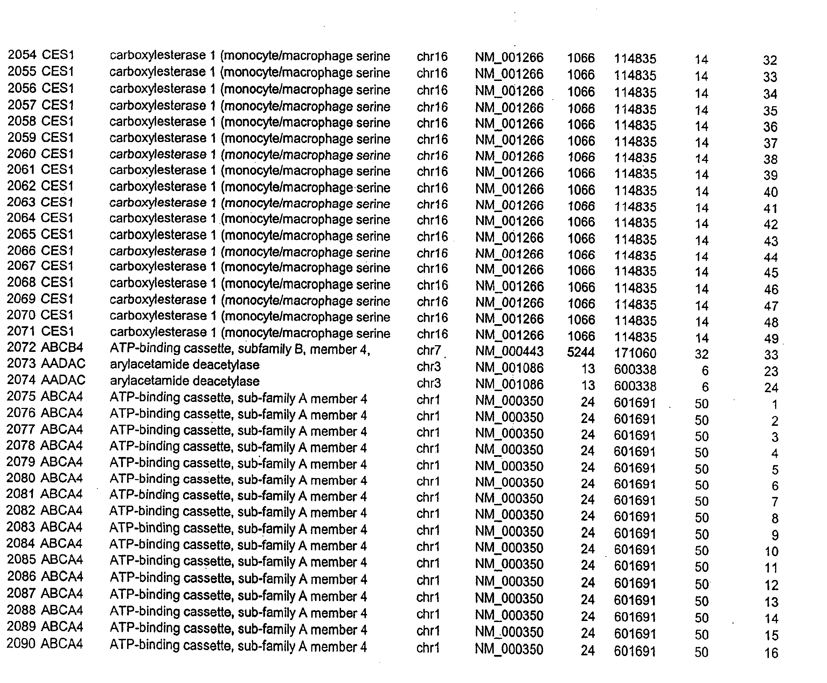

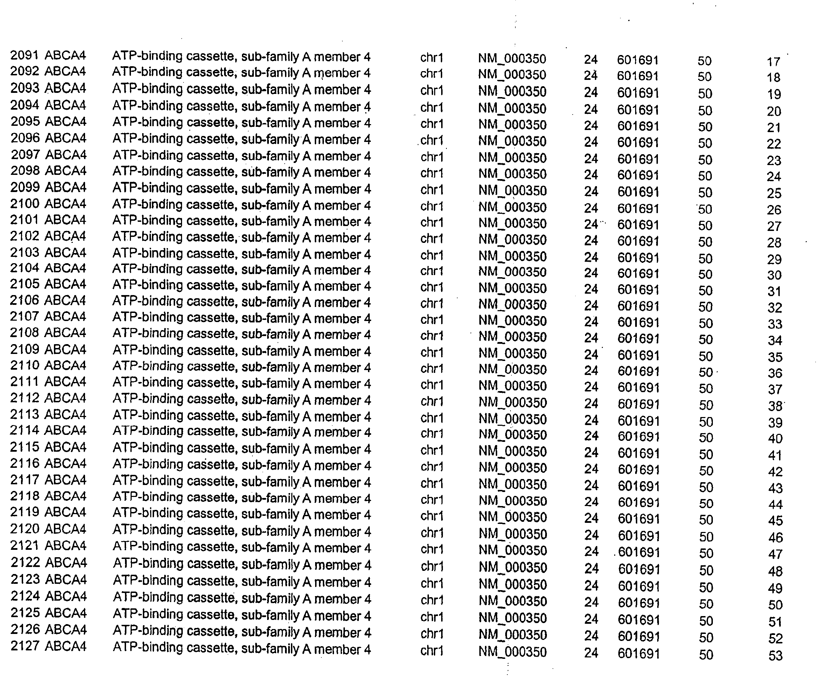

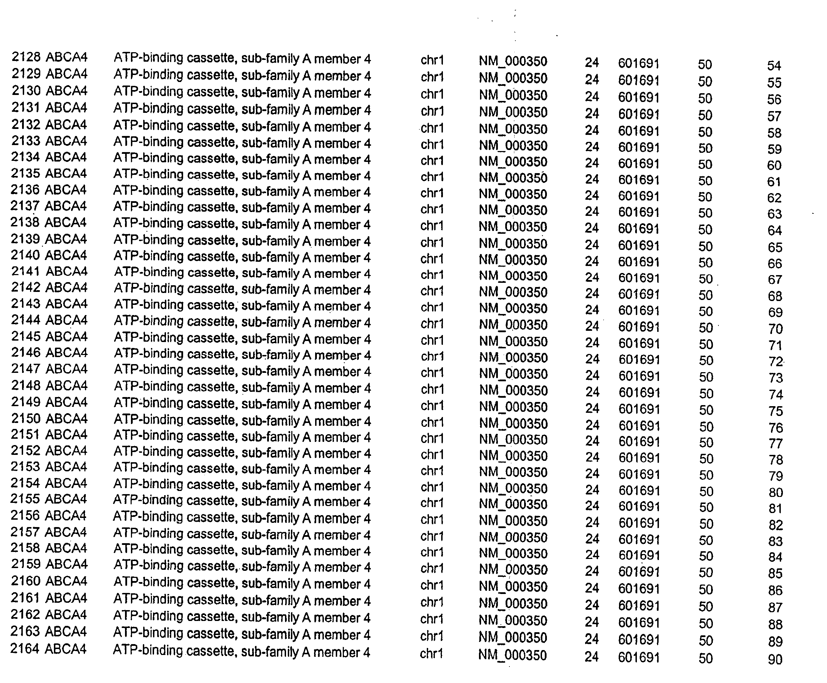

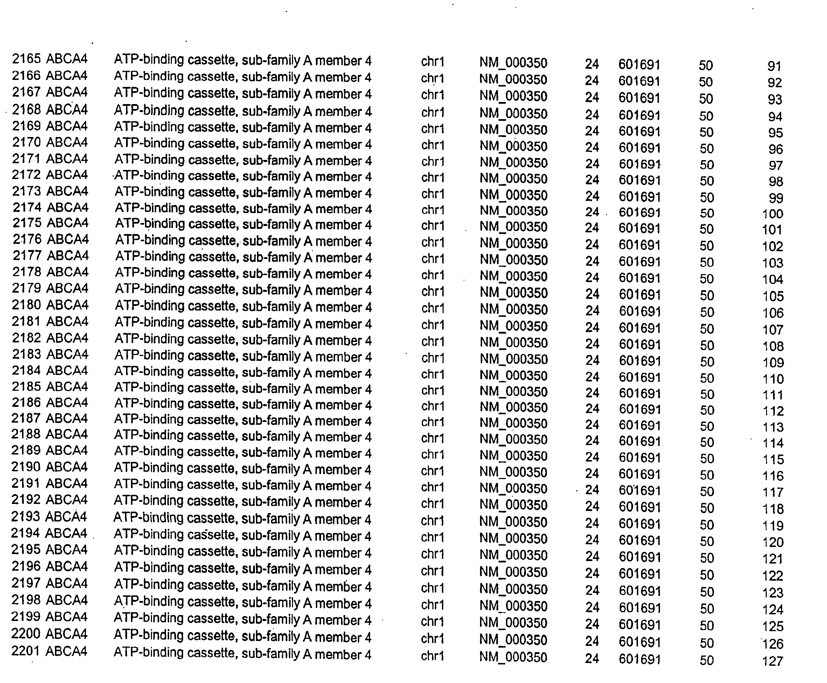

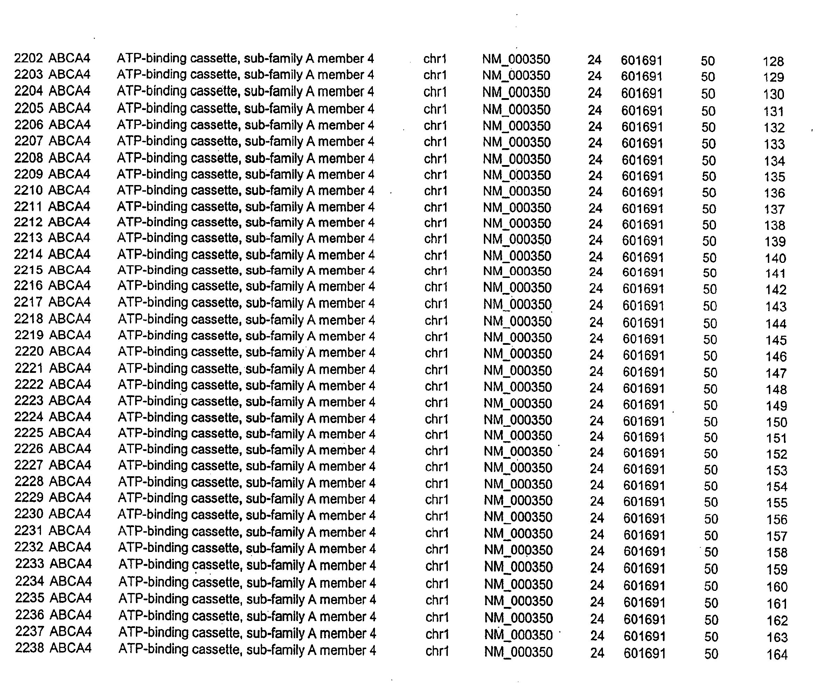

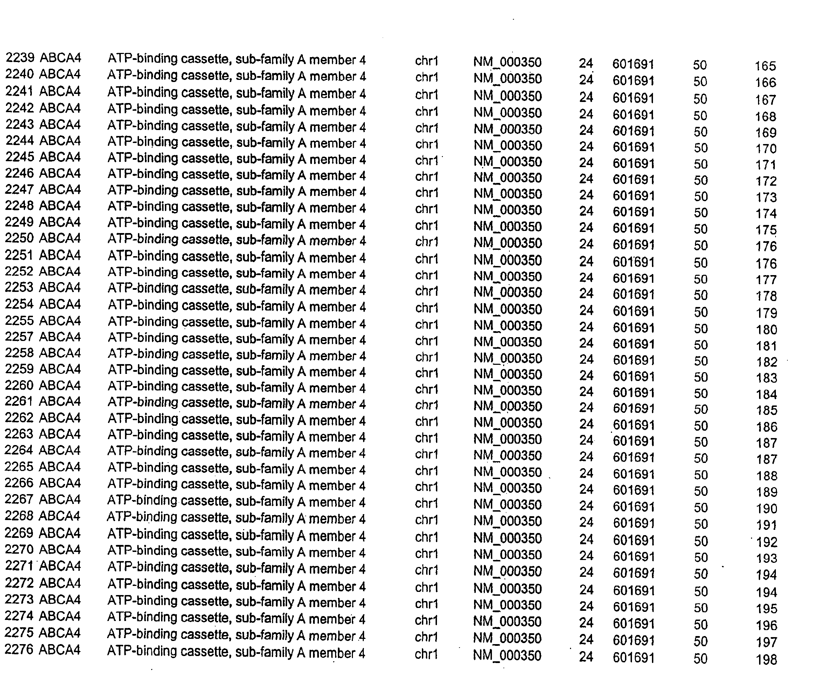

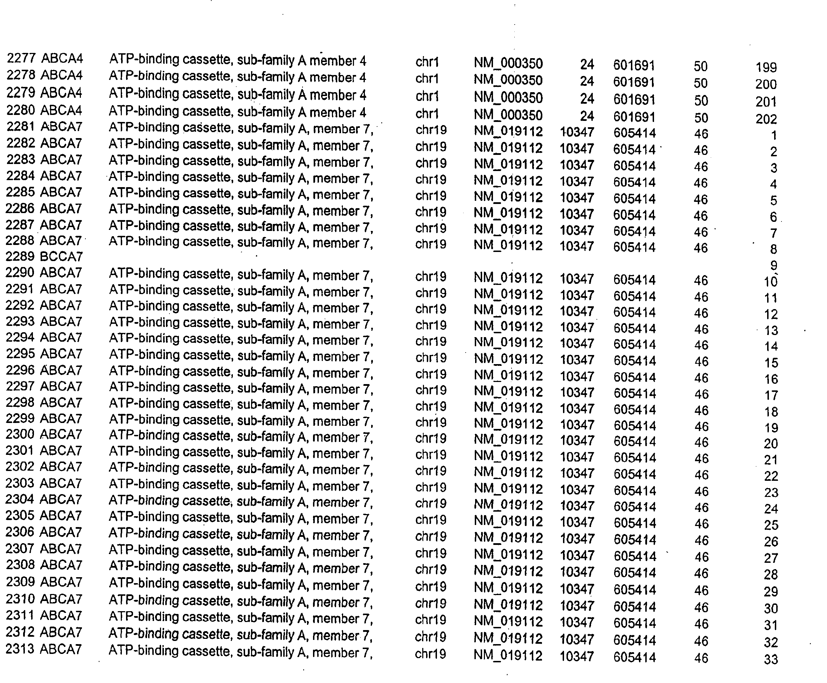

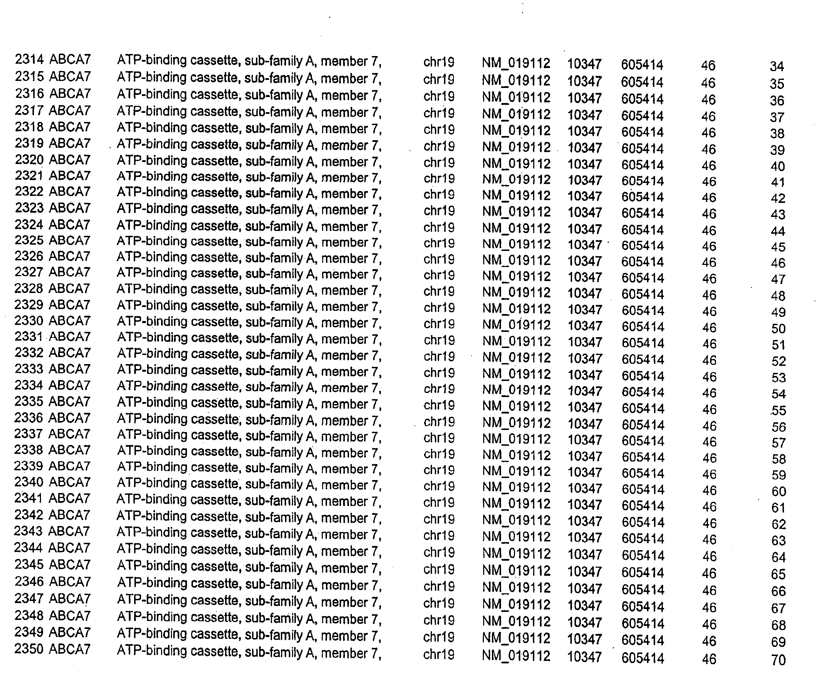

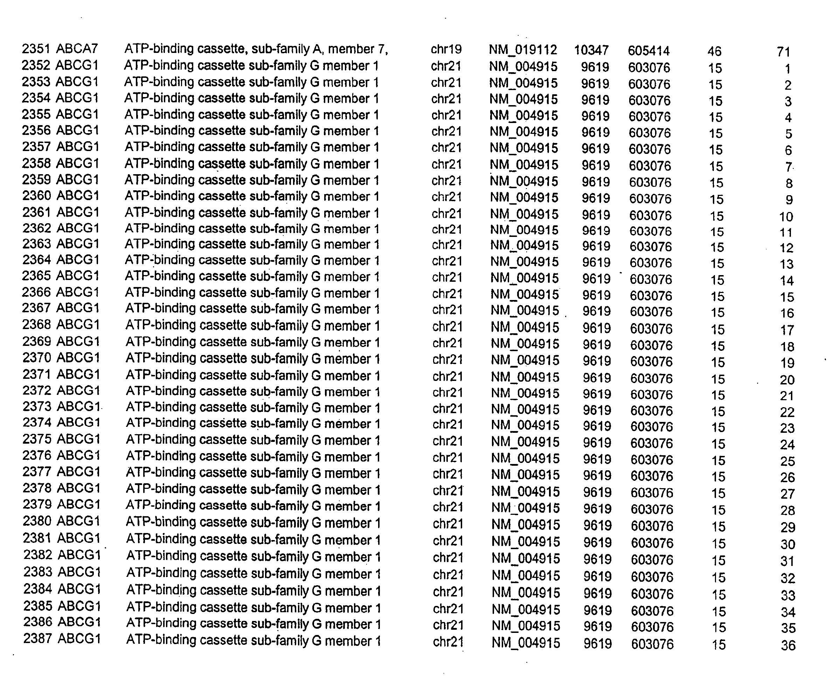

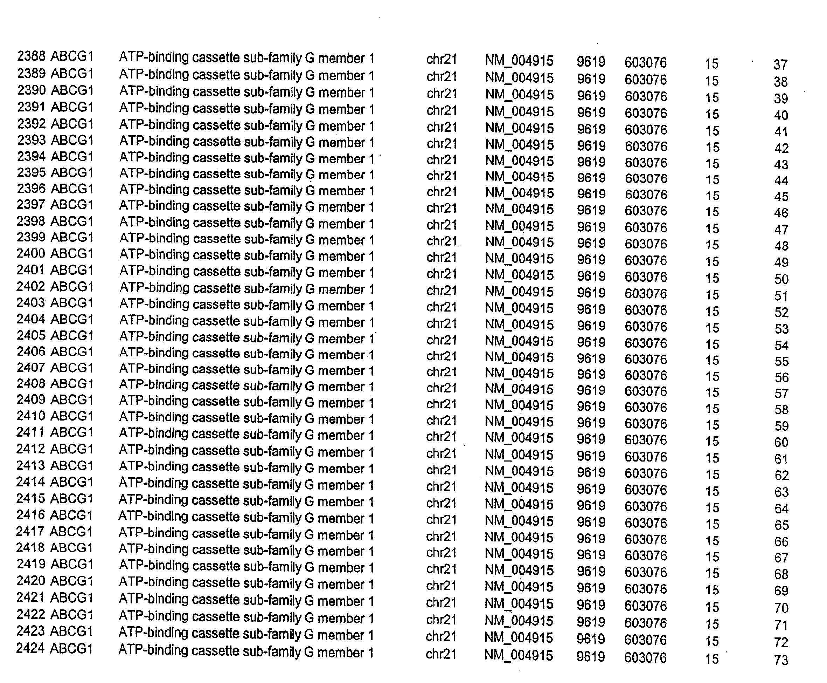

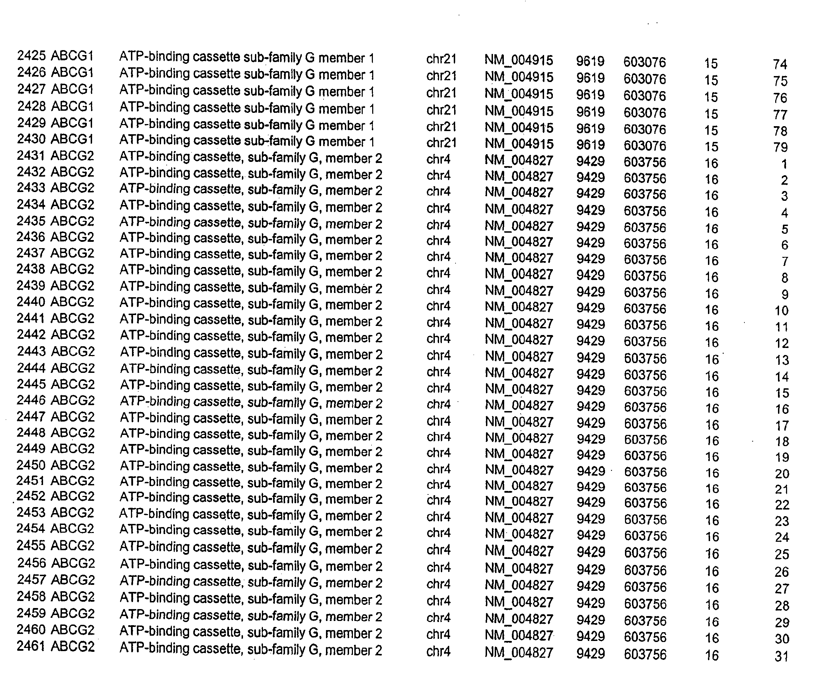

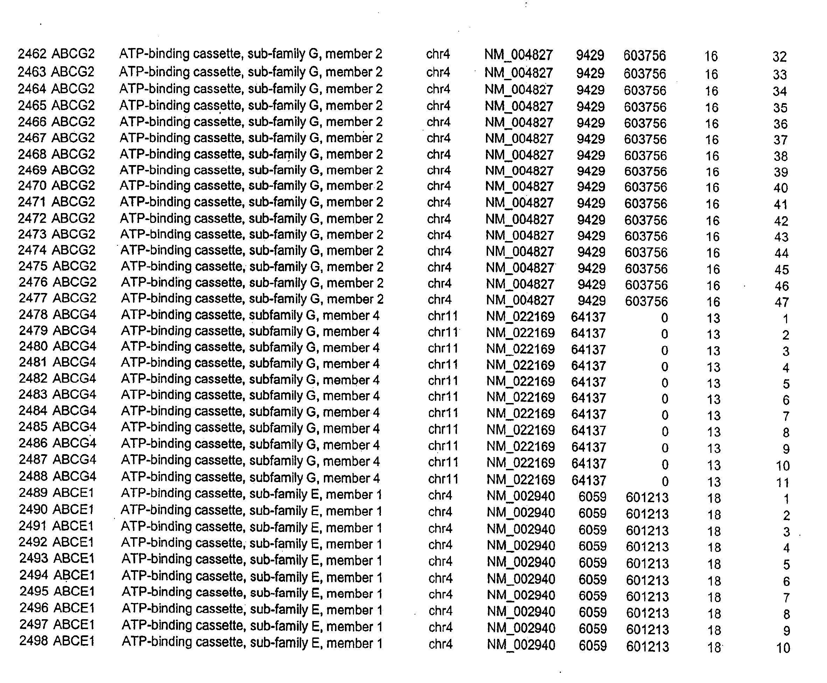

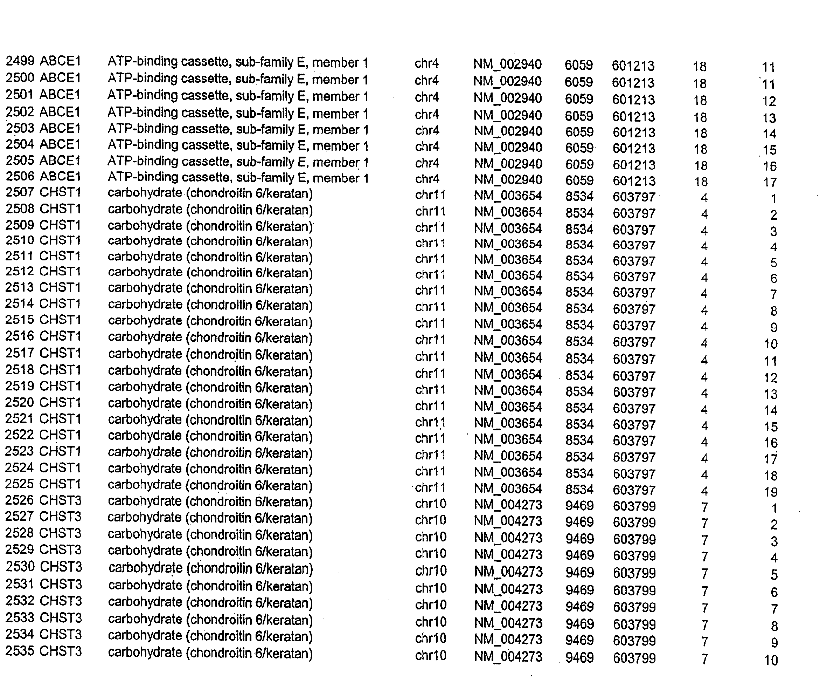

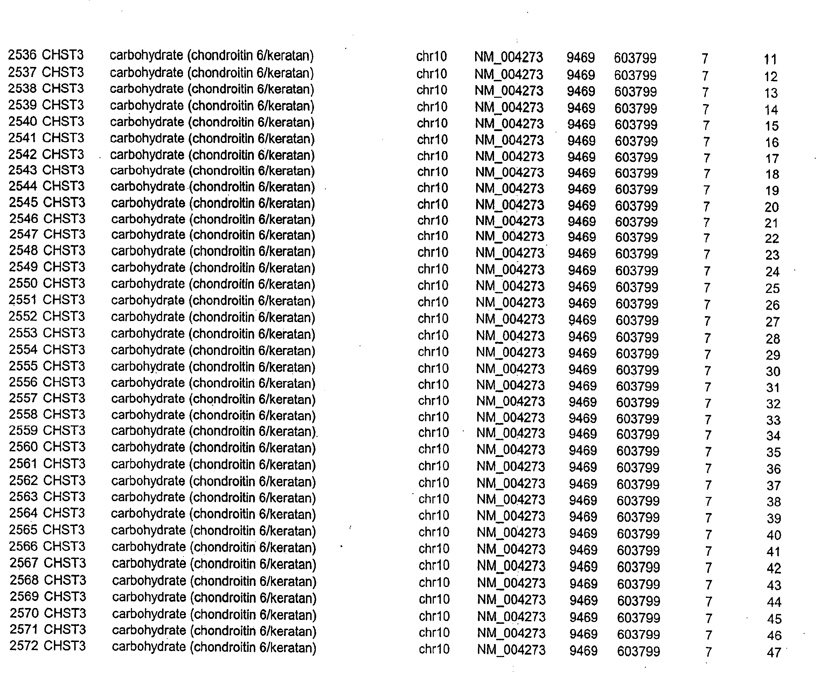

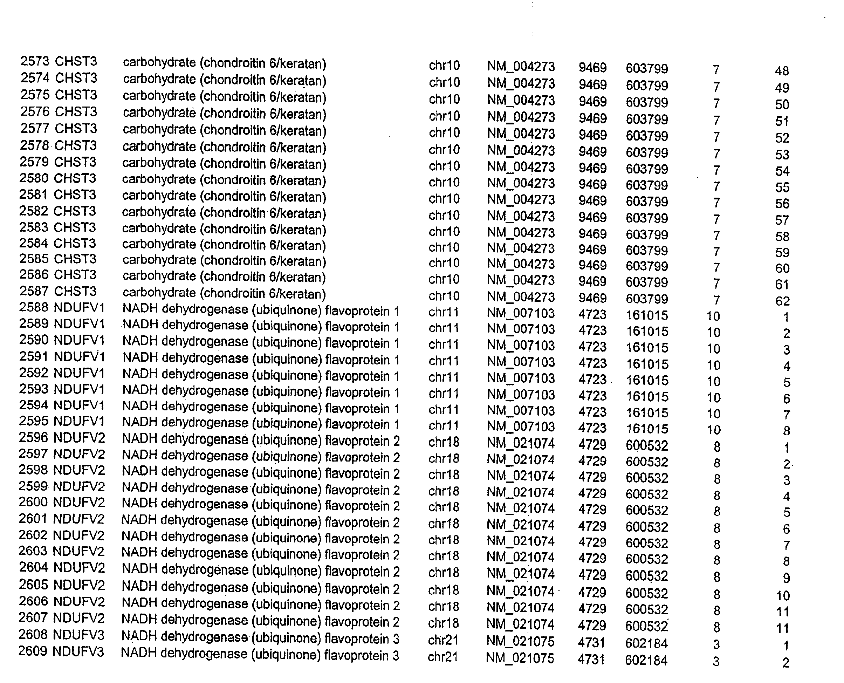

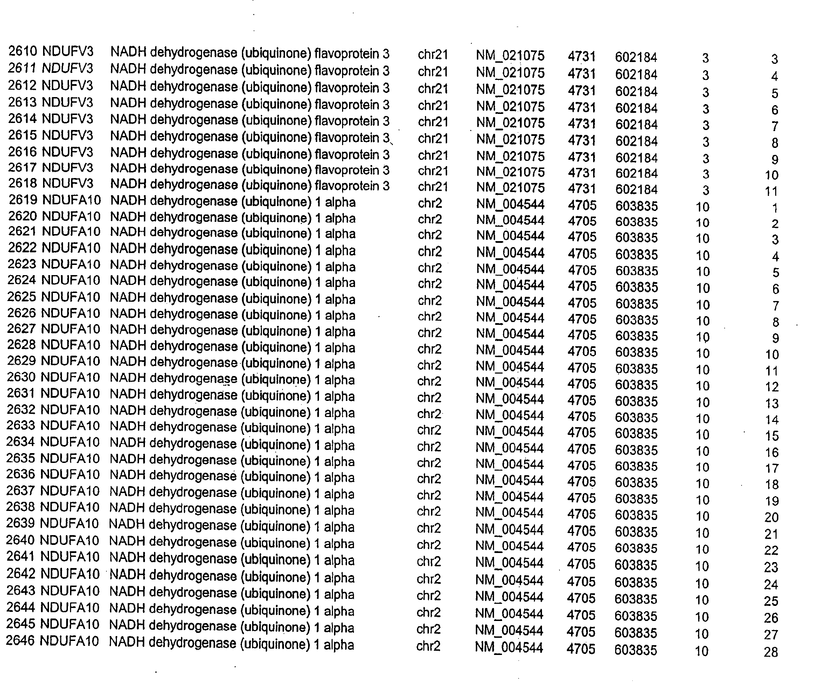

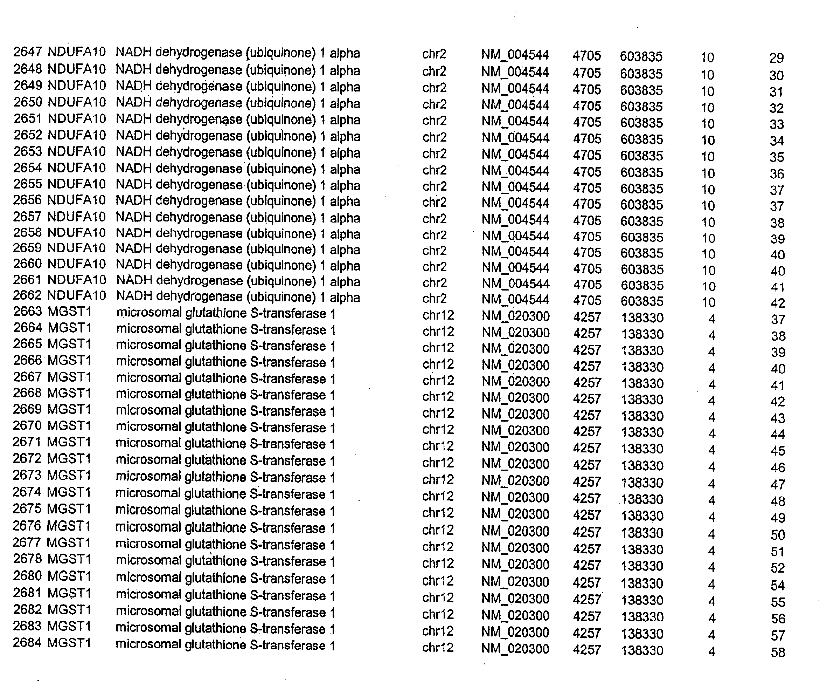

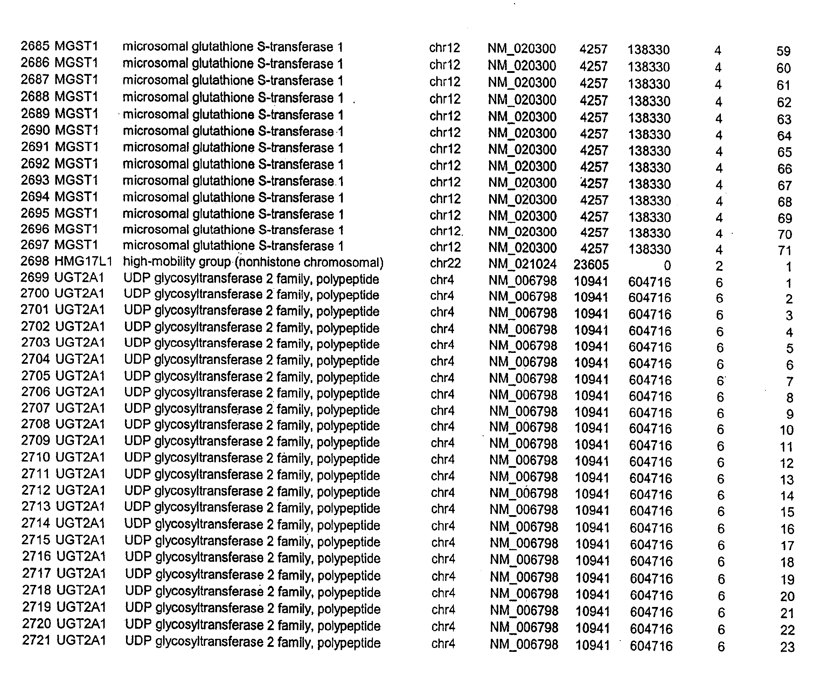

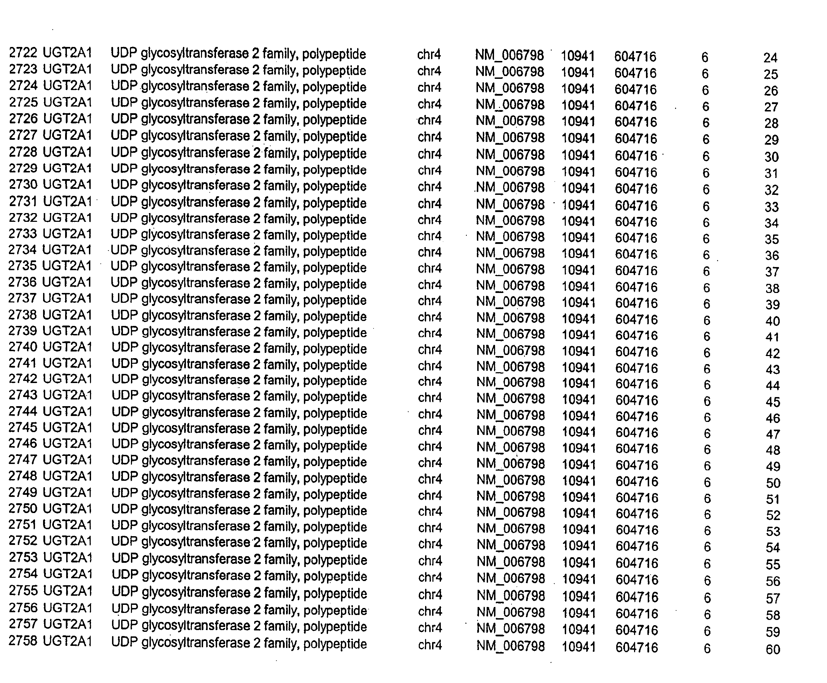

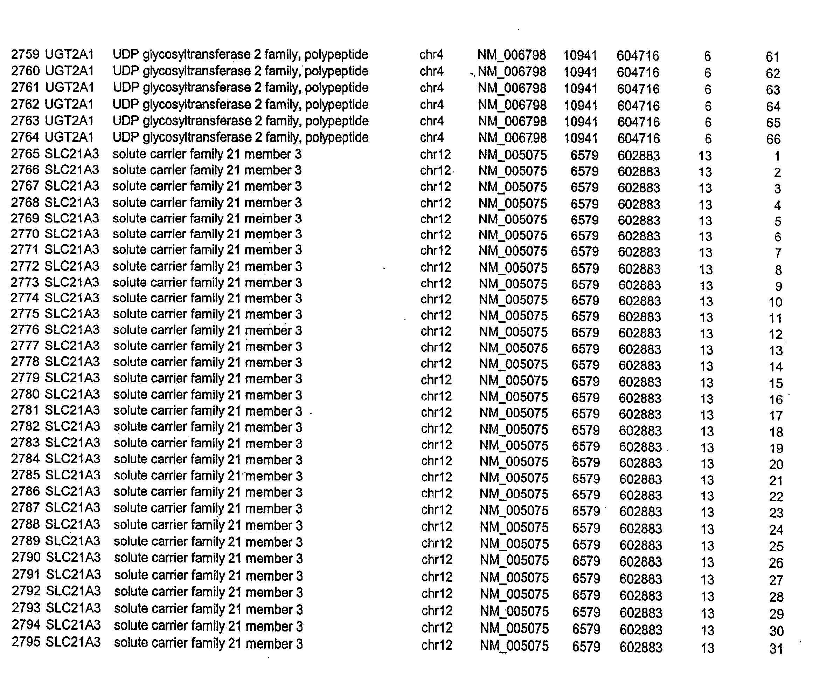

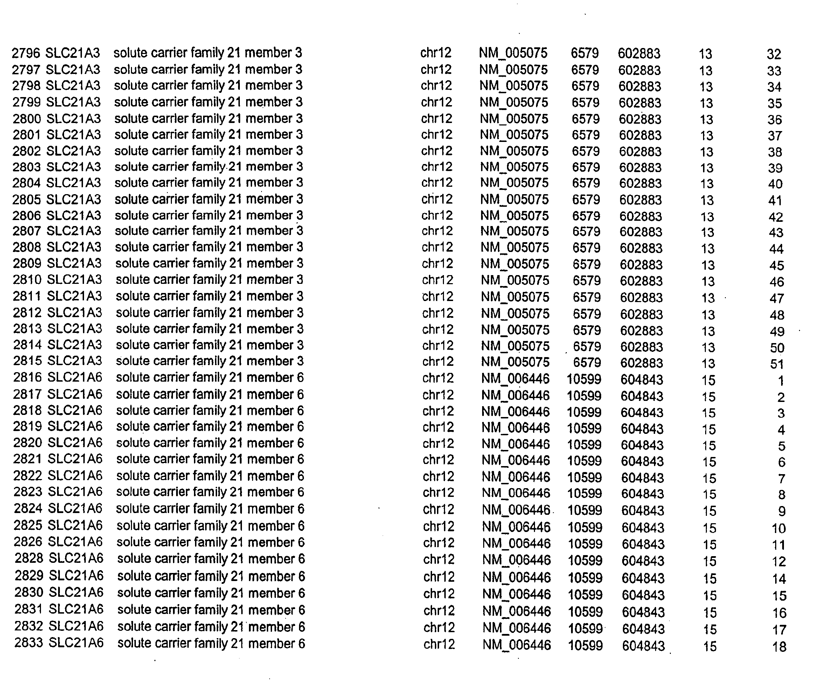

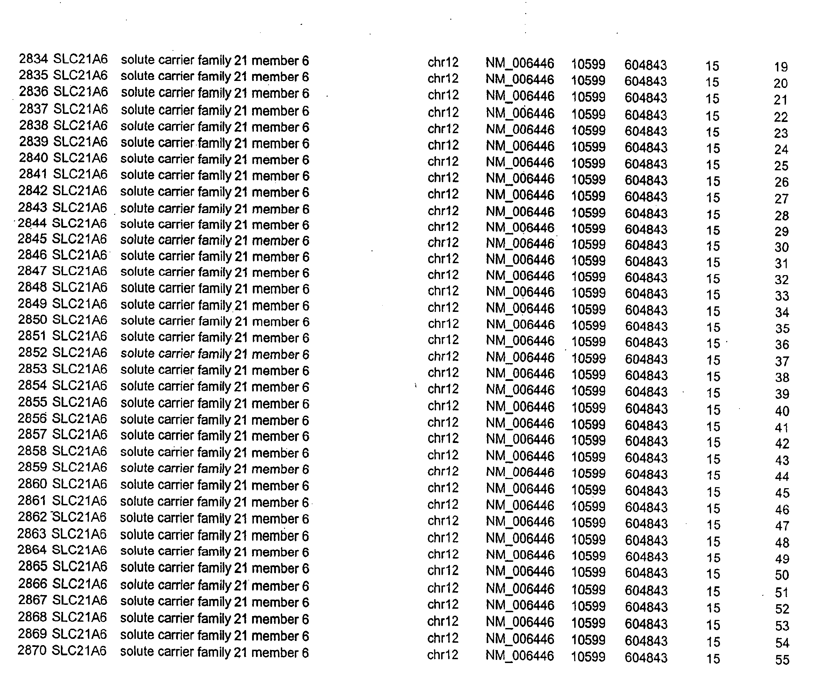

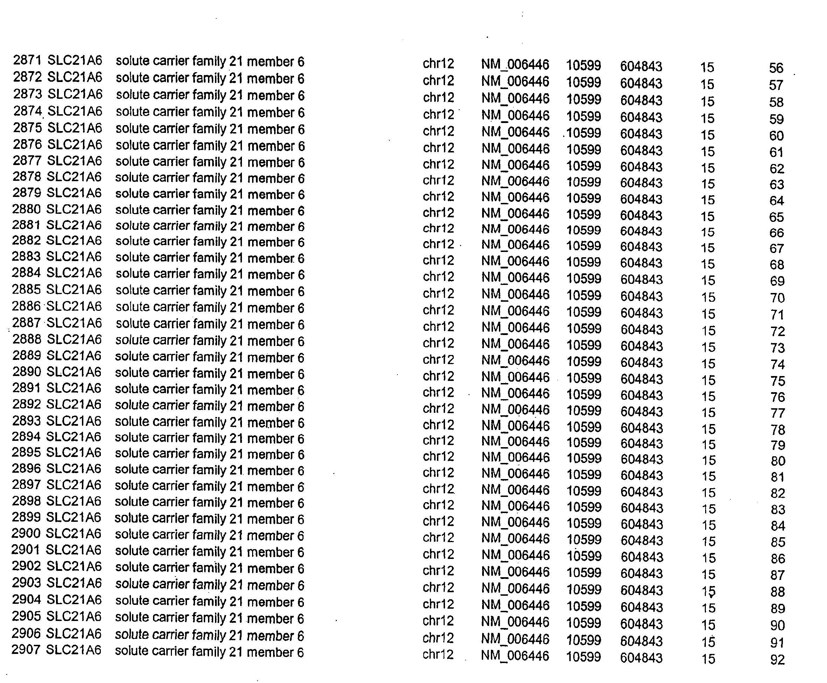

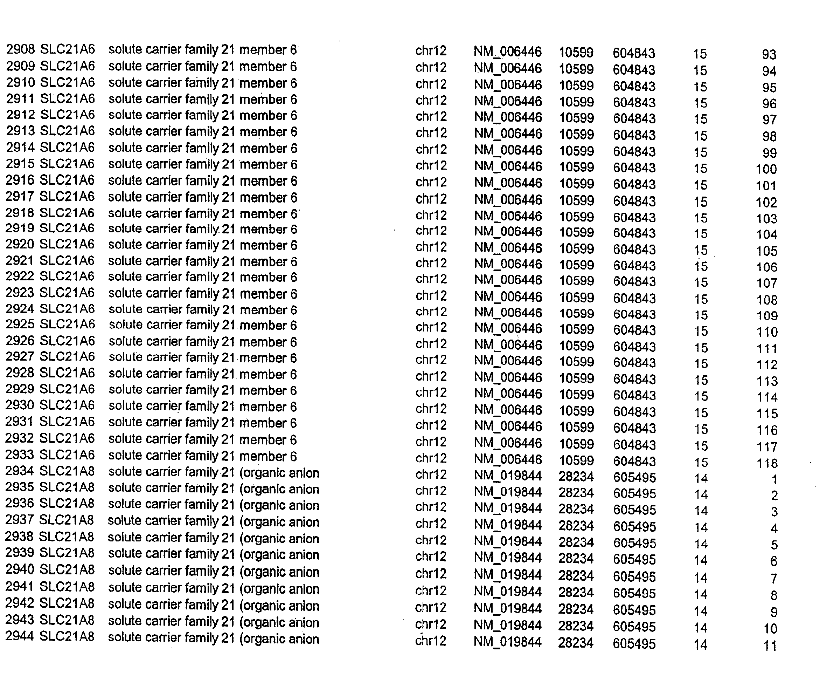

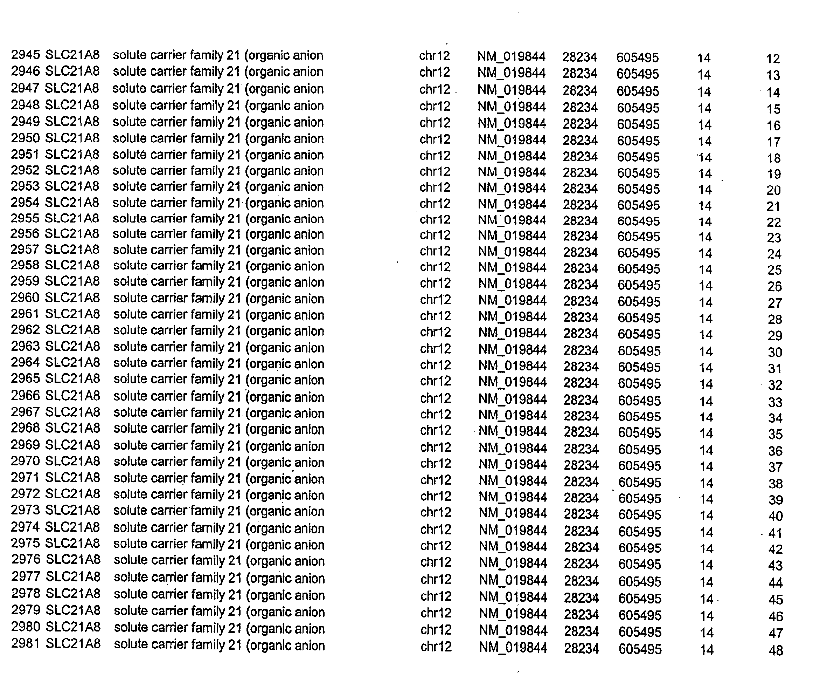

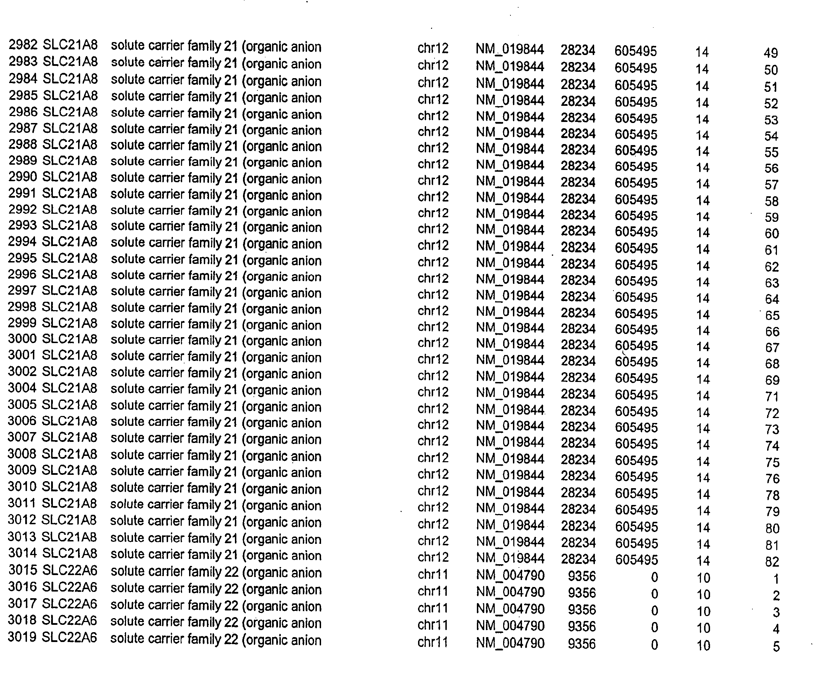

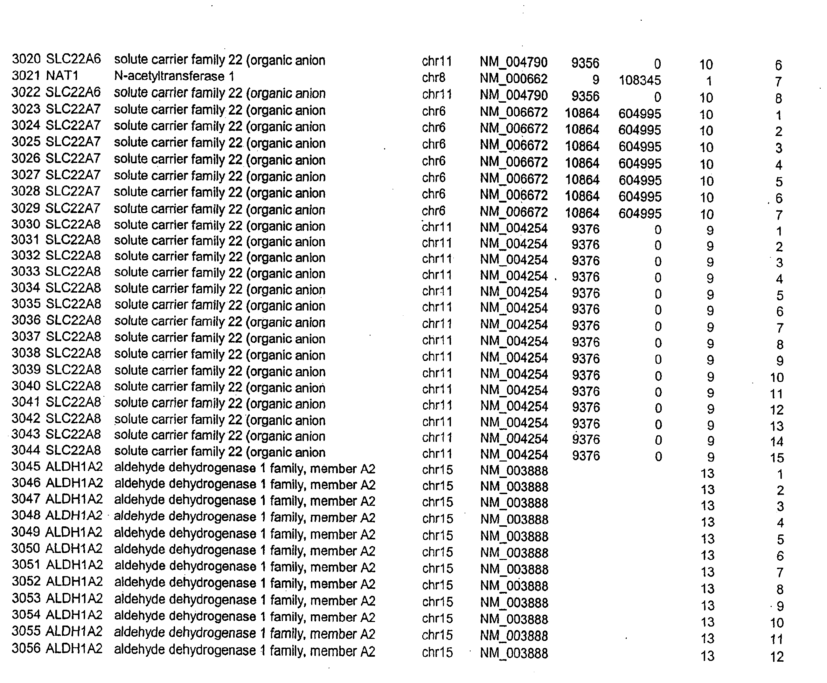

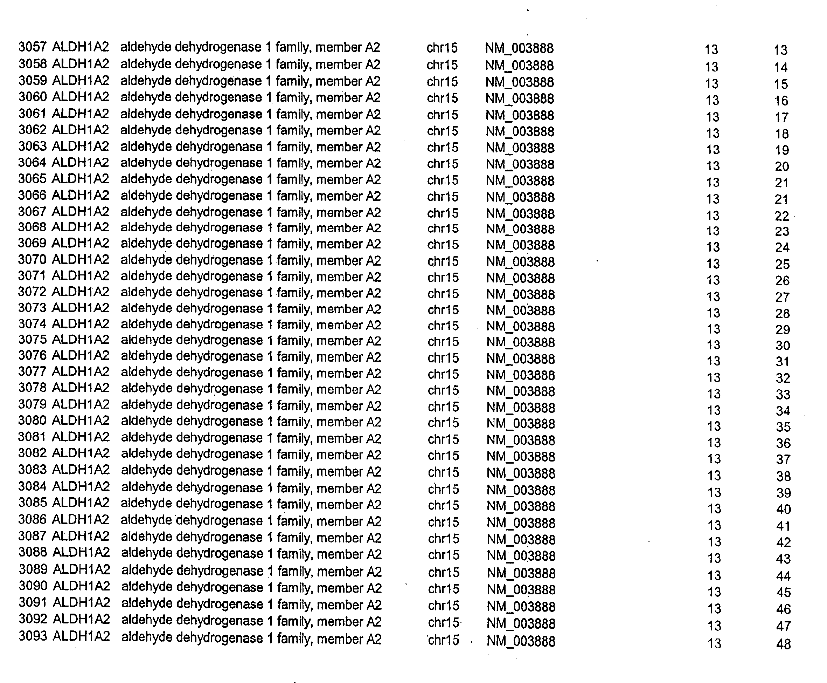

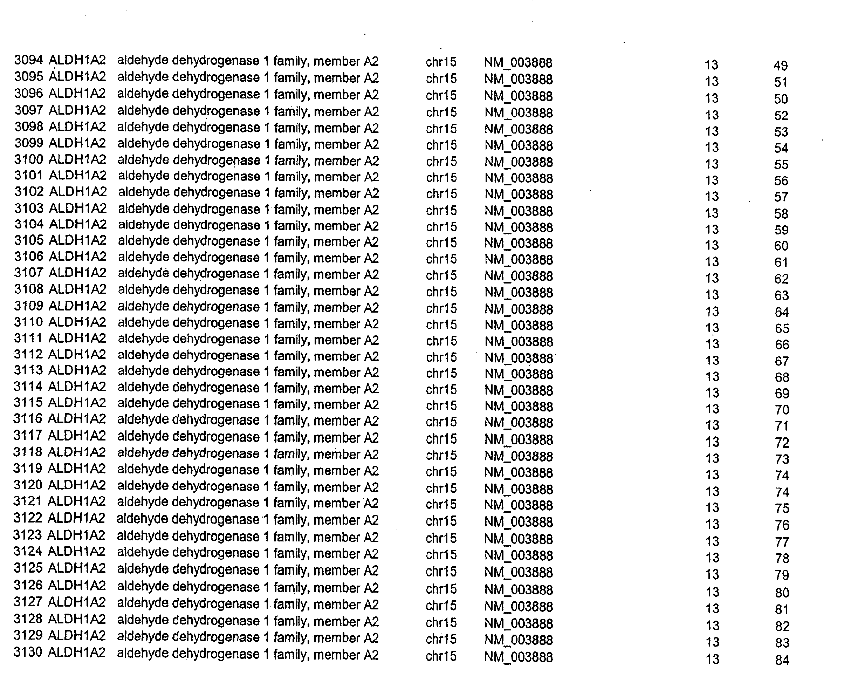

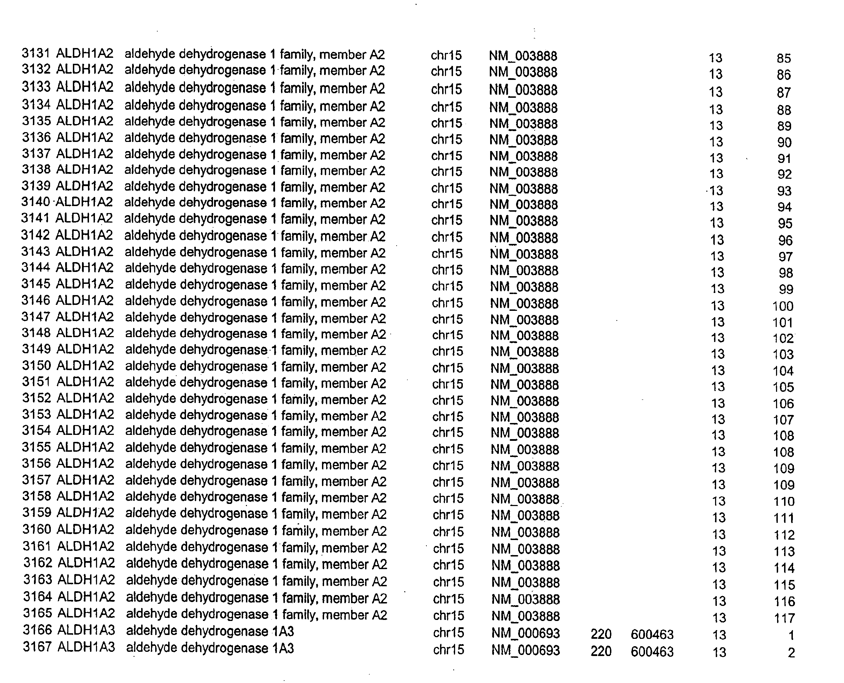

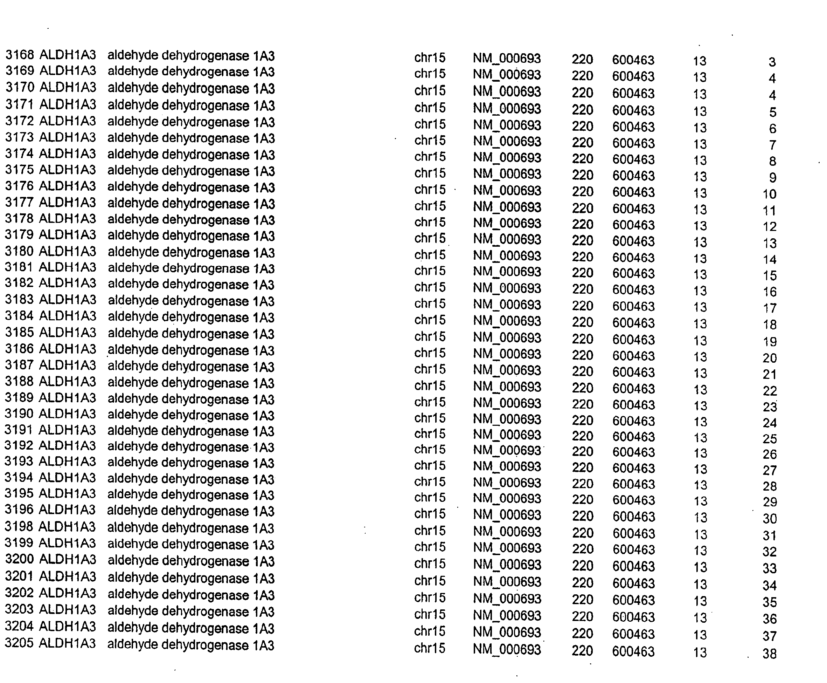

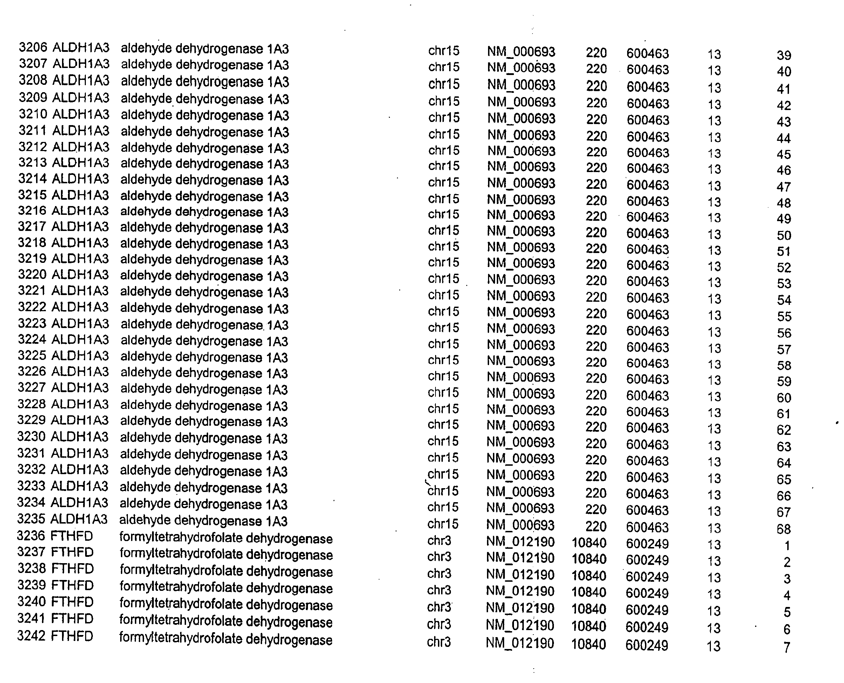

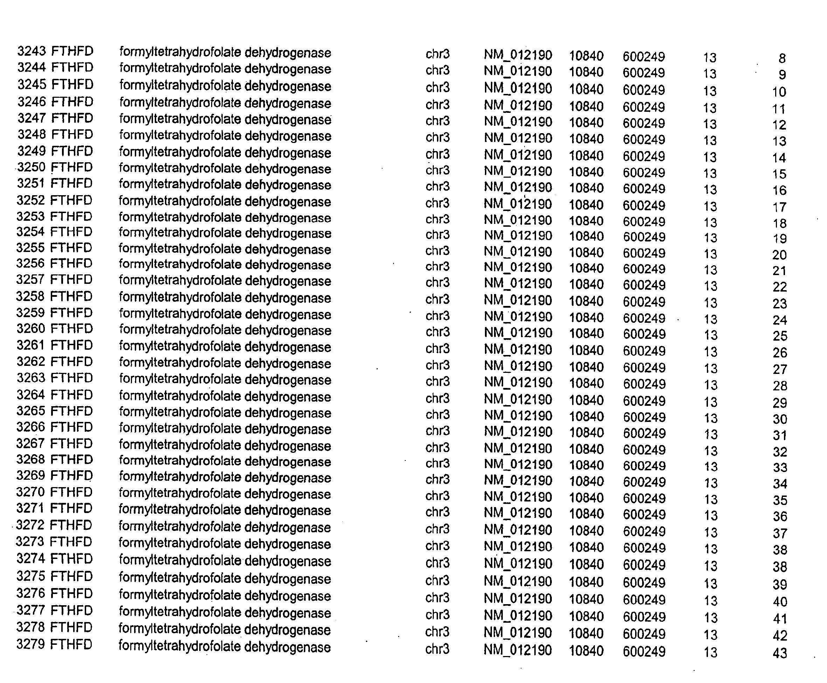

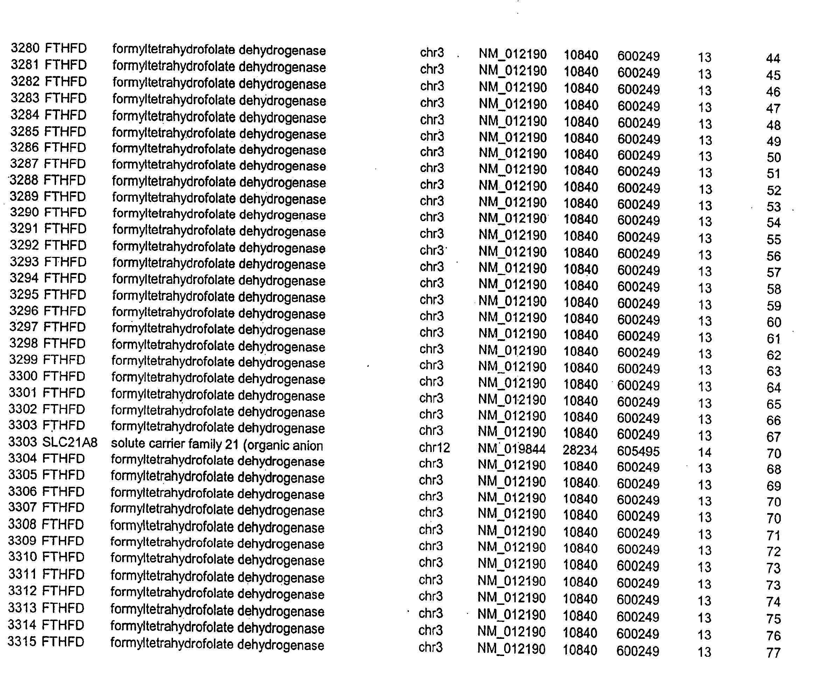

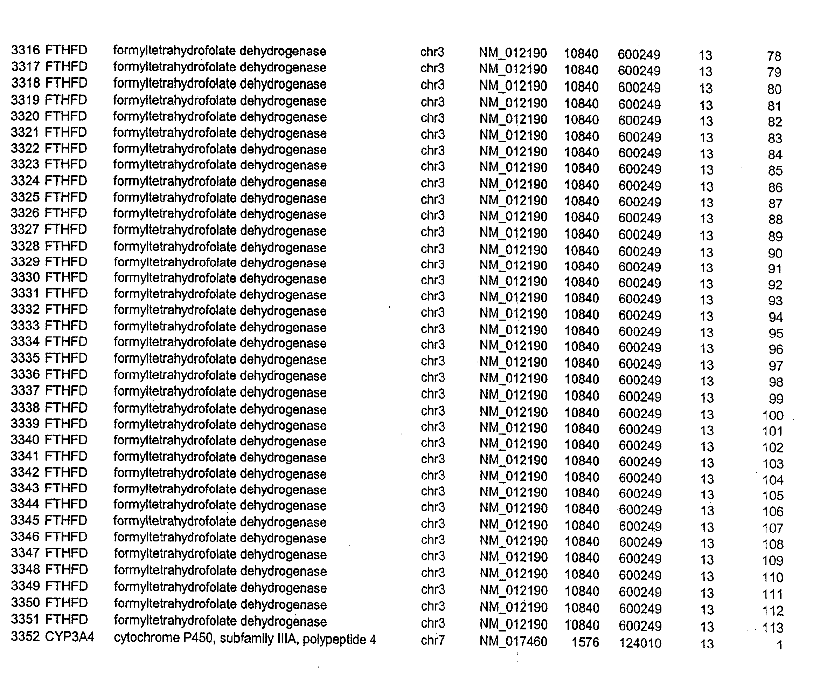

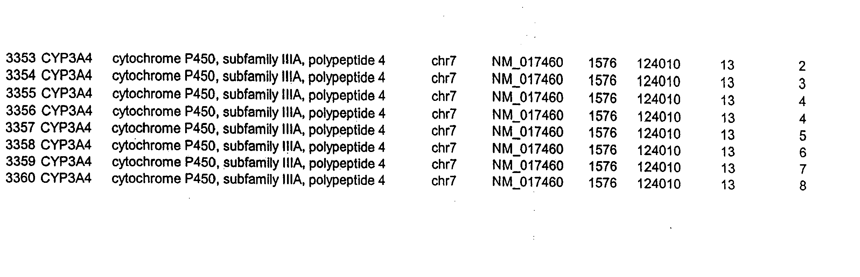

One example of information generated using SEQ ID Nos. 1-3360 and 3361-7669 and information in publicly available databases is provided in Figure 143. The first column in this figure shows that 3360 entries are made, corresponding to the 3360 entries found in Table 1. The second column, entitled "GENE" provides a gene name abbreviation, while the next column provides a long gene name. The next columns show the chromosome (CHROM), a reference mRNA accession number (REF. MRNA), a locus link database accession number (L-LINK), an OMIM database accession number (OMIM_ID) which allows disease association information to be readily obtained, the exon count for the gene (EXONS), and the number of polymorphisms in the gene (NO GENE).

### Creating an Oligonucleotide Probe or Oligonucleotide Primer

In some embodiments, an oligonucleotide used as a primer and/or probe in the detection method of the present invention serves as the template of the base sequences (Sequence No. 1 through 3360 and 3361 through 7669) shown in Table 1 if, for example, a SNP is to be detected. The primer/probe can be designed so it is synthesized as the base sequence itself or as a portion of the base sequence. In preferred embodiments, the SNP is included in the base sequence of the primer/probe (and denoted in capital letters in the base sequence column of Table 1). The primers/probes may also be complementary to the non-mutant sequence.

The SNP in the following example is designed so it is on the 3' or 5' end of the base sequence. It is designed to be within four bases of the 3' or 5' end, and ideally within two bases of the end. The SNP can also be in the center of the oligonucleotide base sequence. Here, "center" means the number of the bases from the SNP base to the 5' end is substantially equal to the number of bases from the SNP base to the 3' end. If there is an odd number of bases in the oligonucleotide, the central region should be essentially five bases in length, preferably three bases in length, and ideally one base in length. In a base sequence with 41 bases, for example, the central region should be bases 19 through 23, preferably bases 20 through 22, and ideally base 21. If there is an even number of bases, the central region should be four bases and ideally two bases. In a base sequence of 40 bases, for example, the central region should be bases 19 through 22 and ideally base 20.

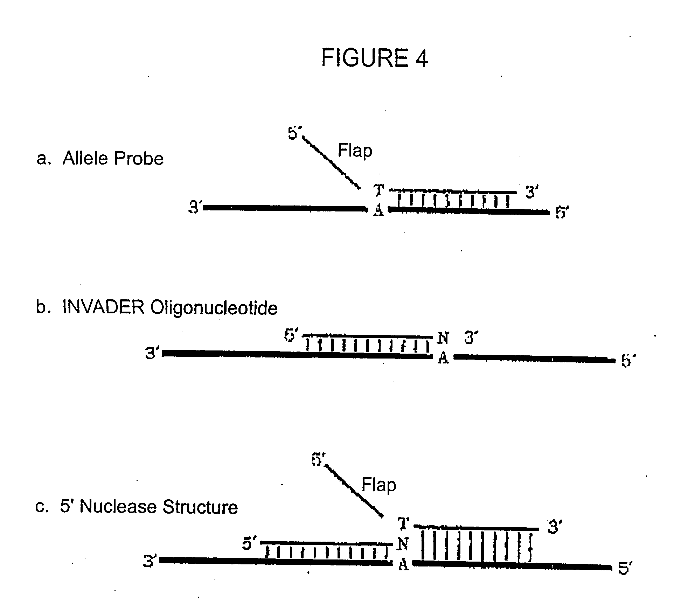

If the polymorphism consists of a plurality of bases, in some embodiments, the probe/primer is designed so the full polymorphism sequence is contained in the probe/primer. In some preferred embodiments, it is designed so one of the bases 1 through 4 on the 5' end or 3' end complementing the primer DNA corresponds to the base at the very end of the polymorphism bases. (This is called the "corresponding base"; ideally, it is the base at the 5' or 3' end). For example, in the INVADER assay, if a probe and INVADER oligonucleotide are prepared to detect a genetic polymorphism (CAGAGGCT) in No. 12 of NDUFA7 in Table 1 (Sequence No. 828), the position of the corresponding base in the probe in FIG 4a (a "T" base in the figure) is designed to become "C" at the far left of sequence CAGAGGCT, and the N base in the INVADER oligonucleotide shown in FIG 4b is designed to replace the "C" at the far left of CAGAGGCT with A, T, C or G). Conversely, if designed so the position of the corresponding base in the INVADER oligonucleotide is the far right "T" in CAGAGGCT, the "N" base is such that the corresponding base in the probe is "T." Further, the corresponding base of the INVADER oligonucleotide and the allele probe can be set anywhere in the CAGAGGCT sequence.

In preferred embodiments, the length of the base sequence is at least 13 bases, preferably between 13 and 60 bases, more preferably between 15 and 40 bases, and ideally between 18 and 30 bases. These oligonucleotide base sequences can be used as probes, as forward (sense) primers or as reverse (anti-sense) primers to detect target genes.

These oligonucleotides can link regions hybridized with genome DNA in tandem to unhybridized regions. The linking order can be upstream or downstream. The hybridized regions in these oligonucleotides can be designed from base sequence data containing the SNP described in Table 1, and created so the sequence containing the region hybridized with genome DNA closest to the 5' or 3' end is the SNP. These oligonucleotides can be used as probes to detect SNP using the INVADER assay.

The primer used in some embodiments of the present invention is designed to determine the functional change caused by the SNPs in the base sequences in Table 1, to determine whether the change is effective or ineffective, and to determine the existence of side effects. It is designed to include the SNP in the PCR-amplified base sequence. In some preferred embodiments, the primer should have at least 15 base sequences, preferably between 15 and 30 base sequences, and ideally between 18 and 24 base sequences. The template DNA regions in the primer base sequence should contain 500 bp or less amplified fragments, preferably between 100 and 300 bp fragments, and ideally between 100 and 150 bp fragments.

The oligonucleotide probes and primers designed in this manner can be synthesized chemically using any method commonly known in the art. For example, the oligonucleotides can be synthesized using a commercially available chemical synthesis device. The production of probes can be conducted automatically by adding fluorescent tags (e.g., FAM, VIC, Cy3) or other labels.

These oligonucleotides can be included in genetic polymorphism detection kits along with polymerase (e.g., Taq polymerase), a buffering solution (e.g., a Tris buffering solution), dNTP, fluorescent dyes (e.g., VIC, FAM), or other desired kit components.

### Detection

In some embodiments, the oligonucleotides prepared in the examples above are used as primers/probes, and the genes or a portion thereof (template DNA) encoding the drug metabolizing enzyme is amplified using DNA polymerase. A primer/probe prepared in this manner can be hybridized with template DNA and used to detect DNA with the target genetic polymorphism. The DNA used as the template can be prepared using any method commonly known in the art. Examples include cesium chloride density gradient ultra centrifugation method, the SDS solvency method or the phenol chloroform extraction method.

### 1 Detection Using PCR

The amplification can be performed using a polymerase chain reaction (PCR). The DNA polymerase can be LA Taq DNA polymerase (Takara), Ex Taq polymerase (Takara), Gold Taq polymerase (Perkin Elmer), AmpliTaq (Perkin Elmer) or Pfu DNA polymerase (Stratagene), as well as other polymerases.

An illustrative example of amplification conditions is provided below. The present invention is not limited to the conditions provided in this example. In preferred embodiments, each cycle in the transforming phase should last between 10 and 40 seconds at 85ºC to 105ºC and preferably 20 and 30 seconds at 94ºC, each cycle in the annealing phase should last 30 seconds to 1 minute at 50ºC to 72ºC and preferably 20 seconds to 1 minute at 60ºC, and each cycle in the elongation phase should last 1 minute to 4 minutes between 65ºC and 75ºC and preferably 2 minutes to 3 minutes at 72ºC. There should be 30 to 40 cycles, although fewer or more cycles are contemplated. In order to completely transform the template DNA and the primer, each cycle in the transforming phase should last 1 minute to 5 minutes at 95ºC before the amplifying cycle. If AMPLTTAQ GOLD polymerase manufactured by Applied Biosystems is used, it should last from 8 minutes to 15 minutes and ideally from 10 minutes to 12 minutes. In order to completely elongate the amplified DNA, the elongation phase should last between 1 minute and 10 minutes at 72ºC after the amplification cycle. If the amplified product is not immediately detected, it should be processed again at 4ºC to make sure the amplification was not irregular. In this way, the gene encoding the drug metabolizing enzyme is amplified.

After amplification, gel electrophoresis is performed on the amplified product, the amplified product is stained using ethidium bromide or SYBR Green, and one, two or three bands are detected in the amplified product (DNA fragments) to determine the portion (DNA fragment) of the drug metabolizing enzyme containing the genetic polymorphism in the gene encoding the drug metabolizing enzyme. Polyacrylamide gel electrophoresis or capillary electrophoresis can be performed instead of aerogel electrophoresis. PCR can be performed using a primer tagged with a fluorescent dye to detect the amplified product. A detection method that does not require electrophoresis can also be used, such as bonding the amplified product in solid phase to a microplate and detecting the amplified product using a fluorescent or enzymatic reaction.

### 2. Detection Using the TAQMAN PCR Method

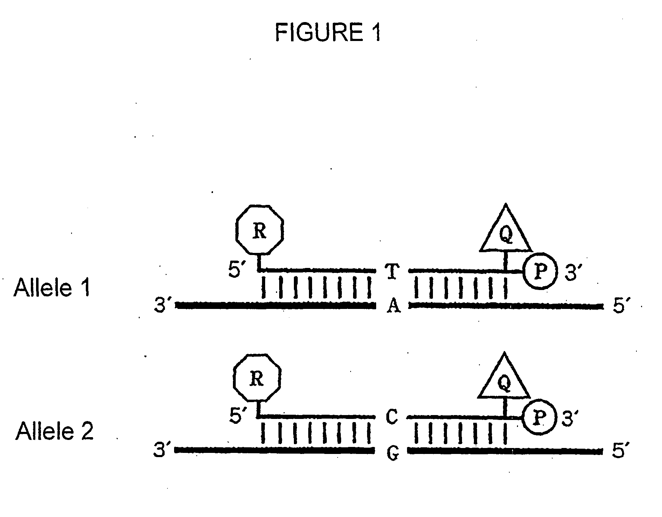

In the TAQMAN PCR method, the PCR reaction is performed using a fluorescent dye-tagged allele-specific oligo and Taq DNA polymerase. The allele-specific oligo used in the TAQMAN PCR method (TAQMAN probe) can be designed based on the SNP data. The 5' end of the TAQMAN probe is tagged using a fluorescent reporter dye R such as FAM or VIC, and the 3' end is tagged using a quencher Q (light-quenching substance). (See FIG 1.). Here, the fluorescent light energy absorbed by the quencher is not detected. Because the 3' end of the TAQMAN probe is phosphorylated, there is no elongation reaction from the TAQMAN probe in the PCR reaction (FIG 1). However, a PCR reaction is performed on the TAQMAN probe with TaqDNA polymerase and a primer designed to amplify the region containing the SNP. The following reaction occurs.

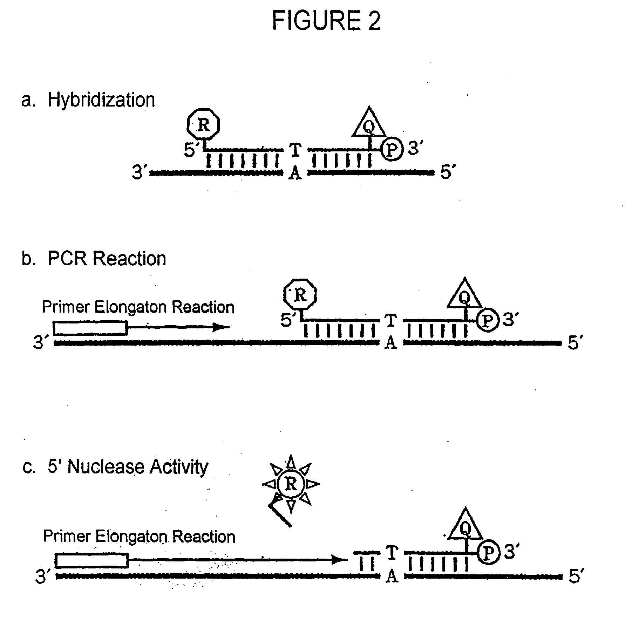

First, the TAQMAN probe is hybridized in a specific sequence of template DNA (FIG 2a) and an elongation reaction is simultaneously performed from the PCR primer (FIG 2b). Because the Taq DNA polymerase has 5' nuclease activity, the hybridized TAQMAN probe is severed as the PCR primer elongation reaction continues. When the TAQMAN probe is severed, the quencher has no effect on the fluorescent dye, and the fluorescent light is detected (FIG 2c).

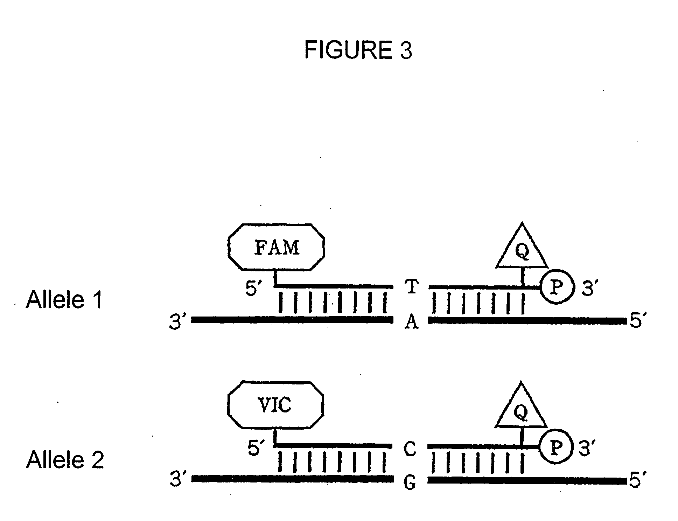

For example, suppose there is an A allele (Allele 1) and a G allele (Allele 2) at the SNP position as shown in FIG 3. Allele 1 is tagged by a specific TAQMAN probe with FAM and Allele 2 is tagged by a specific TAQMAN probe with VIC (see FIG 3). Two different allele-specific oligos are added to the PCR drug, and TAQMAN PCR is performed on the detected template. The fluorescence detector then detects the fluorescent intensity of the FAM and VIC. When the SNP position in the allele and the position corresponding to the SNP in the TAQMAN probe are complementary, the probe is hybridized with the allele, the fluorescent dye in the probe is severed by the Taq polymerase, the effect of the quencher is eliminated, and the intensity of the fluorescence is detected.

If the template is homozygous for Allele 1, strong FAM fluorescence is detected and hardly any VIC fluorescence is detected. If the template is hetermozygous for Allele 1 and Allele 2, both FAM and VIC fluorescence are detected.

### 3. SNP Detection Using the INVADER assay

In the INVADER assay, an allele-specific oligo and the template are hydridized to detect the SNP. In the INVADER assay, two different non-tagged oligos and one fluorescent dye-tagged oligo are used. One of the two non-tagged oligos is known as the probe. In some embodiments, the probe has a region hybridized to the genome DNA (template DNA) and a region (called a flap) that is not hydridized with the genome DNA, and that has a sequence unrelated to the sequence of the genomic DNA. The hybridized region has base sequences corresponding to the SNP (FIG 4a). The flap sequence is complementary to a FRET probe (described below). The other of the two non-tagged oligos is called the INVADER oligonucleotide. This oligonucleotide is designed so that it is hybridized in complementary fashion from the SNP position towards the 3' end of the genome DNA (FIG 4b). In some preferred embodiments, the sequence corresponding to the SNP position can be any base (denoted by N in FIG 4b). When the template DNA genome is hybridized with the two probes, the base (N) from the INVADER oligonucleotide is inserted in the SNP position (FIG 4c) forming a cleavage structure at the SNP position.

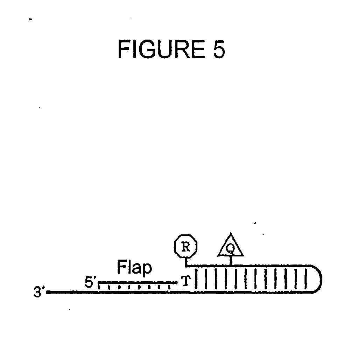

In some embodiments, the fluorescent dye-tagged oligonucleotide is a sequence completely unrelated to the alleles. This probe is a FRET (fluorescence resonance energy transfer) probe (FIG 5). The fluorescent dye R tags the base (reporter) at the 5' end of the FRET prove. A quencher Q absorbs the fluorescence. Here, the quencher absorbs the fluorescent light and the light is not detected. A specific region (Region 1) is designed on the 5' end of the FRET probe (reporter base) to face the 3' end from Region 1 (This region is Region 2). As a result, Region 1 and Region 2 form a complementary duplex (FIG 5). The 3'-region from the regions forming the complementary duplex can be hybridized with the flap of the allele probe to form a complementary chain (FIG 5).

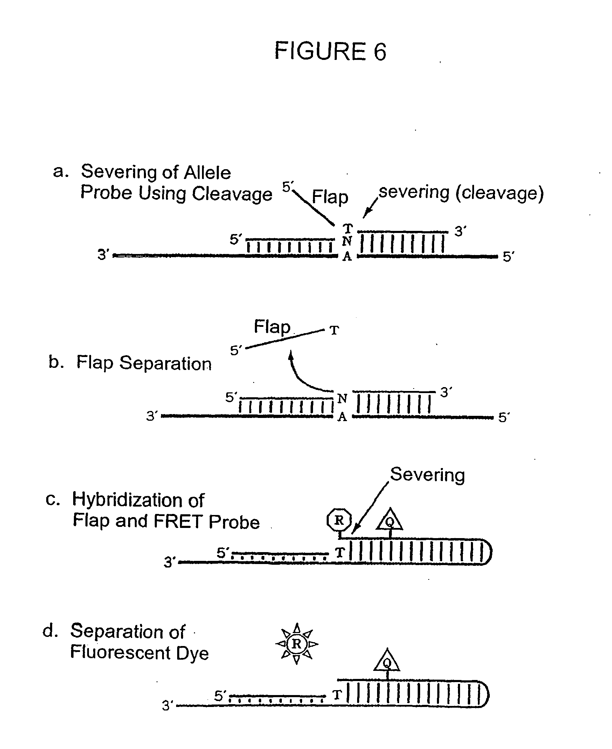

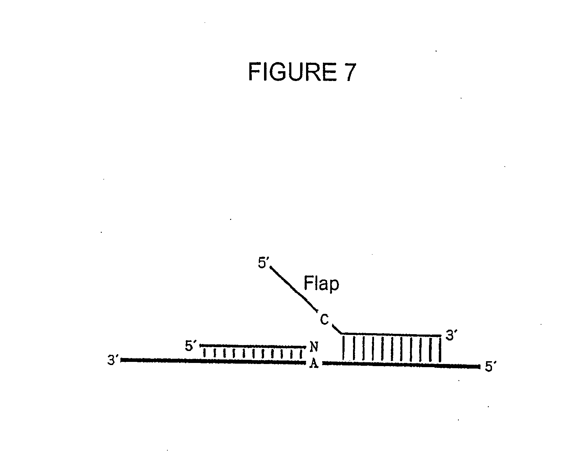

In the INVADER assay, a cleavage agent (e.g., CLEAVASE enzyme, Third Wave Technologies, Madison, WI) is used, which is an enzyme (5' nuclease) with specific endonuclease activity for identifying and cleaving a specific DNA structure. When the genome DNA, the probe and the INVADER oligonucleotide form a cleavage structure at the SNP position, the cleavage agent severs 3' of the SNP position on the allele probe. The section with three bases forming a flap with the 5' end is identified as shown in FIG 4c, and the flap is severed. The structure with the SNP position is identified by the cleavage agent (FIG 6a), the probe is severed at the flap position, and the flap is separated (FIG 6b). Next, the released flap from the probe bonds with the FRET probe in complementary fashion to form a duplex (FIG 6c). The cleavage agent identifies this structure and cleaves the section with the fluorescent dye. The cleaved fluorescent dye is no longer affected by the quencher and fluorescent light becomes detectable (FIG 6d). If the SNP position does not match the sequence corresponding to the SNP in the allele probe as shown in FIG 7, the specific DNA structure is not identified by the cleavage agent, the probe is not severed, and fluorescent light is not detected.

When the SNP is T/C, for example, a T INVADER oligonucleotide, a T probe, a FRET probe with FAM bonded to the reporter for the T SNP, a C INVADER oligonucleotide, a C probe and a FRET probe with VIC bonded to the reporter for the C SNP are prepared. These are combined and SNP detection is performed. If there is a T/T homo, FAM fluorescence is generated. If there is a C/C homo, VIC fluorescence is detected. If there is a T/C hetero, both FAM and VIC fluorescence are detected. Because the FAM and VIC fluorescence wavelengths are different, both can be readily identified.

### Detection Using the SniPer Method

In order to detect SNP using the SniPer method, an allele identifier is amplified using RCA. The genome DNA template is a straight chain, and a probe is hybridized with the genome DNA. When there is a complementary match between the probe sequence and the genome DNA template sequence and a complementary chain forms, a ligation reaction on the genome DNA forms a ring. As a result, RCA continues on cyclic DNA. If the end of the probe does not match the genome DNA, the RCA reaction does not occur because there is no ligation and no ring. In the SniPer method, therefore, a single chain probe is designed to anneal the genome DNA and create a ring. This single chain probe is called a padlock probe. The severed end of the padlock probe is the sequence corresponding to the target SNP. The padlock probe and the genome DNA mix and a ligation reaction occurs. If the severed end of the padlock probe and the SNP section of the genome DNA are complementary, the severed end of the padlock probe connects and forms a ring during the ligation reaction. If they are not complementary, a ring does not form. Therefore, only a padlock probe corresponding to the target SNP forms a ring and is amplified by the DNA polymerase. The presence of amplification is used to detect the SNP. A synthetic oligonucleotide with a hairpin structure and a fluorescent dye and quencher on both ends can be used in the detection process.

### Detection Using the MALDI-TOF/MS Method

In the matrix assisted laser desorption-time of flight/mass spectroscopy (MALDI-TOF/MS) method, SNP typing is performed using a mass spectrometer. A preferred embodiments of this method has the following steps.

### (i) PCR Amplification and Refinement of DNA Fragments Containing SNP

After making sure the base at the SNP location and the PCR primer do not overlap, the DNA fragment is amplified, exonuclease or alkali phosphatase processing is performed on the amplified product, the dNTP is removed, and the amplified fragment is refined.

### (ii) Primer Extension Reaction (Thermal Cycle) and Refinement

A primer ten or more times the template in the region identified as the PCR product is added, a thermal cycle reaction is performed, and a primer elongation reaction is performed. The primer used here is designed so the 3' end is next to the base corresponding to the SNP position. The primer length should be 15 to 30 bases, ideally 20 to 25 bases. If there is a multiplex reaction, a sequence that is not complementary to the template is added to the 5' end. There should be 20 to 30 (ideally 25) thermal cycles at two different temperatures. These should be 85 to 105ºC (ideally 94ºC) and 35 to 40ºC (ideally 37ºC).

The reaction product is then refined using a refining kit so it can be used in the mass spectrometer.

### (iii) Mass Spectroscopy Using a Mass Spectrometer

The elongated and refined reaction product is applied to the mass spectrometer, and a quality of the target product is measured. In other words, the refined product is mixed with the matrix and 0.5 to 1.0 mL spots are formed on the MALDI plate. After drying the plate, the substance is irradiated by a laser beam and a spectrogram is produced.

### Detection Using the Base Sequence Determining Method

In the present invention, a polymorphism can be detected using an elongation reaction on a single base. In other words, four different types of dideoxynucleotides identified by different fluorescent compounds are added to reaction systems including the gene to be detected and a single base elongation reaction is performed. Here, the base to be elongated is the polymorphism. Two reactions are performed; one to stop the DNA synthesis and another to identify the 3' end of the DNA molecule with fluorescence. Electrophoresis is performed on four different reaction solutions with the same lanes and capillaries for the sequencing gel. The sequence is determined by detecting the differences in the fluorescent dyes identifying the DNA bands using a fluorescence detector. The oligonucleotides with one base elongated have the elongation confirmed using different types of fluorescent dyes in a fluorescence detector and mass spectrometer. Instead of fluorescent-tagged dideoxynucleotides, the primer can be identified using fluorescence used with non-tagged dideoxynucleotides.

### Drug Evaluation

Using information obtained by the methods of the present invention, the efficacy and stability of the drug metabolized by the drug metabolizing enzyme can be evaluated.

For example, in some embodiments, the drug can be evaluated using a typing system. In other words, the frequency of expressed and unexpressed alleles (e.g., toxic alleles that cause undesired side effects) can be compared using any one of the detection methods mentioned above. Once they have been compared, markers can be selected to indicate, for example, a toxic expression where the allele frequency differs. In statistical analysis, this is usually set as x2. However, this is different in other methods such as the Fisher method. The active components (altered and metabolized drug components) in the drug will be reflected in blood and tissue concentrations. All of the genetic polymorphisms can be checked against the causes of the toxic effects to isolate specific correlating genetic polymorphisms. The substances corresponding to the probes or primers used to analyze all of the genetic polymorphisms are prepared beforehand on reaction plates, cards or glass plates, and unprepared human genome DNA is added and reacted to determine the allele pattern. If there are genetic polymorphisms correlating with toxicity or other phenotypes, then human side-effects can be expected or predicted. The same is true of drug effectiveness. Because the genetic polymorphisms correlating to effectiveness and side-effects differ depending on the drug, typing performed using genetic polymorphisms can be performed to anticipate effectiveness and side-effects.

Differences in allele frequency can be determined in certain instances by comparing the frequency of genetic polymorphisms to effectiveness/ineffectiveness or the presence/absence of side-effects. If, for example, an SNP analysis is performed on persons with a toxic reaction (side-effect) to Drug A, the results may show a 90% of the people have T/T (e.g., detected based on the intensity of fluorescent FAM light). The same results may show 10% of people with no toxic reaction have a T/T and 90% have a C/C. As a result of the SNP analysis, the evaluation may be not to administer Drug A to persons with T/T.

Exemplary drugs and drug-related data and other information that find use in or with the present invention, including but are not limited to the methods and databases described herein, are described in the PHYSICIANS' DESK REFERENCE (PDR). (e.g., 2002 Edition, Medical Economics Company, Inc., Montvale, NJ). The PDR is expressly incorporated by reference herein as if fully set forth.

### Drug Screening

In the present invention, the genetic polymorphism data obtained as described above is compared to genetic polymorphism data from genes encoding certain drug metabolizing enzymes to indicate the safety and effectiveness of drugs metabolized by these drug metabolizing enzymes. Therefore, the genetic polymorphism data obtained using the method of the present invention can be used to determine the likely effectiveness of certain drug therapies and to select the appropriate drug.

The evaluation methods described above can be used. Genetic polymorphisms with correlations to side-effects and effectiveness are said to be influenced by the activation, transfer and translation of certain enzymes. The cause and effect relationship with the side-effect or effectiveness expression mechanism may be indirect. The metabolization of drugs is being studied by pharmaceutical companies in laboratory and clinical testing. If there are genetic polymorphisms in enzyme genes correlating with severe side-effects, they can be removed and used under different conditions. The same is true of effectiveness. Drugs can be screened, therefore, using side-effects and effectiveness data. A wide variety of conditions and diseases (See e.g., Physician's Desk Reference) benefit from analysis using the systems and methods of the present invention.

In some embodiments of the present invention, a sample is taken from a subject (e.g., by a drug company) and sent to a laboratory for analysis using a detection assay. The laboratory results (e.g., detection assay test result data) is returned to the party providing the sample such that an appropriate decision can be made, including, but not limited to, development or administration of a drug to a subject.

In clinical testing (Tests I through III), the frequency of the expression of genetic polymorphisms can be studied in volunteers exhibiting certain side-effects and volunteers not exhibiting the same side-effects to a drug. In this way, novel genetic polymorphisms correlating with side-effects and effectiveness can be detected. This information can be used to screen drugs.

### EXPERIMENTAL EXAMPLES

The following examples are provided in order to demonstrate and further illustrate certain preferred embodiments and aspects of the present invention and are not to be construed as limiting the scope thereof.

### Example 1

### Obtaining SNP Data

### (1) DNA Extraction

Blood was extracted from 48 unrelated people in the presence of EDTA. The DNA was extracted in the following way based on the method in the Genome Analysis Manual (Yusuke Nakamura ed., Springer-Verlag Tokyo).

Ten milliliters of blood was transferred to a 50 ml test tube and centrifuged for five minutes at room temperature and 3000 rpm. After the supernatant (blood serum) had been removed using a pipette, 30 ml of RBC-dissolving buffer (10 mM NH4 HCO3, 144 mM NH3C1) was added. After mixing until there was no sediment, it was allowed to stand for 20 minutes at room temperature. After being centrifuged for five minutes at room temperature and 3000 rpm, the supernatant (blood serum) was again removed using a pipette to obtain white blood cells. Another 30 ml of RBC-dissolving buffer was added and the process was repeated twice. Then, 4 ml of proteinase K buffer [50 mM Tris-HCl (pH 7.4), 100 mM NaCl, 1 mM EDTA (pH 8.0)) was added to the white blood cells, 200 ml of SDS was added, 200 ml of 10 mg/ml proteinase K was added, and the solution was tumble-mixed. The solution was then allowed to stand overnight at 37°C. The next day, 4 ml of phenol was added, and the solution was slowly tumble-mixed for four hours using a Taitec T-50 Rotator. After being centrifuged for 10 minutes at room temperature and 3000 rpm, the supernatant was removed using a new tube. Then, 4 ml of phenolchloroform-isoamylalcohol (volume ratio 25:24:1) was added, the solution was tumble-mixed for two hours in the manner described above, and the solution was centrifuged. The supernatant was removed using a new tube, 4 ml of chloroform-isoamylalcohol (volume ratio 24:1) was added, and the solution was tumble-mixed. Fibrous white precipitate (DNA) was removed using a 2 ml tube, 1 ml of 70% ethanol was added, and the solution was tumble-mixed. The DNA was transferred to a new tube, dried and dissolved in 500 ml of TE solution [10 mM Tris-HCl (pH 4.7), 1 mM EDTA (pH 7.4)] to obtain a genome DNA sample.

### (2) PCR

A genome sequence was obtained from the GenBank DNA Database. After removing the repeating sequences using the RepMask computer program, the PCR primer was set so there would be approximately 1 kb of PCR product. The genome DNA from 48 unrelated people was prepared at the same concentration. After mixing the same amount of DNA from three people in a single tube, 60 ng was used in the PCR. The PCR was Ex-Taq (Takara 2.5 U) and performed using the GeneAmp PCR System 9700 (PE Applied Biosystems). After reacting for two hours at 94°C, denaturing was performed for 30 seconds at 96ºC, annealing was performed for 30 seconds at 55ºC or 60ºC, and elongation was performed for one minute at 72ºC in each cycle. There were 35 cycles.

### (3) Sequence

After refining the PCR product using Arraylt (Telechem), the sequence reaction was performed using the BigDye Terminator RR Mix (PE Applied Biosystems). After reacting for two hours at 96ºC, denaturing was performed for 20 seconds at 96ºC, annealing was performed for 30 seconds at 50ºC, and elongation was performed for 4 minutes at 60ºC in each cycle using the GeneAmp PCR System 9700 (PE Applied Biosysytems). There were 25 cycles. After the sequencing reaction, the sequencing was analyzed using the ABI Prism 3700 DNA Analyzer.

### (4) SNP Detection

An analysis was performed on the SNP detection using the PolyPhred computer program (Nickerson et al., 1997, Nucleic Acid Res., 25, 2745-2751).

### (5) Results

The SNP results shown in Table 1 were obtained. The analyzed drug metabolizing enzyme, the abbreviation of the enzyme, the databank (GenBank) accession number, the structure of the gene for the drug metabolizing enzyme, and the position of the SNPs are shown in FIG 9 through FIG 141 and FIG 144 through 312. In FIG 9 through FIG 141 and FIG 144 through 312, the exons are blank boxes or black lines in the genes denoted by the horizontal lines. The position of the SNPs is denoted above the genes with solid lines and numbers.

### Example 2

### Typing

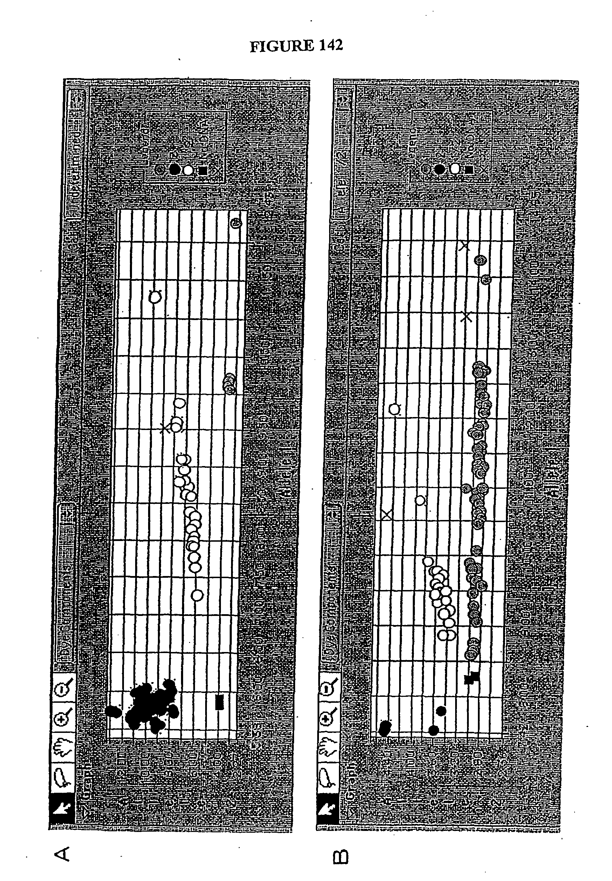

Typing was performed on two different groups of patients using the INVADER assay. In FIG 142, the x-axis (Allele 1) indicates the intensity of the FAM fluorescent light corresponding to T, and the x-axis (Allele 2) indicates the intensity of the VIC fluorescent light corresponding to C. The slanted line indicates the SNP pattern for T/T, the black circles denote the pattern for C/C, and the white circles denote the pattern for T/C. The black squares indicate the background values. The x marks indicate where the detection failed. The group of patients in the graph for panel A (top) had many C/C SNP patterns and the group of patients in the graph for panel B (bottom) had many T/T SNP patterns.

### Example 3

### SNP Detection

Genome DNA was extracted from five unrelated people using the method described in Example 1, and the SNPs in three different drug metabolizing enzyme genes (EPHX1, ABCB2, AANAT) were detected using the INVADER assay method. The INVADER oligonucleotides and probes were designed using base sequence No. 3 (Sequence No. 49) and No. 17 (Sequence No. 63) in the case of EPHX1, base sequence No. 4 (Sequence No. 4) and No. 11 (Sequence No. 11) in the case of ABCB2, and base sequence No. 3 (Sequence No. 561) in the case of AANAT. The positions of the SNPs are shown in Table 1.
The results are shown in Table 2.

**Table 2**

| Drug Metabolizing Enzyme Gene | EPHX1 | | ABCB2 | | AANAT |
|---|---|---|---|---|---|
| | No. 3 | No. 17 | No. 4 | No. 11 | No. 3 |
| | Seq. No. 49 | Seq. No. 63 | Seq. No. 4 | Seq. No. 11 | Seq. No. 561 |
| SNP | (T/G) | (A/G) | (G/T) | (G/A) | (T/A) |
| Subject I | T/T | A/G | T/T | G/A | T/T |
| Subject II | T/T | A/A | G/G | G/G | T/A |
| Subject III | T/G | A/A | G/G | A/A | T/T |
| Subject IV | G/G | A/G | G/T | G/G | T/T |
| Subject V | T/G | A/G | G/T | G/A | T/A |

As shown in Table 2, the SNPs in the drug metabolizing genes of patients can be detected and the patterns determined using the method of the present invention.

### Example 4

### Correlation between SNP genotypes and optimal amounts of a medicament for treatment validity and safety

In this example, validity and safety of medicaments were investigated using SNP analysis.

Thiopurine S-methyltransferase (TPMT) is an enzyme that transfers a methyl group to a sulfur atom attached to a purine ring, and is one of the major enzymes for metabolizing drugs such as the anti-cancer agents 6-mercaptopurine and 6-thioguanine, and thiopurine derivatives such as the immunosuppressive agent azathioprine. This example shows a correlation between optimal amounts of azathioprine and various combinations of the alleles at the 868^{th} SNP of intron 3 of TPMT(Accession No. AB045146.1) (G or T alleles) and the 2682^{nd} SNP of intron 3 (C or A alleles)(Table 3 and Table 4).

**Table 3**

| 868 | 2682 | High | Low |
|---|---|---|---|
| TT | AA | 2 | 0 |
| TT | AT | 3 | 0 |
| TT | TT | 1 | 0 |
| GT | AA | 0 | 2 |
| GT | AT | 1 | 7 |
| GT | TT | 4 | 1 |
| GG | AA | 1 | 0 |
| GG | AT | 0 | 1 |
| GG | TT | 1 | 0 |

Optimal amounts of azathioprine were determined by adopting suppression of rejection after renal transplantation as an index. A group of patients in which the validity of treatment with 100 mg/day of azathioprine was confirmed was designated as a high dose group, and a group of patients in which side effects developed with treatment of 100 mg/day, but in which validity was confirmed with a treatment of 50 mg/day was designated as a low dose group. Table 3 indicates the number of patients having each combination of alleles, with the columns labeled "High" and "Low" representing the numbers of patients of each genotype in the high dose and the low dose groups, respectively. Side effects include leukopenia, anthema, angiitis, nausea/vomiting, anorexia, diarrhea, malaise, myalgia, arthralgia, fever, chill, and dizziness. More serious side effects include, for example, blood disorders, shock-like symptoms, infectious diseases, and hepatic disorders, and renal disorders.

Investigation of a correlation between the high dose and low dose groups and the two types of SNPs indicated above revealed that when G is present in at least one allele at the 868^{th} SNP of intron 3 (G/G homozygous or G/T heterozygous) and A is present in at least one allele at the 2682^{nd} SNP of intron 3 (A/A homozygous or A/T heterozygous), side effects were developed with 100 mg/day and 50 mg/day was an optimal amount for 10 out of 12 patients (low dose group), while 100 mg/day was an optimal amount for 11 out of 12 patients with other allele combinations (high dose group) (Table 4). Investigation of this combination of two SNP loci in patients enables prediction of optimal amounts of azathioprine for treatment prior to the administration of the drug, for improved validity and safety. These results indicate that the validity and safety of medicaments can be predicted using analysis of SNPs associated with medicament metabolic enzymes, e.g., as described in this specification and including but not limited to the DME-associated SNPs listed in Table 1. As used in this example only, the term "optimal amount" refers to the best dosage selected from the tested amounts of 50 mg/day or 100/mg per day. It will be appreciated by those skilled in the art that a study testing additional amounts of a medicament (e.g., a study in which amounts are varied in smaller increments, such as 40, 50, 60, 70, 80, 90, etc. mg/day) would provide additional information regarding ranges of amounts giving optimal performance for patients having a particular genotype, and that optimal amounts of this or any other medicament are not limited to the particular amounts of 50 or 100 mg/day tested in this example.

**Table 4**

| Genotype | Optimal amount | |
|---|---|---|
| | 100 mg/day | 50 mg/day |
| G as the 868^{th} SNP and A as the 2682^{nd} | 2 | 10 |
| Other combinations | 11 | 1 |

| | | |
|---|---|---|
| (Fisher exact test: p=0.0003) | | |

### Sequence Listing Free Text

SEQ ID NO 3510 : n represents at or deletion (Location 21).
SEQ ID NO 3512 : n represents c or deletion (Location 21).
SEQ ID NO 3513 : n represents t or deletion (Location 21).
SEQ ID NO 3514 : n represents t or deletion (Location 21).
SEQ ID NO 3515 : n represents g or deletion (Location 21).
SEQ ID NO 3517 : n represents c or deletion (Location 21).
SEQ ID NO 3519 : n represents t or deletion (Location 21).
SEQ ID NO 3521 : n represents c or deletion (Location 21).
SEQ ID NO 3649 : n represents 14 to 16 repeats of tca (from Location 21).
SEQ ID NO 3650 : n represents 8 to 10 repeats of a (from Location 21).
SEQ ID NO 3651 : n represents cacagtcat or deletion (Location 21).
SEQ ID N03652 : n represents tt or deletion (Location 21).
SEQ ID NO 3653 : n represents 10 to 12 repeats of a (from Location 21).
SEQ ID NO 3654 : n represents c or deletion (Location 21).
SEQ ID NO 3655 : n represents 16 to 18 repeats of a (from Location 21).
SEQ ID NO 3656 : n represents g or deletion (Location 21).
SEQ ID NO 3658 : n represents c or deletion (Location 21).
SEQ ID NO 3659 : n represents t or deletion (Location 21).
SEQ ID NO 3660 : n represents a or deletion (Location 21).
SEQ ID NO 3661 : n represents tg or deletion (Location 21).
SEQ ID NO 3663 : n represents 10 to 13 repeats of t (from Location 21).
SEQ ID NO 3664 : n represents 11 to 13 repeats of gt (from Location 21).
SEQ ID NO 3665 : n represents a or deletion (Location 21).
SEQ ID NO 3666 : n represents g or deletion (Location 21).
SEQ ID NO 3667 : n represents g or deletion (Location 21).
SEQ ID NO 3668 : n represents 9 to 11 repeats of t (from Location 21).
SEQ ID NO 3669 : n represents g or deletion (Location 21).
SEQ ID NO 3671 : n represents tt or deletion (Location 21).
SEQ ID NO 3672 : n represents 7 to 9 repeats of a (from Location 21).
SEQ ID NO 3673 : n represents 9 to 11 repeats of t (from Location 21).
SEQ ID NO 3674 : n represents 9 to 10 repeats of a (from Location 21).
SEQ ID NO 3675 : n represents gt or deletion (Location 21).
SEQ ID NO 3676 : n represents a or deletion (Location 21).
SEQ ID NO 3677 : n represents t or deletion (Location 21).
SEQ ID NO 3679 : n represents a or deletion (Location 21).
SEQ ID NO 3680 : n represents ct or deletion (Location 21).
SEQ ID NO 3681 : n represents g or deletion (Location 21).
SEQ ID NO 3682 : n represents a or deletion (Location 21).
SEQ ID NO 3683 : n represents a or deletion (Location 21).
SEQ ID NO 3684 : n represents a or deletion (Location 21).
SEQ ID NO 3685 : n represents c or deletion (Location 21).
SEQ ID NO 3686 : n represents aaag or deletion (Location 21).
SEQ ID NO 3751 : n represents 22 to 26 repeats of t (from Location 21).
SEQ ID NO 3752 : n represents 8 to 10 repeats of g (from Location 21).
SEQ ID NO 3753 : n represents 6 to 7 repeats of c (from Location 21).
SEQ ID NO 3754 : n represents 12 to 14 repeats of a (from Location 21).
SEQ ID NO 3833 : n represents tt or deletion (Location 21).
SEQ ID NO 3834 : n represents 9 to 11 repeats of a (from Location 21).
SEQ ID NO 3835 : n represents 8 to 12 repeats of a (from Location 21).
SEQ ID NO 3836 : n represents t or deletion (Location 21).
SEQ ID NO 3837 : h represents t or deletion (Location 21).
SEQ ID NO 3838 : n represents t or deletion (Location 21).
SEQ ID NO 3839 : n represents a or deletion (Location 21).
SEQ ID NO 3840 : n represents t or deletion (Location 21).
SEQ ID NO 3841 : n represents t or deletion (Location 21).
SEQ ID NO 3842 : n represents 11 to 15 repeats of t (from Location 21).
SEQ ID NO 3843 : n represents cat or deletion (Location 21).
SEQ ID NO 3844 : n represents t or deletion (Location 21).
SEQ ID NO 3845 : n represents a or deletion (Location 21).
SEQ ID NO 3846 : n represents a or deletion (Location 21).
SEQ ID NO 3847 : n represents t or deletion (Location 21).
SEQ ID NO 3848 : n represents a or deletion (Location 21).
SEQ ID NO 3857 : n represents g or deletion (Location 21).
SEQ ID NO 3879 : n represents a or deletion (Location 21).
SEQ ID NO 3885 : n represents aaag or deletion (Location 21).
SEQ ID NO 3915 : n represents t or deletion (Location 21).
SEQ ID NO 3918 : n represents a or deletion (Location 21).
SEQ ID NO 3926 : n represents at or deletion (Location 21).
SEQ ID NO 3933 : n represents a or deletion (Location 21).
SEQ ID NO 3950 : n represents c or deletion (Location 21).
SEQ ID NO 3953 : n represents gg or deletion (Location 21).
SEQ ID NO 3962 : n represents gtc or deletion (Location 21).
SEQ ID NO 3984 : n represents t or deletion (Location 21).
SEQ ID NO 3991 : n represents tt or deletion (Location 21).
SEQ ID NO 3994 : n represents 9 to 12 repeats of t (from Location 21).
SEQ ID NO 3996 : n represents a or deletion (Location 21).
SEQ ID NO 3998 : n represents 10 to 13 repeats of a (from Location 21).
SEQ ID NO 4001 : n represents ct or deletion (Location 21).
SEQ ID NO 4004 : n represents cagatcttcttcagctaatttagaaatgt or deletion (Location 21).
SEQ ID NO 4030 : n represents a or deletion (Location 21).
SEQ ID NO 4037 : n represents c or deletion (Location 21).
SEQ ID NO 4042 : n represents t or deletion (Location 21).
SEQ ID NO 4049 : n represents 9 to 12 repeats of t (from Location 21).
SEQ ID NO4052 : n represents t or deletion (Location 21).
SEQ ID NO 4054 : n represents g (a)4, a (a)4 or a (Location 21).
SEQ ID NO 4058 : n represents t or deletion (Location 21).
SEQ ID NO 4067 : n represents c or deletion (Location 21).
SEQ ID NO 4069 : n represents a or deletion (Location 21).
SEQ ID NO 4070 : n represents c or deletion (Location 21).
SEQ ID NO 4077 : n represents g or deletion (Location 21).
SEQ ID NO 4079 : n represents 18 to 20 repeats of t (from Location 21).
SEQ ID NO 4084 : n represents 11 to 13 repeats of a (from Location 21).
SEQ ID NO 4085 : n represents gaaa or deletion (Location 21).
SEQ ID NO 4089 : n represents 10 to 12 repeats of a (from Location 21).
SEQ ID NO 4092 : n represents c or deletion (Location 21).
SEQ ID NO 4102 : n represents ca or deletion (Location 21).
SEQ ID NO 4109 : n represents at or deletion (Location 21).
SEQ ID NO 4113 : n represents ctt or deletion (Location 21).
SEQ ID NO 4115 : n represents g or deletion (Location 21).
SEQ ID NO 4117 : n represents ggggct or deletion (Location 21).
SEQ ID NO 4121 : n represents 19 to 22 repeats of t (from Location 21).
SEQ ID NO 4126 : n represents 6 to 7 repeats of t (from Location 21).
SEQ ID NO 4129 : n represents 11 to 13 repeats of t (from Location 21).
SEQ ID NO 4173 : n represents 7 to 8 repeats of c (from Location 21).
SEQ ID NO 4175 : n represents 10 to 12 repeats of a (from Location 21).
SEQ ID NO 4183 : n represents c or deletion (Location 21).
SEQ ID NO 4188 : n represents aaga or deletion (Location 21).
SEQ ID NO 4190 : n represents 9 to 11 repeats of a (from Location 21).
SEQ ID NO 4193 : n represents ct or deletion (Location 21).
SEQ ID NO 4198 : n represents 8 to 9 repeats of t (from Location 21).
SEQ ID NO 4218 : n represents g or deletion (Location 21).
SEQ ID NO 4224 : n represents cttt or deletion (Location 21).
SEQ ID NO 4229 : n represents t or deletion (Location 21).
SEQ ID NO 4234 : n represents c or deletion (Location 21).
SEQ ID NO 4235 : n represents a or deletion (Location 21).
SEQ ID NO 4238 : n represents gtt or deletion (Location 21).
SEQ ID NO 4239 : n represents t or deletion (Location 21).
SEQ ID NO 4259 : n represents at or deletion (Location 21).
SEQ ID NO 4273 : n represents g or deletion (Location 21).
SEQ ID NO 4280 : n represents 15 to 17 repeats of a (from Location 21).
SEQ ID NO 4294 : n represents t or deletion (Location 21).
SEQ ID NO 4298 : n represents t or deletion (Location 21).
SEQ ID NO 4310 : n represents t or deletion (Location 21).
SEQ ID NO 4314 : n represents a or deletion (Location 21).
SEQ ID. NO 4315 : n represents 13 to 15 repeats of t (from Location 21).
SEQ ID NO 4316 : n represents 12 to 13 repeats of a (from Location 21).
SEQ ID NO 4317 : n represents t or deletion (Location 21).
SEQ ID NO 4319 : n represents t or deletion (Location 21).
SEQ ID NO 4320 : n represents 13 to 15 repeats of a (from Location 21).
SEQ ID NO 4325 : n represents a or deletion (Location 21).

SEQ ID NO 4331 : n represents 5 to 11 repeats of t (from Location 21).
SEQ ID NO 4333 : n represents 8 to 9 repeats of t (from Location 21).
SEQ ID NO 4334 : n represents t or deletion (Location 21).
SEQ ID NO 4345 : n represents 9 to 10 repeats of t (from Location 21).
SEQ ID NO 4348 : n represents 10 to 11 repeats of a (from Location 21).
SEQ ID NO 4354 : n represents a or deletion (Location 21).
SEQ ID NO 4361 : n represents a or deletion (Location 21).
SEQ ID NO 4372 : n represents ct or deletion (Location 21).
SEQ ID NO 4391 : n represents t or deletion (Location 21).
SEQ ID NO 4397 : n represents a or deletion (Location 21).
SEQ ID NO 4398 : n represents at or deletion (Location 21).
SEQ ID NO 4408 : n represents tgtccaaaggaaggacacg or deletion (Location 21).
SEQ ID NO 4414 : n represents 6 to 8 repeats of tc (from Location 21).
SEQ ID NO 4416 : n represents c or deletion (Location 21).
SEQ ID NO 4419 : n represents t or deletion (Location 21).
SEQ ID NO 4424 : n represents t or deletion (Location 21).
SEQ ID NO 4425 : n represents c or deletion (Location 21).
SEQ ID NO 4433 : n represents a or deletion (Location 21).
SEQ ID NO 4435 : n represents t or deletion (Location 21).
SEQ ID NO 4442 : n represents 6 to 7 repeats of gatt (from Location 21).
SEQ ID NO 4443 : n represents t or deletion (Location 21).
SEQ ID NO 4448 : n represents t or deletion (Location 21).
SEQ ID NO 4449 : n represents gt or deletion (Location 21).
SEQ ID NO 4452 : n represents a or deletion (Location 21).
SEQ ID NO 4453 : n represents a or deletion (Location 21).
SEQ ID NO 4457 : n represents t or deletion (Location 21).
SEQ ID NO 4460 : n represents at or deletion (Location 21).
SEQ ID NO 4466 : n represents a or deletion (Location 21).
SEQ ID NO 4469 : n represents t or deletion (Location 21).
SEQ ID NO 4472 : n represents at or deletion (Location 21).
SEQ ID NO 4473 : n represents a or deletion (Location 21).
SEQ ID NO 4474 : n represents 12 to 14 repeats of t (from Location 21).
SEQ ID NO 4477 : n represents t or deletion (Location 21).
SEQ ID NO 4479 : n represents cac or deletion (Location 21).
SEQ ID NO 4486 : n represents cca or deletion (Location 21).
SEQ ID NO 4514 : n represents t or deletion (Location 21).
SEQ ID NO 4544 : n represents c or deletion (Location 21).
SEQ ID NO 4552 : n represents aaaa or deletion (Location 21).
SEQ ID NO 4565 : n represents c or deletion (Location 21).
SEQ ID NO 4575 : n represents 8 to 9 repeats of t (from Location 21).
SEQ ID NO 4576 : n represents a or deletion (Location 21).
SEQ ID NO 4588 : n represents taac or deletion (Location 21).
SEQ ID NO 4589 : n represents ctcttt or deletion (Location 21).
SEQ ID NO 4590 : n represents ct or deletion (Location 21).
SEQ ID NO 4597 : n represents a or deletion (Location 21).
SEQ ID NO 4600 : n represents t or deletion (Location 21).
SEQ ID NO 4603 : n represents g or deletion (Location 21).
SEQ ID NO 4606 : n represents aattagaa or deletion (Location 21).
SEQ ID NO 4607 : n represents tttaaaa or ttttaa (Location 21).
SEQ ID NO 4610 : n represents t or deletion (Location 21).
SEQ ID NO 4615 : n represents t or deletion (Location 21).
SEQ ID NO 4627 : n represents c or deletion (Location 21).
SEQ ID NO 4652 : n represents 11 to 14 repeats of t (from Location 21).
SEQ ID NO 4653 : n represents t or deletion (Location 21).
SEQ ID NO 4654 : n represents 10 to 13 repeats of t (from Location 21).
SEQ ID NO 4655 : n represents t or deletion (Location 21).
SEQ ID NO 4657 : n represents t or deletion (Location 21).
SEQ ID NO 4658 : n represents ta or deletion (Location 21).
SEQ ID NO 4660 : n represents 13 to 15 repeats of t (from Location 21).
SEQ ID NO 4661 : n represents c or deletion (Location 21).
SEQ ID NO 4662 : n represents 17 to 20 repeats of a (from Location 21).
SEQ ID NO 4663 : n represents 11 to 13 repeats of t (from Location 21).
SEQ ID NO 4664 : n represents 8 to 9 repeats of t (from Location 21).
SEQ ID NO 4665 : n represents 10 to 11 repeats of a (from Location 21).
SEQ ID NO 4666 : n represents 16 to 19 repeats of a (from Location 21).
SEQ ID NO 4758 : n represents g or deletion (Location 21).
SEQ ID NO 4760 : n represents 6 to 7 repeats of a (from Location 21).
SEQ ID NO 4761 : n represents c or deletion (Location 21).
SEQ ID NO 4763 : n represents tcctcaggg or deletion (Location 21).
SEQ ID NO 4764 : n represents 8 to 10 repeats of cgc (from Location 21).
SEQ ID NO 4765 : n represents 10 to 12 repeats of a (from Location 21).
SEQ ID NO 4766 : n represents caccaggcagcagactctgatgaggaggggaggggg or deletion (Location 21).
SEQ ID NO 4768 : n represents g or deletion (Location 21).
SEQ ID NO 4808 : n represents tcac or deletion (Location 21).
SEQ ID NO 4809 : n represents t or deletion (Location 21).
SEQ ID NO 4810 : n represents 9 to 11 repeats of t (from Location 21).
SEQ ID NO 4811 : n represents 7 to 8 repeats of a (from Location 21).
SEQ ID NO 4847 : n represents agg or deletion (Location 21).
SEQ ID NO 4848 : n represents taacatt or deletion (Location 21).
SEQ ID NO 4849 : n represents 10 to 12 repeats of a (from Location 21).
SEQ ID NO 4850 : n represents 15 to 17 repeats of t (from Location 21).
SEQ ID NO 4851 : n represents 11 to 13 repeats of a (from Location 21).
SEQ ID NO 4877 : n represents 11 to 13 repeats of t (from Location 21).
SEQ ID NO 4878 : n represents t or deletion (Location 21).
SEQ ID NO 4879 : n represents t or deletion (Location 21).
SEQ ID NO 4880 : n represents 10 to 12 repeats of a (from Location 21).
SEQ ID NO 4881 : n represents t or deletion (Location 21)
SEQ ID NO 4883 : n represents 7 to 9 repeats of c (from Location 21).
SEQ ID NO 4884 : n represents a or deletion (Location 21)
SEQ ID NO 4891 : n represents 13 to 16 repeats of t (from Location 21).
SEQ ID NO 4892 : n represents 9 to 10 repeats of t (from Location 21).
SEQ ID NO 4893 : n represents 14 to 16 repeats of t (from Location 21).
SEQ ID NO 4894 : n represents 13 to 17 repeats of t (from Location 21).
SEQ ID NO 4895 : n represents t or deletion (Location 21).
SEQ ID NO 4897 : n represents 8 to 9 repeats of a (from Location 21).
SEQ ID NO 4898 : n represents 8 to 9 repeats of t (from Location 21).
SEQ ID NO 4899 : n represents gcagtattactgtagt or deletion (Location 21).
SEQ ID NO 4900 : n represents 13 to 14 repeats of t (from Location 21).
SEQ ID NO 4901 : n represents 9 to 10 repeats of t (from Location 21).
SEQ ID NO 4902 : n represents 10 to 11 repeats of t (from Location 21).
SEQ ID NO 4907 : n represents 10 to 14 repeats of a (from Location 21).
SEQ ID NO 4908 : n represents 13 to 15 repeats of a (from Location 21).
SEQ ID NO 4909 : n represents a or deletion (Location 21).
SEQ ID NO 4910 : n represents t or deletion (Location 21).
SEQ ID NO 4918 : n represents 13 to 15 repeats of a (from Location 21).
SEQ ID NO 4919 : n represents 12 to 15 repeats of a (from Location 21).
SEQ ID NO 4936 : n represents g or deletion (Location 21).
SEQ ID NO 4938 : n represents aa or deletion (Location 21).
SEQ ID NO 4983 : n represents a or deletion (Location 21).
SEQ ID NO 4985 : n represents aa or deletion (Location 21).
SEQ ID NO 4986 : n represents ca or deletion (Location 21).
SEQ ID NO 4987 : n represents t or deletion (Location 21).
SEQ ID NO 4988 : n represents tgtgtg or deletion (Location 21).
SEQ ID NO 5076 : n represents a or deletion (Location 21).
SEQ ID NO 5078 : n represents g or deletion (Location 21).
SEQ ID NO 5080 : n represents actt or deletion (Location 21).
SEQ ID NO 5081 : n represents ttta or deletion (Location 21).
SEQ ID NO 5082 : n represents 11 to 13 repeats of a (from Location 21).
SEQ ID NO 5083 : n represents 8 to 10 repeats of t (from Location 21).
SEQ ID NO 5084 : n represents 12 to 14 repeats of a (from Location 21).
SEQ ID NO 5085 : n represents cttgta or deletion (Location 21).
SEQ ID NO 5086 : n represents 9 to 10 repeats of a (from Location 21).
SEQ ID NO 5087 : n represents ctt or deletion (Location 21).
SEQ ID NO 5088 : n represents ctt or deletion (Location 21).
SEQ ID NO 5090 : n represents a or deletion (Location 21).
SEQ ID NO 5091 : n represents 9 to 11 repeats of a (from Location 21)
SEQ ID NO 5092 : n represents tgt or deletion (Location 21).
SEQ ID NO 5093 : n represents 24 to 27 repeats of a (from Location 21)
SEQ ID NO 5094 : n represents 10 to 21 repeats of ta (from Location 21)
SEQ ID NO 5095 : n represents 8 to 10 repeats of a (from Location 21)
SEQ ID NO 5096 : n represents 11 to 13 repeats of a (from Location 21)
SEQ ID NO 5097 : n represents 8 to 10 repeats of a (from Location 21)
SEQ ID NO 5155 : n represents ctat or deletion (Location 21).
SEQ ID NO 5156 : n represents atattcacttggtatctg or deletion (Location 21).
SEQ ID NO 5157 : n represents ttta or deletion (Location 21).
SEQ ID NO 5158 : n represents t or deletion (Location 21).
SEQ ID NO 5160 : n represents g or deletion (Location 21).
SEQ ID NO 5161 : n represents a or deletion (Location 21).
SEQ ID NO 5162 : n represents 9 to 11 repeats of a (from Location 21).

SEQ ID NO 5163 : n represents g or deletion (Location 21).
SEQ ID NO 5164 : n represents 4 to 5 repeats of at (from Location 21).
SEQ ID NO 5165 : n represents 7 to 8 repeats of t (from Location 21).
SEQ ID NO 5166 : n represents 19 to 23 repeats of t (from Location 21).
SEQ ID NO 5167 : n represents t or deletion (Location 21).
SEQ ID NO 5168 : n represents tgat or deletion (Location 21).
SEQ ID NO 5169 : n represents 8 to 10 repeats of t (from Location 21).
SEQ ID NO 5170 : n represents a or deletion (Location 21).
SEQ ID NO 5187 : n represents gtg or deletion (Location 21).
SEQ ID NO 5189 : n represents gg or tggtggggtgga (Location 21).
SEQ ID NO 5209 : n represents acaaca or deletion (Location 21).
SEQ ID NO 5210 : n represents 11 to 13 repeats of t (from Location 21).
SEQ ID NO 5212 : n represents 15 to 18 repeats of ac (from Location 21).
SEQ ID NO 5218 : n represents 18 to 26 repeats of t (from Location 21).
SEQ ID NO 5227 : n represents tc or deletion (Location 21).
SEQ ID NO 5231 : n represents 16 to 18 repeats of t (from Location 21).
SEQ ID NO 5246 : n represents 18 to 20 repeats of t (from Location 21).
SEQ ID NO 5247 : n represents tggtaagt or deletion (Location 21).
SEQ ID NO 5249 : n represents t or deletion (Location 21).
SEQ ID NO 5255 : n represents g or deletion (Location 21).
SEQ ID NO 5256 : n represents g or deletion (Location 21).
SEQ ID NO 5257 : n represents c or deletion (Location 21).
SEQ ID NO 5258 : n represents ctct or deletion (Location 21).
SEQ ID NO 5261 : n represents a or deletion (Location 21).
SEQ ID NO 5264 : n represents t or deletion (Location 21).
SEQ ID NO 5271 : n represents 14 to 17 repeats of t (from Location 21).
SEQ ID NO 5276 : n represents 12 to 15 repeats of t (from Location 21).
SEQ ID NO 5277 : n represents 10 to 13 repeats of a (from Location 21).
SEQ ID NO 5278 : n represents 25 to 27 repeats of a (from Location 21).
SEQ ID NO 5299 : n represents c or deletion (Location 21).
SEQ ID NO 5308 : n represents 20 to 24 repeats of t (from Location 21).
SEQ ID NO 5311 : n represents t or deletion (Location 21).
SEQ ID NO 5312 : n represents t or deletion (Location 21).
SEQ ID NO 5314 : n represents g or deletion (Location 21).
SEQ ID NO 5320 : n represents 18 to 23 repeats of t (from Location 21).
SEQ ID NO 5340 : n represents c or deletion (Location 21).
SEQ ID NO 5400 : n represents a or deletion (Location 21).
SEQ ID NO 5404 : n represents a or deletion (Location 21).
SEQ ID NO 5407 : n represents tt or deletion (Location 21).
SEQ ID NO 5410 : n represents at or deletion (Location 21).
SEQ ID NO 5436 : n represents tgt or deletion (Location 21).
SEQ ID NO 5445 : n represents t or deletion (Location 21).
SEQ ID NO 5550 : n represents t or deletion (Location 21).
SEQ ID NO 5556 : n represents g or deletion (Location 21).
SEQ ID NO 5557 : n represents 11 to 13 repeats of t (from Location 21).
SEQ ID NO 5559 : n represents a or deletion (Location 21).
SEQ ID NO 5561 : n represents 9 to 11 repeats of t (from Location 21).
SEQ ID NO 5564 : n represents t or deletion (Location 21).
SEQ ID NO 5566 : n represents t or deletion (Location 21).
SEQ ID NO 5570 : n represents t or deletion (Location 21).
SEQ ID NO 5575 : n represents aaga or deletion (Location 21).
SEQ ID NO 5579 : n represents aaaa or deletion (Location 21).
SEQ ID NO 5583 : n represents 9 to 11 repeats of t (from Location 21).
SEQ ID NO 5591 : n represents a or deletion (Location 21).
SEQ ID NO 5614 : n represents 11 to 13 repeats of t (from Location 21).
SEQ ID NO 5630 : n represents acta or deletion (Location 21).
SEQ ID NO 5636 : n represents gtg or deletion (Location 21).
SEQ ID NO 5641 : n represents 11 to 12 repeats of t (from Location 21).
SEQ ID NO 5651 : n represents tta or deletion (Location 21).
SEQ ID NO 5665 : n represents g or deletion (Location 21).
SEQ ID NO 5667 : n represents a or deletion (Location 21).
SEQ ID NO 5669 : n represents cct or deletion (Location 21).
SEQ ID NO 5680 : n represents gga or deletion (Location 21).
SEQ ID NO 5690 : n represents 12 to 14 repeats of t (from Location 21).
SEQ ID NO 5695 : n represents 16 to 17 repeats of t (from Location 21).
SEQ ID NO 5707 : n represents g or deletion (Location 21).
SEQ ID NO 5740 : n represents c or deletion (Location 21).
SEQ ID NO 5800 : n represents ag or deletion (Location 21).
SEQ ID NO 5806 : n represents g or deletion (Location 21).
SEQ ID NO 5807 : n represents a or deletion (Location 21).
SEQ ID NO 5835 : n represents g or deletion (Location 21).
SEQ ID NO 5839 : n represents c or deletion (Location 21).
SEQ ID NO 5844 : n represents ct or deletion (Location 21).
SEQ ID NO 5846 : n represents gc or deletion (Location 21).
SEQ ID NO 5849 : n represents c or deletion (Location 21).
SEQ ID NO 5884 : n represents c or deletion (Location 21).
SEQ ID NO 5890 : n represents tc or deletion (Location 21).
SEQ ID NO 5902 : n represents c or deletion (Location 21).
SEQ ID NO 5904 : n represents g or deletion (Location 21).
SEQ ID NO 5917 : n represents a or deletion (Location 21).
SEQ ID NO 5921 : n represents ca or deletion (Location 21).
SEQ ID NO 5922 : n represents t or deletion (Location 21).
SEQ ID NO 5934 : n represents ct or deletion (Location 21).
SEQ ID NO 5965 : n represents a or deletion (Location 21).
SEQ ID NO 5980 : n represents t or deletion (Location 21).
SEQ ID NO 5981 : n represents t or deletion (Location 21).
SEQ ID NO 5981 : n represents 11 to 13 repeats of t (from Location 21).
SEQ ID NO 5987 : n represents t or deletion (Location 21).
SEQ ID NO 5989 : n represents 16 to 18 repeats of t (from Location 21).
SEQ ID NO 5991 : n represents ctta or deletion (Location 21).
SEQ ID NO 5992 : n represents c or deletion (Location 21).
SEQ ID NO 5994 : n represents 10 to 12 repeats of a (from Location 21).
SEQ ID NO 5995 : n represents gt or deletion (Location 21).
SEQ ID NO 5996 : n represents a or deletion (Location 21).
SEQ ID NO 6001 : n represents aatt or deletion (Location 21).
SEQ ID NO 6003 : n represents t or deletion (Location 21).
SEQ ID NO 6009 : n represents g or deletion (Location 21).
SEQ ID NO 6021 : n represents at or deletion (Location 21).
SEQ ID NO 6027 : n represents 4 to 5 repeats of caaaa (from Location 21).
SEQ ID NO 6036 : n represents 9 to 10 repeats of a (from Location 21).
SEQ ID NO 6041 : n represents a or deletion (Location 21).
SEQ ID NO 6047 : n represents t or deletion (Location 21).
SEQ ID NO 6051 : n represents t or deletion (Location 21).
SEQ ID NO 6052 : n represents g or deletion (Location 21).
SEQ ID NO 6060 : n represents t or deletion (Location 21).
SEQ ID NO 6061 : n represents t or deletion (Location 21).
SEQ ID NO 6062 : n represents a or deletion (Location 21).
SEQ ID NO 6072 : n represents gaa or deletion (Location 21).
SEQ ID NO 6073 : n represents ag or deletion (Location 21).
SEQ ID NO 6089 : n represents 9 to 11 repeats of t (from Location 21).
SEQ ID NO 6090 : n represents a or deletion (Location 21).
SEQ ID NO 6091 : n represents t or deletion (Location 21).
SEQ ID NO 6173 : n represents tat or deletion (Location 21).
SEQ ID NO 6174 : n represents 14 to 17 repeats of ac (from Location 21).
SEQ ID NO 6175 : n represents 16 to 27 repeats of a (from Location 21).
SEQ ID NO 6176 : n represents t or deletion (Location 21).
SEQ ID NO 6177 : n represents 8 to 10 repeats of a (from Location 21).
SEQ ID NO 6178 : n represents 9 to 11 repeats of gt (from Location 21).
SEQ ID NO 6179 : n represents aa or deletion (Location 21).
SEQ ID NO 6180 : n represents t or deletion (Location 21).
SEQ ID NO 6181 : n represents 8 to 12 repeats of ac (from Location 21).
SEQ ID NO 6182 : n represents a or deletion (Location 21).
SEQ ID NO 6202 : n represents agg or deletion (Location 21).
SEQ ID NO 6204 : n represents 11 to 15 repeats of a (from Location 21).
SEQ ID NO 6205 : n represents 11 to 14 repeats of a (from Location 21).
SEQ ID NO 6208 : n represents gt or deletion (Location 21).
SEQ ID NO 6224 : n represents ta or deletion (Location 21).
SEQ ID NO 6307 : n represents 16 to 19 repeats of a (from Location 21).
SEQ ID NO 6308 : n represents aa or deletion (Location 21).
SEQ ID NO 6310 : n represents t or deletion (Location 21).
SEQ ID NO 6311 : n represents 10 to 12 repeats of t (from Location 21).
SEQ ID NO 6312 : n represents aa or deletion (Location 21).
SEQ ID NO 6313 : n represents ttgacagtccaatat, ttgaca, gtccaatat or deletion (Location 21).
SEQ ID NO 6314 : n represents cta or deletion (Location 21).
SEQ ID NO 6315 : n represents a or deletion (Location 21).
SEQ ID NO 6317 : n represents 9 to 11 repeats of t (From Location 21).
SEQ ID NO 6318 : n represents c or deletion (Location 21).
SEQ ID NO 6320 : n represents gagatgttgtggctcacat or deletion (Location 21).
SEQ ID NO 6322 : n represents cc or deletion (Location 21).

SEQ ID NO 6323 : n represents act or deletion (Location 21).
SEQ ID NO 6405 : n represents a or deletion (Location 21).
SEQ ID NO 6415 : n represents 8 to 11 repeats of t (from Location 21).
SEQ ID NO 6416 : n represents 10 to 13 repeats of t (from Location 21).
SEQ ID NO 6472 : n represents g or deletion (Location 21).
SEQ ID NO 6473 : n represents c or deletion (Location 21).
SEQ ID NO 6554 : n represents t or deletion (Location 21).
SEQ ID NO 6555 : n represents 12 to 15 repeats of t (from Location 21).
SEQ ID NO 6609 : n represents a or deletion (Location 21).
SEQ ID NO 6610 : n represents at or deletion (Location 21).
SEQ ID NO 6725 : n represents 16 repeats of cctgc or 16 repeats of cctgt (from Location 21).
SEQ ID NO 6726 : n represents t or deletion (Location 21).
SEQ ID NO 6728 : n represents c or deletion (Location 21).
SEQ ID NO 6739 : n represents acac or deletion (Location 21).
SEQ ID NO 6748 : n represents gatttgtggtatccag or deletion (Location 21).
SEQ ID NO 6750 : n represents ag or deletion (Location 21).
SEQ ID NO 6751 : n represents ta or deletion (Location 21).
SEQ ID NO 6757 : n represents t or deletion (Location 21).
SEQ ID NO 6759 : n represents 12 to 14 repeats of gt from Location 21).
SEQ ID NO 6771 : n represents cagaggct or deletion (Location 21).
SEQ ID NO 6772 : n represents ct or deletion (Location 21).
SEQ ID NO 6773 : n represents ag or deletion (Location 21).
SEQ ID NO 6785 : n represents gtaaa or deletion (Location 21).
SEQ ID NO 6786 : n represents aaaaa or deletion (Location 21).
SEQ ID NO 6787 : n represents a or deletion (Location 21).
SEQ ID NO 6828 : n represents tc or deletion (Location 21).
SEQ ID NO 6830 : n represents t or deletion (Location 21).
SEQ ID NO 6831 : n represents t or deletion (Location 21).
SEQ ID NO 6832 : n represents gaagaaactgttgacagttt or deletion (Location 21).
SEQ ID NO 6833 : n represents cct or deletion (Location 21).
SEQ ID NO 6834 : n represents tttc or deletion (Location 21).
SEQ ID NO 6835 : n represents ttcttttaaaattg or deletion (Location 21).
SEQ ID NO 6837 : n represents ttcaggccttt or deletion (Location 21).
SEQ ID NO 6839 : n represents ggcctg or deletion (Location 21).
SEQ ID NO 6841 : n represents a or deletion (Location 21).
SEQ ID NO 6870 : n represents 9 to 11 repeats of c (from Location 21).
SEQ ID NO 6871 : n represents 15 to 21 repeats of a (from Location 21).
SEQ ID NO 6872 : n represents ggggtggcggggtggg or deletion (Location 21).
SEQ ID NO 6873 : n represents t or deletion (Location 21).
SEQ ID NO 6874 : n represents a or deletion (Location 21).
SEQ ID NO 6876 : n represents a or deletion (Location 21).
SEQ ID NO 6877 : n represents 10 to 12 repeats of t (from Location 21).
SEQ ID NO 6878 : n represents tt or deletion (Location 21).
SEQ ID NO 6880 : n represents tccctccttgaagctgatcgt or deletion (Location 21).
SEQ ID NO 6881 : n represents 12 to 18 repeats of ca (from Location 21).
SEQ ID. NO 6894 : n represents gtt or deletion (Location 21).
SEQ ID NO 6897 : n represents ga or deletion (Location 21).
SEQ ID NO 6921 : n represents t or deletion (Location 21).
SEQ ID NO 6940 : n represents t or deletion (Location 21).
SEQ ID NO 6941 : n represents t or deletion (Location 21).
SEQ ID NO 6942 : n represents t or deletion (Location 21).
SEQ ID NO 6965 : n represents at or deletion (Location 21).
SEQ ID NO 6966 : n represents a or deletion (Location 21).
SEQ ID NO 6967 : n represents c or deletion (Location 21).
SEQ ID NO 6997 : n represents c or deletion (Location 21).
SEQ ID NO 7005 : n represents t or deletion (Location 21).
SEQ ID NO 7006 : n represents ttc or deletion (Location 21).
SEQ ID NO 7017 : n represents ctt or deletion (Location 21).
SEQ ID NO 7049 : n represents 8 to 9 repeats of a (from Location 21).
SEQ ID NO 7053 : n represents 10 to 12 repeats of t (from Location 21).
SEQ ID NO 7059 : n represents 22 to 25 repeats of t (from Location 21).
SEQ ID NO 7070 : n represents t or deletion (Location 21).
SEQ ID NO 7073 : n represents a or deletion (Location 21).
SEQ ID NO 7074 : n represents a or deletion (Location 21).
SEQ ID NO 7076 : n represents c or deletion (Location 21).
SEQ ID NO 7077 : n represents 10 to 12 repeats of t (from Location 21).
SEQ ID NO 7078 : n represents a or deletion (Location 21).
SEQ ID NO 7079 : n represents 9 to 11 repeats of t (from Location 21).
SEQ ID NO 7082 : n represents a or deletion (Location 21).
SEQ ID NO 7085 : n represents t or deletion (Location 21).
SEQ ID NO 7089 : n represents a or deletion (Location 21).
SEQ ID NO 7101 : n represents a or deletion (Location 21).
SEQ ID NO 7105 : n represents a or deletion (Location 21).
SEQ ID NO 7114 : n represents 9 to 10 repeats of t (from Location 21).
SEQ ID NO 7115 : n represents aag or deletion (Location 21).
SEQ ID NO 7117 : n represents t or deletion (Location 21).
SEQ ID NO 7118 : n represents t or deletion (Location 21).
SEQ ID NO 7120 : n represents t or deletion (Location 21).
SEQ ID NO 7121 : n represents t or deletion (Location 21).
SEQ ID NO 7123 : n represents t or deletion (Location 21).
SEQ ID NO 7125 : n represents a or deletion (Location 21).
SEQ ID NO 7127 : n represents a or deletion (Location 21).
SEQ ID NO 7134 : n represents 7 to 8 repeats of gt (from Location 21).
SEQ ID NO 7146 : n represents cct or deletion (Location 21).
SEQ ID NO 7148 : n represents tc or deletion (Location 21).
SEQ ID NO 7164 : n represents ca or deletion (Location 21).
SEQ ID NO 7186 : n represents g or deletion (Location 21).
SEQ ID NO 7209 : n represents t or deletion (Location 21).
SEQ ID NO 7238 : n represents gccag or deletion (Location 21).
SEQ ID NO 7278 : n represents a or deletion (Location 21).
SEQ ID NO 7281 : n represents g or deletion (Location 21).
SEQ ID NO 7282 : n represents t or deletion (Location 21).
SEQ ID NO 7287 : n represents aaa or deletion (Location 21).
SEQ ID NO 7288 : n represents a or deletion (Location 21).
SEQ ID NO 7299 : n represents c or deletion (Location 21).
SEQ ID NO 7329 : n represents 17 to 19 repeats of a (from Location 21).
SEQ ID NO 7332 : n represents 16 to 18 repeats of a (from Location 21).
SEQ ID NO 7333 : n represents 4 to 6 repeats of ga (from Location 21).
SEQ ID NO 7346 : n represents a or deletion (Location 21).
SEQ ID NO 7375 : n represents 2 to 3 repeats of tc (from Location 21).
SEQ ID NO 7381 : n represents 6 to 7 repeats of a (from Location 21).
SEQ ID NO 7383 : n represents 13 to 15 repeats of a (from Location 21).
SEQ ID NO 7385 : n represents 9 to 10 repeats of t (from Location 21).
SEQ ID NO 7387 : n represents 11 to 14 repeats of a (from Location 21).
SEQ ID NO 7389 : n represents 14 to 17 repeats of t (from Location 21).
SEQ ID NO 7390 : n represents 8 to 9 repeats of a (from Location 21).
SEQ ID NO 7397 : n represents g or deletion (Location 21).
SEQ ID NO 7417 : n represents 14 to 17 repeats of t (from Location 21).
SEQ ID NO 7421 : n represents 7 to 9 repeats of g (from Location 21).
SEQ ID NO 7426 : n represents 9 to 10 repeats of a (from Location 21).
SEQ ID NO 7434 : n represents 9 to 10 repeats of a (from Location 21).
SEQ ID NO 7436 : n represents 6 to 7 repeats of g (from Location 21).
SEQ ID NO 7443 : n represents g or deletion (Location 21).
SEQ ID NO 7458 : n represents 8 to 9 repeats of a (from Location 21).
SEQ ID NO 7461 : n represents 4 to 6 repeats of c (from Location 21).
SEQ ID NO 7483 : n represents ggcgaaggcggcggc or deletion (Location 21).
SEQ ID NO 7485 : n represents ata or deletion (Location 21).
SEQ ID NO 7488 : n represents 11 to 12 repeats of t (from Location 21).
SEQ ID NO 7489 : n represents 12 to 14 repeats of t (from Location 21).
SEQ ID NO 7493 : n represents 9 to 10 repeats of t (from Location 21).
SEQ ID NO 7495 : n represents 6 to 7 repeats of ta (from Location 21).
SEQ ID NO 7497 : n represents tgtatacgtatacatacgtatacatatatacatacgtatata or deletion (Location 21).
SEQ ID NO 7503 : n represents attt or deletion (Location 21).
SEQ ID NO 7510: n represents cct or deletion (Location 21).
SEQ ID NO 7519 : n represents tgtt or deletion (Location 21).
SEQ ID NO 7520 : n represents a or deletion (Location 21).
SEQ ID NO 7531 : n represents 9 to 10 repeats of t (from Location 21).
SEQ ID NO 7538 : n represents a or deletion (Location 21).
SEQ ID NO 7566 : n represents a or deletion (Location 21).
SEQ ID NO 7615 : n represents a or deletion (Location 21).
SEQ ID NO 7649 : n represents gtg or deletion (Location 21).
SEQ ID NO 7651 : n represents gg or tggtggggtgga (Location 21).
SEQ ID NO 7667 : n represents ct or deletion (Location 21).

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be within the scope of the following claims.

## Claims

1. A method of identifying a subject having a polymorphism, comprising:
a) providing nucleic acid from said subject; and
b) detecting the presence of the polymorphism found in SEQ ID NO:7052 in said nucleic acid.

2. The method of Claim 1, wherein said detecting comprises use of a detection assay.

3. The method of Claim 2, wherein said detection assay comprises a hybridization assay, a TAQMAN assay, an invasive cleavage assay, mass spectroscopy, a microarray, a polymerase chain reaction, a rolling circle extension assay, a sequencing assay, a hybridization assay employing a probe complementary to said polymorphism, a bead array assay, a primer extension assay, an enzyme mismatch cleavage assay, a branched hybridization assay, a NASBA assay, a molecular beacon assay, a cycling probe assay, a ligase chain reaction assay, or a sandwich hybridization assay.

4. The method of Claim 2 or 3, wherein said nucleic acid is from a biological sample, said biological sample selected from the group consisting of blood, saliva, amniotic fluid, and tissue.

5. The method of Claim 1, wherein said subject is a mammal.

6. The method of Claim 1, wherein said nucleic acid comprises DNA or RNA.

7. A kit for detecting a polymorphism, comprising at least one reagent that specifically detects a polymorphism found in SEQ ID NO:7052.

8. The kit of claim 7, wherein said at least one reagent comprises a nucleic acid probe.

9. The kit of claim 7, wherein said kit comprises a detection assay.

10. The of Claim 7, comprising at least one PCR primer for amplifying at least a portion of a gene containing a polymorphism found in SEQ ID NO: 7052.
